# EUROPEAN PATENT APPLICATION

(11) **EP 2 578 700 A1**
(43) Date of publication of application: **10.04.2013**
(21) Application number: 11791975.3
(22) Date of filing: 07.06.2011
(51) Int. Cl.: C12Q 1/68

(54) **IN VITRO DIAGNOSTIC METHOD FOR PATIENTS WITH SPLENIC MARGINAL ZONE LYMPHOMA**

(30) Priority: 07.06.2010 ES 201030872
(71) Applicant: Fundación de Investigación Del Cáncer de la Universidad de Salalmanca (FICUS), 37002 Salamanca (ES)
(72) Inventor: HERNÁNDEZ RIVAS, Jesús María, E-37007 Salamanca (ES); GARCÍA HERNÁNDEZ, Juan Luis, E-37008 Salamanca (ES); ROBLEDO MONTERO, Cristina, E-37002 Salamanca (ES)
(74) Representative: Illescas, Manuel
(86) International application number: PCT/ES2011/070406
(87) International publication number: WO 2011/154579

(57) **Abstract**

The present invention refers to an *in vitro* SMZL diagnostic method based on analysis of the expression of a series of genes located in a very specific deleted region of human chromosome 7. The deleted region in SMZL patients has been defined by means of high-resolution array CGH and is located at 7q22.1 between bases 99925039-101754718, preferably between 99925039-101348479, and more preferably between 100260234-100596921 and/or 101244026-101754718, which is the location for the following genes: CUX1, SH2B2, EPHB4, SLC12A9, TRIP6, SRRT, ACHE, UFSP1, TRIM56, SERPINE1, APIS1, VGF, TSC22D4, HRBL, LRCH4, MUC3, MUC12 and MUC17. The present invention further describes in vitro SMZL diagnostic kits that comprise at least one probe that recognizes the deleted region at 7q22.1 or hybridizes with at least one of the nucleotide sequences of the genes mentioned above.

## Description

### Field of the invention

The present invention refers to the fields of biomedicine and pharmacy, more specifically, relates to an *in vitro* method for diagnosis of patients with neoplastic disease: Splenic Marginal Zone Lymphoma (SMZL). Furthermore, the present invention also relates to a kit for *in vitro* diagnosis of patients with SMZL.

### BACKGROUND

The Splenic Marginal Zone Lymphoma (SMZL), is a tumor disease that can involve any organ, but the most common are the spleen and lymph nodes. It has been considered a distinct clinic-pathologic entity since years. (Schmid C et al. Am J Surg Pathol 1992, 16: 455-466). More recently, the classification of hematological diseases of the World Health Organization (WHO) has designated to SMZL as a subtype of non-Hodgkin lymphoma (NHL) (Harris NL, et al. Histopathology 2000, 36: 69-86), based on their morphology, immunophenotype and clinical features, together with mucosa-associated lymphomas (MALT) and nodal marginal zone lymphoma (NMZL). These three subtypes of NHL: SMZL, MALT and NMZL lymphomas are included within the marginal zone lymphomas because have their common origin in the marginal compartment B cells of lymph nodes and spleen.

In the diagnosis of lymphoma, it is important to classify each within subtype it belongs to give the patient the right treatment, as each type has a different treatment lymphoma. Thus, for example, in the case of SMZL, the chemotherapy should not be applied in the first instance, being the most appropriate treatment of these cases, the removal of the affected organ, the spleen generally, so it is very important to make the diagnosis correctly. Diagnosis of NHL and the differentiating between each of them are based on the morphological, immunohistochemical and molecular features, being the genetics changes in general and the specific translocations in particular, one of the features that are helping to us to distinguish some lymphomas from the others. Most NHLs are characterized by the presence of specific translocations, which help for diagnosis of these diseases. However, SMZL not known a specific molecular marker, unlike other lymphomas, has not been identified in them a characteristic translocation, which makes a diagnosis. Although cytogenetic analyses show that in these type of tumours can be detected losses in which are involved chromosomes 1, 3, 7 and 8 (Dierlamm J, et al., Blood 1996.87: 299-307; Solé F, et al., Br J Haematol 1997; 98: 446-449), there are few studies that demonstrate the existence of a specific alteration that is characteristic of SMZL and leading to the identification of a high proportion of patients suffering the disease.

Conventional cytogenetic studies showed that one of the most frequent alterations in SMZL were the losses on the long arm of chromosome 7 (del(7q)), which could suggest that the del(7q) is associated with SMZL (Brynes RK, et al. Mod Pathol 1996, 9: 995-1000; Hernandez JM, et al. Leukemia 1995, 9: 2140-2146). In these first studies was estimated that deletions in the long arm of chromosome 7 were not present in more than 25% of patients suffering SMZL (Franco V, et al. Blood 2003, 101:2464-2472), it was not considered that the development of diagnostic methods based on their detection could be interesting, because these methods identify only a very small proportion of all patients suffering SMZL. Thereafter, loss of heterozygosity studies demonstrated that the presence of an allelic loss in SMZL patients (40%) is higher than the allelic loss observed in other B-cell lymphoproliferative disorders.

The most commonly technique used for the study of chromosomes changes associated with SMZL, has been the Comparative Genomic Hybridization (CGH), which determines the gains and the losses in the copy number between two DNA samples, by competitive hybridization of a DNA normal (control) and a tumour DNA (each DNA samples labelled with different fluorophore) on metaphases of control (normal) subjects, then evaluating whether there are areas in which an increase of the signal corresponding to the fluorophore which has marked the tumor DNA (which is considered indicative of gains) or decreases the signal, increasing the signal corresponding to the other fluorophore, the normal or control DNA (which is considered indicative of losses). CGH studies confirmed that there are regions of the genome involved in these lymphomas, such as 5q, 7q, 9q, 12q and 20q and found also that there is a correlation between the loss of genomic material and shortening of the survival period (Hernandez JM, et al Am J Pathol. 2001 May; L58 :1843-50).

Using these techniques it has been found that in particular the 7q31-q36 region appears to be closely related to SMZL and deletions are often found in this region in patients suffering this disease. This region comprises a very wide genome area in which candidate genes have not been found which would help for the diagnosis of the disease (Algara P, et al., Blood 2002, 99: 1299-1304; Leisure EM, et al. , Clinical Lymphoma 2005.4: 241-245). Despite having been very useful in understanding the genomic alterations in tumor processes and especially in lymphomas, (to whose knowledge, classification and prognosis has been very useful), the CGH method has a limited resolution capacity: firstly, changes involving deletions of less than 7 Mb are not detectable (Bentz M, et al. Genes Chromosomes Cancer 1998, 21:172-175) and on the other hand, to determine the exact point of chromosome in which the amplification is inserted or are the limits point of the deletion, is difficult, since the minimal deleted region is too wide to be analysed with a in situ hybridization probe (Hernandez JM, et al. am J Pathol. 2001 May; I 58:1843-50).

Losses of the large arm of chromosome 7, as already mentioned, is one of the more alterations which have been detected so far in the SMZL studies, either by CGH method or by Fluorescent In Situ Hybridization (FISH). However, the wide deleted regions found by these techniques, have shown problems when defining or delimiting them, because the probes used were large and not showing enough specificity and security for use in the diagnosis of the disease.

Spanish patent ES2284408 solves this problem by using genomic arrays, also called CGH-array or array of CGH. The CGH-arrays are a method to identify alterations in the genome, without being able to detect reciprocal translocations. This is a technique that combine the technology of micro-arrays with the CGH-technology, wherein the hybridization is performed by replacing the metaphase of the chromosomes with mapped DNA segments, whose set represent all chromosomes or chromosome arms, divided into fragments cloned into Yeast Artificial Chromosomes (YAC), Bacterial Artificial Chromosomes (BACs), Artificial Chromosomes based on PI bacteriophage genome (PACs), cosmids or other cloning vectors. Thus, the level of resolution increases from chromosomic levels (distances measured in megabases) to be able to be measured in kilobases. The advantages of this method are the higher resolution and the ability to perform a direct mapping from the clone or the clones that are involved in genomic alterations. Spanish patent ES2284408 discloses the use of a specific BAC/PAC array which includes 3500 clones with a resolution of 1 Mb. The data disclose in this Spanish patent show that patients suffering SMZL have a deletion in the 7q22.1-q22.2 region which is defined by the probe carrying the sequence corresponding to the fragment of the human chromosome 7 included in the BAC/PAC array, called RP11-44M6. This patent also discloses the optional use, in the same assay, of the other different probes that hybridize with another different deleted region on chromosome 7, the 7q32-q33 region, increasing the percentage of patients with SMZL who may be diagnosed in the same test.

The present invention discloses an in vitro method for diagnosis of SMZL based on the identification of a specific deletion, located specifically between the bases 99925039-101754718, preferably between the bases 99925039-101348479, in the 7q22.1 region of the human chromosome 7. More preferably, the deleted regions in the 7q22.1 region are the regions located between the bases 100260234-100596921 and 101244026-101754718. The deletion detected by the method discloses in the present invention has an approximate size of 1.4 Mb. Preferably, the two regions deleted, 100260234-100596921 and 101244026-101754718, have, between them, an approximate size of 0.84 Mb. The genes: *EPHB4, SLC12A9, TRIP6, SRRT, ACHE, UFSPI, TRIM56, SERPINE1, AP1S1, VGF, TSC22D4, HRBL, LRCH4, MUC3, MUC12, MUC17, CUX1* and *SH2B2* (Table 1) are located in the deleted region detected by the method of the invention. To obtain the results shown in the present invention has been used different CGH-arrays and methods to detect loss of heterozygosity (LOH), which have a high resolution, allowing to define a specific region of chromosome 7 located between the bases 99925039-101754718, preferably between 99925039-101348479 bases and more preferably, in the regions between the bases, 100260234-100596921 and 101244026-101754718, in the 7q22.1 region, which is deleted in patients with SMZL.

Therefore, although there are information in the state of the art about different deleted regions of chromosome 7 in patients with SMZL, the method described in the invention, discloses a very specific region, which is specific of the said tumoral process and also, is frequently enough among the patients suffering SMZL, ensuring the diagnosis of a significant proportion of said patients. The obtention of the specific deleted region, in a way so accurate and definite, by the method discloses in the present invention, allows a differential diagnosis of patients with SMZL compared to other types of lymphomas, preferably, chronic lymphoproliferative lymphomas or non-Hodgkin's lymphomas, which are not characterized by the deletion of the region defined in the present invention. On the other hand, the more the deleted regions are defined for the diagnosis of patients suffering SMZL, best known genes involved in the development of these diseases, promoting the design of specific treatments to stop these new tumoral signaling pathways. In this sense, the present invention demonstrates the presence of genes which may function as repressors of proto-oncogenes, tumor suppressors, growth regulators, and which are located in the deleted region in patients suffering SMZL.

### DESCRIPTION OF THE INVENTION

### Brief description of the invention

The present invention discloses the association between SMZL and the existence of deletions in a very specific and defined region of human chromosome 7. The present invention relates to an *in vitro* method for diagnosis of patients suffering SMZL based on the analysis of the expression of a number of genes located in the deleted region of human chromosome 7. The deleted region in patients with SMZL has been defined using high-resolution CGH-arrays and LOH, and it is located at 7q22.1 region between the bases 99925039-101754718, preferably between the bases 99925039-101348479. More preferably, the deleted regions in the 7q22.1 region are located between the bases 100260234-100596921 and 101244026-101754718. The following genes are located in the deleted region detected by the method of the invention: *EPHB4, SLC12A9, TRIP6, SRRT, ACHE, UFSP1, TRIM56, SERPINE1, AP1S1, VGF, TSC22D4, HRBL, LRCH4, MUC3, MUC12, MUC17, CUX1* and *SH2B2* (Table 1) and can be used as candidate genes for disease diagnosis. In this sense, the present invention reveals that patients with SMZL show a differential expression of at least one of the gene selected from: *EPHB4, SLC12A9, TRIP6, SRRT, ACHE, UFSP1, TRIM56, SERPINE1, AP1S1, VGF, TSC22D4, HRBL, LRCH4, MUC3, MUC12, MUC17, CUX1* and *SH2B2* compared to healthy controls or to other patients with a different type of lymphoma, by analyzing samples from tumor tissues, bone marrow or blood. The approximate size of the deleted region is 1.4 Mb. Preferably, the two regions deleted, 100260234-100596921 and 101244026-101754718, have, between them, an approximate size of 0.84 Mb. The data disclose in the present invention show that a 49% of patients with SMZL included in the present study showed the deletion of the 7q22.1 region, detected by the high-resolution CGH-array as described in the present invention.

**Table 1. Genes located in the 7q22.1 deleted region in SMZL patients.**

| **Gene** | **Start (bp)** | **End (bp)** | **N° GeneBank** |
|---|---|---|---|
| *TSC22D4* | 99902078 | 99914838 | BC010406.2, BC001486.2, BC031622.1, BC001966.1, BC002972.1 |
| *HRBL* | 99974770 | 99979594 | BC009393 |
| *LRCH4* | 100009570 | 100021712 | BC018529 |
| *EPHB4* | 100238122 | 100263079 | BC052804 |
| *SLC12A9* | 100288293 | 100302570 | BC000154 |
| *TRIP6* | 100302885 | 100309012 | BC028985, BC021540, BC004249, BC004999, BC002680 |
| *SRRT* | 100310636 | 100324221 | BC109117 |
| *UFSP1* | 100324279 | 100325275 | BC067904 |
| *ACHE* | 100325550 | 100331477 | BC143469, BC105060, BC105062 |
| *MUC3A*/*B* | 100388685 | 100397831 | AF007194, AB038783, AF143372, AJ606308 |
| *MUC12* | 100434795 | 100448936 | AK024856 |
| *MUC17* | 100450083 | 100488860 | BC126315.1 |
| *TRIM56* | 100515505 | 100520609 | BC005847, BC048194 |
| *SERPINE1* | 100557098 | 100569262 | BC002488.2, BC008045.2, BC026916.1, BC003049.1, BC020555.1, BC019273.1, BC017449.1, BC10860.1 |
| *AP1S1* | 100584405 | 100591277 | BC003561 |
| *VGF* | 100592509 | 100595572 | BC063835, BC044212 |
| *CUX1* | 101247602 | 101680064 | BC066592 |
| *SH2B2* | 101715166 | 101748898 | NM_020979 |

In the present invention the term "high-resolution CGH-array" is defined as a molecular cytogenetic method for the detection of chromosomal gains and losses that combines the CGH technology with the micro-arrays technology. The high-resolution genomic array is preferably a solid support to which is adhered by a biorobot and orderly clones and/or DNA sequences known, as a matrix of thousands of equally spaced dots. The affinity or resolution of genomic arrays is determined by the genomic distance between two consecutive clones. This technique is based on the co-hybridization on the array of two DNAs, tumor and control (reference), labeled with different fluorophores.

The high-resolution CGH-arrays used in the present invention are: BAC/PAC array containing 3,523 clones from BACs and PACs that cover the entire genome with a resolution of 1 Mb. To corroborate the results obtained from this platform have used FISH assays. Subsequently, to further ensure these results are made use of two commercial genomic arrays platforms: Agilent 44K CGH-array and NimbleGen 72K CGH-array. The data obtained by these techniques confirmed that patients with SMZL have a deletion in the 7q22.1 region of human chromosome 7. Moreover, a human CGH 385K Chromosome 7 Tiling Array, was used to redefine the specific area deleted in the 7q22.1 region on human chromosome 7 in SMZL patients. This array includes approximately 385,110 oligonucleotides or targets covering the entire chromosome 7 and leaving an average distance between each oligonucleotide of approximately 355 bp. In addition to high-resolution arrays mentioned above, other high-resolution array was used, specific of the regions between the cytogenetic bands 7q22.1 to 7q33, both inclusive (Chromosome 7: 99158137-137302483). This array including 710,953 probes with an average size of probe of 54 oligonucleotides and an average distance between each nucleotide to -0.7 bp, allowing to map that region with a very high accuracy.

Another aspect of the present invention refers to the kits for the diagnosis of SMZL patients comprising at least a probe to recognize the deleted region at 7q22.1 or that hybridizes with at least one of the nucleotide sequences of the genes mentioned above (Table 1), wherein such probes are bound to a compound, preferably a fluorophore, which allows detection.

The *in vitro* method discloses in the present invention allows to delimitate with a great precision the deleted region in 7q22.1, and a to do a better differential diagnosis of SMZL subjects versus subjects with other chronic lymphoproliferative disorders or non-Hodgkin lymphomas, since the specific deletion in the region 7q is highly specific of the SMZL and not of the rest of chronic lymphoproliferative disorders or non-Hodgkin lymphomas.

### Description of the figures.

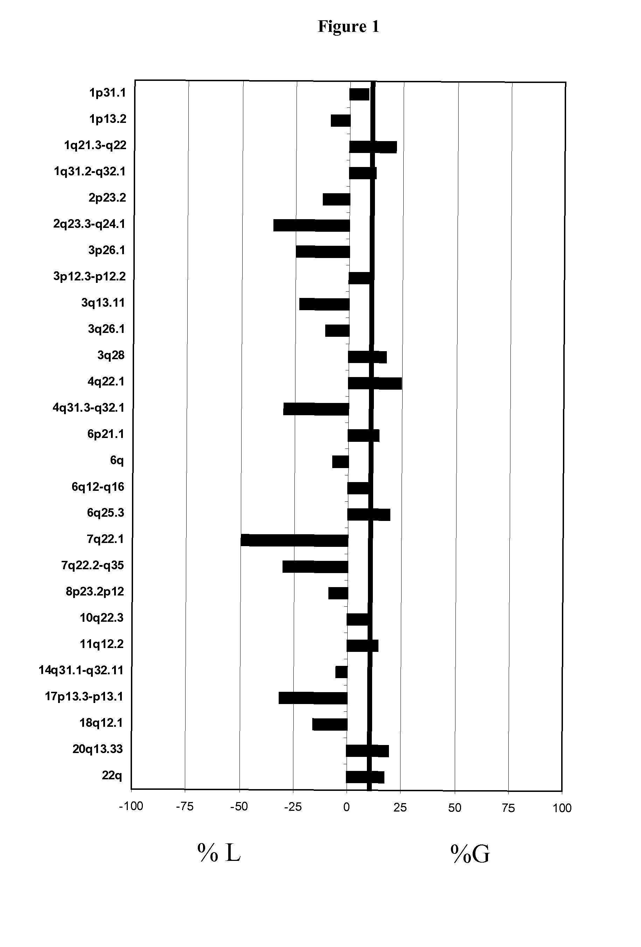

**Figure 1****. Common genomic regions of gains or losses on chromosome 7 of 57 patients with SMZL.** The figure shows the genomic regions that present gains (G) expressed as percentage, and losses (L) expressed as percentage, on chromosome 7 of SMZL patients. For each region, it is shows the cytogenetic location and the gain (% G) and losses (% L) expressed as percentage.

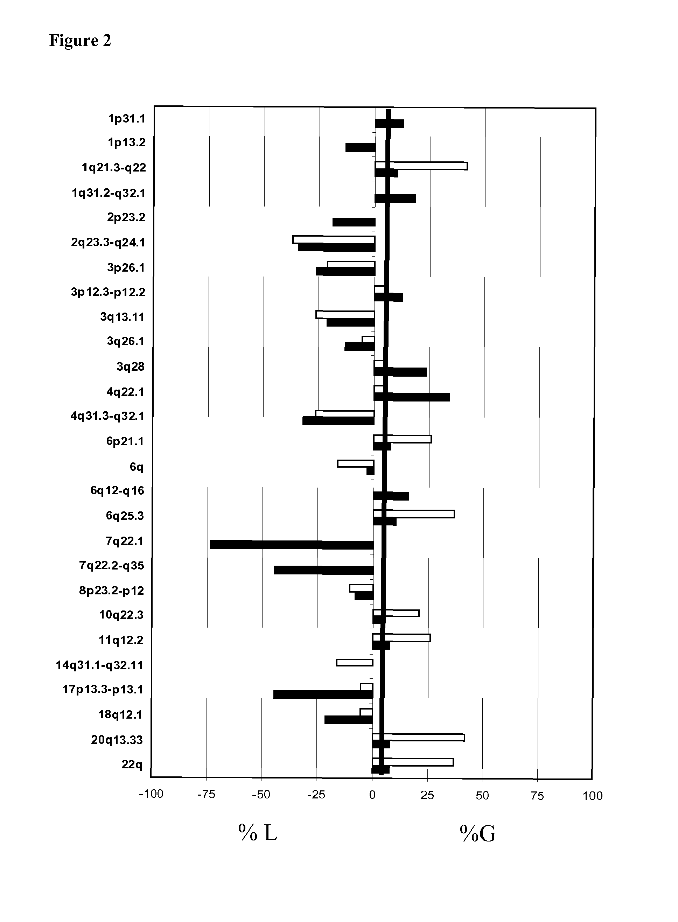

**Figure 2****. Genomic regions involved in SMZL patients showed or not alterations in chromosome 7q.** The figure shows the gains (G), expressed as a percentage, and losses (L), expressed as percentage of patients with losses on chromosome 7q (black bars) and patients who do no showed losses on chromosome 7q (white bars).

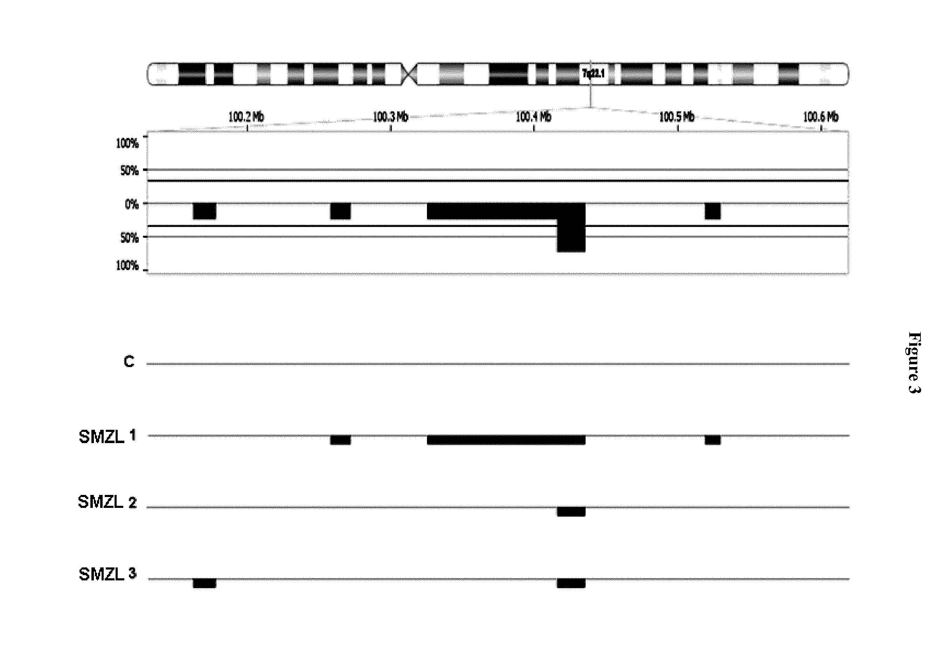

**Figure 3****. Analysis of chromosome 7 by the specific high-resolution Tiling array of the chromosome 7 from NimbleGen (385K) in patients with SMZL.** The figure shows the deletion of the 7q22.1 region on three of the four cases discussed, shown as thick black lines. C: Control; SMZL 1, SMZL 2 and SMZL 3: patients with SMZL.

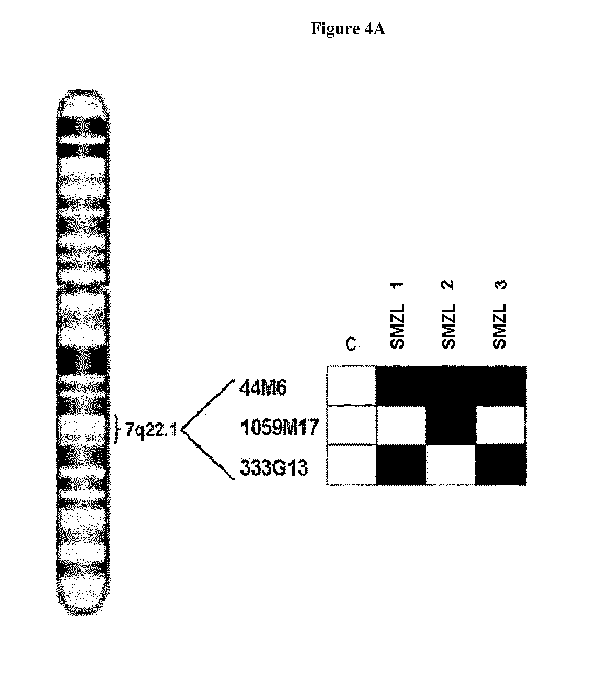

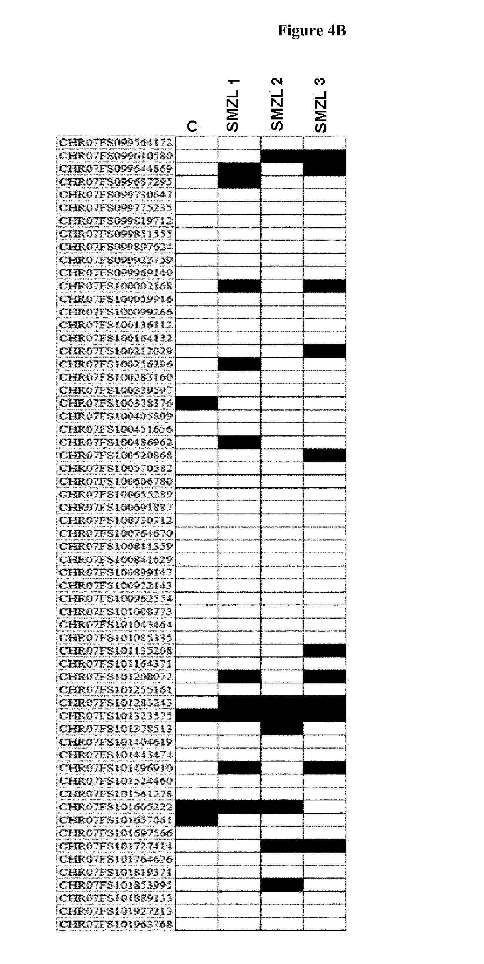

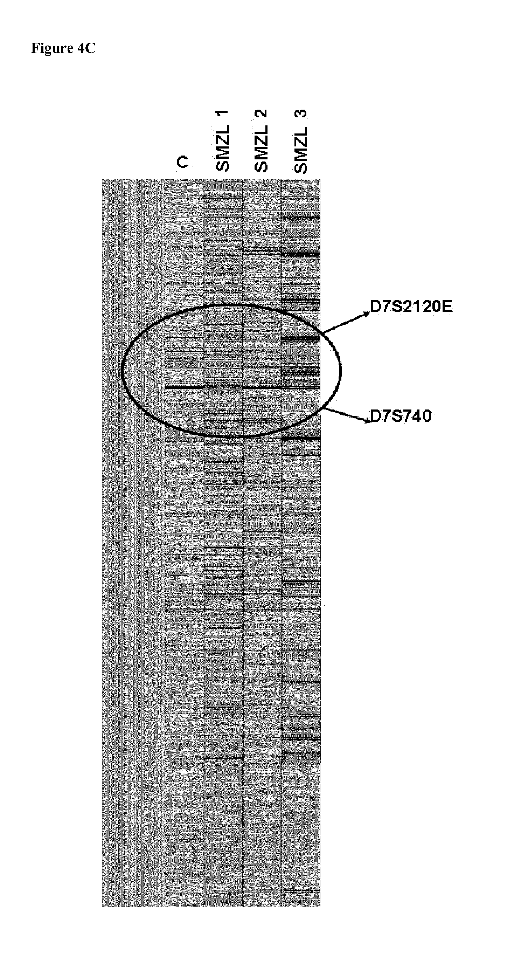

**Figure 4****: Analysis of the commonly deleted region of chromosome 7, 7q22.1 in three patients with SMZL, by different CGH-arrays used in the invention. (A)** The figure shows the three clones covering the region 7q22.1 of chromosome 7 analyzed by BAC/PAC CGH-array. The three patients with SMZL (SMZL 1, SMZL 2, and SMZL 3) show losses (in black) in the region 7q22.1 and no changes were observed in the control subject (C). The region 7q22.1 in this array is mapped by three clones with a resolution of 1Mb. **(B)** The figure shows the 53 oligonucleotides included in the present CGH-array NimbleGen 72 K for the 7q22.1 region. The three patients with SMZL (SMZL 1, SMZL 2 and SMZL 3) showed losses in the 7q22.1 region (black), while the control (C) showed no changes in this region. **(C)** The figure shows the 3.553 oligonucleotides covering the region 7q22.1 in the specific Tiling-array of the chromosome 7. It is observed that the commonly deleted region in three patients with SMZL (SMZL 1, SMZL 2 and SMZL 3) analyzed is delimited between the loci D7S2120E and D7S740. C: control.

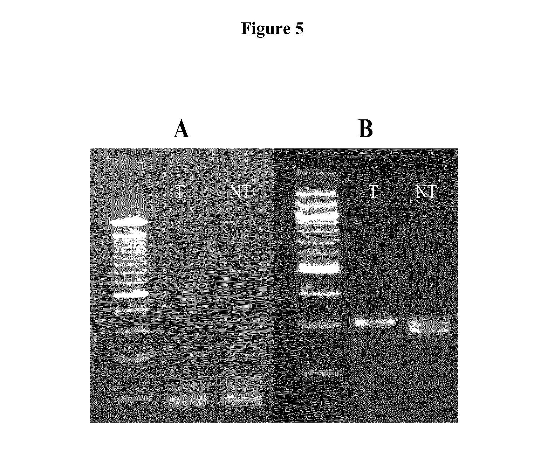

**Figure 5****. Representation of the patterns of loss of heterozygosity (LOH) obtained by analyzing the microsatellite markers in SMZL patients.** The figure shows a photography of agarose gels showing losses or not of the heterozygosity in SMZL patients. A) No LOH and B) LOH. T: tumor sample (spleen), NT: no tumor sample (bone marrow or peripheral blood).

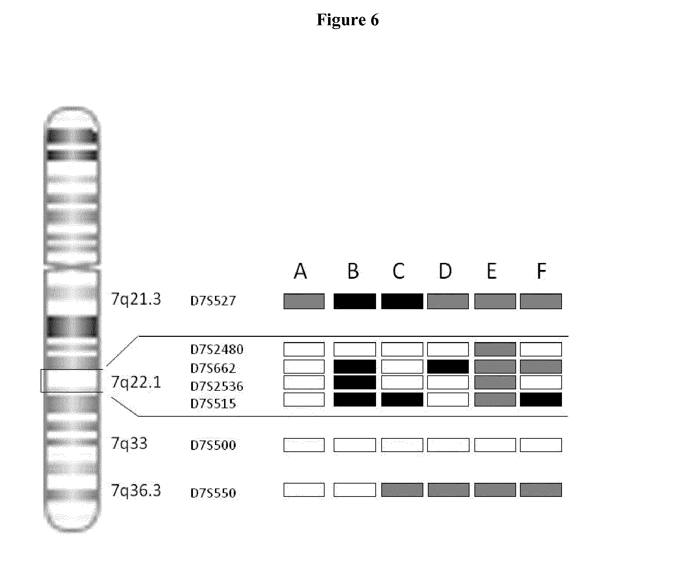

**Figure 6****. LOH analysis in chromosome 7q, specifically in the 7q22.1 region of patients diagnosed of SMZL.** The left of the figure represents the chromosome 7 including cytogenetic bands. To the right shows an enlargement of the 7q22.1 region and the microsatellite markers analyzed in this region. The white boxes represent heterozygosity. The black boxes represent LOH and gray boxes represent non-informative samples. The letters A, B, C, D, E and F represent the patients analyzed. It can be seen as 4 out of 6 patients analyzed (B, C, D and F) show LOH for any of the markers included in the 7q22.1 region (D7S662, D7S2536, D7S515).

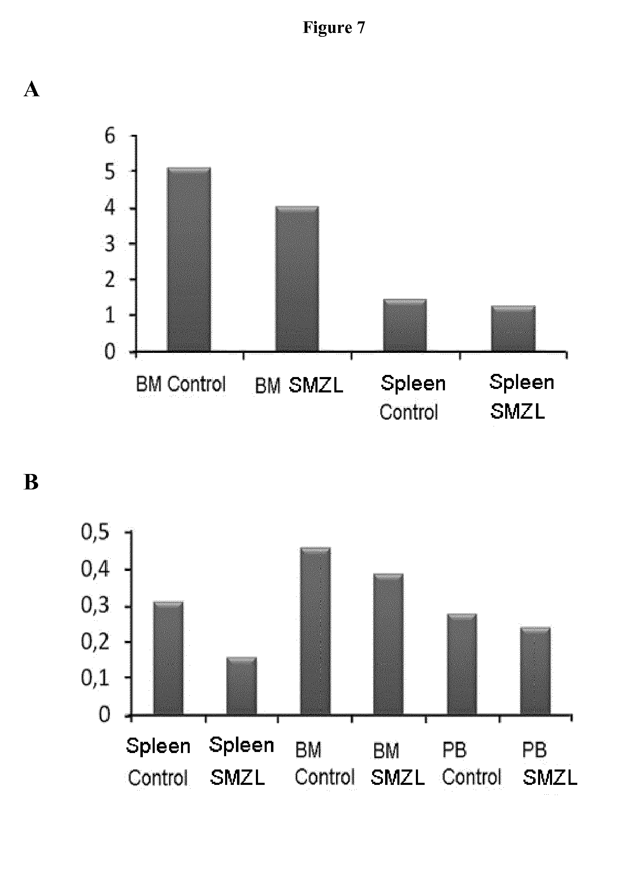

**Figure 7****. *CUX1* gene expression in patients with SMZL.** Analysis of *CUX1* gene expression by RT-PCR in spleen samples, bone marrow (BM) and peripheral blood (PB) of SMZL patients and healthy control (Control). **A** shows *CUX1* gene expression (exons 2-4) and **B** shows *CUX1* gene expression (exon 24). On the Y axis is expressed *CUX1* gene expression in relative terms based on the *ABL* control gene expression.

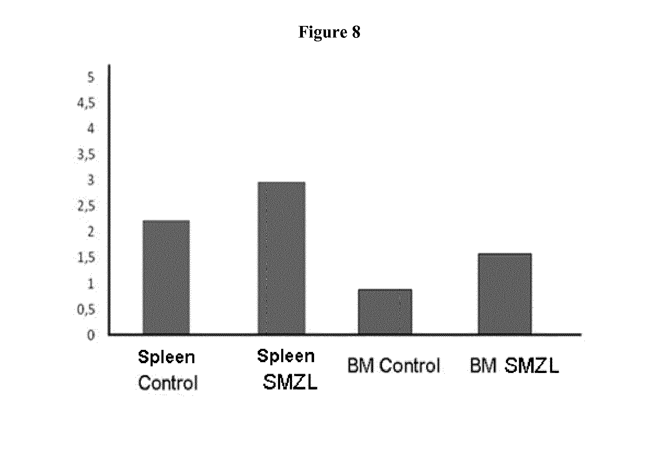

**Figure 8****. *SH2B2* gene expression in patients with SMZL.** Analysis of *SH2B2* gene expression by RT-PCR in spleen samples and bone marrow (BM) to SMZL patients and healthy controls (Control). On the Y axis is expressed *SH2B2* gene expression in relative terms based on the *ABL* control gene expression.

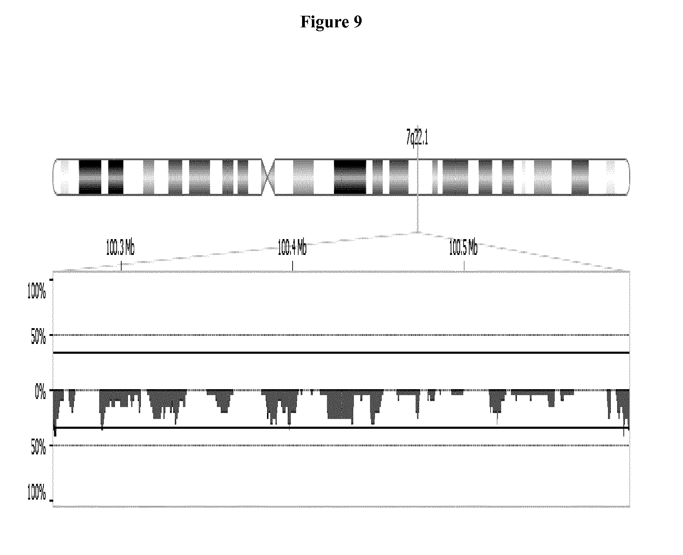

**Figure 9****. Analysis of 7q22.1 region of the chromosome 7 between the bases 100260234-100596921 by high-resolution specific array from NimbleGen for the 7q22.1-7q33 region** in **patients with SMZL.** The figure shows the deletion of the bases 100260234-100596921 in the 7q22.1 region in patients with SMZL.

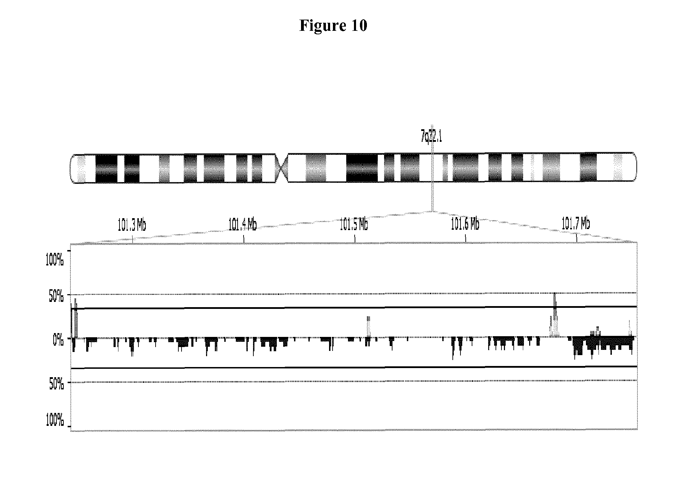

**Figure 10****. Analysis of 7q22.1 region to the chromosome 7 between the bases 101244026-101754718 by high-resolution specific array from NimbleGen for the 7q22.1-7q33 region in patients with SMZL.** The figure shows the deletion of the bases 101244026-101754718 in the 7q22.1 region in patients with SMZL.

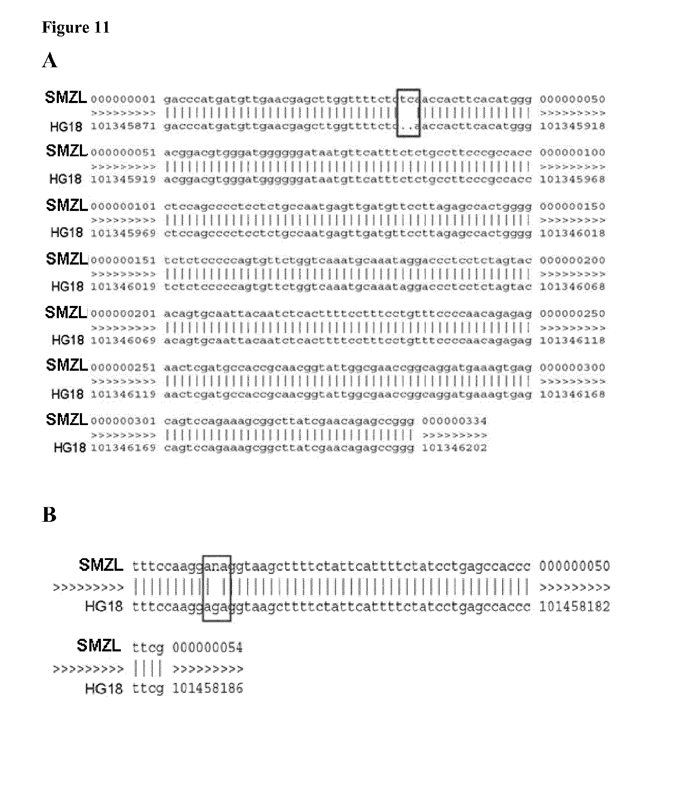

**Figure 11****. Alignment and comparison of *CUX1* sequence gene in patients with SMZL respect to a consensus sequence of this gene obtained from the database (NCBI/HG18). A)** The alignment of both sequences reveals that the intron 1 of *CUX1* sequence in SMZL patients shows an insertion of a thymine and a cytosine at the positions 101345904 and 101345905 (boxed) from the reference genome HG18 obtained from the NCBI database regarding the *CUX1* sequence obtained from SMZL patients. **B)** The alignment of both sequences reveals that the sequence of exon 3 of the *CUX1* gene in SMZL patients includes at the position 101458144 in the reference genome HG18 obtained from the NCBI database the substitution of a guanine for a thymine (G> T) (boxed), ie, the letter "n" represents a thymine, regarding to the *CUX1* sequence gene obtained from patients with SMZL.

### Detailed description of the invention.

The first object of the present invention refers to *an vitro* method for diagnosis of splenic marginal zone lymphoma (SMZL) by high-resolution genomic microarrays, comprising a step for detecting the deletion between the bases 99225039 to 101754718 located in the 7q22.1 region in which is located, at least, the *CUX1* gene.

In a preferred embodiment, the method of the invention is characterized in that the deletion is located between the bases 99925039 to 101348479.

In another preferred embodiment, the method of the invention is characterized in that the deletion is located between the bases 100260234-100596921 and/or 101244026-101754718 in the 7q22.1 region.

In another preferred embodiment, the method of the invention is characterized in that the *CUX1* and *SH2B2* genes are located in these deleted regions.

In another preferred embodiment, the method of the invention is characterized in that further in said region are located, among other, the genes *EPHB4, SLC12A9, TRIP6, SRRT, ACHE, UFSP1, TRIM56, SERPINE1, APIS1, VGF, TSC22D4, HRBL, LRCH4, MUC3, MUC12* and *MUC17.*

In another preferred embodiment, the method of the invention is characterized in that additionally comprises the detection, by high-resolution genomic microarrays, of the deletion between the bases 102949624 to 1459599694 in the 7q22.2-q35 region in which is located, among others, the genes *RELN, LHFPL3, PBEF1, THAP5, IMMP2L, FOXP2, TES, MET, ST7, PTPRZ1, GRMB, UBEH2, MLKN1, BPGM, CALD1, PTN, SVOPL, AGK, KEL, TPK1* and *CNTNAP2.*

In another preferred embodiment, the method of the invention is characterized in that it is performed on a DNA sample selected from: a tissue sample from a lymphoma cell infiltrate, a bone marrow aspirate or a whole blood sample.

In another preferred embodiment, the method of the invention is characterized in that the high-resolution genomic microarrays are selected from BAC/PAC CGH-arrays, Agilent CGH-array, NimbleGen CGH-array or CGH Chromosome 7 Tiling Array from NimbleGen and CGH 7q22.1-q33 region Tiling Array from NimbleGen.

Another object of the present invention relates to an *in vitro* method for diagnosis of splenic marginal zone lymphoma (SMZL) which comprises measuring the expression level of at least the CUX1 gene, located in the 7q22.1 region between the bases 99925039 to 101754718.

In a preferred embodiment, the method of the invention is characterized in that it comprises measuring the expression level of the CUX1 gene located in the 7q22.1 region between the bases 99925039 to 101348479.

In another preferred embodiment, the method of the invention is characterized in that it comprises measuring the expression level of the CUX1 gene located in the 7q22.1 region between the bases 101244026 to 101754718.

In another preferred embodiment, the method of the invention is characterized in that it comprises measuring the expression level of the, at least, the *CUX1* and *SH2B2* genes located in the 7q22.1 region between the bases 101244026 to 101754718.

In another preferred embodiment, the method of the invention is characterized in that it further comprises measuring the expression level of at least one of the following genes selected from: *EPHB4, SLC12A9, TRIP6, SRRT, ACHE, UFSP1, TRIM56, SERPINE1, APIS1, VGF, TSC22D4, HRBL, LRCH4, MUC3, MUC12* and *MUC17,* or combinations thereof, in the 7q22.1 region between the bases 99925039 to 101348479.

In another preferred embodiment, the method of the invention is characterized in that it is performed on a DNA sample selected from: a tissue sample from a lymphoma cell infiltrate, a bone marrow aspirate or a whole blood sample.

Another object of the present invention relates to an *in vitro* method for diagnosis of splenic marginal zone lymphoma (SMZL) which comprises a combination, in any order, of a first step for detecting, by high-resolution genomic microarrays, the deletion between the bases 99225039 to 101754718 located in the 7q22.1 region and a second step for measuring the expression level, of at least, the CUX1 gene.

In a preferred embodiment, the method of the invention is characterized in that the deletion is located between the bases 99925039 to 101348479.

In another preferred embodiment, the method of the invention is characterized the deletion is located between the bases 100260234 to 100596921 and/or 101244026 to 101754718 in the 7q22.1 region.

In another preferred embodiment the method of the invention is characterized in that in the second step the expression levels of the *CUX1* and *SH2B2* genes are measured

In another preferred embodiment, the method of the invention is characterized in that further comprises measuring the expression levels of at least one of the following genes selected from: *EPHB4, SLC12A9, TRIP6, SRRT, ACHE, UFSP1, TRIM56, SERPINE1, APIS1, VGF, TSC22D4, HRBL, LRCH4, MUC3, MUC12* and *MUC17* and/or combinations thereof.

In another preferred embodiment, the method of the invention is characterized in that additionally comprises the detection, by high-resolution genomic microarrays, of the deletion between the bases 102949624 to 1459599694 in the 7q22.2-q35 region in which is located, among others, the genes *RELN, LHFPL3, PBEF1, THAP5, IMMP2L, FOXP2, TES, MET, ST7, PTPRZ1, GRMB, UBEH2, MLKN1, BPGM, CALD1, PTN, SVOPL, AGK, KEL, TPK1* and *CNTNAP2.*

Another object of the present invention relates to a kit for the *in vitro* diagnosis of splenic marginal zone lymphoma (SMZL) comprising a set of probes selected form the group SEQ ID No. 1, SEQ ID No. 2, SEQ ID No. 3, and/or any probe that hybridizes wholly or partially with the deleted region between the bases 99925039 to101754718 by high-density genomic microarrays

In a preferred embodiment, the kit of the invention is characterized in that the selected sets of probes hybridize wholly or partially with the deleted region between the bases 99925039 to101348479.

In another preferred embodiment, the kit of the invention is characterized in that the selected set of probes hybridize wholly or partially with the deleted regions between the bases 100260234 to 100596921 and/or 101244026 to 101754718 of the region7q22.1.

In another preferred embodiment, the kit of the invention is characterized in that it further comprises at least a probe that hybridizes wholly or partially with the deleted region between the bases 102949624 to 1459599694 in the 7q22.2-q35 region, by high-density genomic arrays, preferably the probe is SEQ ID No. 4.

In another preferred embodiment, the kit of the invention is characterized in that the probe hybridizes in the 7q33 region.

Another object of the present invention relates to a kit for the *in vitro* diagnosis of splenic marginal zone lymphoma (SMZL) characterized in the detecting the expression level of at least the CUX1 gene, located in the 7q22.1 region between the bases 99925039 to 101754718 by RT-PCR with the primers defined as SEQ ID No 5 and SEQ ID No. 6.

In a preferred embodiment, the kit of the invention is characterized by measuring the level of expression of *CUX1* gen by RT-PCR, using define probes as SEQ ID NO:5 and SEQ ID NO:6 detected the deletion region between 100260234-100596921 and 10101244026-101754718 on 7q22.1.

In another preferred embodiment, the kit of the invention is characterized in that the expression level of at least the CUX1 gene, by RT-PCR with the primers defined as SEQ ID No 5 and SEQ ID No. 6 it is performed in the deleted region between the bases 99925039 to101348479.

In another preferred embodiment, the kit of the invention is characterized in that the expression level of at least the CUX1 gene, by RT-PCR with the primers defined as SEQ ID No 5 and SEQ ID No. 6 it is performed in the deleted region between the bases 100260234 to 100596921 and/or 101244026 to 101754718 of the region7q22.1.

In another preferred embodiment, the kit of the invention is characterized in that the expression level of the *CUX1* gene it is further performed with the primers defined as SEQ ID Nos. 23-24.

In another preferred embodiment, the kit of the invention is characterized in that additionally comprises the detection of the expression level of SH2B2 gene by the primers defined as SEQ ID Nos 25-28.

In another preferred embodiment, the kit of the invention is characterized in that further comprises detecting the expression levels of at least one of the following genes selected from: *EPHB4, SLC12A9, TRIP6, SRRT, ACHE, UFSP1, TRIM56, SERPINE1, APIS1, VGF, TSC22D4, HRBL, LRCH4, MUC3, MUC12* and *MUC17* and/or combinations thereof by specific primers for the sequences of these genes.

### Examples

The following examples are presented to illustrate the invention and not intended to limit the scope thereof.

### Example 1. DNA extracted from samples of SMZL patients

To obtain the results shown in the present invention Seventy eight patients with the diagnosis of SMZL were included in this study. The diagnosis was established according to the WHO classification. Thirty six patients (56%) were women. The ages ranged from 44 to 86 years (median 69 years). The study was approved by the local ethical committees. Informed consent was obtained from each patient before entering the study.

DNA was extracted from spleen samples, lymphoid node, bone marrow aspirate or samples of whole blood provided that the tissues were infiltrated with cells of SMZL. All genomic DNA was extracted from fresh-frozen samples using the standard phenol-chloroform method. In 53 patients, Tumour DNA was isolated from the spleen, bone marrow and peripheral blood. Normal DNA was extracted from the human placenta of healthy donors. All DNA was quantified using the NanoDrop spectrophotometer (ND-1000, NanoDrop Technologies, Wilmington, DE, USA). DNA quality was assessed by the 260:280 ratios and its integrity by agarose gel ethidium bromide visualization.

### Example 2.- Characterization of genomic imbalances in SMZL by high-resolution BAC/PAC array.

After genomic DNA of SMZL patients and Normal DNA from the healthy donors was extracted, a genomic-wide analysis of DNA copy number changes of patients was performed using BAC CGH-array. To confirm the gains and losses assessed by array-CGH was performed FISH analysis.

In the present stud of SMZL was analyzed using BAC/PAC CGH-array, Agilent CGH-array, NimbleGen CGH-array and by tiling path array specific from chromosome 7

The first genomic-wide analysis of DNA copy number changes of SMZL patients was performed using BAC CGH-array. This BAC/PAC array contained 3528 bacterial artificial chromosomes (BAC) and PAC (P1-Derived Artificial Chromosome) contains targets spaced at ≈ 1Mb density over full genome. Slides were produced at the Centre of Cancer Research (Salamanca, Spain) as previously described. {Robledo, 2009}.Briefly, 2009). Briefly, first we proceeded to the preparation of genomic SMZL DNA patients and healthy controls, to hybridize to the array.

Briefly, 2 µg of genomic DNA of and and reference material (placental DNA) were digested separately with *DpnII* restriction enzyme (New England Biolabs, Beverly, MA) and subsequently proceed to purification by decreasing gradient centrifugations ethanol. The samples of digested and purification genomic DNA (patients and control) were labeled using random primers (Bioprimer labelling kit, Invitrogen) and Cy5-dCTP and Cy3-dCTP (Amersham Biosciences) fluorophores for paired hybridization samples, respectively. The labelled nucleotide incorporation was quantified using the NanoDrop spectrophotometer (ND-1000, NanoDrop Technologies, Wilmington, DE, USA). Labelled test and reference DNA were mixed equitably, co-precipitated in presence of Cot-1 human DNA (Roche, Indianapolis, IN) with ethanol, washed, and resuspended in hybridization solution (50% Formamide, 10% Dextran sulfate, 2X standard saline citrate, 10mM Tris pH 7.6, 2.7% sodium dodecyl sulfate (SDS) and 10µg/µl of yeast tRNA). DNA mixtures were cohybridized to the arrays in the GENETAC (Genomic Solutions) for 48 hours at 42°C according to the manufacturer's recommended protocol. After to array hybridization, images and signal intensities were acquired using the GenePix4000B (Axon Instruments, Burlingame, Calif) dual laser scanner in combination with the GenePixPro4.0 (Axon Instruments) imaging software. Intensity of Fluorescence of every spot were calculated after subtracted the background. Fluorescence ratios were normalized by using the median of fluorescence ratios of every spot computed as log₂ values.

To validate the array were a series of hybridizations man vs woman controls and also marking the DNA used as control hybridizations with samples to be tested with different fluorochromes, to eliminate these clones in hybridizations are altered because they considered that are not affected by to analyze disease. Gains and losses in copy number of regions genomic DNA obtained by array-CGH platform BAC/PAC were identified by creating specific samples thresholds, determining a value of cutting 0.4 (Robledo C et al, Cancer. 2009, 115 (16) :3728-37). To determine which clone is altered to calculate the average values the logarithms of the ratios of the signal corresponding to Cy5 (tumor DNA) versus corresponding to Cy3 (control DNA) of each clone triplicate. A cut off value of 0.4 was used based on the ratios of clones in ten hybridization of normal male versus normal female DNA. The clones with log₂ ratios above or below a control sample's threshold value were considered as gains or losses, respectively. At least two contiguous BAC clones with a log₂ ratio of - 0.4 or less were defined as a loss region and log₂ ratio of + 0.4 or more was defined as a gain region. Furthermore, spots with weak Cy3 or Cy5 intensity (below R² < 0.2) and clones with a standard deviation of more than 0.3 of triplicate spots were excluded from analysis. In total, approximately 10% of clones were excluded.

All data sets were carefully reviewed for frequently affected chromosomal sites of physiologic copy number polymorphisms (CNP). Therefore every clone on the array was compared with the 'Database of Genomic Variants' (available at: www.project.tcag.ca/cariation; November 2009) and that of chromosomal imbalances and phenotype in humans using Ensembl Resources (DECIPHER: available at: http://www.sanger.ac.uk/PostGenomics/decipher/; November 2009).

The results obtained show that 57 (84%) of the patients with SMZL showed genomic changes by BAC/PAc array. The median of changes per patients was four (with a range from 0 to 12).The most frequent changes were chromosomal gains involving 4q22.1 (14/57; 25%), 1q21.3-q22 (12/57; 21%), 6q25.3, 20q13.33 (11/57; 19%), 3q28, 22q (10/57; 18%), 6p21.1, and 11q12.2 (8/57; 14%) while the genomic losses were located on 7q22.1 (28/57; 49%), 2q23.3-q24.1 (20/57; 35%), 17p13.3-p13.1 (18/57; 32%), 4q31.3-q32.1, 7q31-q35 (17/57; 30%), 3p26.1 (14/57; 25%), 3q13.11 (13/57; 23%) and 18q12.1 (9/57; 16%)

Only three cases showed losses on chromosome 14q and only one case had trisomy chromosome 12. Moreover, minimal common region deleted was observed in 18 patients (32%) on chromosome 17p13.1, where TP53 gene is located, which is a suppressor gene tumoral processes involved in induction of cellular response to DNA damage, arresting the cell cycle even if mutations.

In the 19 patients showed no alterations in chromosome 7q median 10 changes was 4 (range 1-8) and more frequent gains were located at the level of 21.3 1q-q22, 20q13.33 (42%), 6q25.3, 22q (37%), 6p21.1, 11q12.2 (26%) and 10q22.3 (21%) while losses in 2q23.3-q24.1 (37%), 4q31.3-q32.1 (26%), 3p26.1 (21%), 6q, 14q31. L-q32.11 (1 6%) and 8p23.2-p12 (10%).

### Example 3.- FISH validation of losses identified by BAC/PAC CGH-array

As methodology confirmation of the results obtained by High-resolution BACIPAC microarray CGH-array, fish studies was performed in a total of 20 patients diagnosed SMZL. By FISH technique can observed by fluorescence microscopy, whether or not a region is deleted, and that the chromosomal region that hybridizes to each of the probes used is duplicate in each of the cells, whether the deleted area is to be noted in cell two signals corresponding to the color with which the fluorophore is labeled the probe. In contrast, if it is deleted, only a single observed color signal corresponding to the fluorophore is labeled the probe.

In all cases FISH analysis confirmed by the results obtained by CGH-array: 7q deletion To corroborate more specifically those results 8 SMZL patients were selected, three of them showed losses in the region BAC/PAC 7q by CGH-array and five showed no genetic changes in this region. The criteria for selection of patients were based on the availability of the sample (spleen sections embedded in paraffin blocks).

The BAC probes used for FISH were RP11-44M6 mapped to 7q22.1 (99873610-100038722 bp), RP11-333G13 mapped to 7q22.1 (101175494-101390682 bp), RP11-36B6 mapped to 7q31.31 (130078078-130270796 bp) and RP-371N6 mapped to 7q33 (134684519-134842787 bp) as previously described (NCB116/hg18). (González MB et al,. Cancer Genet Cytogenet. 2004;150(2):136-43).

These clones were selected from the same BAC CGH-array clones library used for the BAC-array studies (Wellcome Trust Sanger Institute, Cambridge, UK). DNA from the BAC clones was isolated and directly labelled with either Spectrum Green-dUTP or Spectrum Orange-dUTP (Vysis, Downers Grove, IL) by nick translation.

FISH on 7q22.1 region was analyzed in seven of the eight patients Four of these patients showed a loss in these region and the result was confirmed by FISH technique, while the remaining three patients showed no loss by BAC/PAC CGH-array neither FISH technique.

On the other hand, other four patients were analyzed by FISH 7q33.1 region. Two of SMZL patients showed deletions using both techniques: BAC/PAC Array-CGH and FISH. The remaining two patients showed no alterations in the region of 7q33.1 by either techniques used, BACIPAC FISH and array-CGH.

### Example 4.- Analysis of SMZL by commercial CGH-array.

In order to confirm the results provided by the BAC CGH-array analysis, two types of oligonucleotide CGH-arrays platforms were used. Those platforms comprise more affinities and resolution than BAC/PAC array. Those plataforms were Agilent's Human Genome CGH Microarray 44 k (Agilent Technologies).and NimbleGen human CGH 4x72K Whole Genome v2.0 array (NimbleGen Systems, Inc).

Eight SMZL were analyzed with Agilent's Human Genome CGH Microarray 44 k (Agilent Technologies). The microarray contains 44,255 in situ synthesized 60-mer probes spaced at 43,000 bp intervals throughout the human genome and including 3,877 controls. The probes are, according to the manufacturer's description, enriched for cancer relevant genes representing both coding and non-coding sequences on the chromosomes. Experiments using Agilent arrays were performed using placenta human genomic DNA as reference and following the Agilent recommended standard protocol. The arrays were scanned using the Agilent scanner, data extraction, filtering and normalization were conducted using the Feature Extraction software (Agilent Technologies). The Agilent CGH Analytics Software 3.4 trial (Agilent Technologies) was used to export the CGH-array data for usage in Nexus Copy Number Professional trial software (version 4, BioDiscovery Inc) (Baumbusch, LO et al. BMC Genomics 2008; 8;99:379).

Eleven patients with SMZL were analyzed with NimbleGen human CGH 4x72K Whole Genome v2.0 array (NimbleGen Systems, Inc). Placenta DNA was used as reference. The CGH-array protocol from NimbleGen Systems was followed. Briefly, 500 ng of tumor and reference DNA were labelled with Cy3 and Cy5, respectively. Labelled material was co-hybridized to microarrays consisting of 71,341 oligonucleotides probes spaced approximately at 40,000 bp intervals throughout the human genome, washed and scanned at 10-µm resolution using the GenePix 4000B dual scanner (Axon Instruments, Burlingame, CA, USA). Raw data were extracted using NimbleScan software v2.5 (NimbleGen Systems, Inc), which allows for automated grid alignment, extraction and generation data files (Selzer RR et al,. Genes Chromosomes Cancer. 2005 Nov;44(3):305-19)

The CGH-array methodologies employed in the present invention, BAC-CGH array, Agilent's Human Genome CGH Microarray 44 k (Agilent Technologies).and NimbleGen human CGH 4x72K Whole Genome v2.0 array (NimbleGen Systems, Inc).confirm the genomic changes observed in SMZL patients. The most frequently changes, were gains on chromosomes 3, 6p221.p21.1, 8q,17q, 18 and losses on4q28.3-q31.23, 10q24.33-q25.3 and 17p13.3-p13.1. In additional, CGH-arrays showed trimosy of 3, 12 and 18 in one patient. All methodologies of genomic DNA, BAC/PAC array, or Oligonucleotides-Agilent 44K and Nimblengem 72K, showed changes on 7q, it confirmed deletion on 7q by three different techniques.

In order to better localize the lost region at 7q, were analyzed by tiling-path CGH-array. These arrays were designed at higher resolution with fine-tiling analysis of the chromosome 7

### Example 5.- High-resolution analysis of chromosome 7

Fine analysis by 385K "Tiling array "NimblemGen, confirm and define the specific deletion region in SMZL. Four patients with SMZL were analyzed by tiling-path CGH-array (three showed losses on 7q22.1 confirm by previous techniques and one SMZL without deletion). These arrays were designed at higher resolution with fine-tiling analysis of the chromosome 7 (chromosome7: 117,711 - 158,805,222 bp). This array was constructed by mask less array synthesis technology (NimbleGen Systems, Inc.), with up to 385,110 oligonucleotides, with a median probe spacing to 365 bp. These arrays were designed at higher resolution which allows designing oligonucleotides that are often placed overlapping with each other to thereby obtain a high resolution of the area to be analyzed in the present invention, the entire chromosome 7.

After the hybridization, arrays were scanned at 5 µm resolution using the NimbleGen MS 200 Microarray scanner (Roche NimbleGen Instruments). Data were extracted from the scanned images using NimbleScan software v2.5 (NimbleGen Systems, Inc), which allows for automated grid alignment, extraction and generation data files

Chromosome 7 tiling array NimbleGen 385K was performed in four SMZL patients. In the analysis of tiling array we detected that three patients showed losses on band 7q22.1 (Figure3).

Analyzing the results of all studies performed by CGH-array on samples SMZL patients and specifically into account the data obtained through the specific array Tiling chromosome 7, shows that the deletion majority of SMZL patients-49% (27 of 57 cases) - is located in the band 7q22.1 and is mapped by clones or probes of array-CGH BAC/PAC: BAC RP11-44M6 (SEQ ID NO:1), PACRP5-1059M13 (SEQ ID NO: 2) y BAC RP11-333613 (SEQ ID NO:3), more specifically between bases 99925039-1 01 348479. This region involves genes such as *EPHB4, SLCI2A9, TRIP6, SRRT, ACHE, UFSPI, TRlM56, SERPINEI, APISI, VGF, TSC22D4, HRBL, LRCH4, MUC3, MUCI2, MUCI7, CUXI* and *SH2B2.* It should be noted that the size of this region is around of 1, 4 Mb

The accurate detection of the deleted region in band 7q22.1, which involves the 99925039-101348479 bp, has been made possible by the difference between resolutions limits of the different methodologies used: BACIPAC CGH-array, Agilent 44K, NimbleGen 72K and Chromosome 7 Tiling array of 385K.As shown in Figure 3, Chromosome 7Tiling array, this region was possible to refine the mapping of the region better than the other different platforms, 3.555 oligonucleotides (Figure 4C) versus 53 of NimblenGen 72K (Figure 4B) or three BAC/PAC CGH-array (Figure 4 A). As it can be seen, Chromosome 7 Tiling array of 385K due to the fact that the focused array has more probes covering the same genomic area, it can offer a better and more precise estimation of the exact break point of to deletion on 7q22.1.

Figure 4A show the BAC/PAC CGH array analysis of three patients. As such observed that these patients had a loss, shown in black in 7q22.1 region, whereas in the control change is not observed. The 7q22.1 was mapping by 3 clones with 1Mb resolution. Figure 4B show the date of NimblenGen 72K analysis with 42Kb resolution, and 7q22.1 was mapping by 53 oligonucleotides, in three SMZL patients. The results show that these patients had a loss in 7q22.1 region, whereas in the control change is not observed. Figure 4C shows the probe-level view of CGH-array NimbleGen 385K all zoomed in on the area of the deletion (7q22.1).This region were mapping by more than 3500 oligonucleotides. As it can be seen it was possible to refine the mapping of the deletion region between D7S2120E and D7S740.locis.Therefore, Chromosome 7 Tiling array of 385K allows more accurately detect the exact location of the deletion occurs on chromosome band 7q22.1

By the method of the invention, has also revealed deletion at a distal region of chromosome 7, the 7q22-q35 region, between 102949624-145959694bp. The size of this region of 43,14 MB is mapping between BACs RP4-672011 and RP4-558L10. This region involves genes such as *RELN, LHFPL3, PBEFI, THAP5, MMP2L, FOXP2 TES, MET, ST7, PTPRZI, GRM8, UBE2H, MLKNI, BPGM, CALDI, PTN, SVOPL, AGK, KEL, TPKI* and *CNTNAP2.* This region is detected among other by, clone o probe BAC/Pac CGH-array: BAC RPI 1-371N6 (SEQ ID NO: 4), mapping in 7q33 (135034979-135191247). So, using all the probes that recognize the specific deletion of the 7q22.1 region, defined as SEQ ID NO: 1, SEQ ID NO: 2 and SEQ ID NO: 3, together with the use of the probe recognizes deletion 7q33 region, defined as SEQ ID NO: 4, could achieve an increase in the number of patients diagnosed with SMZL.

The results obtained through the use of commercial platforms, NimbleGen, Agilent, and array-CGH BACIPAC confirm the partial loss of chromosome 7 as the most frequent change in patients with SMZL. Furthermore, we show the existence of a new region commonly deleted in patients with SMZL, which allows it to be useful for clinical use, located at the level of the band 7q22.1, in a 49% patients analyzed. In this specific region deleted in patients SMZL, located between 99925039-101 348479 bp in 7q22.1, the genes are located *EPHB4, SLCI2A9, TRIP6, SRRT, ACHE, UFSPI, TRlM56, SERPINEI, APISI, VGF, TSC22D4, HRBL, LRCH4, MUC3, MUCI2 MUCI7, CUXI* and *SH2B2.* The analysis of expression of these genes in SMZL patients versus healthy controls or affected from other types of lymphomas, will detect whether any of them may be used as a diagnostic marker for the disease.

### Example 6.- LOH Analysis

To analyze the extent of the region identified on 7q22.1 for CGH-array, previously described, and trying to define the minimum region of molecular allelic loss studies were carried out in SMZL patients. LOH technique is also one of the most widely used methods in the prior art, from the molecular point of view, to determine the loss of genetic material (Matthew M, et al. Am J Pathol. 1999, 154:1583-1589).

The LOH using microsatellite markers allowed to detected relative loss of one allele in a tumor sample relative to a sample of the same patient tumor presents both alleles. In the present invention, defining the allelic loss is limited to informational markers. The markers were considered informative when heterozygosity detected in tissue samples of normal controls which results in the presence of two bands on the agarose gel corresponding to two different alleles.

In the case that a patient will show a LOH the PCR product only will show an amplified band, from one of the alleles, because the other allele will be deleted in the tumor simple. By contrast, the non-tumor sample will present both alleles as two amplified bands (Figure 5).

In order to assess the loss of heterozygosity the nucleotide repeats from six patients with SMZL were analyzed by PCR. A total of seven microsatellite markers were selected on chromosome 7. These markers were selected based on the data from the databases Genethon, GDB and RHdB. The genome location (NCBI36/HG18) of the markers was performed by Electronic-PCR (e-PCR) (Schuler GD. Sequence Mapping by Electronic PCR. Genome Res 1997; 7: 541-550(. The microsatellite markers used to better precise the location of the deleted region on 7q22.1 in the chromosome 7 in SMZL patients are described in Table 2.

**Table 2. Molecular markers used to microsatellite amplification.**

| **Microsatellite marker** | **Chromosomal band** | **Amplicon size (pb)** | **Location (pb)** | **Sequences** |
|---|---|---|---|---|
| D7S527 | 7q21.3 | 273-297 | 95.453.048 | SEQ ID NO: 9-10 |
| D7S2480 | 7q22.1 | 189-225 | 99.893.591 | SEQ ID NO: 11-12 |
| D7S662 | 7q22.1 | 204-234 | 100.950.585 | SEQ ID NO: 13-14 |
| D7S2536 | 7q22.1 | 118-141 | 101.235.892 | SEQ ID NO: 15-16 |
| D7S515 | 7q22.1 | 128-190 | 101.490.495 | SEQ ID NO: 17-18 |
| D7S500 | 7q33 | 188-210 | 134.759.259 | SEQ ID NO: 19-20 |
| D7S550 | 7q36.3 | 177-200 | 155.210.270 | SEQ ID NO: 21-22 |

PCR was carried out in a final volume of 25 uL, containing 30-50 ng/uL of DNA template, 5x PCR buffer (Promega, Madison WI, USA), 25 mM MgCl₂ (Promega, Madison WI, USA), 2.5 mM from nucleotide mixture (dNTPs: dATP, dGTP, dCTP and dTTP, Roche Diagnostics, Indianapolis, IN, USA), 10 uM from the primers used for microsatellite identification and one unit of Taq DNA polymerase (Promega, Madison WI, USA). The conditions used for PCR were as follow: 94°C for 5 minutes, 35 cycles of 94°C during 30 seconds, 55-65 °C (in relation to the primer used) 30 seconds, 72°C during 30 seconds and, finally, the samples were placed at 4°C. The PCR products were separated in a 3% agarose gel (MSG Metagel, Conda, Madrid, Spain).

The LOH studies showed that from the six patients with SMZL analyzed, four of them showed LOH at 7q22.1 (67%). In addition LOH showed that, for the markers D7S662, D7S2536 and D7S515 (located at 100.950.585, 101.235.892 and 101.490.495, respectively, in the region 7q22.1) the percentage of losses was 50%, 20% and 60%, respectively (Figure 6).

In the previous studies analyzing 13 microsatellite markers covering from region 7q21 to 7q36 of chromosome 7, it was observed that the marker D7S487 located at 7q31.3 was predominantly lost (45% of patients) while the microsatellite marker D7S518, located at 7q22, did not showed any lost in the analyzed patients (Mateo M et al. Am J Pathol 1999; 154: 1583-1589). In the present invention we were able to in deep study the region 7q22.1 allowing us to demonstrate the LOH in the microsatellite markers D7S527 (7q21.3), D7S662 (7q22.1), D7S2536 (7q22.1) and D7S515 (7q22.1) (Figure 6). It should be noted that two of these markers are placed in the *CUX1* gene, located in the deleted region 7q22.1 in SMZL patients. Thus the marker D7S515 is located in the intron 3 of *CUX1* gene while D7S6626 is located in the intron 6 of *CUX1* gene.

### Example 7. Detailed analysis of 7q22.1-7q33.1 region of chromosome 7 by a tiling microarray.

In the previous examples we have demonstrated that SMZL patients had a deletion on 7q22.1 on the chromosome 7, located between the bases 99925039 and 101348479. Moreover these patients also showed a LOH of the microsatellite markers D7S662 (7q22.1), D7S2536 (7q22.1) and D7S515 (7q22.1). In order to better define the deleted region on 7q22.1 we had studied this region by using a tilling-array with high density that we order to NimbleGen System Inc. This microarray was enriched in probes from the cytogenetic band 7q22.1 to 7q33 (both bands were included in the microarray, mapping the positions Chr7: 99.158.389-137.302.483). This high-density microarray included a total of 710,953 genetic probes, with a medium size of 54 nucleotides (range: 49-74) and a medium distance of -0.7 bp (range: (-51) to 59217 bp). This CGH-array allow us the possibility to map this specific genome region with a high precision.

This tilling-array was used to characterized the 7q22.1-q33 3 was applied to study a total of 15 samples from SMZL patients, two cases with chronic lymphocytic leukemia (CLL) and an additional healthy control. Seven out the 15 patients (46%) with the diagnosis of SMZL showed losses at 7q22.1, and the microarray located the losses in two regions: the first region of lost in SMZL patients ranged from the genetic positions 100.260.234 to 100.596.921. The second region was located between the genetic positions 101.244.026 to 101.754.716 (Table 3). Therefore by using this tilling-array it was possible to better define the lost region at 7q22.1, and the size of the commonly deleted region size was reduced from 1.4 Mb to 0.84 Mb.

**Table 3. Location of the deleted regions assessed by a tilling-array in SMZL patients**

| **Chr 7 region** | **Cases with losses (n=15)** |
|---|---|
| 100.260.234-100.262.201 | 7 |
| 100.288.318-100.289.733 | 6 |
| 100.398.197-100.398.435 | 6 |
| 100.593.128-100.593.653 | 7 |
| 100.595.970-100.596.921 | 6 |
| 101.588.268-101.588.318 | 4 |
| 101.698.131-101.698.723 | 4 |
| 101.713.340-101.713.623 | 3 |

### Example 8. Gene expression analysis of CUX1, SH2B2 and MUC3 in SMZL patients.

In the chromosomal region 7q22.1 there are located a high number of genes. The deleted region in SMZL, between the bases100.260.234 to 100.596.921 located at 7q22.1, included the following genes: *EPHB4, SLC12A9, RIP6, SRRT, UFSP1, ACHE, MUC3A*/*B, MUC12, MUC17, TRIM56, SERPINE1, AP1S1* and *VGF.* In addition, the region located also on 7q22.1, included the genes *CUX1* and *SH2B2,* but between the bases 101.244.026 to 101.754.718.

The gene *CUX1* encodes to a protein that is a member of the proteins binding DNA. It has been related to the gene expression, morphogenesis and differentiation, and also it can play a role in the progression of the cell cycle. The presence of a monosomy of chromosome 7, with a loss of the genes located in 7q22 such as *CUX1,* is frequent in patients with myeloid hemopathies, such as myelodysplastic syndromes or juvenile chronic myelomonocytic leukemia. However the mutational analysis failed to demonstrate the presence of mutations in none of the analyzed genes. Therefore it has been considered that *CUX1* could be not involved in these neoplasms (Hindersi S et al. Leuk res 2007; 31: 1323-1324). To our knowledge no studies showed the *CUX1* deregulation in SMZL patients. Moreover, the gene *SH2B2* encodes for an adapter protein from some members of the tyrosine-kinase receptors family and it has been involved in several cell signal pathways, mainly from B-cells (Masanori I et al, Biochem Biophys Res Com 2000; 272: 45-54). The expression analysis of this gene in solid tumors, such as esophagus, lung and kidney, revealed that this gene is usually not express in the cell lines studied. By contrast, a high expression of *SH2B2* was observed in normal tissues (Yokohuchi et al Oncogene 1999; 18: 759-767). Therefore the role of *SH2B2* gene in SMZL patients remains unclear.

In the present invention a gene expression analysis of the genes *CUX1, SH2B2* and *MUC3* in samples from spleen, bone marrow and peripheral blood was carried out by PCR. A total of sixteen SMZL patients and six healthy controls were studied. By using PCR (Polymerase chain reaction) it is possible to achieve a large amount of RNA from the samples. The material from both the patients and the controls was previously frozen in trizol (Invitrogen, Lifetechnologies, Gaithersburg, MD) at -80°C before the RNA extraction. RNA purification was performed by using RNeasy Mini columns (Qiagen, Valencia, CA) in order to avoid the possible DNA or protein contamination. The RNA integrity was assessed by the Agilent 2100 Bioanalyzer (Agilent, Palo Alto, CA) by the RNA 6000 Nano Labchip. To synthesize the cDNA a Superscript III kit was used (Superscript III First-Strand Synthesis SuperMix for qRT-PCR, Invitrogen). In all cases 1ug of total RNA was used. The cDNA was purified by tha cDNA Cleanup kit (Affymetrix sample Cleanup Module kit).

The primers used for cDNA synthesis were designed by the software "Primer Express 3.0" (Applied Biosystems). In addition, the software "Oligoanalyzer" and NCBI BLAST were used to avoid the dimerization and to chek the specificity, respectively. The primers used for the gene expression analysis for the exons of the genes *CUX1, SH2B2* and *MUC3* are described in the table 4. In all cases the ABL gene expression was used as a control.

**Table 4. Primers used for gene expression analysis (RT-PCR)**

| **Gene** | **Primer** | **Sequence** |
|---|---|---|
| *CUX1* (Exon 2-4) | Forward | SEQ ID NO: 23 |
| | Reverse | SEQ ID NO: 24 |
| *CUX1* (Exon 24) | Forward | SEQ ID NO: 5 |
| | Reverse | SEQ ID NO: 6 |
| *SH2B2* (Exon 6) | Forward | SEQ ID NO: 25 |
| | Reverse | SEQ ID NO: 26 |
| *SH2B2* (Exon 3-4) | Forward | SEQ ID NO: 27 |
| | Reverse | SEQ ID NO: 28 |
| *MUC3A*/*MUC3B* (Exon 1) | Forward | SEQ ID NO: 29 |
| | Reverse | SEQ ID NO: 30 |
| *MUC3A*/*MUC3B* | Forward | SEQ ID NO: 31 |
| | Reverse | SEQ ID NO: 32 |
| *ABL* | Forward | SEQ ID NO: 7 |
| | Reverse | SEQ ID NO: 8 |

To optimize the annealing temperature, a gradient test was performed by using a control cDNA from a multiple myeloma cell line in the iCycler (Biorad). Moreover a control gene (ABL) was used to assess the relative quantification of the target probe in relation to the referred gene by the fluorescence measurement by SYBR Green (Applied Biosystem) and the Pfaffl analysis method (Pfaffl MW, Nuc Acid Res 2001; 1: e45). In brief, in following this method the samples were studied three times; a total of 10ng were used. For each gene both a positive and a negative control were used. The reaction was adjusted to an optimal temperature for each primer. The PCR was performed in a total volume of 25 uL and contained: forward and reverse primers, cDNA, 2xFast SYBR Green (Applied Biosystem) and water. The obtained results were expressed in relative units (CI, Threshold cycle) in relation to the expression of ABL gene.

The results showed that the *MUC3* expression is unmodified in SMZL patients. By contrast, the gene expression of both *CUX1* and *SH2B2* was different in SMZL and healthy controls. Thus *CUX1* showed downregulation in spleen, bone marrow and peripheral blood of SMZL patients in relation to controls /Figure 7). In addition, the *SH2B2* gene was found overexpressed in spleen and bone marrow from SMZL patients in relation to controls.

In the next step a DNA sequencing of the candidate region was carried out. The aim was to look for the presence of possible DNA variations present in SMZL patients. A total of 23 DNA samples from SMZL patients and fourteen controls were analyzed. The target DNA (the DNA containing the genes *CUX1* and *MUC3*) was amplified by PCR: several primers specific for the 7q22.1 region were designed according to the data from NCBI36HG18 (Table 5).

**Table 5. Primers used for sequencing studies**

| **Gene** | **Location** | **Primer** | **Sequence** |
|---|---|---|---|
| MUC3A/B | 100387324-100388418 | Forward | SEQ ID NO:33 |
| | | Reverse | SEQ ID NO:34 |
| | | Forward | SEQ ID NO:35 |
| | | Reverse | SEQ ID NO:36 |
| CUX-1 Exon 2 | 101346114-101346225 | Forward | SEQ ID NO:37 |
| | | Reverse | SEQ ID NO:38 |
| CUX-1 Exon 3 | 101458097-101458145 | Forward | SEQ ID NO:39 |
| | | Reverse | SEQ ID NO:40 |
| CUX-1 Exon 4 | 101500338-101500417 | Forward | SEQ ID NO:41 |
| | | Reverse | SEQ ID NO:42 |

After PCR the separation of the amplified products was performed in an agarose gel (1-2%) and a purification of each isolated fragment was carried out by the Kit High Pure Product Purification (Roche).

The sequencing of each fragment was performed by using the same primers used to amplify the DNA. All samples, both from controls and from SMZL patients, were sequenced from 40-60ng of each fragment in a final volume of 8 uL. A sample containing both a sense and antisense primer at a concentration of 3pmol in a volume of 2uL was prepared. Therefore each isolated fragment was sequenced with each primer and several sequences of each fragment were obtained. The sequencing reaction was carried out in an ABI 3130XL sequencer (Applied Biosystems) in the Center for Cancer Research in Salamanca Spain. The generated sequences were analyzed by the software GENE15 (Discovery Studio Gene v1.5, Accelrys Inc). After the analysis the obtained sequences were aligned according to the consensus sequences (NCBI/HG18). The alignment and the analysis of the similar sequences were performed by the informatics tools Clustal W and BLAST (Basic Local Alignment Search Tool).

The sequence analysis of the 7q22.1 region containing the MUC3 gene (chromosome 7: 100387324 to 100388418) is a 1140 bp long. The results showed the presence of five polymorphisms in the region with the same frequence in controls than in SMZL patients. Therefore these polymorphisms could be present in the normal population and could be not related to the SMZL.

By contrast, in the sequence analysis of CUX1 gene, the presence of new variations not previously described were observed. The first variation was observed in the first intron and it was the insertion of one thymine and one adenine in the positions 101345904 and 101345905 of the reference genome (Figure 11A). This variation is not present in the consensus sequence obtained from NCBI/HG18. Moreover in the third exon of this gene other variations not previously described were observed. Thus a mutation in the position 101458144 of the reference genome was observed in SMZL patients (Figure 11B).

## Claims

1. *In vitro* method for diagnosis of splenic marginal zone lymphoma (SMZL) by high-resolution genomic microarrays, comprising a step for detecting the deletion between the bases 99225039 to 101754718 located in the 7q22.1 region in which is located, at least, the *CUX1* gene.

2. *In vitro* method, according to claim 1, **characterized in that** the deletion is located between the bases 99925039 to 101348479.

3. *In vitro* method, according to claim 1 **characterized in that** the deletion is located between the bases 100260234 to 100596921 and/or 101244026 to 101754718 in the 7q22.1 region.

4. *In vitro* method, according to any of the claims 1 or 3 **characterized in that** the *CUX1* and *SH2B2* genes are located in these deleted regions.

5. *In vitro* method, according to claims 1 to 4, **characterized in that** further in said region are located, among other, the genes *EPHB4, SLC12A9, TRIP6, SRRT, ACHE, UFSP1, TRIM56, SERPINE1, APIS1, VGF, TSC22D4, HRBL, LRCH4, MUC3, MUC12* and *MUC17.*

6. *In vitro* method, according to any of the claims 1 to 5, which additionally comprises the detection, by high-resolution genomic microarrays, of the deletion between the bases 102949624 to 1459599694 in the 7q22.2-q35 region in which is located, among others, the genes *RELN, LHFPL3, PBEF1, THAP5, IMMP2L, FOXP2, TES, MET, ST7, PTPRZ1, GRMB, UBEH2, MLKN1, BPGM, CALD1, PTN, SVOPL, AGK, KEL, TPK1* and *CNTNAP2.*

7. *In vitro* method, according to any of the claims 1 to 6, **characterized in that** it is performed on a DNA sample selected from: a tissue sample from a lymphoma cell infiltrate, a bone marrow aspirate or a whole blood sample.

8. *In vitro* method, according to any of the claims 1 to 7, **characterized in that** high-resolution genomic microarrays are selected from BAC/PAC CGH-arrays, Agilent CGH-array, NimbleGen CGH-array or CGH Chromosome 7 Tiling Array from NimbleGen and CGH 7q22.1-q33 region Tiling Array from NimbleGen.

9. *In vitro* method for diagnosis of splenic marginal zone lymphoma (SMZL) which comprises measuring the expression level of at least the CUX1 gene, located in the 7q22.1 region between the bases 99925039 to 101754718.

10. *In vitro* method, according to claim 9, **characterized in that** it comprises measuring the expression level of the CUX1 gene located in the 7q22.1 region between the bases 99925039 to 101348479.

11. *In vitro* method, according to claim 10, **characterized in that** it comprises measuring the expression level of the CUX1 gene located in the 7q22.1 region between the bases 101244026 to 101754718.

12. *In vitro* method, according to claim 9, **characterized in that** it comprises measuring the expression level of the, at least, the *CUX1* and *SH2B2* genes located in the 7q22.1 region between the bases 101244026 to 101754718.

13. *In vitro* method, according to claims 1 to 12, **characterized in that** it further comprises measuring the expression level of at least one of the following genes selected from: *EPHB4, SLC12A9, TRIP6, SRRT, ACHE, UFSP1, TRIM56, SERPINE1, APIS1, VGF, TSC22D4, HRBL, LRCH4, MUC3, MUC12* and *MUC17,* or combinations thereof, in the 7q22.1 region between the bases 99925039 to 101348479.

14. *In vitro* method, according to any of the claims 9 to 13 **characterized in that** it is performed on a DNA sample selected from: a tissue sample from a lymphoma cell infiltrate, a bone marrow aspirate or a whole blood sample.

15. *In vitro* method for diagnosis of splenic marginal zone lymphoma (SMZL) which comprises a combination, in any order, of a first step for detecting, by high-resolution genomic microarrays, the deletion between the bases 99225039 to 101754718 located in the 7q22.1 region and a second step for measuring the expression level, of at least, the CUX1 gene.

16. *In vitro* method, according to claim 15, **characterized in that** the deletion is located between the bases 99925039 to 101348479.

17. *In vitro* method, according to claim 15 **characterized in that** the deletion is located between the bases 100260234 to 100596921 and/or 101244026 to 101754718 in the 7q22.1 region.

18. *In vitro* method, according to any of the claims 15 or 17 **characterized in that** in the second step the expression levels of the *CUX1* and *SH2B2* genes are measured.

19. *In vitro* method, according to claims 15 to 18, **characterized in that** further comprises measuring the expression levels of at least one of the following genes selected from: *EPHB4, SLC12A9, TRIP6, SRRT, ACHE, UFSP1, TRIM56, SERPINE1, APIS1, VGF, TSC22D4, HRBL, LRCH4, MUC3, MUC12* and *MUC17* and/or combinations thereof.

20. *In vitro* method, according to any of the claims 15 or 19, which additionally comprises the detection, by high-resolution genomic microarrays, of the deletion between the bases 102949624 to 1459599694 in the 7q22.2-q35 region in which is located, among others, the genes *RELN, LHFPL3, PBEF1, THAP5, IMMP2L, FOXP2, TES, MET, ST7, PTPRZ1, GRMB, UBEH2, MLKN1, BPGM, CALD1, PTN, SVOPL, AGK, KEL, TPK1* and *CNTNAP2.*

21. Kit for the *in vitro* diagnosis of splenic marginal zone lymphoma (SMZL) comprising a set of probes selected form the group SEQ ID No. 1, SEQ ID No. 2, SEQ ID No. 3, and/or any probe that hybridizes wholly or partially with the deleted region between the bases 99925039 to101754718 by high-density genomic microarrays.

22. Kit according to claim 21 **characterized in that** the selected set of probes hybridize wholly or partially with the deleted region between the bases 99925039 to101348479.

23. Kit according to claim 21 **characterized in that** the selected set of probes hybridize wholly or partially with the deleted regions between the bases 100260234 to 100596921 and/or 101244026 to 101754718 of the region7q22.1.

24. Kit according to claims 21 to 23 **characterized in that** it further comprises at least a probe that hybridizes wholly or partially with the deleted region between the bases 102949624 to 1459599694 in the 7q22.2-q35 region, by high-density genomic arrays, preferably the probe is SEQ ID No. 4.

25. Kit according to claim 24 **characterized in that** the probe hybridizes in the 7q33 region.

26. Kit for the *in vitro* diagnosis of splenic marginal zone lymphoma (SMZL) **characterized in** the detecting the expression level of at least the CUX1 gene, located in the 7q22.1 region between the bases 99925039 to 101754718 by RT-PCR with the primers defined as SEQ ID No 5 and SEQ ID No. 6.

27. Kit according to claim 26 **characterized in that** the expression level of at least the CUX1 gene, by RT-PCR with the primers defined as SEQ ID No 5 and SEQ ID No. 6 it is performed in the deleted region between the bases 99925039 to101348479.

28. Kit according to claim 26 **characterized in that** the expression level of at least the CUX1 gene, by RT-PCR with the primers defined as SEQ ID No 5 and SEQ ID No. 6 it is performed in the deleted region between the bases 100260234 to 100596921 and/or 101244026 to 101754718 of the region7q22.1.

29. Kit according to claim 26 **characterized in that** the expression level of the *CUX1* gene it is further performed with the primers defined as SEQ ID Nos. 23-24.

30. Kit according to any of the claims 26 to 29 which additionally comprises the detection of the expression level of SH2B2 gene by the primers defined as SEQ ID Nos 25-28.

31. Kit according to any of the claims 26 to 30 **characterized in that** further comprises detecting the expression levels of at least one of the following genes selected from: *EPHB4, SLC12A9, TRIP6, SRRT, ACHE, UFSP1, TRIM56, SERPINE1, APIS1, VGF, TSC22D4, HRBL, LRCH4, MUC3, MUC12* and *MUC17* and/or combinations thereof by specific primers for the sequences of these genes.


SEQUENCE LISTING

<110> Fundación de Investigación del Cáncer de la Universidad de
      Salamanca (FICUS).

<120> IN VITRO DIAGNOSTIC METHOD FOR PATIENTS WITH SPLENIC MARGINAL ZONE
      LYMPHOMA.

<130> EP-05939

<160> 42

<170> PatentIn version 3.5

<210> 1
<211> 113687
<212> DNA
<213> Homo sapiens

<400> 1
   gaattcccgg aatcctcctc ggcccccctt ccagactccc gcctcactct gcctggcagt      60
   ccagggacag gagccgccta actacttcca gatgtggaca cagccacgag gcggtggggg     120
   ggagggggtg gagagatgga gggagctgca gtggcgatgg ccctctgatt gaacctggag     180
   gccaggctcc gcgagctcca caccttagcc ctgcccctgt tccaaactac ttcagattct     240
   gactcccaag tctccactca ctgccccctc ctccccctac aatccctctc gaattccttc     300
   ctggaatcca gctaccaggc aagttcagga gcttggctct ctcttgggtt ctatgggggg     360
   tcaaggatct gggtgggtgg ggagacttaa gagaccagag gacgaaggtg ggggaatggg     420
   agcagtggtg gcctgcagga gaccaaggtc ttgcagcaga tggggaggat ttgggggcca     480
   gagaagggtg aggcggtggg gaatctgggg agtggtgtgt cagcaaggga acccaaaata     540
   tctccaggca taaccccaaa ggaatcgggg gtctgaagtt cactggagct gtctggaaga     600
   tgccaagaga cctacctggt gtagttgggg gtctggccct gggcaaaagg cagcagggcg     660
   caggcagtga ggagggcccc caggagggag gctgtggctg caggcagcat ggccccgggg     720
   gaaaggcgct ggggtcctca agacagaggc agcagctgaa ttttgcagag cagcagcagc     780
   ggtggcggca gcagcaggat aatcagcgcc agatggggca gccttgggtg tgtgggcgtg     840
   gggactggcc agtggggcgg gcaatgggga aggcgggaca gtggcgtgtc ctgggacctc     900
   tctaggtcta ggacacataa atgttgacct taggaggggg aggagcagtg aggggcagag     960
   gccgtctccc aggcctcaag taacgtcatc gggaacccca ctctgtccag tgagggagta    1020
   tctccgggaa cttctgagga ctccctagcc cccatcctcc ccccgcccct gcttcctcag    1080
   ctcaggagcc ctcattgctc accccctgcc tgaccacccc tgccagctca ttttccactt    1140
   caatttcttt tttttttttt tttttttttt gagacagagt ctcattctgt gacccaggct    1200
   ggagtgcagt ggcgcgatct ctgctcactg caacctccgc ctcccgggtt caagcaattc    1260
   ttgtgcctta gcctcccgag tagctgggac tgcaggtgca tgcctccaca cctggctaat    1320
   ttttgtattt ttagtagagg cagggtttca gcatgctgtc caggctggtc tcaaattcct    1380
   agcctcaggt gatccgccca cctcggactc ccaaagtgct gggattatag gcgtagccac 1440
   cacacctggc ctccacttca catctccatt gccttctctc cagacctggg ccagactagg 1500
   ccaaggagaa ggaattctca cccatgacct cttccaacta cttgaaggta caggaagcaa 1560
   ggcaggaggc atggggtgtg gatgaaggga gtgtccttgt tactcaggaa gaggtggaag 1620
   gagtagaaaa tagagcccaa acctgggagg taaacagaag ggagagagga ggggggctgg 1680
   cttcaggccg aagggagatg ggtgggaagc tttcgggggt caggaggaac aggctgcctc 1740
   aggatgggga caagtctagt gggcagggga tcccagtggg gtagccaaga gcatctgtgc 1800
   atagagttgt tccagaaggt agggccagaa cttcccagtc caaacgacca ggtgtgctct 1860
   tgggagcagg gagtggacaa caatgtgaag ggttcaggca gcatcagctc tgctgcacct 1920
   gagacctgga gctgcttgtg ctgcctgccc ctgcctgggc tcctgccctc ctgcacccac 1980
   caagccgggc acccacccac cccatccctg cccctgtccc tcccagcccc caaggcttcc 2040
   acaccaggcc tcgctgcagc cttttattct atccatcaaa tctcttgtcc atgatagtca 2100
   ggttgggggc ccctcctggg cagccccctg aacattctta ccctggttag ccgtccccac 2160
   cccctagcaa ccccttccca cacgcatgtg cacaatggtc attgctcctt agtccctggc 2220
   ctggggccgg actccctcct ccagggacta ggctagcatg agggggatta ggtctcaggg 2280
   gctgggggcc ctacaccacc cccgcctcct tcagccctct gcagcatgtc caggcctccc 2340
   cagaagaagt ccttctgtgc ggccatcagg ggcatccacc ctacgatctc cttcatcttc 2400
   tccgtgcggt cctgcaacgt ttggcaactg tggatctggc tgaggtactc ctcgcaggcc 2460
   tgggccatcc ccccggggtc ctggggtgct ctggccccca ggctgggctc aggagtcccc 2520
   accaccctgc tggctggcag gtggcgatag ggggcgtgct gctcaatgtc gtggctggac 2580
   aggatgtata ggcactccct ggtggcacag agggcacagg cacacagagg gaccttggag 2640
   gaggagagaa agggggcatg gctgagtggt gctttccttg cagggtttcc cagggtgccc 2700
   gggctaatct gcctattgca ttcttccctc tcacaccagc cctgagccgg ctaagtccca 2760
   ggggtgtgtg aggttgggca aggccagcac agtctgctaa gggagaaagc agagagcgtg 2820
   gtgtgcggca gaggagggag gtggggtggt gctagggaaa tgggaccccc tgggtccctg 2880
   caggagcccc agctacagcc tgtgggcttc ctggttcatg gctctcatcc tccctgggtg 2940
   cccactctcc tgaccccgct ctgaccttga cttggagctt gacgaaggag gcactcccct 3000
   tgggccagcc tgggaggcgg cccccagccc cacagccgca gcccagcagc tccttgctga 3060
   actctgagct ctggctcagc accagggagt ggttgaggcc gcaccacagg gtgatgggcc 3120
   gctgcaggta acaaacctgg ggacacacgg ccagggtccc tgagcaggga gcaccaaggg 3180
   acgggggggc tggggtggga aaaggttgac tggaaggctc ggggcagaat gaactgggga 3240
   ggaggaaatg ggagccagca ctgtcccacg ccccaggtct gccctgctga cccctgcagc 3300
   ccccatactc taactggtca catagggtct tgcacttctg ttggtatctc atctatgccc 3360
   ccagaaaatc agctccttgc ctgagcccag ggcctgctgt ggtgtacccc tcattctcag 3420
   cccacccgtc tatgccagtc cagcctctca tcctgcctgc cagcctcggg gagacttgct 3480
   tcttgtgcaa ttccaggtta gaccccaaca ccaccctgag gaaggacttt cccttccact 3540
   atccctccct tcctgctcta gtattatctt ttctttcttt ctttcttttt tttttttttg 3600
   tttgagacag agtcggaagg ctggagtgca gcagtgcgat ctcggctcac tgcaacctct 3660
   gccttcctgg gttcaagtga ttctcccacc tcagcctccc aagtagctag gactacaggt 3720
   ccttgccacc acacccatct aatttttgta tttttagtag agaaggggtt ttgccatgtt 3780
   gaccaggctg gtctcgaact cctgacctca ggtgatctgc ctgccttggc ctcccaaagt 3840
   gctgggatta caggcatgaa ccactgagcc cagcctccac tctagcattt ttattttctc 3900
   ttttcttttt ttagagatgg ggtctcattc tgttgcccag gctgaaatgc agtggtgcaa 3960
   tcatagctca ctgcagcctt gacctcctgg gctcaagcca ttcttctgcc tcagcctcct 4020
   gagtagttgg gaatacaggc atgtgccacc acacccggca tgtgcctata atttttaaat 4080
   tttaaatttt taaattttct atagagatag tgtctcacta tgttgctcat ggtgatctcg 4140
   aactccgggc ctcccacctc agcctctagc attttttttt tttttgtaga gacggagtct 4200
   cgctctgttg cccaagctga agtgcagtgg cacgatttca gttcactgca acctccgcct 4260
   ctccagttca agcaattctc cttcctcagc ctcccgagta gctgggacta tgggtgcaca 4320
   ccgccacacc tggctaattt cttttgtatt ttagtagaga cggggtttca ccatgttgcc 4380
   caggctggtc ccgaactcct gggctcaggc aatccacctg ccttggcctc ccaaagtgct 4440
   aggattacag gtgtgagcta gttgaaatgc ttagcctagc attttcaact tatcacataa 4500
   ttcttttcca aaatggccac cctgtttttg aaaaaaaagc ttctacttgc ttcctgccgc 4560
   ttcctgcctc tctctcttaa gcagcagagt tggttacatg accaatctga ggctgaccac 4620
   attactgttc cttcccacag ccagtctatc cagcacctcc ctgggatctc gcttttctct 4680
   ctattacatt actgacatgc tctctctaga ggcacttggc ctctcagtgg ctaaatccca 4740
   gggcgtcttc tgccccctca ttctcctgag cctgttagcg ctttaattct gaagacactg 4800
   ataagtaatt tatggtcatt ctagaaaaat tatgaaacat tggcaagcaa aaaattaatt 4860
   ttaaaatcat tggaaatccc accatctact attaacatct tgctagatat tcttcactgt 4920
   tggctaaaca aacaaataca cataaattgt atatttttta ttttttagag atagggtctc 4980
   agtgtcaccc aggctggagt gcagtggtga tcacagctca ctgcagcctt caactcctgg 5040
   cctcaagcaa tcctcctgtc tcagcctcct gagtagttgg gattacagat gtcagccacc 5100
   atgatgggca agttgtgtat ttttaaacaa aaaatgggat ggggccaggt gtggtggctc 5160
   acacctgtaa tcccagcact ttaggagtct gaggtgggag gattgcttaa agccatgagt 5220
   tcaatataag cttgggcaac atagcaagac cctgtctcta aaaaaaaaaa aaacaaaaaa 5280
   aacaaaaaaa acaaaaactt atctgggcgt agtggcgcat tcctgtagtc ctagctgctt 5340
   gggaggttga ggcgggagga tctcttgagc cctggagttg aaagctgcag caagctacga 5400
   ttgtgtcact gcactccagc ttgggaagca gtggagacca tgtctcaaaa aagtaataat 5460
   aaaaataaaa agaggctggg tgcggtggct cacacctgta atcccagcac tttgggaggc 5520
   caaggtgtgc agatcacctg aggtcaggag tttgagacca gcctggccaa catggtaaaa 5580
   ctccgtctct actaaaaata caaaaattag ccgggcatgg tggtgggcgc tgtaatccca 5640
   tcttggcagg ctgaggcagg agaatcactt gaacctggga ggcagaggtt gcagtgagcc 5700
   gagatcgcgc cattgcactc cagcctggga gacagagcaa gactctatct tgaaaataaa 5760
   ataaaaataa aaataaaaat aaatggtact atgtaaacca tttcataaaa gtctttttca 5820
   cctaaaaata tggcatgact atcttttcca tgtcagtagt aaatgtatct tacattgtca 5880
   caccatcaat taaccctacc acccacccca tttggaactt ccttctcatg tggcttctgg 5940
   gacagtgtta gaggggtgca cggcctgtct tagaggcacc tcttcatccc ctgtctacca 6000
   cagggcctac ttctcagagt tgcttttcag tctctacttc tgtttgttca accatgttcc 6060
   caggtagaat ctaattgcca gctagacgtt tccaattgag tgtggttttt gttttgtttt 6120
   gttttgagac agagtctctc tccgttgccc aggctggaat gttgtggcac gatctcggct 6180
   caccgcaacc tctgcctccc aggctcaagc aattctcgtg cctcagactc ccgagtagct 6240
   gggactaaaa gcacccacca ccacacctgg ctaatttttt atttttctta gagacagggt 6300
   ttcaccatgt tgtccaggct gatctcatgc tcctgggctc acgtgatcca cccaccttgg 6360
   tctcccaaag tgctgggatt acaggtgtga gatatctttc ttccagagcc acctctgtct 6420
   tatacccctc tcttctacat ctaatcagtt ttgaggccct attcattttc ttcccaaata 6480
   gctcctgtat ccattccttc ccattctcgc tgcagcacca ggcgcttgtc tcaagtgctg 6540
   tcatcactca acagcctcct ggcgggctcc cctccttggc ctcctctgca caaggctcct 6600
   gacacattct cagtcacctc ttgggggttg ccttctccta ttccagaacc tgcagtgact 6660
   cttccccaaa tctacacctc tctaagcttg tgcccacctg taccacctca tacctcagtg 6720
   attcccaaga tgccttctgt gctttagtga gacccgtctt gtcactgtcc caggtatgtg 6780
   caagtttcgt gcctatcccc ctcctcatca cacagaatgc cttctctatc cctacctaag 6840
   tcttatgtaa cccacgatgc cctgctcaca tcttttttct ttttctttga gacggagtct 6900
   cgctctgtgg cccaggctgg agtagggtgg cacgatctca gctcactgca acctcagcct 6960
   cccaggttca agagaatctc ctgcctcaac ctcctgagta gctgggattg caggcatgtg 7020
   ccaccacagc cagctaattt tttatatttt tggtagagac ggggtttcac catgttgccc 7080
   aggctggtct agaactcctg acctcaagtg atctgtccac ctcggcctcc caaagtgtcg 7140
   ggattacagg cgtgaggctc tgcacctggc ccctgctcac atcttacctc ttctagaagt 7200
   ctctagcctg cccaggtcac ccaccctacc ccttcactaa attcctattg catttacagt 7260
   gtgcactgca cagaatggac ctcgtagtgc caaccctggg taagacatgg ggtagtacca 7320
   ctggcattac atactgagtg gcttaacttc ccaaaggaac agaaattgga ttctgtggaa 7380
   tctggagaag tggacagatc tgggggttag ggaattggag gaaaggggtt gggatgagag 7440
   ttgctgggaa atagtctcac ctgtgtgggt tcccctcggt ccattttgtc ccctgttcct 7500
   agctgcccgt atctgttatt tccttgcatg aagattcggc caaattcatc caccaggcca 7560
   aggtggttgt agccaagagc acagaatagg atctagcgtg caagatagaa aggggatagg 7620
   gattccattc cctccagagg aactctgagt tccagagtct ccaagccact aacctgccat 7680
   ccccagctga tctctttcta tatatatatt ttagagatgg gatctcacta tgttgcccag 7740
   gctggtctag agctcctggg ctcaagcgat actccctcct cagcctccct ccgcaccgag 7800
   cccccagctg atctcttttg gcatacctct ccctggggat cccctgcccc tctggatcaa 7860
   taatctgatt cctataatac ttcagacctt ccaatcccta cagcgccacc cctctggttt 7920
   gccccgtccc tccaggagcc taccttggca ggcagtgaga gagcaagcgg catctgctgg 7980
   tccagggggt caaaggcttg aagggtccca aagagatcgc gatacacccc tggggtatgc 8040
   acctcaaaat acactccccc ctggtctggg gggtcaggag ccctcagctc agcagctttg 8100
   ggcacccatt tcctagggat aaggccccac ctgggatgaa agctacaaga tccaaggagt 8160
   ctcataccca ctaggaatgc atcaactatg atgcctggga actgaaaaca catcttttga 8220
   gaccgagtct ccctcggtca cccaggctgg agcgaagtgg cacgatctca gttcactgca 8280
   acctctacct cctggttcaa gtgattctcc tgtctcggcc tctggtgtag ctgggattac 8340
   aggcgcctgc catcacaccc agctaatttt tgtattttag tagagatggg gtttcgccat 8400
   gttggccagg ctggtcttga actcctgacc tcaggtgatt cacctgcctc ggtttcccaa 8460
   agtgatggga ttacaggcat gagtcaccat gccggcccta aacacatctt ttcactagga 8520
   tgccctcagg ggttcaccat gcagctggag taagggtgaa gggtgttgat gtttagaggg 8580
   tctaaccaag gctgcgggct tgagccgggg ggaggtggtc catacctgtg acgtagaggg 8640
   tgctgctctg gttggaagtc atgcaggcca cgcgcaggtg aggcaggtag tgggacacct 8700
   tcctcagggc cagctgaacc gtgtaggagc gtggctggtc aagctgggtc tcattcacta 8760
   ccaaagagta gatctttcct tcctctgggg gtaggggaag caattagttg gggaggggat 8820
   tctccaatat ccacataaac atcccaagag actccctagg ccatatgggt gacacattta 8880
   ctgccctaga gtggggctga gaaagaaata agggaagagt gaccccaagt cccccagact 8940
   ccagttccca ccataaatca gggaagtact tcagcatgag agatgagcag agagaaaaat 9000
   ctagcagatt tgttagaatc cgatttgagt gagaggtctg gggattgagg gccctgcctg 9060
   gcattcagaa gtttagaggt ggtcaagtgt ggtggctcac acctgtaatc ccagcacttt 9120
   gggaggccaa ggcaggagga tcacttgagg cagggagttc gagaccagcc tgggcaacat 9180
   agcaagaccc catctctacc ttgctccaaa aaagaaaaaa atattttaag aaaaagccta 9240
   aagggtctca ggcctgggga tgtggttgag tataaaggac attgggaaag tagggagcag 9300
   tttggagtat agtgaggtgg ttggcatgga agctggggac atctgatttt tttttttttt 9360
   tttttttttg gagatggagt ctcactctgt cacccaggct ggagtgcagt ggcataatct 9420
   cggctcactg aaacctccac ctcctgggtt caagcgattc tcctgcctca ggctcccaag 9480
   tagctgggac tacaggtcca tgccaccacg tccggctaat tttttttttt tttttttttt 9540
   gagacggagt ctcgctctgt caccaggctg gagtgcagtg gcgtgatctc ggctcactgc 9600
   aacctctgcc tcctgggttc aagggattct cctgcctcag cctcccaggt agcttagact 9660
   acaggcacgc gccaccacgc ttggctaatt tttgtatttt tttttagtag agtcaggcag 9720
   ggtttcacca tgttggtctc gatctcttga cctcgtgatc cacccgcctt ggcctcccaa 9780
   aaactgggat tacaggtgtg agccgccgca cccgacctaa tttttgtatt ttttagtaga 9840
   gatggggttt cgccatgttg gccaggctgg tctcaaactc cagatctcag gtgatctgct 9900
   cacctcggcc tcccaaagtg ctggggttac aggtgtgagc caccacacct ggacagggcc 9960
   ttttattcat ataaaatatt aagcagaatc ctagactagg agacagagaa gggcagagct 10020
   gtttagatgg agactaggat aaggtggcat atggaaccca gtctgagggg cctccctccc 10080
   cctcctctgc agcagggtct gaggaatctg caggaaacag tttgcaaaag ccattgcttt 10140
   gggccacacc tgtgaggagc agtagagccc gctgggtctc ctgaccaacc agcacgatct 10200
   gcttgaaggt cattgagtgg tggaatgtca tcttgaagac ccgctgccca ctagactgca 10260
   gatagacctc aacacagtca caggcccggc tgctggtggt acccaccact tcctgcggct 10320
   cccgagtggc caagacgtag aggtatttac ggtaaactgt gtcacacctt gggtccgagg 10380
   caaactgggg gaaaagatga gggcgtctgg gatctagagt ttcaaaatgg gatggggctg 10440
   gggcagctgg atcctggggt gggaagtgaa atgcttgggt ccagagttgg gaaatgagcc 10500
   cttgatttca ggttcctatg tgggagggca gaagtcatag gaaagacacc tcaagactga 10560
   tgggggcctg ggtcctcaaa agagaggtgg tgatatggtt aggctctgtg cccccaccca 10620
   aatctcatct cgaattgtaa tacccagtgg gaggtgattg gctcacgggg gcaatttccc 10680
   ccatgttgtt ttcgtgatag tgggttctca cgaggtctga cggatttata aggcagtttt 10740
   ccctgctctt gcttgtgtgc tctcgcctgc tgccatgtaa gacgtgcctc tttcccttct 10800
   gccatgattg taagtttcct gaggcctccc cagtcatgtg gaactatgag tcaattaaac 10860
   ctctttgcct tataaattac ccggtctcag gtatgtcttt ttttttcgag acggagtctc 10920
   cctcttgttg cccaggctgg agtgcaaggg tgcgatctca gctcactgca gcctctgcct 10980
   cccaagttca agcaattctc ctgcctcagc ctcccaagta gctggaatta taggcaccca 11040
   ccaccacacc cagctaattt ttgtattttt agtagagacg ggtttcgcca tttggccagg 11100
   ctggtctcga actcctggtc tcagctgatc cgcctgcctc agcctcccaa agtgctagga 11160
   ttacaggtgt aagccactgc acccggcctc tggtacgtct ttatagcagt gtgaaaatgg 11220
   actaatacag gtgggctctc agctgccagg gttctgctac tcacatcctt ggctccacga 11280
   cacaacacaa catagcgaca ggcccgcttc cactgcatct ggccgagggt ggagaccagg 11340
   gcatttttga ggaagaagag ggtccccacg tagtcaagaa tgaagacgtg atccttggtg 11400
   ggcaagaagc ggcggtagcc gtgggctagc aagggggcca cgctcttgct gagacatcgg 11460
   cggcggcctc caaatgcctg gaaatacagg cccttcgtgt ctggaggaag gaaggagatg 11520
   gaaggttcaa gggatgagat gacacgaggg agatgagttg gagcttgggt agatttctta 11580
   gacacgctgg tttgtagttc ctccccatcc ccaggacagg ggaacaaaca gaccccttat 11640
   gaaggagatg tggaaaataa gcttagatta tactcgacat gtgaacttta gtctccaccc 11700
   cacctcagcc cagggtcaaa catttgcttc ttagtgagca gttctttggt gcaaccttgc 11760
   atcagtgatg ttgaggaaaa ctctaagaat aaagacacag aggctgcgtg cggcatccac 11820
   ccccctcacc ctgagcccct cctcccatcc tcacagctca gtgaattttt gcacgcctgt 11880
   aatcccagca ctttgggagc ccaggagttt gagaccagcc tgggcaacgt ggcaaaaccc 11940
   catctctact aaaaatacaa aaattatccg ggtgtggtgg cgggagcctg taatcccagc 12000
   tactcaggag gctgaggcac gagaatactt gaacccggga ggtggaggtt gcattgagcc 12060
   aagattgcac cactgtgctc cagcctgggt gacagagcaa gaccctgtct caaaaaaaaa 12120
   aaaaaaaaaa aaaaatcaaa gaaaaagaaa gaaaagagaa gagacagaaa caggagtgaa 12180
   aaggaatgat tgatgaaggc cggtcatcct ccctcacccc tagctttgga tctgttttct 12240
   gggtccccct tctcttttaa tctcccagtg agtgtaagaa gtacaccctg ggggatccct 12300
   ggaggggaag agggtttatg gcatggttct caggctaggg gtgattttgt ccttcaaggg 12360
   cattggttgt cacaattaag ggggatgctg ctggccatct ggtgggtgga ggccagggtt 12420
   gctgcttaaa catcccacca tgcagaggac agctccccac atcaaggaat tattttgccc 12480
   agaatatcaa cagtgagttc agaagccttg ggttagagga gggaaaactc caggtttggg 12540
   gagtgaggga tgcctctagg gaaaaagcgg gaagtgtctg tgtacagagg gagacttggc 12600
   agggaggccg ctaacggggg ctgtgggtgg attccgaaga cttggaggaa gtactcaagg 12660
   gagaatgttt gaactggacc cgtttcttcc ggagaaaggg gccctggaag cactgttggg 12720
   agtgtggggg tcatcagggg actttagaga gcaggaaggc taaggcggga cagaggcaag 12780
   gagaacggga ggttctgggg aaggtgtaca gttcagaatg gcagctctct tccagggccg 12840
   gactccagaa ccctgatctt ggaggcgcgg actgagtctg cgacagatgc gtctccacac 12900
   gccttcccca tcgcacactt cgtggaagta gcggcaggtc tggccgaggg caacaaggtc 12960
   tctgactggg aggaatgaga tgatatgctc cacctgggag ggaggaggga agaagcgggg 13020
   agtctttggt ggcccctcgg tgatctggcc cccaatccta ttattctcaa tccccctccc 13080
   caaggactca ccagctctgg ggggaacaac tggatggaaa tcggatttcc tttccccctc 13140
   ttctcttcaa ccccaagctc cgagccacaa gaagggcagc ttctcttcac ctacccaaga 13200
   aggggtgcat agggtcccat gagggtccca catccttttt tcccttctct ccgcctacct 13260
   tggggctcat ctgactctag ctgggcacag ttatttcctc ccctctctct ccagacccat 13320
   ccctaacttc tccaaataac tcctcccgat ctcctgcggc caatccccct cacccggagc 13380
   ccctcctccc gtccacacag cctggtgaat ttttgcagct tccgtctctg gcttcccctg 13440
   accatcctga ttgccccctc cctgctcctg ccctcctgtc tcctaggccc ttggcccccc 13500
   cgttcctgct ggctttccca caccatgtcc ccagcctagg cttgggggac ctctgaggcc 13560
   cctctgcctc ctaggcccct cccccatgga gtctggggct aacaaacctc tctgtgacct 13620
   cactgtttgg ccactgtgac ctgttccctt taatcttgcc caggacccat ctcttagaca 13680
   gctcctgggt tctagctcag ttctagccat cacatggctc aagggatatg cattttactg 13740
   gagaaataaa gtgtgaatta ctggtaggtg acatctttat ttcagggagc aggaggaagt 13800
   tttcctattt gaggcccttt tagcactaac tctgtagaga aacactagtg aattcctcac 13860
   atttcagcag gccaaggcct ctagggattg gtagctcaag agtatagact caaaaaacta 13920
   taaactcagt ttgacaggtg agatggagag gtgactgtgc taataatcgt ttagctcaca 13980
   aatgattcag caaactcttt agacttgtga aaaaacacat ccaaaaggag actgcagcct 14040
   ggtgccggtg gctaacgccc gaaatcccag cacttttggg tggccatggt gcaaggatgg 14100
   cttgaggcca tgggtttcaa gaccagcctg gtcaacatag accctgcgtc tattaaaaaa 14160
   aaaaaaaaac ttaaaaatta cccaggcgtg gtgatgcacc cctgtagtcc cagctacaca 14220
   ggaggctgag gcaggaggat tgcttgagcc cagtttgagg cagcagtgag ctatgatcgt 14280
   gccactgcac tccagcccgg gcgagaatga tacccagttt caaaaaaaac aaacaaaaaa 14340
   gaagactgct tttgctttcc tgagggacag taacatcatt gcatgtataa aggacaacct 14400
   attaaggtgg gagaaaggga tggatttcct ctgaaagctt aggatttctc ccagggtctc 14460
   tgatattagc tttgtcttat gtggggtgac atttatcagg agaaacagca ggggtcaggg 14520
   ttgtgtctgt cccactcagg ccagatctac accaataaat ccaaggagtg gctggctctt 14580
   gcaggggctc aaaggagtcc catcctgcca gaccaccagc cctcaccaca cagtgtcaca 14640
   gatccgttca ttgactgcac cagactgacc acccaaattc ccttatgtat ggtgcctagc 14700
   catggctagg ctgggatgcc tgagatggaa ttttaattct tcagcaacta ctcaggaata 14760
   tcaacctctg agatgtgaac taaggagaaa gcaacttcag tgacttattt ctgggcaacc 14820
   taccacatag ggaggggact gcattaggga ctcatgccac ttagaaagga agatctccaa 14880
   gtagacagaa aggtattttc cattcctctt gcatttgggt cccctttatc cactccattt 14940
   caccccagga ggcaaacgag aggttgtgaa tagaaaaagt ccactgtggg ccaggctttg 15000
   tggctcacgc ttataatccc agcacttcgg gaggccgagg tgggagcatt gcttgagccc 15060
   aggagtttga gatcagcctg ggcaacatag taggaccctg tctctacaaa aaatacaatt 15120
   agctggcatg gtggtgcgtg cttgtagtcc cagttactcg ggaggctgag gtgggaggtt 15180
   gaggctgcag tgagctgcga tcaagccact gcactccagc ctgggcaaca gagcgactct 15240
   gtctcaaaag aaagaaaaag tccactgtgg gccgggtgcg gtggctcacg cctgtgatcc 15300
   cagtactttg ggaggcagag gcaggatcgc ttgaggtcaa aagtttgaga ccagcctggg 15360
   caacatagca aaaccccatc tctacaaaaa ttgaaaataa attagccagg tgcagtggtg 15420
   ccgcccgtgg tcccagctac ttgggaggct gaggcaggag gatcacttga gcccaggcat 15480
   tcaaggctgc agtgagccat gactgcacca ctgcactcca gcctgggcga cagaatcaga 15540
   ccttcttaaa aaaaaaaaaa aaaaggccgg gcgcggtggc tcacgcctgt aatgccagca 15600
   ctttgggagg ccgaggaggg catattgcct gagctcctga gtttgcgacc agcctgggca 15660
   acacggtgaa accccgtctc tactaaaata caaaaaatta gccgggtgtg gtggcgggcg 15720
   cctgtagtcc cagctactcg ggaggctgag gcaggagaat tgcttgaacc cggacagtgg 15780
   aggttgctgt gagccgagat cgcgccactg cactccagcc tgggcgactg agcgagactc 15840
   catctcaaaa aataaaaaaa taaaaagagg aagggaacgg aagggagagg gagggaggga 15900
   ggaagagagg aagggagtga aaacatccac tgtgaaaact gagggagcgg gaaagggctg 15960
   tgcgtggagg atccagagac ctggtttcta gagcaagcca gctgtcagga acttgctgtt 16020
   cgctcttgct aacgcatcaa cttccctctg cctcagtttc tgcattcgta gcaccaaggg 16080
   cgtgttctag gcggtgagat cctttctaag cctctaaaac cccgtggtct ggtcccctct 16140
   ccattcctgt ccaggggagc tcctcgggtg agacggcaga ccgtgcaacg ctgcacctac 16200
   accctccctc ctcgagctgg gcatctccag gctctgggaa gccccccgcc cgcccccagc 16260
   ccgggggtcc cgagacccct gccagaggct tcacagtcgg aggtcccccc gctgcccctt 16320
   cagctgcgac aaccgccagt gcactggcct caagcagtcg cccgaatccc ctcaagccct 16380
   ggacgccggc tagcccccta acacgtccag ctcgcactcg ccactgcagc ggcgaactgc 16440
   gttcccggcc ggccaggggc tccgcgttca ttcttaacct cagtcttaaa gttctagctc 16500
   acccgcctcc tccttagcaa agggaccgcc ttctcgccca tgctattggt aggtagagcc 16560
   gtccctcaga gaagaggcag gtccagattc ccttcggagg ctctaattgg ccttcttgac 16620
   agtcactcgc ctgtatctgg cttacgattg gtctttgggg gacgaggact aggcaccccc 16680
   tgcttgcggt ccctcggccc gttttcctgc tagtggagtg ccggtgtccc ctcctcttgg 16740
   ttggacctga ttggctccac ctcccgatct gagcattgtg attgggtgca gctgcgctgg 16800
   gcgggacggc ctggagcgtc ggccgttggc gagcgctcta tccttgttcc cctcccttct 16860
   ctcgtcagac tccggcgccg gagctccacc cccactgacg ggttctgatt ggccgcttct 16920
   gacgcgtctc ggggccacga ttggtccgtc cggtcccgga caccgctccc tcccccttga 16980
   ggcaactacg gcggctccaa ggagggggtg ggggaaggag ggacggccgg tcccgtcagt 17040
   caggcagcgg gagccgccgg gagcggatgg cggcggccgt agcggctcca ctcgccgccg 17100
   ggggtgagga ggcggcagcc acgacctccg tgcccgggtc tccaggtctg ccggggagac 17160
   gcagtgcaga gcgggcccta gaggaggccg tggccaccgg gaccctgaac ctgtctaacc 17220
   ggcgcttgaa gcacttcccc cggggcgcgg cccgtagcta cgacctgtca gacatcaccc 17280
   aggctggtga gtgcagccgg gccccgggcc caaccgggtc cctcatttgc atagccccac 17340
   cccgggcccc gcccggcccg ggagtcgggc ccgccccttc acctggcggc cagggttgct 17400
   gcccctcctg attgccctaa acctgcagcc atcctctccc atctttgaga accccctaga 17460
   caatgaacgt gcttctcgtt tgcacagaac gccctgcgcc tccgagcgtt gggagagatt 17520
   cctcctttct ctttatttgc atatctgcgc acctgtagga tggtcccgcc cctcacctgg 17580
   ttggcttatt tacatgatct cttaacctca gctccaatcg gtctatgaag cagtccccac 17640
   gagctcctct aataactgct ttctctccct cttcatctac acctcttaac ccccgtccca 17700
   ccgagatccg agcagccccg ggcaccccct tcccagtgac tggcccctaa gcccgaggtt 17760
   tctgctttct gtgccgcaac cccgccccct aaggtcctgg ccctctccac tgtccacgca 17820
   gctctccagc ctatgggaaa gaaacaaatc tctacctccc ctcactcttt tctccctgag 17880
   ctcttccgcc tattatcccc aaccggctta tctgagattc ctcctcctcc ttcctaagcc 17940
   tgtgaggtca ctgtggagcc tgtgacctca tagggatctt agaattccca tttcagtttc 18000
   tgctttcccc cgggggaggg acttgaggcc gttgctggag ggaaggaaat cttatctcct 18060
   agctgggagt gctggccagc tagagactgt ccttccacgc ctccacccgc aggatctgtc 18120
   caaccatgca ccctccaccc tggtgtctgt ggatggtagg gggtggggga cttaactgtg 18180
   acaggtgagt caagcctcag ctactcagga ggcagcctat gaccttgggc cggcagctgg 18240
   gggtggggat caggatcagg gaggaggact gcattcccga agcctcaccc cagcccaggc 18300
   ctcttcctcc tctcatctgc acggtggggg accagagctg gaggatagtg cttacttctc 18360
   cctaagcagg tgcctgctca cctgggaaca ctcaggccgg cactggcaca catcctcttc 18420
   cctcttctgg ggagaaccag tcgaaacctc cccactgatc tcgcctgctc tgcaacactt 18480
   gagatgggga atgagtgagc tccaggagcc attcctgccc cttacagccc tgttccctcc 18540
   cacccccacc taaccccaca ctccagattg acctcagcag ctgtgtgaca ctcactgtct 18600
   gttcccttcc cctccccctt ggttgttttt tgacaaagac caaatgttat tctttacttc 18660
   cttaggctgt ctctccctgt gtggactctt ggggaaaact acccggagct ccccggcccc 18720
   tcccagctcc cgcccccggc cccctcctcc cccagcattc cggctcaggg tgcagccgtg 18780
   ctcctgtgag cttggggggg ggtcggaaat cccagcttcc caagaaccct cctctgcccc 18840
   actctctaca cagggcccag gttctaggcc aagtggggtc cctcttgtcc agaattctct 18900
   cctctgtccc ctctccgcaa gcagggacag aaaggggaag tgacaaaaac atacagttgc 18960
   ccataccctg ccctccatct ctcctcgccc tccaagcaga cgaaaagcat cagtgccttg 19020
   ctgcccggac tgccttgagc cttcccctcc cgtctcccat ctctttctga gtgtcttcgc 19080
   tgcgtgtcgc ctagtcttgg gtccctgatc tcccttggac taggctccct ggggtgatag 19140
   taggaagaat cctgaggtgg cggggagggg ccatcttgca aaggagaagt gcagctcatt 19200
   ctctcccaga ccacatcctg tgcccccagt gccctgcctc ctgcctgagc tcccttccgc 19260
   tcctagcttt tgtccccgcc accgcctacc ccatcagaac tctgagaact cggtctccac 19320
   ccccttcgtc cacatgcaca cacacaaaca catttgcaga gaaagcgggc aggtggaggc 19380
   gaggctggga ggggcaaatg ccatcccccc accctctcca gaacggtcat ctctgaggcc 19440
   agggcttctg tctgtgcacc agggctgggc agagaggaag agaaaggggt cccggggcat 19500
   caggctgtca tccaccctgg taaacacggg gcctaccaga gccagaacct gacagattgt 19560
   ttctacttca tttcctgtca caagactcat ttccttgggg gatatacccg gcttctctcc 19620
   tctgcttgct tttgctggcc tgcccttccc tgagaagggc ccttggagaa gctcaccagg 19680
   tctgtggcct gggactccgc ctcgcgggcc ctgggcccct cccaggacag gctgggccga 19740
   ggaaacagaa aggcaggctt ctccaggtct aagccgttag tgggtccgtg ctacagggaa 19800
   tcccaaggct gccagcctcc gcatagcaag gcagtgctcc agataagagg gcgattgtag 19860
   accccaggcc ctcccacact ctccaccctc gccgtgcctg agatgctcac acagttgctg 19920
   atctcttgaa gtctgtgtca gtaagagagg aagcagagcc gggctgctag ggctagcaga 19980
   gggtaggaag gatgcctgcc ctgggccaga gaagcccaga tgggagggac ctgggaactc 20040
   cagctcccat ctccaaccag gtcttcttgc ccttccctcc cagcccttgg gaatcagtaa 20100
   ctttatgatt ctttggacct ggcttgctgg tccttggggc tggcgaaaaa ctcgtccggt 20160
   gtcggggact ttatctctag aaccaagata gagtgacctg tggtttttcc cgtgttgaaa 20220
   acacactgga gtagggaaga agggccccga ggagggatgg gcactccctg tggtttgccc 20280
   caggggtttc ccatcttccc tccgggagcc ccccagcccc tcgccttact gttttcccat 20340
   gagcactgac ccctcctctc accttcggtc ctccgcctca cctggcatct ctgaacagga 20400
   accacaggag ttagagattc cagctgctct ccaggatagc cctccattga cattccccaa 20460
   cccccaagtc tgaggcttct ctagcccagt gacgctaagg cctttgcctt ccctgtgcca 20520
   ttctggtcac gcctggaatg tgctcccgtt ggcagaaggc tttgttatct ctcctgggag 20580
   gccacattga tggccttgaa tctttaggtg tggaggtgag ggagtgggca gccggctctg 20640
   gggggactga aggtcctgtc cgggctgggc ctttccgcaa ctctccccga cacctccttt 20700
   tccccagacc tgtcccggaa ccggtttccc gaggtgcccg aggcggcgtg ccagctggtg 20760
   tccctggagg gcctgagcct ctaccacaat tgcctgagat gcctgaaccc agccttgggg 20820
   aatctcacag ccctcaccta cctcaacctc aggtagggag gccgagccag ggccacgtgg 20880
   gagcgtgtgc ttctcagtct caagtactac tgagtgaccc acccagtgac cccgagagga 20940
   tggcagtgct gggatgccat gcctggggag ccagtccgct gcctttctcc cttccacagc 21000
   cgaaaccagc tgtcgctgct gccaccctac atctgccagc tgcccctgag ggtcctcatc 21060
   gtcagcaaca acaagctggg agccctgccc cctgacatcg gcaccctggg aagcctgcga 21120
   cagcttgtga ggatggggac gggaccagga aagggacccc tgagccaggc agcgggacct 21180
   tctaggggga ggttggggag ggcctggagc aaagacagag atgggagggt gggttctgct 21240
   gggcctggtc tcatggcccc ttcccactgc cttcctgata ccaggacgtg agcagcaacg 21300
   agctccaatc cctgccctcg gaactgtgtg gcctctcttc cctgcgggac ctcaatgtcc 21360
   ggaggaacca gctcagtacg ctgcccgaag gtgagaagaa tgaagaactg gagaggaggt 21420
   gtttgtatgt gcgtcccagt tggctcaggc tgcggtaacg aaaacactgc agaccgggta 21480
   gcttatacat ggcaggcatt catttttcac agttctggaa gctgaagttc aggatctggg 21540
   tgcttccatg gtcagcttct ggcagggtgc ttttcctggc tgcagactgc cggctccttg 21600
   tggtactctc cgatggcgga gagcagagac agccctctgg ggcctctttg ataaaggagc 21660
   acaaatctca ctgatgaggg ctccaccctc atgatctcaa ttacttccct aaggcctcgc 21720
   ttcctaatcc catcactttg ggggttagga tttcagcaga ggactttttg ggtgacacat 21780
   ttcagtccat aacagtgtat gtgctttgaa gaagttacag gccaggaatc ttcagaggag 21840
   aattacacct tcattgtcca ggcttttgca gtatataaaa caaactgtaa acgatctgat 21900
   ttgatcttga cctagcaacc ctctgatgct ggcagggcgc agatgagcag ctgtgttaga 21960
   aacgtggaca caggcacaga aggggagctg gtgggtgcat cagctctagg tagactggca 22020
   aggtcagaag gggccttcaa ggtcacccag cagccctgct caccccagca ggggctctca 22080
   aacacctgag tcgggatgct ccgccactcc tcccggagat gccagggcca gtgccagagc 22140
   ccaggtggcc catctaatgt gcttgttgtg atgcactgat agcctctgcc ccaaagcagg 22200
   catgcctttt gttcagcagg cagcagcatg tgtgtgtgtc tgtgtgtgtt atgtcttgtg 22260
   tctgcgtgca tgtgtgtgtc tgtgtgtatg tatgtgtttg tgtatgtgtg tgttgtgtct 22320
   gtgcatgtgt ctgtgtgtct gtgcatgtgt gtctatgtgc atgtgtatgt gtgtctgtgt 22380
   ctgttcgtgt gttgtgttta tgtctgtgtt atttatgtcg tgtgtgtgcg tgtgtatgtt 22440
   tgtgtgtctg tgtttgtgtg tgtgttgtgt ttatgtctgt gttatgtcgt gtgtgtctgt 22500
   gcatgtgtat gtgtgtttgt gtctgtgtgt gtctttgtgt gtgtgtttgt gtttatgtct 22560
   gttgtgtgtg tttaggtctg tgttgtgtgt gttgtatgtg ttgtgtgtgt ttgggtgtgt 22620
   gttgtgtggg ttgtgtgtat gtgttgtgta tgtttatgtg tgtgttgtgc atgttgtttg 22680
   tgtctgtgtg tatgtatgtt gtttgcgtgt gtgtgggttt tgtgtgtgtg tgtgtgtgtg 22740
   tgtgtgtgtg tgtgtgtgtg tgatgtgtgt atgattcctg tgttgggatc aggtagatgc 22800
   gggtgatgtg actaagatct ggggccatct cttagggatt taatggcttc ttgaggagag 22860
   acagtcatag ggaagttccg tcagggggtt ccccagacgg gggctagagg gaggtggcac 22920
   tggactctgc gtgggggtcc tggggaagac ttcaccggag gcgcaggcgc agtgggagcc 22980
   gaacattgaa ggagaattcc ccagccttct gcatccaggc agaggaccca gcatgtgcaa 23040
   agcatggggt taggaaggtg agccaggcat ggcaggaagg cctgtgccat gctcaagtgt 23100
   gaacttggtt ttgaagaaca aggagggagt gaggtggtca tccactttcc ctgtctccct 23160
   actccctgac cctttctgca cctgccctct tccttcccag tcaaaagggg taggaagggc 23220
   tctctctggg ccataaaaag aaatccaagc aagatgtcct ggggacagag gcaagagcaa 23280
   tgagttccat ccaatctggg atgtctggaa cgatccaccg aggaggtggt gatgcttgag 23340
   ctgaaccttg aaagatgcat ggatggggaa aatggagttc tcccagctga gcaaacagca 23400
   cctgggaagg ctcagaagct gagggcccgg aggggtgggc agtgaggtgg gaaacgcggg 23460
   tggagccagg ttgtggaagc cttcctcatg ctgggttagg gagctgtggg ttttattctg 23520
   tgcttagggc cgccatgaat ccagactgtg cctttttttt tttttttttt gagacagagt 23580
   ctcgctctgt cacccaggct ggagtgcagt ggcgcaatct cggctcactg caagctccgc 23640
   ctcccaggtt cacgccattc tcctgcctca gcctcccaag tagctgggac cacaggcgtc 23700
   cgccaccaca cccggctaat tttttgtatt tttagtaaag acaggttttc accatgttag 23760
   ccagggtggt ctcaatctct tgaccttgtg atccacccgc cttggcctcc caaattgctg 23820
   ggattacagg cgtgagccac cgtgccggtc ccagactgtg cctttttaag atgagcaaaa 23880
   ggcacctttc cctgtgggca ggcagcattt catgcagggt gcagtgctgg gcccgtgctg 23940
   gggaaagggg actcccgggc ggccacaatc cgctcaacat ctgcagccct ggttgcatgt 24000
   cacaggaggt gtttgaacag ggtttgtgcc ttgtgctgat catttgaaaa tggagggtgg 24060
   gttcgagtgt ggagtgggca ggcgaggaga tgaggaggac agacagggct gggcgggacc 24120
   tgacgtccgg atgggccttc ccggggaagc tgagctgacg tccagatggg ccctctcagg 24180
   gaggctcgct tagaccgtgg accagaatca tgtggagaga ggagcaggga ggtttctccc 24240
   ggggaatgac ttgagccacc caggggagag gctgtgctgg gcccagggtg actggccagc 24300
   tggagccaag ggtgagggag tgagcacgag gccacagccc tgagcatgtt cctgactttt 24360
   cccttgccca cctgttccag agctggggga cctccctctg gtccgcctgg atttctcctg 24420
   taaccgcgtc tcccgaatcc cagtctcctt ctgccgcctg aggcacctgc aggtcattct 24480
   gctggacagc aaccctctgc agagtccacc tgcccaggtg aggcgggtgc ctgggggccc 24540
   acgaggacag gggtgtggct aggcaggagt ggggttggtc cccttggata gctgagcagg 24600
   atccggggag ctgccctccc tgccaactcc ctggcgtgtg ctgccccgca cacaggtctg 24660
   cctgaagggg aaacttcaca tcttcaagta tttgtccaca gaggccgggc agcgtgggtc 24720
   ggccctgggg gacctggccc cttctcggcc cccgagtttc agtccctggt aagtcctagg 24780
   tggggaggaa ggaagccctg ggatactgaa tgccccttcc cggccccagg caccccctgc 24840
   tttctggcag gaggatgggg ggctgttgtc cagcatcagg ctgaccagat ccgcctggct 24900
   ggccccagcc ctgcagagga tctatttccg ggacatcggt acgatggtgg gctggactca 24960
   ggcttccaca gcgttgatag tggcagcaag aggtggtctg gaaatgaggt aagggcgtcc 25020
   tgtccatggg tgattgaggg tgtcagccct gggagagcac ctctgaactg gggacctcca 25080
   aattgcaggg tgccaggcag aggctggagc tgtcctcttc cttccctctg cccagcatcc 25140
   cagggaaggg ctggctcagc agagaccctg agcttagtcc cacacaggac cccccaggtc 25200
   ctgagccccc atgtacagag aggccatctc ccatctctgc ctctgccttt acagtcaaca 25260
   gatgaatttt cagagctgtc attccggatc tcagagctgg cccgggagcc ccggggaccc 25320
   agagaacgca aggaggatgg ctcaggtgag tagcagggct ggccgggagc tgggctggac 25380
   cagggcctgg gtccccgctc acttgctgac atgctggcct ccccagcgga cggagaccct 25440
   gtgcagattg acttcatcga cagccatgtc cccggggagg atgaagagcg aggcactgtg 25500
   gaggtgagag cccagctatg cctctcccaa gggaggggag ctggaggagg ggacagggca 25560
   ggcgtgggca caggcctgcc ctcagctgct ctgctgttct ctccttagga gcagcgacca 25620
   cccgaattaa gccctggggc aggggacagg gagagggcac caagcagcag gtaccccaag 25680
   cccccaaact gacgccactg cccgatcctc ccacccccca accgcctcct tccctgaccc 25740
   cggggtctgc ccctcttgcc ctgtcttggt ccctcaggcg ggaggagccg gcaggggagg 25800
   agcggcggcg cccggacacc ttgcagctgt ggcaggagcg ggaacggcgg cagcagcagc 25860
   agagcggggc gtgggggccc ccgaggaagg ataggtggga cccagggccg cccggggctg 25920
   ttgctggaca ctgagccctg gaaatggggc caccgagccc cgctgagccg ttctgccttg 25980
   gggctagctg accccacctc tccttgttcc agcctcttga agccagggct cagggctgtt 26040
   gtgggagggg ccgccgccgt gtccactcaa gccatgcaca agtaggtttc ctgccagcat 26100
   cccctcccca ctcctcttcc tatgaccatc atccctctaa ttcccttctg tctctcctca 26160
   gcggctcgcc taagtccagt gcctcccaag caggggctgc agcggggcag ggagcccccg 26220
   cccctgcccc tgcctcccaa gagccccttc ccatagctgg accaggtggg acagggattt 26280
   tgggaggagg atggggcctg tgttggtgag ggctggttgg gagagaggga cacagagggg 26340
   atgcctgtct cacctgcccc tgtcgctgtg tccctggttc cggccccagc gacagcacct 26400
   gctccacggc cacttggctc cattcagaga ccaaacagct tcctcttccg ttcctcctct 26460
   cagagtggct caggtgggtg tcctgccctc cccagtgctg acccagtgct tgccagcacc 26520
   aacggctaca tcacccttct gccctgggag ccctctgggt ctgttgtacc ttttgtacct 26580
   ttgcgttgaa aatcccacaa gcaagccggg tgtggtggct cacacctgta atcgcagcac 26640
   tttaggaggc ccaggtggga ggatcgcttg agcccaggaa ttcaagacca gcctgggcaa 26700
   catagcgaga ccccatctcc atgaaataaa aaaaaatccc acaagcagag aggcaggagg 26760
   tgtaggtgcc aggccactgg gacccagcta cagagtcagt ggtgagcagt ggaggcagcc 26820
   ccatgccccc ctttttctcc taggcccttc ctcaccagac tctgtcctga gacctcggcg 26880
   gtacccccag gttccagatg agaaggactt aatgactcag ctgcgccagg tgagcagagg 26940
   cgggagctgc gtgggcaggg gcctgggccg ggcacctcct tgtgttgctg tccttgggac 27000
   cagcctccct ccacatcctc tgtgcccact ctggcacctc ccctgaggac aggaagggac 27060
   aggcacaagt tccctgctag ccctgagccc cccactctcc ctgcctccca gacgcctctc 27120
   cttccctgac ccctgctcta tgtggtctac actgccaggt ccttgagtcc cggctgcagc 27180
   ggcccctgcc tgaggacctg gccgaggctc tggccagtgg ggtcatcctg tgccagctgg 27240
   ccaaccagct acggccgcgc tccgtgccct tcatccatgt gccctcccct gctgtggtca 27300
   gttggggctg gggaagaggt gggggatggg ccggggcgcc tgtggcctct cccttaccac 27360
   attttcttct ttcacccacc ccagccaaaa ctcagtgccc tcaaggctcg gaagaatgtg 27420
   gagagttttc tagaagcctg tcgaaaaatg ggggtgcctg aggtatgggg gctgctttct 27480
   aaagagtggt ggcatgccgg actcagccga gcatgtggca tggatggtgg gcttccattt 27540
   gcagcggatg tctgctctca gatgaaggca caggctgccc ctgccctgcc cagcatgccc 27600
   cctgccctgc atgccccctg ccctgcatgt cacctgtcct acacatcccc tgccctgcag 27660
   gccccatctt gtcctgcatg tcacctgtcc tgcacatgcc ctgccctgca ctcctcctgc 27720
   actgcactcc ccctgcccag cacgtcagca tgtgggcgtg gttctggccc cagcacgtga 27780
   gggcaggggt gggagggact gtcctgctgc tgctgctgac gtctgtccct tgtccttgtc 27840
   cacctgctct cctctccccc aggctgacct gtgctcgccc tcggatctcc tccagggcac 27900
   tgcccggggg ctgcggaccg cgctggaggc cgtgaagcgg gtggggggca aggccctacc 27960
   gcccctctgg cccccctctg gtctgggcgg cttcgtcgtc ttctacgtgg tcctcatgct 28020
   gctgctctat gtcacctaca ctcggctcct gggttcctag gccccaaaat cggccctccc 28080
   tcaccccttt cccttcctct ctatttataa ggtccctgct ccacccgacc ccacctgcgg 28140
   tgccttcagc cccaaccaaa gacactagtg cacccccttc acagacactg acctcagagg 28200
   ccccactctg gtgcccccag accctgggcc cccagcctct ggcctccctc cagtagcccc 28260
   acgagtcccc acctctcagt gctgacggtg ccttcatgtc cccgccggcc ctgcccctgc 28320
   cctctgtacc ccgtgagggg tggcaggagc tggagtctcc cccttcctcc tgtgccctcc 28380
   ccttcccccc ccaacagctg ctatgggggg gctaaattat ctctattttg tagagaggat 28440
   ctatatttgt aggggttcgg ggcccaggcc gggtccctat ctctgtgtat aaactgtaca 28500
   gaccgtggcc gccctgcctg tgtgtgtgtg tgtgcgcgcg cgcgcgcgtc tgctccgcgt 28560
   gttggtggct gtggccatgg ctctgtgccc accagcatct ccctcctgag atgccggcct 28620
   ctcatgctcc cggagcgtcc gccaaccccc cgtgtcacct cccttctgtt atcgctgaca 28680
   gctttcttgc gtctcatttg tcgccgagcc ccgagcgcac ggtgatgctc gggtctgccc 28740
   ccgaccccct gccacaggcc ggaagccgca gggggcaccg tggggaagct aacccggccc 28800
   cttcccccag gagtcactgt gccagcccca ccacatcctg gaagaggagg gggccccggg 28860
   aaggggcctc ccctacatcg ctgctgtcgt ccacgccctg ctggaccggc cttaggggtg 28920
   aaggtggggg cacagggccc accccgcccg ggaccccggg agcagaagac gcgcctgggc 28980
   tccgcgctct cagagaagca cgtggtgagg gtggcctggg cctgggcacc cttggcctgc 29040
   cggcctggct gcctctgggg tcgagggtct gggtggaagg accgcgggtg tcgcaggtgt 29100
   cgtgtcagcc gcaataaaca gaagccagaa gccctctggg tggccctcag gtgtaactgt 29160
   gtctgagagc acgtgcgtcg cggggttgtt gggggggcct ctgtttccgg aattcaccgc 29220
   ccagtcgcca gggggcgccc gcgggagccc ggcgggggca cgcgccacgt ggccgggaga 29280
   cgcacagggc gccagcgtcg ccgcgaggcc tctggggtgg gttcgccagg agggcacgtt 29340
   cgcgccgcgc cgcggaggga ggctgagagg gccgggccgg gcggcgggga cacggcggta 29400
   ccgtcctgcg ctcgggaacg cggaatgttg ccctggtcgc cgccgcggtg gggcgcgggc 29460
   ccggaggagg cgcccgaggg accaggcctt ccggcgggca aggaccaggg cgagccctgg 29520
   agcgccggag cggacgcccc cagggagctg ggacaccccc catgggacag gtgaggggct 29580
   cggacggagc cgtgggggga cagcgactgg gggcctccat gccttcacca cgcgctctac 29640
   ggaggcttct ccattgtccc tgctggagat tgggtccctc ggtctccctt gcaccaggca 29700
   cccttgggct ccaggggagg gtgcgtgggg gcggtgtggc tgcagcctca gtccctgccc 29760
   ctccagccca cagcccccat cccgtagccc ttcctggatc tggagagagc agctgctgct 29820
   gccccaccac ctgccaggcc cggccactga gcagcccccc ggagccccag gtaggacagg 29880
   ccctgctgcc tctactaccc ctaagagcct ctcactgact gtgagcctgt tataatcccc 29940
   aagcgtcccc ccagatcccc gttcccccca ggtggcctgg gaggtggccc cctcgaggat 30000
   gactccacta gcgccctggg accccaagta tgaagccaaa gcaggacctc ggccggtgtg 30060
   ggtgagttgg gggcaaacct gtgggactgg ctggggtgct cagggagctg tgcggtggcc 30120
   tgaggctcca gtgctctgtc ctcctcaccc tagggggcca actgtagctc aggagcctcg 30180
   ttctcaggcc ggacgctgtg tcacccctca ttctggccgc tgtatgaagc agcctcgggc 30240
   aggggtctca ggcccgtggc ccctgccaca gggcactgga atggacagca ggcgccccca 30300
   gatgcaggta cctcccttgg tcccccgggg gcgcaaggct gccccaactc ctctgggaac 30360
   cagtgcccag gggtgggagc ccgcagcccg ccaccctagt ggtgtttcct cctagggttc 30420
   ccggtggtgt gctgtgaaga tgtcttcctc tcggaccctc tgctgccccg ggggcagcgt 30480
   gttcccctgt acctgtccaa ggccccccag caggtgagag ctctttcccc tccctgtcct 30540
   taccccagcc tccaggcttg cctggcagga aacacattcc attctctctc ctcagatgat 30600
   gggctccctg aaactgctgc cgccgccccc catcatgtct gccagggtgc tcccccgccc 30660
   atcaccctcc cggggcccct ccactgcctg gctcagcggg ccggagctga tcgctctcac 30720
   tggcctgctg cagatgagcc agggggagcc taggcccagc tcctccgcgg ttggcccccc 30780
   agaccatacc tctgacccac ccagcccctg tggtagcccc agcagttctc agggtgctga 30840
   cctctctctc ccacagaccc cagacaccca ttgtccatag ccttctcagg gcagagtggg 30900
   ctggttgtgt tgacaataaa acagtgttgg tttgcaaaac aggctccgtg tggctgggag 30960
   cagagtaagt catagaggca ggaatggggt ctctttctgt ctcactgcag atcaccccct 31020
   gctgtgttag gcggagggcg agggtatcca tgacctaagg catttctggg ctgactgggc 31080
   agagcccctt taatgagtgg gtgggtggtc tgcatggcag tgcagggctg aatgtatagg 31140
   aagcaggcgg gggagtaagt aggggcacct cacttttagg ggacactagc cccacacctc 31200
   ttgtgctggg gctccaggag ttcagttctg ggctgggtgg ggtagttgtc tggctcagag 31260
   gagacacaaa gaatgcactg caggtgtgtg ggggagggtg ccggtccatg taaatgtcac 31320
   cactggcaga aaggtggccc tgccgcagcc tacctgagac tctagagatt cttgcacgaa 31380
   ctctgtagag aaacaggatc agggtttcag ctgagaaagc ccagaagtgt gtagtctcgg 31440
   tggtttgggc ggggataggt tctagaattt tctcccataa aatagcccaa cttcttggag 31500
   aaccttctac tcaagactta ggtgccacag ctggaaggga cacagggggc aggaccccag 31560
   cttgttgccc tgcaggtcac ttgtccgagg tggcatctgg gccttaaagc ccctcacacg 31620
   gtggggagtt aggaggcagg gcgggggcgt tacaatgcca ttccctccag tccctccctc 31680
   aaccaccccc ccatcagtca gcaggagaga gctgcctggg tctcaggtct cctcaccgca 31740
   tagctatgtg ggcggagcgt gtgggccctg gggacgtcac caagtgcccc cagagaaagg 31800
   accaggtcca gatcgacgta gctgaggcag ctcgtcaggg gctctggcct ggcggtgccg 31860
   gttccgaggc tctctggggc cgataacttg gtgcacactt cactccctag ccagctctga 31920
   gggggcaggg agcagcgctg agtccctacc catccccagc cagcccccac atcgcgatgg 31980
   gcactcctgg gcctgtgtcc aggctcctga acccagactt gtaggaatgg gctcatagtc 32040
   actccctgct cccttggtta tgtagccccc acccagcccc ttagggtaga aggctgctgt 32100
   ccccagcctc gccaaggtct taagggctgc ttgctcgatc ccctctccca gacaccctgg 32160
   acggctcagg ccacttgact ggtccgcaga agctggggtc tcagggatgg ggaacaggga 32220
   gtctgaaggt gctcatagga gggtccctga gcacctgggc ctcgtcattg tgcctggaga 32280
   actctttttt tttttttttt tttttttttt tttttttttt tttgagacag agtctagctc 32340
   tgttacctag gagtgcagtg gttgatcttg gctcactgca acctccgctt ccccggttca 32400
   agcgattctc ctgcctcagt ctcctgagta gctgggatta caggcatgtg ccaccacgcc 32460
   cggctaattt ttgtattttt agtagagatg gggtttcacc atgttggcca ggctggtctc 32520
   gaactcctga cctcaggtga tacgcccgcg ttggcctccc aaactaatgg gattataggc 32580
   gtgagccacc gggccgggcc tgtgcccgga gaactctaaa cctgcccctt taatgtttga 32640
   actttcggcg acctggggga ggggcgagcc acggagaaac cacagccaga agcccccgag 32700
   gcccccggaa gcccggggct cgggcctcac cggcatcacc gcgctcctcg agcagggtgt 32760
   tgtatcacgc ctgctgctcc gcctcgcctg ccgccgccac gcgcaggctg ctgtcgcggg 32820
   tgtagaggac gatcaggtgg tgctggcgcg catctgcgca cttgctggtg gtgcacgcac 32880
   ccgccaggct caggctgcgc tggagccgcg ctcggcacgc gcgaaacttc tgctcgctct 32940
   cggaacactc gggacgcggc gggtctgcgc gcaggtggcc gcagaaccgc acgtcggcag 33000
   ggcaggccca gggcagcggc tggctgaggg gcacggcggc cattcccggc tctccccggg 33060
   gccgccgcct gcgggcttca ttcaggggcg aaggcaataa agtggctgca gaagaggcag 33120
   agacggctgg gtctcgtgtc ttgagcacgg ggagaggcca gcaggggtgg gggtgaggtg 33180
   gcgaactcgg cgggccggga gcagcggccg ggcaggctgc cacccgtctt gatgcccagc 33240
   gtctctcccg ggctcccgac tggaagtttg gggcaaggac tctgcgcaca gcgagtgtcc 33300
   cggtgactgt tgcccctggc ctgggctccc cactacctcc ctccctccgc cgaccgctcc 33360
   ggaaccccca ccccacgaga ctaatcctca cgtcgcccag gaactggagt ttgagtaggg 33420
   aagtccaaga gaggcggcct cgaccccaag actgagcctt tataggggca gagagcgtgg 33480
   gggcgtctct accgatcagc tgactgcggt ccggacacgt cgtcggggca accgagaaag 33540
   cctcggtgtg aggccagtga tcggacttgt ggcagcggtg gtggcgctgg cgattagagg 33600
   gctttggaac ggggcctcca tgcaccctgc tggctcctgc cacgccccgc tcggcctgta 33660
   ggtgtccttc ctgtgtgtac cccttcccca tccaggagtg gttttgtgcc cttccaagta 33720
   accaaaggag ccaccgccac tgagcactgc gctggattat ttctgagctg gagattgagt 33780
   ttcttgaaag tttggggtga taggaggtga cttgggctaa gtaagctcag ggggtagtcc 33840
   ctgtgcacag accatacccg ctggaaccca gtcaagtggc cactgaagga gacctcggag 33900
   gaagaggcca tctctcagga agaccccttc gggctgcccc attgctctgg gcccctcctt 33960
   gccaccactg cccaaaaata ctaccccggg accacctgaa ggctctgacc atggacgtgt 34020
   ggacttcctc atggaggtac cctttgcttc ttgaaaaaat tgcgattggg ctggggactg 34080
   gacatttttg cactcagttc tgatccaagg accactgacc ccctccaagg ggatgggggt 34140
   gaaggtgcac cagacaaacc agcaggtgac acctggtggg accttagagg accctgagat 34200
   atcacccaag gcaagtaagg tgcctaaagg ggaaagccga gggtcaggga ggagaggcca 34260
   acatgcagcc cccataaaga tggcattcag ggaggagctc cctcagggaa ggaacaagat 34320
   atgacaaaga cagaacaaga gaacagatgc aaggacccaa acctagccca agggccaggg 34380
   cagcctgagc agggtgggca gggcttcccg ccacacttcc tgcttcctgg ggaaggggcc 34440
   ccacgtgagt gggaatatgg gaggcaagtg ctgtgtgcat ggagctgggg tcctcacaca 34500
   cctattccaa ggtcacattg agagggagat gcagggaacc aaccagcacc cagccagcag 34560
   gcaggtggga cccagggcct gctccaggtg acctctggta agccaaagcc ccactggggt 34620
   gcaggagagg ctcgaagcac aggtggtgtg gtgtggggcg ggggggccgt ggggcggggc 34680
   tcggcaactg cccagtgttg ggtgttggcc acagaggcaa tggctccgta agtggccaac 34740
   aaaaccggac tgtgttcaag gccatagccc cacgtggcca agggccttga tacaggcacc 34800
   atgtgcaaag gggcagggac acaaaggaca tgtgcacacc cctccccctc ccagtcctgc 34860
   tgctgcctca aagaggccac caaagggccc ctgtccctgc tgcactccgg gcccaggagt 34920
   cttttttact ccctgaggct tgaagatacc tgaggaagat ctctttattc cttccccttc 34980
   ccactcccca tcaaccaggt ccccctgcag cacccttcct tgtgaaatca cagctgctat 35040
   gacagtgacc agctccaaac caccctgtgt cactgaacct tgggtgctgg gtgacacatg 35100
   ggcaggaaag gcaggaagag cagggatctg agggcctggg ggctgggcaa gcctcagggg 35160
   tggggtgggt gctgatcaat gccaggtgat caatgccagg tgattggacc cactgagtac 35220
   caggacaaac ctggagtcac gcaactggcc tacatcagtt gccttggcaa cacactgccc 35280
   ttgaccctac ccggaaaaga tttatttcag ccggtagccc tggtaacttg agtggctccc 35340
   cagggacagg aagtggcaga acctcaactc tgcctctcct gacctgaggg gttcactttt 35400
   catccctgtc agggacgggg cactgcgttc tcagccccta tggaagaatg ttatgggttt 35460
   gggggaatcc acggggagga gactaggctg ttccagcgtg cccctccttc ctcagcctga 35520
   ggccttgctc ctctgtggaa ggcaacacag ggacagagcc acacattcag cccctaccct 35580
   cagccctgct cccatcccct ggcccacagg tctgacccgc aagctccgat gtggagacac 35640
   ttggtggctg actcctgctt cctcacacat ggggatggcc cacaggtgac cgactggctg 35700
   aatgctgggg ccatgctaga ggactgctgt ctgcagagca tggtccctgc attggccaga 35760
   agttggtgtg agcccaggtg agtcactcta gccccgtggt gtgtgtggtg ggtttgaatc 35820
   tgcagctacc aggcaatgtt ttctccattt cctctcaagc caggcctttc tccattcttt 35880
   gctactctcc aggagaggcg gctcactgga gggctggagc ctgagctgct ggagctgtcc 35940
   ccggccaagg agagcagctc ctagtgtcag cctgtgccca gggaaccctg gggcccgagg 36000
   ctgacctggc gttaacttct tactgctcat ccagctctca ccagccagca ggctggagtc 36060
   cttgtgggct ggaggggagg gagtctgcag ggaggacccg ggcctggacc cagatagtga 36120
   ggagatgccg ggagacagca cagggtgaca gaagcaggag aaagcccttc ggtttagcct 36180
   cagttccaaa cctcggcgct cctggcatta agggaagggt gatggaagct ggggggaaat 36240
   aacagaaagg ctcctccccc taatacacca taacagattc cagcttccct ccctccggct 36300
   ctaagcagaa aggaaagaac tggtaagaga attaggtaga gggacacaga gataatctag 36360
   ggtggcgaag aggggagaaa gagaagagat ggaacatgca aaggccagga gcgaagcggg 36420
   agaccaccca ggagacagaa ggagggcaga ggctgggaga agaacaggaa cagacaggat 36480
   ggggtcctga ttgttggaaa agacagggag gccaatccca gccttgcaaa tagcagcagc 36540
   tagaggttat ttccagaaaa atctgcaaag taaatacact gatcacccaa ctcccatatc 36600
   tctttgctgc tctctgtgga agactcagac aaaccccaag aatgaatggc cacggggcca 36660
   gggcaatggt ggaaagggag gggcctcacc tggccttggg ccaaacagca aggtggcctc 36720
   tgcccagcca ggtgaggtgt ttgccaccca ccccctgacc aggtctgtct tgtcaccaca 36780
   caaatagccc agacaccaca gctggatgtt gtcacaacat ccccaggtgg aaagagcctg 36840
   gggcagagaa tgttccagac ccaggagggg tctacttcca gcaccacatt ggcaccaacc 36900
   tcctttgccc accctggccc cagtgggcat tcccctcaca tccaacaaac tccagtcccc 36960
   ggcagtggtg gctcctctcc cgccacgctt cttctcattg ctaatgcggg actcttgttc 37020
   agacagggac gggctctgcc ctgtctctgg ccaatggcag cattatcact ggcccaggca 37080
   gtccagccca gcagagccag gtcaaggatc agtcatgttg actgacctgc aacccctcaa 37140
   agtggcctga agcaaaacca caggcgccgt tttctctttc gttcaaatgc agttcttttc 37200
   caacattgcc tgcaatgagc tttggtgcag agatttgggg acagggaggc acatggagaa 37260
   aaaaaaaaaa aaaaaaagac caggcaattg cacaaagcct ttccctggaa aacccaggct 37320
   ctggggatgg tgggggtcag gacacatggc tgccccccct ccagccctgg agctccggct 37380
   ttgatggtcg aggcacagat ggggattcat cccattcccc acatacgaga gcagtctggg 37440
   agcgggagat cggctgggtg tgtgtatata tgtatgtatg tgcatataga tagatctatt 37500
   tatatataat aggctatatt agaaaatcct ctctatgtat aaatataaaa cactggtatc 37560
   caaacatgac accaacagct aactaaagtg cttgacagga gccgggcccg ggtaagctcg 37620
   atggtaaacc gctcatcctg ggcgctgggg aagcaaaaca atgggggacc ccttcagcag 37680
   ggggtgctgc aggaggaaag tgtgtgctgg ccaagctggg ccaccaagag cacttgggcc 37740
   acctgggcct tctgggtctc aggactgtct ggtcactgcc ccatcaacac tgtgcctcat 37800
   ctttccccta ccttccccac tgcgctcccg tccatgagga gcacagcgct ggcgagctag 37860
   gcccagggaa gagctgttcc acaggggact gatcatttga aaaaaatccc tccccctcaa 37920
   ccatcactcc tccaggtcag gccctgcccg cactcctcct gggcttcctg cctgggtggg 37980
   ggtgggacga gggaggggag agaggctttg tggaatctga gggcagccac ctttcacctg 38040
   taggccccaa gcaaagcatt agcacctcca ccatccccaa ggcccataga aatgcccaca 38100
   ggcaagtggt caccagagct ctagggagca gaggggcccc agaaggcagg gaagaggccc 38160
   cccaaaaaca cagtgctaca agaaggggtt ggtggttgga ggtttggagg cgaatgggct 38220
   tgatgagggt ccagtctgga aagaaagaac aatacatttt ccagttaggg gagaggaact 38280
   ggaagcttag aagaaaggca gccaggagtc ctttctcctg aaaaatcgct atgctgaggg 38340
   cagtgggaaa gtgagggaga aaaggccagg cacggtagct catgcctgta atcccagccc 38400
   tttggcaggc caaggtgggc tgatcacctg aggtcaggag ttcaagacca gcctggccaa 38460
   cacgggaaaa ccctatctct actaaaaata caaaaattag ccatgtgcgg tggtgcacac 38520
   ctgtagtccc tgctacttgg gaggctgagg caggagaatt gcttgaactc aggaggtaga 38580
   ggttgcggtg agctgagatt gggcctggga gacagagtga ggctgtctca aaaaaaaaaa 38640
   aaaaaggaaa gtgagggaga agggagccaa gggcagggct ggaacagcat gggagatgcc 38700
   agtgcccaga agagctgagg cccaagctca gccctggctt ggacaacgca gaccttcacg 38760
   aggaaggggc acaggggcca cctcacatca gcctcttctg gaaggattgt cccagaggga 38820
   aagcccacga gccctaccac agcacccctg ctggcacact caccatgaag gggttggtgg 38880
   agatcccagc tggctggctc agtggcctct gccccaactt ggctgatcct aagtccccga 38940
   aggaagagcc tagggggcga gagcagtcac tcagctgaca ccagcctgcc ttcctgcagg 39000
   cggacagcaa ggggggccct cctaaactgg gggagtaagg gaacagagca gtggaagtac 39060
   ccgggcgggt ctggtgcttc cctggtcccc cctgctggtc agaaagggca ctgcatgggc 39120
   tctgatgagg tcggaggaac aactaactga tctctatgca gagatgggat ggggtgaaag 39180
   ggtttgtcca tctacagctc ttaccattct gctgctgaac aagcggggtc tgcgggggga 39240
   acagcggtgc tgggaaggag ctggcaaagg ccccagtggg tggcactgcc tgggggaagc 39300
   caggcccagc actgctcatc ccaaagccgg gccctatgga gtgaaagacg ggaggctcag 39360
   gtgaacttct ggaaggtggg ctggagaact gtttttccta agtccctcca agatgggaaa 39420
   atgggatata gttttttcct gacatgagga acgagggacc cagccatgga actcttctgc 39480
   ttctcacacc accaaacaac cctgccctct ccctgcccgg gggtacgtgg cagggagggc 39540
   atggtagttc caggcacggg cgcggtgcag gactgggcta gcctcccact aaactgcaaa 39600
   aacagcacaa acttctaacg ctgtctgtgc ttcagttctc atctctaaaa tggggacagt 39660
   ccgtctcata gggctactgt gaagatcaaa tcagggccac gaagccctca gcactgggcc 39720
   caccacacag ctcttctgtg tttgctctta ttcctctctc cccagcctca aggcttcagc 39780
   cagcatgagt ctgaggggag agcagcagag gctggggagg gccagggtga gagaagacaa 39840
   caggcctgga gttgatggaa gcagggaaca gcgtgagtgt ttcaggtggc ttaagcttcg 39900
   ggacacagaa gggactgggg ctgggtgccc aaagggacac tgctgagggc tggagacagg 39960
   agaatcgctg ccactgtcag tcaactctaa aacagtcttc agtgcggtct ccggtaagta 40020
   gccacctgtg tgcttctaat tctaaaatct ggctctgtga cctgggtgct aggtggcctg 40080
   gcaccgtgtg ggcaggggaa gaaccaggct gtctgcttct cctgacagcg tgagacaaag 40140
   ggttaaggga gaggctgcag ggtaatgtgt gccttggaag tctcagatgg tcaggacaag 40200
   ggaccatgct ggaggcttac ctggcgccaa gccattgggc tggaacgggt tggtggaagg 40260
   cagcggggac tgggcggcgg gagctgtgaa agggttagtg aaggctgccc aggtggaatg 40320
   gggagcggca ggaaagagag agatggttac tttttttctc acgtgcttca tcctgtgatg 40380
   cctgagcatt cctctcaatc tctccccttc ccaccctgtc cagagtcctt agactcctcc 40440
   tggcctcagg tcccctcccc tgggtctcca caggcaggac tcacctccaa aggccagccc 40500
   tgtgctgctg ccgccgccgc cgcccatcgt gacagactgg agcgggggga cctggccagc 40560
   catcccgaag aggctacaac acggggagag agaagtcata cacagctgcc agggccaatc 40620
   aaacctgagc aaggggccag gagatgccaa ccctgctctc cagggtgagt aagggagagg 40680
   aggggagggg agaactgcca ggagatgccg atggggctgg gaggaacatg gccaggcttc 40740
   gggcggctct cctgccctac agcagactgc ccactgtctg tacctacctg ctaggaacac 40800
   ctgcagcggg caccccgggt cccaggaagc tgcccacgtc tgcgaggctg tttggctgac 40860
   tggcgggtgc caggggagtg gcaccaaatg gagtcccctg gctgctccct gggcagttgt 40920
   ggaaaataca acagggagag ctgaggtaag acagggcggg aggggcagga gagcagatgg 40980
   aggtggtggt ggtgatggat tggagtcatg gcatgacagg tgcctgctgg gttctctgag 41040
   gcagcccatt ttctcaggga ccacccagtt caggaggtct tgtctcccac caagagttct 41100
   gggcaaggtg acagcccagc ccttctccgt gtcccctgcg ctcaaagctg cgctgcggag 41160
   aggaaagcag ctcgtcccca ggtcaggctg tcctcctcct gctccagttt ggctttcatg 41220
   tctcactttt ccctcaagag atggaaaagc ctgtcctttc ctcaagcccg catttccatt 41280
   tatcagaaca tggcagggag gggcaggcag aggcaccatg gaggacagca agtcccatca 41340
   cactcaggag gctcctggcc tgtgcagcct ccagctatca gcactgtccc ttccccattt 41400
   agccgctgct tctcgggagg ccgccccagt gccctgagcc tggaggcact gttcatgggg 41460
   ccagccccgc tgcggggttc tcctgccctg cctcttcttc agagccaaaa agaaaaagca 41520
   cagagggcaa ggcccacatg gaaatcttac aaaacagctg agggccaaga gtcctggaaa 41580
   ttccaagcaa tcttgtctca ggggaagagg ctgagtccct gtcattttaa gaaagcctcc 41640
   tgtctgcaca tcaaagtctc gtctctatct tgggacccta tcgaccacac tgggctgctg 41700
   tgaatgtaaa aggagggtcc agatggaaag aggctttatt gacaggacgc ggcagttcat 41760
   gcctgtaatc ccaatacttt cagaggccaa ggcgagcaga ttacttgagg tcaggagttc 41820
   gagaccaccc ttgccaacat ggtgaaaccc tgtctctact aaaaatacaa aaattagcca 41880
   ggcatggtgg caggtgcttg taatcccagc tacttcggag gctgaagcag gagaatcact 41940
   tgaacccggg aagtggaggt tgcagtgagc taggatcaca ccattgcact ccagcctggg 42000
   caataagagc gaaactccgt ctcaaaaaaa aaaaaaaaag ggagatctgg ccaggtgtgg 42060
   tggctcatgc ctgtaatcct agcactttgg gaggccgagg agggcggatc acgaggtcag 42120
   gagatcaaga ccaacctggc tgacacggtg aaaccccgtc tctactaaaa atacaaaaaa 42180
   aaaaaaaatt agccaggcat ggtggcgggt gcctgtagtt ccaactactc aggaggctga 42240
   ggcaggagaa tggcgtgaac ccgggaggtg gagtttgcag tgagccgaga tcatatcact 42300
   gcactccagc ctgggcgaca gagtgagact ccgtctcaaa aaaaaaaaaa aaaaaaaagg 42360
   gagatcttcc acatgccagg cactatgcat aaataacagc gacccgggcc ctaaatgggt 42420
   gaagctgaga atctagtagg gaagacgaag tgcccagtgc ctacctgctg ccaggtcctg 42480
   aagccccgct ccctctcact gagtgtcggc acttgtaaga cagtgctctc ctttgccacc 42540
   tccttcctaa atgcctgggc tttctagatg aatgagtgct ggctgccagc ttcctcctgg 42600
   gcaaggctac aatgccactg tgcttacttg gtcatcaatc ctttctaatc cacaggatgc 42660
   aagacaggct agaaagccac ctgggtgggg ccaggcccag tggctcacac ctgtaatccc 42720
   agcactttgg gaggctgagg caagcagatc gcttgaggtc aggagtttaa gacaagcctg 42780
   gccaacatgg caaaaccctg tctctactaa aagcacaaaa ataagctggg cgtggtggtg 42840
   catacctgta atcccaacta cacgggaggc tgaggcacca gaatcacttg aacctggaag 42900
   aggaagattg cagtgagcca agattgagcc actgcactcc agcctgggcg acagggcaag 42960
   actgtttcca aaaaaaaaaa aaagccagca gcatggtgtt ggatcctcta ctgactctcc 43020
   cagccgaggc ctttccctgc ctgcctgcct ccaagtacag tatgtggaat ttccacgcaa 43080
   gcacggctcc ctttcctcgg atggctgact ctttgtgctg ctgtggtcct gcatgttcca 43140
   ggagcttcac ccagtgagga ccaggctggg agagaggctg caggccatgc tgtgcctccc 43200
   tgtggacact agggggcaga accatcccta gcccaggcag ggttggccct ttgcacagag 43260
   ggtgcattct cacaaaggag gcggtggccc aagaaacacc tgcactcctg tttgcttttc 43320
   atttcatgca cttttttttt ttttttttga gacagggtct cattccaatt gcccaggctg 43380
   gagtgcagtg gcatgatctc agctcactgc agcctcgacc ttcagggctc agctgattct 43440
   cccaccttag cctcctgagt agctgggact acaggcacgc accactatgg ccagctaatt 43500
   atttgtattt ttggtagaaa tgaagttttg ccatgttgcc caggctggtc tcaaactcct 43560
   gagctcaggt gatctgccca cctcagcctc cccaagtgat gggattacag gcatgagcca 43620
   ccacacccgg ccttcatgcg cttcttatac cacgcagcta gatttctacc ctgccttatc 43680
   acagtttgag ggagagaagc agttttgaaa acccctctct ccatactccc cctcagaaat 43740
   ataaaaataa aagcaggttt gtcagaagag acacccacct ctcaaaatct cctctctcag 43800
   ttcacaccca acagctaagt cacactcacg ggaacacctg catgatcatg gtgtagtggg 43860
   aagcggttct gccaattatc agcattagat ctgggccaag ttgagccagt ttcctgcaaa 43920
   gggaagctct ttccttcctc atggacataa tgcatgaagg cacccaacaa tggcaggatc 43980
   ctcggaaatg ttccttggct ctgggtttgg ctgtagattg actgtttttg ttttgtttga 44040
   gacagagtct tgctctgtca cccagagtgc agtggcgcaa tctcagctca ctgctacctc 44100
   agcctcccgg gttcaagcaa tcttcctgcc tcagcctccc cagtagctgg gactacagtc 44160
   acgcaccacc atgcctggct aatttttgta ttttggtaga gaggggtttc accatgtagg 44220
   ccaggctggt ttcaaacttc tgacctcaaa tgatgcaccc cactcagcct cccaaagtgc 44280
   cgggattaca ggtatgagcc actgcgccca gtgactgact gctttttgag acagccagat 44340
   agtggacaaa aggtttaatg cttgggacat ccgtggagct gagcccttcc ttctctctga 44400
   gtctgcccca aaaactgtct ttacggtgaa accctgtctc tactaaaaat acaaaaaatt 44460
   agccaggtgt ggtggcgggt gcctgtggcc ccagctactc aggaggctga ggcaggagaa 44520
   tagcgtgaac ccgagaggcg gagtttgcag tgagccgaga ttgtgccact acaccccagc 44580
   ctgggtgaca gagtgagact ccgtctaaaa aaaaaaaaaa acaaaaacaa aaacaaaaaa 44640
   aactctgtct ttagagcaat caattgtact gggaaggcat ttcagaaggg acgcatctat 44700
   aacaacttta tataggctgt ttcctcgagt gctcccattc ctaggagcac acctttctta 44760
   ctaaatcgaa tattcctgca ccaatgtaca attcctgcca acagcgcaca agaaggtcat 44820
   tttcccccac ataatactgg ccattatcaa tctaaaaatt ttgccaacat gatgtgcaaa 44880
   actatcttat cctagtttgc atttctctga ttaattaggt tgaggatatt ttcttttgtt 44940
   tttttgtttt tcgagacaaa gtctcgctct tgtcgcccag gctggagcgc aacagcacga 45000
   tgtctgctca ctgcaacctc cgcctcccgg gttcaagtga ttctcctgcc tcagcctccc 45060
   aagtagctgc tattacaggc gcctgccacc atgcccggct aatatttgta tttttagcag 45120
   agacagggtt tcaccatatc ggcaaggctg gtcttgaact cctgacctca ggtgatccac 45180
   ctgcctcagc ttcccaaact gctgggatta taggcatgag ccaccgcgcc tggcctattt 45240
   tcatattttt ttttctgggt gttacctgct tatatgtctt gtccattatt atattttcat 45300
   attcacagga attttaaaaa tactttatgg atactaattt ccatctgttt tccatgttgt 45360
   atcttctctc catctgtttc atacctttaa gctttgtttg tgtcatcttt tatcatacag 45420
   aatgtgacct atggccaggc atagtggccc atgcctataa tcccagaact ttgggaggcc 45480
   aaggcaggcg gatcacttga ggtcaggaga ttgagaccgg cctagccaac atggtgaaac 45540
   cccgtctcta ctaaaaatac aaaaattagc cgggcatggt ggcatgcgcc tgtaatcctg 45600
   ctactcagga ggctgaggca ggagaatcgc ttgaatgaga tcacgccatt gcactccagc 45660
   ctgggcagca gagtaagact ctgtctcaaa aaacacacaa acaacaaaac aacaacaaaa 45720
   accaaaatgt gacctatgtg atttgtcttt gttagaattt atggagattt tgtttcctat 45780
   agtttatttc ttacttgtct attagtcatg tgggaaagca tattatagtc tcctagtcct 45840
   ttggcttatt aaattctgat tgtgtctaat agtttttgct tcgtgaatct catgggtatg 45900
   cggtcagtta ttttaagtac atgattttga gaacctcttt gtgaatttta actttttttt 45960
   ttttttttga gacaagagtc ttactctgtc actcaggctg gagtgcagtg gcaggatctc 46020
   ggctcgctgc agcctctgtc tcccaggttc aagtgattct cctgcctcag tcccctgagt 46080
   agctgggatt acaggtgcct gccaccatgc ccagctaatt tttgtatttt gagtagagac 46140
   ggtgtttcac catgttggcc aggcttatgt caaactcctg acctcaggtg atctgcccac 46200
   ctgggcctcc caaagtgctg gcatcacaga tgtgagccac cacgcccagc caacttttat 46260
   caatataaaa tgtttcttct tatagccctc caaaacgata aaataaaaac aaaagcagct 46320
   aatcagctca cagtggcaca gtgagaaggc tgtgggcaga gcatgagtca agaagtctgg 46380
   gctctggtct cagcttggtc gctctgtggc ctgcatgact agggaaattg cttaactgcc 46440
   ttatctgtca aatggccaaa ttttaagggt tccttccagc cctcaaaact ctatgatcag 46500
   aggcacttgg gtaggctctg gggtggggat aataacttac caaagctgta actttcagtt 46560
   agcatccgac tggctggcag tgagggtgga gaagagaagg gtgggtagag gaagggagaa 46620
   agaaagagtg tatgtcactg gcttttcccc agacatctga gcctgacggc gagcactgca 46680
   gctgccctcc catgtgtcca ggatgtccaa tggcagaagg cacccaaagg ctatgaagac 46740
   tcaatgtgta gagcttagga aggagcagaa tgctttaaga aggcctgaag agtctcttgc 46800
   aatggctaaa taacatagtt tatttggttt tgtttgttcg ttcgtttgtt ttgagatggg 46860
   gtctcactct gtcacccaga ctggagtgca atggcacaat aatcactcac tacagcctca 46920
   aactcctggg ctcaagcgat cctcccacct cggcctccca aagtgttagg attgcaggcg 46980
   tgagccattg tggctggcag caacatagag tttacaggcc tttggtctta gagggatgga 47040
   tgaggacttc tggagactac cttgaccctt gaccctttgg gacagcgtac tatgcagaca 47100
   agagcacctt agagaaaaag tcacgtccat tttcaatttt tctaggactc aggtcttcca 47160
   tacttcctag tgaaaagttt cctagtgtat aacccacaca gctcctgctg caatcttagt 47220
   cattttcctt tcatgaaaaa gcagaacagt agagccccct ctccctgaca agaatgtgtc 47280
   agacacgctc atcctctcct ggagctgctt ctctttgtgc tttgctgctt tgagcagttc 47340
   tttaggatag tgaggatatg aatggtgatt tatagacatg aacaaatgga cacggttgga 47400
   agagaaagga agccagtggg gcagagttta cctgcaggag ttggctgggc ctggaacgag 47460
   gcttgaccag ctggagggag gcttccaaac acagaagagc tggggccact gctaaaggca 47520
   tcaaagttgg caaagcctcc ttgggaaggt gtctggcctg taggtggaag aaacataaag 47580
   gttatataga aaattgtcgg gtgtggtagc tcacgcctgt aattccagca ctttgggagg 47640
   ccgaggtggg cagatcactt gaggtcagaa gttcaagacc agcctggtca acatggtgaa 47700
   atctgtctct actaaaaata taaaaattag ctgggcgtgg tggtacatgc ctgtaatccc 47760
   agctaccttg gaggctgagg catgagaatt gcttaaacct gggaggtgga gctagcagtg 47820
   agccgagatt gtgccactct actctagcct gggtgacaga atgagactct gactcaaaaa 47880
   aaaaaaaaaa aaaaaaaaaa aaaccacaca cacacaacag aaaaagaaaa gaaaatgtat 47940
   atgcctatcc atgtaatata gctactaatt ctaaaggcca aaggccactc ttatgggaca 48000
   tctgcctttc cacagggttc tcttcagagg gaaatgggtg gggcctgggg ccaagagcca 48060
   gacccacaca ggggaacagg ggcacccagg gcctccaggt tcactccctg ccctcttctg 48120
   tccttactcc atgaggcatt aacatttttg tcagggccta gtttggagca tgaaaatttt 48180
   ttttcatgga ggaaggagct tgaatttttt tttttttttt tttttttttg agacggagtc 48240
   tcactctgtc acctaggctg gagtccagtg atgcaattgt gattcactgc aacctccacc 48300
   tcctaggttc aagcaattct cctgccttag cctcccaagt agctgggact acaggcacat 48360
   gccaccacac ttggctaatt ttttttttct ttttagtaga gatgaggttt caccatgttg 48420
   gccagactgg tcttgaactc ctgacctcag gtgatctgcc cacctcagcc tctcaaatgc 48480
   taggattaca ggtgtgagcc actgtgtctg gcttgaaaat ttttaaaaag ggaaaacaga 48540
   ttctatgctg ctgaaccctc tactggctat tgctgatttg ggggaagaat aatctttggt 48600
   gaactggaac acatgataat ccccctcctc ccattttaac agagacagaa aataataaaa 48660
   aaggctattt ccagtgttct aaaaggaagt gctaggagcc ctcaactata atgttttagg 48720
   atcaaacccc caaacataat cctggctgta cttatttgac cccagtcctc ccagcctcac 48780
   tgcttcagag tgaccctcac atttgtttgc atgagaagat ctagagccat ggttacctcc 48840
   agggactgga tctctcatgt tgtaagcata tacgctgtag ggagggtccc atcccaaaac 48900
   ccacttaccc ccaaaggcag ggaatgcagc aaaagctggt gccatctggg gtgcagcaaa 48960
   ggggtctcca ccgatgtcag ccagcaggtc agtactggct tttttgacag aggagtgggg 49020
   aggtggctga gtgctccggg cctgggatgt ccgagcgtga gactgactga cgggcttgga 49080
   ggagaaggaa tgcatacagc ccttagaatc ccagacatgc cccaggccct cctctggctc 49140
   cccaggttgc cctcagatag gcaggtagag agagaagaca gtgagtaact gccctcaacc 49200
   tagaagattc ctgggcagga gctgccttag cccctctcag agctcatatc atcctcatct 49260
   tctcagtaca cctgctccaa gaaccacaca ttttacagca catgagccaa ggtaaccctg 49320
   agatatacag gaatactgcc caatactgcc cccagggctg ttactcctca accttcccag 49380
   gtattggaca ccatattcca gccccttctg tgtttctttg atggaatgct caggtttgga 49440
   gctgatattc tgactttcct aaataccact tcctgttagg ttctgacttc caaaagatat 49500
   gaggtccaga agtgacaaat acaagataca ggttaacagc agagccgaga cccaactcac 49560
   agctcctgca ctgttgtctg ctaagtccgg ggaatctgat tcaggaaact gacactcctg 49620
   tctcccaaat ctcaaaagaa acatgacgag cagatctgag agttacctgg ctcgaggtgg 49680
   aggcagcaac tgagagagac ggtgcaggat cacccagaag tgtccgaagg ggcttccctt 49740
   ctgggatgga gccctgcaca ggggtggagg cactgccttt ggtataagtg ggccccttga 49800
   cttggtctgg ggggacatac ctttgagaaa gaatagttag gctgtaaaga gagggataaa 49860
   actttggacc acgctcagct gagatgtttg ggaagacaca ggagtcaaaa tctatgctca 49920
   tttttctcta ctgattccct tttttgaagg gaaccgagca acttagcaaa gttctgccta 49980
   tcttgagcat ccccttgaac atcttttggt ttttttgaga cagggtctcg ctgttgccca 50040
   gggtggagtg cagtggggca atgttggctc aacacagcct taactaccta ggctcaagtg 50100
   attctcctgc ctcagcctcc caagtagctg ggaccacaaa caagtacatg ctaccatgcc 50160
   tggctaattt tttaattatt tgtagagaag aggtctcact atgttgccca gggtggtctc 50220
   aacctcttgg cctcaggtga tcctcctgcc ttagcctcca aagttctggg attacaggca 50280
   tgagccacca cacctggcaa aggtccccct cttttatttc ctgggtgttt cttcagactc 50340
   cttcttctca gctccataga aaagctgcct gccgcctcat cctctaggtg ctcacaatcc 50400
   acacttccat cctgccagct cctgccggga caattccctt ccactacagt gtatgtggcc 50460
   cctactctgt aagtgtcagc ttaatcctga ggacatccct aggagggact ccacagtgtg 50520
   gtcttatcca catcaatctc tttgttctct gcattctctt ctctacctca cagttacctc 50580
   ccaacatgta cacaccccag agatcccaca agaagtgata ccaggggaga ggaaaggtaa 50640
   gggagaatgg agatgctgtt tccttcatca ggaagacaaa atatagttgt cccatggtat 50700
   cctcagggaa ctggttccag gatccccctg acaattccat aatcagagga cgggacactc 50760
   aagtctctta tataatatgg tgaagtattt gcatcaaata tgtatatcct ccaatatact 50820
   ttaaatcatc tctaggttac ttataatacc taatacaatg taaatgccat gcaatagttg 50880
   ctatattgta ttgcttttta tttgtattat tatttttttt ttgagacgga gtttcactct 50940
   gtcacccagg ctagagtaca gtggcacgat ctcagctgac tgcaaccact gcctcccggg 51000
   ttcaagcagt tctctgcctc agcctcctga gtagctcgga ttacaggtgc ccatgaccat 51060
   gcctggctaa ttttttgtat ttttagtaga gacggggttt caccatcttg gccaggctag 51120
   tcttgaacta gtcctgaccc tgtgatccac ccgcctcggt ctcccaaagt gctgggatta 51180
   caggcatgag ccaccacacc tggccttatt tgtattattt tttattgttg tattgttatt 51240
   ttttgtgttc cctacttttt tttttttttt tttttttttt tgagacagtc tctctctctt 51300
   tgcccaggct ggagtacagt ggcatgatct cagctcactg caacctccgc ctcctgggtt 51360
   caagcaattt tcctgcctca gcctccagag tagctgggat tacaagtgtc cacccccaca 51420
   cccagctaat tttttttgta tttttagtag ggacggggtt tcaccatgtt ggccaggcta 51480
   gtctcgaact cctgacctca agtggtctgc ccacctcagc ctcccaaagt gctgggatta 51540
   taggcgtgag ccaccttgcc tggcctattt tcccaaatat ttttgatgtg aggttgattg 51600
   gatccatggt tgcagaatct acagatacag agggctgact gtacatataa atcatgaaac 51660
   aaggccgggc acggtggctc acgcctgtaa tcccagcact ttgcgaggcc aaggcaggca 51720
   gatcatttga gcccaggagt ttcagaccag cctgggcaac gtggtgaaac ctcatttcta 51780
   caaaaaatat aaaaattaag gccgggcgca gtggctcacg cctgtaatcc cagcactttg 51840
   ggagccaagg tgggcggatc acgaggtcag gagatcgaga ccatcctggc taacacggtg 51900
   aaactccgtc tctactaaaa atacaaaaaa ttagctgggt gtggtggcgg gcgcctgtag 51960
   tcccacctac tcgggaggct gagacaggag aatggcgtga acctgggagg tggaacttgg 52020
   agtgagccga gatcacgcca ctgcactcca gcctgggtga cagagcgaga ctctgtctca 52080
   aaaaaaaaaa aaaaaagaaa tatatatata tatatatata tatatatata tatataaatt 52140
   agccaggcat ggtggcacac acctgtagtc ccagttactg gggaggatga ggtaggagga 52200
   tcacctgagc tccaagaagc tgagactgca gtaagctgtg actgtggcac tgcactctag 52260
   cctgggcaac agagtgagac cttgtctcaa aaaaaaaaaa aaatcataaa atgatgggtt 52320
   aaaggaataa aactatagga tgtgtgcttc cagccatctc tccccataca taagatcagc 52380
   tgagctataa aaatgaacac ccacaggcct acctcccgac tccaatcagt ctactatctc 52440
   tccaaaagag aggacataaa atctgcaggg ttagtgactg agaacaagtt gtcctttcat 52500
   gtagatttat tttagccgta actgaatgtg agtggaacag aatcgaccca agtctagtct 52560
   tttgtgagaa ggccaaattc aactctagaa actggactgt cagatgaaga atgtcttgaa 52620
   cacgtttcca tagcaacctc tttcctactc cttaccatct cttcttctca tatttttcct 52680
   ggagaaactc cttcactttc tgaggatccc tggaatctgg tactaaagat gtccgagcat 52740
   caaacagacc caaccaaatc ttccggcaaa cctaaaagaa tgaaggtgtc agaggagtgg 52800
   gagcagaaag caggaaatac aaaaaatacc acttcctttt tttttttttt tttttttttt 52860
   tgagacggag tctcgctctg tcgccgggct ggagtgcagt ggcacgatct cagctcactg 52920
   caacctccac ctccggggtt caagcaattc tcctgcctca gtctcccaag tagctgagac 52980
   tataggcatg cgccaccacg cccagctaat tttgtacttt ttaatagaga tgaggtttca 53040
   ccatgttggc caggatggcc tcgatctctt gacctcgtta tctgcccacc tcagctaccc 53100
   aaactgctgg gattacaggc atgagccacc gcgcccagcc accacttcct atttattcat 53160
   cagtaaatac atattggctg gatgtggtgg ctcacacctg taatctcagc actttgaaag 53220
   gccgaggtgg gtggatcact tgagtacggg agttcaagag cagtctgggc aacacagcga 53280
   aacccactct ctaaaaaaat acaaaaatta gtggagcatg gtggcatgtg cctatagttc 53340
   gagctacttg ggaggctgag gtgggaggat cgtttgagcc caggaggttg aggttgcagt 53400
   gagccaagat tgcaccatta agctccagcc tgggtgacag agcaagcaag agcttgtctc 53460
   tgataaataa ataaataaat aaataaataa acaaatattg agcatggaca cagtgctagg 53520
   atctgggtct acctacaaac cagatatgat tcctgtcctc gcagagttta taatccaatg 53580
   gggcagtcat atcttagata aataaaacta aagttataat cacattttgt gacatgtatt 53640
   gaaaaaggaa aatagagggc catgagaatt atggagaatt aaatttcact aagggtaggg 53700
   tccaggacaa ttttcagagc tctggcttcc aggacacctc cagaggccct tgaggatacc 53760
   ctctaagtaa accaatcacc cataagctgg ggtactgcta acgccaagga tagcaacttc 53820
   tgatcggcag atttagctag ggaaggggtt taagtcactt tccagtacaa gatatgcact 53880
   taaatccagg ttgtcgaggc tgaaggcagc agagctggct ccaggaaagc ttcctcagag 53940
   gggctggccc agtcaactct gaatgctcac gctctggagc tctggtcatt ctacccctcc 54000
   attgctcagg tgccacatcc tacaacgata ttgctacttc ccctgactcc tgaaaaggag 54060
   gcctagacaa accatgattt gtataacaga ataaagattc tgtaaatata cctaaggaaa 54120
   gaaatcatac atatgaaaaa attaagagaa actcaaccaa aaataaaacc gtaaggtttc 54180
   cctacaatct tgaggcaaag ccccacctgc agataggtga ccttctcacc actccatcgg 54240
   tggcagctca cctcatttcc acgggattgc aggaatacta cttcaggctc agtgaaagtt 54300
   gtcatggaga ttgacttgac acgatgaggg gggttcagcc ctctcctatg ggagagaaac 54360
   aggaagaaag agaagtataa tgcagttagc atggcaggct gttcatggga ggaggaggag 54420
   gagggagaaa gggtgaaaga atagcatata aactaagctc agatagaacc cccccatctg 54480
   aagctacttc ctaattccag gaaagaaggg aaatatttgg aatattttgg agatattaga 54540
   agtctccaaa gagaggccag gcacagtgac tcacacctgt aatcccagca ctttagaagg 54600
   ccgaggcagg cagctcactt gaggccagga gttcgagacc agcctggcca atatggtgaa 54660
   aacctgtctc tactaaaaat acaaaaatta gccgggcatg gtagtgcacg cctgtaatct 54720
   cagctactca ggaggctaag gcacaagtat cgcttgaacc tgggaggcag aggctacagt 54780
   gagccaaaat caccccactg cactccatcc tgggcaacag caagactctg tccccactcc 54840
   caccaccccc ctccaacccc caaaaaaaag aatctccaaa gagacaggga agagagagtc 54900
   aggaacatcc tgtgatccca gtagttctgg agaggaaagg tagagcctgc ccatccagaa 54960
   gcctcctcta gcctggctca gatcccagta gtttcagtgt tcagcctgta ggccgagggg 55020
   ggtacttctg ggaactcaga cacgtgacca cttcccactt cctactttat cattgtttta 55080
   gacctacagc ccatccccaa ggtcccaggg cccacaaaaa cacaaatcct tcaagcatga 55140
   gggaaggcaa aaatgaaggc ccagtactat tcttgataac actaacaaaa acaggatatg 55200
   gtgacaaaaa acagtctcta gcccagctgg tcctccttgt gtctacaaag aagggtggag 55260
   tggtggtggg atggagaggg tccctctctt tcaggaagcc atctctagga aaagaaatat 55320
   ttgattttct tatttctgta tctctcaata aagcaggaag aagtcaagtt caaccatgag 55380
   ctataaggct ggcatgggcg aggaatatgt aacctgtggc aatgcttttg tgcctcctct 55440
   gtatgttctg ttaactccct gggaatccag ttctgcctcc aggaaccctg tggagattat 55500
   aagagaagag aaggcttcct tgtccccact caactggtct caggagtgtt ctaatacagg 55560
   aaacttgggg ctaagccagg cagggcttca taggttcccc tgtccttgca ccttaagtat 55620
   tattctggtc taggtctact accctcactg gattaaacaa aactgcccaa accaaaacaa 55680
   gctagatcct gggacaggag ggaagcagtc tcagcagatg aggcctcttc agacttttac 55740
   tctcatggaa aaaaaaaaaa tggccaaatc gagttgctca aataaaaatc cagaataggg 55800
   tcctggctga catcctcagg aagcgcaaag gggaaaaaag agacaaaaat atttaacaga 55860
   tgaaaatcct gaaaattcac aaagaaggtg gggaaaacat acattcacac taacaggtgc 55920
   acacacagaa tccaagtgct cctgccaagt ctgggagaac agcagcgagg tgtaagcaaa 55980
   agcagcggtg gtgggggtgg ggggcaggga gtgggcggac tcagtacaga gtgaacagta 56040
   agggcggcca ccctgcggat gataaattac tcattatggg attttaacaa aggacaggac 56100
   actggaggct ccagttgacg ctgtttgcag caattctgct gctagacctt taggtataaa 56160
   gggtctcaaa aatgctctta taaaagacaa gcaatatcaa agaggactga tgcccccaag 56220
   gaaagataag ctcagggaaa aacgaaagca aggctttgtt actacatatc aaacaatagc 56280
   tcttaagctt ggtctgcccc gaagactgtc ttccgatcac atttcagaga tagtccattc 56340
   tctctcctaa ggcctctctt gctaatggct tggtttggtt ttgcttttaa ttctagccag 56400
   cctgctttta gactttctca ccttaaccac taggcccttc cccttcagaa tgtcagggct 56460
   ttctgaagtc tagccagacc tcattgtccc tgtgatctca aaggtcttct ccttcacctc 56520
   ctacaaacaa aatttacttt gccaaaacac aaggcattat gggaggcact gtgggggata 56580
   caaaaattat aggacagagt tatcttatca ccaggagctc acaagaagaa cccaggaagc 56640
   ctggacaaag gtacttctta ctgcagtagg gtgctattca gtcaggcact gatggatcag 56700
   ggagaacgtg actccaggat gcagcagttg cggaaggaag agaaacagtc atggggaagg 56760
   taaaatgtat tatgtttaca ctagaaaaac agattctaag cctatcacaa aaaggtcatt 56820
   gacatttcct gaatggataa ccaggaactg ttagcgaact gtaaacaggg aggtatgctt 56880
   ccttgttagg gggaggaggc aggactcaac ccaggcatca atcaaaatgg gatgcttaga 56940
   acagaactta tggttgagcc tggactaaaa gagtttattt ttggtggggg tggcgggcgg 57000
   ggggggcggc agacgtgaca ggatgatggg aaggcagaga aaataagaaa tgatttaggt 57060
   gctttgggcc aaatgaatct gatggaacag gatgtttcca tagcacagta caaaaagcat 57120
   tgctctgacc ttgatgtctg tagactggat tttgtacaac cagagccaca gtctgggggc 57180
   agggagaatt ctctacaagg caacactgca gcctaggaag ggggcctagt tacatggcct 57240
   atgagtcaga gtttttcttt ctgaatcaca gagaggccac tgctggccaa aagaagcaat 57300
   ttagaaacga gagccccctt cctcctggta gctaaaacca tgtggcctag gtgtgctgca 57360
   tttcactggg tacacaccag ggtcatgcag cggaccagat gtgagtcact ggcctggaat 57420
   taaacaggcc cagctctccc acttgcaccc agctccccaa cataactctc aggttcctcc 57480
   caaaagaggt aatcacagca aattcattct ggtatttcta accaggaagt gctggggagg 57540
   aacagagatc aatttatttt ctcagaatag agtaagtttt tccaatggaa agtacattat 57600
   tactaggact caagcctcca gccagtcagg ttgtctttat ccacttcagt ttctttttca 57660
   cagggcacct aaagggcata tgaagtattc tgaattattt ggcagctgaa gccacttgta 57720
   tgtgtagctg ctaacaactg cccagtcctc tctctttccc tccctcccgt ctcctctctg 57780
   attccattag gcatagtact atttccacaa ataccacacc ttttgttttt ctgtgatcat 57840
   caccctcatg ctggcccata tttttgcttc tatcttgctt cataaaagca tcaaacctgc 57900
   tcagctgctc agataagaaa gttctgccag gtcaacaatg gaaaaaaacc agttccaccc 57960
   agcacccaag ttctttttca tttgtgccaa atttggactt gggaaatcct tgcctttgaa 58020
   caattgtgtt cagattcata caccccagat cgtgtttctt tgctctggga tttcactcct 58080
   ctgcttctga acaggataca actaaaccag tttagctctt atcttggatg caaacatcac 58140
   catgaagagt ccctgttagg aacttttaat agtccctaag aggaaggctc tcctattacc 58200
   ctgctccagg gtcaatttca ctaattttag gaaccctctt cctgatctaa attctgcttc 58260
   aacccatttc ctttgttcct cctcagcagc aatgataaac agctgtcctg ttgattagag 58320
   ccaggtagac tcctgatagc atccagccgc ccttcccttt aggctcaacc tcaatctttt 58380
   ttacatgtct ccctctgccc tcacctctct catccccacc cccccgccca ccaccccacg 58440
   tcgagctttt tctcatccct ttagtaattt atcacttcct tcggggcttt tcaagtggtt 58500
   catgcacccc ttatgaaaca gcacctacat ctgtaattta gtgagagact ctgtccacct 58560
   ctgcccagca gaggtcaaca tgaaaggtga cagaggaggg gatggaggga tgggactaaa 58620
   acaggggcaa ggtacatgtg tacctctcca acccagacag atggaaaagt actgaattat 58680
   tgaagaacat actgtacctt tcctcccatg gagattaaaa tgcttcaact aagaaccacc 58740
   agcaagcccg acagggaaag taaggcacaa ggagggcagg aacttgtcac aaataaagag 58800
   ctgtcaagtg cattcttgac acccagccca gagctctgcc caccttaatt ctgactcctg 58860
   tgatcacagt aaagttgggc agaaatagaa gagataagtg tctattacaa acacctgaag 58920
   aacgatggat tgaagatgga cccaaacagc cacatcaagg aaatatgtca tactttgctg 58980
   aaaaaactgg gtgtcaaagt tagcgtccat ccttgtgtca atgagatctc aggcctcaac 59040
   atccattttc tttctgagga acagtggttc attcacagac gcattcaggc aaaccagaac 59100
   aagaaagaac atttaaagca ctgctttgca aatagatgct ttttagcagc aatttttacc 59160
   acgtcttaag attcaaatga cagattcatc ctccaagaat tagaaatgta actaaacaaa 59220
   caagcatatg tgtatgttgg ggaaattaaa cagatccaca tctaggttaa ggaaattagg 59280
   tactggaaga aaaccaaatg aataaaacta ggtcaggtgt gggacctaca attgtggcag 59340
   tatgtatgta aatttaggaa cagcagaaac tagcagcctt tctctcaacc cctcacacca 59400
   acgctgtagg tttttgctac agctgagaaa atatttctca ttaaaaaaga gagaacaaga 59460
   atgagttcat acatgcaggg atgcaggcag agtcataccc acccaccaat attaaaatgt 59520
   gtgtttttct tcagatctta ggaaattcaa ctttggctgg aaccatcaac tttgctacat 59580
   cggccaatcc aaccagctgg gaattctagt aattttgctc actgccccca gacgactcta 59640
   tccccttatg aaggagacat atttgtaacc cactgaacag aaaactggcc tttaaaataa 59700
   tgtcatcctg ctgtctcctt tctgtggcta cggtgctaat gtggtatttc aatttcttcc 59760
   ctcaatatcc tcttcatttt aacaaagaat atttcttctc gaggccaggc acagtggctc 59820
   acgcctgtaa tcccagcact ttgggaggcc aaggcaggtg gatcacctga ggtcaggagt 59880
   ttgagaccag cctggccaac atggtgaaac cccgtctcta ctaaaaatac aaaaattagc 59940
   tgggcgtggt ggtgtgcaca tgtaatccca gctacctggg gaggctgagg caggagaatc 60000
   gcttgaaacc aggaggtgga ggttgcagtg agccaagatc ggaccactgc actccagcct 60060
   gggcgacaga gcgagactcc atctcaaaaa aaaaaattaa taataatatt tcttctcttt 60120
   gcatcatacc tcagtttggt atcactctaa cccacagagc ttcattttac ttcatagctt 60180
   ttagcaatac agaacgattt ctttatagtc catggcttcc actaggatgt cagctccgtg 60240
   aaaatgggaa cttatcacta ctgtatctcc agtacctaaa acagtgtctg gcgcttgata 60300
   ggcactgaat aagttcgtgt tgactgaccg aatgaataaa catgcaggct gtcagccgac 60360
   cacaattttc acttcccacc aacaccaaac cctaatctac aggtaatcaa tcatggggat 60420
   ttaagatgct aattggtctc cgtgccacca tggagccatg cctccagaaa taagcatccc 60480
   ctgcctcaca ttctgcctta cagcccacct gccagctccc actccttccc acagcatatc 60540
   aaagggacac aggtaatact gtgttaagac ccctttacaa ggattatatg ccttgccctt 60600
   gtcatgctaa ttatagggaa actccagctt tcttgcagag tttctcctga aatttctcac 60660
   tctcaacata ccttcaattc agcttcattt actggcctaa cctcttgatc tttcatttca 60720
   ctctcatttc ctactaacaa agggttggtc tagcttctca tttcccaaac accaaaatat 60780
   gctctctaaa tcctcctctg tgagaatcac tccctggttt agtctcataa tgctttacca 60840
   aaaagctaag caggctgggt gccgtggctc acgcctgtaa tcccagcact ttgggaggcg 60900
   aggcgggcgg atcacttgag atcaggagtt cgagaccagc ctgaccaaca tggcaaaacc 60960
   ccgtctctac taaaaataca aacattagcc agatgtggta gcgcgtgcct gtagtctcag 61020
   ctactcagga ggctgaggca agagaattgc ttaaacccag gaggcagggg ttgcaatgag 61080
   ccgagatcgc gccattgcac tccagcctaa gcaacagagt gagactccat ctcaaaaaaa 61140
   gaaaaaagaa accaggtttt taacgtgtcc cagatacctt agctttcttt gtgtcctgat 61200
   tgaggggtat tataagggaa atgttatctg tgggacactt taacaaagat ctacaaaata 61260
   atgataacaa tgtcagcact tcccagttac tcaattttat ccatcagttt catccttctg 61320
   cttctcatag caggatcttc aggaatcagc tctttctctt tctactttac cctatacata 61380
   ctcctttttt tttttttttt gtgagacaga gtctcgctct gcgacccaga ctggagagtg 61440
   cagtggtgcg atctcggctc attgcaacct ccacctcttg ggttcaagca attctcttgc 61500
   ctcagcctcc taagtagctg ggattacagg cacctgccac catgcctggc taatttttgt 61560
   atttttagta agagatgaga tttcgccatg ttggccaggc tggtctcgaa actcctgacc 61620
   tcaagtgatc tgctcacctt ggcctcctat acatactgag ccaccgagcc cgacccctat 61680
   acatactcct aaacgttgct caatctctcc ccagagttct tcacccactc tcgagtctct 61740
   atcctcttgc ttcatttcag tccatctcag tttatgacac attactgttt gggttctcaa 61800
   tcattatccc aagagccacg atgactttta cttagaccaa agtcaagttc atccttacaa 61860
   cccacaagtg aggttgatca gaacccagag agaagtttag caaaggaggt taaaacgaag 61920
   taaataattc caaacggtat cttgagaaaa ctaaatcctc attgctggtc tgccatgatc 61980
   atcactaata tcaaacaaca aatgttggaa accgcaatgc ccatatggca ctggataagt 62040
   tacgaaacct ctctttgctt ctgttctccc atctgtcttg caagaacaat agtgctcgct 62100
   ccctggctcc taaaggtatc aagacaggga aagctttttt tttttttttt ttttttgaga 62160
   cagtgtcttg ctctgtcgcc aggctgaagt gcagtggcgc aatctcagct cactgcaacc 62220
   tccacctccc gggttcaagt gattctcctg cctcagcctc ccaagtagct gggagtacag 62280
   gtgcgcgcca ccacacccag gtaatttttg tatttttagt agagatgggg tttcaccatg 62340
   ttggccagga tggtctcgat ctcttgacct catgatctgc ctgcctcggc ctcccaaagt 62400
   gctaggatta caggcgtgag ccacagcgcc tggctggaaa gcatttttta aaaaacaaaa 62460
   caaaaacaac agtgtcttaa aataagacta aaaaattgtc aaaataaatc ccttgacaaa 62520
   cttattaaaa aacagagatc caggcctgat atcagatgtt aatcatgcat ctaaccaaat 62580
   aatatcttaa tttgggagta ggaggagtgc agaggggagt ggagggggct ttactatata 62640
   accagaaatc taggctagaa ttgtggctgt aaattcactc tggcatgacc gaacagtacc 62700
   aatgccaaga gtagcactca gatgatgcac caaagccccc ccaaaatcta gagtttcctg 62760
   gatgctacca ccaggatcca gtcatggcaa cagggatctt aaaggtgtaa atgattaacc 62820
   ttcaggatgg ctcacctaga cgtgcaaacg tttgaaaaag ctaagctctc taccagggta 62880
   acccattggc tctcatctct tagatttcgt aagagtttta ccttctgggt aacatttcaa 62940
   agccctgcta agattagtat ctacacctga actctggacc atggctccct tccagccact 63000
   atctctaggc tgtcaatcgg ggttaattcg cttttttttt tttttttaat cctgtactct 63060
   aatcatagga atttcaacca agtttcagct ggcttctgca tctcacactc cagcagggac 63120
   acaagcccga aggaagaaag gtgcccgtcc atctccaggc cggctttccc cacctttttc 63180
   ctttttgctt tccacctgca tgaagttccc tctaaaggtg catgtcattt tctggccctt 63240
   taagtcccga caccccatcc cactccgagt tcccttccat cctccgcact ttctccctcc 63300
   ccacttgcag tttgcctcga ccccctcggg gagtttctcg gtccctctcc gggcgtcttt 63360
   ctgcacccgt acccttctct ccgcagagcg actccggcct tgcttcctct cccctgccct 63420
   ttcccctccc caccacaccg ctgcagcaga cgaccccgtc cccacagccg gcgcgttccc 63480
   tgacctcccc tccgcccctc acctcggccc ccttccctcg cgccccggag ccccggcctc 63540
   cccaccccgc ccccgggtcc ccccacgccg acctcggcca ctccctcccg gtcactcaca 63600
   ggaggccgga gcaggtggtg cacacgaagc tgcccacggt gatatccacg taggtgaccc 63660
   cgcgctgggc gcactcgaag cagtggcggt tcccggcctg gctgcagcca cccagctccc 63720
   gcacccgacg gcaccacacc tccgaggccg cctccgcctc cgccttgccc ccgctgaccc 63780
   cgccgcccgg gcccgggccc ttcttcgccg ccatcaccat cgctgcccac tccctccccg 63840
   ctagtcagca atcacccctt caaccactgc cgccttcccg cctcctcagc cgcctccgca 63900
   cgcccggctc ccctgacaga agctcgggag cgggcggcat ccgcaggaga cttcgccgtc 63960
   caccaccctc cttgcccgtc ctgcacactg attggctagg tttctcccac ctccttactc 64020
   cgattggccc tccctgggac ggcctccggt tgctggagcc gccaatgccc cgccccaccg 64080
   ccttcaggcc tcaggcctct cctattggcc ggcagagacc ccagcagcga gaaccgtgcg 64140
   tgccctctcc ttgtccccgc cccctctcgc cgctgcctcc accgtggccc gcctcctagt 64200
   tccgattggt gagtcctcgg cactcgggct ccgacgctaa ttttgattgg ctggcggggc 64260
   tgcaaagtta gcgcgcgaga cagaggaggt gccccgcgcg tgcgtagctg tcgtcctccc 64320
   gactgatgac gttaattccc ggcaaatttc aaaggcttac tctagactgt gtagagtgtc 64380
   atgcaaagcc gaagagaagt ggccatgtga ggggctgagc aggaggggga aagaagagag 64440
   cgcttaaggg agagagggag agggagcaag agtgctttac tagaccagtg ctctaattcc 64500
   tgagccacag agcgaaggga gtgctttata aatgtcattt cattcgctcc gcctatccca 64560
   gaggtggaat ccccatttta cacccttagg ttcaacaact tgcggcagat cacacgagtg 64620
   cgtggcccag gattccactc tgggtcttcc aactttagaa accctgcctt taaccctcct 64680
   agtaggtgga ctattattcg attattcatt taacaaacac tgagtgtctg ctaggtttca 64740
   ggcacgttct agacactggg aatagaatac agcagcgaag caatcaaaca aaatattctt 64800
   atagagctta gtttctagta gggggaagca aacaatacac aaattaaaga atcaccagtt 64860
   agtaataaat cctacgcaga gaattaaaat cacatcttgg aaaagacttt ttgggtactt 64920
   ttttaatttt ttaatttaat tttaattttt tgagatggag tctcactctg ttgcccagac 64980
   tggagtgcag tggcacgatg tcagctcact gcaacctctg cctcctgggt ttaagcaatt 65040
   ctcatgcctc agtctcctga gtagctggga ttacaggcgg ccaccaccac ccccagataa 65100
   tttttgtatt tttattatta tgattattat tatttgagat ggagtctcgc tctgtcgccc 65160
   aggctggagt gcagtggcac gatctcggct cactgcaagc ttcgccttct gggttcacgc 65220
   cattctcctg cctcagcctc ccgagtagct gggactacag gcacccgcca ccacgcctgg 65280
   ctaatttttt tgtattttca gtggagacgg ggtttcaccg tgttagcaag gatggtctcg 65340
   atcttctgac ctcgtgatcc gccggtcttg gcctcccaaa gtgctgggat tacaggcgtg 65400
   agccaccgcg cccggccaat ttttgtattt ttagtagaga caggatttca ccatgttggc 65460
   caggctggtc tcaaactcat ggcctcaagt gagcctcccg ccttggcgtc ccaaagtgct 65520
   gggattacaa gcatgagcca caatgcctgg ccacctgttg ggtacttcag attgggttgg 65580
   tcaccatttt taaaacaatt actttttttt tgagacaggg tctcgcttta tcacccaggc 65640
   tggagtgcag tgacacaatc atggctcact gcagcctcaa ccttctggac tcaagtgatc 65700
   cttccacctc agccttccaa gtagctggga gtacagctgt gcaccaccat gcctggcgaa 65760
   tttttaaaaa ttttttgtag agattgggtc tcactatatt gcccaggctg gtctggacct 65820
   cctgggctca agtgatccgc ccacctcagc ctccatctcc caggttcaag cgattctcct 65880
   gcctcagcct cccaagtagt tgggattaca ggtgcgtgcc accatgtcca gctaattttt 65940
   gtatttttag tagagatggg gtttcaccat attggccagg ctggtcttga agtcctgact 66000
   tcaaatgatc cacccacctt ggcctcccaa agtgctagga ttacagacgt gagtcaccgc 66060
   acccggcctc tttttgtttt gttttgtttt ttttgttttt tttttttttt ttgagacaag 66120
   gtcttgctct gctgcccagg ttggagggca gtggcacaat catacctcac tgcagcctca 66180
   acctccccag ttcaagtgat cctcccacct cagtctccag ggtagctgag actacaggcg 66240
   cacaccacca tgcctggcta atttttgatt ttttgtagag aaggggtctc actatgttgt 66300
   ccaggctggt ggctggcagc ctgctttctc tctctctctc ttgttttttt ctttttgaga 66360
   cagggtctta ctctgtcacc caggctggag tgcagtggca caatcttggc tcactgcaac 66420
   ctccacttcc tggggcttga gtgctcctcc cacctcagcc tcccgagtag ctgggactac 66480
   aggtgcatgc caccatgccc agctaatttt ttgtattttt agcatgttgg ccaggctggt 66540
   ctcgaacacc tgagttcagg tcatctgccc gctttggcct ctcaaagtgc tgggattata 66600
   ggtgtgagcc accgcacccg gccccatttt tcaattaatg gcaccatgag ccacctagtt 66660
   gcccaaagca aaaatctaga gctactctct tcaatacggc agccactaat catatgtggc 66720
   tatttaagtt taaattaaat taaaatgggc tgggcgtggt ggcttatgcc tgtaatccca 66780
   gcattttggg aggccgaggc aggcggatca cgaggtcagg agttcgagaa aagcctggcc 66840
   aacatagtga aaccctttaa tctactaaaa atacaaaaat tagccaggca tggtggcagg 66900
   cacctgtaat cccagctact tggaaggctg agtcaggaga attgcttgaa cgtgggaggt 66960
   ggaggttgca gtgagctgag atcgcgccat tgcactccac cctgggtgac agagcgagac 67020
   tgtctcaaaa aaaaaaaaag aaagaaagaa agaagaagga aagaaaaaga aaaggaaagg 67080
   aaaaggggag actaaaggag gagtaggtta gggcagggcc taacctactg ggccatggac 67140
   cagtacctgt ctgtggcctg ttaggaacca ggccacgcag caggaagtaa gtggtgggca 67200
   aatgagcaaa gctgcatctg tatttacagc cactacccat tgctcccatt accgcctgag 67260
   ctctgcctcc tctcagatcc gcaacagcat tagattctca taggagcacg aaccctattg 67320
   taaactgcgc atgcaaggga tctaggtcat gcactcctta taagaatcta atgcttgatg 67380
   acctgtcact gtctcccatc acccccagat gggaccgtct agttgcgaga aaacaagctc 67440
   agggctccca ctaattccac atgatggtga gttgtataat tattttgtta tacattacaa 67500
   tgtaataata atagaaataa agtgcacagg ccaggcgcgg tggctcacgc ctctaatccc 67560
   agcactttgg gagctgaggt aggcagatca cgaggtcagg aaatcgagac catcctggcc 67620
   aacatggtga aacctcgtct ctactaaaaa tacaaaactt agctggccat ggtggcacgt 67680
   gcctgtaatc ccagctactt gggaggctga ggcaggagaa tcgcttgaac ccaggaggtg 67740
   gaggttgcag tgagccaaga tcgtgccact gcactccagc ctggtgacag agcgagactc 67800
   cgtttaaaaa aaaaaaagtg cacaataaac ttaatgtgct cagatcatcc caaaatcatc 67860
   ccccaactcc cacccgccgg ttagtggaaa aattatcttc ctggtccgtg gtgccaaaac 67920
   ggttggggac tgctgggtta gggggtgctg ataggcagaa atcagtagct tggttttgga 67980
   catgttgatt tgagatcctt gtgaggcatc cagtgggctg ataagcaggc aggtggatat 68040
   gtatagactg gatttccagg ccaggccaga gctggaaatg taggtttagg agtcatcgct 68100
   tagagccatg ggactggttg cttaggtaca gcatgtagag agagactaga agcaggccca 68160
   gaagtaaacc ctaggcaccc aatgtgtact ttgttccttt tctccagtta tcacagccac 68220
   atgcatttca acatccctaa aaaaaggcaa caccagaccg ggtgcagtgg ctcatgcctg 68280
   taatcccagc actttgggag gccgaggggg gtggatcacc tgaggtcagg aatttgagac 68340
   cagcctggct aacatggtga aaccccgttt ctactaaaaa atacacaaaa ttagctgggc 68400
   atggtggcgc atgcctgtaa tcccagctac tcaggaggct gaggcagaat cgcctgaacc 68460
   cagaagttgg aggttgcagt gagctgagat cgcaccattg cactccagct tgggcaacaa 68520
   gagcaaaact ccatctcaaa aaaaaaaaaa tacaaaaatc agctcggtgt gatggcgcat 68580
   gcccgtaatc ccagctactc aggaggctga ggcacaagaa ttgcttgaac ctggaaggca 68640
   gaggttgcag tgagccaaga ttgcatagct ccagcccagg tgacagagcg ggactctatc 68700
   cccctcaccc ccctcttcaa aaaaaaagaa ggcaataccg tcacatccag atatgcagat 68760
   ggggcatctc agcctactaa acattccttc tgaattgcag agttcacaat ttccaagtgc 68820
   tgcccatgcc tcctctagct tctcattagc caagggttat ttcttttttc ttttcttttc 68880
   ttttcttttt gagatggagt ctcactctgt tgcccaggtt ggagtgcggt ggcacaatct 68940
   cggctcactg caacctccgc ctcctgggtt caagcgattc tcctgcctca gcctcatgag 69000
   tagctgggac cacaggcgca tgccaccatg cccggctaat tttttgtact tttagttgag 69060
   acaaggtttc accatgttag ctaggatggt ctcgatctgc tgatctcatg atcagcccac 69120
   ctcggcctcc caaagttctg ctatacaggc gtgagccacc gtgctcagca tttttttttt 69180
   ttttgagata gagtttcact cttgttgcct aggttgagta caatggtgcg atctcgactc 69240
   actgcagcct ccgcctccca ggttcaagaa attctcttgc ctcagcctcc ctagtagctg 69300
   ggattacagg cgcccaccac cacacccggc taattttttg tatttttagt agagacgggg 69360
   tttcaccatg ttggccaggc tggtcacgaa ctcctggcct caggtgatcc acccgcctca 69420
   gcctcccaaa gtgctggaat tacaggcgtg agccaccgtg cccagctgag ccaagggtta 69480
   tttcttatca tcttgcaact ttgagcaaat tggtaaattt aactaccacc aatagtgagt 69540
   gaaaaaaatt agaacgttca tgtagatagt gatggttttc tgctgatttt tgccactggg 69600
   tgtggtggtg tgagggccgt tgaggagctc accctcctcc catctctact ccacccaggt 69660
   catactgggg agcaacactc ccattttccc ttcataatca gggggagcac agccagcatg 69720
   gggagacctt ttgccctggt ggcaggagga gtcctaaata accaggtaga actcttattg 69780
   ggttccctgg caagaacctt gcccttgggc agttaaacca cagagcatga gtgacttggg 69840
   gcgtggaaag caaatatctt tttttttttc tttgagacgg agttttgctc ttgttgccca 69900
   ggctggagtg cagtggcgcg atctcagttc actgaaacct cccgggttca tgcaattctc 69960
   ctgcctcagc ctcccgagta gctgggatta taggcgccca ccaccatgcc tggctaattt 70020
   ttgtattttt ggtagagacg gggtttcacc atgttggcga gactggtctc aaactcctga 70080
   cctcaagtga tccagctgct tccacctccc aaagcgctgg gattacaggc atgagccacc 70140
   gtgcccggcc agcaaaaaaa ttttagagag aaaaataata gtgacagcta ctccccactg 70200
   tcccccttaa ctgcaaccct tggtttctgg acctctctat tgactgagga agactgggcg 70260
   ctgcaattca gtcactgtga ggatcacata tcactatcag cgagggagcg ttgcagcctc 70320
   acaagtgtgg tctcgtggat actggctaga tttgtttttg gtacaccagt tcattgttct 70380
   acaaggccag ctgtttctgg tacatgggaa ataggataat ggggcccatg gctggccaac 70440
   tgtcttgttt ttcttttctt tctttctttc tttttttttt tttttttttt tgacacaggg 70500
   tcttgctctg ttgcccagcc tgcaatgcag tggcaccgtc atagctcatt gcagcctcta 70560
   attcttcggt tcaagcgatc ctcttacctc agcctcccca ggaggtggga ctacaggcgc 70620
   acaccaccat gcctggctag ctttttattt ttttgtaaag agagagtttc accatgtctc 70680
   ccagactggt ctcgaactcc tgtgctcaag caatctgctc accttggcct ctcaaagtgc 70740
   tgggattaat tataggcgtg agccactgtg gccagcccat ggtaacttgg ttttgtttgt 70800
   ttgttttgtg tgtgtgtgta tgtgtgtgtg tgtgtgtgtg tgtattgaga tggagtctta 70860
   ctctgtcact caggctggaa tgcagtggca tgatttcagc tcactgcaac ctccgcctca 70920
   tgggttcaag cgattctccc tcctcagccc cccaagtagc tgggattaca ggcacctgtc 70980
   accacgccca gctaattttt ctatttttag tagagacggg tttcaccatg ttggccaggc 71040
   tggtctcaaa ctcccgacct caagtaatcc acccgcctcg gcctcccaaa ttgctgagac 71100
   tacaggcata agccatgtgc ccatcctatg gtaactttgt atcccagata tttacactaa 71160
   atttcaggaa caagttagga gatgttcccc ggaagaagaa taattatctg ctgaaatgtg 71220
   gtcttgtttc aacaccccag aggcctccag ggagattgtc tactgggcat ggcaaaggtt 71280
   gagtgggcta gacccctctg gcaccactgg attagccaag ccataagggc atcttgcttt 71340
   gtaacctgga ctaatttaga aacttcctct ttcacacagg tgcggtggct catgcctgta 71400
   atcccagcat tttgggaagc cgaggcgggc ggatcacgag gtcaggagtt caagaccagc 71460
   ctggccaacc aacatggtga aaccccgtct ctactaaaaa tataaaaatt agccaggcgt 71520
   ggtggcacac acctgtaatt ccagctactc aggaggctga ggcacgagaa tcacttgaat 71580
   cagtgaggtg gaggttgcag tgagccaaga tcatttcact acactccagc ctgggtgaca 71640
   aagagagact ctgtctcaaa aaataaataa ataaataaat aaaaagtaaa aatgcccaag 71700
   tctatcctcc agaaatgaaa tctggaatgt ctgagatgaa aagtacagtg aatgggacta 71760
   acagcagact agatactaca gaagagaata ttatgaactt gaagacataa cagtagaaag 71820
   tataaaaatg atttatagag ctaggcacag tggtgtgcac ctataatccc agctacttgg 71880
   gaggctgagg ctggaagatc agttgagccc aggagtttga gaccagccta ggcaacatag 71940
   cgagaccctg tctcttattt tttaaaatat tggaaaaatg aaaaaagact gataaaaacc 72000
   caaaacccac agatgaacag aacaccagtg agctgtgaga caacttcagg tggccccctc 72060
   aggaattggt ggcaacaggt ataatacatc tggggtaaag gtagaggtta aaaataagta 72120
   tattgactga aaatcagttt tattttattt tattttattt tattttattt tattttattt 72180
   tatttttaaa aatttttttt gagatggagt tttggtcttg ttgccctggc tggcgttcaa 72240
   tggcacgatc tctgctctct gcaacatcca cctttggggt tcaagtgatt ctcctgcctc 72300
   agcctcccaa gtagctggga ttacaggcac ccgccaccac acccagctaa ttttgtattt 72360
   ttagtagaga cgaggtttcg ccatgttggt cagcctggtc tcaaactcct gacctcgtga 72420
   tccacctgcc tcagcctccc aaagtgctgg gattacaggc gtgagccact gtgccctgcc 72480
   taaatgtcag ttttaaaaga cattagagcc agatatggtg gtgtgcacct gtagtcacag 72540
   ctattcagga ggctgaggca gaaggattgc ttgagtccag aagtcctggg ttgtagtgtg 72600
   ctctgctgat tgggtgtctg ccctaagttc aacaacaatg tgctgacctt ccagaagcga 72660
   gggaccacca ggttgcctaa ggagggttga acctgcccag gttggaaatg gagcaagtca 72720
   aaactcctgt gctgatcagc agtggagtca cgcctgtgaa tagtcactgt actacggcct 72780
   gggcaacata gtgagacccg tctctaaatt aaaaaaaaaa aaagggcatt agactcacac 72840
   aaacgttggt tgcacttcta tatattaaat atgagcaatg tcaaaaggaa attacaaaaa 72900
   caattacatt ggccaggtgt ggtggctcac ctctgtaatc ccagcacttt gggaggccaa 72960
   ggcgggcaga tcacttgagg tcaggagttc gagaccagac tggccaacat ggggaaacct 73020
   cgactccact aaaaaaaaat acaaaaatta gttgggtgta gtggcccacg cctgtagtcc 73080
   cagctacttg ggaggctgat gcaggagaat ggcttgaacc tgggaggtgg aggctgcagt 73140
   gggcagagat cgtgccactg cactccagcc tgggtgacag agtgagactc catctcaaaa 73200
   aacaaacaaa caaacaaaca aaaaaacccc acaaaacata aaaacaatta catctacaat 73260
   agcatcacac agaataaaat acttagggat taatttaacc ctaattaaat taaggaggtg 73320
   aaagacttgt aaaatgaaaa ctatacaaca ttgctgaaag aaattaaaga agacaaaaat 73380
   ttatggaaac acatcccagt tcatgaattg gaagagttaa tattgttaag atgtgaatac 73440
   taaccaaagt gatctacaga tttagagcca ggctcagtgg ctcactcctg taatcccagc 73500
   actttgggag gagtaggtaa gcggatcacc tgaggtcagg agttcgagac caccctggcc 73560
   aacatggaga aaccccgtct ctactaaaaa tacaaaaatt agccaggcat ggtggcatgc 73620
   acctgtaatc ccacctactc agaaggctga ggcaggagaa tcacttgaac ccaggaggag 73680
   gaggttgcag tgagccgaga tcgcaccgct gcactccagc ctgggtgaca gagcgagact 73740
   ccgtctcaaa caaacaaaca aaaaccaaca aagtgatcta cagattcagt gcaatctcta 73800
   tcaaaattcc aatgatgtgt tctgcagaaa tagaaaagcc catgctaaaa tttctatgaa 73860
   atctcagcgg actccaaata gccaaaacac tattcaaaaa gactaacagg ctgggcatgg 73920
   tggctcacgc ctgtaatccc agcactctag gaggccgagg tgggcagatc acctgaggtc 73980
   aggagttcaa gaccagcctg gccatggtga aaccccatct ctactaaaaa tacacaaaat 74040
   taggcgggca tggtggtgca tgcctgtaat cccagctact cgggaggctg aggcaggaga 74100
   atcacttgaa cccaggaggc ggaggttgca gtgagccaag atcgtgccat tgcactccag 74160
   cctgggcaac aggagcgaaa ctccatctca aaaaaaaaaa agggactaac aaaattggag 74220
   gactcacact tcttgatttc aaaacttatt acaaagccat gtgatcaaaa cagcatggta 74280
   ctggcataaa gacagacata taggctcggg tgtggtggct catgcctgta atcccagcac 74340
   tttgcggggc cgaggtgggt ggatcatttg aggtcaggag ttcaagagca gcctggccaa 74400
   cgtgctgaaa cgccgtctct actaaaaaaa aaaaaaaaaa aaaaaaaaaa aaaatacaaa 74460
   cattagccag gtgtggtggc acgtgcttgt cttcccagct actcgggagg ctgaggcagg 74520
   agaatgactt gaaccaggga ggcggatgtt gcagtgagcc taaattgtgc cattgcactc 74580
   cagcctgggc gacagagcaa tactccatct caaaaaaacg aaacaaaaca aaacaaaaaa 74640
   aacctgtctt ttcaacaaat ggtattggga taactgcata tctacatgtg aaagaatgaa 74700
   gtggaactct tacctaacac catatacaaa aattaactca aaatggataa aagacctaaa 74760
   tgtcagacct aaaactaaga aagatttaga ataaaccata gggcaagagc ttcatgacat 74820
   tggattttgc aattacagtt gacccttgaa caaagcggga gttagggaca aaggacttga 74880
   acagacattt ctccaaagaa gatacacaaa tggccaataa gcacacaaaa agatgctcaa 74940
   catcattagt cattaggaaa tgcaatcaaa gccatggatg agataccact tcacatccac 75000
   taggatggct gtaatcaaaa aagggaaaac tagcaattgt tggtgaggat gtagagaaat 75060
   tgtaaccctt gtacattgct ggtggaaatg aaaaatggtg cagctgccat ggaaagcagt 75120
   ttaccagttc ttcaaatagt taaatataga ggccgggcac agtggctcac gcctgtaatc 75180
   ccagcacttt aggaggccaa ggcgggcgga ttgcctgagg tcaggagatt gagaccagtc 75240
   tggccaacat ggtgaaaccc catctctact aaaaatacaa aaaataaaaa ataaataaaa 75300
   ataaaaaatt agccgggtgt ggtggtgtac acctgtaatc ccagctactc gggaagctga 75360
   ggcaggggaa ttgcttgaac cagggagaca gaggatgcag tgagccgaga tcatgccact 75420
   gcactccagc ctgggtgaca gagtgagact ctgtctcaaa aaaaaataaa gttaaatata 75480
   gaattactat atgactcagc tgtttcactc ctagttatat accctaagga gtttaaaaca 75540
   gctattcaaa tcaaaacttg tacacgaaag ttcatagcag caccatccat gatagcctaa 75600
   aggtagaaat aatctaaatg tccattgaca gatgagtgga tagagaaatt gtggcatata 75660
   cagaacgggg catggtggct cacacctgta atctcagctt tggaggctga ggcaggagga 75720
   ttgcttgatc ccagtagttt gagacgagcc tcgacaacaa gtgagacccc atctctacaa 75780
   aaattttaac aaattaaaaa attagccaag tgtggtggca catgcctgta gtaccagcta 75840
   cttgggaggc tgaggtggga ggatcacaca gcccaggagc aagagcctgt cttaaaaaga 75900
   aaaacaaaaa caaagagaag caattggaaa gaaacagata ctatgtaaag agaagaaaat 75960
   tttacccaaa acccactata attaatatcc ttggagagat aagataccat tttcatgaaa 76020
   tatacacaga acgttatttt aaaaggaata ttcaggccgg gtgcagtggc tcccgcctgt 76080
   aatcccagca ctttgggagg ccgaggcagg tggatctcct gaggtcagga gttcgaaacc 76140
   agcctggtca acatggcgaa accccgtctc tactaaaaat acaaaaatta gccgagtgtg 76200
   atagtgggag cctgtaatcc cagctacttg ggaggctgag gcaggaggat tgcttgaact 76260
   cagcagacgg aggttgccgt gagcccagat catggcattg cactccagcc tgggtgacag 76320
   agcaagaatc cgtcaaaaaa aaaaaaaaaa aaaaaaaaaa acagaaaaaa aggaatattc 76380
   agagaacaag aaagaaatcc tggaaattaa atatataaac atgaaaacct cagttgaatg 76440
   gttggaagat aaaattggag aaatcctgca aaaagcagtt taaaatatga gatgaaaaaa 76500
   atgattaaag gtaaataaaa gaacagttca gtagatctaa tatataaata atagaagtta 76560
   cagaaggaga gaagagaaaa atggagggga aaaatattac tgaaaaattt agcaaaattt 76620
   tccagaatta aagggcatga gtttctagat taacaagacc ttagtcaggt gtggtggctc 76680
   acatatgtaa tcccaacact ttgggaggct gaggcaggtg gatcacctga ggtcaggagt 76740
   tccagaccag cctggccaac atggtgaaac ccggtcttta ctaaaagtat aaaaattagg 76800
   ccgggcgcgg tggctcacgc ctgtaatccc agcactttgg gaggccgagg cgggaggatc 76860
   acgaggtcag gagatcgaga ccatcctggc taacatggtg aaactccgtc tctactaaaa 76920
   atacaaaaaa ttagccgggt gcagtggcgg gcgcctgcta ctcgagaggc tgaggcagga 76980
   gaatggcttg aacccgggag gcggagcttt cagtgagccg agatagcgcc actgcagtcc 77040
   agcctgggca aaagagcgag actgtgtctt tttacttttt ttaaaaagaa gtataaaaat 77100
   tagcttgccg tggtggcgtg cacctgtggt cccagctact caggaggctg aggcaggaga 77160
   attgcttgaa cccaggaagg agaggttgca gtgagctgag attgcgccac tgcactccag 77220
   cctgtgtgac aagagtgaaa ccacctcaaa caaacaaaca aacaaacaaa aaaaaaaaca 77280
   aactaaaaaa caagacctat gaagtgccat ggtataactg ataaaaacca agaaccggct 77340
   gggcgcggtg gctcacgcct ataatcccag cactttggga ggccgaggcg gatggatcac 77400
   gaggtcagga attcgagacc agcctggcca acatggtgaa accccgtctc tactaaaaat 77460
   acaaaaatta gccggacatg gtggcgggcg cctgtagtgc cagctactcc agaggctgag 77520
   gcaggagaat ggcttgaacc tgggaagcag aggttgcagt gagccgagat cgcgccactg 77580
   cactccagcc tggcgacaga gcgagattct gtctcaaaaa agcaaacaaa aaacctaaga 77640
   acctcacgaa agcatatcct tgtgaaattt agaatggtgg agataaaaaa atatcttaca 77700
   agttcctaag agtacgaaca aatgacctat attaggattt ttaatagcaa ccctggaagg 77760
   tagaaggaca aggtgcattg cattcacaat tttataggaa aggccaggca tggtggccca 77820
   tgcctgtaat cccagcactt tgggagattg agataggagg accacttgag gctaggagtt 77880
   cgagaccagc ctgggcgaca ttttgagatt ctgactctac aaaacagaaa aaaaatttga 77940
   aggaaatcat tttcaacaag taattccata catggccaat ctacaactat gaattattct 78000
   agaataaaga tattttcagt aattcaaggt tcagatgcaa gtgacagaaa acctaaaaca 78060
   atagttacaa ttttctttct ctctgacata aaagtctagg tcagtggcca agcgtggtgg 78120
   ctgacgcctg taatcccagc actttgggag gctgaggagg gcagatcacg aggtcaggcg 78180
   atcgagacca tcctggctaa catggtgaaa ccccatctct actaaaaata caaaaaatta 78240
   gcagggcgtg gtggcacatg cgtatagtcc cagctactcg ggaggctgag gcagaagaat 78300
   cgcttgaacc caggaggcgg aggttgcagt gagccaagat caggccactg cactccagcc 78360
   tgggtgacag agtgagattc catttcaaaa aaaaaaaatc taggtcagta tagtggctct 78420
   gttccatgaa actctcagca acccaagttc attccagccc caactctgca atgcccaggg 78480
   tgtggccctc atcttcatgg ataggatggc agcatgtgag caccagacac tgggtgatag 78540
   aagagatgat gccctctccc tctccctctc tttccacggt ctccctctcc ctctccctct 78600
   ccctctcttt ccatggtctc cctctcatgc cgagccgaag ctggactgtg ctgctgccat 78660
   ctcggttcac tgcaacctcc ctgcctgatt ctcctgcctc agcctgccga gtgcctgcga 78720
   ttgcaggcgc gcgccgccat gcctgacggg tttttgtatt tttttggtgg agacggggtt 78780
   tcgttgtgtt ggccgggctg gtctccagct cctaaccgcg agtgatccgc cagcctcggc 78840
   ctcccgaggt gccgggattg cagacggagt ctcgttaact cagtgctcaa tggtgcccag 78900
   gctggagtgc agtggcgtga tctcggctac aacctccacc tcccagccgc ctgccttggc 78960
   cccccaaagt gccgagattg cagcctctgc ctggccgcta ccccgtctgg gaagtgaggt 79020
   gcgtctctgc ctggccgccc atcgtctggg atgtgaggag cccctctgcc tggctgccca 79080
   gtctggaaag tgaggagcgt ctctgcccgg ccgccatccc acctaggaag tgaggagcgc 79140
   ctcttcccgg ccaccatccc atctaggaag tgaggagcgt ctctgcccgg ccgcccatcg 79200
   tctgagatgt ggggagcgcc tctgccccgc cgccctgtct gggaggtgag gagcgtctct 79260
   gcccggccgc cccgtctgag aagtgaggag accctccgcc tggcaaccgc cccatctgag 79320
   aagtgaggag cccctccgcc cggctgccac cccgtctggg aagtgaggag cgtctccgcc 79380
   cggcagccac cccgtccgga agggaggtgg gggtcagccc ctgccaggcc agccgcccca 79440
   tccgggaggg aggtgggggg gtcagccccc cacccggcca gccaccccgt ccgggaggtg 79500
   aggggcgcct ctgcccggcc gcccctactg ggaagtgagg agcacctctg cccggccagc 79560
   cgccccgtcc aggagggagg tgggggagtc agccccccgc ccggccagcc gccccgtccg 79620
   ggagggaggt ggggggggtc agccccgtgc ccggccagcc gccccatccg ggagggaggt 79680
   gggggggtca gccccccgcc aggcgagatg ccccgtccgg gagggaggtg ggggggtcag 79740
   ccccccgcca ggtgagacgc cccgtccggg aaggaggcgg gggggtgggg gtcagatccc 79800
   cgcccggcca gccaccccgt ccgggaggga ggtggggggg tcagcccccc gcccggcgag 79860
   acgccccgtc cgggagggag gtggggggtc agccccctgc ccggccagct gccccgtccg 79920
   ggaggtgagg ggcgcctctg cccggccgcc cctactggga agtgaggagc ccctctgccc 79980
   ggccagccgc cccgtccggg agggaggtgg gggagtcagc cccccgcctg gccagccgcc 80040
   ccgtccggga gggaggtggg ggggtcagcc ccccgcccgg ccagccgccc cgtccgggag 80100
   ggaggtgggg gggtcagccc ccagccggcc agctgccccg tccgggaggt gaggggcgcc 80160
   tctgcccggc cgcccctact gggaagtgag gagcccctct gcccggccag ccgccccgtc 80220
   caggagggag gtgggggagt cagccccccg cccggccagc cgccccatcc gggagggagg 80280
   tgggggggtc agccccccgc ccagcgagac gccccgtccg ggagggaggt ggggggtcag 80340
   ccccccgccc ggccagccgc cccgtccggg aggtgagggg cgcctctgcc cggccgcccc 80400
   tactgggaag tgaggagccc ctctgcccgg ccaccacccc gtctgggagg tgtacccaac 80460
   agctcattga gaacggccca tgatgacaat ggcggttttg tggaatagaa aagggggaaa 80520
   cgtgggaaaa agattgagaa gtcggatggt tgctgtgtct gtgtagaaag aagtagacat 80580
   gggagacttt tcattttgtt ctgtactaag aaaaattctt ctgccttggg atcctgttga 80640
   tctgtgacct tacccccaac cctgtgctct ctgaaacatg tgctgtgtcc actcagggtt 80700
   aaatggatta agggtggtgc aagatgtgct ttgttaaaca gatgcttgaa ggcagcatgc 80760
   tcgttaagag tcatcaccac tccctaatct caagtaccca gggacacaca cactctgcct 80820
   aggaaaacca gagacctttg ttcacttgtt tatctgctga ccttccctcc actattgtcc 80880
   tatgaccctg ccaaatcccc ctctgcgaga aacacccaag aatgatcaat aaaaataaat 80940
   aaataaataa ataaaagaag agatgatgaa gaaggcacca ggggcatgtg ccagctattt 81000
   cttcagaaag ttcttccaaa gaactttcac ttctccttgc atcccattgg tcagaatttg 81060
   gtcacatggc cacaccaagc tacaaggtag ggtggaaaat gcagaaatat agactttatt 81120
   ttgaattatc tttttttttt ttcttttttg agacagcgtc tcactctgtt gctcaggctg 81180
   gagtgcagtg gcacaatcat ggttcactac agcctggacc tcctgggctc aagtgatcct 81240
   cccagctcag ctgctggagt agctgagact ataggcgcat gccaccatgc ccagctaatt 81300
   ttctcatttt ttgtagagac agagtctccc tatgttaccc aggctagtct tgaactcctg 81360
   ggctccagtg atcctactgc cttggcctcc caaagtgctg ggattacagg tgtaagccat 81420
   agtgcctggc ctttggatgg ttttatgcct agataaaaac tctattacta tggaagaatg 81480
   gaaggatact ggtggaatta gtcacattct ctgctacaca gtcttgaaca tttcctgaac 81540
   attctcagga agctatcagt agagtacacc acctacgtaa agcgataaag caagaaagaa 81600
   gacttgggat ccatgagtga gggtatccaa ttcaacaaag acgaagggag tccctagggt 81660
   aatagtgaga ggagacccca aaatgacagc tgagttgctg ccctagaaag cagctagtcc 81720
   aaagtagaga agatcagaag gtaggtttca cagagagcag gcaataagat ttttaaaaag 81780
   gtaaaaaaaa aagaaacaaa tcagaaggtt cttggaggga ttccttcaag aagatgaaac 81840
   tgactgaaga cctaataatt taccttcacc tagcaggtgc agcaatatgc cttcactgcc 81900
   ctgtgacaat ctactctgta tgtttgaaca taatgagaag acttatttta cagcagggat 81960
   tcaattagga ttaataccta gaaacatcgg caaaccaaaa gataaggcaa ttattaactc 82020
   caagggaaac aaatggctca attatgaata gcatatatac acttataaaa atataaccct 82080
   aggacaggcg cggtgggtca cacctgtaat cccagcactt tggggggccg aggcaggtgg 82140
   ctcgcctgag gccaggagtt tgagaccagg ctgaccaaca tagcgaaacc ccgtctctac 82200
   taaaaatacc aaaaaaaaaa aaaattagct gggcgtggca gtgggcgcct gtaatcccag 82260
   ctacttggga ggctgaggca ggagaatcgc ttgaaaccag gaggcggagg ttgcagtgag 82320
   ccgagatcgt accattgtac tccagcctgg gtgacaagag tgaaactccg tctctctctc 82380
   tctctctctc tctctctctc tctctctata tatatatata tatatacaca cacacacaca 82440
   tacttacaca catatacgta tatatatgtg tatacatata cacacatata catatatact 82500
   tatatatata tgtatacata tatgtgtgtg tatatatata cacacacata cataaaatcc 82560
   taaatctctt tctaactaaa atgacaaata cagagcctcc ctgacttatg atggaactgt 82620
   gtcctgatta attcattata aaatcaagaa aatccttaag tcaaaccatc ctagctccct 82680
   gacttatgat gggactgagt cctgattaac tcattgtaaa gttaagaaaa tcattaagtc 82740
   aaaccatcct aaattggggg actgtctgtg ttcagaggat ggtgggatgg gaagtgtgtg 82800
   tgtgtgtgtg tgtgtgtgtg tgactggtgt gtgtgtgtgt gactggtgtg tgtgtgtgtg 82860
   actggtgtgt gtgactggga agtgaaagag agctaaccct cactgtccac acggtaagtc 82920
   aatagataat aggtaatact gaaaaaaaaa accaagagct aaagacataa ctgtattatt 82980
   tagacatata aatataaacc aaataacaaa agatcagcta taagaactga aaaaatgctt 83040
   acttctgagg agctgaaaat ggaggagagt tgaaaaggat cttctctttt ttctaacatg 83100
   tctttggaag tatttaactc tttaaattac gaccacgtcc aaatttatta aaaataagac 83160
   acaaatttgg ctgggcgtgg tgactcatgc ctatacttcc agcactttgg ggggccaagg 83220
   caggaagatc gcttgaggcc aggggttaga cgccaggctg ggcaacacaa caagactcca 83280
   tcactacaaa aaataaaaag taaaataatt agccagtgtg gtagccagct actagggagg 83340
   ctgaggtggg aggattgttt gagcccagga gtttaaggct gcagtgagcc atgatcatgc 83400
   cactgcacta ctgcagcctg ggcaacagag tgagaccctg tttccaaaca aaacaaaaca 83460
   aaacaaaaca aaacccaacc taaaacataa gttttaaaaa atgaaaagat aggctggccg 83520
   cggtggctca tgcctgtatt cccagcactt tgagaggccg aggcgggcag atcacctgag 83580
   gttgggagtt caagaccagc ctgaccaaca tggagaaacc ccatctctac taaaaataca 83640
   aaattacccg ggcggtggtg gtgcatgcct gtaatctcag ctactcagga ggatgaggca 83700
   ggagaattgc ttgaacccag gaggcagagg ttgcagtgag ctgagatcgc gccattgcac 83760
   tctagcctgg gcaacaagag cgaaactcca tcttaaaaaa taaataaata tgaaaagata 83820
   aattaatcag attttctttc ttgggccttt taggtgagaa atgtggaacc gggtagaagg 83880
   agacaaacac taacaagtgg taggtggtgg ctaaatcaca ttgacacttg gagggagagg 83940
   gaacattcac caattccttt tttttttttt ttgagatgga gtctcgctct gtcgcccagg 84000
   ctggagagca gtggcacaat ctcgtctcac tgcaacctct gcctcccagg ttcaagcgat 84060
   tctcctgcct tagcctccct agtagttggg actataggcg tgtgccacca cacccagcta 84120
   atttttttgt atttttagta gagacggggt ttcaccgtgt tagccaggat ggtatccatc 84180
   tcctgacctt gtgatctgcc cgcctcagcc tcccaaagtg ttgggattac aggtgtgagc 84240
   caccgtgtct ggcccattcg ccaattccta ttgtgaagca cctagagttt ttcctggact 84300
   cagaacttcc ttgggtttca acctacatga attctgattc cactatggct tagttctaag 84360
   agatcaggtc tcccttatct atctctttct cttacttgag ctttgttgag tgaacttttt 84420
   tgtttcttgc agtgaaaaaa gccctgctaa aatagaggta gatgctgctt ccagaattcc 84480
   ttattagttg aaagtataaa atgtagcaag ttggcctgat gcagtggctc attcctgtaa 84540
   ttccagccct ttggtaggcc aagacaggat cacttgagct ccggtgtcca agaccagcat 84600
   gggcaacata gggagaccca catctctaca aataatttaa aaataatatg actgtgacaa 84660
   atgattggaa agaaaaaaaa tttaaaaatt aataaataat ttttaaggcc aggcgtggtg 84720
   gctcacacct gtaatcccag cactttggga ggctgaggca ggtggatcat gaggtcagga 84780
   gatcgagacc atcctgacta acacggtgaa accctgtctc tactaaaaat acaaaaaaat 84840
   tagctgggtg tggtggcggg tgcctatagt cccagcaact cgggaggctg aggcaggaga 84900
   atggcgtgaa cccaggaagt ggagcttgca gtgagccgag atcgcgccac tgcactccag 84960
   ccgggatgac agagcgagac tttgtctcaa acaaacaaac aaaaataata ataattttta 85020
   aaaagaaata aaaattagct gggtgtggtg gtgtgtacct gtggtcccag gtactccaca 85080
   ggctgaggtg ggaggctgag gtgggaggat cacttgagcc ctggaggttg aggcagcagt 85140
   gggccatgat catgccactg cactccagct tgggtgacag agcaagaccc tgtctcaaaa 85200
   aataaaataa ataaaatgca gcaatttgtc cttcattctc tgccctccca tcccccacat 85260
   tttgaaggag atcttatatt actaaggaat attcattgct aattattatt cattactaat 85320
   gaatattcat tacaacttac tattcattac taatgatttt ctaagctcta ctgatgtgac 85380
   aggtccttgc atttctgcca gttatatctc acaaccccat tacatccacg tctcagcaag 85440
   acctggggga tctttgatac ttcaagtcca aggcaaatca gcatgatatt gtcaatgacg 85500
   atcaggatga catcctacag aggagtcagt tagtgtgatc aaccatccca gtttgccagg 85560
   gacctggggg cttcctagga tgcagaactt tcagtgcccg aataacagtt tcaggtaaac 85620
   ctagatggct ggtccctagg taaagtggac taggtccctg tgaaaggata ctgctacccc 85680
   ttccaggtaa agataaatag ctgggatggg taaagagaaa cagaggccag gtgcaatggt 85740
   tcatgcctat aatcccagca ctttgggagg ctaaggaggg agaattgctt ggtgctagga 85800
   gttgggagac cagtctgggc cacatagtga aatgccatct ctttaaaaaa aaaaattaaa 85860
   aaaattaaaa aaaaatttag ccaggtgcag tggcatgtgc ctgcagtttt ttatttgttt 85920
   gtttgggttt ttttgtttgt tttgatttgt tttgagacgg agtctctctg ttacctaggc 85980
   tggagtgcaa tggcgtgatc tcggctcact gcaacctctg cctcctgggt tcaagcaatt 86040
   ctcccgcctc agcctcccga gtagctggga atacaggtgc ccgccaccac gccctaattt 86100
   ttgtattttt ttttttttag tagagacggg gtttaaccat gttggtcagg ctggtctcaa 86160
   actgttgacc tcaggtggtc cacccacttt ggcctcccaa agtgctggga ttacaggcat 86220
   gagccactgt gtccatccgt gcctgtgggt ttgaggctgt gatgagctgt ggttgtgcct 86280
   ctgtactcca ctgtgggcaa cagagtgaga ccctttctct aaattgaaga cagcatatca 86340
   agtacccacg taattaagcc caaggtaatc ctccattatt tttcaagtcc tgtccatctt 86400
   tggtactaac cagcctggag aaacagcctc caaggttccc atcctcctct tcaggaactc 86460
   tgggtcaaga aacttattca gggccaggca cagtggctca cacttgtaat tccaacactt 86520
   tgggaggcca aggcaggagg agaccagcct gggcagcaga gtgagatccc atctctacga 86580
   aaaaaaaaaa aagagagaag ggatggttaa ggctagaagg actgtgacta tgtagtgtgt 86640
   tgacttgact tagatctttg ttcattaagc taaggaatta tcttgtttta caaaccaagc 86700
   tgaacccagg cccgcccatc tgctctgttc ctgccactac ttcaaacaga agccaaaacc 86760
   tcaactcagt atgttaggcc atttcttgca gcaaatttac cacacctact tggcttttgc 86820
   ctttttgtgg ccacagccag ggtgatattt caaaatattt cacagctggt taccacatgg 86880
   ccggcagcca tttagaacag acactgattt tagcactaag tcctgttcct ggagatcaac 86940
   agctatgctg gggattaact ctttagttgc tgaaagagag ggaggtctgg ccagcacggt 87000
   ggctcatgct tgtaatcaca gcactttggg aggctgaggt gggctgatca cttgaggtca 87060
   ggagttcaag accagcctgg ccaacatggt gaaactccat ctctactaaa aaagctagcc 87120
   aggcgtggtg gtgtgcacct gtaatcccag caactcagaa ggctgaggca ggagaattgc 87180
   ttgtacccag gaagcagagg ttgcagtgag ccaagatcgt gccactgcac tccagcttgg 87240
   atgacagagc aaaactccgt ctcaaaaaat aaaaaataaa aataagaaga gggaggtctg 87300
   ggggcctgga ggcaggtgga tggggtgggg aactcaggag gctgttggca gtaatttacc 87360
   aactgctagg aaagcttttc agtatttcaa caaccagtat cggcaggaca tggtggctta 87420
   tgtctataat tgcagcactt tgggaggcca aggcgggcag atcacttgag gtcaggagtt 87480
   cgagaccagc ctggtcaaca tggcaaaacc ctgtctctac taaaaatata aaaattagcc 87540
   gggcgtggtg gcacgcgtcg gtaatctcag ctacttggta ggctgaggca ggagaatcgc 87600
   ttgaatccag gaggtagagg ttgcaatgag ctgagattat tccactgcat tctaggcagg 87660
   gtgacagggt gagattctgt ctcaaaaaaa aaaaaaaaaa agtatgatca accaggccac 87720
   gtgcaggaga agggaaatga ggggatgggg tggagagagg gagagagaca aagagaaatg 87780
   aagggaggga agaggcagga aggaaatgaa tgttctatcc ttgatttggt ctgtcttcct 87840
   tgattacaac cccacaggat tcgagtccac atagttgctg ctgtaacaac tccctagaaa 87900
   attagcctct aattcctttt ttccctgctt ggattttgca caccatccat ggttgcatga 87960
   tatttccaaa ttcctgtgat cagatgttcc tcattaggaa gtgactcatc acatttgctt 88020
   ggcaatcatg aatacaggag aaattacttg gaggggccca ggggactggg aagccccatg 88080
   ccctcaagat attgctcttc caacctccag gatttcagtc tttgataatc aaaatcttaa 88140
   tgttaactac agaaagccag cacagttgtg aacttagcaa atactgtgat ttgtcaattt 88200
   acagtatctt tttgtattat aaaatatttg gcaggtaaat tttataatgt attttaaaat 88260
   atacaaataa ctctgtatta tacctgttga ttatgatgcg taaaaataaa tgaacactgg 88320
   gctggatgca gtgtctcacg ccagtaatcc cagcactttg ggaggctgag gtgggtagat 88380
   cacttgaggt caggagttcg agaccagcct ggccaacatg gtgaaaccat gtctctactg 88440
   aaaatacaaa aattagccgg gtgtggtggc acgagtctgt aatcccagct actcaggagt 88500
   ctgaggcagg agaatcgctt gaacctggga agcggaggtt gcagtagctg agatcgcgcc 88560
   actgcactcc agcctgggtg acaaggtaag actctgtctc aataaataaa taaataaata 88620
   aataaataaa taaataaata aacaaacaaa aaaacactgg taaacccacc actcaacata 88680
   agtagaatgt agtcaatatt cactttagag atctaatttt tagtcttttc caatcactgg 88740
   ctgatagtag taaatgtgtt ttttggtaag gtacagtggc tcatgtccca aagtacccag 88800
   cacttttaga tgccaaggtg ggaggatcgc cagaggccag cagttcaaga ccagcctagg 88860
   caacataaca agatctcgac tctacaaaga aataaaaaat tagccaggca tggtgacgtg 88920
   tgcctgtagt ccctcctact ttgggggctg aggtggaggg atcccttgag cccaggaggt 88980
   agaggctgca gtgagctgtg atggtgccac tgagctccag cctgggtgaa cgagttagac 89040
   tctgtcaaaa aaaaaaaaaa aaaaaagtca aaacaacaac aaagatgtgt tttttttgct 89100
   ggtatacaga atggccttga atgttgtttt ctttctctct tttttttttt ttttagatgg 89160
   agtcttgctc tgttgcccag gctggagtgc agtggtgcca tcttggctca ctgcaacctc 89220
   cgcctcccag gttcaagcaa ttctcctgcc tcagcctccc aagtagctgg gattacaggc 89280
   acatgccacc atgcccagct aatttttgta tttttagtag agacggggtt tcaccatgtt 89340
   ggccattctg gtctcaaacc cctgacctca ggtgatatgc ctgcctaggc ctcccaaagt 89400
   gttgggaatt acaggtgtga gccactgtgc ccggctgaat gttgttttct gattctaccc 89460
   agtcatccta attcctcctt ttttttttca tttccttcct tctacttatc tctctttctc 89520
   cgtttttttt ttttttttga caaggtctca ctctgtcacc aaggctggag tgcagtggca 89580
   tgatcacggc tcacggcagc cttgatctcc caggctcaag tgaccctccc acctcagcct 89640
   cttgagtagc tgggaccaca gagtgccact acgcccagct aattgaaaaa aaaaaaactg 89700
   tagttataga gtctcactat gttgtccagc ctgatctcaa gctcctggcc tcaagcaatc 89760
   ctcaccactc agcctcccta agtgctggga ttacaggcct gagccatcac gcctgtcctt 89820
   atttcccaaa gtgctgggat tacaggcgtg agccactgcg cccagcccaa aactttactt 89880
   tcaaaggaca tagtatctga atactaagac tttacgaact gttttatcaa tccatggact 89940
   tctaacctcc cattcaggga catggaaatc atctctgact ctctctcctg tgttactatg 90000
   aagattccat gcctctttgg agattctgtt gcttgcaacc ccgctcctgg aatagaattc 90060
   tgtgcttgtt agagtagttt ggtatttcat ttgcaagaaa caagaactaa ctgcaaattt 90120
   tagggggaaa aaagggcttg gacataccat aaggacatag gtgggctgga actgaaaatc 90180
   taagaatttg aggttggctg ggaactggcc actgtctctt tcatctttca tggcccgcat 90240
   agcctcctgc atggcatcct tttctctctc actgttaccc tcctaagcag tggggatttc 90300
   attaggctgg ctaatcattt ttagactctg aatgtcagag cccaagtcag taataataat 90360
   aggagacttc cataccccat ttaaataatg gagaagtcat taggcagaag atcaacaagg 90420
   aaatagaaga cttgaacaac agtataaact gatcagacct aacaggtatc tataaaatat 90480
   tcaacccgcc aactgcataa tctatattct tctcaagtcc acaaggacac tctccagaat 90540
   gaactatatg ttaggccata aaataagtct caatatattt aaaaagattg aaatcataca 90600
   cagtacattc tctaaccata ataaaacaaa attagaaatc aataatagag cccaggcaca 90660
   gtggctcaca cctgtaatca caaaattttg ggagaccgag gcaggaggat cacttgaggc 90720
   caggagttca aggctagcct ggccaaagtg atgaaacctt gtctctacta aaaatacaaa 90780
   aaattagcca ggtaaggtgg catacgcctg taatcccaac tacttgggag gctgaggcac 90840
   aagaatcgct tgaacctggg aggcagaggt tgcagtgaac ctcagtgaac tgagattgtg 90900
   ccacagcact ccagcctggg tgacagagcg agactctgtc tcagaataaa ataaaatgaa 90960
   atataaaata aatcaataat agaaatttga gaaatttaaa actatgtgga aattaaacgc 91020
   agtcctaaat aactcatagg tcaaagaaga aatcacggct ggacatggtg gctcacgttt 91080
   gtaatcccag cactttggga gaccaaggtg ggtggatcac ctgaggtcag gagttcaaaa 91140
   tcagcctggc taacatggtg aaaccccgtc tctactgaat atacaaaatt agctgggcgt 91200
   ggtggtgcat gcctgtaacc ccagctactt gggagggtga gacaggagaa tcgctctaac 91260
   ccaggaggca gacgttgcag cgagcaggga tcgtgccact gcactccagc ctgggtgggt 91320
   gacagagtga gactccatct ccaaaaaaaa aaaaagaaag aaaaagaaat cacaagagaa 91380
   attagaaact acttggagat aaatgaaact gaatgatata ccaaaaactt aaaggattta 91440
   gctaaagcaa tattttgaag gatatttata gttgtaaatg cctacagtga aaaagaagaa 91500
   agatctcaaa ttcaacaacc catttattta tttatttatt tacttattta tttatctatt 91560
   tacttatttt ttgagacaaa gtctcgctct atctgaggct ggagtgcagt ggcgcaatct 91620
   cagctcaatg caacctgcga ctcccctgtt caagggattc tcctgcctca gcctccttag 91680
   tagctgggat tacaggcgcc tgccactgca cccagctaat tttgtatttt ttagtagaga 91740
   cggggtttca ccatttggcc caggagggtc tcgatctcct gaccttgtga tccacacgcc 91800
   tcagcctccc aaagtgctgg gactacaggt gtgagccacc acacccggct aatttttgta 91860
   tttttagtag agacagggtt tctccatgtt gcccaggctg gtctcaaact gctaacctca 91920
   agtgatccgc ccgcctgcct tggcctccca aagtgctggg attacagaca ggagccactg 91980
   cgcctggcct gcatgtcttc tttgatctgg ccccaccctc ctttacttac tctgttcctt 92040
   caaattggaa tgtccttcag cctccacatc tacaacaatg gctggaattg tctccgttct 92100
   tcaaggcctg attataattc catcatcttc atgtaacctt tttttttttt cttcatccta 92160
   ctcttcaact gatggagcac aaagtctggt agaggagata aaggagacaa ttgggttctg 92220
   tcttgcaatg tgataacact ctggtgggtg tgtgcacaga gtcctaggaa ggcaggaggg 92280
   tgaggtagaa actgttgggt tagttgaaag atagaaaatt gcacactcca gttgacttcc 92340
   acttctgagg gcttagaact ccatgaggag gcatcgattt catcttaaag gatccctttc 92400
   tacactcatt tcttttcttt cttctctttc tttttttttt tttttgtttt tgttttgttt 92460
   ttagagactg ggtcttattc tgtcacccag cctggagtgc agtggtgtga tcgtagttca 92520
   ctgcagcctc caactcctgg gttcaagtga tcctcccatc tcagcctccc aaatagctga 92580
   gactacaagt ttgcatcacc atgcctggct aatttttttt acttttggta gacacaaagt 92640
   ctcactctgt tacccaggct gatctcgaac tccttgcctc aagcaatctt cccacctcgg 92700
   cctccgaaag cactagaatt tcaggcatga gccaccacac gtggcctaac ctgctgtttt 92760
   ctcaagagca ggacagtttt taatttctgg acctatcgca tctagcaagg ttcctggcca 92820
   gaaaaagtga aaataatttt tttttttttt tggagacaga gtctcgctct gttgcccagg 92880
   ctggagtgca gtggcgtgat cttggctcac tgcaacctcc acctcctggg ttcaagtgat 92940
   tctcctgcct tagcctctag agtagctggg attacaggca tgcgccacta cgcctggcta 93000
   attttttttt ttgagatgga gtctcactct gttgcccagg ccagagggca gtagagtgat 93060
   ctcggctcac tgcaacctct gcctcccggg ttctagcgat tctcctgcct cagcctccag 93120
   agtagctggg attacaggca cccaccacca tacccggcta atttttgtat ttttaataca 93180
   gacaggtttt caccatcttg gccaggctgg tcttgaactc ctgaccttgt gatccacctg 93240
   ccttggcctc ccaaagtgtt gggattacag gtgtgagcca ccacaccctg ttgcctggct 93300
   aattttgtat ttttagtaga gatggggttt ttctatgttg gtcaggctgg tctcaaactg 93360
   ctaacctcaa gtgatccgcc tgcctcggcc tcccaaagtg ctgggattac aggcatgagc 93420
   caccacacct ggctgggact tgcttttaat aaactaaatg tgatggaaat gacagtgtgt 93480
   gatttctggg gctaggtcat aaaagacatt gtgcttctgc cttgctctct ctctctcaga 93540
   gcccccactc cgggggcaag ccagctgccg catcacgagg acatccacgc aacctataga 93600
   gctgctcagg aggaactgag gccggctgcc aataaccagc accaacttgc ctgccgtgtg 93660
   agtgagccat ctgggaaacc aagcctccag cctccatgaa gcctgcaggt aacgtcagcc 93720
   ctccagtctt ccggtcttcc agctgaggcc ccacacgtcg tggagcagag ccaagccaat 93780
   cccgtcatgc cctgaattcc tgatccacag aactgcgaga gaatacatga aaattgtttt 93840
   aagccactac gctctgagat agtttgttat gcagcagaca acaactgatg taacagtggc 93900
   aggtgtacac ggagtgccaa gggaatgcaa agaacggaca cttaggggct tggagtggtg 93960
   gctcatgcct gtaatcccag cactttagga ggctgaggca tgagaatcac ttgaaaccag 94020
   gagttcaaga ccagtttggg caacatacga gacccctgtc tctacaaaaa aattttaaaa 94080
   attagctgcc cacactggca catgcctcta gtcccagcta ctctggaggc cgaggcagga 94140
   ggatggcttg agtccaggaa ttggaggctg aagtgagcta tgactgcacc actgcactcc 94200
   agcctgggag acagagtgag actgagaccc tgtctcaaaa aatatatata ttataaatac 94260
   ttaattctaa attttatgat ataattgtat tgagaggata ggggaaggga aagagttttg 94320
   tgtgggggtg taaaagttaa atcctcattt cctgttttaa gaaatcatag atcgtatcta 94380
   aaatagaaaa atcaaggaat agctatacaa gcaagttatt tagaaactgg agctgggctc 94440
   atgcctgtaa tctcagcact ttgggaggcc gaggcaggag gatcacctgc ggtcaggagt 94500
   tcaagggtag cctggccaac atggtgaaac cccatctcta cagaaaatac aaaaattagc 94560
   cgtgtgtggt ggcacgcgcc tgtagtccca gctactcagg aggctgaggc acgagaatcg 94620
   cttgaaccca ggaggcggaa gttgcagtga gccaagatca tgccactgca ctccagccta 94680
   ggtgacagag caagactctg tctaaagaaa aaaaaaaaaa aaacagaaaa gaaaccaaga 94740
   ggcatatact ggaagaaaca actgaagaag ttggttgaat ccaagaagca ggtcaagagt 94800
   agaaagagca gggattgctt ttcaccattg ttaacctttt agtacaattt gactcttaaa 94860
   attatgtatg tgtataactt gacaaaataa aaacaaacta aaaaaaggtc agagagaagg 94920
   agcaataaaa taggagattt aggtggagcg atggaacatg ttgttctttg tattttattt 94980
   tatttattat tattattatt attattatta ttttgaaaca gagtttccct cttgttgccc 95040
   aggctggagt gcaggggcgc gagctcagct caccacaacc tctgcctcct ggcttcaagc 95100
   aattctcctg cctcagcttc ccgagtagct gggattacag ccatgtgcca ccacgcccag 95160
   ataattttgt atttttagta gagatggggt ttctccatgt tggtcaggct agtctcgaac 95220
   tcccaacctc aggtgattcg cctaccttca cctcaggtga ttcaccctcc caaagtgctg 95280
   ggattacagg cgtgagccac cacacccagc ctgttctttg cattttaaat taaggggcag 95340
   ggcacatcaa actatttaat atgtgcaatg aacaataaaa tatatcctta ggatttatat 95400
   ggggcaatta tagagcaaag atacaggaag agatcgatgt caggagacag tgtgatggag 95460
   aaacaaagga cagaaggaaa gagaggggaa gagatagaaa caaatgccaa gacccaggaa 95520
   aacacagaaa gacataaccc agcctcatat aaactcaaag tgagaaggtg gagagacata 95580
   aagaaatcca caggtggctg ggcgtggtgg ctcacgcctg taatcccagc actttaggag 95640
   gccgaggcgg tggatcacct gaggtcagga gttcgagacc agcctcgcta acatggagaa 95700
   actccgtctc tactaaaaat acaaaaagta ggcgtagtgg cacatgcctg taatcccagc 95760
   tactccagag gctgaggcag gataattgct tgaacccggg aggtggaggt tgcagtgagc 95820
   tgagattggg tcactgcacc gcagcttggg cgacacagcg agactccatc gcaaaaaaag 95880
   aaaaaagaaa gaaatccatg ggctatggaa gtatgtacgc tcactcaggc acacaccacc 95940
   aagcgtgatg aagacccaag tctacataga tagcacctca cacttttgtt aaatagcaca 96000
   gaattaaaga attatcccag cctggacaac atggcaaaac cctgtctcta tcaaaagtac 96060
   aaaaattagc caggtgtggt ggtgcgcgat gtagtcccag ctacttggga ggctgaggtg 96120
   ggaggatctc ctgaacccag caggctgagg ttgcagtgag ccatgatcac accaccgcac 96180
   tccagcttgg gcaacagagt gagaccctgt cttacaaaaa aaagaaaaaa aaaaaaagaa 96240
   aagaaagaaa aggaaaggaa aggaaaagaa aagaaaaaat gattgttgag gtaggaggca 96300
   agactcaact ccagaggcag ggcacggaca ccagaccaaa ttgaggacca actaaaaatg 96360
   ggttgggaca gaagcagctt tccataaaga catcccttcc agagtgccat gtcagtttac 96420
   cgttgccatg acaacaccca gaagttatct cccctttcca tggcaaccac ctaccgacct 96480
   ggaagttacc accctcatcc tagacatttc tgcataagcc accccttaat ttgcatataa 96540
   ttaaaaatgg gcataaatga gtttaggtct gcctctcagc tgctattctg gacacagtgc 96600
   ctatgggtca tccctgctct gcaaggagcc gtacctttgc tgcggctgtg cactgaagct 96660
   tcaataaaag ttgtaacact ggctggacac gagtgctcaa acctataatc ccagcacttt 96720
   gggaggtgga ggtgggcgga tcactttagc tcaggagttc aagaccaacc tgggcaacat 96780
   ggtgaaacct cgactctaca aagtacaaaa catattagcc aggcatggtg gtgtgtgcct 96840
   gtagtcccag cttctcagaa gacaggggtg aaggattgct tgagcccagg aggcaaaagt 96900
   ttcagtgaga cttgatcatg ccactgcact ccagcctggg tgacagagtg agaccctacc 96960
   tccaaaaaaa aaaaaaagtc ttgttctacc agctaattgg attggcaggg acctcctttt 97020
   taatatatat atataggtat gtgccaccgc acctggctaa tttttgtttt tctttttttt 97080
   tttttttgag atggagtctc gctctgttgc ccaggctgga gtgcagtggc gtgatctcgg 97140
   ctcactgcaa gtgggttcat gccattttcc tgcctcagcc tcccgagtag ctaggactac 97200
   aggcgcctgc caccatgccc ggctaatttt tttttttttt tttttttttt gtatttttgg 97260
   tagagacagg gtttcatgtg ctagccagga tggtctcgat ctcctgatct cgtgatccac 97320
   tcaccttgac ctcccaaagt gctgggatta caggtgtgag ccaccgggcc tggcctaatt 97380
   tttgtatttt tagtagagat ggggttttgc catgttgccc aggctggtct tgaactcctg 97440
   ggctcaagtg attcacctgc ctcggcctcc caaagtgttg ggattacagg cgtgagccgc 97500
   cacgccaggc ctcatgagtt tcttatgtag ctgtatcaaa cttgtttctc ttttgaactg 97560
   tgattacagt tccagtgttc atatgttgcc ctgcaagtag gttataagtg catgggggac 97620
   aggaaccaag ccttatctac tgaagaatct tcctcgggcc cagcttccag ggaggctctg 97680
   aatttgaagc ccctctctgc cattcacttg ctgtgtgact ttgggcaggt tacttgacct 97740
   ctctgtgtgt cagagctttt atcttcagag ataccactgg gttgaatgac ataaaattgc 97800
   caatattcta ttatatttga ccccaaaatg gcaatttcat atagttcaac ctaataaaac 97860
   cactacctct taggtatttt gagggttaat tgtcatgtct attgtacccc aggcaagcac 97920
   catcctgctg agtacaaagt ggacctgata gtaaagcccc tcagactttg tggaacaaaa 97980
   gcagaaccct aggaacggca gagcaaccag ttagaaagag cctggctcct gatgaactgc 98040
   agcttccgta tctgttatgg attgctaatg tctggaccct tatatgagca agatgtaatc 98100
   ccccatttag gttaaaccac tgttctttgg gtctgggttt tttgtttgtt tgttttttct 98160
   ttgagacgga gtctcgctct gtctcctcag ctggagcgca gtggcgtgat cttggctcac 98220
   tgcaatctcc gcctcctagg ttcaagcgat tctcctgcct cagcctcccc aagtagctgg 98280
   gactaaaggc gcccaccacc acgcccagct aatttttgtg tttttagtag agatgaggtt 98340
   tcgccatgtt gaccaggctg gtctcaaact cctgaactca agtgatctgc ccctcttggc 98400
   ctccctaagt gctgggatta caggtgtgag caaccatgct cagcctgggt ctgttttgaa 98460
   agaggtaaac tgacatctta attaataatg ggtgtgtgtc agagagaaat aaaccatgta 98520
   cttccacata gagatttctg ccttaaggcc gggcacagtg gctcacacct gtaatcccag 98580
   cactttggga ggccaaggcg gaaggattat ctgaggtcag aagttccaga ccagcctgta 98640
   caacatgacg aaaccccgtc tccactaaaa atacaaaaat tagccaggca tggtggcgcg 98700
   cgcatgtaat cccagctact cggaaggctg aggcaggaga atcacttcaa cctgggaggc 98760
   ggaggttgca gtgagccaag attgcaccac tgtattccag cctgggtgac agagcaagac 98820
   tccatctcaa aaaaaaaaaa aaaaagtttc tgccttggga gactgaatgt gatgacactg 98880
   gctgagatgg tcagtccaag acatgagggc tggttgggga agtaatgagt tcaattgcag 98940
   atagattttt gtttttgttt ttagagacag ggtgtctgca gatagatttt tgtttttgtt 99000
   tttagagaca gggtgtctcg ctctgttgcc caaggtggag tgcagtggtg tgatcatagc 99060
   ttactgcagc cttgaaatcc tgggctcaag cgatcctccc acctcagcct cctgagtagc 99120
   tgggactaca ggcatgtgcc accacggctg gctaattatt atttttattt ttttgcagag 99180
   acgggggtct ctctatgttg cccgagccag tctcgaactc ctggcctcaa gtgatccatc 99240
   tgcctcaaca ccctaaagtg ctggcactat aggcatgagc cacggcaccc agccactgta 99300
   agcctttttg cttcacctag ccaccaatct aaatgggggg aagtgttctc cattttttta 99360
   gattttcata tctgtaagta gaccaaccat gacagcttta cgacccaaat acaaggctac 99420
   atttgatgtt actagcgagc aaaataactt cagatctaca tgggcagtga aataaatagt 99480
   gcgcatcttt aaaatttccc ctctagttct aatcttttca ggtttccccc tcattactct 99540
   gaaatttctg gaatgtgtac atgttgctct cacacccatt cctgggcttt aaggcaggaa 99600
   catcaaaaaa catcagcatg aggtcaccac ttggtcagtg gaaaattttt tttttttttc 99660
   tgagatggag tttcgccctg ttgcctaggc tggagtgcag tggcgtaatc tcagttcact 99720
   gcaacctcca cctcccgggt tcaagcaatt ctcctgcctc agccttccaa gtagctggga 99780
   ttacaggagc gtgccagcat gcccagctaa ttttttgtat ttagtagagt tggggttttg 99840
   ccatgttggc caagctggtc tgaaactcct gacctcatga tctgcccccc ttggcctcct 99900
   aaagtgctga gattacaggc atgagccacc gcgcccggcg ggtcagtgga aattttttta 99960
   aatggtggtg acaccatctc tggtgcttcc accctgtggc tcctggctcc cagccatttt 100020
   tcatggtggc aaagactgcc cccagggtgg tggcagaggt cagtgtgtgg cctggacgag 100080
   tcccagccaa catgctcctc accccatcct ggacgcaacg tggaggtggt agagagagag 100140
   gagcaaggga agctggaccc ccgcagagtg gaccactact ctacagggac atgcatgagg 100200
   gacacctgga tctgaacagc tagcactgag acactccaac acacggtacc tggaaaccgc 100260
   aggtggcact ttgaggaaac aaccaggtac gtgcagggat tttttttttt ttttgagaca 100320
   gagtctgtca gcccaggctg cagtgcactg gtgtgatctc agctcaccac aacctccgcc 100380
   tcccaggttc aagtgattct cctgtctcag cctcccgtgt agctgggatt acaggcgcct 100440
   gccaccacac ccggctaatt tttgtatttt tagtagagac agggtttcac catgttggcc 100500
   aggctggtct tgaactcctg gcttccagca atccacccgc cttggcctcc caaagtgctg 100560
   ggattacagg cgtgaaccac ctcattctgc ctttttgttt tttttttttt ttttaaagag 100620
   acagggtctc cctctgtccc ccaggctgga gtgctgtggt gcaatgatag ttcactgcgg 100680
   cctccatctc ctaggttcaa gtgatcctct catctcagcc tcctgaatag ctgggactag 100740
   aagggtgtac caccatacct ggctaatttt ttaattttct gaaaataagg tcttgctata 100800
   ctgtccaggc tggttatttt tagttagaga gatgttgtgt gttttggaag gggcttccta 100860
   gatcatctgc tcccaatcct tatttttttc aaaggagaaa aatgtttgac tcatcatact 100920
   gactttaaaa aaacccactt tttattgtgg aaaatttcaa gcagactcac gcacaaacaa 100980
   aacaatataa tgaacaccca tgtacccatc atgaaaaaat taccatgagt tggtactcat 101040
   ggacattctt tttctttttt ttaagacaga gtttcactct gtcgcccagg ctggagtgca 101100
   gtgacgcgat ctcggctcac tgcagcctct gcctcctggg tttaagcaat tctcctgcct 101160
   cagcctccca agtagctgag attacaggca ccaccaccac gcctggctaa tttttgtatt 101220
   tttagtagag atggggtttc cccacgttgg ccaggctggt ttcgaactcc tgacctcagg 101280
   tgatctgcct gcctcagcct cccaaagggc tgggatcaca ggcatgagcc actgtgcctg 101340
   gactcgtggc cattcttgtt tcatcttttc ctcatgtacc ttctccttcc cattttattt 101400
   tgaatcaaat cccagacctt gtataatttc ttcattaata tttcagtatt atttctttaa 101460
   ggacttttaa aaatatatac ccacaatgca ttatcatgcc taaaaattaa taataattcc 101520
   ttaataccat caaatatatc cagtcagtgg taatattccc aattgtctca taaatgtaat 101580
   aaatgagggg tttcattggt ttgttttaga gacagtctct ctaggaaaaa aattcctgga 101640
   agtggaattt ctgagtcaaa actatacaca ttttattttt aaataaatat gaaactgacc 101700
   ttcaaaaggg ctatattaat ttatatttac ataatgaatg acttttctgc atatatttgc 101760
   catactgatg ttataaattt tcttcagttt tgtctgaaag gtgaaaagta ttttattatt 101820
   gttttatttt gcattatttc attaatgcta aggctgaaca tgtttactct taacttctgt 101880
   atctggtaag ttatttgttt atgttcttgt tcatatctgt ctgggttcct ttctcctctt 101940
   cttattgact catgaaccta tatactgtat taatcttttt catatgtgtt agaaatatat 102000
   ttgtagtttg acttgctata tagaagtatt tttgttttgt tttaaagaga tggggtctcg 102060
   ctatgttgtc caggctggtc tggaactcct gggctcaagc aatcctattg tattaatctt 102120
   tttcatatgt gttagaaata tgtttgtagt ttgacttgcc atatagaagt atttttttgt 102180
   tttaaagaga tggggtcttg ctatgttgac caggctggtc tagaactctg gtgctcaagc 102240
   aatcctcctg ccttggcctt ttttgagaca gagtctcact ctgttgccca ggctggagtg 102300
   cagtggcgcc atcttggttc actgcaatct ctgcctcctg ggttcaagca attctcctgc 102360
   ttcagtctcc caattagctt ggattacagg tgcctgccac catacctggc taattttttt 102420
   tttttttttt tgtattttag tagagacagg gtttcaccaa gttggccagg ctggtctcaa 102480
   actcctaatc tcaggtgatc cgcctacctc tgcctcccaa ggtgctggga ttacaggcgt 102540
   gagccacctt gcccggccca cattttcctt ttaatgtttg tttttacact gaagtccttt 102600
   ggcataataa atgaaataga gatgcagctt cccaccctcc cccttctgag tagttaattc 102660
   tttaagaaac aactattagg ccaagtcagg aggatcgcta gaagccagga cggtgaggct 102720
   gcaatgagct atgatcgcac cactgcactc cagcctggat gacagagcaa aaccttgtct 102780
   ctaaaaaaat atataaaata tggctagacg cagtggctca cacctgtaat cctaacattt 102840
   tggaaggtct aggcaggagg atcgcttgag gccaggagtt caagatcagc ctgggcaaga 102900
   cagtgagact ctttctatac aaaatatttt tttaggctgg gcatggtggc acacgtctgt 102960
   aatcccagca ctttgggagg ccaaggcggg tggatcacga gttcagtaga tcaagaccat 103020
   cctccctaac atggtgaaac cccatctcta ctaaaaatac aaaacattag ctgggcatgg 103080
   tgacacgtgc ctgtagtccc agctactcgg gaggctgagg caggagaatt gctggaaccc 103140
   aggaagcgga ggttgcagtg atctgagatc acgccactgt actccagccc gggcaacaaa 103200
   gtgagactcc atttttttta attatcctgg tgtggtggtg catgcctata gtcccactcg 103260
   ggagactgag gtgggaggat cacttgaggc caggagttcg aggctgcagt gagctatggt 103320
   catgccattg cactccagcc tgagcaacag agtgaaactc ttgtctcaaa taatggtaat 103380
   aaataaaaat aaaatttaga aaggaacaat tattgaataa ggaatctttt cccaaccaat 103440
   gtgcaatatc atctttataa gtgctaaatt cccatgtgca tttggggcta tttctggacg 103500
   cttcattccg atggattata tggattatgc cagtcctgtg ccaggacaag catgctttga 103560
   cttttatttc ctgttttaat atttgatagg gcaggtcccc ctattactct tctgtttcag 103620
   aatgttctgg tttttctcat tatcattctg cataaacttt caaattattt tatcaagttt 103680
   cccaaataat ccttttggga tttagagact gcattgaatt tacggattta gggaaaactg 103740
   acatcttcta tatcatccaa tcttcttatc taagaacaag ttattaccat ttgattattc 103800
   aagtcttttt aaattaatta attaattttt tttttttttg agacagagtc tcactctgtc 103860
   gcccaggctg gagtgcagtg gcgtgatctc agctcactgc aagctcgcct cccgggttca 103920
   agagattctc ctgcctcagc ctcccgagta gctgggacta caggtgcccg ccaccacgtc 103980
   cggctaattt tttgtatttt gtttagtaga gatggggttt cactgtgtta gccgggatgg 104040
   tcttgatctc ctgaccttgt gatctgcccg cctcagcctc ccaaagtgct gggattacaa 104100
   gtgtgagcca ccacgcccgg cccttttttt tttttttttt aagacaggtt cttgctctgt 104160
   tgcccaagct ggagggcagt ggtgcaatca tagctctctg cagtgttgat ttccttgggc 104220
   ttaagtgatc cttacacctc agcctcctga gtagctggga ctacaggcac atgccactat 104280
   gcctagcctt ttttttttct tttaactttt tgtagagaca aggtcttgct atgttgccca 104340
   ggctgatctg gaactcctgg gctcaagtca tcctcctgcc tgggcctccc aaagtgctgg 104400
   gattacaggt gtgagccact gtgcttgacc ccacaagcct tccttctccc tcgctccacg 104460
   atagctgttt gtcctgaagg ttttcctttt gtatttttgt atttattttt acttttattt 104520
   ttttgagatg gagtctcgct ctggccccca ggctggagtg cagtagcaca atctctgctc 104580
   actgcaacct ccacctcctg ggttcaagtg atttgcctgc ctcagactcc agagtagctt 104640
   ggactacagg tactcaccac tatgcctggc taatttttgt atttttagtg gagacagagt 104700
   ttcaccatgt tggccaggct gctctcgaac tcctgacctc aggtgatcca tgcgcctcag 104760
   cctcccaaag tggtgggatt actggtgtga gccaccacgg ccagccaagg tcttcccttt 104820
   ttaagtgact caaaatactt tagtgttatc aactgatcca atataggttc tataatttct 104880
   tgttaatgtt actcctattt attttaaggt ttttattgct cttatgaatg ggatatttcc 104940
   ttccattaat tttcctttcc tttttttttt taattttttt ggagacagag ttttgctctg 105000
   ttgcccaggc tggaatgcag ctacatggtc ttggctcact gtaacctccg tctcccagat 105060
   gcaagtgatt ctcctgcttc agcctcctca atagctggga cctacaggca cacactacca 105120
   tgcccggcta atttttttca ttttcactag agaggatttt gccatgttgt ccaggctggt 105180
   ctcgaactcc caagttcagg caatccaccc acctcggcct cccaaagtgc taggattaca 105240
   agcgtgagcc actgcaccca gccccttcca ttaattaact tttttttttt ttttttgaga 105300
   cagagtcttg ctctgtcacc caggctggag tgcagtggtg cgatctcggc tcactgcagc 105360
   ctctgcctcc cgggttcaag caattctcct gccttagcct tctgagtagc tgggattaca 105420
   ggtgcccgcc accacaccca gctaattttg tatttttagt agagacaggg tttcaccata 105480
   ttggccaggc tggtctcgaa cttctgatat caagtgatct gcccgcctcg gcctcccaaa 105540
   gtgctgggat tacaggcatg agccacctca cccggtctaa ttttctggct agttaaaaag 105600
   agctactgcc tgtttatatc ttgggttttg tttttgtttt tgttttgttt ttttcaagtt 105660
   ggggtctcac tctgttgccc aggctggaat gcagtgaagg gatcatagct cactgcagct 105720
   tccaattcct gggctgaagc gatccaccca tctcagcctc ctgagtagct ccagctacag 105780
   gtgctcacca ctacatccag ctaattttta ttaatttttt ttgtagagat gaagtctagc 105840
   tttgttgccc aggcttgtct caaactcctg gcctcaagcg atcttctaac cttggcttcc 105900
   caaagtgctg ggactacagg catgagccac tgtgcctggc ctgtatttca tccctagctc 105960
   ttttctctag ccctgccctc cttgcagagg tcagctgtgt tgggcttgag tgccatttct 106020
   atgtcaatta ctcctaatgt ccatagtctt gtcctgtctc ctgagctatc atcattttac 106080
   ttttaacttg tttcctaaat ttcaaatact cacataccac tgtcataatt tttcatattc 106140
   tgtacattgc ctgtactatt atttattggc ttaacatttt ttctttaaac cagctcatta 106200
   aaaataactc tagacttatc ttgtgtaatc acattcatga aataacaagt agccttatgt 106260
   attggttata acttttctaa tataagtgaa aataaataca aaactattac agtttaaaaa 106320
   aaaatgctct tcttcataaa gtgtaaaaac atctcttgtg gcatctacca catttttttt 106380
   tttttgagac ggggtctcac tctgttgccc aggctagaat gcagtgaagc aaccatggct 106440
   cactgcagcc ttgacctcct gagctcaagc gatcctccca cctcaacctc ctgagtaact 106500
   gggattacaa acgtgtgcca ccaagcccaa ctattttttt ttattattgt ttgtagagat 106560
   ggggggttct atgttgccca ggctggtctt gaactcctgg cctcaagtga tcctcctgcc 106620
   tcgggcctcc cgaagtgctg ggattacagg cgtgagccac cacgtctggc tatccaccac 106680
   attttccgaa actctgttct attcagtaac aaaccattca acctaaatat tcatcattgc 106740
   ttcaaattca atgttcttcc tccaaagtca ctctcaagcc cacctgcctc actctgtcag 106800
   ttccccgatc accagacctc caaacttggg cttcttgtat gaccaattct gctccttcac 106860
   tcccatccta aggtttcctt tcagtggcct cttgcaactg tccctagccc tccatcctcc 106920
   caggcatcct ccttatcctc ctccctggtg acagatcacc tcccagcagg attcccctgg 106980
   cttccctagt gaggacctga gtcttgcctt gtggcccgct tcaccatggc tggaccccct 107040
   tcttttttga gatggagttt cgctcttgtt gcccaggctg gagtgcaatg gcgtgatctc 107100
   ggctcactgc tgcaacctct gcctcccggg ttctggcgat tctcctgcct cagcttcctg 107160
   agtagctggg attacaggca tgcgccacca tgcccagcta atttttttgt attttttagt 107220
   agagacgggg tttctccatg ttggtcaggc tggtctcgaa ctcccaacct caggtgatct 107280
   gtcccatggc tggacctctt aatgccatca ccatcattta aacatacttc ctacctcccc 107340
   ctgcccccat ctcctactgt tccatagaca atcaaaacca tgaccgtgac cttgggagtt 107400
   tgcctcttcc cctccccaaa cttcacctgt cctttttttt tttttttttt ttgagacagg 107460
   atcgcattct gttgcccaag ctggagtgca gtggcatgat ctctgctcac tgcagcctct 107520
   gccttctagg ttccagccat cctcccacct cagcttcctg agtatctggg actaaaggcg 107580
   catgccttca cacctggcta atttttgtat tttttgtaga gatggggttt ctctatgttg 107640
   cccagactag cttacctgtc cttttagggt aactttgcag gaagccttct tagatgattc 107700
   cagccctgta ccccctcttt tttcttttct ttcattgttg ttgttgagac agagtctcgc 107760
   tctgtcgccc aggctgaagt gcagtggcat gatctcagct cactgcaacc tccacttcct 107820
   gagttcaagt gattctcctg cctcagcctc ccaagtagct gggatatatc gccaccacac 107880
   ccggctaatt tttgtatttt tttttttagt agagataagg ttttaccatg ttggtcaggc 107940
   tggtcttgaa ctcctaacct caggtgatct gcccacctcg acctcccaaa gtgctggaat 108000
   tacaggtgtg agccaccacg cttggccctg tcccccctct cttactcctt caacagttta 108060
   ttgtcatctc agtggaggtc atctgagtag atcacaaggt acttgaaggc aagcttcatg 108120
   cccaaatatt ccttctgcat cagtgcaatg ccaagaacac attctgtgca caaaaaaaat 108180
   gctaatggag tcatggccca gtgcattccc aaagacttac ctggaactcc acctcgccct 108240
   gtgacttggc cacagctact cacccctccc cgcacaacct tcaactccca ccaaccaggg 108300
   gcaagatggg aaaaggagca agtgtaagaa tagcagagca cgctgtgaaa tagaagaagc 108360
   cagctttagg atcatgagtc ttttaatata ttattctgaa aagaaaggaa ttaaaaaaaa 108420
   gttcctgaca tctcttctgt tttgtttttt aatttaaata aataccctgc ccaccccctc 108480
   cacccgctaa aatttccctc ctcctccccc atcatcctgc ccatccctgg cactagaccc 108540
   tcaccccaga actaaataag atgcctgatt cagagcaggc cacactgact accaaattct 108600
   ggcaaattgt aaatactgtg aatggtcttg ggccctctgc ttccctgggc cccttcttct 108660
   ctcggtccac tccccatgca gacccctcct tggctgtggg gtggcatgga cggcggggcc 108720
   tttaggcatt ggcattgggg agagtgcaaa cttggccctg attctccact cctagctcag 108780
   gagtaggtca gaaaggccca gaatcgccct ccagactgaa ataaataaca aaataaatac 108840
   cccctcccca actcccggct ctctagaaaa gagctcctcc atctggcact ctagtccctc 108900
   tctttcagct cacccacccc cctcttcctc ccatcccttc cttcctccct tccccatgct 108960
   gacctctggc ttggttcagg aggaggggtg tacaacgccc agcgggaaga acagccagag 109020
   cgactctcgc cccaacctca agcaaaccct cctcctcctc cgtttctcag cagcgctgtt 109080
   ggcatcctaa gcccaatggg cctggcagac tctccccatc ccggggggta ggttggaggg 109140
   caggggttct ccctggcact ctcccacgta gtctttcaat gtctctcaag gcgaggctcc 109200
   cgggagagcc aggcgtgccc gccccctccc cagtccaggc gccccggtac ccccccgccc 109260
   gcctcagatg gcggtgtccc agaggaccgt gtgctgccgg cggcagggcg gggtgccgga 109320
   cttgcggggc tcgggtcccc gccgccagct ggggaggatg ggcatgctct cctgcttgca 109380
   gaggcctcgg tccgggcggt ggcgcgcctt gatctccttg cacacgcagc gcttgcgctc 109440
   gatcacctcc agcagcttcc cgtcacgctc gcgggccggc tggggcggca ggggcagagg 109500
   cagcggcagc gcggggtggg gctgtgccgc gggcggggga gaaaggaaaa cgcgggtaag 109560
   gtcggggacc tcagcatgac cggacgggtc cgcactgccg agacccacgt ctcccctcag 109620
   aagcaagtcc ttgcttgccc cccaacggcc tctgctggct ccctctggct gtggggccca 109680
   tgggggccaa aatccaactt gcgcagcttt ggcccagcca ctccctagtc ttcctagaca 109740
   ggtgcatttt ttaagttggt ccccctagaa gggatctccc cttttttttc tttcttttgt 109800
   ctttttgaga cagggtctgg ctctgtcgcc caggccagag tgcagtggtg caatcatagc 109860
   tcactgcagc ctcgacctcc tgggctcaag caatcctcca gtgtcagcct ccctcccaag 109920
   tagctgggac tacaggcatt taccactaca ccagctaatt gttaaaaaat taaattaaag 109980
   tttatttttg tatttatggg atcttgcttt gttgccaagc ctggtcttga actcctggcc 110040
   tcaagggatc ctctcatctt ggcctcccaa agcactggga tcacaggtat caactaccgt 110100
   gtccagctgg ggcaagggta gacaaggtcc ctcccttcct cctttcctcc ctttctttct 110160
   ttctcttttc tttcttgctt tcttttcttt cttgcttgct ttctttctct ctttctctcc 110220
   ttctctcttt cttccttctt tctctcactc tctctctttt tctttctttt gacaggttct 110280
   ccctctgcca cccaggctgg agtgcagtgg aacgaccatg gctcactgca acctccacct 110340
   cccaggctca agtgattctc ctgcctcagc cttcagagtt gctggaacca caagcatgca 110400
   ccagcatgtc tggataattt ttaaattttt tgtaaagaca gggtctccct atgtcgccca 110460
   ggctggtaca gaattcctag gctcaaccac ttctcttggc tcggcctccc aaagtactag 110520
   gattataggc gtaagctacc atgcttgacc tctttatttt tatttctttt gagacacggt 110580
   cttgccctta cacccaggat agagtgctgt ggtgggatcg tagttcattg caacctcagc 110640
   ctcctgggtt caagcgatcc tcccgtctct gcctcccaca gttctgggat tgcaggcaca 110700
   agacatagtg cccagtgggg aggtcttttt cctaaatcgc acaaaggtgt gctgttggct 110760
   agtggtggcc ctgactgatc tccaagctgg gccctacctt gctgcccatc ttaagcaaac 110820
   cctcaaaccc ttcccagatc ctctcccaac cccctcactc tcattcctgg gttcccgctg 110880
   tctgcgctgg ctcaggcccc agtctgcctg gccccttttt ttttttttga gacagagtct 110940
   aactctgtcg cccaggctgg agtgcagtgg catgatcttg gctcactgca gcctccgtct 111000
   cccgggttca agcaattctc ctgcctcagc ctcccgagta gctgggatta cgggtgcctg 111060
   ccccaatgtc cagctaattt ctgtggggtt tttgtttttt gttttttggt tttttttgag 111120
   acagagtctc tctctgttgc ccaggccact ggagtgcagt ggtgcgatct tggcttactg 111180
   caacctccgt ctcccgggtt caggcgattc ttatgcctct gcctcccgag tagctgggac 111240
   tacaggcgtg cgctaccacg cccggctaat ttttgtattt ttagtacaga tgggatttca 111300
   ctatgttggt caggctagtc ttgaactcct gaccttgtga tccactcgcc tcagcctccc 111360
   aaactgctgg gattacaggc atgagccacc gcacctggcc ctaatttttg tatttctagt 111420
   agagacaggg tttcaccaca ttggccaggc tggtctcaaa ctcctgacct caagtgatct 111480
   gcccgcctcg gcctcccaaa gtgctgtgat tacaggcgta agccaccgca ccctgcctgg 111540
   cccctttctt tctttttttt tttttcttct aagacagggt ctcactctgt ggctcagact 111600
   ggaacgcagt agcgtgatca tggctcaatg caacctccac ctcccagggt aaagccatct 111660
   tcctacctca acctccagag tagctgggaa ttcaggcttg cgacggcaca cctgactaca 111720
   ttttgcattt tttatagaga tgggatctca ctatgtggtc caggctggtc tcgacctcct 111780
   gggctcaagc aatctacctg ccttggcttc tgaaagcatt gggattacag gtgtgagcca 111840
   ctgcaactag ccttctttta aaaaaaagaa aaaatagaga cagggtctca ctatgttgct 111900
   caagctaatg aacttctggc ctcaagtgac cctcccacct tggcctccca aagtgctggg 111960
   attacaggca tgagccactg tgcccagccc tggccccttt attgcttttc tttgcatatt 112020
   caagccttct ccacctgctc cttcacaaat aaaagaaaaa ccaagcctca cctggctcag 112080
   ggcatccctg ggctggctgc agggccgggg tgtgtggagc acgacctgcc ggactccgga 112140
   ggtggaggcg gagcgcccca ggcggtagcc tcccgtgaag tctgaggtgg aagaaggagg 112200
   agtgaggagt gcaggggcct caggtacccc tcccctagtt cccagtcccc tgcccttgaa 112260
   tggtccaggc tgcccctgcc tcatcagaag ggaaaaaggg gtggagatgt ggtatgagga 112320
   tttagggccc tgggcacaga cgtgaacagg tgagaggaag tcaggacagg tgcagctaga 112380
   ccccgggaga tcccccagcc agccccgctg tgcacaggtg tgtgcaggcc gcgtcacctc 112440
   cattgcggtg gcaggcgggg aaggtctggc agggaatggg cagcgcggtg cggctccctc 112500
   ctcggtctcc atggtggatc ctggccagca gtccctctgc cccctggctt ctcactggga 112560
   tccccgatgt cccagggccc ctgtcctcac gctccacctt gcctggaccc tcggcactgc 112620
   cattccaggc ccgggcaccg tcctcgacct ctgctgggat gacaaggaga gctgtgtgcc 112680
   tgcatgttgg aggcgtcatt ctgtccctct ggccacacat gctgcagtat gggtggacca 112740
   ggagacacaa ttgccccttt tgcaagtaac ggctgggatg gggctccttc acactccccc 112800
   aacatacata caaacacaca cacacccctt cctttccacc cattggtacc atctcttggg 112860
   gctgagaatc atgtctatgt gctgcatcca aacccttttc cctggtcaga ggggcctccc 112920
   caaccgcagc tttagcttcc aagcccctcc atctccagat agggcgagaa tgaccatccc 112980
   cctgggatca agacctgtag ggggagaatc ccaattcctg agcccagaag ggggagcgct 113040
   ggggggaagg gctgcctgtg aactaagctc atctctgaga ggaggcctgg cagagcattg 113100
   cgttgccagg caacggtagg ggcctccctc tcctctccct cctgtctgga tgggttgcca 113160
   tggtaacagc tgggaggcag ctgtgtttag ttcctggtgc gcacactcac aagcacacac 113220
   ccctgcagcc ccgggaaggg aggggcaggg agcagagcag gacagggcca gatttgaccc 113280
   ccttcgcctc ccagaccttc ctgcccctcc agcgcacact gggggacccc ggtctttctg 113340
   tctatcgccc acccccacct ctctggtctc ctagaaaaca tcccagactc aaggctgtga 113400
   cttccaaata acccctccaa cacctccccc aaaccatcgc aggcatcccc agctgccccc 113460
   cacccctgat gccagccccc actccccacc cctcactcac tgtccaactc ctttgctttt 113520
   ctccctccat agaggacctt cctctgcagg gcccagcctg ccccaaatgt cgcggcgccc 113580
   acctcctctt cttcctcctc tggtgtccct gcgctggcga tgacgtgggc acaggagatg 113640
   ctggcaaaag cacccccatc ggtcccttgc tcctgcagct ggatctt 113687

<210> 2
<211> 146407
<212> DNA
<213> Homo sapiens

<400> 2
gatcctccca cctcagcctc ctgagtagct aggaccctag gcatgtatcg ccacatctaa 60

ctaatttttt tttttttttt tgagtggaga tggggtctca caatgttgcc caggctggtc 120

tggagatgtg gtctcactat gttgggcagg ctggtcttga actcctggcc tcaagtgatc 180

ctccctcctc agcctcccaa agtgctggga gtacaggtgt gagccactgt gcccagcctg 240

gggaagggtt ttatgaggaa ggtgatggca ttgccctggg gccctgggtc tggttcagga 300

ttggccaggg tgaatcaggt gaggggctca tcctggaggg ccatgggaga tgggttgtta 360

aacagactcc acccctgctc caggcagtgt ggtcgccatg atgtaatcag actccccagg 420

cacagctcag aggggtgggc atggagggct tcctggagga gtgagccagt tgggtgcagc 480

aggatgggcc tgtgggatgg agcctggaag aagatgccag cctggggcat tggagaagtg 540

agcagggcag acccagagtc tgaagagcct ggaatgttct agaaggtatt tatgttttat 600

atgaagctga ccgacctggc tttccctgct ctttgtctgg gcctctccgt gctcctctga 660

gcagtggggg caacagtatc tgcagcgggc cccagggtga tgggaggagc ccccctgggc 720

ttcgtgtggc ctggctcagg gctcagtgcc ccggaccagc ctcccagcac tgccaggtct 780

gccccaccag acccctggcc gctccgctct tcccacagat ggagactcaa ggctggcctc 840

tgccatcgtg gacacagtgc tggcaggtgt cccaggaccc cggggtcccc ctggtccacc 900

aggtaagtga atggtgggct ggcctgcatt gaggactcca tccccccagg gctggcctga 960

gccatggggc cttgagggga gctggaggtc ggctggcggt ccatggcttc tgggcaatgc 1020

tggggactgg gtctgtccca cccgctgtgt ggcaggaaat gaagcccctc gtttcctagt 1080

ggcagtgccc ctgggggact ggatgcatga aacccacgtg attctgctgg aataagatgc 1140

ctgcatgagg ctgcccttgc atgggccaga gtgggacaca caccagtggg ggatggggtc 1200

aggacagagg tggcaggaag tccctacctt tgaggcagaa accaaggggt tcaaaggggc 1260

tgggacctgg ctgtcagtcc tggcctgacc ttccatcatc cacattcctc attgggggaa 1320

gaatgcaggg ctgactgcct tgtccctttc tgcccgggaa gggagtcatt cattcattca 1380

ttcatttgtt tattcactga ttcattcatt cttttattca ttcatttatt cattcattcg 1440

ttcattcatt cattcatttg tccattcatt cattcgttca ttcattcatt cgtttattca 1500

ttcattcatt catttctctg tgcgtctcac aaacattcat tgaacatctg ccactacatg 1560

tgccagacag tgatctaggt tcccggaact cctgaataat caaaacaaac atcatcgaga 1620

aactctcatt ctggtggagg acaggaatat catcataaat tgtgacttcg acaggatctg 1680

agggagctac aagggagaag ctggggcgtg gcggggaggc gccgtcaaaa cgtgacattt 1740

cagaagatgc tctagggagg agagggagta agctcagagg gtacctgggg gccttcaggt 1800

gacatgccag gtctctggag aactggacag actcctgaca tttgtcccct ggagtgcctg 1860

tcctcacatg ccctctccaa acctgggagc caggccatgc tggacagccc aacatgcggc 1920

tccctgcacc acagggaggg atcatgagag accctccctg ggcccgtgtg gggtcatgag 1980

agaccaagac cttactcttc ctagcaggct ggagattagt catgaatctc cctccctgag 2040

ctgggcacac acatgagacc cagcccggcc ctctgggtac tccacatccc atggggcaga 2100

cagacgtgta aacctcagtg gccatgggcg gtggctggtt tacataaggg atttgcctgg 2160

agaagcagtg gaaagctggg aaggcttcct ggaagaggtg atgctctgtg tcagactggt 2220

aggaaaacca gtaggaaagg gtttgctggg agagtggagg aatagggagg ctccaggagg 2280

aaggacagct ttagcagaga cgccaggagg gtgtctggct ggggcaggga gggtgggaag 2340

gggccgggct gaggacttca ggagccctga aagggatgta actgggcttg gatttttgag 2400

agtgtactct tgggggccat aggaagggtc gatgatcagg aggctgttat agtaacccca 2460

gcaagaggga tgggtcccag gtcccgaggc gggaaagacc ggagagacgt accaggggac 2520

caactggctg gggcgtggga gcagtctgga cggacttcct cccttgcctg ccgggtctcg 2580

ggattggggc tgcgtcgtgg acgaggccct gggaggagga ggtgtgggag ctgtgggcag 2640

ccggtggaga ggaccctggg ctccgaggat ggagacctcg ttgagagggg gtgtgcctcc 2700

ctcctcccca caccattcat tcccaaaccc tcctttactt cccatggaga cagatcaaga 2760

acaggtgctg gggtgggagg ctgggggcgc cccctctctg gccccaggtg accatctgtg 2820

actctgccca caggtccccc tgggcctcga ggtcccccag gacccccagg aacacctgga 2880

tcccaggtaa ggactttgcc attttaggta gggtgtggga ggcggcgggt ggcgggtggc 2940

cttgtctccc taggggagaa gaagcacctt tttaccagca agagtcactc aggaaggagc 3000

cagtcggcct ggccggtgag attttatttt attttgtttt atttattttt aaatttattt 3060

ttgagatgga gtctctctct gtcacccagg ctggagtgca gtggcgccat ctcagctcat 3120

gcaacctcca cctcctggat tcaagtgatt ctcctgcctc agcctccgga ttagccggga 3180

ccacaggcgc acgccaccac gcccagctaa tttttgtatt tttagtaaga gatgggattt 3240

caccatgttg cccaggctgg tctcgaacat ccgacctcaa gtaatccatc cgcctcagcc 3300

tcccaaagtg ctgggattac aggtgtgagc cactgcgccc agccgagatt ttaattttat 3360

tagcagcagt gactctttgc tgagtatatc ctcctcctgt tatgccaggg aggtgttagc 3420

atcactcgca tttcacagat gagctaactg agtcccagag agaccagatg ctgcctgagg 3480

ttgtacagag ggtgtaggca gagttgggcc aggtcacaga atctctgcct cagcccctca 3540

ccttgccctg agaattgccc atggggaggc ggcaccccaa gcctgactca gcccctaacc 3600

ccaggttcgc cctgcctctg cagctgtcag aagtcaggaa gagcaggtac aaaggccctg 3660

aggcagggaa aaggctggca ggtagtgagc aatggagtgt gtggtgtcgg atgaggcaca 3720

gtgggcaggg ccttatctac gggtcccagg tgtgggcttt tgttacagtg gatggaatta 3780

ttgagtttca gggaggtgga ttccatgatc ttatttacat ctttaaaaag ctcctctgag 3840

gggccaggtg agatggctga cgcctgtaat cccagcactt tgggaggcta aggtgggagg 3900

atcgcttgag cccgggagtt tgagaccagc ctgggcaaca tagcaagacc ccatctctat 3960

aaaaaattta aaaattagcc tggtgtggtg gcgtgcgcct gtagtcccag ctacttggga 4020

ggctgagatg ggaggatggc ttgagcccag gagttggagg ctgcagtgag ctctcatggc 4080

accactgccc tccagcctgg gtgacagagc aagaccctgt ctcattaaaa aaaattaatt 4140

aattaattaa atatgtaaaa atagaaagta gactcggcat gtctggccaa tggaataagt 4200

ctggtgagaa gggcagggaa ggctgtagtt tcactccctg gtgtctgtca ggagcagctg 4260

ggaggaccag ggaggcattt cctgagaagg ggaggcttcg ggatgggggg caccaggagt 4320

tccctttgga gagaggggga gtgggcacct catggtcagc tgggtggcga tgtcagggga 4380

cagctggaca cgggcctggg gctcccggga gccgtctggg ctgacatcag cacacgtggg 4440

accctggcca tcgctgttag tatcactagt tctctccaca caccggacca cctgctcccc 4500

ttctctgcct gacagccgta cgtggcattg gcccattttg caggtgagaa gactgagggc 4560

tcagctgggg tccctagtca atagcagctc caggtggctc caggactgga ggcctttgag 4620

tctgcccttc ccgtgccggc cgggccatcc tcctctccct tcctcttctg cagactcaga 4680

gcacagtggg cggggagggg ggtggccaga gggcactgga gacttggcca ggaccggcag 4740

gcctcacacg cttcctttgg ttttctgcag ggcctggctg gagagcgagg cacagtgggg 4800

ccgtccgtaa gtgtgggctg tgcagatggg cctgggccac tcccaggcag aggctctgat 4860

gcaagcccag ggcactgggt ggcgggggtt ggtggggggg ttcagccctg ggtggccctg 4920

ctggagctca ggcaacaggg agggactgag aaaggactca aatcgcctcc tgctgcgggg 4980

tgaggaggga agcgacctcc agagagtgcg ctgacagtcc tgagtgcctg cagccctgga 5040

ttaatgcact gtgtgaccct ggcctttcat tcatgcgctg tgtggcctgg ggccataggt 5100

taacatgccg tgtgacctgg gaagtccctt cttttctctg aacctcccag aaataggctt 5160

agacagcctt cctttaatcg actttcttgg aaacaagaac ttatgagtct cccagcctga 5220

ccaacatggt gaaaccctgt ctctactaaa aatacaaaaa ttagctgagt gtggtggtac 5280

gcatctgcag tcccagccac tctgaaggct gagacagaag aattgcttga acccggaggc 5340

agaggttgca gcgagccagg atcacgccac tgcactgcag cctgggtgac agagtgagac 5400

tccatctcaa aaaaaaaaaa aaaattatga gtatagactc ctgcctctag ggccaaggag 5460

tcaggcgctt ccttgatgcc caagcctcac tgtaggggtg tggggcgggg aggaggagtg 5520

gagctttgct gagcacctac tatgtgctgg ccacagtgga ggtgctctct tcccccacag 5580

cgcatgtaag cttcacagcg accccaagag accatgcttt attatccccg tttgccaaaa 5640

ctggccacta aggctcagag aggttaagta acttgcccaa agccacacag cgaggcagtg 5700

gtggagcctg gatttgaacc agggagtgtc aagctcccaa gccatgcctc ctcccgtgct 5760

tgtctagtga ccaaacacaa cacaggagac acaatgatgg cacttctagg tgccaggctc 5820

taatctaagc tcattgtccc tattgattaa cttaatcctc ctaacaacag atgagataga 5880

tactattact gatcccattt tacagttggg aaaactgagg caccaagaag tacagtgacc 5940

cactcaaggc cacacagtag tagctggaca tttacatgtt actgtgagct gggcatggtg 6000

gtggctcata cctgtaaccc cagtgctttg ggaggccaag gcaggaggat cattttgagg 6060

ccaggagttc aaggccaccc tggacaacat agtgagaccc acccccatct ctataaaaat 6120

attaaaaaat tagctgggtt gtggtggtgc gtgcccatag tcctagttac tcaggaggct 6180

gaggttggag gatcacttga acccaggagg tagaggctgc agtgagccat gatcgcgtca 6240

ctatactcca gcctgggcaa cagagcaaga ccctgtctca aaaaattggc tgggtgcggt 6300

ggctcacgcc tgtaatccca gcactttggg aggccaaggc aggcagatca cctgaagtca 6360

ggagttcgag atgagcctgg ccaacatggt gaaaccccgt ctctactaca aatacaaaaa 6420

ttagccagac atggtggcgc atgcctgtaa tctcagctac tcaggaggct gaggcaggag 6480

aatcgcttga acacaggagg cagagattgc agtgagccga gatcacaaca ctgcacccca 6540

gcctgggtga caagagtgaa actccatctc aaataaataa ataaataaaa tacaataaaa 6600

tatgtgactt tgaatctggt attactggcc ttgtgttgga gacagacagc agcccttcca 6660

ggcaggctac tcaatcctgg aagggcccag gcatgaactt gccggcttcc tagcctgagt 6720

ctcttctgcc ccagaagcag aatgaagcac ttacagccaa ccctggacac ttccctgcca 6780

tgccaggctg agcaggctgt gccccggagc cgtggcctcc cctgctgtgc tcagcccagg 6840

gcaggagtcc cctgggccca gcacccgttt cccaggccct gcctgcccag cctgcccctg 6900

actccctgca ggcccccagg gaactggaga gggtggagag gtgcccccac ctcccgttcc 6960

agtgttgact gtgtgacttg cttcttaggg tgaacctggc gtgaaggggg aagaaggaga 7020

gaaagccgcc actgcagagg taaccatggc tgcccttcac gttccctccc gcctccttgg 7080

ctgtgagcac tgtcacggga gggcaggtgc cccacagatc tgcgctgacc gtcagccagc 7140

cccgagctgg gtgctgggcc accgggtgta cagggcccct gccctgagaa gttcacagcc 7200

ccatgggaac tcagctgagt acagcagtga ttgcaagaga gtgagaaggt ggctgggtgg 7260

ggatgccaca gagggggtga ttggcccttg ggggggcttc ccgggacagg gagaggagtc 7320

gcaaacttgt aggaaagaca gtctccctgg aggaggcgtc aggctgcaga gtccctctca 7380

acggaccggg ttctcccgtg aatggcgtct cggcatgggc gtgcttacaa ggtcaagggt 7440

gctgggggac tgaaggggac agggctgctg gatgtggtag aggatggatg gaggaagagg 7500

gacttgcaaa ctgggaatct gaggagagaa gattggagca agcggaggct ggggccagtg 7560

ggagggggca gccagggcac gggcttggct gtggggacct gcggcttctg ggaggaggag 7620

gggaggctga agctggggta gtggatgaag gggatgaagg gctcatctga ggtccaaagt 7680

caaattcctc ccctcagctg gccctgagct aggtgctggg ccaccaggtg tacagggtcc 7740

ctgccctgag aagttcacag cccagtgagg gacacagctg aggacagcag tgattgccag 7800

agaatgagaa ggttgttgcg tgggggtgcc actgagatgg ggccggcagg gggggccttg 7860

aaagctaaaa gcgaaagtgc ctgagcaggg tttcccttga gtgtgtcaaa gctgggtggg 7920

caaaacagct gggaggccca gggcgggtcc aggggacctt caggaagtcc ttgcaagacc 7980

agagaaaggg aagaggctgg aatcaaggcc gctgtgaggg gtgcaggagg tagtaagagg 8040

ggctgaggca tgagcctgat acactggtca ctcacagcag tactctgaga ggtgactgtg 8100

accccctcca tttcataaca gcaacctgcc tggcctgtaa tcccagcact ttgggaggct 8160

gaggtaggag aatcgcttga gcccatgaga tcaagaccag cctgggaaac ctagcgagac 8220

cccatttctt aaaaaaaaaa aaaaaaaaaa aaagtaatga gacaggcgtg gtagtgtgtg 8280

cttatagtcc cagctacttg ggaggctgag gcaggaggat tgcttgagcc caggaggttg 8340

aggctgcagt gaactacaat cgtgccactg tactccagtc tgggtgacaa agcaagacct 8400
tgtctctaaa acctaaaaaa accccaaaaa acaaaaacaa aaaaaccaac cacctgtggc 8460
ttaggaacat gaggctcagc caggtccctt ggtctcccct gctccttctc tgagttgggg 8520
acacgacact gacatccctg tcccagaccc tgtaattggc tcaacttccc attgtctttg 8580
gtcccctggt gggcagacct ttctcctata acccagtagg atgctttttg gtttcctttt 8640
gtttcatttt aagctaaatt aatctgaaca taagacagat ctgtcatgtt acaccagcaa 8700
aaggcaccca ttctgctgcc aactcccagg gctgggcagg atggggacag tggctttgtc 8760
tcctcatcat cacttttcag agtggcctcg acacctgcct tctcaataag tcagcaccct 8820
gtcttggctg actcaggtgt ctctgtgcac aaggtcctct gcttgtgtgc agaccgggcc 8880
cttgaaatac agactctgcc atcacgccag cctgcctgcc tgcactttga aatggtggct 8940
ttctctgctg tctttcttgc ctggcatggc cacacctacc ctgccccttg cctgctgttt 9000
ggtggcgagc tcttgggcat tcaaggaagg aaacaagcaa agaacttcct accttcgggc 9060
cctgggatag gtgctctgtc ctggttaggg tattttcagg acatgtgggg gaagctgaca 9120
ggcagctatg gtggaaggaa aagctcaggc tggaggctga caggtgtgga tctgcagtct 9180
ggatcggccg ctttgccagg ctgtgtggcc gtggggaggt tgcctgccct ctctgtgttt 9240
caagctcttt gtctgtgaac agggtttgaa gccctgtctg gcacatagtt gatgatgttc 9300
agtctatgct tatttgcagt ggtgagggtg tcgggtggga agaggcaggg gtggggggct 9360
ttgaggacac atacactgtc actgtatcag gatggccctg accccttcct catggccggc 9420
cctgaccctg cctgtttcct ccccgccagg gcgagggggt gcagcagctg agagaggccc 9480
tgaagatcct ggcagagcga gtcctcatcc tggagcacat gattgggatc cacggtgagc 9540
agtgaccagg atcagcgggc tgggaatctc tctcccctcc ttcctctccc aatgctgccc 9600
tcatggctgc tgcctagggt ctctggtctc cctcccttgc ccctagtctg accctccccc 9660
tcccctgctc aaggaccctc agtggctccc tgctgccctt gacagacttc caagtgcatc 9720
agcccagttt cccagcccct ccctagtata gccatggtgg gcagagccct gcccagccat 9780
gttgaggccc ctcccacctc catgcctttg ctcgggccat gcctctgcct ggtgaacact 9840
gactgccact tcgggacctg ctcaaagatc tcggcctctg tgaagccttc tctgaaccct 9900
cctctttgtc cctatggtga cataggggag gcttccattc catgctgggg tttctgtgac 9960
cctctgggag tcccttgtgc agaacagtga atgatacctc tgcagggcct ggagtagagg 10020
cccatcagca tttgctggaa aatgaatgga tgaatgaata ggtgaatgga caggtgggtg 10080
ggtgagtgag tgagtgaacg aatggctggt tgcctggatg aatgtatgca tgggtggatg 10140
catagataga tgaatgaatg aatgaatgaa ttgttgaatg gacaggtgga taggtggatg 10200
agtgagtgga tgggtagatg catggatgaa taaatgaatg ggtggatgga tagatggata 10260
aatggatgga tggatggatg agtggacagg tgggtgggtg aatgagtgaa tggatggatg 10320

gatggatgat ggatgagtga acaggtgggt ggataaatga gtggatggat aagtgggcag 10380

gtgggtgggt aaatgagtga atagatgcat ggatgaatga atgaatgggt ggatgggcgg 10440

gtgagtggac aggtaggtgg gtgaataagt gagtgaatgg atggatggat ggatgataga 10500

tggatgggta aatgagtgaa tgaatggata gatgcataga taaatgaatg actgagtgga 10560

tggatggatg gatggatgga tggatggatg gatagatgca tagatgaatc aatgagtgga 10620

taggtgggtg ggtggataag tgagtggatg gatgggtgga tggatggatg gatggatgga 10680

tggatggatg gatggatgga tggatggacg ggcaggtaaa tgagtgaatg aatggataga 10740

tgcatagata aatgagtgac tgagtgggtg gatgaatggg tgaatggatg aatgggtgga 10800

tacacagatg gatgaatggg tcaatggaca ggtggatggg tgaatgagtg aatgaatgga 10860

tggatgcatg gatccatgaa tgaatgaatg cataaggggc tgaaagcctg ctgctgcctt 10920

gcttctccac gctgggcaag agcatctctc cgtggccaca tatcatgatc aaatgtaccc 10980

ccaagtgttc ctaaggctct acctcgagtc caccctcctg ctctccttcc agatcccctg 11040

gcctccccag aggaggttct ggccaggatg ctgccctgag agccaacctc aagatgaaga 11100

ggggtggcgc ccaacccgat ggggtccttg ctgccctgct tgggcccgac cctggacaga 11160

agagcgtgga ccaggccagc agcaggaagt gagagcccac tgctccagga caccctatcc 11220

tggctagaga cccagcccca gaggcctgag ccgccgctgt ttcctaaaga tgcccccagg 11280

ggaactgggc tccaggcatg gatgattgtg aggacatggg gggctttggg gacagataat 11340

gtctccaggg gcagggtctg gaggggccaa caccctcatc agagccctcc tctggcctgt 11400

cccctcccct acccccactc ccggctggag acggggtctg ggtgggctgg gtgctgggaa 11460

tgagaataat cctaataccc atcatttatt gagtccctgc tgtaactggc cctgtccagg 11520

gaactttcgt tacgttgtct cattatttaa cccttaggag gtaagcttat tatccccact 11580

tcacaagggg accgaggctc agacggtaga aataaccctc tcttggccac cagatcacgg 11640

gtggcagggg caggatttga acccaggacc ctcagttcct gaagccatgt tctctcgacc 11700

ctgccagctt cccctcttcg agagaaaact cagacaggga gggcgcaggg ccagaacggc 11760

tccttaaggc aggcagggct gcggggaggc tgggccagcc agcctgaggg ggtaggcagg 11820

gtcctgcagg gcctggggct tcccttcctc atcccccttc cccactggct gggggagcag 11880

cctggactgc agctctcaga ggccagggag accctgaata aatcactgcc agccatacgg 11940

acttgaggac cttgagagag aagcattggg ggtgcaagtg tccaccaaac cagataggca 12000

tccctggcca ctgcctccca gtcttgctga gcccacccgc tctctctggg tccatcccat 12060

ccctgagcca ggggaggcag catttcatgg gcatgtctat cagaggtggg acttgcagcc 12120

tctgccccac gagtttgtct ctcagtggac tctataggtt tgctactttt gcatgacaca 12180

gcaagcccag tgtccccttt gcaagctgca gagagggaaa cagagtccca ggcctgctgg 12240

gaagagatgc tcctgcctcc caccttccag agttgggggc ctggcaggct ccatggcagg 12300

caccaggtcc ctagcagccc agaacagctc tgactggagc cctcaaggcc tctggggcca 12360

gggtctccgt gatcagctca gccctggtgt tcctctcttg ctgggctggg acctgggaca 12420

cagccacggc agcaaactca gagaattgaa ggtgctggcg ccaccctggg gcactctgtc 12480

ttcacagcag gagtgactgt ctccagtgct cttggtactc ctgtttggct gtggcctggt 12540

ccctctgggc cctgggatct tcttctggcc cttgaggcag gtctggagag gcagtctctg 12600

tcttcatgga ggggtggtca gaggcgggcg ggaccaccag cctgtagcgt tattattata 12660

tgtgacaata aaggtgctct ccccactgtg ggtctgtttg tgtggtgaca gctctgcagg 12720

aggctggtct gcatcttacc ggccaggtgc cagggaagct gtgagcagct ccagccaggc 12780

ccggggtgac cacagggcct ctgtggcagg gaggtccagc tctgccctca ggacctccag 12840

ggagggggct gggagtgaca cacgataggc aagccccgga ttggggctta acctgcaagg 12900

gctcttggct tcacccagca aagaattcaa gggcaagctg gaggcaagag aaacagcttt 12960

attggagggg caggactaca gctccatgac tgctcgtgca gagcagggct tctccccagg 13020

cagagaatag cagctcaggg cagttctgca ccatatttat acctactttt aattgcatgc 13080

agattaaggg ctggtttatg cagaaatttc tagggaaagg gtggttattt ccgggtcatc 13140

aggtctttgc catggaaaag ggcagtggct cctgagtgtt gccatggtga tggcaaactg 13200

acatggcaca ctggtgggcg tgtccaatgg aaagatgctt ccgctctgtc cctgttttgg 13260

ctagtcctca atttggtcct gtgtctgagc cctacctcca gagtcaagat ctgtttctta 13320

cctccagagg gtgagggaag ccccttcaga ggccacctca cccctccacc caaccacact 13380

gtctataaac tgggcctggc ccccagctct gcaagaaggt gagttaagtt ggggcggcct 13440

cagggctctt ggaggagcaa ccctgatcct gctccatgct cctgtccagg tctggggtca 13500

gtgacatatc agagacccca gggagaccca tataccctga tgacaagtcc caggtacaac 13560

acagagaccc aaaccactgc tgaatcaccc tgtgaccctt gtaggtatta agctcttgcc 13620

ctgtgtatta tgtctgttct cactctgcca ataaagatat acccgagact gggtaattta 13680

tggtaattta taaagaaaaa gatgtttaat ggacttacag ttccatgtgg ctggggaggc 13740

ttcacaatca tggtggaagg ggaaaggcac gtcttgcatg gcagcaggca agagagagaa 13800

tgagaaccaa gcaaaagggg tttcccctta taaaaccatc agatctcgtg agacttactt 13860

attcactacc atgagaacag tatggaggaa ccgcccccat gattaaatta tctcccaaca 13920

ggtccctccc acaacacatg ggaattatgg gagctacaat tcaacatgag atctgggtga 13980

ggacacagcc aaaccatatc accctgtgtc cagtgcccag tgctcagcta cagacagaac 14040

cctgcctgcc tctgcctgga gcttgaaatc ctatagagag ggtgagcatt gaacaccaca 14100

ccaggccagg catggtggat cacacctgta atcccagcac ttagagagga caaagtggga 14160

agattgcttg aggtcaggaa ttcaagacca gcctggacaa catagctaga ccctgttgct 14220

acaagtgttt tttttttttt ttttaaagtt agcctggcat ggtggtggct tgtccctgta 14280

gtcccagata cttgggaggc tgaagtggga cgatcatttg aacccaggag ttggaggctg 14340

cagtgagcca tgattccgcc attgcactct agcctgggca acagagcgag atccggtctc 14400

taaaaattaa acataaaaaa acaccacagc aatcatgtgg aggaaggaga ttccaggctg 14460

ctctgagagt ccaggaccca gagaccaaaa gggcagggag agcagggaaa cagaaagaga 14520

gaaagaggga gggaagggaa aagagagaca gagcaagagg cgagagggag agagaaacaa 14580

gagacagaga cagagagatg gcctgccaga gcttcatctg tctttatttg ctggtgaata 14640

ggcaacacgt tccttaagcc ggagcctctt cttccaagcc ccattggctc tgggatactt 14700

cctccagcca caggtcctgg gcccagcagg cacagctcat tcagcaaaca gggagacccg 14760

gggtcgggca ggtgcctgaa gtcaaacctt ggcaggaacc actgggcaac tgtcccaggc 14820

agggagtgtg agtaggttta ggctggagaa atgtcctcgc cccttttatt ccttttccca 14880

gggcaggagt tacaaaacca cgcatctttc cttccttcct cctttccttc atgtaacaaa 14940

tcttttattg atcaccttaa ttgagccagg ccctgagaac tttccatgaa caaaagacag 15000

gaatctctgc cctcaatgaa ccaacgcttc aaggggaaga agtcaaaata ggcaataaac 15060

agcctggaca acatagtgag accccatctc taaaaaaaaa aaaattagcc agatgtggtg 15120

gcatgcacct gttgtcccag ccacttggag gctgaggcag gaggatcact tgagcttggg 15180

aggtcaaggc tgcagtgagc tgagatcaca ccactgcatt ccagcctggg caacagagca 15240

agaccccgtc tcaaaaacaa aacaaaacaa aaccaaaata ggtggtaagt gtatgtattt 15300

ttaaagccaa taagatgata ggctagaagc ctagccacgt ccagatgatc agcaatatgg 15360

aagcgctaag gactgggagc tgacaattag tcttattttt taatttttaa aacttatttt 15420

aattttgtgt gtgtgtgtga aaaaacacct cctctcctcc ttggggaagc agaccagcag 15480

gcattcattc ccccagaccc agtggcagag atgggatcct aagagagaga tttgcctgca 15540

ttacctagga caccaaaacg tcctgcccca gggctgcccc cacttccccc aaggtcttcc 15600

ccttcctttt gtcttaaagg cagactccac gcccaatgac acctttgtct ttcaatcaag 15660

gggttgctga gtttggaggg tgtgaactga gcttagacgc tccaggtggg caccctcagg 15720

tgggcacacg aagccccccg tggtgcaagg tggaagctag tgagggaagc agaagggctg 15780

gaggatgaca ggggctggct ctcccggctg tcaccttcca gcacaagact ctgacagtca 15840

caattccaac gtcaaacctg gccttccagg tccttggaaa aagatatctg tcaaagtagg 15900

aagatagagc ctgtgttact taacagctgg ttagctcaat gtataacttt ttttctctct 15960

ctctctctgt cgcccaggct ggagtgcagt ggcacgatct cggctcactg caacctccgc 16020

ctcccagttc aaacaattct cttgcttcag cctcctgagt agctgggatt acaggtgcct 16080

gccgccacac ccagctattt tttttttcct gtatttttag tagggacggg gtttcaccat 16140

gttgtctagg ctggtctcga actcctgacc tcaggtgatc cacccatctc agcctcccaa 16200

agggctggga ttacaggtgt gagccaccgc acccagcctc gatgtgtaac ttttaaatag 16260

actcatggga tgccttcatt tttcctctgg tccctggatc ccgctatggg agagcctgga 16320

tgggaccagc agagtctagg agggtaggga aagggctgta tgaggaatgt ttggggaaaa 16380

acaacttcca agctcccgcc agcagggtgg acgggccagc ccggagtgtg ttcgatggag 16440

ccagggtagc agatactgtg ggtcccagag aaggtctgag acagagctga gggagaaggt 16500

ctcccccaag gggctgggtg tgagttggtc cttaatatgg tgaggacaga tccagtgcta 16560

aacagtgaaa catgtcacct cctttaacgt cacagccacc tctctcattt tggggtgagt 16620

cagctgagcc ccagagaggt tacatagctt cctcaaggtc acacaggcgg ttggcggtga 16680

agctgagttc cagatcaggt ctgtgggatt tgggaagcca tgctctgctc tgcccccgag 16740

aaagcggttc tggaaggaaa aaagagagtt cacataaacc cagggatggc gctgccgaag 16800

aggccaagtg tgagcaaagg ctgggggcgg ataaagggca gaggagattt gggtgggggt 16860

ggaacgaatc tcccaggaca ctgaagcacc tgccagggtg gggatctccg aggaccgtga 16920

tgctggaggg agaggaaact gaccactgag ggtctcagtc tggcctctcc ttgccatctg 16980

gccgtagaat gacctttctg aagtgcaagc ctggcgttgc tcctccctca ctcatgaatc 17040

tgctgtggct cccctgttcc tggcctcaag cctgagctct gccccctggt gctgtcacct 17100

gggtccctgt cctgaccacc ttctgcacac atagtggctg cagccccagg agtcagccag 17160

gaggtctgcc cattgtccgg cctcatctct gtccagctgt ggaggctggc tccccccgcg 17220

caaagcagtg ctaagcgtct gcaggaaatg ggacagcgct gttgggggaa gggcctcaaa 17280

gccccagggg gagcccagct cttggccttg tgtgaccccc tcaaatcttc acctcctctc 17340

acccctgcag ccaaggagac agggcgggcc catctgatcc tctgcccagc ttcaggaagg 17400

ttcttgcatg catgtgtgga caccgccgct tgcacttcca agctgtccac agggcccaag 17460

actcggagta ggaagcaaag caccccaggc tcccagctag gacgctgcag ggctggactg 17520

tggtcatcct cccctgccca agattgcctt ccctcctccc tctccacagc ccctggggaa 17580

aggggactca ccagggtcag ctcagtcctg gcaggagcct ccaggtcccc tcctggctca 17640

cagctgagct ttgagggcca gatctgggga caggctggct agagccctca ctgcctgggt 17700

gcccagaact cccagtctga ggccacctgc ctgggcacta aggagggaga attagcatag 17760

gcccctgctc acctcgcccc gccctgctcc accctgtctc cactttgcct ggcccagaca 17820

tggctgtctc gaatttcctg cctgagtctc ctctgctctc ctgcgcttcc tgcctctgcc 17880

tccggccgct gtttcctgcg cataccccct tctgtggccc ggggtccaga tcaaacctga 17940

catttcttct gaaatgccaa ctggctctag gccctgctat acatgtgcgt gtgcatatgc 18000

aagtgtgtgc atgcataaga atgagtgtgt tcatgtgtgt gcatgttctg tgtgtacaca 18060

tatgtgcacg tgtgtgcatg tgtaagagtg tacatgcatg agaatgtgtg tgtgcgtgtc 18120

cagtgcgtat acacatatgt gcacgtgtgt gtgcttgaat gtatgtgtgt gggtattcgt 18180

atctgctacg gtgtgtacac acagtgtgga ggtggctcgg gctgggctga acctcagagg 18240

ttcccacgaa ggcacgctct tcttagcctg gtcctggcat gtcctgaaga cccacgaggg 18300

gcaactgtcc taccggaatg gcagaaacag cccgactctg gctttgggct cagccaaggg 18360

tggtcaaggt gccattgggg tcccccagct tcactcgtca cctccctaaa tccaaagcgc 18420

accctcccaa ctctgtctct tccagtcggc tgtgagcgtc ctgtgtgcca ggtcacgtct 18480

gcgtgcccag ctcaggccag gtgcagagca gatgaatgac tgcaaaggaa tcactggcac 18540

aaggcaggac attcacggtg ggggcgtggc ttcttccctg acactggcac atagcaggac 18600

attcacgatg ggggcgtggc ttcctccctg acactggcac atggaggaca ttcacgatgg 18660

gggtgtggct tcttccctga cactggcaca aggcgggaca ttcacgacgg gggcgtggct 18720

tcttccctga cactggtaca cggtgggaca ttcacgatgg gggcgtggct tcttccctga 18780

ccctaagagg ccatgttgga ggttatgggc aggggcaggt gggagccaag actggcctac 18840

atcagaacag cagccccctc cccactccaa ccctccacgc ctgcagtttc caccaccccc 18900

gagatttctg gaacacctct ctcccagacc ggctttatgg attatataaa aaaagaattt 18960

catgaggttt tcagttaatt gaattccagg gagatttatt cccaatgtta ttaataaatt 19020

ctaatgccac ctataacttt caattgccag ttaattaact tgcaatgggt gcattgtaaa 19080

aggcaaatta aatacttttt caggcagggg ctggcaaatt taatgagctg atgtgtccca 19140

agggagacgg ccgggctcac acatcccatc aaatactcct cccatcagca gatgagagtg 19200

tgatctgggt gagtgacaac tggacctccg catcctcatc tgtaaaaagg gatcacaaaa 19260

cctgcctcca ggaccctcag atacctggag tggatgctgt gttttattca tacacaaata 19320

caacgaaggg gccctggggc ggtgaacttt atgtgtcaac ttgactgagc catggggtgc 19380

ccagacaact ggtcaaacac gcctgccctg cccagcccag gtcaattcat ctggtcagtc 19440

tacaggggat tgggggcagg ccagcctctg cctccttctg agtagagaac aaggatggca 19500

gtaaggacgg cagggaagac aaggagcagg ggatcccagg gaggaccacc gggctcactg 19560

gagggaagag agtcctcccc taggaaggga cacagctgag gagtctgaga gatttagtag 19620

gagttgtctg ggtgaaggag aaggcagggc aagttgggga ggcacgagca cgtgcaaagg 19680

cccagtggta tgagaagggg aaaagcactg gaaattgttg gaaatgctct agggtggagc 19740

cggcagggga aatatgataa tgtgggctta ttacaaggct acagggatct cagtggaccc 19800

caaggacagg agctggagtc agaacaggtc ccaagagagt agtactggat ctgagttagt 19860

cccaaggcct cagtgtcagc aggagattac tgattcccat gggctctgcc tgttgcctgt 19920

ttgtccctct tcttgtggca atgtgacatt ttgtcttggc ctcatagctt cagcatgctc 19980

atgacttctg cttcttctga tggcttgcca tgacctctca ctggctatcc ttcaacagca 20040

gtcagctttg cagtgacaca atattcaaac tgggtgtcaa gcaccttcat tgtctcccca 20100

gtgacccagc acgcaggctc cctaaaggta gagttctcag gcagtacctg gtgctgtaac 20160

aaatatccca ttgacctggg ggcgacattt ctcacagctc taaaggatag gaagtccaag 20220

atcaaggtgc cagcagatcc ggttcctggt gagggctctc tttctggttt gcagatggcc 20280

gccttcttgc tgtgtcttca catggtgggg agagagagag gaagcaagct ctgttgtgtc 20340

tcttcttata aggacattaa taccattatg agggccccac cctcatgaac tcatataaac 20400

ctaatcacct ttcgatggcc ccacctccaa atgtcatcac aatggggatt caggctttaa 20460

catacaaatt ttaggggcac ataaacaagt ggtctacagc agtagaatgc cttgtccttg 20520

taggcagaca aggacagacc tcggtctggt ctcccctagg cctagacaca gatacttgat 20580

aaggagggag gattctaggg aaccaaggtc agaagcctca tagagcagga agtatgggtt 20640

ttgtggcctg ttgatggcag aagcttctat cttaggctta gagtctccag ctaggctctt 20700

gctgggaaaa cagtgcagtc tttgctttct tttatatcag agctgcattt tcaaacgtaa 20760

ccatcccaca cccactctcc agctattcga ggactttgat gacagccctc cagtttctca 20820

ggatgtaaca gcagcaaatc actgtgcact ttctcacttg ctgcccatga tcccccatgc 20880

tccaaccacc gggcaggccc atattgggca tgaaaggatc ttggccttaa tttttctttt 20940

gcctcgtggc gataaaggga gcattttgtt tttttcaaaa gacaaaacca aaaggtgcat 21000

cttaattagg gaacaggtgc agtgagacca gactgaactc tgccaggcag cagcctttca 21060

ctttgtgcgc acaatgcttt taatgcagcc ccgttgccat ggcgattacc atgtctcagc 21120

ttactgaggt ggcgttgcaa acctgtaggg aagaaatgga ttattcaata aatgatgccg 21180

ggacatttgg ttagctattt ggaaggaaat aaaattagct ctttccttca caccatacat 21240

taatataaat tccagaggac ctaaagaatt aaatgtaaga aagggaaatc attaaaaagc 21300

aaacaaatgt ttgatcttgg gggtacatgg aaacatttta aacctaaaag cagtagaaaa 21360

aataacaaag attaaggtag aatttaatac ataaaattta aatctccttg ttttaaaaat 21420

tatttagtta attttattgg gagactacaa aaaagaataa aatggttgca agaaatatga 21480

caaaagatta atacctctaa tatgtaaaaa gtaattacaa atcaataaaa atgaacagtc 21540

caatagaaaa atagtgaatg agaaatgccc aaaagatgaa ataaaaatat gtaataaaat 21600

atttttaaat attcagcttc attggtcatc aaagaaatgc ccattaaaac atcaatgaga 21660

ttttctccta tagcttatca aagaacgttt aaatataatt atagcattca atctcattga 21720

gtggagtaaa attgagtatt cctattcatc tcacatgtga aaatacaagt tggtataaaa 21780

tttctaggaa aaaaattaag tttatgccac aagagatttt aaaatgttca cactattcag 21840

ctggtaattt cactccgaag atatcctaag gtgctgtcca ggattgaagc caaaaatgta 21900

gatatgataa tattcattat aagtatttaa tagaagttta taatttacta aaacacgtgg 21960

gcatggttaa ataaattatg atacataaat atgatgaact attgtgcagc cattaaaata 22020

atattttgaa taatacttaa tggcatggaa atatgtctat gacataatat taagcggaaa 22080
atagtatacc atttgggttt cttagctgac agcaatagaa acccactctg gctgtctgga 22140
taaagcagaa aaggaattca ttggaagaaa attgtagggc ttggaatcaa caggaacaca 22200
ggagaactaa actgagaaag aaagaaaaat agagaaagaa atagaggaag agagagaaag 22260
caaggaaata tagagaaaag aaagagagaa agaagaatga aggaaagaac caaggaagga 22320
aggaaaggag caaaaaggca aaacagggca aacatccatc aacagctata gttacggtac 22380
caaagtagac atatggtatg atcccaagtc aaggcaaaat tggctattca gaaataccaa 22440
attcatcccg gaatctcagt gcttcaaccc ttgcaatgtt tccttcttac tcacacaaag 22500
taagtactta ccaggttggg cagcctgtca ccatcttgta gcccagccat tgaaagaacg 22560
taggctctga gactgtcaca acagggaaga cagagatgga ggagtctcca tgcgagtgat 22620
gctgtcattt ctgttcacag tccattgggc agcactagtc atatggcccc caacctcact 22680
gcaatggagg ctgggataaa gaaggcagtg agccagttga ggctgcaatt tgaaacacat 22740
ggtacactta atttaggata atttgaggaa gatttaattt caaggagact attttcaaag 22800
gtgtgggtgg gctgcagaag aaccagaagg gagaatggga gaatgtgatg agctagggct 22860
attaacagcc caaagagacg agggtcagat tggattttca aaatctcaac tatatgctgt 22920
ttataagcag cacattttga ataaaagatc actatagaag atttgaagca tacagttatc 22980
cagcttcacc caatggacaa tatatagaac acacgcctac caattgcata atatgtgctc 23040
ttttaaagtg tacatggaac attttccaaa atagaccata tgcctagcca taaataaatc 23100
tcaacaaatt tcaaatgatt aaaataataa aatatgtttt ctgactacat tgaaattaaa 23160
ctagaaacta ataacaaaaa aaaatagaaa atatcccaaa cccttggaaa taatccacat 23220
tcttttagat aattcaatat gtcaaagaag aaatcacaac ataaattata aattcatgga 23280
aataaaaatg taacgtataa aatttgtggg atgcagttag cacagctctt ctaagaatat 23340
ttacaacttt aaatgaatat gttagaaaag aaaaaaggta gaaaatgaat gatctatgct 23400
ttaatctcaa gatgctaaag aaaaaacagc atatttagaa aaaggagaag gaaaactaaa 23460
ggaaataata aagataatgg cagaaatgaa taaaatcaaa agcaaatgta tagagaaaat 23520
ttaaaagcca cacatcgatt aagattaata aaactgataa atacttagca ggctggatca 23580
agaaaaagaa atgagaaaac ataaattagc catgccagga gacatcacta cagatcccca 23640
agactttcat gatacagcaa aaggatattg tcaacagctt tatgccaata atttaaaaat 23700
ttagatgaaa tggccgggtg cagcagctca cacctgtaat cccagcactt tggaaagcca 23760
aggtgggcag atcacaaggt caggagatcg agaccatcct ggctaacatg gtgaaacccc 23820
gtctctacta aaaatacaaa aaattagcca ggcatgatgg cgggcccctg tagtcccagc 23880
tactcgggag gctgaggcag gagaatggca tgaacccagg aggcagagct tgcagtgagc 23940

cgagatcatg ccactgcact ccagcctggg cgacagagtg agactctgtc tcaaaaaaaa 24000

aaaaaaaatt agatgaaatg gataaattca ttgaaaaata caatgtacta aaatcattat 24060

aagaaaaaat aaaaaattga tatactttta tatctgctaa agaaattgaa ttcataatta 24120

gaaacattcc cacaaagaaa actccagcct cagattgctt aagtgatgaa ttctaccaaa 24180

cattcaagga aactcataga gacagacaga cagatagtag attagtggtt gccagcggct 24240

gggaagtatt tgcttattct agacatgtta tatcaatgga atcatacaat atgtggtctt 24300

ttgtgtctgg cttctttcat ttagcatatt ttaaagattc acccatgtta aaaacaaata 24360

aataaaattt tattcatttt tatggctgca taatattcta ccatatagat aaaccagatt 24420

ttgtttatcc attcctcagt tgattgacgt ttgggctctt tctacttttt gactattatg 24480

aataatgcta ctatgtacat ttttgtacac gtttttgtgt tgacacatgt ttttgctttt 24540

catggatagc tatcttagag tggaatggca gggccatttg gtaactctct gtttagcttt 24600

ttgaaacact gccagactat tttccaaagt ggctgcacca ttttacaatt ccaccatcaa 24660

tgtatgagga ccacaacctc tctatattct catcaacact tgttattgcc tttttaaatt 24720

ttaggcaaat tagtgaacat gaagtggtag gtcactgttt tggtttgcat tttcctaatg 24780

actaagaata ctgaacatct tttcatgact tattggccat tgtttatctt tgaagaaatg 24840

tctcttaaaa tcctttgccc acaacagacc tctacctccc accatataaa aaagtgaact 24900

caaaatagat tatacaccta aatgtgggag ctaaaactat aaaacgcttg gatgaaaaca 24960

ttgatataaa tcttggtgac cctggattag gcaacttgtg gattgaagtt ctaattggta 25020

tgtttgcatt tttactgttg agttgtaaca gttctttgta tattcttgat aaaaagtccc 25080

ttatcaggta tatgatttgt aaacattttc ttccattctc tcattctgag ggttgtcttt 25140

ttactctttg atagtacctt tagagcacaa aggttttatt tttgagaaac tcctatttat 25200

ctaatttttc ttttctttct tacattttgg atgtcacatc aaataaacca ttgcttaatc 25260

caaggtcata aaaatttaca cctatggttt cttctaaagg ttttgtagtt gaagctctta 25320

catgtaggtc tataatatat tttgagatag tttttacatg gtgggaagta ggagcctgct 25380

gtgggtggaa ttgcgcctcc atcaaaagat atattagagt tccagctccc aatacctgta 25440

aatattacct tgtttggaaa tagggtcttt gtagatgtaa acaagatagg attggaccta 25500

tatccaatga ctggtgtcct tataagaaca ccatgtacag ggatatagag atacacaaga 25560

agaatgccat gtgccaatgg aggcagatat aagagtaaag tgtctgcaaa ccaaggattg 25620

caaagtatat acaagccaga agctaggaag aagcaaggaa ggatcctccc caagaacctt 25680

cagagagagc aaggccctgt tatgacaccc taattttgga cttctagcat ccagaactgt 25740

gtgacaatag atttctttgc tcttaagcca cacggtttag agtaatttgt tatggcagtg 25800

aggaaactaa taggggatcc aacttcattc tttttcaagt ggatatacaa ttgtctcagt 25860

cttttgtttg aagagactat tctttcccca ttgaatagtc ttgacaccct tgttgaatat 25920

cagctgaccg taagtgtaag tgtttatagc tggactccaa ttagattcta ttgagctaca 25980
tgttttgttc ttataccagc atgacattgt cttgagtatg gtaccttagc agttttgaaa 26040
ttaggaagta tacatcttcc aacttttgtt tttctttgtc aagattgttg tggctagtcc 26100
cagttccttg tatttccata ataatcttgg cattagtttg tcaatttcta ccaaaatagc 26160
cagctgaaat tttgatagcg attgatcaat ttggggagta ttgctatctt aacaatatta 26220
actctttcaa tccatgaaca tgagatgtct ttccatttat gtattttatt taattacttt 26280
cagcaatgtt ttataggttc ccacataaaa gtcttgcact acttttgtta aacttaatcc 26340
taagtatttc cttctttttg atgccactgt aaatggattt gctaaaactt tttcaggtgg 26400
tcacagtggc tcacacctgt gatcccagca gtttgggagg ccaggatggg aggaacactt 26460
gaggccagga gtttcagacc agcctgggca atatagtgag atccgtctcc ataaaaaatt 26520
taaaaattag ctaggtgagg tggcaaatgc cggtagtcct agctacttgg gaggatgagg 26580
ttcaaggttt gcttcagctc atgagtttga ggctgcagtg ggctatgatt gaaccactgc 26640
actccagcct gggcagcaga gtgagaccct gtctccaaaa aaaaaaaaaa ataaacaaaa 26700
taaaatgaaa tattttcaga ttgtttattg ctagtctata gaagtaaact tgaattttgt 26760
atgccaacct tatatcctga aactgctgaa cttgtttatt agttctaata gtgtgtgtac 26820
atattcttta agatttgtta tatacaagtt cttgtcatct gagaatagaa atagttttat 26880
ttctcttttt ccaatctgga tggctttatt tatttttctt gcctaattac tctggctaga 26940
atctctagta caatgctgaa tagagatagt gagagaggac atcctagttg tgttcttgat 27000
tttagggtag aaacattcat actttctcca ttaaatatga tgttagctat gggttttttg 27060
tagatgccct ttttcaggtt aaagaggttc ccttatattc ttagtttatt gttttgtttt 27120
gttttgtttt tatcatgaca gtgtgttaga ttttatcaaa tgctttttct gcaaattttg 27180
agatggtcct gtgtgttttg tcctttcttc tattaatatg gtgtattact ttgattgatt 27240
tcagatgtta aaccaatctt gcattcttgg gatgaatccc acctggtggt gatacataat 27300
cgtcttatat gttcctgaat ttggttttcc agtattttat tgaggatttc catctatgtt 27360
cataatggat actggttcat aaatttcctt tctggtgatg tctttgtcta gttttattat 27420
cagggtaata ctggcatcat agaatgagtt ggaaaatgtt ctattagctc atattttctg 27480
gaggttttgt gaagaatttg agtttattat ttgtgttagt ccattttcac agtgctataa 27540
agaactacct gagactgggt aatttatgaa gaggtttaat tgactctcag ttccacaggc 27600
ttaccaggaa gcatgactag gaggaggcct caggaaactt atagtcatga tggaaggcaa 27660
aggagaaaca agcaccttct tcacatggtg gcaggagaga gagtgaggga agcaggaagt 27720
gccacacaat tttttttttt tttcaagacg gagtctcact ctgcaacctc cgcctcctgg 27780
gttcaagcga ttctcctgcc tcagcctact gagtagctgg gattccaggc acatgcccag 27840

ctaattttta tatttttagt agagacacgg tttcaccatg ttgatcaggc tggtctcaat 27900

ctcctgacct tgtgatctgc cggtctcagc ctcccaaagt gctgggatta caggtgtgag 27960

ccactgcacc tggttgagaa ctcactcaat atcatgagaa cggcaatggg gaaatctgcc 28020

ctcatgatcc agttacctcc tatcaggccc ctcctccaat tcaacgtgag atttgggtgg 28080

ggacacaaat ccaaaccata acattctctg catttttggt agaattcgcc agtgaatcca 28140

catgagcctg tgtttctatt ctctatttta tctacttcac tccaacagat acaggagcta 28200

gaaagaaatt atttaggaag atagtgaggg taagagaatc ctagtgtagc aggacgagcc 28260

acagacaaaa ctcctcagac actgagttaa ataaggaagg ggtttattca gccgggggca 28320

ttggcaagac tcctgtctca agagctgagc tccctgagtg agcaattcct gtccctttta 28380

agggcttaca actctaaggg ggtccacatg agagggttgt gatcgattga gcaagcaggg 28440

ggtacgtgac taggggctgc atgtaccagt aatcagaacg aaacagaaca ggacagggat 28500

ttttacaatg attttccata caatgtctgg aatctataga taacataact ggttaggtcg 28560

gggtcgatct ttaactatca ggcccagagc atggtgccag gctgtcttcc tgtggatttc 28620

acttctgcct ttcagttttt acttctttct ttggaggcag aaattaggca taagacaatt 28680

atgaggggtg gtctcttccc ttactaggta aggtttcctt ttaataaaaa gcagccccca 28740

aataatttct tttctaacaa aaagcagcct gaaaaatcaa gcttcaagca tagataagca 28800

agctcaaagc gtgcataggt aaatgccaac agctgtgcca atagaaaagg gacacctgga 28860

agctaggtat attcaacatg gaggttcctt cttccctttt ctttgttgcc acatgtgtag 28920

taaaaaagca ggcaacatgg cattggccaa gtagagaccc catctgcata acaaaagatt 28980

agggtgggat ggccagcttc ttcgcacact atgcaaatgg gacacctggt ctgaccagtc 29040

tctcatgtcc tatgtaaatc agacactgcc tcctcaagct cgtctataaa accccatgca 29100

tttcaccatg aagcccaaag accctacttg ggagtccctc tccctctgca gaagagagag 29160

cttttctctg cctttcactt attaaacctc tgctcttaaa ctcattcctt gtgtgtgttt 29220

gtgtattcag tttccctggt gtgaggcaat gaacctcggg tattttcccc agacaaacca 29280

tgccccttca caatctttat tatttccttt cttcaacttg ttttaggttt agtttagact 29340

tctttttcaa gttacaggaa gtggaagttt aagtgactga tttagatcct tattcttttt 29400

catataggtg attacagcta taaattttcc tctaagcact gctttatggc tgcattctac 29460

aagttttggt gagttgtttc atttttattt aaagcatgct ctaatttcct tgtggtatct 29520

tctttgatcc attggttact taagagtgtg atgtttaatt tccacatgtt cctgaatttc 29580

ccaatttcct tctgttatgc attctcatga gattagaaaa catactttgt atgattccaa 29640

tctttttaaa cttactgagt acttttttat ggcctaacat atggtttaca gtgcagaatg 29700

ttccatgtac acttgagaag aacatgtatt ctgatgttgt tgactggagt gttctgtagg 29760

tgtctgttag gttccactga ataatagcgt tcctttaatt cttaagacgt ggtttcctct 29820

agtttttgca catattcata acagtgtact tgaagacttt gcctagtaat tccagtgtct 29880
ggacttcctc agagacagtt tcctgtgaat ggaccatact cttctgtttc tctgcatgtc 29940
tcataatttt ttgttgttga atattagaca tttaaaataa tataatgtag caaatttgga 30000
aatcagattc cctgaccccc caccacaaag agtttgttgt tgctattgct gtttgtttcc 30060
ttagtgattt ttctggatta attctataaa gtctgtgttc tttttcatgt tttgctgcta 30120
aaattcctgt tagcttagta gtcaactaat gattgcacag gctttttttt tttttttttt 30180
tttgacagag tcttgctctg tcatccaggc tggagtgcag tggcgtgatc ttggctccct 30240
gcagcctcta cttcctgggt ttaagcaatt ctcgtgcctc agcctcccaa gtggctgaga 30300
ctacaggcac acaccaccat gcctggctaa tttttgtatt tttggtagag atggggtttt 30360
gcgtgttggc caggctggtc tcaaactcct ggcctcaagt gatccaccca cctcggcctc 30420
ccaaagggct gggattacag gtgtgaacca ctgtgcccag cccagatatt tctttaaatc 30480
ccttaacccc ataagtgtcc cagcctttgc tgaggaacac tgtgtgtgtg tggagcctga 30540
cttcaatgct ttatcaagca gtttgcagct ctgccgtagc cttcatttcc ttcttgtgca 30600
gagcctcaag gtcagccaga ggtgagacat cagggtctta tcaggtcttt ctgtgcatgt 30660
gcactgctct gcacatgcat gaagcctcta tcctatcatc ccaggaatgt atcgaaactt 30720
ttaaaacccc caaaggaaaa aaaaacccaa aggataactc atttcccagt ttttcctttc 30780
atgattttac atagcctatt gaccccaaat ggtattgcca cataggcagc tacgatgttg 30840
aacagttact gctgattgtt tttgacaagt gccctgaggc cagggctggt cacacagagt 30900
aagctctgag tcaggtcaga taaagacaat ccctgggaac agagcttttc aggcagcttc 30960
cagacaggtc aaatagtggc cattttctgg ggatagagct ttttgtgggc tggtttcgca 31020
gacatcatag ttgcaaggct gttggttttc atgcctgcaa cagaactgta aagagggctg 31080
tgggaataag acaagttaaa acttcacaaa gcttgctgtt cttactgcaa tacggcctta 31140
ttttattttt tttagagact gggtctcact ctgtctccca ggctggaatg cagtagcatg 31200
atcataactc actgcaacct tgaactccca gcccaagtga tcctcctgcc tcaccctccc 31260
cagtagctaa gactacagcc atgtaccact gcaccctgat attttttaaa aaatatatag 31320
tatttgtaag gatggggtgt cactgtgttg cccgggctgg cctcgaacgc ctggcctcaa 31380
gcgatcctcc tgcctcagcc tcccaaagtc agccttatta aaaaataata attgtaataa 31440
taaacactcc tcaggtagct gcaagccttt ggttaatttg cagaagtatg aaaaaattga 31500
ttttgactgt ttttgccagc atgctcattg cttttatgaa ggaacagatt tcagaagtcc 31560
ttattctaat tatcctctca ttctggtcat gctcctccac ctgtttttgt gcctaaagtt 31620
ttattggaat gcaggtttgc ccattcattt acatagtgcc tatagtggct ttcacacaca 31680
actgaaaagt tggacagttg cgacagaaac catatggcct gcaaagccta aaatatgtac 31740

tatctggcct ttaggaaaag gtttgccaag ccccaccata aggcaagaaa atgatataca 31800

gattgcaagg aaaaagtaaa gtgccatcat ccacaggtga aattttttgg catttagaat 31860

ttttttattt tttgatcatt tattcattta tttagagaca gggtctcact atgtcaccca 31920

ggctggtctt gaactcttgg gctcaagtga tcctcccatc ttggtctcac agagtactag 31980

gattataggc atgagccatt gcacccagcc cagaaaattt taaaatctgt gaacaacttg 32040

ttagaattag taagtggctg gacatggtga ctcaatgcct ataatcccag cgctttggga 32100

gactgaggca ggaggattgc ttgaggccag gagttcaaga ccagcctggg aaacacaaca 32160

agaccctgtc tctacaaaaa caataaaata aaaatcagct gggtgtggtg gcacctgcct 32220

gtagtcccag atactggaag gctgaggcag gaggatagct tgagcctggg agttggaggc 32280

tgcagtgagc tatgattgta ccactgtact ccagcctgag tgacagagtg agactgtgtt 32340

tccaaaaaaa aaaaaaatag taagagaatt tagcaagatc attgactaca ttagcatgca 32400

aaactcagct gcaggactta tgaacacacc cctccctgcc tctctcagtg atcacgacta 32460

aaaattctga acagaatgcc tatagtaact gtctgagaac tctgaaaagt agatcatagg 32520

tggcagatta aggaaggaaa tgaaaactga aagaatgatg ggcattgctg tgagttcctg 32580

gactggttgg ttggttggtt tttctccaca ttccataaga tagattctag cacagctcaa 32640

atcctggagc tgtgaagtgg ttggaggaaa tgccttgtct ttctggccag agaaccagga 32700

atgggggaca aagagtcagg gggtgggggc aactccaaat ttttattttt tatttccctt 32760

ttgactgcat ctccatttgc tctcttctct tctgaaactc attttcaatg gatgtctcct 32820

cgtaggttga ccctctagtg tttttgtctt ttctctccta atatccaatc actctcccat 32880

ttctcctggg attttccgtt gcacatgcca tttcctcttc agggaacatc aaagaacact 32940

cttcctgccc ctgcaaagag atgtctactt aactccacca cccaacattc agatggcttt 33000

tgccatcttc tctgactagc caagtctctc tactggatgc tgtatttagt ccattcaaat 33060

gctgctaata aagacatacc agagactggg taatttgtaa aggaaagagg ttcaattgac 33120

tcacagttct gcatagctgc agaggcctca gaaaacttac aatcatggca ggaagggatg 33180

caaacatgtc cttcttcaca tggtggcagc aaggagaagt gccttaacaa aagcagggaa 33240

agctcttata agaccatcag atcaggcctg gcatggtggc tcatgcctgt aatccctgca 33300

ctcttggagg ccgaagtggg cagatcactt gaggtcagga gttcaagaca agcctgacca 33360

acatggtgaa accctgtctc tctactaaaa atacaaaaaa ttacctgggc atggtggcgc 33420

atgcctgtaa tcccagctac ttgggaggct gaggcaggag aactgcttga acccaggagg 33480

cggaggttgc agtgagatga gatctagatt gcaccattgc actccagcct gggcaacaag 33540

agcgaaactc cctctcaaaa aacaaaaaaa aacaaaacca atcagatctc gtgagaactc 33600

actaattatc atgagaacag cagcatgggg gtaaccaccc ccatgattca attagctccc 33660

accggctttc tcccacacca catggggatt atgggaacta caattcaata tgagatttgg 33720

gtggggacac agccaaaccg tatcagaagc actatgtctt tttcatttcc agcactggtt 33780

agagttgtca ccttgcattt acttgcacaa ttcttagatt gctgccagtt tcctgttcgg 33840

tccataagcc ctatctgact ttggtccatc attgtgcttc cagaatctaa aatggtgtct 33900

gacatgtaga gggcttccaa aaatgtttcc tgaatgaatg atgaatgtct ctatgtcttt 33960

gcacatgttg ttccccctac ctgaaatatc caccctttcc atcccctgtc agtacacctc 34020

tgcattccta caagaaccag cccaagtgtc accacctctg ggaggcctcc cctggtgccc 34080

taggcagaca cattgcttct gcagcattgt gaccatgggc tggaagaaag cagccttgtc 34140

accttgcact acagtagcct ggggcataaa tattgtctaa gaaatcattg cagtttccat 34200

atttttatgc aataaggaag cctacttcca gcatgaaccc aacttagaaa atactctcag 34260

ccaatatttc agatttcagc acaaccctcc atcagggtgc atgacgtgag gactttacaa 34320

atgccgcggc agaacctatg ggcctagatg agagggaagg ggtggaagct gtgcaagggc 34380

ccacagcacg ggctgccctg tggaggcggg tctagatgct gctgctgatg caggtctgat 34440

gtgtcagcac agagacccac cctgagccct ggaggagtcc agcccaacgc ttagtagcaa 34500

ggcatttaca gaggatccat tccactgtgg agggagcatg atttatcctc gtaaataaca 34560

cctactccac attgatatgt gtcttccttg gcacaaccct gccagctcta cacatgggat 34620

ttcaaaagtg actgatttct caatctagaa tcctgcacaa tgtcaccttg gaccaagaaa 34680

tacttttttt ttttgagaca gtcttgctct gttgccctgg ctggagtgca gtggtgcaat 34740

ctcggctcac tgcaacctct gcctcccagg ttcaagtgat tctcctgcct cagtctcctg 34800

agtagttggg attacaggtg cccatcatca cgcctggcta atttttgtat ttttggtaga 34860

aacagggttt tgccatgttg gccaggctgg tctcgaactc ctgacctcaa gtgatccacc 34920

cacctcgacc tcccaaagtg ctgagattac aggcatgagc cactgcgccc cgcccaagag 34980

atcaatttta cagcaaaagg tccacaatgg atacatggca atgggatcca ttggtcctat 35040

caacaactac atgagctgga cacagtcagc ctggatgacc attgaaatgg cttattaaag 35100

gctaggctaa ggggtcaatt tgagctttct ctgtggggtt tggagcttgt attaaattaa 35160

aatgtatctg gtggctcatg cctgtaatcc cagcactttg ggaccccgag gaaggaggaa 35220

cgttttgagg ccaggagttc aagaccagtc tgggcaacac ggtgagaccc caactctaaa 35280

aaatatatat aaaatagcca gccatgttgg tggatgcctg tagtcccagc tattcaggag 35340

gctgaggtgg gaggatcgct tgagcccagg agttcaaagc tacagtgagc catgattgtg 35400

ccactgcatt ccagcctggg caacagagca agactctgtc cccaactctc tgcctccaag 35460

aaagtacctg ggatttgttt taatcaggta tggtgaggcc aacaggtcag gagatgaatg 35520

ccgtgaagca aaaattttta ctcacccatc cctagaaaca ggaggccacg tggagccact 35580

caggaaagca ctcgggtagg tcaggaggga gaggcggagg gggaacgggg caagagcctt 35640

cactgtggtt tgtgcaagaa ggaacgggta aggctggggg aatgggttga ttgggtttgg 35700
gattggctca tttgagtaat ttcagcagcc tccagggtat aggggccact cctagctgtc 35760
tgatgtctgg ccctgggatg ctgggggtag aggaatattg tctccaagag tgcaaaatcc 35820
agatggagaa ggtggttgag tttaggctct ggattggtcg gtttgcatac caaaggcgtg 35880
ctagcgctcg caggaaagtt gtttaccatc tctaggaatt cactagcgct ggaaggagca 35940
gtctctccag gattagcaag gcccccagaa gtcaaagcat cataaagtac agaaaattta 36000
aacaaatatt actgatacga gctatcttct acaatgtgcc gtacgcattg aaccaacagt 36060
tgatgcaccg tatcccagta gctggaattg aaattttccc aaagcaagga ctgacccctc 36120
ccaaatcact cccagtgacc ccagtggcac cagacagccc gattttccag ttgccagaga 36180
aacacttcca ttagaaaatt cactgaaatg ggccaagtgc agtggttcac agctgtaatc 36240
ccggcactct gggaggccga ggccagtgga tcgcttgaac ccaggagttt gagaccagca 36300
tgggcagcat ggtgaaacct catctctacc aaaaaaaaaa aaaaaaaaaa aaaaaaaacc 36360
aaaaagttag ctgagttgta gtggtgtgca tctgtggtcc atgctacttg ggagactaag 36420
gtgggaggat taccgagcct gggaggcaga ggttgcagtg agccatgatg gcaccactgc 36480
actccagcct gggtgacaga gtgagacctt gtctccaaaa taaataaata aataataaaa 36540
ccatgaaaag ttcctctgaa cctgcatctg tacatgtgta aatgttatct gtcactttgg 36600
gtttcttgtg ccgatgaaat agaattcaaa ggaaggagct gccatactaa tgggggtcat 36660
tactgagtat tatgaggggt atggatggct gctgcacagt ggaggtaggg aggagtctgt 36720
ctggaacatg gggttttacc aggaccccag gagcccagca gaccatgagg atcttggtgc 36780
attcgtgcct ggcgataacg gtaaacaggg ggcagtcatg cctggcagag aacaggcaac 36840
caaaggctgg ggtccctcag ggatgagggc ctgggtcacc ccagcaggca agagccttag 36900
accatctgaa gtgctggctc agggtgaagg aaatcttgca tgggttctct ctagagggga 36960
ctgatgacaa atattaatta tggtcttggg accagctgca gctgcccaga atgtagctca 37020
ttccaacaca tctttgtatt cactctctct gtaggtcatg cctgtccact ctcccgaaga 37080
ctcagtgcca ccatggactt ggggtgggca cacgtggatc tgagtggtgc aaggggtgga 37140
ctgtggcaga cactttctgg tgctctgtgt cactttccat taatttcctt ctgatttctg 37200
cagcagcagt ggtggtcagt tacgggcaag cacaggcatg cagggagtgt cccacatcaa 37260
gcatgtgaca gcatcttttc cctctctggc tcagggtcct ttcaacaaca agggtctgtt 37320
gaggctatga gcaagctctg tccaggattg tgaggaagtc agtgtcctca agggacaccc 37380
tctacctctc ggggggtgga ggaacaggtg gatacatgct ccaggctcct tcttgcagaa 37440
ggacaattct gggagacatt ctgtactctt tgcaagggtt cctacagaat caagccccct 37500
ttgctgaagc agcgaccttg ataactcacc ctgacaccag cttttcctct ttccctttaa 37560
cactcttttt ttcttctgtc tgttccttga ggtgcctccc aaataaactc ccaactccca 37620

agaccttgtc ttaggttctg ctatgaaggc ccccaaacaa agacaccccc gtgtttttgt 37680

ttgtttgttt gtttgtgacg gattttcact cttgtcgccc aggctggagt gcgatggcag 37740

gatcttggct cactgcaacc tccgcctccc gggttcaagc gattctcctg cctcagcctc 37800

ccaagtagct ggaattacag gcatgcgcca ccaagtccag ctaattttgt attttttagt 37860

agagacgggg tttctccatg ttggtcaggc tgttctcaaa ctcccgacct caggtcatcc 37920

gcctgcctca gcctcccaaa gtgctgggat tataggcatg agccaccaca cctggcctgt 37980

ttgtttgttt tttgaggcag ggtcttgctc tgttgcccaa actggagtgc actagtgcaa 38040

tcatagctca ctgcatcctt gacttcccaa gttcaagtga tcctcccacc tcagcctcct 38100

gagtagctgg gactacacgc atgcatcatc atgcctagct aattttttgt tgagatggga 38160

tctcactatg ttgcccaggc tggtctaaaa ctcctgggct catggccagg tgtggtggct 38220

catgcctgta atcctagcac tttgggaggc tgaggcgggt ggtcacttga ggtcaggagt 38280

tcgagaccag cctggccaac atggcgaaac cccgtctcta ctaaaaatgc aaaaaaaatt 38340

agctgggcat gtggtgcatg cccgtagtcc cagctacttg ggaggctgag acaggagaat 38400

cacttgaacc cgggaggcag aggttgcagt gaactgagat cgcaccactg cactccagcc 38460

tgggtgacag agcaagacac catctcaaaa aaataaaaat aaaaagtaaa actcctgggc 38520

tcaagtgatc ctcctacctc agctttccaa agtgctagga ttgcaggcat gagccaccgt 38580

gcctagccaa agacaccctg taatcaagca gtcctggtgg aagggctggg gtggcccagg 38640

gcagcaggtg tctggccaac agtttctagt gttttgtgac tgctacttac tgacacacat 38700

aaaagagaca gtcatactgg tgtgtactgg tggaatgtcg actcttatct tcaacagtgc 38760

caaagttcag ggtttagtaa aagtatttaa acagggcgtg gtggctcaca tctgtaatcc 38820

cagaactttg ggaggctgag gcaggtggat catgaggtca ggagattgag atcagcatgg 38880

ttaatactgt gaaaccctat ctctactaaa aatacaaaaa attagctggg tgtggtggca 38940

catgcctgta gtcccagcta ctagggagct gaggcaggag aatcgcttga acctgggagg 39000

cagaggttgc agcgggctga gatcatgcca ttgcattcca tcctgggtga catagcaaga 39060

ctctggccct ccccgcaccc cccgcaaaaa aaaaaaaaaa agtacttaaa ctgggcctgg 39120

tggctcacat ttgtaatccc agcactttgg gaagctgcag caggaggata gcttgggcac 39180

agaagttgga gaccagcctg aggcaacata gtgagaccct ccatctatac aaaaattaca 39240

aaaattagcc aggcatggtg gcatgtacct gtagtcccag ctacttggga ggctgaggtg 39300

ggaggattgc ttgagccaag gaggtcgagg ctgcagtgag ccatgattgt ggcactgcac 39360

tccagcctgg acaagagtga gaccttgtct caaataaata aaaataaaaa acagtattta 39420

caggtctgag aaccgcgcag gtcaagccat tcataactag ttctttcatg tattcataac 39480

attccaagac aaaacctcca tttaggtgat tatctgaatg gctggggcct atgccactgg 39540

tatacagagt atgccacttg gtaagtggtc tctcttttcc taaatggggc ttcagccaga 39600

aatttgtctt gtttggtttt gtagggtggt cagattgtgc caagcattac atatgatgct 39660

tgagtcataa tcacgttgat aacagaaaaa gaaaccattt atgggccctt cctgtactaa 39720

gcacttcata tgcaggatct tgtatgcctc agaattaccc tccatggtag ctattgctgt 39780

tacttccaat ttcttgatga ggacactggc tcagagaggt taagtaactt gtctggggtc 39840

actcagcagt taggtgacag agctggtatt cttgccctct ccctgggctg ttttcttagt 39900

agcacctgtg agctcccgcc tggaagagat gcagtcattt ccgctcacat cctattggct 39960

ggaactggtc acatgacccc aggtgaattg ggaaggaggc tgggaggtag agaagcacgt 40020

ggctaactgg tgaacatttg cagctcagca tcagatacag ccgggtgcag gcagggctct 40080

gtgagatgtg gtcatctctg cacattgatt gccatgcaag gcttgactca gagcatctgt 40140

tggcctgtgt ctgcttctcc aacctgtgca cgcacaaatg ggaccagccc tcggcatctc 40200

tgagagacta gaagaggcct tgaactccac ttgggatcag ctcttccgaa cgcagattaa 40260

ttgctgttga tgttctttgg acataatttt ccgcgagaca ggatcatttg gaaacccata 40320

aatacacatt cagatgcacg tgatggataa gaaactcggc tccaacgagt catttgagct 40380

gccataaatc ttgtttgcat tttcttccct ggtgcctgta aatcaggccc tatctccgcc 40440

ttctgggtcc tggtggcccc tacctgccat ccattacact gcaggtgggg catctgacag 40500

tcagccccac acgccgccct catgccctac gcacatccat cacgtggcca accctgcatc 40560

ctgccaccct cctggggggc caaggttccc tgaagtttta cttgtctctc gtgcttttct 40620

gaatagcagt tgtcccctac ccccaaaatc acagattaca caagcctgat tgggttttaa 40680

cgacatgcaa gttttaaaag caaactggag agagctgctc ctgggtttct accttctcct 40740

tggcctgtgc ccctgtgagc tgttttgttt tcaacaagcc aaccatgcaa agagcaggca 40800

aaacctcacg gggtttggaa tcagacgcgg tgggctgcaa tggttctgta attaagagat 40860

ttcagccgag ttctttctct gggcctcagc gtgctcatct gcaggataga gataacaaac 40920

cctacttcct cagattgtat ctcagtgtaa taatactcag gtaatcgacg taagagaaac 40980

ccccaaaact cagttgctta acccaatgta caggtttgat ttttgcccca tgcaaaatcc 41040

agtatggcag cttccttctc tcttacagct ttgttttctg gagtggatat tcctctaaga 41100

tcaccgcggc aggagaggag agagacagaa agaggacctt ggctcctaac tgctccagtc 41160

cagaagagat gcagtcactt tctgctcaca tcctattggt tggaactggc cacatgactc 41220

caggtgaatt gcaaaggatg ctgggaggta gagaagcaca tggctaattg gtgaacattt 41280

gcattcctgc aagagtggtt gtgagtgttc aatcacatgc agttctcggg gcagggcacc 41340

gaaggggtct agcagagctt cgggatgtca gagatgagcc taaaataaga ttggccgggc 41400

gtggtggctc atgcctgtaa tcagagcact ttgggaggcc gaggggggcg gatcacttga 41460

ggccgcctgg gcaacatggt gaaactccgt ctctactaaa atacaaaaaa ttagctgggc 41520

atggtggcac ctgcctgtag tcccagctac ttgggaggct gaggcaggag attcacttga 41580

acctggtagg tggaggttgc agtgagctga gatcacttca ttgcactcca gcctgggtga 41640

cagagtgaga ctttgtctca aaaataaaat aaaataaaat tttgcctgtg acctgctctg 41700

ggctgggcag caatttttgt tcctttggaa tccaggcagg acccagaggt cagccctgga 41760

tcagagggac tgttcctcct ttcttccttc tcccacccta aggatgaaat ctgttgcttg 41820

tcctggtgga attctctttt tccagctccc attctcaaaa gcaccccata aaataccaaa 41880

aataaacact gttcatggct tccatttggg gaggatttat ctttttcctt taatacttct 41940

taccacaaca taccagtctt tcatctttgc atgcctgggt ctgtgaccct accctgggag 42000

gcaggccaaa ggcggtgtat gcaggcctct tgggtcaaat gaggacttac ccctctgaat 42060

gctgaggaaa gcacagggga ctgtctgggg aaggcagcac gcagtgaaca gacagggaga 42120

tgctgggtct gggcctggaa ggatgcgtag gagtttgtcg agtggcaagg ttgagggaac 42180

agacagggag acattgggtc tgggccttga aggatgcata ggagtttgtc aagtgacaaa 42240

attgagtgtg ttcctggcag agggagcaaa gtgtgcagag agataggaat gggaatggac 42300

tggcgtgttc tgggagcaac tggaagttcc ggaagtctgg atgttagggt tcctggggat 42360

ggagaggcca ggaggactgg gttgtggaag accagacttt atctccagga cagtggggag 42420

ccagggagca gtgcaagtga ggcggggacg tcccaggaca cctgtccacc acggccaact 42480

tttgtgacag cactgactct tgtattggtg gcagggtggc ttgttttctc cccacatttc 42540

acttcctgag caaaggcagt cttgcaagaa tctgaaagga ggcaccacat aaaatgactc 42600

tttttttttt tttaagtaaa aagtcaacag ttccgtgatc tttgtaaaaa ttaatcagga 42660

acacatgcgt tctatttttc gtaaattgca agggatcaaa aggaaaggaa aatgctctta 42720

gggtgaaaga caatgggagt tccatcacca gctggaacac tccacacact gtcttggagt 42780

tggggggcct tccctgcacc cggcgtcctg gtgtgcctcc ctggagagag agcatgggct 42840

gggcacgaga tatggggtgg gcttagatac gacccagacc tgagatgctc ccagacagga 42900

gcaggggacc aggcccagca gggggcaatg acaatctctg gagggacagt ccagggccga 42960

gggaacccag agaaggcctc taagtgtctc agacacagga ggaggaacaa ttggagagga 43020

gaccaaatgg gtgggctggg gatgtattaa gactgtcttt tttttttttt tttttttttt 43080

tttgagacag ggcctctttc tgtcacccag gctggagtgc agtagcacga tctcagctca 43140

ctgcaacctc tgactcctgg gttcaagcaa ttctcctgcc tcagcctgag actgtctaga 43200

atgtacagct aatgaatctc tatttttctc ttgtactttt ctcccttact atctgaggat 43260

gggggcacaa tggcaaaagc cctgggcaca gaaaatcaat ccagcttttc tcttacagtg 43320

aggccaatcc catactttgt agggtttttc attttttttt ttctttttga gatggactct 43380

cgctctgtca cccaggttgg agtgcagtgg cacgatctcg gctcactgca acctctgcct 43440

cctgggttcc agctgttctc ctgcttcagc ctcctgagta gctgagatta caggcatgcg 43500

ccaccacgcc ctgctaattt ttgcattttt agtagaaatg ggatttcacc atgttggcca 43560

ggctggtgtc aaacttctgg cctcaaggga tcagcccgcc tcagcctccc aaagtgctgg 43620

gattgcaggc gtgagccacc atgcccagcc ccaatcccat cctttgaacc tcaatgccct 43680

catctgtaaa atggagatca taatgagccc tacttccccc aggggagcta tggacaggtt 43740

cacgctctcc ggggcctctc tcacactggc cttccccttg ccggcctccc ctgggtctat 43800

ctgctgtggg ggctgcagtc actcatgggc aggagaggtg aggaaatgca gttgcacagg 43860

cctagaccca gctgatgccc tgtgtgtcca aagcccagtg gggcctgggc ttctggtggc 43920

caggacgccc ctatgtccaa cccagggtgg agaaagaggc caggaggaca tggtactctg 43980

aaggcctttt gtgtggcatc ctcaaggaac cagatccagg gctcctcctg caccctgagc 44040

cccaccacgg gaccagagca gcacccagcc tgccgccagc cacccccgcg gtggcctggt 44100

gccagccctg caggcgggca gtgagataat ccggctagac gggctgccat cttggcacgt 44160

cccaagacgc ccgaggccac ggggagcttc aaaaggaaaa ttaatgtctc ttaccagcaa 44220

ttagaggaaa ttattcagat agaaatcttg ttttctatct cctcatcctt gtttttgaat 44280

ccccagcctt tgatttcttt cttctctggg gattctcaga aagacactct gagagcctgg 44340

gctgtgcact gaggcctaat gagggcctgg cacacctggc atcacagcct cgcccggggt 44400

agtcggcttc tgaggcctgt gcctcctgaa cccagccctg aggtcttcca aagaggctgg 44460

cgggcccttg gcagctgagg gcagctctcc cgaggtgggt gggcctctgc tggggctggc 44520

tccccctgat gcccaaggga ggtggcaccg ggctacaggc ggacgtcatg cacctggggt 44580

gaaggggtgc tgagggcact cttgggtgga ggagaggccc aaggggtgga ctcagtggga 44640

actgacaccc ttcctgtctc agcggggacc cctcatgctc tccgcccgtc agtctttccc 44700

cactgcctca gatgcagcct caacttccca atctggcgct caaagccctg cagatctatc 44760

ctcgtcagct tccctggccc ttccccccat cccctcctct aaatgtcccc cgctccactt 44820

gcccagggtg ctgggcttat tccagacagg agctcagccg ccatgaccca cggtggcaca 44880

caccctgacc tgacgtaaat gaccttcttc tctttttcta tctggcagac tcctgcacct 44940

cctcaggtgg cccagccctg cctcaccttc tcagggatgc tggcttggac cccacccaag 45000

agaacagttg gcatctccat cacctagccc aggccactgt cctgagctaa tcaggagcta 45060

ctcatgggct ctcttcccta gaaactcctc gaagccaggg agaagggcgg cttcatctcc 45120

aggttcccag caccaggtgg cccagaatgg gctttaataa gctcctgctt gaatgagtga 45180

cacccaatga taatgatggt ttgaataaat aaaagaatga aagaatgaat gggactgatt 45240

agaagaggca ccaggaacaa ggtgtcaaga gactaatctt gaatcatgat tcagtcactt 45300

cctccctgtg tgacatggag caagttgctt tacctctctg ggcttcattt ttcagttctg 45360

tagaatggga ttaatagttt tagccctgcc ttcaaaacag ttgtgtggct aaaacaaaat 45420

aaaggtgttg ccaggcatgg tggctcacat ctgtaatccc agcactttgg gatgacgagg 45480

tgggtggatc atttgaggtc agaagttcga gaccagcctg gccaacatgg tgaaaccctg 45540

tctctactaa aaatacaaaa attatctggg catggtggcg ggcgcctgta atcccagcta 45600

ctcaggaggc tgaggcagga gaattgttga gcccaggagg tggaggttgc agtgagctga 45660

gatggcacta ctgtattcca gcctgggtga cagaaggaga cttaatctct aaataaataa 45720

agaaataaat aaaggggttg agcatgcaat tatattaaaa ttacatgtac cactcatccc 45780

tccctccatc catccatcca tctactcatt catccaatta tcaattcatc catccatcca 45840

ctcatccatc cacccgtcta ttcatctatt cacccatcca ccatccatcc actcattcat 45900

ccatccacgc actcacacag tcattcaccc cctcacccat ctaggtatcc atccaattat 45960

ccacccatcc atccattcat ccatccattc actcatttat ttatccatcc aattatccac 46020

ccatccatcc attcactcat ctatttatcc atccagttat ccacacatcc atccattcat 46080

ccacccattt atttatccat ccaattatct atccatccat catccatcca tccatccatc 46140

catccatcca tccatccatc ctcttgtcca ttcctttaac aaccactcac tgagacctcc 46200

tgcatgccag gcctgtgttg aggaatgggg gacagtgtgg ggtcagaaga agtacctgcc 46260

cagggagagg tcatcagtgg cagcagagaa gcccagacag gagctaaagg aaggaggctg 46320

agcatgccat ctcaagagtg cagcccaggc tgggtgtggt ggctcacacc tgtaatccca 46380

ccactgtggg aggctaaggc aggcaaatcg cttgagccca ggagttcgag accagcctag 46440

gcaacgtggt gaaactgtct ttagaaaata ttagccaagg ccgggcacgg tggctcatgc 46500

ctgtaatccc agcactttgg gaggccgagg tgggcggatc acctgaggtc aggagttcga 46560

gaccagcctg gccaaaatgg tgaaaccccg tctctactaa aaatacgaaa attagatgag 46620

tgtggtgtcg tgcacttgta atcccagcta cttgggaggc tgaggcagga gaatcgcttg 46680

aacctgggag gtggaggttg caatgagcca agattatacc actgcactcc agcctgggtg 46740

acagcgcaag actccgtctc aaaaaaaaaa caaaacaaaa caacaacaac aaaaaaaaaa 46800

acaaaaagaa aagaaaagaa agaaaggaaa agaaaaaatt aggcaagcgt gatggcgcat 46860

gtctgtagtc ccagctactc gggaggctga ggcaggagga tcacttgacc ccgggaagca 46920

gaagctgaag tgagcccaga tcacaccact gcactccagt ctgggtaata gagtgagacc 46980

ctgtctcaaa aacaaaacaa aaccaaaaaa gagtactacc caaatctctg cttcccaccg 47040

cctcaaatgc acctgctctt gtcatgctca gccggcttca gaaaccctag gaggccagga 47100

acacttctgc caggggtcca gttgggagga cacctggaga acctggaggt ggaggtaaag 47160

aggaagggaa aaccagacac agagaggtga agaaatatgc ccccaactac tgttgaattg 47220

ggatgtgagt cctgagccac attcttaacc aggtccacat cctggatcaa atgggccctc 47280

aagtccagtg cttggaaagc ctccaataag tggctggcca gcatctgtta catacttccc 47340

ctgacgagac actcactacc tttccaggct agtgaggctc atctgtgtac ggctctgact 47400

gtgaaaagct ctttcttgga tagaggatcc acattggccc tggctcggcg cactggaccc 47460

tgtattcctg ctggcctgga atgtccgtgc agaaacttga agacagtggt gtgtctctga 47520

gccttctctt tcccaacatg ggtactcttc cctgctcccc ccaagtcctt tcacctccca 47580

gctgtccgtc tggcacagaa gatcaagtgc attgttgaga ccacaaggga ggggaggcag 47640

cttaggagat caggaaaaaa acttatgaca ctaatttagg gtcttagcct cactcttggc 47700

cttatcctgt gttttctaga cattaacttt cgtcagcccc caaatcttga tttaatcatt 47760

ccatcttaat gtattgtctt cattgctata aaatgccttg attccttttg gggagtagag 47820

ataagtaata aataaaggta ccagttaacc ccccctaagt tttgggggat gatcacatct 47880

gggtcctttc tctcttgcct gcttcaagaa acaattgtta attcttttag taagtttcag 47940

gccagttgat gtaatgacaa tatcttgaaa tcaaccatcg tggccaggca tgatggctca 48000

tgcctgtaat cccagcactt tgggaggctg aggtgggtgg atcacatgag gccaggagtt 48060

cgagaccagc ctggccaaca tggcaaaacc ctatctctac taaaaatacg aaaaattagc 48120

tgggtgtcat ggtgcgcacc tgtaatccca gctactcggg aggctgaggc aggagaatca 48180

cttgaacccg gaatctggga ggcggaggtt gcagtgagct gagatcacac cactgcactc 48240

caacctgggt gacagaatga gactctgtct caaaaaaaaa aaaaaatcaa ccacagtgag 48300

ggtctttaca ccatgagaat tggcaaacgc taccagtcag ggctttcccc actacatttt 48360

ccagcacaca actgaatatg cctctcatct ttcttctccc catagagcag tcaagtgagg 48420

aggactgcct gatcctagag actctgatcc tcccaagacg cagaccaggg ctctattctc 48480

acaggacttt cccctcaagt gactctcctc tgtgcacaca tgctgtctta ctctgtattc 48540

ttttaggagc cacagggctt caagcatgca cccattcatt caacatatgt tcaacaccac 48600

tatctggcag gccccaggct ggggacttgt gactggggac ttacagattg gacagagctg 48660

ccttctctac cagtgccttg aggggactca ctctatgtca tgtgcatgat tcatgctatc 48720

tcatttaacc ctcacaacat ccgggcccat tttatagatg agaaaactgc agagcctgga 48780

gtgccaagtg ggtgatttct atctgttcca ctccgaagac ccaggcttac cactccaaaa 48840

tgacagtggt ggtgattttt tcttttcttg ttcggtcttc actatggctc tgagaggaag 48900

ctgttcattg ctctatttgc caagggggaa aacctgaagc tcagggatgt tgaccggtcc 48960

aacttgcaag cttaatgaaa atgcaggact ctgtctccta agccaggtgg tggtgcaaaa 49020

tgagccccac tcctggggtg gaaccttgag ttgggagccc tgagttcttg gctcagctcc 49080

actggggatg tcaagggtga ccttgggcaa gtcacttctc atgatctaag ccattctgtg 49140

cccctcagcc ctgattgctc tgagccccag accctgccat cccctgtcac ttgagcttca 49200

tcataagatg ttccatgagc attgccactt attgcaacac agatggaact ctctaagcct 49260

ctgtttcctc atgtgtaaaa actggataac agtagtttct actttacaga atattgtgag 49320

gatacaataa tatcatactc tcaatggatt cctcacactg cagtggtgtg atctcagctc 49380

actgcaacct ccgcctcccg gattctgggt tcaagtgatt ctcctgcctc agcctcctga 49440

gtagctggga ttacaggtgc actcaattaa tgttagccat taactctctg ggaacatgtc 49500

cccactgtct agctggctgt ggtactcatg tatgaagccc acaaacattc aagtatccaa 49560

cgatcaccta aaatgcacac aagttcatcc attcagtgtt gacatggaca gatggtatga 49620

tggattacag atggctataa attatttgtc atgcctttca agaaatctat ttcccctcct 49680

gtttagtcta gactatcctc atggctgctt acccaataga atgcaatgga aggaacagtg 49740

gccagtcagg gtcctagtct ttgtgaagac tggcagtttc tgcttctccc tcttggaaca 49800

cttgctcttg ggacactgct tctcagaaac cagccatcat gctccaaaag acgtacagaa 49860

gccacattta ggcactctgg ttgacagccc cgctaaagtc ccaggcaaca tccaacatga 49920

gctgccagtc atatggatgg gccatcttgg atattccagc tcgctatagc tcccagatgg 49980

ctgcatcccc aaccaacatc acataaagca gaagaacctc ccagccaagc acagtcaact 50040

cataggataa tgagatttaa gatggttatt gctttaaacc actaagtgtt gggacaattt 50100

gttatgcaga aaaccaaagt gttggggcaa tttgttatgc agaaaaccaa aacatatagt 50160

atacccattt atgaaacttt aaagtattta tttactactt tttgaaaaag attagtcatt 50220

ctgtactaca gaaaactata acaataattt gctgcctgta tttgtatagc ctgcaaggta 50280

agcaaggctt ttacactata acatggctgg aaaaaatcaa aagaagaata ttattttgtg 50340

agctatgtga gaaatttata caaaattcga attttagtat ccacaaataa agtttgattg 50400

gaatacaggc atgctcattc atttatagct tgtccacagc tgctttgtca tgagagcagt 50460

agagttccat ggtggtatca gaggccatat ggaccacagc acctaaaata tttactgtct 50520

ggccctttcc aggaaaagtt caccaactcc cttctctaga ccattaaata tgtatcatca 50580

ttttgaactt aaaatcctgc atataagaac ttgtatgtgt ctgaaactta ttgactgagt 50640

cctgtaagga aaaagcaaaa aagcaacagt gggcattttt gcttatttat ttttaatgtt 50700

tatagagaca cgatctcagt ctatcacctg gctggagtgc aattgtgcaa tcataactca 50760

ctgcagcctc catgtcccaa gctcaagtga ttctaccacc tcagcctcct gagtagctgg 50820

gactacaggt gcatgccacc atgcccagct acatttttta tgttttattt tgtagacaca 50880

gggtcttgct atgttgtcca ggctggtctc aaactcctgg gctcaagcaa tcctcccacc 50940

ttggcccccc agagttctgg gattacaggt gtgagccacg gtgccaggtc tatttatttg 51000

tttgtaagtt tgaaagcttt cagaagtata agaatggccc aagctgggtg tggtggctca 51060

cacctgtaat cctagcactt tgggaggcgg agctgggtga atcacttgag gttgggagtt 51120

cgagaccagc cttaccaaca tggagaaacc ccgtctctac taaaaataca aaattagctg 51180

ggtgcggtgg cacttatgtg taatcccagc tactcgggag gctgaggcag gagaatcgct 51240

tgaacctgga aggcagaggt tgcagtgaac caagatcatg ccattgcatt ccagcctggg 51300
caacaagagc agaactccgt ctcaaaaaaa aaaaaaaaaa aaagaatagc ccaacagtgt 51360
gcaaacatag gaggaatgcc tagaaagcat tcaaagagag atcaaaagga aactctgatt 51420
atgggggtga acaggggact ttgaagaaag aaaccagaaa tggaaggggt cctagtggga 51480
aaaggacatt ccctaaggcc aggacagtat ccatgccccc agccccttct atcctcccca 51540
cccccactgt cctgccctgg ccagagctcc aggccacact gttcccactg tagggcctac 51600
actagctgtc tccatctttg tcccctgccc atcttcctcc actagggctc agcttgacaa 51660
cccttcctca tgcagcctac cacctctaag cctgcattcc ctcccttctc tatgctctga 51720
agttctgtat acaggacttg cctctaagat cccactggcc aggtcgtaac gttgagaccc 51780
aaccactcat tctgctccct gggctgggtg cacctcgtgg gtagtcattg gccttattta 51840
tctctgggcc ccccgggaag gcatgagagc caagctgctg aatacagggg ccctgcagct 51900
tgtactgagc atttgagtac cagacacttc taggatttca cgatggaaga ccctatgata 51960
cggcatctcc ttcccatgcc ccactatcag ggacaagggg tgcagagaca acggggcctc 52020
ccagctggag ggactgttca gggccagctc tgagctgcct cccaccctcg cctagcctct 52080
ggcagggcct ctggccaggc aggaacggcc aacagagtca cctcctgctg acaagcctgg 52140
cgctgacagc caggatctgg gagaacaaga agtccattca aggcgcaggg ccgggcgggc 52200
ccactttcag gtgtatgatc tcacctctcg gaaactggca agccgccgga gggcctcgtc 52260
aaggctttga tgaccctgtg ctgaggacgc aaatcctagg cctggccgac accctgctcc 52320
aaggaccaat gactgaggcc agagagggtc ccagatggag cctgggtagg ggtgaaactg 52380
cacaggcgga cgggcagagc gtccaccctg gagaagagac ccaggcccaa ccaggcccca 52440
gagcctgcac gagcacctgg agtggcaggg acagcccagc cccctcctgc cccacttcct 52500
gggaagcagc agcttgtgtg ttctggtcgg tcggctttgg caaagctgac tggtgacgtc 52560
aggcaggcac cccctctcat ccagatcctg ggccccccac acactgggcc cagctggggg 52620
atggagctaa aggaactttg ctcccagctc atcttgtttc tgggctttgt tgcccagtgg 52680
ccactggcac cctggtggaa gggaccagcc agggggagta gtaggcccca tctcagctgg 52740
aatctcccag ggtaactggg cctgcagctt gctggagtcc gtacttttct gtgagctcac 52800
agggcagatc ccagaagcca aaactgaccc agagggaatg gaactcagat ggtcaagaaa 52860
ccactgccct ctgccctctt ggcatgtgac cttagttcca cccatagaga ccagatggca 52920
gtctctggct tttccaagga cccgcggggc aggaatgcaa gacagagcac acactggcct 52980
ctcactgtga ccagcagtgg cctctgtcgt cacctctgtg gacggcaaca cgggccttcc 53040
atccattccc caaaaggaag gcaaagccac agcacccatg caggggccga agcctcacgg 53100
gaggttcccc atgccttcga aatgactact ctgctctggc cggaggaccc gggtcctgcc 53160
acctgacctg agagcctctc tcagctcagg tcctgcctcc tgatcactcg ctgagcttgg 53220

cctcccaaca actgtgatga caacaggctg tctactgcct ctcggagctc ctcctgccct 53280
ggcgggcctg gctcattggt gccctggatg gctccccatc tcccagcaac cgggacttct 53340
ccctcctcca ggcccccggg actctgccta gcccatgccc tgggatgttt atggatgtgt 53400
ccactgccca gcttctcaag aacagaggct gggtgtgagc ccacaccgat tcctagtgct 53460
tagcataagc ctggtccaag gaggcaccca ctggtggttt gcaaagtgaa gtctctggta 53520
attgaaaggg cagtgtgacc acatccttgg agtggaaaga tgacaggctt cgagagcaga 53580
cagccgtggg cagttgtggc ccttggacaa gtatcttagc ttatctgagc ctcagtttcc 53640
tcatctgcag aatgggaata ttagcaccta ttcccctaca aaatagcaca gcaacacaag 53700
gctaaagaga ggtgtttgtt gtttttgttt ttgttttgag atgaagcctc actctgttgc 53760
ccaggctgga gtgcagtggt gcaatctcag ctcactgcaa cctctgcctc ccaggttcaa 53820
gtgattctcc tgcctcagcc tcacgagtag ctaggactac aggtgtctgc caccgtgcct 53880
tgctaatttt tgtattttta gtagagatgg ggtttcactg tgttggccag gctggtcttg 53940
aactcctgac ctcaagtgat ccgcccacct cagcttccca aagtgctggg attacagcgt 54000
gagccaccat gcccggccct aaggctggag agagatttta gagagaccaa ccgcgtgaag 54060
ccccgtctac cagatccagc acaggcgctc aaaaaaatca gtccctgctc cagctctggg 54120
cttctctgtc actggccttc tgtgcccagt cccccggaaa ctgatctgag ctggggtgtg 54180
agccttcaca gcagcgtgta gggaaagtgc tgggccaggg tcctccgccc tgacttttgg 54240
gctcatcctg ccacttcctg cctatgtgtg caaacgagag ctgccttttc tctctgaatc 54300
ccagctttct caactgtaaa atgggtactc cagcctgcct ggcccacctc acagtggagg 54360
tccaggtgct gtgtagactg ttgagggcca tgtgtcaggt ggcccaggcc acccagggac 54420
tgggcaacct cactggcagg tgggcatagg gacggctctt gtccaggagg gcctgcctgg 54480
atggtgagcc agtgagagtc ctcagtcccc agccaggact gctctgggcc cagcctggcc 54540
ggagatccca tcccaaaggc aatgaccatg cttcagcctc tcgagtagct gggattacag 54600
gcacacaatc atacccagct aattttttta gtagagacgg ggtttcacca tgttggccag 54660
gctggtcttg gactacagac ctcaagtgat ccgcgtgcct ccgcctccca aagtgctggg 54720
attacaggcg tgagccgctg catctggcca ggcctccagc ttttatggga gactcacgtc 54780
tgcccgcttg atggaagttc tgggcagggc aggagggaga agaaccagta tttaccagaa 54840
tggatgaggg cattccagga ggggcctttc gctgaatgga ggcagtgtgg aaaaggggca 54900
ggtgggaccc agacggtgca ggagcctgag gctgtccctc caggctgtcc cagggatcca 54960
gagcctcatc gaggccccat ccttggctct tctaccgccc ctatccatgg aagccaggaa 55020
tcttgttgcc ccactttggg agtcaaactt cacagctttg tctgtctcca agcctcccac 55080
tggaggtgct tggaggcctc aaacctgcag ccttgaatca cccaatgccc tgatgggctg 55140

ggctgcagag agggagggaa aggttatggg agtgctggca gtggaaatgg ctggaggtat 55200
aatgtcattc catttgcaga ccaggaaaca aaggatggca gaggggacca cacagccagg 55260
cagtgacagg tggggacaga aagggacacc cccctgccac ccattgtcct gggggtgctg 55320
cccaggttcc tgagtgagtg gttgggtcag gtcacctaaa gaacacttgg aagcccgcga 55380
ggagaggaaa cccactttga acaaatgtgg agaaaggcgt ggatcagagt ctaacagctg 55440
aggtccccga tgactgtgtc ccgctccatt tagggcccgg gacacacaac cctccttggt 55500
cccctcccgt ggacatggga ccaggaccaa ggtctcttcc ccctgaatat cccgccctcc 55560
ccagcctcct gccaactgcc tgccttccag tgcccccgag gacacaggac tgaggctgga 55620
tagaggattg cttcgtggac ctttgctggg gacatgtgga caggcagggg ccaccaactc 55680
agagcgaaga cgtcacagat gtgtgaaaac actgaagatg gggacacaga ggcaccgagc 55740
gttaggtggc tggagtcact ggatcacagc gtctggagcc aagggtttcc actgttccca 55800
ggtgtaattc ctccccgcag ccgtgcaagg cgggtgcccc agctgtactc acttggctgc 55860
tctgtaggtc gcacagtcag taagcggcag agctgggaca ggccaggcag gtaggcgtct 55920
tgactcggtg ccttaccctc tctggcctca gtcttctcat ctgtcaaatg gtataataat 55980
aggatgtact cctcagggtt gctgggaggg ttacacacgt tagtgcacac tgcctggggc 56040
tcggtgactg ttctctaagt gtttgctgct attttttttt acttaaggaa acttaacatc 56100
gaataggtta agcagccggg gcttgaattt agtcttctct gcaccaaagt tgcgctgtgg 56160
actcttgtgc cgactgtgat ctagagattc ccaagcccca gaggctttgg ggaaggcgga 56220
gccaggagag gctgctcagc tgtcagtaga tggcgctgtg gagccacaag ggccgccctc 56280
gctgcaaaag cccgttgacg caccaggctg tgcacagact ggggtctccc cgccctgggc 56340
caggccctgc accagatatg gccccaggag agggggtgcc gctaaccctg tttccctgat 56400
gagggatatg aaggctcaga gaggttgaac cactagcctt gaggtcacac agctataagg 56460
ggcagggctg gggctcccca ccccagatgc ctcactgagc tctccagccc cacccccatc 56520
ccacccctca ccctggccct caagaggcca cagtgactcc ctggcgcttg atgggagcca 56580
ggcgcgtccc tccttccccc atggctggac attccaatga agctgaagcc accagctttg 56640
aagtgagagc taaacccgga ttagggcggc tccaggctgg cctctccttc ccagccccat 56700
tgccaggccc acgctcacca accacgtcag gccagagtcc tggcggcaca aaggggtctg 56760
gggcctagag gaggcaggac ctgccccgca caacacagtc cctctcatag tctgttaaga 56820
agcctcgggg gcccccacga cctgctacta gcctgaaacc ccacgtcacg cgggtgccag 56880
cacagaagct cccacggggc tgggaggctg tgtgtccgtc tgtctgtgag cagccacact 56940
tccctggcct caccctgcct gtttgggttc caggatcaag cctctttatt caaggagatt 57000
tggactcaag actggaagat gacactggta tagtgaagct acaccagttc tggaacccgt 57060
gttccagaag gttctggaac atccaccttc aaattaatct ctcacagcct cttcttcaga 57120

attcagctct tcccagactt cacttttttt tttaatttta ttttatttta cagagtctcg 57180

ctctgtcgcc taggctggag tgcagtggta caatcttggc tcactgcaac ctccaccacc 57240

agggttcaag tgattctcct gcctcagcct cccaggtagc tgtgattaca ggcatgagcc 57300

accatgccca gccttatttt attattatct tttgagacgg gagtctcact ctaccgccca 57360

ggctggagta cactggtgca atctctgctc accgcaacct ctgccccccg ctgggttcaa 57420

gtgattctcc cacctcagtc ttcccagtag ctgggattac aggcatgtgc caccatgccc 57480

ggctaatttt tatattttta gtagagacgg ggtttcacca tgttggccag gctggtcttg 57540

aactcctgac ctcaggtaat tcactcacct cggcctccca gagtgctgag attccaggcg 57600

tgagccaccg cgcctggcct tttcccagac ttcagtgatt ctcatttcac cttcatgact 57660

tttttttttg tagctaaata ttgtttacat ttatcttttt ccttaaatcg actcctatac 57720

atatgtttat tttaaagaaa gcttttataa aaaagaaacc ttgctatcaa aaatgaaaag 57780

ctagtcccct gtgccacata cagacaacat gcataaaaat gcatgccatg gctgggagca 57840

gtgggtcatg cctataatcc cagcactttc ggaagctgag gtgggtggat cacgaggtca 57900

ggagttcgag accagcctgg ccaacatagt gaaaccctgt ctctactaaa aatacaaaaa 57960

tcagccaggc gtggtggcag gtgcctgtaa tcctagctac tcaggaggct gaggcagaag 58020

aattgcttga acccaggagg cagaggttgc agtgagccga gatcatgcca ctgcactcca 58080

gcctgggtga caatgcaaga ctccgtctca aaaaaaaaaa aaaaaaatgc ataccatgaa 58140

aacggaacaa tgccgttcaa ttctggctgg ctgccggggc ctagggaagg ctctcagcct 58200

ggtgtctgct cccttgctaa agagggcagt aggctggtca gagacctgtc tgcaccaaat 58260

ccagcatctc tccttgatgc aaccaggaga gaacattgtg gaaggagact ccctttcagc 58320

atctttcatt tggagactgg tcatcatctc ctctttgtct gcatttctgc cacccattct 58380

tcaagttatc acctcctcca ggaagccctc caggctgaaa agggcatccc cactgtgctt 58440

cttcagctcc acaggcattt cctgacttgg catttgtcat cctggtttaa taaatcacat 58500

ttctggttat gtcactccca atggagtgaa agtgtccctt ttttattcct tagagacagg 58560

gtcttgccct gtcacccagg ctagagtgca gtgggacaat catagctcac tgcagcctcc 58620

aattcctggg ctcagtcaat actcttgcct cagtctccca agtagctggg accccaggca 58680

cacagtacta cacccagctt tttttttttt tttttcagag atggggtctc actatgttgc 58740

ccaggctggt ctcaaacccc tggcctcaag cagtcctcct gcctcagcct cccaaagtgc 58800

tgggattaca cacgtgagcc accacaccca gcaagggcca aacctttacc atggcccaca 58860

cagttgtggg ccactctctt ccatccacca cagcctgtcc tctggctgtc tacctctctg 58920

tcaccttgag tccttcttgc atcatgatgt cctcactggt catggcgttg gccttctcct 58980

ggatataagc tcagtgtggt ctggtgaggt gtttctgtcc accctcggat ccccaggcct 59040

agcctgcttg aaggaggaga gcagcctgga aacttggcat cgtcactgtc tccattttcc 59100

aaatggagaa gctgagtccc agtgaggttg gtgactttcc cagggacacc catctagccg 59160

tggcagacct ggatgcaaac ccaggcctcc ggggccagca gggatccacc ctcccctaaa 59220

ctccctccct ctcagggctc tacttcacct tgatcaagcg agaccttgtg tttgagagag 59280

acaggaagag aaaaacagac acggacagac ccctgggatc cccggaggcc caagccaacc 59340

cccagtcaga ctgaaatcct tagcccagcc cgggcctcag ctcacagcag gcatagagta 59400

caattttcca tccagactct agctatgggg gcaccccctc ggaacctcac cgcatgcctt 59460

caccagcccc tgaggcctga aattccatga ggacatttca ttgccctgaa gtgtggtgtc 59520

ccaggctaaa gacaccatca gcccctagtc cccacttcca ccccagccat gtcctggctg 59580

tgaaaaaggt gcagccgctc ccagggttgc cagaaaaaca gagtgatggt ggaagccggt 59640

gccaggcagg gtccctccac cagccacgct gtcccgggag gggccccacc cttctgcgat 59700

cagcagagga tccctgggag agtcgccgcc cctccagctc caagcccctg gggtccgtcc 59760

tggaaagacc ttcaaaggac ttggccattc ttgagtgagc gccaggacgg aatctgctcc 59820

cagagtcaat ttaagcagcc acacgggtgg aaggaatttg ctgtttctct taacagcgac 59880

cccctcatga tttatgcggg ggccactgtc cgaaggcccc cggctgatgg ctcctgagcg 59940

gaaaaatgaa atgccaagac ccaggcatct tgctgggcta attactctca atgagaccca 60000

gctggctgtg ggagccggcg gcgccggggc aggcccggat ccctgtaccc agctccctgg 60060

ggctacaaag gcctgtccca accctgcctg tgttccctgt ctgtcaccta ccaggccaag 60120

tgtggggcta ccaaggattc taggagagcc actttggcca gataacaaga tgcatcacct 60180

tggatgctgc caagcaagcc caggactggc catggccttg ggtttgctgc tgccctgggg 60240

ggtacatatc cttgagacac caggggccct ggggttccat tctgcttatg ggaagactaa 60300

ggcccaggca gacagggaaa gtaccctgaa gcctccagga ggctcaggaa taattgcaat 60360

tccagcctca gcttctgccc tgcagtgtct tggaggccca aagccacaga gctgcttcca 60420

gataactccc gtgattcggg gctggctctc tgggtgtcct ggagacagtc aaatatttcc 60480

caagagcctc ctgcatgcca ggccccatgt agggtgctgg gggcatagct gtgggcaaat 60540

ccagcctact cccttccctc agggacttat ggcactctgg gggaagacag acaccactta 60600

ccaaaaagtg aaagttcttg gtgccatgaa actacatgga aagtgatttg gcctatgccg 60660

ctgtcagggc cttaggatac gctgctgacc tgagaccaga aagctgagta gggagcatga 60720

tggttactac tgggtgtcaa cttgattggg ttgaaggatg caaagtattg atccttggtg 60780

tgtctgtgag ggtgttgcca aaggagatta acatttgagt cagtggactg ggaaaggcag 60840

acccaccctc aatctgggtg ggcactatct aatcagctgc cagtgaggcc agaatataaa 60900

gcaggcagaa gaatgtgaaa agactagcgt ggcctagcct cccagcctac acccttctcc 60960

tgtgctggat gcttcctgcc ctcgaacatt ggactccaag ttcttcagct ttggaacttg 61020

gactggtttc cttgctcctc agcttgcaga cggcctattg tgagaccttg tgatcctgtg 61080

agttaatact taataaactc ccctttatgt atatctatat ctctctctat atatatatcc 61140

tattttatat aatgttatat aatatatatt atataattct ttatatataa atctatatat 61200

atctctctct cttattagtt ctttcccttg tgatcctgtg agttactact taataaactc 61260

ccctttatat atatatccta ttttacatat atcctatcct attatatata ttatatataa 61320

tacaataatt ctttatatat aataattctc tctctatata tatatatccc attaattctt 61380

tcccttgtga tcctgtgagt taatacttaa taaactccca tatatatata tatatacaca 61440

catcctattt tatatatata tatcctatcc tattatatat ctatcctatt agttctttac 61500

ctctaagaac cctgactaat gcagggggga ttgggcaaag ggggcaggga gtgttccagg 61560

tagagggagc agcatgagca agactctgca acaggaggaa acgtagggtc cccaatgaaa 61620

ctgtgcagct ggaacccaga gcccagggca cggtggtgca gggaagttca agaggccggc 61680

aaggggttct tttgtttgtt ttgttttgtt ttattatttc ttttagagat agggtcttgc 61740

tttgtttctg aggctggtct agaattcctg ggctcaagca atcctcccac cttggcctcc 61800

caaagtgctg ggattacagg tgtgagccac cgcaccccgc cacgttcttg tacctttaga 61860

aaggtgcttt cagaaagaac actggaaaga cgatgtgtcc acccacatgc tcattcttgc 61920

tgatttctct ctggcttttg ggagcctcag ccctctaccc ctggaagggc agctctgtgg 61980

attaccccag ctacagggac gggaggaaga actgtagagc ctggggtcat ccaggtattc 62040

ctcaaggact ggacagaaag gcgtggatat tgaaggcagc tagtcatgtg cccaggtcag 62100

gggcagtgga gaccaccaat gaaccacagc ttgggagaga ggggcctcca ggaggccatg 62160

ctgtgtaact cccaagagtg gcagagacca gggagttgga gccgccagca gcctcacagc 62220

catcttgtgc cttctgagag gccccctgct tttcttttcc ctggggcccc acttgtgccg 62280

catcactccc atgcctgtag cctaccccca tcctggtatc tcccagccag agaccattga 62340

gggcagaggt gaggtgttac tggtgttatt gatcaccctc tctgagcact ggtacagagg 62400

aggtatagac aagtggaggc actgaggaca gaggaataaa tgagatgaat gccccggcct 62460

cctctctgag ctgacgtgga gtccctctgg ccttccctac cggctggccc tgtgtcttgg 62520

aggagctggg ctcctgcagg tgggcaggcc tagctgcagg gagcagctgg caagcagatg 62580

cctgtggcct gcagtacctt ggcgctggct tggttctgcg gggcacaaac aggtctgaca 62640

agttcctcag cagcagagac agggctcggt ccgtgatttc taggaaagtc aaagtcttgg 62700

agagcaagcg gagccaggct tggggtggag ggaggagctc ctctgctatg tacctgtccc 62760

ctgtgtcctg agcccagagc tccagactga tggtggtgct tctctctccc gcctcccaca 62820

gagggaacag gagctcagca gtggggaaag aggaggggag acctttcact gtggccaagg 62880

ggtaggggag tgtggcccct gtggtgaagc agctaccgtc aaagccaggg ctggccctct 62940

gcagggtcca acatgccact ctccttggcc tccttcctcc tggcctctca gggtctgaat 63000

tctacccctc aacccacagc ctccaatcct tggctccagg gacctgctcc cccaacctca 63060

gccacccgat tccagggtca caccttagac ctgctcttct cttccttaaa cactcggttc 63120

ttgtttctta cttttctttc ctttccctcc ctccttccct ccctcccttc cctccctccc 63180

tctctctctc tttctttctt tctttctctc tctttttctc tctctcttgc tttttttctt 63240

tttctttctt tctctgtctt ccttcctccc tcccttcctc cctcccttcc ttccctcctt 63300

tcttttcttt tcttctcttt ctctttcttt ctctcttcct cccttcctcc ctccctccct 63360

tcctccctcc ctcccttcct ccctccctcc cttcctccct ccctgccttc ctccctccct 63420

cccttcctcc ctccctgcct tcctccctcc ctcccttcct ccctccctgc ctcccttcct 63480

ccattcctcc ctccctccat tcctcccttc tcccttcttt tcttctttcc tcctttcctt 63540

tcctttcttt tctttatttt tttttctttt ctttttgttg atacagggcc tcaggatctc 63600

tctctatcac ccaggctgga gtgcagtggt gcaatcacag ctcactgcag ccttgaactc 63660

ctgggctcaa gtgattctcc tgcctcagcc tcccaagtag ctgggactac aggtgtgtgc 63720

caccatgcct ggctagtttt tttgtatttt tagtagagat gatgtttggc catgttggcc 63780

gggctggtct cagatttctg acctcatgtg atctgtctgc ctcggcctcc caggggccgg 63840

ggttacaggc atgcgccact gcatctgccc aacagttcct tagatataac acccaaagca 63900

cgagcagaaa tagctaaaat agacttcatc aaaatgtaaa actgttgtac atcaaagggc 63960

actatcaaga gagtacaaag acatcctaca gaatgggaga aaatatttgc aaatcacata 64020

tctaaaaggg tccaggctgg gcgtggtggg ctcttgcctg taatcccagc actttgggaa 64080

gccaaggtgg gaagactggt tgagcccagg agctcaagac cagcctgggc agctctccaa 64140

ataaataaat aaataataaa ataaaataat atagaaatta accaggtggg gtggctcatg 64200

tttatagtcc cagctacttg ggaggctgag gtgggaggat tgcttaagcc tgggagattg 64260

aggctgcagt gagctgtgat ggtgccactg cactccagcc tgggtgacag ggtgagaccc 64320

tgtctcaaaa ataaataaat agacaaataa attaaaataa aaagatctag tatctagaat 64380

atataaagaa cttctaaact cagcaacaaa aaagtgtaac acaattttaa aatgggcaat 64440

gaacctgaac agatttctcc caagagatac aaatggccaa taaaaacatg gtaagatgct 64500

caatgtcatt atccattaga gaaatgcaaa tcaaaaccca aatgaaatac cacttcatac 64560

ccattaagat ggttataatt ttggaagaaa agaaaaacag aaaataacaa gtgttggcaa 64620

gaatgtggaa aaattgaaac ccttgtacat cactggtggg aatacaaaac ggtgtaacct 64680

ttttgaaaag caatttggca attcctcaga aagttaaata tagagttacc atatgaacca 64740

gtaatttcac tcctaggtat atgcctaaga gaattgaaaa catgtattta cacaaaaact 64800

tgtacacaaa tgttcatagt agcgttatcc ataatagcta acagaaatat ctcaaatgtg 64860

catcaactga tgaataaaca aaatgtgatc tatccataaa atggaatctt atctggctac 64920

agaaaggaat gaagccaggt gtggtggctc atgcctgtaa tcccaacaat ttgggaggct 64980

gaggcagatg gatcacctga ggtcaggagt tcgagaccaa cttgaccaac gtggtgaaac 65040

cctctctcta ctaaaaatac aaaattaacc aggcgtggtg gcgtacgcct gtaatctcag 65100

ctactcggga ggctggggtt ggagaatcac ttgaacccag gaggcagagg ttgcagtgag 65160

ccaagatcat gccactgcac tccagcctag gcaacaagag tgaaaccctg tctcaaaaat 65220

aaataaataa ataaacaaat aaagtctaca acaaggatga accatgaaaa tgttatgcta 65280

agtgaaagat gccagacaca aaaggccatg tattgcagga tcctatttat atgaatgtcc 65340

ggaacaggca aatccataga gacgggcagt agtagcttca tggttgccag gggctgagtg 65400

caggaagaat aaggagtgac cactaatgaa tttcattttg tggtgattaa tattctgttt 65460

gtaaatagtg gtaaaaggca gggcgcggtg gctcacacct gtaatcccag cactttggga 65520

ggccgaggcg gttggatcac ctgaggtcag gagttcgaaa ccagcctggc caacatggca 65580

gaaccccgtc tctattaaaa atacaaatgt tggccgggca tggtggcacg tgtctgtaat 65640

cccagctact tgggaggctg aggcaggaga atcacttgaa cccggaaggc ggaggttgca 65700

gtgaaccgag atcacgccac tacactccag cctgggtgac agagtgagac tgtctaaaaa 65760

aaaacaaaaa agaagggggg ggttttatgg tatgtgaatt gtatctcaat aaaaaagttt 65820

ttttttaagt atgcactctt tcgcctcttt ttccatacaa catagtatat atgagcaatc 65880

aaccaaactg ttgtgtacat cagtagtttg ttcttattgc catgtggtat tcccatgtat 65940

gagtgcccta tcatttattt ttatttattt atttatttat ttattttttc agacagggtc 66000

tcgctgtcgt ccaggctgga gtgcagtggt gtgatcacag ctcactgcag ccgccaactc 66060

ctgggctcaa gtgatcctcc cacctcagcc tcctgaatag ctgggactat aggcatgcgc 66120

caccatgcca ggctaatttt taaatttttt tttatttgta gagatggggt cttgctatgt 66180

tgctgggact ggtctcaaac tcctggcgtc aagcaatcct cctgcctcgg cctcccaaag 66240

ctctgggact acaggcctga gccactgtgc ccggcctctt ttcagatctt tatggccatt 66300

tggatattat tattgtcgtc tacctttttt ggggttataa tctttctatt atagatatgt 66360

aggattttaa aaaaatacat cttctggata tgagatcttg tcatgatcta cgttgccagt 66420

atcttttctc gtccggtgcc tttttgcatt cttaactgta tcttttggtg agcagaagtt 66480

tttaattttg atgaagtcca acttatccac ttttatgatc acagttatgt gtgtcccatt 66540

taagaaatcc ttgcctaccc cctaaacatc ccttgaagcc acctcttccc ttctgacgtc 66600

ttcacctcta gccagctctt tgcagccagt tttctcccgt gcttctccat tctcctccca 66660

ccgatgtccc caccccactt tttgccccct cctctgcctt cctcactgca gtcagcacca 66720

tatcctgccc agggacagtc ctggctcctc tgagagtccc tgggaccagc acagcctctg 66780

gcatgcacta ggtgcttaat aaaagctcca taaatgaagg caatatcccg tggcttagca 66840

gggaatcgtt aaaactgtca attagctcta aacaccagct cctcgggaac atttgagcag 66900

agccagagga acaaaaaagg tttggaaaaa ccaacaagat aggaatgttt cgttttctcc 66960

ctcgctggag catttaccgc acaaactcag acctcattag cattatttat tcttgctttg 67020

tccccaaccg taggacaagg gaagcctttt tcctgcagcc tctggctgca agagctccct 67080

gtcacacccc acaacagtgt ttgctgcccc tgaatgtcgg ggagacttgt gatcccctaa 67140

tccttctctt caccgtgagt gatgacgtag cacaggtttc tgtagtccag gttgccgcac 67200

acatctgggg gaaatgctgc aaacatctgc ttgacctgag aaggagcaga gagtcagcct 67260

gcaccaggcc aagtgggggc cagagcagag aatccgccgt gacccaccag aatcccagtt 67320

tacctcctcc tcactgaagc ggtctgcctg ggtcataagt ttttctttga tgctgttcaa 67380

gggagagaca cagtcatgtt cagggaacgg gatacccagc ttgaacatgc agatcccagg 67440

ggtgatgagg ggcaagtggg ggcaggggga catcctggac caggaggaca tcaacctggg 67500

gctgaccctg ccagcccccc cgatggccaa gtagcttctg aacctcggtt ttctcctcca 67560

tgaagtgggc tttcctccca gcatcaggta gggaccaaaa gcaatgacct ggggacccca 67620

ccatgagggc tactctgatt tcccatcatg cactataatc aagctctgga tcattgaggg 67680

gagacacctc aggtccccca ccgcagagcc tcttttgcct gattctggac acactctttc 67740

ctccagcttg tgacgttcac tgacacctgc atagtgtttt agagacaaga tgcctgtgct 67800

ctgccaaggg tacaggaagg cctgcccagg tccgtgtcct cctgggcctg caagggcctg 67860

ccccctcaag cttggagggt cctgcagggt tcttaaggga gctcagtggg caaagatggg 67920

atcagacggc tgagtggggc ctcgtccacc cagagtgtcc tctggcaccg gctgcggcag 67980

gcctcgctaa ggaccaatgc acattttcca ttatttatgt ttctgtggga cagtcacagt 68040

ttccagctct ccaagccggg gacttttcac agactctgtt tcaaccagag cctccaccgt 68100

ggcctgtctg gcctgcctgg cctgcagcca cagatccagg tcccatgtcc caaggcagtg 68160

ccttcagttc ccaaattagg tccccaaagg gtcttcccag ccttgtcctt gcactggtga 68220

gagagaaagt ggttgacagt agagggaggg cttggggtta ctccgggggt gactctgatg 68280

cctccccagg tccccaggat ggaggtagga gccacagacc ccgagggagg ggtgaagggg 68340

aggactgtgg actgcagcaa ccctcagcat cccctcccca tcccagggga gtatgtgact 68400

catggtcttg gagttgagaa tgtgtgagac gggacagacc ctctccacct ccacccctgc 68460

tgcgtctgcc ctctcccttt gagatgcgag aatctcaaaa ccagcaaaac tagcctcaaa 68520

atgaaggctg gcagcattac agtaattgat caggaagcct gggcctgggg aggaatctca 68580

cctgccccag gccaccctca ggtccagcaa ggttctgaag ggactgcgtc ggcacagctc 68640

agaggtcagc cctgaatctt gggtcctggg aaggctgcca ctcccccgcc cactcctgcc 68700

tgcaattctt ggctctctgt tcccttgctg tcactgggtg gggcacccct accctcatga 68760

tggcagcccc ggtgcctgcc agacagctgc ctgcagccac ttccccttca tgcattccaa 68820

gtgagctggt cactttcccc aatccttact gccctcttgc ccgaggcggt gctggctacg 68880
atctggctca gtcctctccc cagaccctgg atcctatggg cactggcccc tggctctttg 68940
cttatctgtc cgcccgactc catctcgacg gccatgaccc tcctgggagc tgcaaccctc 69000
tcctctcctt ctccaggctg gcctccatgg tgtccttgcc taactcctcc tcaggttctc 69060
ccatgctccc agggcagacg cagggtccag gggctagggt gtgagcgctc ccccagaggc 69120
cccgcctgcc gctctgtgct cagtctcccc tacaccacct ccacccgcac cctccagctg 69180
cagccaactc tgaacaaaca tgcacatgcc aagccctggc cctccaagct ctctgttccc 69240
tcatctagaa tgctgttccc cccctagcct gggcaacctg cagtcattct tcaagatctg 69300
cctttgcccc cacacccacc ccactaggaa gctctcctgg agtgacccac cacttcttct 69360
tttttttttt ttttttgaga cagtctcact ctgtcgccca gtctggagtg caatggcgtg 69420
atctcggctc actgcaacct ccacttcctg ggttcgagcg attctcctgc ctcagccacc 69480
agagtagctg ggactacagg catgcaccac catgcctggc taattttttt tattattttc 69540
agtagagatg gggttttgct gtgttggcca gggtggtctc aaactcctgg tctcaagtga 69600
tctgcccacc tcggcttccc aaagtgctga gattacaggc gtgagccacc gcgcccagcc 69660
tgacccacca tttcttatcc atttctactt ccacgcacac atagagaagg ggacaccaag 69720
gcccagagag gggaagggac atcaagcgat atcactgggc tgggcagcgg ccccccagct 69780
cctggcttct gggagaggag actgggcgac catccaccct gaccccagcc caaagctggc 69840
ttattcccct gagcattttg ggggagtcag gggaaactta cacatcggcc ttgacgaaac 69900
ctttcccttc agtgtcgaac actttgaagg cgtggagaat ggtctcctct gggtccgtgc 69960
ctataagcag cacatacagg gcagggagag aggcaggtga agagggcccc acagggacag 70020
gcacaaggct gggcaggggc tggggctggg ggcaagtccc acggcccctg cacagaggca 70080
cagctctggg gaacttcttc cccaactttc actgcacagc gagggccgag ctcacttgaa 70140
gcaagaacaa ggagggaaac aggtaattgt aatcatagac aagggtacca catatatata 70200
ttttgtgaac aacctgagca actgtacacg gagaccctga ttgagcattt aatttgtgct 70260
tagcccttcc ctattttaca gatggaaaag ctgagaccca gagaagttaa gtgacttgcc 70320
cagggtctca cagctgcatg accagtggtc tgggtgaggg ggcatgactc tggggctcac 70380
gtgttaccca agctgtgcac aagaggccgt gggtggagga aaaagagttc tgtacgtgga 70440
tgccaaaagg ctggggcttg ggcagtgtgg cattggaggg gtctgagaga gattgtcccg 70500
gccagaagtg gggcaggtgg gccaacaggc caagggaagg gattcacggg ggcagaagtg 70560
aagccagaga agttctgctt ttatcccggt cacagcacag ccttggggag agatgtcatt 70620
agaagaaatt aatctgatac ccacaatggg attgaatgtg cagcccccac attgcaccaa 70680
tgggaaactg aagcccagag agaggaaggg tcttgcccaa ggcttcacgg gctgcgtgaa 70740

tgacagcagg tgacctggcc accctcgccc ggtgcccttt ccccttctgt caccatgtcc 70800

cccttgttca ctgtggatgc gggggtgccc ccttcctttc ttcagtgctc aaatgtgtca 70860

ttggtgaaag gggacagaga tggtgcttgc tacccacctc acaggagcag ctaagataac 70920

ataccattca tggtaaggtt ttaacgaaca tgggttgatc agggctgcaa atacatctga 70980

gcgctctatg cctgcagctt taagatttta tcccctggag ctctgagagc acccatggag 71040

tagatgtgga tatcttggtt tatcatagaa aaaacaggct cagagatgtt tcattacttg 71100

cccaagtccc cactgataag agaagggagg atttaaaccc acctctgtgg actccagagt 71160

cccctgctcc ttttgtcacc ctgtgctctt gccagtcaga ttaggtcagc cagggccatg 71220

tgcctccctc atcagactag gtcagggcca tttgttcctc caccatcata ctaagtcagg 71280

accatctgtg cctcccccat tagagtgggt catcgccatc tgttcctcct gcatcagact 71340

gggtcagggc catctgtgct tcctccatca gattagatca gggcctcatc tgtgcctccc 71400

ccatcagact gggtcagggt catctgtgcc tctcccatca aactggatca gggccatctg 71460

tgtctccccg atcagactgg gtcagggcca tctgtgcctc ttccatcaga ctggatcagg 71520

gccttctgtg cctcttccat cagactgggt cagagccaag tgtgcttccc ccctcagatt 71580

agatcagggc ctcatctgtt cctcccccat tagactgggt cagggccatc tgttcctccc 71640

ccatcacacc agatcaaggt catctgtgcc tctcccatca gactgggtca gggccatctg 71700

tgcctccccc atcggactag gaactagcca ggactgggca gtgtctgagt ctctgggcat 71760

ctctgaaact ggcacagagt gaaaaaacct ggcacacact ggccttgctg gcctgggaac 71820

tcctagaggg cagcgccatg gactgcctct gggcagggtg gcagggagct ggtgtgccca 71880

gcagagaggc tcagcccagg gtgcagcgag ggcagggctc caggccctca cagcctggcc 71940

aagcaggcag ggggtagcag gtgcctctag gcgggtgagg gttgagtgac cagagcatca 72000

cgtggggcac ccatgtggga tttgcctccg aggccacagc ctcccaggtt tcactgatta 72060

gttgaaaatt cccaacgtct ttgaagaggc ctcactgctg aggcaggttt gtggccggct 72120

ccagcctatc cccctgcgga caatgcacat ggatttaggc acccagcgca gccctgccac 72180

tccctgttac cgtggctggc agtcctccct tctctcctcc ccagaaagag cctctgttct 72240

tgagactgca aacctcataa aatctgctat agagtggcca cttcctccca tcatcgttgg 72300

gaatctcatg acacagtctc ctcttcccaa ttagcggggt gaggcggcgc cggccccagc 72360

accctgccct cccggctcct gtcccctccc accagcctct cacgcagtct ggccacgtct 72420

gcatgacagg ctcagccgga gccccagtcc cggcgcccct gcaaaaatga gaaaaagctg 72480

cagccctgca gcccacacca ggccctggat cacagcttca gctcaaccat ggaccgacac 72540

gagcttttag tctcaatctt cccatctgcg aaacaggttt ggggcccact gtgagctgac 72600

agagtcccct cccctccccc tgagcactct tggaggtctc tctaaactcc tgctctggca 72660

gcagggcccc tcgcccatgc ccaacaccag gggcgggagg tgctcacagc ctcacctcct 72720

gggcccggcc ctgggccgtc caccctggca gaaccacagg ccacttcccc aggccctccc 72780
actggccttt aacacgcctc cttcagttat gtgcctgtgg ataaaagaat caggcctgtc 72840
tgcttccttt gaagttgctg gctggagtca cacatgtctt ttccacagag cattcttctc 72900
tcccggcctc ctgggcttct gagctcttcc aggaggcgca catggtcctg ggaataggcc 72960
ccgacaagct gcctggaggt gggcccagtg atgctcctga ttcggctaca cagccccaag 73020
ttcaaagcct gctccagatg agcctgccct ccccagccct gggtttcctc atctctcgca 73080
cccaggctga tggctgcctt ccttctggct ctggatctgc caggacacgg tgcgtggagg 73140
ctgctctacc ccacagcagg tcacgaccca tgacagtact gatttttcac cccatcaaac 73200
ctgtgatgtc aggtcggatc accctgtggt ggactgaatg gtggccccta aaaagagacg 73260
tccaccagag gccgggcacg gtggcttacg cctgtaatcc cagcactttg ggaggccgag 73320
gaaggcggat cacgaggtca ggagatcgag accatcctgg ctaacacagt gaaaccccat 73380
ctccactaaa aatacaaaaa aattagacga gcgtggtggc aggcacctgt agccccagtt 73440
acttgggaac ctaaggcagg agaatggcgt gaacccggga ggcatagctt gcagtaagcc 73500
aagatcacac cactgcactc cagcctgggt gacagagcaa gactccgtct caaaaaaaaa 73560
aaaaaaaaag atatgtccac cagagcctgc aaatgtcacc ttatttggaa aaaaggactt 73620
tgcagatgta agttaagcat cttgagatgc agagatcatc ttagattatc ccagtaggac 73680
ctgaatccaa cggcaggtag agtccaggcg cgagggctca tgcctataat cccagcagtt 73740
tgggaggcgg atatgggcgg atcacttgag gtcaggagtt cgagaccagc ctggccaatg 73800
tagtgaaacg gtatctctac taaaaataca aaaaaattta gcggggcatg gtggcgggtg 73860
cctgtaatcc cagcttcttg ggaggctgaa gcgtgagaat cgcttgaacc ttggaggcgg 73920
aggttgtcct aagctgagat ggcgccatta tactccagcc tgggcaacag agtgagactc 73980
catctcaaaa acaaaacaaa ataaacaaag aggcaggtgg agggaggtta aatagaagag 74040
ggcggggcca ttgacggcga ggctgagatg ggagaggtag agccaccaga ggctgggaga 74100
gacaacggat tctccccaaa tcctctgcca gatttcgcac ttctgggctt caggactgtg 74160
agagaataca tgtctgttgt tttgagccac ctggtttcac agaatttgtt acagcgggct 74220
cagaaaacta ttataagccc attttacaga ccaaaaaact gaggccagaa agcagataaa 74280
tctgtgcaag gtcaacctag gagtcaatgg gaagccaggg tctgacccta ggcctagcca 74340
aggcaggggg ttcaagggag ggggcctggc aggggcagcc tgagggtgca gagctgacag 74400
tgctcacttc ccagggacca ctgcctgggc tgacccttcc tttttttttt tttttctttt 74460
ttttttttgt agtggagtct gtctctgtca cccaggctgg agtgcaatgg caccatctcg 74520
gctcacggca acctctgcct cccaggttca agcgattctc ctgcctcagc ctcccgagca 74580
gctgggatta cgggcaccca ccaccatgcc cagctaattt ttgtattttt aatagagaca 74640

gtgtttcgcc atgttagtca gggtggtctt gacctcctga cctcaggtga tccacccacc 74700

tccgcctccc agagtgctgg gattacaggc atgagccacc gtgcccggcc ttggcccttc 74760

cttctgacca cggaaccctc aagctccagg atgtcactat gggagagctc ccccgaggcc 74820

aggacagaaa ggagacacag atggctccaa ccctccaccc catgcctaat gctgtgtctc 74880

gggcgggtac agtggttaga gccagggttt ggggttgagg ttgcatcccg gccacttttt 74940

gctggagcat ctggggcagg ggtcgtgact cctctggatc tgagtctctt catttgtgaa 75000

tgaggctccc cgatccttcc tctgggggct ccagtgacca cagaaaaaga caaggggagt 75060

aacttgccct gcacaggggt cgaccacagc atcttttgga aacaccctcc ctccggcacc 75120

ctctgcctat acccccagcc ccatgtgcag agcgggtaaa aggagctcac aggtaggaag 75180

gtagggaggc agcatggctg ggggcagtgg gatgggtggg caaaggggtt aaaggcagtg 75240

tttagttttt atggttgttg ttgttgttga gacagggcct tgctctgtca cccaggctgg 75300

agtgcagtgg tgcgatctcg gctcactgca accttcacct cccaggctca agtgatcctc 75360

ccacctcagc ctccaaagta gctgggacta caggcatgtg tcaccatgtc tggctaattt 75420

ttgtattagt agagatgaag tttcattata tcacccaggc tggtctcaaa ctcctggact 75480

caagcaaacc gcccgcctca gtctccctaa gtgctgggat tacaggcgtg agccaccatg 75540

cctggccaag gcaggggtct ttatagttac cccagtctgc ggtgaagaga gggaaaccga 75600

ggcagggccc ctgaaggtgc gtgcacctgg gggctggtgt ctggggccac atcatgctcc 75660

cacctgtggt gtgtatgtgt gtcaggcccc tgccttccta catccgtccc cctgccattc 75720

cctgctgccc ctccaggact ccaggagcct ggctcaccct tcagcttctc cccaaacatg 75780

gtcaggaaca ccgtgaagtt gatgggtccg ggggcctcct tcaccatggc ctccagttcc 75840

tcgttcttga cattgatgcg gcctgtggga ggtgagaggt gggggcaggg aggataagag 75900

agatcagagc ttgggcctcg gaggatccca caaagcctcc ccactcagct gctccacgtg 75960

ccccccactc agccctaagg ccctgacgcc tttttggggg actctttagg gggcatttgg 76020

gccacagtca gcttccggct gcccccagcc tcatgagaag ggagcaagtc actctcatcc 76080

ccattttacg gtgaagaaac tgaggcccaa gaagctgcag ctgaggtgca cgggggctgc 76140

aggctcaggg ttcctgtgcc ccaggagggg atcagtggat gcctggatgc tgggattccc 76200

tgtggttcca ggggacactc agattcccag gggatcaggg cctggttacg tccgttctgc 76260

cacctaaacc ctgcataggc gacttcaggc tcttccctgg ggccttagag ggatcagaaa 76320

cctcaacctg ggagttgggt ctttccctct gactcagctt ctcccctgct ttcctggccc 76380

ctccccacac ccagggtccc ctacaccagg cccaggccca ggcccaggcc catgctgcta 76440

ctcctgtccc tgaagtgttt tccccttccc tggccgaatc cttctccaac cccccaggaa 76500

ggtccccccc tgactttcca gacagtggct gcctttaccc tgtgctcccc agcacaggag 76560

cctctcacgc tcaccgcgag ccctgccctg ctggccccaa ccgcccactc tgtctggctg 76620

gcctggccct aatctccccc ggctgctggc tcacccaccc agcgcggcaa aggtgtccct 76680

caagtcctct ttgtcgatga agccatcacg gttctggtcc atgatggtga aagcctggca 76740

gagacacagg tgctaagtag tcacctgccc ttccaagccc ggccacctcc cccagggacc 76800

gtcctcctgc cgactgctgc ccaaggagcc tcggggcagg tccctctggc agcccagctg 76860

ggatgggctc catccccaac ccctcatttc aggctcacag caactccagt ttgcagacag 76920

ggaaactgag gctcagagag agaaaagtct ggcccgaggt cacacagcaa gcacagggtc 76980

aggtacagag agacaaagag gctgcctgag ggcaacaggt cactcaccca ggctctgggc 77040

catggccatg gtggcctcaa aaaataatgt cattggccag gcttggtggc tcatgcctgc 77100

agtcccagca atttgggagg cagaggcagg aggattgctt gaggtcagga gtttgagaca 77160

agcctgggcg aaataatgag accctgtctc tacaaaacat taaaaaatta gccaggcatg 77220

gtgatgaatg cctgtagttt cagctactca ggaggctgag gtgggaggat ggcttgagcc 77280

caggaatttg aggctgcagg gagcattgac tgcaacactg tactccagcc tgggtaacag 77340

agcaagaccc tgtctcaaaa aaaaaaaaaa aaaaaaaaac ctaatcattt aaaaatgtag 77400

taatgggcgc tgggcactgt ggctcacgtc tgtaatccca gcactttggg aggccgaggc 77460

gggtggatca cctgaggtca ggagttccag accagcctgg ccaacatggc aaaaccccat 77520

ctctactaaa aattcaaaaa attagctggg catggtggtg cgtgcctgca atcccaccta 77580

cttgggaggc tgaggcagga gaattgcttg aacccgtgag gcagaggttg cagtgagccg 77640

agatcatgcc attgcgctcc agcctgggcg acagagccag actctgtcaa acaaacaaat 77700

aataataata ataatagtaa taatgacatc ttcagcccct cgagactgag gactgagcct 77760

ctgcccagct ccttgagcaa ctggcatgca ttccaagaat cctcataaca gccccatggg 77820

gcagcagacc aacctcttta aactcctgga tctgggactg atcaaacatg gagaagacgt 77880

tggagctggc ggtgccttct gctcttttcc gagctcttct cggtgcctgc gaggtagcag 77940

acaaggcctt gcagcggccc ctgcctcgag ggtcagcccc caccccctgt ctcacctccc 78000

agggcatcac ggtccagaat gacaccgctt tgcccttccc ggtgcctaca gcagacagac 78060

aaggctaccc ctgaagcctt gcaggtaatc aggacgtttt cccagcccat ggcaagaagc 78120

ctgcacggcg tgcaccccca cagagctgga cagctgcttc ctggccctgt cccccgggct 78180

ccggaaggtg ggcagccggt gcccgtgctg cagaacagag gcgggcatct cttccttcag 78240

cctccccatc tctaccccgg catcctccac tacagctgga atctgacctt atcgccagct 78300

gctaaaaacc ttcttcgtgt tctcaggaga acaactgcgc tcttagcccc accacaggat 78360

ctgcctcttg ccctgtccca gagctgctac tccctctcac ccaggccctc tactctgtcc 78420

cagagctgcc ttccctccca gcgtttgccc ctgcctggaa tgcgttgctc tgttggaatt 78480

ccctgcttgg aatgccttcc ctggagaatc tatctagaga ctcctaccca tcctgcaggc 78540

tcagctccct gtcacctcct ccgagaagcc ttccctgacc ccaggccact ctcccctccc 78600
cacatggctc cactgtggac tgtcacccct acagcatgta tcacactgtc ctgacatatc 78660
tgtgcgtctg tgtcctcgac taagtggaca tctcaggggt aaaacccact ttgccccatc 78720
tctgatctct cctatcccca ccctgaacct ggctcaaagt ggggatcagc cagtgcttgg 78780
aaatagctgc ccagaactca gggccaaccc acgcagcagg caaagcagga ggccacccac 78840
gttgagtagt cagatggttt ctggccagcc ttcctgcctc tgtgatgtca gggtgtctgg 78900
aagtccacca ttgggcattt gctgaaagcc ctaggtcccc aagaccctca agagtcctgt 78960
tcctgggcag ccatccttac agcccccagg ggcgctgtga gccccagaat gggctctcga 79020
tgggggaaaa gaattgctgg attccagacg gggaggtgga cgggatggca cagaaatggg 79080
ttcccaccac cagctcctgt ctccgtcaca acttgtacca ccctctcctg ccaagccctt 79140
gtctacctgc ccccaccaga gcagaggagt cccctcatcc cctgctcccc tcccccaacg 79200
cctgtgtggt ctcccctgcc cccagctcac tgctgtcccc acccccgccc aggagcccgc 79260
gtcgtcgagg gcaggggcag aagcgccggc tgacttataa ttagctttta ctgggtgctc 79320
tgtctctcct aatcctttga aagggggctg ccaggcggcc cgggacgtta tgatgctgta 79380
aaacctctgt aatatttata aatcctttca gacatcataa agaaaagaac ttccccacag 79440
agttcatccc acataattac atatacattt cctccgatta ataaaaaatg atcacttccg 79500
tctgtgggag gttctgccct ttctctggca cacctggctg tggcctcggc gtcctcagtc 79560
cctgcccccc acgcccccag cctctcatga tccccctggt tacaccccca ccctacactg 79620
tcccagagct ccacgaggga gggagagatc attttcttta cagcacaaga gtcctaaacc 79680
ctagggtctt tgcagcccag ctgtcttgaa aattcctttt gagaatgggc ccctgaaaaa 79740
taaaattccc ctgaaaggaa catctcaggg aagaaggggg ggtcacccgg atctgggttc 79800
aaagcctggc ttggctgctc ccagcctccg tgatgttaag aaattcactt gtcttctctg 79860
agcctgtttc cccatttata aaacctagag caggtcaacc tcaaagcatc aggacatgta 79920
ttagacacct acggacctcc aggcaggtcc gtcctgggga cccagggctg aatcaggccc 79980
cgtcttggtc cccaagaaga tcacagtccc gctggtgaca ggcagtccat agaggctgac 80040
tcggaggagg gacaagcgct gagaagacaa gacagagaag cccctaggcc ctcgggcagt 80100
caggaggtga caagccgagg ctgacagcca agtcagagct ctgtgccgag agggactaac 80160
taatgaggca ggtgagacac aggcacagag ggacagccag attgataccc cgcagggagt 80220
ccccaggggc aggaacccag agcaagccct gcgtcacatg aggtgcacag gcaaaggtga 80280
acagggggac ctcggagctc cagaggtgcc acgctgcccc aaatactgac ccccggccct 80340
cccagggggc ccctgccctc agagccactg cccctgtgaa gcccccaggc cctgcccacc 80400
tcgtctctct ccaccctcct cttcctcatt ccccagcagt ccccacaaag tccaggaaag 80460
cagaggctca aatcaggacc cacgtggaga ggagtcccga gtggggcagg tccaagcatg 80520

ggatcaagag caaagagcag gtcagaccag ggacaacatc agattaaggt caggaaggtc 80580

agtgagaggc aggatgaatt caggcaggag gtgaaggtca gaatcacaca cagagtcaag 80640

gtcacagaac aaggtcaaag ttgagaaatg aggccgggcg ctgtggctca cgcctgtaat 80700

cccagcactc tgggaggccg aagcgggtga atcacttgag gtcaggagtt cgagaccaac 80760

ctggacaaca tggtgaaacc ccgtctctac caaaaataca aaaaaaaaaa aatagccatg 80820

tgtggtggcg ggcgcctgta atcccagtta ctcgggaggc tgagacagga acctcactcg 80880

aagccaggag gtggaggttg cagtgagccg agatcgagcc accgcactcc aacctgggca 80940

acagagtgag attctgtcta aaaaagaaaa ggaattgcgg agtgaggtca ggggcagggg 81000

caggggcagg ggcaggggtg ggagggaggg gaccgcagtg ctgaagccaa agacacaaca 81060

gccacaggcc tgagctcccc agggaggagg tggaggtgag aggaaggtag ccgtggcaga 81120

gcagaacaac cccagcctac catggtctgg agagctccct gaagctgcag ttggcctggg 81180

gatagctcag agatggccct taaatagtgg tcctcccagg gcccctggca tgcaccgggc 81240

ccagtgtgga aggtgatcca tgacaggacg gagacctccc tgccaggcac ctgccttggg 81300

ccattcagag tggatagggt tggggggaat ccgtgggcaa ataccacgga cgcagggact 81360

gcctaagcct ccaggatctc cgagagccct gggccaactg ttttctctag agccaaggga 81420

ccaagaccca ggaaggggca tttggccaag gacacagagt aagttgaggc tgcctacccc 81480

aatgggaaga cttggccttg agccgtcagg gagtgttgcc cagggtgggg ccacccatgc 81540

tggctgggac ccactcagac tgcagtgtgg ccggacctct ggagctgtgg gactctgggt 81600

gggacatcac cctctaggag gctcagccat ctcataggaa taggggggag tgtatgaaac 81660

ccccggtctg cttatggaga gagtttctgc accaacctgg ccctcggggg ccccttctac 81720

caatatgcct attatcaggg tgtccattaa gcacccccag gccaggggac cccctcaggg 81780

cactgaaatg ctcaatgaac agagaaagca acaggacaaa taaaagaaaa atgggtgggg 81840

cgcagtggct cacgcctgta atcccagagc tttcggaggc caagagggaa ggatcgcttc 81900

aggccaggag ttcaagacca gcctgggcaa catagtgaga cccctatctc tacaaaatat 81960

caaaaaaatt agccgagcat ggtggtgtgc tcttgtagtc ccacctactc aggaggctga 82020

ggcaggagga ttgcttgagt ccaggagttt gaggctgcag tgatctatga ttgtgccact 82080

gcactccagc ctgggcgaca gagtgagata ttgtctctta aaaaaaagaa aacagaaaac 82140

tggaggtgac tctgatgagg aagaactgaa aaatgagacc tccattccac cctggcctca 82200

cacacacaca tccatcccca ccaggcctgg cccagcccaa gaattctcta gtagatctgc 82260

caaacagagc ccacacctgc cctggaaagc ccctcctgtg tctagtgcag ccccagcagc 82320

cccagctcgg cctcagaggc tgccactgtc catctggggc ccgccatatc tgaacagctg 82380

ccagggccgg cagtgctggg ttcaggcagt gagcccacgg gtccccaggg gctggagagc 82440

ttaactgtcc ctggagctgg cctcaggcct tcagccccgg gtacagctga tcccctgcct 82500

ttggggaagg gaatatcata ataataataa gaagagcagc taatatacaa cacagaaggt 82560

caggccatgt cttaaaacac gtgtcctcac attcaatcca tatcccattt tccagatgag 82620

gaaactgagg cacgcatggg ttaagtcact cacccaaggc agataagaag tggggccaga 82680

ggccaggcac agtggctcat acctgtaatc ccagcacttt gggaggccga ggagggagga 82740

tcacttgagg ccaggagttt gagaccagcc taagcaacat ggtgaaactc tgtttctaca 82800

aaaaaatttt ttttaattaa aaaattagtc aggcatagtg gtgaagtgaa aaagttccct 82860

tatcccgttc atagggcatg cgatgggggt gtggctcgct tcttccatgc ccagctgctc 82920

aaacctctag gcggaccatg cagacaggca ggctatgggg ctccgacccc acggcagtgt 82980

ctaggggtgg atgcttacag ctcctgaagc cccagtgggc gtgtgttaca ggatgctctt 83040

ttacttttgc catctgtagg tggcttgtgt tcatcagctc agttagaccc tctgccttat 83100

tgcaaggaca aagggctttt tgtatcctgg ggttcttgcc ttggtgtact gtgaaaattg 83160

aatcacatgt gggcttggag aatgagtgcg aggttttatt gagtggtgga agtagctctc 83220

agcagatgat ggggagccag aagggggatg gagtgggaag gtgatttttc ccctgaagtc 83280

aaggtgctca gcagccagac tctcctctga ccatccccag ccaaattccc ctgggcattt 83340

gtgttattca gccagtcgat ggcctgccag catctgccag tgctgtcggt gtgctcttcc 83400

gatcctctgc tcctcttgat gtccagccac ctgtgtgtgt acccactggg gtctcagggt 83460

tttcataggc acaggatggg cgggggggcg gggggcgtgg tgggccaggg tggtcttgga 83520

aaatgtgaca tttgggcata taaacaggag tgcctgtcct cacctaggtc cgtgggcaca 83580

ggcccaaggg tggagccctt gccagggacc ctgcctttct cctctcagca cttccctgtc 83640

ccccctcctg tatcagtggc acatgcctgc aggcccagct acttaggagg ctgaggtggg 83700

aggatcacat gagcccagga ggttgaggct tcaatgagct gtgattgcac cactgcactc 83760

cagcctggac aacagatcaa ggtctcattt ctaaaacaaa aaaaaaaaaa aaaaaaaaga 83820

gagagagaga gggaggccca ggacaagagc ccctctttct agtgtttcca gaaggaagct 83880

tccaaaaggt tctgctcatc caaactctcc cctcctcctg ctccctccca gtcatccctg 83940

actacagcta tttctgatgt ccctaaactc ctgtgactga gtgctcctcc cagagctggg 84000

gccaggtcca gccatgatgc tgtgcagccc gctttctgca gcaggaaacc gtgtgcctca 84060

gtttccccga cagggctgct ctcctggtgt cctggcgttc ctctccgggg agaaaaacca 84120

cacgttccac aggtgttcaa caagccaggc ggaaaggaga acccagctct ggggacggga 84180

gcctggattc cagtcccagc cgcctggacc tgctacagaa gggggcctgt gtgaccgcga 84240

cagtggctag ggtgcaggct ttccatgccc ggtctgtgag cctggccctg gggcctcctg 84300

gctctgggct tacagagtgg gtagcagacc tataactcct gcttcctgcc tctggcttcc 84360

tgctgccctg aggcctctgc aagcaagggc tcttgcctcc ggggagcacg cccaggaccg 84420

tggcaggggc tcccaaaggc cggggacagg cctgagcaag cggagagccc ccccgcctgc 84480

ccggaggagg ggaagcggcg ctggtgaatc acaccgggct tacaaattag tctgagtgga 84540

tttcctcccc tggcctcctc cttcatagga ccggaggcag gaagaaccgg tgagaaatct 84600

ggtagagacg tgaaaacccc ggggtgagat ggttgggaga tgtttgttcc tggggggcgg 84660

gaagtgtgag ggtgcagtgg ccttgagaag ggggagccgt gagttctgca cccacagtgg 84720

gaccctcagt tttccccatc tgagaaatgg tgccagcagc caggacccct tgaggctgcc 84780

atcgggaagc ttagtgccat tggaaggagg tgggctttgg ggtcatcggc ctggggtttg 84840

gagggcagag ccttcccctg tccccctttc cccctgtgta gatggagtcc ttgacagaac 84900

ttgcagcctc tccaagctgc agccccctca cccaggtgac actgcaatga gtcagttgtc 84960

gtggctgggt cacactgttt gactcaactg accacagggc cagggccagt tcttggggtc 85020

agagacactc tgagaggtct ctgctcccag ccaggggacc ctggagagta agtgtcctct 85080

ggaactggaa ttccaggtct gcctggggtt gagtcagaga atacttagca ctctcggttt 85140

ccagaggtaa ctgaggtcct gggagtggcc aagccaggaa gggagcttgg aacgagccca 85200

gctgcctggc agggggacag ctgttcccca gacaacactg gaacaagccg gggagatgcc 85260

ttcccctttc aaccctcacc cctaacctct cacctataac tcctcagggt cagcaggctg 85320

tccctgtccc ccagccccac agcccttgac cccttccctg gccagccatc accctgacct 85380

tggctgttct gcaaattctt cccgaaatct ccttcctcca agcctttggc cacattgtat 85440

gtttccctac tcatcacaat cagtctccag cctgctttag aacacctctc ggacggccgt 85500

ggtggctccc gcctgtaatc tcagaacttt aggaggccaa agtgggtgga tcacctgagt 85560

tcaggaggtc aagaccagcc tggacaacac ggcaaaacgc tgtctctact aaaagtacaa 85620

aaattagcca ggcgtggtgg tgtgcgtctg taatcccagc tactcgggag gccgaggcag 85680

gagaatcgct tgaacctggg aggtggaagt tgcagtgagc tgagatagca ccactgcact 85740

ctagcctggg taacagagtg agactgtgtc tcaaaagaaa acacaaaaaa caaaaaacaa 85800

caaaaacaaa aggaagccaa cagtgctgcc ttcagtctgt ggccgaaggc ccaagagctc 85860

ctggcaaacc actggtttaa gttcaagagt tcaaaagctg aagaacttgg agtctgatgt 85920

tcgagggcag gaagcttcca gcacagaaga tgaaggctgg aagactcagt gagtcttctt 85980

attccacttt cttctttctg ctttattctg gcctcactgg ctgattagac gctgcccaca 86040

cagattaagg gtgggtctgc ttctctcagt ccaccgactc aaatgttaat ttcctctacc 86100

aacaccctca cagacatacc cagaaacaat actttgcatc cttcaatcca atcaagttga 86160

tacttaatat tagccatcac acctacctac tgaatttgag agcctaggcc actttctgac 86220

cttccctgag caatcccagg ctgccgtaaa aaatgcctgc aaattctttg acactttttc 86280

catggagagg cgggatctat aacatcattc cttgccaaca ggcaggcttt tgtgactgct 86340

ttgaccaaga aagtagggta gaagtgtcac tatgtgactt ctgaggttaa gtcacaaaaa 86400

gccatggagc ttctacctta tttgctggga ctttgtgctg gagccctgag tcactctatg 86460

acgagtctga ctaccatgag gcggccatgt tgtagggaag accaagtcac acgaagaggc 86520

cccgtgtctg tactccagtc agcagtccct gcctttgagt catcatccga gcctcccaga 86580

caacaaacgt gtgaatgacc agtcttcaag tgattctggc accagccatg gagtcatccc 86640

catccttaga gtcccccgag ctgaggccct agacattatg acaagccacc ctcaccatcc 86700

cttttctgaa tttctgatcc ataagaaccc ctgagcacac tgctatgctt tggggttttt 86760

ttttgttttt tgtttttctt tgagatagag tctcactctg ttgcccaggc tggagtatag 86820

tggtgtgatc tcagctcact gcaacctcca cctcctgggt ttaagtgatt cttctgcctc 86880

agcctcccga gtagctggga ttacaggcac ccaacaccac gcccagctaa tttttgtatt 86940

tttagtagag actggtttca ccatgttggc caggctggcc ttgaagtcct gaccacaagt 87000

gatctgtccg cttcggcctc ccaaactgct gggattacag gcatgagcca ccatgcgctg 87060

tgctttgggc tgattcttta caaaatatca taataaccat cacagtgaga tttaaatgtg 87120

ctctgtaggg ggacaggcta cacagccata gtctactcct cagggctctg gggaagatga 87180

gagcatgaat ttgaaatcag atgaaagtga cttcaagtcc tgctggacca cttgctctgt 87240

gacctcaccc acatcactga atttcccaga atcctcagct tctccacctg tcacatggaa 87300

tcagttttca cttggcaagt ttgttacatg cctggcgctt gtagactgcg gctaccaatc 87360

ctgtctggtt attggcagag gacatgctgc ctgtgacaag acactgagcc aagagatgcc 87420

tctgttgggc ttccagaacg tgcttcctgt gccaaggctc cagtgtcctt agtggaagag 87480

agacgccctc acggcctgcc ctttctggcc tggccagggc agcatgactc attggatgac 87540

ccctcgcccc atcccaaatg tcacagccct gcaccatggc ttcccttaga ccctgtggcc 87600

caaggacaca cagtaggctg ggtccacagg aaacacccaa agccacagaa tgccatttgc 87660

ccactccccc atctgtatct cctttaccca tctctgtcac atattgagaa aatggctcca 87720

gattgggggt gcacacagcc cctgagctcc tgcagccatc aaggctcctg acaggctgga 87780

gccgaccgag aactagaggc cggctcaggc cagagcagcc acacatagca tttcagacag 87840

accctgactc agggccagtt cgcctctttc tcccttaagc cagaggaaat agcctcgtat 87900

gtccatcaac tctctcattg ccaggtggga tgcctgagat gttgacagtt agagacggtg 87960

ggctgtcttt tccgaaagcc cctggtgtcc cctcctcccc agctcccgtc ccatctggag 88020

ccttcttcca actggagatc acttagaaaa gacaaagaaa cctttaaact aagaatgcct 88080

cctggaatgc ctcttgcctg ttttatagct gcagctcagc gggctggtga atgtgctctc 88140

tcgcccacag aagctcaaga gaggcatccc attcaagggg cctgtggtca cagcatcctg 88200

ctgtaaaaat agctgtggat ggccaggcac ggtggctcac gcttgtaatc caagcacttt 88260

gggaggcaag gtgggaggat cactgaggtc aggagttcga gaccagcctg gctaacattg 88320

tgaaacccca tctctgctta caaaaaaaaa aaaaattagc cgggtgtggt ggcacacacc 88380

tgtaatccca gctactcagg aggctgaggc aggagaatca tttgaaccca ggaggcggag 88440

gttgcagtga gccgaaatca caccactgca ctccagcctg ggagatggag gaagaccctg 88500

tctcaaaaaa aaaaaaaaaa aaaaaagaaa agaaaagaaa aaaaaagctg tagaatcaat 88560

ggctgacagg aggagaaagg ggtagggttt ttctgcccag tgggactgga agtcacccta 88620

aaagtgagta gatgaggtct ctgagccagg tctgggaaca tgtggctgga tcttgtttgc 88680

tctttttttt ttttcttttt tttgagacag ggtctcactc tgttgcctag gctggagtga 88740

agtggcacaa tcagggctca ctacagcctc ccagcctcaa gcaatcctcc cactttaacc 88800

tcccgagtag ctgggactac aggtgcacac caccatgcct ggctaatttg tgtatctttt 88860

gtagagacga ggtctcgcta tgttgctcag gctaacctca aactcctggg ctcaaacgat 88920

cctcccgcct cagcctccca aagtgctggg attacaggtg tgagccaccg cacccagccg 88980

gcttcctgac ttttgaggtt ttgggactcc aactggcttc cttgctcctc agcttgcaga 89040

cggcctgtta tgggattgtg tgagacaatt ctccttaata aactcccttt catatataca 89100

tccatcttat tagttctgtc cctctaggga accctggcta atacagtagg ggagccagaa 89160

ttcagatcca ggcctgaggg actctgtccc acagccaact atgtgagccc aggaagtcca 89220

ggaaactcag tggggggtgg cacagcatgg caggtggtga ctctctggct acagccagga 89280

aggcctgcat ttggaaagga ggccaagaaa ggaagagagg tcggcccccc ttttgctgct 89340

gagagtgacc ttgagaggag gctgggcctt ccttcaatgc cagctccctc tgactcagcc 89400

tggactgggg catcacctct ctgggtctgc gtgtccccac atgcccttcc ttaagcccct 89460

gacccaagcc ctgggggagg gaggggctta cactgctccc tcttgcctca ctctccattg 89520

acagctgtag aaactgaggc tcacaggctg ggcgtggagg ctcatgccta taatcccagc 89580

attttgggag gccgaggtgg gtggatcacc tgaggtcagg agtttgagat cagcctagac 89640

aacatggcaa aaccctgttt ctagtaaaaa tacaaaaact agccaggtgt ggtgctgcac 89700

gcctataatc ccagctactc gggagcctga ggcaggagaa tcgtttgaac ccgggagaca 89760

gaggttgcag tgagcccaga tcgtgccact gcactccagc ctaggcaaca gagtgagact 89820

ctgtgaaaga aagagagaaa gaaagaaaga aagaaagaaa gaaagaaaga aagaaagaaa 89880

ggaaggaagg aaggaaggaa ggaaggaagg aaggaaggaa ggaaggaagg aaggaaggaa 89940

ggaaggggaa gggaagggaa gggaaggaag gaaggggaag ggaaggaggg gaaggaaggg 90000

aaggaaggaa ggaaggaagg aaagaaggag aaagaaagaa actgaggctc aggagggctg 90060

agcctcctaa gccagctgct tcaccaacct tctgccagct ctagcccttt catccttctg 90120

ccctgtcagt ccctgccctc tgcaagtggg acaggggtga ggggagtgta ggagctcagg 90180

tacgtgcatt ctccagcccc cgtggcattt ctgcaggggt ttggagcaga agagaatgat 90240

gaggtgggca gaatccccgg agcctgcgtg gaggaggaga gagaggatga gctgggccct 90300

gctggatgcc tggtttccct atctttcccc taagagccag gccgacctcg tcctgctgga 90360

cggctgagga aactgaggcc tggagcagcg aggggcctgc tcaaggtgca ccactgcaat 90420

gccagccgcg actccaccca ccccattccc taagggatgc ctgggaggtg aataacctgg 90480

aagttcaggt gggacctccc agaggatcta ggcatccctg tagtggacag tggctcaggc 90540

aggcagtgcc caggctcgga accctcccag gggcctggag gttgccaaat gggcccaatt 90600

tgctgtgcta atgtgggctt tctgctgctt ggaacctgtg cacaccctcc tccaagagct 90660

gagccagcag caacaggaca gactataatt atgacaagtt acatcctgtt gtcctggctc 90720

agttattagc cacatcgccc agcacccagc agcttgcact ccgggggtct gccagaagca 90780

gggagggtga ggtgaaccat ttgttaacac tcgcccccca tcctgccata caacttctcc 90840

cccaccccct gccaactagg gatgcctttt cctgctagtt actcatcccc taaacctgac 90900

ctcaatcacc ctccagggga gatcgctgag ctggacattc caccagcctc tacagaccgg 90960

ggcacccaca gcagggagga tggtggacag tagggagtcc tcccgaaaga tatcccacag 91020

caaccctcat ttttccgcct ttaatcccac acttctgggg ctgggctctg agacaggaat 91080

gccatccatg tttgttcctt gcacaactcc agggaagcca ctcacataga ctgcaacaac 91140

ttagaacctt agactccaat taaactgtgg cctctgcagt tgggcagtgt acaacttgaa 91200

cggctgtaca tgttctgcaa ggtacaattt ctcaactcct tccctcaagc aaataagaca 91260

caagtcctgg aggcacagat ctgtccaccc agctgacaaa ttctggggct gcatttccag 91320

aatgttctat gtgaagtggg aaaaggattc tttcttttct ctcctcctct gggccagtgt 91380

catcatggga gttctgagta cagttgcaag cctgctctgg gagagaaaca gatacatgga 91440

aggagaaatt ccaggagctc gggagacagc aaggacccag acccacattg tctgagcctg 91500

gctgatgttc ttcagtcagc agagcttctc ggacctcaag tttagagagt tagattcatc 91560

atgaattttg gttcatggaa cacccgatca ttgacccata gctggccctt tttatctcca 91620

tctggttgat taattgattg attggctgat acaatgaaca tccatgaaca ccacccaact 91680

cagggactag aatgtcacca ataacttatg tcacttgtgc cttctgatat agtttggctg 91740

tgtcccccgc caaatctcat cttgaattgt agctctcaca attcccacgt gtcatgggag 91800

ggacctggtg ggaggtcatt gaatcatggg ggtgggtctt tttcatgctg ctgttctcat 91860

gatagtgaat aaatctcatg agatctgatg gttttataaa gaggagtttc cctgcacagg 91920

ctatttctct tggctgctgc catgtgagac gtgcctttca ccttccgcca tgattgtgag 91980

gcctcctcag ccacgtggag ctgtgagtcc attaaaccta tttcttttgt aaattaccca 92040

gtctcgggca tgcctgtatc agcagtgtga aaacggacta atacatattc ctacccacct 92100

gccctccttc acacagagga acgaccatcc tgaacttatg cctattattc tcaccctgat 92160

tttatgacat ctacatgtac acttaaacaa tacatacttg aattttgctt gtttttaaac 92220

tttttttttt ttttttttga gatggaatag tgctctgtcg cccaggctgg agtgcagtgg 92280

catgatctca gctcactgca acctctgcct ccagggttca agcgattctc ttgtctcagc 92340

ctcccaagta gctgggacta caggtgcaca ccaccacgcc cagctaattt ctgtattttt 92400

agtagagatg gggtttcaac atgttggcca ggctggtctc caactcctga cctcaggtgt 92460

tctgcccgcc tcaacctccc aaagtgctgg gatttcaggc gtgagccacc acgcctgggc 92520

gatttttttt taagacagag ttttactctt gttgcccagg ctggcatgca atggtgtcat 92580

ctctgctccc tgcaatctcc actttctggg ttcaagcgat tctcctgcct cagcctccca 92640

agtagctagg attacagaca tgcgccacca cgcccggcta attttgtatt tttaatagag 92700

acagggtttc accatggtgg tcaggctggt cttgaactcc tgacctcagg tgatctgccc 92760

gcctcagcct cccaaagtgc tgggattaca ggtgtgagcc actgctcccc gtcactacat 92820

gatttttatt acaaaaaaat ttcttggcca ggcttggtgg ttcacagctg taatcccagc 92880

actttgggag gccgaggtag gaggatcgct tgaggccagg ggttcaagac cagcctgggc 92940

aacacagcta aactctgtct ctactaaaaa taaataaata aataagtaaa taaataaata 93000

aataaataaa ataattagct gggcatggtg gcatgtgtct gtcatcccag ctactcagga 93060

ggctgaggtg ggggaagatc actggagccc aggggtttga ggctgcagtg agcaaagacg 93120

gcaccattgc actttagcct gggccacaga gtgagatcac ttctcaaaaa atgtgtattt 93180

ttctttcttt agtaataact aacctcagct tactgtaact tttttacttt ataagccttc 93240

caattctttt taactttttt actcttttgt aataacattt actttaaaac acaaacatat 93300

catacagctg tacaaaaata ttttcttagg ccgggcgcgg tggctcatgc ccgtaatccc 93360

agcactttgg gaggccaagg tgggcggaac acttgaggtc gggagttcga gaccatccca 93420

gccaacatgg tgaaaacccg tctctactaa aaatacaaaa aattagccca ggcatggtgg 93480

cgggtaactg tagtcccagc tacttgggag gctgagggag gagaatcgct tgaacccagg 93540

aggtggaggt tgcagtgatc tgagatctcg ccactgcact ccagcctggg tgacagagca 93600

agtctccatc tcaaaataaa taaataaata aaaataaaat aaattttttt ctttatattc 93660

ctattctata agctttttgc tatttttaat ttttgttttt acttcttaaa cttttttgtt 93720

aaaaacgaag acacaaacac acacattagc ctgggcctac atgggatcag catcatcaat 93780

atcactgtct tccacctcca catcttatcc cactgaaaag tcttctaaaa ctcctggggc 93840

tgtcatcgcc tatgacaaca atgccttctg gatacttcct gagggaactg cctgaggccg 93900

ttttatagtt aacttctttt tttaataagt agaaggagta cactataaaa taatgataaa 93960

aagtgtagta taataaatac ataggccggg tgcagcggct cacgcctgta atccccacac 94020

tttgaaaggc caaggcgggc agatcacttg aggtcaggag ttcgagacca gcctggccaa 94080

catggagaaa cctagcaaaa aatacaaaga ttagccaggc ttggtggcat ataaatctca 94140

gctactcggg aggctgaggt aggagaatca cttgaacctg ggagtcagag gttggagtga 94200

gccgagatca agccactgca ctccagcctg ggcgagagag cgagactctg tctcaaaaaa 94260

aaataataat aaaatacata catacataaa ccagtagcat agtcatttgt tatcattatc 94320

aaatattatg tactatatat cattgtatgt gctagacttc tttttttttt tttttaagac 94380

agggtctcgt tctgttgccc aggctggagt acagtggtgc agtcacagct cactgcagcc 94440

tcaatctcct gggctcaagt gttcctcttg cctcagcctc ccaaagtgct gggattacat 94500

gtgtgagtca ctgcacttgg ccttgcttat ttttaaactt taaataaagt ttaaagttag 94560

caaaagtctc tagaagttgc cttttcactg gacatgacac aatcaggcat cacttaatga 94620

cagagataga ttttctgaga aatatgttct taggtgattt tattgttgtg tgaacatcct 94680

acagtgtatt tgcacaaacc tagatggact agcctcctac acatctaggc tagagggtct 94740

aacctgttgc ttctgggcta cagacctgta tagcgtgtga ctgcattgaa tgctgaagac 94800

gcttgtaaca caggggtaag tgggcaggca tttggccatc tcacactatt tagatgaggg 94860

attcctcctt caccaatggg gtgaatagga gattatcagg gcacatgtga cttgctttga 94920

tcaatggcat gagaggagaa atgatgggcc agtcccaaat ctaaagcctt gactctctcc 94980

ttctgccttc accccatgcc atgaccaaga gagaagctgc accctcagcc tgggacccag 95040

gaagaatata ctcagagcag agcagccctg gccaatcccc agactctcag cttgagtcag 95100

gacttcccac caagctcaac atagccccaa cctagatcaa ccaagcccca gccaagctgc 95160

agagccatta gcattaacga atgtagcttt gagccaccag agatgggggt gatctgttac 95220

tcagcattat ggtgtcaacg gctaatccat tgaatcaaaa ttcacttatg ttgctatatg 95280

acgccacatg tggcttattc ttaattgtgt acctataggc ccattaatgt atccactttt 95340

cttattgatg agtatttgag ttgtttcaat cttttgctac cataaatagc actgtcttaa 95400

ccttctttta aatttctcag ccaggtgcga tggtatacgc ctgtaatccc agcactttgg 95460

gaggctgaga agggcagatc aactgaggcc aggaattcaa gaccagctca gccaacatgg 95520

tgaaaccctg tctctactaa atacaaaaat tagccgggca tggtggcggg tgcctgtagt 95580

cccagctgct cgggaggctg aggcagagaa ttgcttgaac ctgggaggcg gaggctgcag 95640

tgagccaaga ttgtgccact gcactccagc ctgggtgaca gagtgagatt ccatctcaaa 95700

ataaataaat aaataatctt gatgtgtgtg ggcaagagtt tctctggaga atatagctag 95760

aagtagaaac gcacatattc aactttacaa gatagtatca aattgttttt caaagaggct 95820

gcaccaattg tcactcctac cagtcatcca tgagacccgg ctcatttcat cctatccaat 95880

atctgctgtc agagttgggt ggttttttat ttttgctttg ttttgttttg ggttttgttc 95940

ttgcttttcc tttctttctt tctttctttc tttttttttt tttttgggga caaagagaca 96000

gggtgtcact ctgttgctca ggctggagtg tagtgttgca atcatagctc actgcagcct 96060

caaactcctg ggctcaagca atcctcctac ctcagcctcc tgagtaactg aaactacagg 96120

tacacactcc atgcctggat aatttttatt tttttatttt tgtagagatg ggggggtctc 96180

gctatgtttc ccaggctggt ctcaaactct tggcttcaag caatcttctt gcctctgcct 96240

cccaaagtat tgggattata ggtgtgaacc actgcacctg gcagaaaaat atttttttat 96300

gaatgtagtg tagcccaagt tctccatctt aatgtagcca aatttttctt tgctgttttg 96360

gtgtgtctca caccacttca ctaatagcaa gcaacattaa tggcaactag atgctaaatg 96420

ttctataaca gggataagtt gaattatgtc aaatccacac aatgaaatac tatactgcag 96480

gccaggcatg gtggctcacg tctgtaatcc ctgcactttg ggaggctgag gtgggcggat 96540

cacctgaggt caggaattgg agaccagcct ggccaacatg atgaaactcc atctctgctt 96600

aaaatgcaaa aattagctgg gcatggtggt gtgcacctgt aatcccagct attcaggagg 96660

ctaagacagg aggatcgctt gaacccggga ggtggaggtt gcagtgaact gaggtcatgc 96720

cactgcactc cagcctgggt gacagagcaa gaccccgtct caaaaaaaaa gaaaagaaaa 96780

gaaaagaaaa atattatact gtagttaaaa atcattattt caaacacata tgatattaaa 96840

agatattcac attgtaataa gtaaaacggc taagtaacaa aatttgattc taaatgtgtt 96900

aagataaact gaaatgtctt ccacctgtca ttagacccag acttagaata aaaatcaatc 96960

taatccaagc tgagtccatc cacgcaggtt agaaaagagc cctttattgc tagagagggt 97020

tttatggtga tgtaagtcag gcttcccagg aaaatctacg gaggggacaa gaccaacgcc 97080

gtataaatga gggaaacaga ttcatctttt tttacagagt cttttttttg agaccgagtc 97140

tcactctgtc acccagagtg gagtgcagtg gtatgatctc ggctcactgc aacctctgcc 97200

tcccaggttc aagcagttct cttgtctcag cctcctgagt aactgggatt acaggtgtgc 97260

gccaccatgc ccgcctaatt tttgtatttt tagtagagac ggggtttcac catgttggcc 97320

agacttgtct tgaactcctg acctcaagtg atccacccac ctgggcctcc caaagtgctg 97380

ggattacagg tgtgagccac cgcacccagc ccaggttcat cttgatttga aattggccat 97440

gttacttctg agaaagaaat gatgaaagca gtggtcattt tggtgagatg tgcttagttc 97500

ttgccctcct gagggtggtc cctgccgccc tctctgggag gatcctgagg tggtctggag 97560

tgccaatctt tgttccccag caaagcacca gaagggccca gtggagggta ggggagacag 97620

ggagagtcaa aaatttctgt catataagat tagaaacaag tcaagtgtgg tggctcatgc 97680

ctgtaatccc agagttttgg ggggccgagg ggggaagatc gcttgggccc aggagttcaa 97740

gaccagcctg agcaacatag caagacccca tctctacaaa aaaatgtaat aattagctgg 97800

gtgtgatggc acaggcctgt agtcccagct actcaggagt ctggggtgga aggatcctcg 97860

agcccaagag ttcaaggcta caatgagcta tgatggcacc actgcactcc agcctgggtg 97920

accgaggaag accctgtctc taaaaaataa agtcaagagg gcaggcacgg tggccagcac 97980

tttgggaggc cgaggtgggt gggtcacttg aggccaggag ttcgagacca gcctggccaa 98040

cagggggaaa ccccatcttt actaaaaatg caaaaattcc ccgttgtggt agcgcatgtc 98100

tgtaatcgca gctacttggg aggctgaggc aggagaatgc agtgagccaa gattgcgcca 98160

ctgcactcca gcctgggtga cagcaagact gtctcaaaaa ataaaaataa aataataaaa 98220

taaataaaat cagtaactat ctattggaag gtggaacttc tttgagatct gcatcaaagt 98280

tacatttgta taacaaatgc cctaatttat ttatgttgtc tatttcaggt ggctgggcgc 98340

aggtggtggg gggaagcccc tacagagatt ggagggggag ctaaagattc cctaagccct 98400

cacttgtgct gtgtgtggtg taggtgaaag tgacaagggg tttctttctc cacaggggag 98460

gagaggcgta tctaagaccg ggatgtatct aacatgggga cttagaggga gggggtgcag 98520

aggagaaagg aaaacaggaa gagggtcttc tccttctctc tgctccacct cagagccgct 98580

agcagagctg ggcacgtggc gagttctccg ggaaacctcc caggctccgc tcacgctccc 98640

acatggcccc aggtgacctc ggggtgcccg cgctgtgggg agccttcaag gcgcccgagt 98700

gaacctgggg actgtttgaa gtcccggcac cttctaagga ttccgtttaa caagctgggt 98760

gccgtgagca cttggatgaa aggcgctatg taaagacatg atatcataac ctattaaagc 98820

tcgttttctt agaaacagga aggtccattt gccaagagta aacagtgagg gcaagccctg 98880

cccgggcaca ggatcccaca gttgagaaag tcattctggg tgggctcagg agccagcgtg 98940

ggtcagagcg tgggggtgca gaggggttgg aggtctcccc cacgccccaa ccccaggagg 99000

gagaagtcag cagaaacagc agctggggcc ccagagggct ggagaattcc agcctagcag 99060

gaatgaggga attactggcc cggtctggat ggagggggcc actccgtcaa actcagaaac 99120

accccgacat cctctgcagg atgtcacctc catgaaggga caaatggggg gtgctggccc 99180

tgtaattccc ctttcattaa ttctacaaat gtcaggccgg gcacggtggc ttacacctgt 99240

aatcccagca ctttggaaca ccaaggcggg tggaccactt gaggtcaggc gttcgagacc 99300

agcctgggca acatgttgag atccctgtct ctattaaaaa tacaaacaag tagccagatg 99360

tggtggtgtc tacccatgat gccagctgct tgggaggctg aggcaggaga atctcttgaa 99420

cccgggagct ggaggctgca gtgagctgag atcgacccac tgcactccag cctgggcgtc 99480

tcaaacaaac aaacaaacaa acaaacaaaa acattttttg gaagaggtgg tgtctgagct 99540

tcacctggaa ggataagtag gacttgaggg acagaacagg aagggacaca agcaccttga 99600

atatgtccag ggatttccag aggcaaaatg cacttcactc acccagcagg agggccctgg 99660

gctggaagca gggccgggct tcccatccct tcttgagcat gaacaagcca aggcttgcag 99720

tgggcctgtg acccacccct gaccagaccc cccacctccc acccagctct cccctacccc 99780

ccaccacgcc aggccctggc actgaggctg taccgcgtct ccccgtcctg cagagtccaa 99840

tcagctcccc caaactcaac ctcaggccgc gaccctggcg aaggcccgga ggagccaggc 99900

gctgcttccc agcaactgcg tttcctcagc cttgtttaca gttgttgaac tattttcgtc 99960

aaattaatta aatacatgct aaatcctctc caatttgttg tgcgataaat taaagatgcg 100020

aagattgtga aacgctcaag aaaacccact gatgctgata aaagccggca ccacagcccc 100080

gagtcaagca tcaggcagtc cagagtctgc ttctaccacc cggctgaatc agagtggggt 100140

tctgaggtct cagcggccag ggtcccaggg agacttcatg tctacccaca aaggtgactc 100200

aaggcccagg ggtggcccat gtcactggag agcacagcgc atggctgtca ggggcacagg 100260

acacaaagtc cttccccaga gtgtaccgca gggcttccta caacccctca ggtcagcagc 100320

ttgcaggaat agcaacagct tgaccaggca tggtggctca cacttgtaat cccagcactt 100380

tgggagactg aagtgggagg atcacttgag cccaagagtt tgagatcagc ccgggaaata 100440

cagggagacc ccatctctac caataatttt ttaaaaaact agccgaggcc aggcgcggtg 100500

gctcattcct ataatctcag cactttggga ggccaaggtg ggtgaatcat ttgaggccaa 100560

gagttcaaga ccagcctggc caacatggtt ggccatctct actaaaatac acatctctac 100620

taaaaataca caaaattagc tgggcttagt ggcatgcacc tgtaatccca gctactcagg 100680

aggctgaggc aggagaattg cttgaacctg ggaggcagag gttgcagtga gctgagctca 100740

cctcattgca ctccagcctg ggtgacagag cgagactcga tctcaaaaaa aaaaaaagaa 100800

aagaaaagaa aaattagcca ggcgtggtgg tgtgcaccat gagtagtctc agcgactccg 100860

aagctgaggc aggaggaccg cttgagcccg ggaggtcaag gctgcagtga gctatgatca 100920

cgccactgca ctccagcttg gacgacagag ttaagaacct gtctccaaaa agaaaagaaa 100980

agaaaagaaa ggaatagcaa cagctaatgt tgatctgaga tcgcagtgca cagtaactca 101040

cgtaatcctc atgcaactct ccaagggggc aggtgctgct attatcccat tgcacagagg 101100

agaaaatgga ggcacaggaa gtaactggcc aagttcacac agcctgggag gacaaaaggc 101160

ctggatagag gctgggctgt ccagctgtag agtctgtgct ggtaaccact atgtacacga 101220

gatcctcctc gagatcctcc tctagctctg gcctgtgcca ggcatggctt tggcagaatc 101280

agccaccaca ggccagacca cacccctagg aggcctctgg ggtctgggac acacaccatc 101340

ccacccaccc cacagctagc atctacctcg ttctagtctt gttctgtgta aagtgtaccc 101400

cattggccta catgagaggg caggtcacct atgaaaatcc cttcactcag acaggggcct 101460

cctgcacctg cccgggaaaa aaagagaggc atgagtgttc cagccagaga aactgaggca 101520

cagagggtct tgcatctgtg agtccgtggc ctaagccccc tttcctggag caaggtgatg 101580

cgtagattca aagtgacccc acagccattg cccatgtgga tccagtggaa ccccaggctg 101640

tgtgtttgtt tgtttcgaga cggagtttca ctcttgtcac ccaggctgga gtactatgga 101700

gtgatctcgg ttaactgcaa cctctgccta cagggtttaa gcaattctcc tgcctcagcc 101760

tcctggaatt ataggcacgc accaccacac ccagctaatt tttgtatttt tagtagagag 101820

ggggtttcac cacgttggtc cggctggtct cgaactcctg atctcaagtg atccacccac 101880

ctcggcctcc caaagtgcta ggattacagg catgagccac cacgcccggc ctgtgttttc 101940

tttattccat gttgtggatg aagagactga ggtccagaaa agaacaggga cgcgtgccca 102000

acatgcaaag agagtgggta ggagagctaa gttcagaccc gggaagggct ggctgagctg 102060

ctggtaccca aagcccaggt cccatgctgg ggctccccgg tcactgccat cactccactc 102120

ccccaaggtg gcaggcagtc tgggtgtctt ggggcccagg gtacctttca cagagccttg 102180

attccactct ttgaggtggg cacgggtgta ccccacccgc taagcagcac tgagaggcca 102240

gaaacaactg tggatttgaa aacatctcgt aaactccaaa tcccaaaaag gatggtgatt 102300

gttggaaatg ttgatgctgg agaagaggat ggggacaaat taggtgagtt ccaggccagt 102360

gcctcttgcc aagacacatg caccccctca acagacctca ttcctttaat atatatatat 102420

atatattttt tttttttttt tttttttaag agacagggtc tcagtctatc acccaggctg 102480

gaatgtagtg atgcaatcat agctcattgc agcctcgaac tcctgggctc aagtgatcct 102540

cctgccttac cctcccaagt agctgggact acagaagtgt gccaccacac ctggctaatt 102600

tttttgtatt tttagtagag acggggtttc accatgttga ccaggatggt ctcgatctct 102660

caaccttgtg atctgccccc gcttggcctc ccaaaatgct gggattacag gcgtgagcca 102720

tcatgcccgg aagcattttt tttttttttt ttagctcatc agctatcatt agtgtcagtg 102780

tatttcatat gtatggccca agacagttct tcttccgatg tggcccaggg aagccaaaag 102840

attggacacc cctggattgg agggacacca gtggggaaga gccaggagga tatgggacca 102900

cctgcgatca aggagaaagc tcacccccac ttcctgtgct gtggccaatg ggtggtgcca 102960

gtcctgctcc ttcaaggaag cagaaccagg gggggtattt attatagaat aagaatgagc 103020

attggccagg ctcagtggct catacctgta atcccagtat tttgggagcc tgaggcgggc 103080

agatcacttg aggtcaggag ttcaagacca gcctggccaa catggtgaaa ccctgtctcc 103140

actaaaaata caaaaattag ccaggcatgg tggtgcatgc ctgtaatccc agctactcag 103200

gaggctgagg caggaaaatc acttgaactc gggaggcgga ggctgcagtg tgctgagatg 103260

gcaccactgc actccagcct aggcgacaga gcaagactcc atctcaaaaa aaaaaaaaaa 103320

agagggggag atgtactggg gaccaggaaa aagtcagcgg ccagccctcc cgcagcaggg 103380

aggcaggcag aagagctgga gcttgtgggg aatcccggag cccggcggat gtggctgctg 103440

caatatgaag gcagggccag tgtagacgct tatggaggtc tcttcacacc atctctgtaa 103500

gatcacagcc aagcgtccag ggaaggcacg ggtcactgtg acttcttaac agggccagca 103560

gcctgggaga agagccagcc accaatcagg gaataggaca tgcagaagag tgtggataaa 103620

ctggaacctg cgggcacctc tctgtccgtc tctctgccct gcctctagcc atgatgatgc 103680

tcagagcaca aagttcgctt tagtccaact ctaactcaga cccacagcgg gaaggggatt 103740

ctaggaaaca aggctccagc ttggccaact tgactcagta caaaaccacc acagcaggaa 103800

gagatggagc tgcttgctga gaccaggcgc cggggatggg ggcaggttca gggatgcctg 103860

gtatggttgt gtatgttgtt cactgctcaa gagcacctgg ctagggagcc aggaggggct 103920

gaagctgacc ctggcatgaa gccatatcta cctggaggaa ggggcgtctt tttctaactt 103980
gcataagata ccattcaaac ctgggcagga tgagggaaag gcgtggtgtg ctggttgagg 104040
tggaaggtgt gggggtgtcc ataaggttaa acttctccca gcatccatca tccccctctg 104100
tgaccaacac tgtaagggcc ccatacacag ggcccttgct gtcagttgga ctaaccagta 104160
acaggcttgt ggcatatggc gatggtccta aaattcccag agggcaggca gtgcaaaagc 104220
aggcagagcc agctaacttg gagggaggat tatgccttag gcagagctgt ttcagataga 104280
acgagatgtc ttttgaggta gtgagctccc cgtcactgga ggtattcaag cagagccaga 104340
tggatgcttg cagggtttgg tgcaggggat gtcatgaagc tccaggtggc agttcagcca 104400
cttgctcctc ctggacagtg agtcagacat gaacaagact tcagaggatt ggtggcaggt 104460
ctcgggcagg gttgggggga gttcctctcc agccctcccc caccctttgt ctctgctcag 104520
ttctttttgg aaccagttta ggtatttaga agattaattt catatttgtg caaagcaggg 104580
gattaggagg ggaggagact gaggctctct gcaggtcgtt aatctaaaaa aagactgcaa 104640
aatcgctctg gccacgagtc acagctgagg gtgccactca ccgccagcaa gccagggctt 104700
ttcatcagta gtttgctttc attttctgcc gtctcaggac caacactctt gagctctggg 104760
gaaggtcaaa ggcagaacat ggtggggtgg cctccatgcc acagagcaag gaggctggag 104820
tgccaccccc atcacgccaa ggtccagact gcagacctca aacagaatta ctgccatgag 104880
gaccaccctg attcctcagt ccactgccac ctcctgcctc actcttcggt gctcctggag 104940
aaaccagagg ctcagagagg gcatggattc accccagcac acacagcaag ttgctgattc 105000
ctgactcaga gctcctcctc gatgctgcca cttagagttg aggtggtggg agctgctgga 105060
gccgttctgg gactatctgg aagcacagcc catacatcca ggagtctagg gggatttttt 105120
ttttttttgt aacagcatta ttgggataga attcccaagc catacaattc atccatttaa 105180
agcagggatg tccaatcttt tggcttcccc gggccacact ggaagaagaa ttgtcttggg 105240
ccacacctaa aatacactaa cactaatgat agctgataag ctaaacacaa acaaacaaaa 105300
aaactgtcgc aaaaaaagtc tcataatgtc ttcagaaagt ttacgagttt gcgttggatt 105360
gcattcaggg ccgttctggg ccacgtgtgg cccatgagcc tcgagttgga caagcttgat 105420
ttgaagtgta caattcaata aactatcgaa cttttgtata ttcagagttg taaagccacc 105480
accaaaatca attttagaac agtttcatct ccccagaaag caaccctgta ctcattggca 105540
gtcactcctc acttccttcc ccatcccagc tcctcgtaaa taatctactt tctggccagg 105600
cagggtggct cacgcctgtg atcccagcac tttgggaggc cgaggcaggt ggatcccttg 105660
aggtcgggag tttgagacca gactggctaa catggtaaaa ccctgtcttt actaaaaata 105720
caaaaattag ctgggtgtgg tggcgcacat ctgtaatccc agctactcag gaggctgaga 105780
caggagaatc acttgaacct gggaggtgga ggctgcagtg agctgagatc acgccactgc 105840

actccagcct gggcaacaga gcaagactcc gtctcaaaaa aaaaaaaaag acaagctcag 105900

gaaagatttt ggctagtaaa ctagaaggga aaaaggaaga ggctggaaat gtcggggttt 105960

gcagggtttg aaaagccaac tgtaggctgg gtgcagtggc tcctccctct aatcccagtt 106020

ctttgggagg ccaaggtggg aggattgttt gagcccagga gttggcgacc aaccctggca 106080

gcatagccag accccatctc tacaataaat tttaaaaatt agctgggcat ggtagcatgc 106140

acctgtagtc caagctactc gggagactga gtgggaggat cacttgaagc caggggttcg 106200

agaccagcct gggcaacata gcaagtctct gttggagtcc aaggctgcac tgagccatga 106260

tcgcaccact gcactccagc ttgggtgaca gagcaagaac atgtctcaaa caaaacaaaa 106320

caaaatagaa gagagagaga aaagaaaagc caactctttc tggcttttaa cttcatatat 106380

gttaaatgtc aataaattta ccttcgtgta tgtaaagtgt caattaaaaa gtgcccttga 106440

cgaaacaaag ttaattcagg agacagaaga taacttgaac aaaaactagt taacatcccc 106500

aggggattca ctaagtaaac aaaaaaagag ctctggggaa ttcaaaatat ctttgctaga 106560

atttttaaaa ggtaacaaaa gagttggtga ttaaagctga ggaatctccc agaaaggaga 106620

aaaagagaga tgggagataa aagatcagac gtagagggga aaatccaacc gctatccctg 106680

ggcgttccag aaggacagaa cagagaatat agaaggaaat aagtgatcaa agacacacaa 106740

gaagagaagt tcccagaaca gaaggctagg agccttcaga ttgacggcca tgcaatatga 106800

taataaaact aaagatggcg aggtgcggtg gctcatgctt gtaaccttag cactttggga 106860

gaccaaggtg ggaagattgc ttgaggccag gaaatcaaga ccagcctggg caacatagtg 106920

agaccccatt tctatttaaa ataaaataaa aatttatttt gtttttattt ttatttattt 106980

atttattttg tggcagagtc tcgctctgtt gcccaggctg gagtgcaatg gtgcgatctt 107040

ggctcactgc agcctccacc tcccgagttc aagtgattct cctgcctcag cctcccgagt 107100

agctgggatt acaggtgccc accaccatgc ctggctaatc tttgtatttt tagtagagac 107160

aggatttcac tatgttggcc aggatggtct caaactcctg acctcaggtg atccgcctgc 107220

cttggcctcc caaagtgctg ggattacagg catgagccat cacacctggc caataaaata 107280

aaattttaaa taaaactaaa gaaaaggcat gaagaaacat cactgagaaa cttcagtagg 107340

tgtaccagtg ataaacagat ggtcctaaat atttccagtg ggcagaggta aaagcaagtc 107400

actacaaaag aactgctgat cagctggaca cgatggctca cgcctgcaat cccagcactt 107460

tgggaggcca aagtgggtgg atcatttcag gtcaggagtt caagagcagc ctggccaaca 107520

tggtgaaacc ccatctctac taaaaataca aaagttagcc gggtgtggtg acgcatccct 107580

gtaatcccag ctactcggga ggctgaggca cgagaatcgc ttgaacctgg gaggaggagg 107640

ttgcagtgag cagagatcat gccactgcac tccagcctgg gcgacagaga gagactctgt 107700

atcaaaaaag aaaagaaaag aaagcaacag agctgtgtct tctcggttct caaggataag 107760

ttttgttttt tgttttttgt tttttttgag atggagtctc gctctgtcgc ccaggctgga 107820

gtgcagtggt gcgatctcgg ctcactgcaa gctccacctc ccgggttcac accattctcc 107880

cgcctgtctc ccgagtagct gggactacag gcacccgcca ccacgcccgg ctaatttttt 107940

tttttttttt ttttttgtat ttttagtaga gatggggttt caccgtgtta gccaggatgg 108000

tctcgatctc ctgacctcgt gatccactcg cctcagtctc ccaaagtgca gggattacag 108060

gtgtgaacca cagagcccgg ccaagggaaa atatttttaa cctaaaattc tatacccaat 108120

ccaactattg tgaatgcgga aaatctgaga cgggtctgcc ttaatttaga aagtttattt 108180

tgccaagatt gaggacacgc acccgtgaca cagcctcaga aggtcctgat gacatgtgcc 108240

caaggaggtc ggggcacagt ttggttttac acattctagg gagacatgag acatcgatca 108300

acatatgcaa gatgaacatt ggtttggtcc ggaaaaatgg gacaactcaa agcaaaggtg 108360

ggaagactca agcaaggagg gggcttccgg gtcataggta gataagagac aaatggttgc 108420

attcttttga gtttctgatt ggcctctcca aaggaggcaa tcatgatgac acgcacctgt 108480

aatcccagct tctcggaagg ctgaggcggg agaattgctc caacccggga ggcggaggtt 108540

gcagagagcc aggattgtac cactgcactc cagcctgggc aacagcacaa gactccatct 108600

caaaaaaaaa aaaaaaaaga tatgccttta tctcagtgag cagaggggtg actttgagta 108660

gaatgggagg cagggctggg aggcagaggc tgcaatgaac cgagatcagg ccactgcact 108720

ccagcctgag cgtcagagtg agtgagactc tgtctcgaaa aaaataaata aataaaaata 108780

aaaataataa aatgaattta aaaatcgaaa aatgcttttg tagagatggg gtcttgttat 108840

gttgcccagg ctggtcgcaa actcctgggc tcaaacgatc ctcccacctt ggcctcccaa 108900

agtgctggga ttacaggcga gtgccactgt gcctggcctg tgaatgcttt ttgctttagg 108960

gtccagtttt tctttctctg gcagtttcta aatgttgttc ttttgcctgc attttctgca 109020

tcttgagttc ttctgatgtt tcagaaatga catcacatct atggggacct tgttttccct 109080

gggtttctgg tcactggtgt tcactactca cccatcctct tttgctaatc tggatcctgg 109140

catctggaca ccccctggac tgcagctgct attgcctgca tcccatttct ccctcttttt 109200

ttctttcttg gttttttttt tttttttttt gagatggagt ctcactgtgt cgcccaggct 109260

ggagtgctat ggctcgatct cggctcactg cagcctctgc ctcctggttt cacgcatttc 109320

ttctgcctca gcctcccgag tagctgggac tacaggtgtg agccaccacg cccggctaat 109380

tttttatttt ttagtagaga cagggtttca ccatattggc caggcatgtc tcaaactcct 109440

gacctcatga tctgcccgcc tcagcctccc aaagtactgg gattacaggc gtaagccacg 109500

gcgcctggcc ctccctcttt cttggtttat ttatttactt ttccttctgg gagggatgga 109560

tagacaatac attcgtgaaa attttggaag tctaaaaaat aatatggtcc ccaggcttgt 109620

tcaatagtgt gaaaggaaaa taaatcctgg ggcccccaca tcgctaagct aaagagaaaa 109680

gtcaagctgg gaactgctta gggccaacct gcctcccatt ctgttttgtt ttgttttttt 109740

gagatggagt cttgttctgc cgtcacccag gctggagtgc actgaggtga tctcagctca 109800

ctgtaacctc cgcctcccgg gttcaagcga ttctcctgcc tcagcctccc tacaggtgcc 109860

cgccaccatc cttggctgat tttttgtatt tttagtagag acacagtttc atcatattag 109920

ccaggctggt ctcgaacttc tgacctcagg tgatctacct gccttggcct cccaaagtgc 109980

tgggattata ggtgttggcc accatgccct gcccccccaa atttttttta attagccagg 110040

tgtggtggag tgcctgtagt cccagctact tagaggctga ggtgggagga tcacttgagc 110100

ctgggtggtt gaggttgcag tgagctttga tggtgccact gcaatccagc atgggcaacg 110160

aagtgagacc ctatctcaaa acaaacaaac aaacaaaaat tctggggtca cgttatggtt 110220

gatgagcaag ccaaggtgtg acagtcaagg gaagcatctg agtggggtgg agaggaagtc 110280

atttgcctca ctgtgagata ggtgaccctc gaggctcaga ggctgtgagg gtggaggagt 110340

gagaggaacc ccacaaatgg ggctaatgtc ttcactgaat gaggagttat ggggatcaat 110400

gaatgagcct ccaaggatcc tcagagacgt ttctgctgcc tgaccaggcc agcatcccac 110460

agccaaggag gctgggctct gtatttggtt cattgatcca agttccctct agaccccggg 110520

atcatcttcc taccccaccc cacactgatc cctcccacct ctcatctcag cccactccag 110580

ttctgacaaa cacattcccc aagacctcat caaatcccac atcagtacat gctcgggaag 110640

gagtggagtt ggttccagag aggttggcag tgaactggag tgagagatgg acagagaaat 110700

taagggaatt ttcctggagg gaagggcttg agggtggagg ttgggaggct cagctgctag 110760

aagatccgag gaagaggacg cagaccaact gtggtctggg accccagatt gctgagccat 110820

cagcaagggg agggtgctgc agggagttcc atttgggttc aaataaggaa gccctcttga 110880

aggagtgagt tctccatcca agggggtgtg taagcagagg ctgcacatcc accccgctgg 110940

gatggtgctg gtaggattca ggcatgagat gaggatctcc agccctgaga ctctacgaaa 111000

aggagatgaa gagcaatggg agggagggga gagcaagaca gtgcccgagg ctaaagaaca 111060

gacgccacta tctcagctgt ggaagcagca gctggagatg gaacaagccc ttatgtgtgg 111120

ggaatttgcc tcagcagatg aggtacagcg aggatgctca tgatatctcc tgaaatgaat 111180

acaatctgga aaaaaaacaa attcaacaga acccattttt tttccactaa taaaccaagc 111240

caacttcagt gccatcctaa tttatctgtt gagaaagaag gtctttattc tcaggaggtg 111300

ggaataccac ttctttatta tcatcatcat catcatcatc atcatttttt tttttctgtt 111360

tgagatgaag tcttgctctg tcgcccaggc tggagcacag tggcacgatc tcagctcact 111420

gcaacctcca tctcccaggt tcaagtgatt ctcctgcctc agcctcccaa gtagctgaaa 111480

ttacaggtgc atgccaccac gcccggctat tttttttttt tatttttaat agagacaggg 111540

tttcaccatg ttgaccaggc tggtcttgaa ctcctggact gatgtgatct gcccgcctcg 111600

gcctcccaaa atgctgggat tacaggcatg agccaccgca tgcccagcca gactcccaag 111660

ttttctttgt caattcttgc tttaaatatc ttgaggctat gttattaatg tataaaagat 111720

aaaaatgttc tctctttctg atgaatttac tttttttattt ttcattgacc ctctttatct 111780

ctagtagtgc attagagctt aacgtctatt ttttctaaca ttaatatagc tacctcacct 111840

tttttcttta tttgcctaga gcatattttt catccttaga tttctaatcc ttctgtgttc 111900

ttatttttgt gtgtcttatg tttttgtgtg tcttataaat accatatact tgaattttat 111960

tttaattttg tttgacaatc ttcgtctttt aactggagag tttagtttag tttgtttatt 112020

tatttattta tttatttatt tagagatgga gtcttgctct atcgcccagg ctggaatgca 112080

gtggctcaat cttggctcac tgcaagctcc gcctcccggg tttacacggt tctcctgcct 112140

cagcctcctg agtagctggg actacaggca cccaccacca cacccggtga aatttttgta 112200

ttttttagta gagacagggt ttcacggtgt tagccaggat ggcctcgatc tcctgacctc 112260

gtgatccgcc agcctcagcc tcccaaagtg ctgggattac aggcgtgagc caccacgcct 112320

ggcctattta tttatttatt tattttttag atacagaatc tcactctgtc gcccaggctg 112380

gtgtccagtg gcacaatcta agctctctgc aacctccacc tcccgggttc aagcgattct 112440

cgtgcctcag cctcccaagt agctaggact acaggtatgt gccaccatgc ccccgctaat 112500

tttttgtatt ttagtagaga cagagtttca ccatgttgcc caggctggtc ttgagctcct 112560

gagctcaggc aatctgtctg cctcattctc ccaaagtgct gggataacag gcgtgagcca 112620

ccacgcccag ctagtccatt tacattgaag tgattggtga gcaattaggg tttattgcta 112680

tcatcttatt atattttcct atttgactca tgttttctcc ttcttcgtcc ctctcttttc 112740

ttgtcttcct ttggtttatt agttagatat tattgtaata atgttgcata acagaaaact 112800

tcaaaatctc cgtggtttac aacaccaaaa acgatttctc actcgaagat ctgaggctgg 112860

ctaccgctct gctaagtctt ctaattgttc tatattgtgg gtctcctcta agcatatttt 112920

cacagggagc aggctgaagc aggagaggct acctagggca tgtttttctc acagcagtga 112980

caggcacaga gggacaaagc taaatcatgc aaggacattt aaaacctcag tttgcagcca 113040

gctgcagtgg ctcacacctg taatcccagc actttgggag gctgagatgg gcggatcact 113100

tgaggtcagg agtttgaggc cagcctggcc aatatggtga aaccccgcct ctactaaaaa 113160

tacaaaaatt agccaggcgt ggtggtgtgt gcctgtcatc ccaactactc aggaggctga 113220

ggcatgagaa tcacttgagt ctgggaggca gaggttgcag tgagtcaaga tcacgccact 113280

gcactccagc ctgggcgaca agagtgaaag tccgtctcaa gaaaaaacaa acaaaccaga 113340

atgtagattt tccccatgaa agacagtttt gtagggccat ttcaaaatac atcaaagaaa 113400

tatatttgtg ggtaaaatac tttgatttct ttcagggcct gctatctgtc atgctggtat 113460

cttattgcta aagagagtct gttttgtcag tcttaaggtc tctgttttaa tgtgaatgct 113520

ggtcagctgt acctgaattc caaaggaggt tgggtataat gacgcatgtc tgaccaaagc 113580

cattcccatc acagcctgaa ctagtgtttc agatttactt tagaatgccc ttggccaaga 113640

gaagggaact attcacttgg ctggtggggg tgcttagaat tttatttttg gtttacagct 113700
cctaggagaa atatgtgtgt aggtatgtac acccaccaca cacacacacc ccgcactcat 113760
atagattata atataaaaac aacacattct ggctgggcgc ggtggctcac gcctgtaatc 113820
ccagcacttt gggaggctga ggcgggcgga tcaccggagg tcaggaattc gagaccagcc 113880
tgaccaacat ggtgaaaccc tgtctctact aaaaatacaa aaattagccg ggcatggtgg 113940
agggtgcctg taattccagc tactcaggag gctgaggcag gagaatcact tgaacctggg 114000
aggcggaggt tgcagtgagc cgagatcacg ccattgtact ccagcctggg caacaagggt 114060
gaagctgtct caaaaaaaag aaaaaaaaga gagacaagaa gaaggaaatg catttaatac 114120
acctaattta ccaaacatcg tagcttagcc aagcctacct caaacacgct catgtaacca 114180
agtatcgcca tttttctaag agatagttta tttttctctc tcccttcttt tctctttcct 114240
cctttacgct gcttcctatt tagctattta gaaatgcaat tatagccttt tacctcccct 114300
gcaccagata ctctctattt taggcatccc cagcccccag gccatgttag gaacctgtta 114360
ggaaccgagc tgtgagtggc aggtgagtga gcattactgc ctaagctcca cctcctgtca 114420
gatcagcgat gccattagat tctcatagga gcgcgagccc tattgtgaac tgtgcatccg 114480
agggatctac gttgtatgtt ccttatgaga atctaactaa tgcctgatgg tctgtagtgg 114540
agcagtttca tcccaaaacc atcccctgca tccgtgggct atggaaaaat tgtcttccat 114600
gaaaccagtc cctagtgcca aaaaggttgt agactgctgc cctacaggca agttcatcta 114660
accatgtgct tagaagctcc agagcggaac tctctcccac cacgaagttg ccttggcaga 114720
caacagtcca tctgcagccc aaagtgtgcc cgctacgaaa ctctccccta cctggagagt 114780
ttttggccac ttctccaatg tagttctatc cacaaaggtg ccagcagtca ccagctcaag 114840
ggcctggtca tgctagcacc aaagctatca tgaagagccc cccaacctgc ttgcttcctt 114900
ccctgcatgt cactcatgcc aagccccctt ctaaaagcac ctgccttctg ctctaaaagt 114960
gaagggatac ccttaaggca ggaagcctgt acttctcccc ctaagctagc tttggaataa 115020
aaagtcactt tcttggccag gcgtggtggc tcacgcctgt aatcccagca ctttgggagg 115080
ccgaggcagg tggttcatct gagttcagga gttcaagacc agcctggaca acatggttaa 115140
actctgtctc tactaaaaat acaaaaatta gccaggcgtg gtggtgcgca cctgtaatcc 115200
cagctacttg gaaggctgag gcaggagaat ggcttgaacc caggaggtgg aggttgcagt 115260
gagccaagat tgcaccactg cactccagcc tgggtggcag agcgagactc tgtctccgaa 115320
aacaaacaaa caaaaagtca ctttcttttt tttctttttc ttttcttttt ttttttgaga 115380
cagagtttca ctcttgttgc ccaggcaatg gcgccatctc ggctcacagc aatctccgcc 115440
tcctggattc aagcaattct cttgcctcag cctcgtgaat agctgggatt acaggcatgt 115500
gccaccacgc ccacccagtt ttgcattttt agtagagaca gtgtttctcc atgttggtca 115560
ggctggtcct gaactcctga cctcaggtga tctgcccacc tcagcttcct gaagtgctgg 115620

gattataggc gtgagccagc aagcctggca aaaagtcact ttctttatat cagactttgc 115680
tcttgttcat cggactctac aagcagcaag tgactgaacc cgcaattcgg ttacactcag 115740
aacactgaca ttagcctacg gttgggcaaa atcatctggc aacacagtac actgtacagt 115800
atcattgttg actctcatgg ttgcctggct gactgggagt tgcagctcac tgcaagagag 115860
tatcatactg catatcgctt gccccggaaa agatcaaaat tcaaaattcg aagtatggtt 115920
tctactgaat gcatatcatg aagtcaaaaa atcctaagtt gcaccattgt aagtctgaga 115980
ctttgtatgt aatctttcat cctctgccag aaatatctct tcagtaattg tccagtgctt 116040
taagtatatt tccagggagc tggggagtag cagggcaaag ggccttgggc agaatggtct 116100
atgcctttgt gcctccgaat ttcatctgtc aaatagaaaa aagaatccag gtcagatgag 116160
caaatggaat cgtgtcaaag tgctcagcaa acaataatgt acaagaaagt gcttagcaaa 116220
caataatgta cacgaaagtg cttagcaaac aatagtgtac atgaaagtgc ttagcaaaca 116280
ataatgtaca cgaaagtgct agcaaacaat aatgtacacg aaagtgcgta gcaaacacta 116340
aggtacacaa aagtgcttag caaacaataa tgtacacgaa agtgcttagc aaacaataat 116400
gtacacaaaa gtgcttagca aacgataagg tacacgaaag tgcttagcaa acaataatgt 116460
acatgaaagt gcttagcaaa caatagaagt gttaatacca cctgcattct tgcaatagca 116520
ctgaaactac aacttcagtg acactctggt tctaagcatg attttcagtt attcccaagg 116580
aactgaagtt gatgtagcag tgaatgtccc aggagcaatt agacccactt cacttctctt 116640
gattgtcatt taatattctt ttattaagca tcagttgtga gtgcaacctg gagaaccact 116700
tgatgtcaga gacatcagaa ctgagctcaa gagacactca gatacctgat ccagacctca 116760
aaggaaagat ctgtccaaag ttgagaaact aagagaatga tctgtccacc tagacaacgc 116820
caggccctag gagaaagtct gtccactcag accctacagg agaagctcag ggaacagttt 116880
gaccacacag gagctgctga agaacagcca gtcataggtg ttgggggaga agggtgccgg 116940
gaaagaggca ggaggaagat gtgcttccag gggggcagaa gttaatggcg cccaatgcag 117000
ctgagattgc aagtatgaca cagactgaga attacccatt gggtttgggc catgatgtgg 117060
tttggttgtg tccccaccca aatctcattt tgaattgtag ttcccataat ccccatgtgt 117120
cacaggaggg acccagtggg aggtaatcga atcatgtggg cagttacctt catgttctca 117180
tgatagtgag tgagttctca tgaaggctga tggttttata aaggcttttc ccccttttgc 117240
tcggcacttc tccttgctgc tgccatgtga agaaggacat gtttgcttac ccttccacca 117300
tgattgtaag tttcctgggg cctccccagc catgctgaac tgtgagttaa ttaaacctct 117360
ttcctttaca agtcacccag tctcgggtat aactttatta gcagcatgag aacagactaa 117420
tacaccccat gttccatctc atttcatctt caagcacccc tctggagatg ggttcagaga 117480
ggcaagtggc ttgccaaagg tcacacaggt gagaggagcc agaggcagaa tttagaggct 117540

ggccaggcac agtggctcat acctgtaatc ccaacacttt gggagactga ggtgggaaga 117600

tcacttgagg ccgcaagctc aagaccgagc ctgggccaca tagcaagacc ccatctctac 117660

aaacgaaaaa gaaaaaatga tccgggcatg gtggtgcatg cctgttgtcc cagttactca 117720

agaggctgag gtgggaggat cacttgagcc caggaagttg aggctgctgt gagccatgat 117780

cacgagactg cactccagcc tgggcgacag agtgacactc ttgtctaaat aaattaatta 117840

attaaataaa gcctcagttt gtgttacctc cactcacaat cattggctaa agcaagtcac 117900

accgtcagac tcaatatcaa aggtgggggg agtttgaaaa ccaatagcct caaattcttt 117960

ggttttgaaa cttgaatgtg atgagaatct ccaggctact cattaatttg catctgagat 118020

gggactcaga attccactgc atacccacga ggccccagta gcatgcatgg cctccttcct 118080

actgaccctt gtagccgggg gtccctgagt tctgttgggc tcccctcacc accctgcctg 118140

caaacacatc tgagtatatc accgaggttg ctccccaagg gaggagggtg gcccccaggg 118200

cactcgcggc ccctcccctc tgcctggccc tcccctgagc tctctgcaga ttcctcatta 118260

gcaagtgcaa agccagcccc ttcacgcctt cccaggcctt gcaaggactg agagaggctg 118320

gaatgcaagc cccgaaccac cctccagcaa tccttcatta tcccttccct cgcaggtccc 118380

accaccaggt aattccattg ttaattaaaa tcctgcagcc tttaatatct cggctcataa 118440

ttgctgtttt ccccagccct ggattggctg tgcaggcttc gggggggacc cgggtttttt 118500

tttcctggga tctgctttgt acaaccaccg gctgtctatt tcctcaacct cagccaaaaa 118560

ccttcttctc cgtgggcttc aatagtggag caatggggac caccctgggg gccatattag 118620

caccccctgg acacctccta ggaaaggagg ccaaggaagg agtcagtctc ttcccccaac 118680

cccaggcagg cctccaagta caacccccag caacacagcc tgggagacag agacagccag 118740

ctcctgtaca cgcaggccac gaaggctcca gagtcccgca gaggccccct gaggccatgt 118800

tcatgggcaa caaggatctt cctcatccac gtatcatttg ctgctccccc gagccccgag 118860

cccccattgt tcagctaaag aagaaaaaga tcagagaggg aggtgaattt cccaaggcca 118920

cacagcagag ccaggaggcc tagagtgcag accctctgct cttctgacca taccctctgc 118980

ccgttgacct tcaggccctt tctcaccttc ctgctctcag cgttgatact tttcttcctc 119040

ctctgctagc ctcactcaca cttagcccgg cccctgtctc ttcctctgcc attcccattc 119100

tagctgagga tgtgaatgct gtagatgccc acaggggagg cagggggcca acaatgagaa 119160

aggacagagg actgtgacta ttggtcacag atttcaaagg ctggaggacc acagtggagc 119220

caggaggggg acgcgctgag gttagccctg tctttttttt tttttttttt tttttttttt 119280

gagatggagt tttgctcttg ttgcccaggc tggagcgcag tggcacgatc tcggctcacc 119340

acaacctccg cctcctgggt tcatgcactt ctcttgcctc agcctcctga gtagctggga 119400

ttacaggcat gcaccaccac gcccagctaa ttttgcattt ttagtagaga cggggtttct 119460

ccatgttggt caggctggtc tcgaactccc aacctcaggt gatccacccg ccttggcttc 119520

ccaaagtgct gggattacag gcgtgcgcca ccgcgcccag catgctccat cttaatcact 119580

ctgtgctgtt gacccaaagg ccccgtaagg tctgaaggat caacccccat cccctgccca 119640

ctgggaaggt gaccaggtat tgctctctgt ggaagacagc acctcactca aggccaggcc 119700

cctcttgggc aactgtgcag atgggggcag agcttggctg gcctggctcc agctttggag 119760

ggtctgaggg tggaccgagc tcttgtcaca actgccctgc tcctccctct gtggcccctg 119820

ctgccatcct cctcccggca ggtgttgacc ccaaggccat atgaccataa aacctccatc 119880

cagagtctgc atcccggggg acctggcctt ctagagccat tttctttttt gggatttcta 119940

gggcctgctt cctctcttct cccttcccac agggacccca gaccctgtgc accatgacct 120000

attacccacg cctgtcccat gctcctggct ggtctgtcct ttcagaactg gagaacggag 120060

acgtatcctc tccactctca agaacctcag ctcgaccggg tgctgcagat cctgcctgta 120120

accccagcac tttgggaggc tgaagcagga ggattgcttg agcctgggag ttggaggcca 120180

gcctgggcaa tgtagcgaga ccctgtttct acaaaaagtt taaaaagtag ctgggcgcat 120240

ggtgcatgtc tgtagtccca gctactcagg aggatgagca gggaggatca ttgagcccag 120300

gagtacgagg ctgtagtaag ccatgattgc accacagcac tctagtctgg gcaacagagt 120360

aagaccctgt ccccccacca aaaaaaaaaa acctcagctc cccctaacag aaagtctcca 120420

ggggccgagg accccttcag agcccttgat gtttctgagg tcagctgtgt catcccaaat 120480

ttccaggaac tggaaaatct gtcacctgtc ccagttgcat agctgggagg tgttggagat 120540

ggagttctga acacatctgg ttctcatcta atcctcagct cacagctgcc tcagtcagcc 120600

tccaacaaga ttcctctgtc actggtggct ctgcgagaaa accaatccct gaactgacca 120660

gctcttctga ctgctcagaa gtaggatcat gtttttgttt ttgttttgag atggagtttc 120720

actcttgtca tccagtctga agtgcattgg cacgctcttg gcccactgca acgtccacct 120780

tccacattca agcaattctc ctgcctcagc ctcctgagta gctgggatta caggcacgtg 120840

ccagcatgtc cagctaattt ttgtattttt agtagagacg gggtttcacc atgttggcca 120900

ggctggtctc gaactcctga cctcaggtga tccgcctgcc ttggtctccc atagtgctgg 120960

gattacaggc atgagccacc gtgccaggcc agaagtagga tcgtgtttac agagcactgc 121020

ttgcattcac tcattcattc attcattcaa taaacatgat gtcttgagca tcccttgtgc 121080

caggcaatgt gtttgttgct ggggacacct tagggaacaa gatggacaaa gccctgatcc 121140

tcttggagct tctattctag ggagaaagat acacaatcaa caagataagt aaaataacat 121200

acaataggtt agatagcgct aagtgctaag gaggaaaata acagggaata tggacaggaa 121260

atgcctgcgg tgggttagag ggtgaaagtt gagtcagggt ggccagggaa ggcctcaatg 121320

ggaaggtggc ttttgacaaa agggtgcagg aagaccaggt gcagtagctc acggctgtaa 121380

tcccaaaact ctgggaggct gaggcagaag tttgagacca gcctgggcaa tgtagcagga 121440

ccctgtctct acaaaaaaac ttttttaatt agctgggcct agtggcacgt gcctgtagta 121500

ccaactactc aagaggctga ggtgggcgga tcacttgagc ctgggaggtc gaggctgcag 121560

tgagctatga tggtgccact gcactccagc ctgggtgaca gagtgagacc ctatcacaaa 121620

tgattgagag agagagagag agtgaaaggg tgaaagagtg aaagggtgca gggcccagct 121680

gggaacaata ggtcacacgg agccctttct ctggactctt ggtggggaag ttcctccaag 121740

gaagaagttc ctccaaacag aggctaagca gagaggaacg gctgctgccc cagcttctca 121800

gtctggttgt cctgacaggg gcagcaggaa gggtgcacgg acacccacct gagtgaggag 121860

cttgctaggc aggggccccc cttgtcccag tgcttccacc agtgcaccca acctgagccc 121920

cacccaggtc ctgctcacct gctgaatggc cttccaacac tgaagacctc agtcagctgc 121980

ttcctagcaa ggaaacacct gcagcctcct tgcaacaagt actatatatt ggaagggcac 122040

cctgagattc caggtgaagg aaatcggtca cccagaggtg ttgtgtgttc cctgcaggag 122100

ataatgcagc ttcaaaccag gtcccccagc ctccgtgagg aaagtgccct gcactgtcat 122160

cctgaccaca gttcctggaa acccacgagg aaattggcac attctgcaga tctgatctgc 122220

tggcttcatc tctgtgaggc acctcatcag tgacaacacc cagatccagg ctagaaccct 122280

gtaacattcc actagtgaat ctgtgctggc tgccccttcg tgtttgctaa caagctttct 122340

gaggtatcag ggagggacac caagctctcc ccgctgtgtg acctgagtgt cgtgtctctc 122400

tttagctccc atgtgtggag ggaagaaagg gaaaggtgcc agctgatatc aaaatggaca 122460

gagtaaaacc atgacaaata caagacaaac tacccaaagt cctttggaac cattctggcc 122520

ttgggaggtg tctgaatcca caagaaagac tcagtgagag ttaaaaagaa gcattctgct 122580

caagactgga gagcagctgg gatgaggcta agtagtaatg gacaacagct acaaattcat 122640

gagaatggga actgccatgg attggacagc agggccctcg tgtgtgtgtg tgtgtgtgtg 122700

tgtgtgtgtg tatgtgtgtg tgtgtttaag agatggagtc tcattctgtc acccaggctg 122760

gagtgcaatg atgtgatcac agctcactgc agcctcaatc tcctgggttt aagtgatcct 122820

tccacctcag cctcctgagt gcctgggact ataggcatgc accaccatgc ccggcttatt 122880

tttaaaactt ttttgtagag atggggtctc aaattcctag cctcaagcaa tcctcctgcc 122940

tcagcttccc aaactgctga gatcacaggt gtaagccacc atgcccagac cacacacact 123000

ttttattcat gagaaaagtg aggctcagat aaattaagta tcttcttcaa gtggtgtggt 123060

agggatttga acctgcatct cttcaaattt caagaggcat cttttctcct ttgagacagc 123120

caaacccctc aggatgtgcg tgttttccat aggtggaggg agcaccttga gtgccagcag 123180

gaggcagtgt ggagcagagg aaaggccaag gctttggact cagacccacc taggattgca 123240

tctcaatgct gtgctatctt gaacaagtta ctaagattct ctgaatcctg ttgttgttgt 123300

tgttgttgtt ttccttattg gtgctgcaat aagttatcac aaacttagca gctgaaaaca 123360

acacaaatgt attatcttat cattctggca gtcagaagta tgagatgggt ttcacagagc 123420

tgaactcaag gtgtcggcag gactatgtgg atttctggaa gcttgaggag ataatctgtt 123480
ttcttaattt tcctttttct tttatttttt tattttgtat ttctttattt tttttttgag 123540
acagcatctt gctctgttgc ccaggctgga gtgcagtggg gcaatctcag ctcactgtaa 123600
ctccacttcc tgggttcaag caattctcct gcctcagtct tccaagtagc tgggattaca 123660
ggtgtgcgcc accatgcccg gctaattttt ttgtattttt agtagaaaca gagtttcact 123720
atgttggcca ggctggtctc gaactcctga cctcaggtga tccacccgcc tcagcctccc 123780
aaagtgctga gattacagga gtgagccacc atgccatgcc cggccttaat tttccatctt 123840
ataagactac ccacattcct tggcctgtgg tccccttcca tcttccaagc cagtgtatta 123900
gtcccttctc gtgctgctat gaagaaatac ccgggactgg gtaatctata aagaaaagag 123960
gtttaattga ctcacagttc cgcatggcgg gggaggcctc agaaagctca caatcatggt 124020
ggaaggcaac tcttcacagg gcggcaggag acagaatgag tgtcagcaga ggaaatgcca 124080
gatgcttata aaaccatcag atctcgtgag actcactcac tatcacgaga acagcatggg 124140
ggaaactgcc cccatgattc aattacctcc caccaggtcc ctcccacaac aggaggggat 124200
tatgggatta caattaaaaa tgagatttgg ggccaggcgc agtggctcac agctgtaatc 124260
ccagcatttt gggaggccaa ggaaggcaga tcacctgagg tcaggagttc aatatcagcc 124320
tggccaacat ggtgaaaccc catctctact aaaaatacaa aaattagcca agcatggtgg 124380
aatgtgccag tagccccagc tactcgggag gctgaggcag gagaatcact tgaacctggg 124440
aggcggaggt tgcagtgagc cgagatcacg ccactgcact ccagcctggg caacagagca 124500
agactccgtg tcaaaaaaaa aaaagaaaaa gagaaagaga gatttggatg gggacccaaa 124560
gccagaccgt atcagccagc aaggtagcat cttcagatct tctaatgctg acctcctctt 124620
ctgcctcctg ctttaaagaa cctgatcacg gctgagcgca gtggctcatg cctgtaaccc 124680
cagcactttg ggaggccgag gcgggtggat cacctgaggt caggagttcc ataccagcct 124740
gggcaacatg gtgaaacccc atctctacta aaaatacaaa attagccaag cgtggtggct 124800
catgcctgta atcccagcta ctcggaggct gaggcatgag aattgcttga acctgagagg 124860
cggaggttgc agtgagctga gattgcgtca ttgcactcca gcctgggcaa cgagtgaagc 124920
tctgcctaaa aaaaaaaatt aaaaaaaaaa attaaaacca gttttaaaag cgattatctc 124980
accaaggtta tcaaaatctt taaaggagcc ggggccagat gctccctgcc acccgcaaac 125040
ccacccctgg tctgctgtag gcaagctggg tcacagcagg cttggcaata agggggtccc 125100
caggggaagt gctccctcca actggaggct cctgcccggg ctgtggggat gccctcctgg 125160
gaggtgaact ggctggagtt gtgtgccaag acttaattaa ccctggagca accagatatc 125220
caaggacaac tctggagccg ctgagagtga cccaacctag atctggcact tcccccaaac 125280
agccccaggt gcaacagact aggggggcca cctcccagtc agccacagcc agctggctcc 125340

aggcctctgg aagtgctagt gcttggggtc cactgagctt tctgactcct cctcagatat 125400

tttaaaaatc ctagcctaaa tgaatttgct acttgcagtc ttgtgccttt aagtgaaatt 125460

caagccactg atttccagag tctaagtcac cagctggttt tacaaataga catgcaaggt 125520

cctagaatct tccagctgtt tgccttaaaa gacaggacac cagatttctt ttatgaggca 125580

acccttgagc ccctacaggc ctagctttgg aaggaaggaa agatttaggc tgcttaagtc 125640

ttattgcagc cactcatggc taccatagac agttgtgcag cctgcgcact acacaaatgc 125700

ctctgggatg tcagggggag cgaacatggg aattaagatc cagccacact gcactcacca 125760

atggcatata tgatctcaga ctggcccctc ccagagggag gtgcgcacac agcgcaggct 125820

caaactctgc ttctcacact agctgtccat catgcacaaa acgtggccta cccgcacaaa 125880

atcaggatgc tgctcaaatc aaatgtgaca cagctcagaa gctcacaaaa ctcaaactgg 125940

tctattcccc cgtgtgtttg aggatcacaa gcttagatgt caaggcaatg tcgaatttcc 126000

atctgggtgc ctggacgtta ctctcaattt ctgtgttcat tgtgtctcaa ccagtaactg 126060

tttgcccagt gctgggccta gaggcagccc tgctctcacc cattgtgggt ttcctgagta 126120

tactctgggg cctcctaacc tcactctttg gtagatagtc agaaatcaaa gccaggggta 126180

agttctgagt ccagaagaag aggcagtgag gacctccttc cacaggcccc atttttcttc 126240

ctttcttttc ttccttcctt ccttccttcc ctctcttcct tccttccttc tttcccttct 126300

tccttccttc ccttcttcct tccttccttc tttcctttct ctctctcttt cttttctttc 126360

tttctctctc tctctttctt tctttcttac agagtctcac tctgttgccc aggctggagt 126420

gcagtggcgt gatctcagct tactgcaacc tcacctcctg ggttcaagca attctcgtgc 126480

ctcagcctcc caaatagctg ggactacagg tgtgcgccac cacacctgac taatttttgt 126540

atttttcgta aagatggggt ttcaccatgt tggccaggct ggtcttgaac tcctggcctc 126600

aagtgatcca cccaccttgg cctcccaaag tgctgggatt atagccatga gccactgtgc 126660

ctggcccagg cctggctttc tgttacgcct ggacgtgctc tcagcagctt actagctggt 126720

atggagagga cgtttcagag gaaacacaga ctgtctcggc tctgaaaagc tgcctagatt 126780

ctgtcctgtt tgtgagaagg ctggcactga aacctccaca tcaccctgaa gtcctggtct 126840

agacccagga ggcatcagca gtgtgaggtg ctaggctagt tatctgttgt ttcctaacaa 126900

gccactcaaa gcactgtggc tttacaaatt ttatgattct cgtcctcatg gtttcataga 126960

gcttactggt ttcagctggg tggttggttc tggcctgggg tcacttgtgg agttacagtc 127020

agatggcatc agtggctagt gtcatctgac tagaagtcca ggatggtctc cttactcaca 127080

tgtctggtgg ctcagctaga agacccaggg cctggccaga tcactctctc tccctgacag 127140

acttctccat gaggctcctt gggcttcctt ccaacatggc cacctcagag gagtcagact 127200

tgttacacag tggctggctt cccctagaac aggtgttacc agagagccag gtggaagctg 127260

caaggctact tctgacttag cagggcacac agtcctgccc actggaggta tacccagggc 127320

cagcccctat gtattcaagg ggaggggatt atataagggc atgacaacca agaggcagac 127380

tgggcgcggt ggctcacgcc tgtaatccca gcactttggg aggcagaggc gggtggatca 127440

caaggtcagg agatcgagac catcctggct aacacggtga aaccccgtct ctactaaaaa 127500

tacaaaaaat tagccgggtg tggtggcggg ggcctgtagt ctcagctact cgggaggctg 127560

aggcaggaga atggcgtgaa cccgagaggt ggagcttgca gtgagccgag atcgcgccac 127620

tgcactccag cctgggcgac agagccagac tccgtctcaa aaaaaaagaa aactaagagg 127680

catggtttat tggggccctc tctggttact atccatgaca agggtgggca ggtccagggt 127740

gggacaacag agaacaggtg gcctgactct tccctccagc taggactgag ctgggctttt 127800

ccacaagcac catcatccat ttatttcaca attgttctgt cacctgccac agagatgact 127860

cagcccctgt gctcaccaac tatctttcct ataggaaatg atccttccag gtggagaaac 127920

agtgcaagat attcccgtcc tcagtattca atccaaatca ctgattcaat atctgcatat 127980

ttgataacag gcctgtaatc caaatgcttt gggaggctga ggcaggagga tctcttgagg 128040

ccaggggttc cagaccagcc tgggcaacat ggcaagaacc catctctata aaaaagattg 128100

gcgtgttagt gcacaccaat agccccagct actcaggagg ctgaggtggg aggatcattt 128160

gactccagga ggtcgaggct gcagggagct atgatcacac cactgcactc cagcctgggc 128220

aacaaagacc ctattgcaaa tatatttata tcatatttat atttatatta tatatttatt 128280

tgtattatat aattatatta taaatatgtc ataaaatata atataaatat gaatttatct 128340

ttataaacat atataatata tatatataca catatacagt attgactata gcccactctg 128400

tttttcagct agttcagaaa gatccacaat ttttttcctc aagccacagc ctccaaatga 128460

ttgaactctt tcgtggattt ccagccagac caaggacatg cacagctctc tatccatcca 128520

cccaggaact ctaaacaagc tcctcccctc ccaagtcttg gttttcttct ggaagaaagg 128580

gggtttatgg agacagagag gttgaccaga tggctggact ccaagagcca ccattatgat 128640

caaggagctc cagctacctt catactcttg ggcaggttcc tcccttatca gagtcttaat 128700

ctcctacctg gatcacagaa agtcagacaa gatgactcta tgggccctcc cagcaccacc 128760

atgcttcaga ctctcatccg acccccttct gcacatccag gaatataacc attgccttgg 128820

gattgggatt tcagcccaga tgttcagtgt agcctgttct cccccagatt ccactcctct 128880

gaagttcaaa caattgcaaa caggcagaca ctgcaatgga agctgatctg aatccattct 128940

ctggctgggt gcagtagttc acactggtaa acccagcact ttgggaggcc aaggcaggag 129000

gatcacttga gcctaggaat ttaagaccag tctgggcagc atggcaaaac cgcatctcta 129060

caaaaaataa aaataaaaat tagctgggcg tggtggtgca cgtctgtagt cccagctatt 129120

taggaggctg aggcaggagg atcacttgag cccagcaggt caaggatgca gtgagctgcg 129180

atcacaccac tgtgctccag cctgagcaac agagtgagac actgtctcaa ataaaataaa 129240

atgaaatatt aataaaataa aaataaatcc agccggacgt ggtggctcac gcctgtaatc 129300

ccagcacttt gggaggacaa tgtgggtgga tcgcaaggcc aggagttcga gaccagcctg 129360

gccaacaagg caaaaccccg tctctactac aaatacaaaa atttgctggg catggtggtg 129420

catgcctata atcccagata cttgggaggc tgaggcagga gaatcacttg aacccgggag 129480

gcggaagtta cagcgagcca agatcatgtc attgcactcc agcctgggca acaagagcaa 129540

aactccatct caaaaaaata aaaaaaataa tttaaatcca ttttctggcc cctagctacc 129600

actgagtgat ggggccaggg tgggcagcag cttagtgtag cagaaaagat tgagatgagg 129660

ggtctgcccg gctgggtgac aatccaggct ctcctacttg gtgacggcat gaccttgagg 129720

catttcttaa cctctctgaa cctcagtttc cccatttgca aaatgagggt tataatgccc 129780

acctcacagg atagttccta tctgagaagg ccggaggagt gcatgttatt attcaaactg 129840

catgctggaa agtgacatct gtttcaaagg cagcggcgct gattcttgtc accggctcag 129900

agccctccct gatataattt aggagttctg ggtttgggag tgatccccgg gcagagctgc 129960

catctccctc cacccctgac atccgcagga tgaataaacc ctgcagcccg cctggaaggg 130020

ctcttgatgc agtggcctca gaagatgccc tgggctgtcg tactctccaa caggccgtgc 130080

taaaaaatga ctccaaggaa aagtaagcag acactccagg ccataaatat ttcagagcgg 130140

aatgaacgtc ctttccccag caccgacttg ccatatatat tatttataaa accataactt 130200

gagttgaaag gtagtcgtcg aaagaataga cagtctcact cagattcacc cggcgattgg 130260

gaaaaccatc tagagggaga gaaaataatg tctgggctag gtgaggtcat aggaagtcac 130320

ctggccccat cccctggccc cagcctcgcc tggagggaca gcccagagag gccccagacc 130380

cagcaaactg ccaggtttgc acaacccagg gcctccctgg tttcttagga aatctccatc 130440

aaatgccaaa caacagtcac aaccagacgg ttaaaaaaaa atctgttatc ccccctttat 130500

aacaggggta gggcttgtca tgagagagcg agttgagaag ggcttcccag aggctattgg 130560

gtgagcagac tcactcccca gctaattttt gtatttttag tagagacagg gtttcactgt 130620

gttgaccatg ctggtctcga actcctggcc tcaggtgagc tgctcacctc ggtctcccaa 130680

actgctggga ttacaggcgt gagccaccgt gctcagccct atattgattt tgtaatatgt 130740

atgtagatat gaggtaggag gtggggcttg acttaggacc agattgagga ctggctgaaa 130800

cagggaagca gcagcaaagc ccctctccat aagacacgcc caccagcacc atgtcagttt 130860

ccctttgcta caacatccgg aagttaccac cccctttcta taaatttctg aatactccac 130920

ctcttcattt gcatgtaatt aaaacagagt ataaacatgt acaagaagac agcttccact 130980

ccctttgatt ttatccctga ccaatcagca ctcctggctc actggcttcc cctcacccac 131040

caagttgtcc ttaaaaactc tgctccccaa atgctcaggg agactaattt gagtaataat 131100

aaaactccgg tctccggcac agccggctct gcgtgaatta ctctttctct attgcaatta 131160

cctgtcttga tgaatcggct ctatctagag agggcaaggt gaaccccttg ggcggtttca 131220

gccccaccct gccatggtgt gcgtgcgcat gtgtgcagat gtatcttcct cccccatcca 131280

atcctcaggc cgggcttgtg cccctgaaag cccgcagttc tgcaggagga ggtccctgtg 131340

ttccctccca gatcctccca ggttatgcca ggaacccagg gaggactcac agatgccctg 131400

gaaactccag cttggctgca gtgggcccct gaggctctcc ctctcctgtt gtaaggcact 131460

gcagccccct gagctactag gaccctcaag ggttggccac actgcccacc tcatggagga 131520

cgccaccaca gcagacacag gtgaagtccg ctccaaagta tgggtgtcca atgtcaacag 131580

catcacaatc ttctacgagg caccatctca tcatcaccaa agctgccagc cccgtgatgt 131640

ggaagcacaa gaggaaatgg tctcagccgg ggacggtgac tcatgcctgt catcctagca 131700

ctttgggaga ccaagacagg aggattgctt gagcccagga gttcaagacc agcctgggca 131760

ccacagcaag acctccccgc tccaaccatc tctacaaaat atagaaacat gagtcaggca 131820

tggcggagtg cacctgtagt ccaagctgct ggggaggctg agatgggagg ctcgcttgag 131880

cccgtgagtt tgagaccagc ctggacaaca cagcaagacc tccgtctcca gccatctcta 131940

caaaatatag aaaaatgaac caggcgtggc agagcacgcc tgtagtccca gctactgggg 132000

aggctgagat gggaggatca cttgagctca ggagtttgag accagcctgg gcaacatagc 132060

aagacccctt ctccacaaaa acaattttaa taattagccg ggtgtggtgg taagtgcctg 132120

tggtcctagc tacttgggag gctaaggtgg gaggattgct tgaggccagg agtttgaggc 132180

tgcagtgagc tgagtttgta ccactgcact tcagcttggg cgacagagtg ggaccctgtc 132240

tccaagaaaa gagagagaga aaaagaaaca aaatagaaat cactggtaac tccatgaccc 132300

agaggtactc caattaccat tggcgtccat gtgtgtggat ggacggttac atatgcataa 132360

atatctatac acatacatat gtatatatcc ctgtaggaca gtatgaatga actttaattt 132420

atttaaacta ttccctacta ttgttcccac ctaaggcgtg ggaatcccag gtcccagctg 132480

gagagagact gtcccaaggc aataccgtgg cagcactgag gtcagtgaat caccagcaga 132540

aatgaaagcc gctgggcgct gggccgaagg gctcaggaga ctcagaagac acttctggga 132600

tcaaggccca gcccagctgt ctgggggctg tgtggtatct ggggacaaac caaggaagga 132660

cagggcgctg gtggggtctt cctggcactg tcgtgttcaa aatgaacaca gcaccacctg 132720

ccccggcccc catcccacag accccctgct cagactcatg actccagtgg ggtggcccat 132780

accaccctac atgcaggctg gggctcttct ccttccccac agtggatccc aatctagcag 132840

ccacggtcag caggaccagg gcacggcgga gtggggcatg gaaaggtggg agaagttgcg 132900

gggagggcgc atgagctagg gaaggtctcg aaggctctca gggaaggcag aagttaggct 132960

gctaagggcc tggatggggc ctgatacaga gagaggaggg gaccttccag gagagaaaaa 133020

gtgcaccaag accccaacca gaagcagaca tcgcctggtc ggcggggagg cccacaaggc 133080

cacagtccct caccaccacc caagggaagc cccagggatc ggaaccactg gccaccctca 133140

ggctcattgg cttggtatgg ctctttattt tattttatta tgatcatttt ttaagagaca 133200
gggtctcaac ctgtcatcca ggctggagtg cagtggacca tgatagctca ctgcagcttc 133260
caactcctgg gctcaagcaa tcctcctgcc tcagcctccc gagtaactgg gactacaggt 133320
gtgtgccacc acacctagct ctttttattt ttcacagagg caggggtctt gattgctgcc 133380
caggctggaa tgcagtggcg ctatcatagc tcactgcagc ctcaaattcc tgggctcaaa 133440
cgatcctccc gcctcagcct cccgagtagc tgagactacg ctacaggcat gtgccaccac 133500
atatggctaa ttttttaaat tttctctagg gatggggtct cacaatgttg cccaggctgg 133560
tcttgaactc ccagcctcaa ctgatcctcc cacctcggcc tcccatagca ttggggttac 133620
agacatgagc cctggcacct agccaactct ttaagatcaa atgctggccg ggagcaatgg 133680
ctcatgcctg taatcccaac actttgggag gccaaggtgg gcagatcacc tgaggccagg 133740
agttcgagac cagcctggcc aacatgacga aaccccatct ctactaaaaa tacaaaaaca 133800
tttagctggg cgtggtggtg cacacctata atcccagcta ctcaggaggc tgaggcagga 133860
gaattgcttg aacccaggag gcagaggttg cagtgagccg agatcatgcc attgaactcc 133920
ggcctgggca aagagagcca aaccccttct caaaaataaa ataaaataaa atgccttttg 133980
tacccccaac catttgaggc ggggaagata cagggcccta ctgagaggcg aagggagcag 134040
ggcccggggt cactgtgtga acttgggtga gattcccatg ctcagggggt aaaacagggt 134100
ttgcggtacc taccttaggg ctagaagtaa gaatgaaatg caagagcaaa cataaaaacc 134160
ccaggctgga ggactgagcc catcggtgcc ttgggaacca caacagtggg atcattcaca 134220
cacagacagg cctggtggaa tcttccagac cggcgccttg tggcgaggag gcaatatgtc 134280
ccacgaggcc cactcaggcc ccaggagccc ccctcacttg ggcacagccc agctaccatg 134340
ccctcctggt tagtgggcac aagagtgtcg agggaatatg ctttgtgggc agcaaagtcc 134400
ttcttcaaag gggtgtttcc acctgcaggt ctgagcagga catgaggtca tgtcgggccc 134460
caggacagca gcccctgtcc taggctcggc cctgacccac cttctctgga agccggtcct 134520
cgagtaccct tagggccttt acacactcac acgtgcagac acacgcacat gcacacacac 134580
acgcacgcac acacacgtgg acacacatgc acacacaaac acacacatgc atacacgcac 134640
acacggacac atgtgtgcac acacgtgcgc agacacacgt gaacacacgc acacactcac 134700
acacacatgc acacacagct gcctccactc actcaggcct ccaccaaccc cctccccagc 134760
tgtagccacc actgccaccc ccaaaattaa aaagaaataa aaataaaaaa ctgaacgagt 134820
taatttgcta aaatgaacca gaatgcaata actgattaca tttttcccca aaggaaactt 134880
gagttaattt ggccggtgac tcatatgact ggctgccagt gacaacacac accatcccag 134940
acacagctcc tcgctgtgcc ccgttgccag ccgtccccct cctggcttcc ctcctgcggg 135000
aggcggggac agaggggtgg aggcagaagt gcccactgga taaagagctg tttgaggccc 135060
ttctgaaagt gcaggataca cacgcatgca cacagacata tatctgtaca gaagtgcgtg 135120

tctgtgcact cacaccaggc acgcacatgc acacacacat acacacacgc ctgcaccaca 135180

gctgaactct ggcatgggct actcagtaaa tcattcaacg ataacctctg cataccaata 135240

ctgggggaaa atccaattaa agtaactcat tactaagtca ttcctatttt cccagcatct 135300

taaagctcct gggaagagaa gggaaggaca ggcagattac taatgtgacc tctcctaggc 135360

tgtggggaaa attcctggcc cggctcccag gagtggaggc cccagatgga tttctctgtc 135420

tgctcggggg tggggggaac actgggtaca gcccccctcc ccatccctgt gccctgtata 135480

cggccatagc ttctcctgaa aacagcacac atagctcaag ctaagtccta gggtcagcag 135540

acccaagagg cccagccttg tcattcacat gctgtgtgac attgcacaaa gcacttaacc 135600

tctctgagcc ttaatctctt ctctaaaata tcttcatctg atggttgttt tttgggtttg 135660

ttttgtttgg tttggtttga gacaaggtgt tcctctgtca ctcaggctgg agtgcagtgg 135720

cacgatcata gctcactgca gccttgacct ccctggctca agcaatcctc ccgcctcagc 135780

ctgctgagca gctgggacta caggtaagta cctccatgcc tggctaattt ttttgtattt 135840

ttagtagaga tggagtctca ccatgctgcc caggctgcat catctgaatt tttttttttt 135900

tttcctgaga tggagtttag ctcttgttgc ccaggctgga gtgcaatggt gtgatctccg 135960

ctcactgtaa cctccacctc ctgggttcaa gcgattctcc tgcctcggcc tcctgagtag 136020

ctgggattac aggcgtgcac caccatgccc agctaatttt ttgcattttt agtagagatg 136080

gggcttcgcc atgttggcca ggctgatctc gaactcctga cctcaggtga tccacctgcc 136140

ttggcctccc aaagtgctgg cattacaggc atcagccacc gcatccagtc catctgattt 136200

ttttaaacta gggggtgggg gaaaaggaaa ggcacacact tttcctcttt cccccttgcc 136260

actgggtggg ttacagaagc gatggcggga gctgaagcag ccatattgga cccaaagata 136320

gaagtcacct actgagcaag acaagaaaac ccagcaagat acaaggaaaa cggaaaatgg 136380

ttccttgaga ttgtggagct gccgtggaag ccctggattg tttgtcatca gactattata 136440

tgcaagagaa acaaacttct atgttgtctt taaaatatgt tacgtatatt tatatatcac 136500

catcatccct atgctcacac atggtgaaat ctaaaaccat tcagaggcca ggcgcggtgg 136560

ctcacacctg caatcccagg actttgggag gccgaggcag acagatcact tgaggtcagg 136620

agctcaagat cagcctggcc aacatggtga aaccacgcct ctactaaaaa tacaaaaatt 136680

agccaggtgt ggtggtgcac acctgtaatc ccagctactc gagaggctga ggcaggagaa 136740

tcacttgaac ctgggaggtg gaggttgcag agagccaaga tagtgtcact gcactccagc 136800

ctgggcgaca gagtgagact ccgtctcaaa aaataaaata aaatacaaaa ccattcagag 136860

gaggtcactc gataaagaca agagatgtcc ccagactcct catccaggaa tccagcgaaa 136920

gggcactatc cagggctcca cgttttggcc caactcgccc agcaatttct gtgcatcctc 136980

aggtacatca ttttcctccc catgcctcag tttccctacc tgtcttttga cactaatatc 137040

agtctcaatt tttaaatgct gaccaatgaa ggactcaagc caaggatggg caggaaatca 137100

ctttgtaaag aactgggatt gggcaaggtg gctcattctt gcagcacttt gggaggctga 137160

ggtgggagga tcacttgagg ctaggagtct gagaccagcc tggagaacac agaaagaccc 137220

ttgtctcaaa aaaaaaaaaa ttaaattagc taagcatggt ggtgagcatc tatagtatag 137280

cccagctact ctggaggctg aggcaggagg atcgcttgag cccaggagtt tgaggctgca 137340

gtgagctatg ctcaagccac agcactccag cctgggtgac agagaaagac tctgtctcaa 137400

aaaaaaaaaa tattttaaag agcaagaagg ctggacatcc gggcatggtg gctcatgcct 137460

gtaatcccag cactttggaa ggccgaggtg ggaggatcac ttgagcccaa ggagttcaag 137520

accagcctgg gaaacatagg tagaacccca tcgctataaa aattaaaatt aaaaaattag 137580

ctaaacatgg tggtgcgtgc ctgtggtccc agctacttga gaggctgagg cgagaggatc 137640

ccttgagccc aggaattgga ggctgcagtg agctgtgatc acaccactgc agtccagcct 137700

ggcaacagag agagaccctg tctcaaaaat aaataaatag gcggggggat cacaaggtca 137760

ggagatcgag accatcttgg ctaacacagt gaaaccccgt ctctactaaa aatacaaaaa 137820

attagccggg cgtgttggcg ggagcctgta gtcccagcta ctccggaggc tgaggcagga 137880

gaatggcatg aacctgggag gcggagttgc actgagccga gatcgcgcca ctgcactcca 137940

gcctgggcga cagagcgaga ctcagtctca aaaataaata aataaataaa taaataaaca 138000

aacaaataaa taaataaata aaggacagag aaaagcagga actgggtcta ggtgtctagg 138060

tgggaccgat gactggctgg agcacagagg gcagagacac ccgcagccgc caggctggtt 138120

tcaagctcac ctgatcctgt tgctaaacca cctggcagga gaggaagctg agaaagagag 138180

aaagctggag gagaagctgg agtcccacca cccaagccct accgcacagc tgcaggtcgt 138240

cagagcctgc cagagccctg tgcccgcctc ccaggccaga caatggagac aaatgcaatc 138300

ccaagtgggg cacaggtcag ggctccatgt ggccccagga atgatgacac ctggggacgg 138360

ttctcagagt ggccagacgt ctgcctaaac gctgaaccca aaattcaagc acagaataac 138420

tgtccaccat gaccttgaag gccagctctc acagccccgg ggagcaacag ccaggcttct 138480

aggcagcggg cgccagcggt gcccaccctg aggcacaccc aggccccacc gtccccttgg 138540

tgtgggtctg agctcgtgcc tttcggcagg tgcacagcct tggggtcggt ctagcctgga 138600

ttcgaatccc acctgtatca cttacaaatg gagggacctt gatcgaggag ggtttataac 138660

ctcccagagg ctcagccctg cccctacacc cagggggctg ctgagaagcc gagaagctga 138720

aggagttcag ggcatagaca gccgcgcatg ctgtaggctc acacatagga aggggacttc 138780

ccctccctcc caactgtggc catctgcacc ctgactggca tgacaaaggg gcaaaaaaca 138840

ttggtcaaat ttctatgcag catccttcag tgtccccaag ccctgtcatt tcaagataga 138900

aatcgggcca atgtgcttcc tcttctccag aaaacagact ccaagcatgg tcaaaaaacc 138960

aaaggtgact gggcacagtg gctcacgcct gtaatcccag cactttggga ggcagagatg 139020

gctggatcac ctgagaccag gaatttgaga ccagcctggc cagcatggca aaaccccatc 139080
actaactaaa aatacaaaaa ttagccgggc atggtggcac acacctgtaa tcccagctac 139140
tcgggaagct gagacaggag aatcgcttga acccaggaag cagaggctgc agtgagccaa 139200
gatcacacca ctgcactcta gcctgggcaa cagagtgaga ctccatctca aaaaacaaac 139260
aaacaaaaaa caaaacaaaa caaaacaaca acaaaaaaac gaacagcgag ggaaccttaa 139320
ggatccaaga ttcccactga gaaaccaggt ctctatttct ccttgttcgg ttctggagcc 139380
gcacgttctg gacttctcca ggccccagac acagcagaaa aaaaacacga gaatcctcat 139440
gcacctggct ttttcattta attaattaat taattaatta tttgtttgtt taagacagag 139500
tcccgctgta tggcccaggc tggagtgtag tggcgcaatc ttggctcact gcaacctccg 139560
cctcctaggt tcaggtgatt ctcctgcctc agcctcctga atagctggga ttacaggtgc 139620
ccgtcaccac acccacctaa tgtttgtatt ttaatagaga tggagtctca ccatgttggg 139680
caggctgatc tcaaacacct gaccttccat ctcagcctcc caaagtgcca ggattacagg 139740
ggtgagccag cactcccggc cacacctggc tttttcttat tatttattta tctatttaga 139800
aatggggttc tcactctgtg gcccaggctg gaatgcagtg gcgcaaccac agctcactat 139860
agccttgact tccagggctc aagtcattct cctgcctcag cctcctaagt agctgggacc 139920
acaggcacgt gccaccacat ccggctgatt ttttaaattt tttgtacagc tagggttttg 139980
gtatgttact cacgctggtc tcaaactcct gacctcaggt gatccgcccc cctcggcctc 140040
ccaaagtgct gggatcacag acatgagcca ccgcgccagg cccacatttt gatttttaaa 140100
gcaccaccac atagctttat gtcctcgttg ttttccacct gaatctcctt atacaagact 140160
caggctgggc caggcacagt ggctcatgcc tgtaatccca gcactttggg aggccaaggc 140220
agatggatca cctgaggtca ggagttcaag atcagcctgg ccaacatggc aaaaccccgt 140280
ctctactaaa aatacaaaaa ttagctgggc atggtggcgc atgcctgtaa ttccagctac 140340
tcgggagact gaggcgggac aattgcttga acccgggagg cagaggctgc agtgagccga 140400
gatcatgcca ctgcactaca acctgggcaa cagagtgaga ctcagtctca gaaaaaaaaa 140460
taataaaata aataaataaa aagattctca ggctgaagtc acgaagtagg gtggccccga 140520
gcccccctca gcagacacag cccaagaggg catctgcaaa gagcaggact gtgggttgtg 140580
ggcaagacgt ggagggaggg tagaggagag aaggggaccc cctacaccag gaggaatttg 140640
caggcttcac cagggaaata ggacggcacc ccctgcccca gggaggtggg cagggcatag 140700
agggcatttt ccaaattgta cagccacagg gaccactctg cccagagctc cactcacacc 140760
ctccaacccc ctagtcacac agacacacat tcacacacac acactacagt cgtacacaca 140820
cagacatgca ttcacatata cactcactga catacagata catactcaca ggcacacaca 140880
gacatgcatt cacatacaca ctaacgcaaa gacacacaca gacattcaca cagagtcaca 140940

cttacacaca gacatgtatt tacacattca aatgaataca cacactaaca cacagagaca 141000

cacacaaaca cgcacacaca gacatgtagt cacacaccag atgcacacac atacactaac 141060

acacagagct gtactcacat agaccacaca catacacaca taggcatcat cactacgcta 141120

atgtctctca gtgtcctacg acaggtgtgc acggccttgc cttgcactga ggtgcccctg 141180

cagtattggg ggttccaccg tagtactggg gtccactcac ccatagaccg tgcatctcca 141240

gcttcatgcg caggaatttc cccatgccac agggcctttt gctggggaag atgctggtct 141300

gcccaaagtc agcgtggaat ctgggcagag ccagaatgac ctgccccagg cttgggagcc 141360

gagcatataa cccccctaca cacagcgcca ttcagaccct ggcaccccac cgttgggctc 141420

cctaaagatg tgtccattct gcttgactta aacactgtat tcctggttgg tccttcagtg 141480

acaggggagg gaggaagagg ctgtggcttg caggacaggg ggtcaggata tgggcaatgg 141540

cttggggacc tcctacttgt aaggaaccag ggtggggtcc tattgggtgg acaggagacc 141600

ctgcagccct ggcctcactg ccttctggga tcctctgcca caggtcccac cccgcagccc 141660

atcctgggcc tgcgacccac agcctgtgcc tcctgtcccc tgtccctccc agtgcaccct 141720

gagcccaccc tacccagccc ccgccacccc cctgctgcac tcccagggct gaccttcttc 141780

cctgcccagg ctggacatcc ctcctctctt gcctccccct tgacctccct gatgggacta 141840

agtggaggct tctgttcatg gaaactgggg ttaaggaagc aaactacagg gcctggggtc 141900

aaggtcaggt ccaaggtcag ggctgagtca gccctgactt cgttcctccc gcctcttccc 141960

ccgggcatcc aggcccttag gtgtcaggag ttacagcctt gccctcgcct cctgctcctc 142020

tggccgaggc caggcggccc ccttcctgtc cccctgccc gtcgttcgct ccccgagggc 142080

gcagcagaag cacggggaac tccggacccc acgaggcgct ctggacgagg tcgagccgcc 142140

gcttcccccg ggcgccgcta ggtggcgccg cgctaacgag gatcgggata gaccggaggg 142200

gccgcgctgc caccaaccgg gtctccactc cccaggtcct cactccccgg gccccagcct 142260

cgccccaaag ctcaaacacg cacccgggaa cccttccacc accctccctc cccacccctg 142320

gcatccttag ccctcggcag ctcccgtggt cccaggggcc tctccggtcc ctcagcagga 142380

gacctggagt gcacggcctg tccatgcctc tgggacgcag gacaaagtgg acgaatagga 142440

atcaaccgtg gggagggagg ggccacccag gagaatccca gtccacggag taagggaggg 142500

tggcatgctg agtgccaggc agaaggcctg gcccaggcgg tgctccctaa gtgagggtgc 142560

accctcactt acatccttcc aggatagttc ctcttcacat ccttccagga tagttcctcg 142620

cctaggcgtt ttctccctca gtttgggggt agtggttttt agggccccgt gtgagagtga 142680

gaaggctcaa gggtgccact accccaaaac caagggggca aggggcagac cccaactgta 142740

ccaggagcaa ggagctgctt taatttactc ctcccaacat gtctattaga cgttttacca 142800

acgaggaaga tgggctcaaa gcccaaggtc acatgggggt ggatggccaa ggcagggttg 142860

gaacccaggt ctggcagatg cccttgccat gccctggggt gccccaactc tgaccacaat 142920

gagcccttcc agctccttcc cagcccaaaa ctaaaccctg aggcagagtg actgggccac 142980
gctcagacgt ccctgactct atgccctacc tctgtccctc tgagactccc actgtcctac 143040
agctgccttc tggtctttgc aatggctgat cgtcctgccg ggaaaggtgt ttccccagcc 143100
tcaccagggc ccactctggc atcaactcct acaggaagcc ctccctgatt gatccctaag 143160
tggaggcttc ggttgggtcc cagcctcagg aaactggcat cagagcctcc catggtcccg 143220
ggctcagaca caatagtgaa ctcagccagg tgaaggaaga gctgagagcc caccaccacc 143280
acgatgctgg tccatcggca gcctgtagca cgccccaaac accccattta caaagaaact 143340
tttcaaaggg catttagggc agatgataaa actatggccc ggagaggcca agtgactagt 143400
gcagagtcac acagcaaacc agaagagact agacaccaac caagatctgt cactatattc 143460
ataaaagaaa agaaggctgg gtgcggtgtt ttgtgcctgt aatcccagca ctttggaaag 143520
tcaagggggt gcagatcgct tgagcccagg agtttgagac cagcctgggc aacatagtga 143580
gacctcatcg ctactaaaaa tgtaaaaatt agcccagcat ggggacgtgt gcctgttgtc 143640
ccagctactc aggaggccga ggcaggagaa ttgcttgagc ccaagaggtg gaggctgcaa 143700
tgaaccgtga ttgtgccact gcactccagc ctgggtgaca aagcaaggcc ctgtctctaa 143760
aaagagaaag aaaataaaag gaagaccaaa agcaatgaat ttaaaacaca cacacacaca 143820
cgcatgcatg agtgcatgca tgtacttaag tggtagggca aagtgccgct gcggactgca 143880
gctgggagag agggtgactc agtgccaaga tgccctgcac agggccccac gcttctgaga 143940
acagtccaga agccgctccc agcatgcagc taaagccatt ctctcctgtg gacacatccc 144000
tggggacaga ccccgtctgt catcacctgg cttgggtaac ccagaaagct ctgccctcca 144060
ggcaccagga cagtgtcacc cataatgaat gccccagagc acctaggggt aagttgagct 144120
gggtcaggcc gggccagtcc aggcagcccc caggaacccc aaaacccact ggcatctacc 144180
tctcagggtc tcaggctgag agccataggt gctgggggac attctggaaa gtgcccccaa 144240
gaaggcttca cagagcaagt cctacacttg ggagttggat ccaggttcag cccctgctct 144300
gccctacctc ggtgtagccc tgagtgcttc tttgagtttc attaatacaa ccatgggctg 144360
gggtgggtgg gcacagagta gcatgtgctg gagagagaga gagagagaga gacagaaaga 144420
aagagaaggc acatgcatat catacatacc ttcacactac tacttctttt cttttttttt 144480
ttttttgaga cagggtcttg ctctgttgcc caggctggag tgcaatggca tcattgaact 144540
cctggcctca agcaatcctc ccaccttggt ctcaccacac ctggctcatt tttttttttt 144600
tttttttttt ttttttgaga cggagtctcg ctctgtcgcc caggctggag tgcagtggcg 144660
ggatctcggc tcactgcaag ctccgcctcc cgggttcacg ccattctcct gcctcagcct 144720
cccaagtagc tgggactaca ggcgcccgcc actacgcccg gctaattttt tgtattttta 144780
gtagagacgg ggtttcaccg ttttagccgg gatggtctcg atctcctgac ctcgtgatcc 144840

gcccgcctcg gcctcccaaa gtgctgggat tacaggcgtg agccaccgcg cccggcccac 144900

ctggctcatt tttaaaattt ttttgtagac atggaatctt gtgctatgtt gcccaggctg 144960

gtcttgaact cctagtctca agtgattctc ctgcctcggc ctcccaaagt gctaggatta 145020

cagggcatga accacccctg gctcctctct ctctcttttt ctttctttct ttctcttctt 145080

tgttccttcc ttcttctttt tttttttttt ttgagatgga gtctcgctct gtcacccagg 145140

ctggagtgca gtggtgtgat cttggctcac tgcaacctcc acctccaggg ttcaagcaat 145200

tctcctgcct cagcctcccc agtagctgga actacaggca cccgccacca cgccaagcta 145260

attttttttg tgtttttgga gacggagtct tgctctgtcg cccaggctgg agtgcagtgg 145320

catgttctcc actcactgca agctccacct cccgggttca cgccattctc ctgcctcagc 145380

ctcccaagta gctgggacta caggcgcccg ccaccacgcc cggctaattt tttgtatttt 145440

tagtagagac agggtttcac catgttagcc aggatggtct cgatctcctg acctcgtgat 145500

ccgccctgcc ttggcctccc aaagggctgg gattacaggc atgagccact gggcccggcc 145560

ccttccttcc ttccttcttt ctcttttcct ttctctttca tgcttttttt tttctttctt 145620

tctttttttc ttttgagagc cagttgtaaa tcatttacct gcaaaggctg agagagcagc 145680

ctcttcctcc tccagcccag gaagccagcc agcagcacat ccaagcagca agcctccctc 145740

cctgggtgtc gctgccgccc acccggctgc acagggaaag cttgcgacgt ctgttcttcc 145800

tcttgcttta tttcttcgtt tccttgccta agcctgggaa cctgcactga ccctgccctc 145860

cctaccccct cttgacctct gccccatgga tgtctggccc cagagtgttc ctggaggaca 145920

tcctctctga tattttcaga gtcacctctg ggcctcccgg ggactatcca cacgaggggg 145980

tgatggtttc tgaggttgga ccccgggaga acacagcagc aagccctgag cctccaggac 146040

aatctccgca ccactcagca agagcccgtg ctgatcaatc agtgaaacgg gaaggcaggg 146100

ccatcgctaa ttcaggcagc accggcatgg agttggccta atttggaagt ggcctctgct 146160

aaagctggcc ttcgaaccct ccaagggggg aaaaaaaaag aaaagaaaag ctttactaag 146220

ctataagcag aagcaggact gcaatcgagc tgcttcacct atttaaggaa agtgatcatt 146280

ttcaaatgcg tttaatgcat gaattcgggt acaccaaaaa ctgatatcct gattatgatc 146340

tgccctttgt cccctaaagg tggtgacact ctccttagca ctaccagcag tcagaattgc 146400

tgccagc 146407

<210> 3
<211> 42544
<212> DNA
<213> Homo sapiens

<400> 3
agatggccat gaatcctatg actgccgatg gtttggtccc aggatgcatt attcagatat 60

tttctccctg tgggcttatg taaacagaaa gctaggtgag tggaagtaac acgtcaaata 120

ctgatttgtg gctgggtgcg gtggctcacg cctgtaatcc cagcactttg ggaggccgaa 180

gtgggtggat cacctgaggc caggagttcg agactagcct ggccaacatg gcgaaacccc 240

atctctacta aaaataaaaa attaaccagg tgtggtggca cacgcttgta gtcccagcta 300

ctctggaggc tgaggcagga gagtcgcttg atcctaggag gcggaggttg cagtgagccg 360

agatggtgcc actgcgttcc agcctgggcg acagattaaa aaaaaaacaa aaaaaaaacc 420

tgatttgcaa caattttgtg tcctagccta gagtttaccg tgctattgaa aatatctttc 480

ggctgggcgc gctggctcat gcctgtaatc ccagcagttt gggaggccaa ggcaggcgga 540

tcacgagatc aggagatcga gaccatcctg gccaacatgg tgaaaccctg tctccactaa 600

aatagaaaaa attagccagg cgtggtggca cgcacctgta gtcccagcta ctcaggaggc 660

tgaggcaggg gaatcgtttg aacctgggag gtggaggttg cagtgagttg agatcgggcc 720

actgtgctct gacctggcaa catagcaaaa ctccgtctaa aaaaaaaaag aaaaatgaaa 780

aagaaaatat ctttcaatga tagttaatat atatatacac acacacactg tatttgaagt 840

gacatgttac atggttttcc actatatgat ttctcatatt tcatcaaatt gatcctcttg 900

aactcctgac ctcaagtgat ctgcctgcct cggcctccca aagtgctgag attccaggcg 960

tgagccaccg cacccggcct ctgatctttt tttcatcttt ttcctagttt ctgacctcac 1020

taccactctg caccacgttg ggatgggtat ccttgtacat ccgtgcagct tttctatgat 1080

ttttaatgtc tgattatttt cttaggttaa atttctaaaa gtagaagtga acagcgtgtg 1140

cacattttta tgactttggg tacatttcat tacattactt tccagaaaag ttggaacaat 1200

tcatagcagt aactgcactg aatttcttgt attacttacc tatttttaag caagtaatat 1260

aatctcttgg ttccaaaaac aaagcataaa gaaaagtctc ctatctgcct acctccccac 1320

ccactgttgt tgattttttg gtgtgtatcc ttccagagtt tcattatata cacagaaaca 1380

aataccagtg tcaccctcct ttgtatgcac atagtctagc atgccatgca cactcttgtg 1440

caagtggctt tttatttttt aaaattaaca ggccaggtac agtggctcat gcctgtaaac 1500

ccagcacttt gggaggccaa ggaaggcgga tcacttgagg tcaggagttt gagatcagcc 1560

tggccaacat ggcaaaaccc cgtctctact aaaaatacaa aaattagctg ggcatggtgg 1620

cgggcacgtg taatcccagc tactggggag gctgaggcag gagaatcgct tgagcccagg 1680

aggtggaggt ttcagtgagc cgagaccgtc ccactgcact ccagcctggg tgacagaacg 1740

agactctgtt tccaaaataa ataaataaaa attaacagtg tttcttacac ttttcaaatc 1800

agtacattaa aatccctcca attgtgtgtg tgtgtgtgtg tgtgtgtgtg tgtgtgtgtg 1860

tgtgtgtgtt tattaaagcg acaccattgt tcaatgatat gaataggtat attttatata 1920

cttggttcca ttttgataga tatttgggtt gcttccttga agaatgttgt gcgtacaccg 1980

tttgacacag gtgcaagctt atctgcaggg ggaagccgat ttgcaggact cattcctata 2040

gggagagggg ccaggcccag ggacttaagt gtttgtgaat ttgtaggaac tgccagatta 2100

cccacactta gcagtttaca ccccttccct ccagcactgg ctgcagagag ccctgtcctc 2160

cagggcccta atgctaattc tttcatgatt gcactaatta tatgcacaaa aggaatgcaa 2220

cagaacaaaa caaaccacct ggttttgtag gtgtggaagt gcacattgca gtaaggagtc 2280

aatgaaatcg gttactgtgg gacaggaccc tcccctcccc ttggttattt tactaacttg 2340

gaataacgcc acttccttgt aggaaacagg cagtgactgt cagtgagtgt gcctaggaga 2400

ggggtgggtc cttttagaga ccaacctcgc agttagccag caacaggctg cagcttagct 2460

agatgaaaag ttagtttgag aaactgaagt cattgccgct aaggctggca aggtgtggct 2520

ggggccaagt tgggggaatg tcgtagtagc cggccctgtg cgcctgagcc cacaattact 2580

gcgtcgctgc tggggtctgg caaagctcac cttgatgcag ttttgttcaa agcagtgcaa 2640

tttccaagcc tcaaaacaga tccttttcaa tacaatgtga ttgtaaaaat tctattaata 2700

aagagaaaaa aaagacgcac cagcggctta caagatcttt ttttgttttt ttttttaaga 2760

gacagagttt cactgtcgcc gaggctgaag tgcagtggta tgatctcggc tcactgcaac 2820

ctccgcctcc caggctcaca caattctcct gcctgagcct cctgagtagc tgggattaca 2880

ggcgcgcacc accatgccgg gccaattttg tatttttagt agagatgggg tttcgccatg 2940

ttggccaggc tggtctcaaa ctcctgacct caggtgatcc tcctgcctgg gcctcccaaa 3000

gtgctgggat tacaggcatg agccactgca cccagtcacg agatcttaaa gtagaagtaa 3060

ctatggtatt tttttcgaga attctgttag ggcgagaagc ccattcgtac tgtgctggaa 3120

ccatcctgac cctgtctgtt ctttgcagga actcaaacca atatgcctaa gtagctgaaa 3180

ctgttgcatt tcaaagcctt ttcaagcata tcttgaggac actgttccct gcagctgaac 3240

tgaagtcttg atctgagccc aggaggagtc cctggggttg tcttgttggg ggtggggaag 3300

tgggaatgct tgcattcagc tttgggggac cttaaccctt tgccccctcc cctgtccccc 3360

cttcacatag ctttaacatg aaagtctttc tgtctgtcag atctcccctg tacaaagagt 3420

ggtctactct aaaaattgct ttgaaattgg aatggacccc tccctctctt ctattttagt 3480

tctagtttct agctctttaa aataatgcaa cttttcgctt tattgttcta aatcaatgca 3540

ttctagccgt tggctgtccc agttgcttcc ctgtggctgg gagcccctct ctgtggttga 3600

ggctggtctt accattgctc cagttgtctt cggccttctg gggcttactt cccctgccct 3660

ggaggcctga ggccagccag ccagtgtggt gtgacccccc tgacagtggg ctctcccggc 3720

tggcccagtt gagggtggtc agttcctttt gtggccttgc cttcttggga gataaaagtg 3780

gccccctggg tgccgtgcgg cccgcccgca gggtccttgg tggagctcca gcattgattc 3840

ctgcaggttc agctcctgcc ccaaggcagc gtccagctgg ctgagtgggg ttctcccagg 3900

gtctctaagc acaggacagc ctgcttctgc atgggggcgc acaggggacc gcagagtcac 3960

actcagaccc ttctaccacc tcctgacttg tggactttgg tggtgggtgg cgacaaactg 4020

gagtccactg ggtctgtggc agagaaaggt ggctttgctg ggggctgggg gagaggaggc 4080
gtttttggat cgcagcatat accttgtcta gaattgtata aaaacctaca ggaaaaggct 4140
gatttttttt taaaaaggct gtttttttaa aaacaactaa aggcacaaaa actccaagtg 4200
tgagaatatc cagaatgcgt ttatataatg tgcaccgttg aaaggaacat tgaaaaaaca 4260
aaaaactccg aggaacaggt cattgggttt ggagaagttt ccttcagaaa gttctcttcc 4320
tgctcgttca ctgtggatta aagctcgttg gcaatttcgg aagttcacgt tcttgagact 4380
cggtgccttt ggaacaatgg ctcacaatga ggacggtgtg cgttcccggc aagaatgagt 4440
gttgggggct tttctcctta tcacccgttc cccagacccc tctcatcggc ctcataaaga 4500
aacagatggt tcatcaggaa tagctcagaa atcacgttgg ctcctggctg tataaagagt 4560
gggaactggg atcataaaca tttgaaatct cctaggaaag gacaatgatt ccctcaatga 4620
aatattgatg taaagggagc attttaaaag gcagaaatga tgaacctgat tcaggttgaa 4680
gctggtagtg tactttaacc tttttgagaa ttgttaattt aaaagcaaat agaatgaaat 4740
atagactata aaagcagatt aaatcatttt tatgcatcct gggttttttg gtgtcacgtg 4800
acaggaagag ttactacaaa gaagaccagg ttttggaaag acggtggggt tcacgtgatg 4860
aaacacaggc catgacccct ttaggaattg gatggaagcc agccaggcca ggccccccac 4920
ccccaggtcc tgggttgggg ctctgtgggc ccaaggagtc ctccagaacc aaattctcca 4980
ctaagagcaa tttttaaaag agcagctgct catcccatgt ccaattagag tgagcagatg 5040
tgtaagaaac atgtctttga ttgattttgg gggagccccc cgcagtttga tgttttgctt 5100
ggaggtaggg gaaacggggg ctgagttaca agtattacct gtaggaataa acaattcttt 5160
tgaaacatca gtagggaaga aagaaaaaaa aaggcagctt aagatttatc taaaaaggaa 5220
gtggaaagtt aaagttgctg gagatctcat tactgtctgt ctcgctcacc gtaattaaaa 5280
aatgcctgaa tgatcttaat aacacatgat tcagctgaca agcactaatt gccctgtatc 5340
aggccaagag aagatgaatg ttgtcaagaa aaccacaagt cagacaagct gattctctgt 5400
gagtctgaca ccgtgttccc atgtacagac cagtttttac tggagttctt ccagcatcaa 5460
aagcagcttc ttaccttccc attaattttc atcgctggca ggcccgagtt gtttgcgata 5520
cactgactac ctcccttgat tcctcctcgc tggctcagga gcttgggtct gcagacaaaa 5580
tgtaaatatg tttcttgctc ttatctttgt cctttttcag ggaagaaatc tgttcccgtc 5640
caatatgaat gccctggtgg gcttgtcagt aaattacatg gtatttggga attgattttc 5700
tgacgccacc gatttgtcaa gaacttatcc tgccttttca gcgatttgac agcacctgtt 5760
aggctgtagg gccatgtgat tgggcagttc tcctgccgag aaaaagggga ctgatgactt 5820
tctgtcactt gggcatccag aaacctcctt gaaggtggag gttattgagg gaggaggcag 5880
gagtggaaaa atgctattgg tttattcctg tgcttttata gaacagccga accattctat 5940
ggcaataaaa cagaatcaca gggcaaggtg cagtggctca cacctgtcat cccagtattt 6000

tgggaggctg aggcaggagg atcgcttgag cccaggagtt tgagaccaac cttggcaaca 6060
tagtgagacc ttgtctctac ccaaaaataa aaaaaaaaaa tcagctgggc ttggtggtgc 6120
acgcctgtag tcccatctcc tcgggaggct gaggtgggag gatcgcttga ggctgggagg 6180
tgggagcttg cagtgagcca tgatcacacc actgcactcc agcctgggca acagagtgag 6240
atcctatctc aaaaacaaac aaacaaaaaa ggaacttgtt tcctagcaag aagcagatga 6300
ggctggtgaa taagcatacc acataagtaa gtaatccact aacttaggga atttcgtgac 6360
cagtcactca gcacgccctg ttgcgaaggg gacggctgac caataggatc atctcagagt 6420
caccctcaca caacttggct tagcctagtt ggcaaaagtg tctcttcaga gacctaccct 6480
cctggaattg aaataaaatg aaaatttgtg atgacgatca gctactgtca gtgtgactta 6540
ggtgtagaaa ttaaaaaaga aaaaaagacc actctaaagg catttgtgca tgaagacacc 6600
tacagaaata aagccggggt gctgttcttg gtcttcattc aggctagcac ctctcgtcac 6660
cttttcactt tggagctcag ggcctggttc actcagcaag tgagcggctc acacagtaga 6720
tgctctgcac atagtagtag gtactcagaa tatagcagct gttgatgtac atgtttccag 6780
aggggccaac acaggaatct gcacgtgggt ccttgcttta agcccagccc ctctctgtgg 6840
gcgtgggatg gagtcttcct ttcttggtga ccggcgccct ctggggtcac tggaggcctg 6900
gacttcccca ctggcccagt gaccttgttt ccttgttccc tgtctcgccc agccttggct 6960
gtcctgggat ttgaaaatct gggataaagc ctttttcggg cctctaaggc cctgctggtt 7020
ctgcgtgttc cagagagctg cacctctggg tggtgagctg gcctttttgg catttgattt 7080
atgtatagcc ctgagtcact gggagcggat ctagagtcca ggccagtgac tgatttcaaa 7140
cgctaacact cttggctcgg gctcaggttt caagtcagcc tccaaagggg atgtttgctc 7200
atttcgtcat cttcctatca cagcaatttt cgtcatatgg aggggtggca ctcatgtacc 7260
ttgatgatta ttattctttt aaaaaatgaa gttatttatg ttatggggtg agttcgtcac 7320
acagtccgaa tgtggaatgt tgccatcctt ttcatcccaa tcctgccagt gatttctgtt 7380
tacaatagaa ctattgacat tgtatgggga tgaaactagc atgagatagt aattgttggc 7440
ttgcctgaga ttttcatcat atgtacacat agacaaaacc tggctttagt tcctaaagct 7500
attgctgttt attgtacttt tattagccaa tgttacagat ccccgtagca tgccagaggc 7560
tattaagaaa tacaggggtc gggggcagga acgaagtccc tggccatgga tcatgcaatt 7620
gatctatagt tatttttttt tctttttctt taattttttt ttgaaatttt aaattattat 7680
tattactgtt ttttgcgatg gagtttcact cttgttgccc aggctggagt gcaatggcat 7740
gatctccccc tcccaggttc aagcgatttt cctgcctcag cttccggagt agctgggatt 7800
ataggcgcct gctaccatac ctggctaatt tttgtatttt tagtagagat ggggtttcac 7860
catgttggcc aggctggtct tgaactcttg acctcaagtg atccaccgac ctcgacctcc 7920

caaagtgctg ggattacagg tgtgagccac catgcccggc ccaaattatt attatttttt 7980

ataagatagg atcttatcac acaggctgga ctgcagtggt gcaatcatag ctcactacaa 8040

cttccaactt ttgggctcag gtgatcctcc cacctcagcc tcctgagtag ctgggacagc 8100

aggtacatgc caccatgcca ggctaagttt taaaatttta ttttgctagg gggtctcgct 8160

gacttgccca ggctggtcgt gaactcctgg ccacaagtga tcttcctgcc ttggcctccc 8220

aaagtgctgg gattataggt gtgagccatg cttttggcca atttttagtt attgtggata 8280

cataatagtt gtacatattc atggagtact tgtgatgttt tggtgtaagc atacaatgtg 8340

taatgatcaa gtcaaggtaa ttgggatata tacttatagt tagcctacag tcttattgat 8400

aatgttctca gaaatttctg attccagagc tccttgctcc tctcccataa ttagataacg 8460

tgtttttctg tccggtaatt gctgtagtac tcatttttag atggggtagg gaaggccatg 8520

tgccatgttg gggcctcatc ccaaagacct tgtgctgttt gggacaggta attatttaaa 8580

gccccgtgtg aggtgggtgg gtgttaaact catgctctcc acaccttccc agagtgcctg 8640

ggaaagcagg cttctctaag aaagtggaat agcgaatagg cagtgatccc tgagagagat 8700

acataatgat tgtcagtttc atggattaca gataattccg tttaatgtcc tctgtttttc 8760

ctttttgcaa atttctatgc attatataga acattgactt tagcatagaa agtgctaatt 8820

cttgatttgg cactttaatg gatatttaca cagtgacacc ataaacattg gataaacaga 8880

ggatttgttt taatgagatc ttcagtgatt tgaagccgtt tcccccttgc cttctggaat 8940

ccccggctcc agttctgcct ggttttggtg atgctgtcac agtggagggc tgactgcagg 9000

cctcgactgt gagccctcac tcggccacct tttcatttcc tcgctgggtt ccaggctctt 9060

tgaagcactc gcctcctccc ctccctgcat gtcctctgta gtgcctattt ctgtgatttg 9120

catatagcgg aaatatttgt tgaactgaat tatttttaca cacaagcctt tcatgtggcc 9180

aacattttga ggtctgtctc ctttggcggg agtctgggag gaatcttcca gttcactaag 9240

aagtgtttga tagcttcagg gcaggcgtgc cctgattggg gctggatgcc ctgcctccat 9300

ccagaggctc tctggccagg aggggaatga accaagtgcc cagattcccc cctacaatgt 9360

ttatgcagag gtgactgtca cctgccaatg gtgacagtcc accagggaag tccagaaggc 9420

ctttggatta aagctacagg agaggcaggt ccccttgcag ggtcagaagg ggaagagaga 9480

agagagagag aaccaaaggt gggaggttca gctactcggg aggctgaggt gggaggatcc 9540

ctcaagccca ggaggtcaag tctgcagtga gctatgattg caccactgct ctccagcctg 9600

ggcgacagag cgagaccctg tcccaaaaaa taaaaaataa agatggaaag atgggaggat 9660

tctccccagg aaggcccagg ccgcagggtc cccttgtcct taaaggccag ctcagtgagg 9720

ctacaggtcc ccttgcccca gttccccgag cacagagcag gcatggcctc ggacgacttc 9780

ctctttgtgt ctcgtggact actcaataaa tattaatgtt ttccaaagat atcttgccca 9840

gctctccctg acgaggctgt tggcttgaag gaataacaac actcgttgca ctcacggaat 9900

attaagtctg tgccagggcc tgttctgagc tcatgacttg tgctactcgg tctctcccca 9960
caagctcaca gggcggatgc tattttcacc gccgtttata ggagaggaaa ctgaggcaca 10020
gggtccttca gtgactggcc cccatgagca ggcagcacat ctggaattcc agcctgtgca 10080
gcctggcccc aggatcccca tgcctctgtg ggggacactc tggaagtgac aggtgaggca 10140
cttctctggc ctctctggat ctcctgctgg ccctgaatgt gggtggttac ccttttgggg 10200
tgctgtcggt gcctcttaga tgtctcagtg accctacagg gtctgccttc cataggactg 10260
cctttttctt tttttttgag acaggttctc actctcctgc caaggctgga gtgcagtggc 10320
acaatctcac ctcattgcaa cctctgtctc ctgggctcaa gtgatcttcc taccccagcc 10380
tcctgaatag ctgggactat aggtgtgcgc cgccatgccc aactaatttt tttttgtagt 10440
ttttttggta gagatggggc ttcactgtgt tgcccaggct ggtctcaatc ttctgggttc 10500
aagtgatctg tccaccatgg cttcccaaag tattgggatt acaggtgtga gccactatgc 10560
tcggccggca cccacctttt tgtggacttt tctttgttga cttggcattc ggcaaggtgt 10620
aggcttttgc caccaacacg atacccgtgg ggagaagccc cctggccccc gtgcctgcca 10680
gaagaaaggc ttagggtccc ctgcgaggac ccacacgtgc actgcatgcc tggatagccc 10740
atgccagcct cactgaggct ggatttagtt cctgagtatg ttatggagaa gtgcccagag 10800
cagggaccgc aggcacaagg tgggcctggc ctttggagga ggctgggcac ggccacctgc 10860
tcctcctctc tcccatgggc aggttggcct tgttgccatt ggaaactctt gagagcccac 10920
gggtgttttc tggccatgtg acctaaatgg gaggcttgct gaagggtaga tgctgcttca 10980
ccagcaacaa gaggagcccc cagacctcag ccacaggatg gcccccaccc tgctgagccc 11040
ccactcttcg ctccctgctc agcagagcaa cacataccat tgcccaaagg atggaccagg 11100
cacagcgtgg ggcccaatag caaatgttct gacgaaggag gtcagtgccg gacactgctg 11160
ggcgttgttg agtcctgggc tggcagggac agttttgcag ggacggtttg gaaggggctt 11220
tgctggggag ggggtctcta ggaggggcag cccctgaata gggcagccga gttgctgtga 11280
ctgcatcttc ctttctttcc gatgggggca aaactcactc ttctttttaa ccattctctg 11340
ttatctgttt ttcttttctt ttgaaacttt ttttttcttt cttttttttt agaaagagga 11400
tcttgctctg ttgccaggct acagtgcagt ggtgtgatta tagttcactg aagtcttgat 11460
ctcctgggct caagcagtcc tcctacctca gcctcctgag tagctgggac tacaagtgtg 11520
agccaccatg cctggataat ttttatttta tttttttaga gatagggtct tgctatgttg 11580
cccaggctgg tcttgaactc ctggcctcag gtgatcctcc tgcctcggcc tcccaaagtg 11640
ctgggattac agacatgagc cactgcaccc ggccatgtcc tctttttctg attcgggagg 11700
ataagggctc cccatgacac tggcatcagc attgggagca tttccgtgac cccagggctg 11760
ctgatacacc ctgtggacag ggcaggaccc acagctaagt cagtaaagca ccatcctctg 11820

ggagtggctg tcatacgaca tttcttgctt ttaggaccat tggactgaaa aaccaaatcc 11880
tgccactgga tctggtgatg acggtgacat tttttttttt tttttttttt gtttagaagc 11940
ttatgattca tcggatctct tttggtttac ggaaagccca gatgtcgtgg tccctctgcg 12000
tgcccactcc aggagaacgc attcaatgtc aaggaaccct gtagtgtgca aagttggatt 12060
tagggatttc ccgaataaag ctcactgagg gtcaatgagt tccataaaca ttattgaggc 12120
cgggtgtggt ggctcacgcc tgtaatctca gcactttgtg aggctgaggt ggggggatca 12180
cctgaggtca gaagttccag accagccctg ccaacatggc gaaaccccgt ctctactaaa 12240
aatacaaaaa tctgggtgtg gtggcacatt cctgtaattc cagctactcg ggaggctgag 12300
gcaggagaat ctcttgaacc caggagacgg aggttgtagt gagccgagat cgtgccactg 12360
cactccagcc tgggtgacag agtgaggctc cgtctccaaa aaaaaagaaa aaaaaattat 12420
tgaatgtctt ccttgtacct gacactgtgc tttatcccat ttaattccac tggataatat 12480
gttaaattca gacttgttcg tagatttata tacatgtaca tatccctctt aatacattgg 12540
tttattctgt ttctccatgt tcataccgcc agcctgatgc ctcatttgca caaatgaatg 12600
gatgagtgaa tccagcttat tcttttattt ctccctggga tctggtatac aagagatggt 12660
tattttttaa tttgagacag agtctcactc tgtcacctag gctggagtgc ggtggtgcaa 12720
tctcagctca ctgcagcccc tgcctcctgg gttcatgtga ttctcatgcc ttagcctcct 12780
gagtagctga gattacaggt gcacaccact atacccagct aatttttgta tttttggtag 12840
agtcagcatt tcaccatgat ggccaggctg gtcttgaact cttgacctca agtgatccac 12900
ctgcctcggc ctcccaaagt gccggaatta taggggtgag ccaccacacc ttgccaagag 12960
atggtcatta aagttgaatt ttggcactcc ttgcagaaac cctacaaagt tggtatcacc 13020
tctattttgc atatcagaaa actgatgctg gccaggtgca gtgactcatg cctgtaatct 13080
tagcactttg ggaagctgag gcaggaggat tgcttgaggt caagggttca agaccagcct 13140
gggcaacata accagacccc gtctctacaa gaaaattaaa aaatgagttg gatgtgtacc 13200
tgtgatccca gatacttagg aggctgaggt gggaggattg gttgagtcct ggagtttaag 13260
gctgcagtga gctatgattg caccactaaa ttccaacctg ggcaatggag caagaccctg 13320
tctcaaaaaa aaaaaaaaaa aaagtgctga gagagatcgt ttcccgaggg tctttggata 13380
gtttgtagaa aaacagtact gttccttcta aagccctgtg gctttttccc tattccaggt 13440
gcacattggc ctttgaggct ggaaaacact tttacttact tccagcttta tgggattcat 13500
aaaatggcct tgagaaaagt aaaccgattt ttctccttct aaaaacatcc agtgggccgg 13560
gtgcagtggt tcacacctgt catcccaaca ctttgggagg ccgaggccgg cggatcactt 13620
gaggtcaggg gtttgagacc agcctggcca gcatggtgga accccatttc tactaaaaat 13680
acaaaaaatt agctgggcct ggtggtgagc acctgtaatc ccagctactc aggaggctga 13740
ggcaggagaa ttgcttgaac ctgggaggtg gaggttgcag tcaggcgaga tcacaccgct 13800

gcactccagc ctgggcgaca gagcaagact atatctcaaa aaataaataa acaaataaat 13860

acatctggtg caggtaaagt gttcaggaag taatttcctg gaagagggaa ctctgcagtt 13920

gcaaatgcgg ccttcttaca agtcgcttgt agggtgttga aactctctcg cgatcccagt 13980

gacttgagat gctctgtttt ctggataact aagcttttcc accttcacta cggggtacca 14040

gggtctttgt agcagagagg cagctgtctt gatggtggaa ccactcccgc cagtgtgtgg 14100

tggggtgccc ccggggattt agggagagag aaggccagtg gcagggactg gctggaggaa 14160

gagggtgcga ggaaaaacac tggctgacat ttgaaggaac gtgcttcagt atcagggaag 14220

gccagcgtgg gataatcggt gtcttggcag atggggttga cggggagccc tgaagccggc 14280

agtgtttaga gattcaaaga ataggggggc tgtggggagt cttttggttc acagcagtgt 14340

cccccaaacc ggcacagtgg gatgagagtg ggctggccag ggaaggggac agggtgttag 14400

gtccagaggt gctaaaatcc ccaaccatgg tcaaaatggg gacatgagag actcggggtt 14460

cagagctcac atgaaggaca gctgtccccc cctccacccc cgccttgggt cagagctggc 14520

taggagagcc caccctgcag cctgtccttc gtgggaatca gcatgagggg tgcaaagtgc 14580

tcagcaaggc ccggcaccag gtttgttctt gggatcagag tgatacacgc gccttacatt 14640

ttttttaagc tgctggtttt gcgggtcagt acggggaaaa acggtgagtc ccttttataa 14700

aatatataga gaggttggtg cgaaaatagc agcttgtttc tgcttgcgta tttctactca 14760

ctgctgggac acaaatctac ccttccattt tctgaattgt attcattcaa actctgattt 14820

aacctctatt tagttaatga attgggcaag ttaacctcga actcttccag gctgtggctg 14880

ttaccgtcac catctcctgc ttctcacttt acacactcat atgagacctt ttttatttct 14940

gaagtgtggc ctgacattgt ttaggctgtt gtttggtgct tatttttaac tcttcttttg 15000

tcacttaagt aggatgtctt aattcttgtt tagtgttaga tcacttagca ggattttttt 15060

tttgagcggg ggcgggggga atatgatttt gtgtcacatt ttgaatactt ctctatggca 15120

gtatctttta gagttattat ggaccctgta attctgttct caaagacgtt taaccactag 15180

aggtagacac caaaaatgtt ttatttgtct catttgggac tctaattcag tgaagttggc 15240

tgtttggagt ctgagactgg atctgttttt ttattttgtt ttgttgttgt tttgtttgtt 15300

ttcagacagg gtcttgctct tctgcccagg ctggagtgca gtggtttgat cacaagtcac 15360

tgtggcctct aacttacatg cttaagcaat cctcacgcct cagactcccg agtagctggg 15420

accacaggtg ctcaccacca ggcccagctg acttttgtat tttttctaga gacaaggcct 15480

cataagtagt tgtttcccag gctggtctca aactcctggg ctcaagtgat cctcctgcct 15540

cagcctcccc aaagtgctag gattataggc ataagccctc acacccagcc aggactggat 15600

cccttgactc acttaatgat cggggtttac ttctgacctg catggggtgc tgtggggaac 15660

ccagagatca ataagacagc ccgtaccttc ttggagctgt atttagcagg gtcatagaag 15720

acgcactcat aatcagaaac catccagaat gtgacttctg gccagggaga atagggaagg 15780

gttcatggag atggtggcat ttgattgaat tctgaatttg gggttgtagg tttcaaatat 15840

tagtttgggg acagtcatcc ttttattttc ttctctaaat ttaaagaaat tgttattttt 15900

aaaaaccaat tggaatgggt attgaatttc tgtgtggaga atgtctacca gtttttcatc 15960

attttctcca ctgtatagtg tcaataagtg ttaaatgcaa tggtctgtaa gactttgttg 16020

ataattttct tttctttgac atagacttgt aaactggaaa ccctgtgtgg gtaatgggca 16080

gtgcctattt cagcatcttg tccccagaaa tccactccct aaaaggcttg ttgaatgaat 16140

cagatctagg agcatctctt ttgtgttgga gcggagagtc agtccggact ttgagcctct 16200

ccttgcactg accagcggca ggctgaagac agttggactt aagaaaactt aatctagaag 16260

caatatgctg gtcagattct ttcaatgcta ggtttagtat cgttcctttt tctcccattt 16320

ggtgaagtgg tgtaatgctt tagcacattt taaataagat tacacaagcg gctgttagtt 16380

ggcatcaaga atcttttaat ctggaaagaa aatgttacct ccttcatttg caaaacagag 16440

tgtttctggc accgtatccc aaagtgtctt ctggttgttc cgtttttcag aagttactgt 16500

gggcttctaa ttaccctcat ttagacatcc aggacagaaa ttgccacttc aacaagaaaa 16560

ttttgtaatg agccggagga aatggtctca tcattactac gttatacgca gtctgagagt 16620

ctgcgtggat ttttatttca caagaatgtg actgtttaat tgaaaaatgt tgttaaaatg 16680

tgctttctgt tcctatcagt atgttgttag tgcttatata gccatgctcg agtcagtgaa 16740

aattggtgcc ctttgccaaa atactttttt tcatttttgt gtataatttt gtgagctagg 16800

tctatttttg tttaattagg ccctgcaaga gaatgaggga gagctgtatt taaattttta 16860

gaccattaaa gggcctaaaa ctatcctaga tgaaaggccc ccaaaattag ctttaatatt 16920

tattatatga ataaaaccaa tagggaaaac gtttccagaa accctttctc tttttccttc 16980

ttcagacagc tggctaagat taattgcagg cctgaacgtg ggcatgtttg ctagtttctc 17040

ttgttctatt actagaaaat aactcttaag agaggagaga aataccgtct tcttgtattt 17100

ggggggcagg ctgtacattt tataactcac taatataatg aaatgtttgt tgataacatc 17160

atattttgct ttctgataga aaagtcctaa tttctatttt ctttttactt tttattttta 17220

ttactttttt tttttttttt tttttttttt taaaatagag atgagggctt gctgtgttgc 17280

ccaggctggt ctcgaactcc tgggcttaag caaccctcct acctcagcct cccaaagtgc 17340

tgggattaca ggcatgagca actgcgcctg gcaagtttgt tttacttttt atttttatta 17400

ctttttgttt ctaatttcta ctttcgacag gattcctttt aaaattttaa tctaggaaac 17460

aaaactgcac gtgaaagatt tgccttgtag acggaaagtt ctggaaacat cagctccagg 17520

ctgtggtgtg aagctgtcgg gctgatttgc ctggtaagaa gcgcgtttgg ggcacggagg 17580

gtcaccttgc cccctgcgag cgttctttca tcatttgagc agccaagcat ccctttgggg 17640

ccaagtttgc caaagactct aacgtagaga agtggacaga cccagttgca tgctgatgtt 17700

aggggcagag agaaagaagg aaagatcctt gcctagctta agcccttgtg cttcttcccc 17760

acatttattt gacttcccaa attggattgg ctgttgtgat tgaaaagatt gtcagcacta 17820

tgtaaatgag aatgagaagt aaaagtgcat ctcatgcaga taatttctat gtagataatt 17880

tgccataaaa atttatattg cagtccttaa aaccttcgga gatttagtct gtctagggaa 17940

aggtgattta tacggcatta aaacactttt tatgtcccag tcctttagat ttaatgttta 18000

ttgcctataa aaatgaaatc ttcttgtgga tatttaaaag ttttggtact gtgtgaaata 18060

tatattcaag ttagaaatgc tttggagatg ttgtaaataa gtggcaaatc tcttgctcca 18120

aagtccactg tgttgtctgg gcccgcatct tggctttgca cgcctgaaaa gctgcctcgg 18180

gtaccggtgt ctgatcatca ccatagtggg tcgccaagta ggagaatgtc ttatgtaggg 18240

atttggtggt ttttcaaaaa acttaaatta gctaacaagg atgggactat tataattcct 18300

cagaaagtta tgcaaggtct gaccagaatt tcattccaat ctgcagaaag actgaattat 18360

ggtgcagttt ttttggtttt ttgtttgttt gttttttgag acggagtctc gctctgtcgc 18420

ccaggctgga gtgcagcggc acgatctcgg ctcactgcaa gctccgcctc ctgggttcac 18480

gctattctgc ctcagcctcc caagacgctg ggattacagg cgcccaccac cacacttgga 18540

taattttttg tatttttagt agagatggga tttcgccgtg ttggccaggc tggtctcaaa 18600

ctcctgacct caggtgatcc acccgcctcg gcctcccaaa gtgctgggat tacaggcgtg 18660

agcccctgcg cccggcctat ggcgcaggtt ttaaagagtg gaagtgcttt gacaatggaa 18720

tgcccagtac aaagcagttt ttaggctttt gcagccactc agattcataa cagcacgctt 18780

caattctggt cttaaataaa actctcatgg agtaaataaa tcatatggtg gggacagcac 18840

tgctgtggag gagggtactg tttaagcatt ttgggggttc ctgaaagatg tttctcaaag 18900

tgagcgctgg agaggagtac ctggtttgat tgttgaacta aagtgtggtt tctgtaaccc 18960

tagggagttc ttccctaggt atgattgagc tccctgagtg ctgggatggt gacctcccca 19020

gcctgtctcc aagtctgagt actggtcggc ttcctagact ccttaaaacg ctgggtattt 19080

tatttaatta atttatttat ttttgagatg gagtcttgct gtgtcgccca ggctggagtg 19140

cagtggtgcg atcttggctc actgcaacct ctgcctcctg ggttcaagca gttctcccgt 19200

ctcagcctcc tgagtagctg ggattacagg catgcaccac cacgcctggc taatttttgt 19260

atttttagta gagacggggt ttcgccattt tggccaggct ggtcttgaac tcctgacctc 19320

aaacactggg tattttggaa atcatgtggg aagtgctttt cctagagttt tgtagctcct 19380

caaaaaggca gaactaatca accaggcttc cccattgtag ccacagttgg atcccaatgg 19440

taagggccat tgggaaacca atggtctggg tgagtctggc ctgggaccct ggcatgggga 19500

agagactgga cttgaaggct ttggatgggc agcttggtct ggacagtatt ggaaataaag 19560

gaacttgaga ctttgaaatt gctgtgatgg gggtgttctc acctgtccat aggggtggga 19620

tgtcagtaag ctttgccggt gtctgtccca tagatagact cagctctgca ttgcagcccc 19680
aaaaggagcc tgcattctta aaagaatttt aggggaagaa agatttaaga gagcacctga 19740
agtcctgaat cccttcagaa ggaattcatg gctgcctgta gtcagctgcc agtccaacct 19800
tgccctccaa aagaagtcac agagattgag ttttcagcga ggcagggaga agttgtcaca 19860
tttgcctaat tcttgtagag gtgagagtgg gagtaaactg gaagggggcg gattattggt 19920
gatttggggg ccacgtttcc ttgtatcacc tgtaaagttt tttttttgtt tttgtttttt 19980
tttttttttt ttggagacag ggtcttactc tgtcatgcag gctggagtgc agcggcacaa 20040
tcacggctaa ttgcggcctc aacctcccca gactcccaag tagctgggac tataggcatg 20100
caccactgca cttggcgtat ttatttattt atttattatt ttttcgaaga gacagggtct 20160
ccttatgttg cccaggctgt tctcaaactc ctgaagcaat ccgcccacct cagcctccca 20220
aagtgctggg attacaggtg tgagccacca tgcctggctc acccataaaa ttaaaatgaa 20280
aagagcagac cacagccagc agtcttccga cctccagctc ttgcaggtcg tgggcagcct 20340
tgccccaggt gcagcaggta ggccaggaag caggtgtggg aacagagcaa cagcaggcac 20400
cgtggggaca tgcacagtaa gcgtcgaatc ggtgtgcgtt tgcagaaacg tgaatgggtg 20460
aatgaagggg tgtatgaagg tatacacagg aacgccacag tcagctttgt gtaaagtaga 20520
ccagtgaaca gattgctggt attgcttctg acagctctgc tcctcagggg aaaatggtga 20580
actcacttta gaactcgcct ttgtcagctc caactagact ctggaaggaa cacaaatgct 20640
cagactttcc catgacagac tgtgaagcaa agccagttac acagctaccg tcactaatat 20700
ttttggtata aagcagttgc taaaattaat tccagtctga aacttgatga attctcagtc 20760
atggaattaa caggtcttaa atgggaaaaa taaaaaatta gcttgattaa aaataaatgt 20820
ttgtgctgaa atgtggagcc tgtctgtccc taacaaacta aatatgaagt ttttcatctt 20880
agaggatttg tttgcctttg agtattcata gggaaagtgc acgtttgtac cagaaattta 20940
gagacgcact cccagtaagt tcttacgagc agtcagtatt atcctttttg tataaggcaa 21000
gcagtcagtc cctatgaaga gtaagataca agcctggctc agcagtggcc cccctgagcc 21060
tatctgatgt gcagaaaagt cacgcacaca caactgtctt gaagggcagc ctcctttcag 21120
ccagcaccca tttactccct cttattaggc cttcctctga atttatatca agcaaactat 21180
ttaggacgca tctgcgaagc aggaagtgtg tttcattttg tttttaatga ggtttcaaac 21240
tctctcttcc catctctgtc cctggatgac agcgcagagt tttacgtcat ttgctattag 21300
acatcttgtg gcttgacatc ctcgaaggct cagccacccc tgggtgtgtt tggttaatac 21360
agcaagtgaa gcctgggcaa catatcgagg cctcatttct acagaaattt ttaaaaagta 21420
gctgggcatg gtggcatgca cctgtcgtgt cagctacttg aaaggctgag gtggaaggat 21480
tgcttgagcc caggagttca agcccgcagt gagctatgat cgtgccactg tactccagcc 21540
tgagtgaagg gcgagatgtt gtctcttaaa aaaaaaaaat aataataata aaataaagaa 21600

aagaaaaaag tagaggttct gtgcttagtt agaagatatc taacaacaca attggttaga 21660
ctttcataat tatttctgct gatgtaagta tcacagcgca ggtcatgggc aacagtttac 21720
tgttttcaca cccagcttca atgttaaatg aaccccagac tatagcgtga ccagtctgat 21780
attatcatcc ccaaatcttt atagtttcag ctcttgggat ggatgactga ataaccattt 21840
tttttccagt gtggtagtct tagggttttg cttattggtg tgtgtgtttc aagaggaacc 21900
tctgttcctt ggacttgatg tgagtggcaa gtcatgtaaa aagctggatt ttatcaatag 21960
tttttgtcta aaaaccaaaa ccaaaaaatt cttcccctgc agtaccagtg cttgaaataa 22020
accaccctcc tacccccacc cagcttgcct gcgtggatcc agtagcaccc ccggtacccc 22080
ggtgggcctt tttggcagcc ccgaggggtc agggatctag ggggaagagt caccgcaggg 22140
agcgcagatg tacttggagt ggggaatggt tgggggggac ccattcccct gctttaaacc 22200
gcaaatgtct tttgatatct agtaaaattg gcaagcagaa tgattgaaaa catgtgagag 22260
gtaactggat ctaagtctca agtagtacat atttgagaag ttttccccaa tgtaaaagtc 22320
actctttaag agccttaaac taataaaaac aaggaaattc catgttacgg ctgccactcg 22380
gccttttcat ttttattcca gaagtcagat ctgacaggtc agcagctcac tggctctggg 22440
ctccccgtgt ttaaagcagt ggggacgagg agaagaactg gcagccgccg ggacggggaa 22500
gtctccaatg ccattcagtg ccaggctcgt aattccctct tagtttatcc tattttctct 22560
tcttggggcg gcttcctgta tgtactttat tcactgaagc tgcttcagga ttccttcaga 22620
gactattctt gtttctttcc atttctgtgt cttttttttt tttttttttt ttttgagaca 22680
gagtcttgcc ctgtggcctg ggctggagtg cagcggcgcg accttggctc actgcaacct 22740
ccgcctcccg ggttcaagtg attctcctgc gtcagcctcc tgagtagctg ggattacagg 22800
cacgcaccac cacacctggc taatatttat atttttagta gagacagggt tttgccatgt 22860
tggccaggcc agtctcgaac tcctgaccac aggtgatctg cctgcttcgg ccccccacag 22920
tgctgggatt acaaagcata agccaccgca cccggctaat ttcctttata gttacactag 22980
cttggaagaa taacatactg agtggcgtgg aaggcgttgg tgcacatagg tgcagcatcc 23040
cagagccccg tccactggcg gctatttgtg catggctgcc tgtggcttct tatctgtgat 23100
ggaacctcgt gggcagagaa gtggctggag agcatcacca aggctgcctg gtccaagttg 23160
cttcctgttg ctgcatgggc agtgttctgg ggtccagacc ctgggtggta gtgataagat 23220
ggtgatgggg tggagtgggt ccttatagat cccacattac tgagcggcct tttgcccttc 23280
aaaccagatc acaatcccct ccctgccagt tggtcctcaa gaaatcacag gaggctgggc 23340
gcagtggctc acgcctgtaa tcccagcact ttgggaggcc aaagcaggtg gatcacttga 23400
ggtcaggagt tcgagaccag cctggtcaac atggcaaaac ccggtctcta ctaaaaatac 23460
aaaaaaaatt agccaggtgt ggtggtgcac tcttgtgatc ccagctactc gggaggctga 23520

gacaggagaa tctcttgaac ccaggaggca gaggttgcag tgagtcaaga ttggattgtg 23580

ccactgcact ccagtctgag tgattctgtc tcaaaaaaat aaaaaaataa agaagtcaca 23640

ggaggcccag catacagaga acatcaggta tagagagatg tccttcctgg gccaaaacag 23700

gtggtgtcat tttgctgagt ttgatagaga aaaagtcctg atgtctgtgt acataagaac 23760

tcacgtctag agttcgagaa aaaccggtga tgagaacttg aacttggagg agtcacaggg 23820

ggatcaggaa agcttggtaa ttggcatgaa tttcctacct ccaggggcct ggcacagaga 23880

cctaggatga agagggggcc atcgagcatg gtgtgaactt ggagagtgtg gggagctgcc 23940

ccagccgagg gcactggatg ggaattcatg ccagaacccc atcacatagc ggcaggagct 24000

gtaattgtgc ttcttaattg tgtatcttca tttagtgcga gtttaggtgg ttagcaatag 24060

ctctttagct agctttacag agtctaagtt aaagagcccc tcagctatct tgtagaaagc 24120

gccctggaca ggatggggtg atgctgacag ctggagtcac tgttccagcg cccagaatgg 24180

ggagactagc agcacttctg ggcagcccat gaagcagcct cttccctctg ccggtgtccc 24240

tcatgtccag cagagctaga agtgaaactc actgaggatt gacgcggccc ctttaaaatc 24300

gcatctccgc tcttggcacc aggagctgtc atgtagtgta acctgttggt gcccagaacc 24360

caggtttcct ggtggcagcc agcggtaccg gtgccaggag gcagcgaagg aatcacttgc 24420

gacgcccgct ctctctgcca tacgagtgtc gttctgcggg gccatctggc tggcagcagc 24480

tctcagccaa tggcaggatg ttgtaaacct cactcgctga atgcggcacc gaaaatcttg 24540

aaaattaact gcaaaaataa aatcaaagct ggctccgaga tggtatttct tggcctggca 24600

taagctctcc ggatgaactt gctctgcctt gttgatagat gcctcttact agtttggttt 24660

tttcccagga gccagttggc atcttttaac ttttccttgt aaaatccagg cagaaacatt 24720

ttgagagctt tctggagttt ttatttgcta attaaaatgt gagaagggaa cacagccaca 24780

ggtagaggtc tagtgcaagc cacagctgtg attcagaaga agaaaaaaaa aagccaaacc 24840

tgacagtttc aaaatgaagt tattagcact tcacctataa ctgtgatagc ttttaacttg 24900

caggatcctt ctcttaggca ccacctccca tccgaacccc agagcatatg ggaaaaggag 24960

ctaatgtgtg taagttgcag gatacttaat cactgaagaa tctaacaatg ataactacta 25020

tctctctgga actttttttt aacttagaag agtatgtctt gttctgttta tatatcacat 25080

agttttccaa cgaccctggg aggtgaggta ggtggtatat tttaccaatg ggaaaatcga 25140

tgctcaggaa gtcttggtga cttgctcaag ttcccataac aagaagctgc tgaatccagg 25200

atggaacctg gtctacccca aatgccccta ctgcttccta gggcccagta atgcccctgg 25260

gtagggcggg cccagctctg gaagaaacag ggctatacaa aaaatcaccc aatattgggt 25320

caccagcctt cttctacttg caaaataatt aagcaaaatc tgtttttatt tgtttttgag 25380

acggagtctt gctttgtcgc ccaggttaga gtgcagtggt gtgaacttgg ttcactgcaa 25440

cctccgcccc tagggttcta gcgattctcc tgcctcagcc tcccgagtaa ctgggattac 25500

agacttgcac caccacgccc ggctattttt ttgtattttt aatagagaca gggttttgcc 25560
atgttggcca ggctggtctc gaactcctga cctcaggtga tccacctgcc tcggcctccc 25620
aaagtgcggg ggttacaggt gtgagccact gtgcctggcc acaaaatcta ttttaattct 25680
gcaagttata agcagccagt ggcccaccag gcccagctgt acgttcccag ggacccccca 25740
aagcgtatat gtcccgggca cacacacact aggtggtttg ttttgttccc cagcagcccg 25800
tgtctgcccg ggcagatgtt agtggctgag aaaccccaac agggagtgag ccacaggccc 25860
gcgggtggct tccctgccag tgttctcctc gcgaagggga agggagccct tgccagaatg 25920
gctggtgccc taacaaattg attcctgcca aacaatagaa ataaatattt tcagcagaca 25980
gaaatgttgg ctctgaaagc attcaaatta aaggcttatt tctgaaacaa atactttagg 26040
aggaaaacat ttttaccagc gcagatgaga tcagagctat tccttttggc agtggcagga 26100
ctattttgag actcagccac aacggtttcc ctgccccgtc tcgtttttca ctgatgacct 26160
cgccccctgt ttcatggaga cagggaattg cattcagcta gaggcatggg gaagagatgg 26220
tggcatggtg ttctgcagag agcctcagtt ggacagtcgg ccgaatctgg attcaaatcc 26280
aggccctacc tctcctgcta tgatttcgac aaagccggtc agtcatctga tcatcagctt 26340
cctcagtctg tgaaatgggg cggtacctcc cttgcaatgg ttaatagcgt tatcctcagg 26400
acctgggggt cattggaggc cagcactgtc cctagaactc tctgtgttga ttaaatggtc 26460
tctacccgcc ccagcaaata taattgccac tagctacatg taactgttgg gcccttgaag 26520
tatggcagct ggaatggagg gaacaaattt taaatttaac ttagatgaaa tgtaatccac 26580
atgtggccac tggctaccat tttggatagt gcagttccca ttcatccctc tcatttcatt 26640
agttggtccc tggagccaat taattggttg gagggtcaac ccactacaaa tctcccaagt 26700
ctttctctgt gccggatgca gctgtgcgga gttgggacct gtctgttgtc tttgacatcc 26760
gtcttctgtc ctgcagacgg agcagtgggg cagtgccatc gagctgggca gaggaattaa 26820
tatcttagcc atcaactgcc caaatcaacc ttcatgggca tcttttaatt ttctaagaga 26880
tggggtcttg ctgtgttgtc caggcaggac tcgtgggctc tttccgcctc agcctcccga 26940
gtagctggga ctacagttgt gcaccaccac acccctctga tatttcttaa ttttaatggc 27000
tgatgtttct taattttaac aagttcccat atgtctcatg atgtatgcta agtataaaaa 27060
gaataataga aggcatgaag tcaaggggcc tgtttcccgg gcatttgttt cagtggtgag 27120
tcacattcat gcgaagcctc ggtttcctta tctgtaagac cagatcagca aaccacctgt 27180
ggctgcctct ctccctggtt gaccccgagg ccaagaggag gggcagggtc agcagccggc 27240
cagacctcag tagcccctcc ccatcctggt atgggggaac acaggaggta gaaaacaagt 27300
gacctacaca aggcgggtgg tggcggggga ggtcccactt taacaggagg caagatggcc 27360
gggagaaagc tatggggaac ccaggcaaat ggagatgagc agaccccaag aaccgtaccc 27420

tagggggctg gctgggtcgg gggaattagg gtcctttggg ctccttgcag agccgaagat 27480

tgtagtgggg agcactgcgt gtgcctacaa ggctgagcct ggcaggcaga attatgtgtt 27540

gttttgagat aacagccaca acagagcagt aaagaatact gcttataaaa cggacatttt 27600

tttgtagctc aaaaatggtc aaatatcagt aatgtcatgt ggtttgagta atccataaag 27660

cagagccctt ctcgttaggg ctgagaatta tcctcccctc tccctgccac agagccggcc 27720

acgtggcctg ccccggcacc tgctccgcct tacatgtcat tatgcgtctc cgagacagcg 27780

tctctcacat ctcaccgttt aaatgagcgt ctagcaagta ttctatggag tgcaatgctt 27840

gttggataca tctggcattt tcatcttcgc agagtgggtt tgtgacgggg tgaaaaacaa 27900

aaaaacaaat tcttgattct tgatggaaat ctctcagtcc tgagagattt ataaaaaagg 27960

cttgtaggaa aactttgtac gcgtagcttt tatactcata gaccaaatta taatttttta 28020

ttcaatctta ttttctgaat aggtaacgtg ttcacttggt ctaaaatcaa aattttagcc 28080

aggtccggtc actcgcacct gtaatggcag tactttggga ggctgaggtg gggggcggtc 28140

acttgagctc aggagtaaca tggcaaaacc gtgtctttac aaaacacaca cacacacaca 28200

cacacacaca cacacattag ctggacatgg tggcacgcgc ccgtaatccc agctactcag 28260

atggctgagg cgggaggatt gcttgagccc aggagttcga ggctgcagta aactgtgatt 28320

gcatcactgc actccagcct gggtaacaca gtgagaccct gtctccaaaa aaataaaatt 28380

tgtatgttta tatactgaga aattttgtct ccgcctctgt ccctttttac ccctactgtc 28440

taccctaagt cagtactgta cttctgttca ttttctgggg gtgtgctgaa cagattcaag 28500

agaatagcat ttccatggaa aggccgcagc ttgtaagttt cacttgaatt tccgtgattg 28560

ggagttaata tcaaatgatc tctcagaatc aagaaatact gatacatatc aaatgtataa 28620

taacattttg agaatttttt tttttttttt ttcagacaga gtctcactct gttgcccagg 28680

ctggagtgta gtggtgtgat cttggctcac tgcaacctct gcctcccggg ttcaagcgat 28740

tctcctgcct cagcctccca agtagctgag attgcaggcg cccaccacca cacccagcta 28800

atttttgtat ttttagtaga gatggggttt caccatgttg gccaggctgg tctcaaagtc 28860

ctgacctcag gtgatccccc tgccttgccc tcccaaagtg ctgggattac aggcgtgagc 28920

cactgcgcct ggccccattt tgagaattaa aggaacatgg gcacgtaatc tttatacgag 28980

gtggagtcag attgaatagc aagaggaaga gaaactgaca tccacgctca ttgcactggg 29040

cgttttatgt gacttaatcc tcatagcagc tctatgaagt atgtatcaga gggtgtcccc 29100

attttacatt aaatcacttg cctggatcac tcagctgggg agggtgagag ccaggtgacc 29160

tacccaaggc tgtgtctctg ggtggggggc tcagaacctc tctccctcgt tttgtagtag 29220

ccagacattg tctcgtataa actatggcaa ctggaagtga accaggtctt ttcctgccct 29280

ctgattttgg tggaaaatgt gacctaccca ggaaaagcac tatgatttta ggagtttagg 29340

tgcacaaagc tgttttccaa gcatgctcat tcttctccca atttgggact cattgctgcc 29400

tctctgccct catctgtggg gccaagggag ctggagcgac agccacagca gaccgctctg 29460
catccctgta tgcgttgtgg gaaacagcag aggaggtggc gttgcgccca tcttacagac 29520
tccagattct gttcacccga caggcagtgg gaatccgtgt tcacgctggt cccttggatc 29580
agaaattcag tacccttccc tccacaccat gctgtagaca cctctttcct ctcctagacc 29640
cggaacatcc tctcttcttc agggtactgg ggaccaagat ccaccctcgt ggtgagaaaa 29700
ccacaccata aataaacaca gccaccatac gccgtgagat ctggccaaga ggtttccttc 29760
tgttcattcg gccctaatct ccaagatggg aaggcattgc cagctgccct gtgtagagat 29820
tcctgaacac gcagtgtttc ttattaaccc gccagataac cagaaaatca gtctttgggc 29880
aagcgcttgc ttctccacgt gcccagtatt ttctttcttt ctttttattt ttttaatgag 29940
acagggtctt gctctgtcgc ccaggctggg ctcgaacttc tggcctgaag tgatcctccc 30000
acctcagcct cccaaatagc tgggactaca ggtgtgcgcc accatgccca gctaactttt 30060
tttttttttt ctggagagac agggtgttac tatgtggccc aagctggtct caaagtactg 30120
ggctcaagtg atccccccac ctcggccttc caaagtgttg ggattacagg tgtgagccac 30180
tgtggccagc tattctttaa tttactagcg ctgactacat gcctgtgatg tccccaggac 30240
cctgcaaagt tctgggaaat tcatagtgag caaaacacag tccttgctgg agttcacagc 30300
ccatggggag acggcaccaa ccctgtgccc agactagggg agctgccacg cagccactca 30360
ctgagctggg cacacaggtt cccatctctt atgaggtgag gcaggaggag cagggaggga 30420
gctggggaag tggcatcaga acattttttg gaagagagaa aggaaaggtt tccaagcaga 30480
aggaacagcc tgtggcgagg tccgagggga aaggagggga ccctgttctc tggggacact 30540
aggggaacgt cagcatgcca ggtcagggtt ctggggagcc ctgggtggtc ttatcgatgg 30600
cccccaggaa gccacagaag gcatttgagc agggaacgcc aagctcacgg cgggctttgg 30660
cagggcgcgc cccgtgtcct ggaggactgt gtcctggtca tctttctagg cccccactgc 30720
atgcccttga ggactaggaa gggtgcctcc ttctctgcat caggctgact cctagcagct 30780
gccgtatctc cagctttcct gctttcaact atgctttttg tctccttttc ctcattgcga 30840
gcctaggcct gtatggggtg gtggagtggg tgggagctca ctgcttaggt gggtgggcag 30900
tgtgggtggt ggtggttcca tgggtctctc cctcccactg tcccatcttt gccactcatc 30960
tgctgccccc cactcccgga ccccttttgg aggtcagtct cactctgtgg aaaccctggc 31020
ttcctggttc gagatgcccc ctctggccca cgtgtctgac atcacgtacc agaacactgt 31080
ggccatcatt caggattttt tatgacacag tagtgaatca aagcctgtca gtgggaaagt 31140
gatggggaag gattaagtgt ctgagaacat ttgggaaata tgaggttaga cctggttctc 31200
aaacttatga aataagtgaa aacatgaacg ttcactttgg gaattctgca agattctcct 31260
ttaaatggag tccatgaaga gaacaaaatg aattttctgc agcgttacgc aggagcactt 31320

ttctggaaat gaggtttcct aggactttgt cagcccttca aagcctaggt ctccttacta 31380
gaggggaaga aatgagattt tatttatttt tatttttatt ttttattttt tgagaccaga 31440
gtctcactct gtttcccagg ctggagtgca atggcacaat ctcggctcat tgcaacctcc 31500
gcccccaggg ttcaagcgat tctcatgtct cagcctcccg aatagctggg attacaggtg 31560
tacgtcacca cacccggcta atttttgtat ttttagtaga gacagggttt caccatgttg 31620
gcctggctgg tttcgaactt ctgacctcag gtgatccacc tgctccagcc tcccaaagtt 31680
ctgggattac aggtgtgagt cacccactgg ccacaaactt atgaaataag caaaaacatg 31740
aactttaact ttaggaatct acaaggttct cctttaaatg gagcccacga agagaacaaa 31800
tgaactttct gcaaatgaga tgtcctaaga ccttgtcagc ccttcaaagc ctaggtctca 31860
ttactggagg ggaaaaaata agattttaaa atatcaatgt ttagccagac tcagtggctt 31920
acccctgtaa ccccagcact ttgaggaggc tgaggtggga ggatcgcctg agcccaggaa 31980
ttcgagacca gccaggacaa catagcaaga cttccatcta ttaaagaaaa aaaaaaccat 32040
cgtttacttt taggagtagg cattacattt acgtgcttaa aataccaaag gtacccaagt 32100
gtagatatga aagactttcc cacccccaag tgagtgccca gccccttaat ttccctccct 32160
ggtatggata ctaccagtat cttatgtttg tagaaccttc tacagaacct tgtgtaggta 32220
gaagcagatt ctatgcatct atgtgtatct gtgtatttgc agtgactaga ttgattgctg 32280
ttgatggaca tattctgggg aggtgttttt acacttccat tactgagcac aggctcacag 32340
gatggcactt tcccgcatgc accaccatgc ctggctgatt tttgtatttt tagtagagac 32400
agggtttcgc catattggcc aggctggtct ggaactcctg acctcaggtg atttgctcgc 32460
ctcggcctcc caaagtgctg ggattacggg cgtgaaccac tgcgcccagc ctgttttttg 32520
tttgtttgtt tgtttgtttg tttgtttgtt tttgaataga gacagagttt cactgtgttg 32580
gccaggctgg ttttgaactc ctgacctcag gtgatccacc cacctcagcc ttccaaagtg 32640
ctgggattac aggtgtgagc caccatgcct ggcctcaatt tctccctttt ctaagccccc 32700
cacctctgtt tttgtcccat ggtctggtcc tgtcggcctt gttgcacctg gcagtcaggg 32760
ttgacttatg atttacttga accatccctg gagcccctgg cctggtgggg gatgctggtg 32820
gcagaatgaa ttcgggtctg aggtcaagca tgtgtctgtg agagtacaca aaatgaactg 32880
tgatgtagcg tttactgtgc cacaaacttg aaatgcatca tcttgattat gtccagagaa 32940
aggaagtcat ttgaccacag ccacacaggt tacaatgggg atgcggcctg gagtccaggt 33000
gtgagctacc acacccagcc cacatacact ttaaagatga gcaaatccag gctgtaatcc 33060
cagcacttag ggaggctgag gtgggaggat cacttgaggc caggagtcca gtcaggtttg 33120
ggaggccgag acaagaggat cgcttgagcc caggagtcca gtttaggttt ggtaggctga 33180
ggcgggagga tcgcttgagg ccaggagtcc agttcaggtt tgggaggccg aggcgggagg 33240
atctcttgag gccaggagtc cagttcaggt ttgggaggcc gaggcgggag gatctcttga 33300

ggccaggagt ccagttcagg tttgggaggc cgaggcggga ggatctcttg aggccaggag 33360
tccagttcag gtttgggagg ccgaggtggg aaaattgctt gaggccagga ctccagttta 33420
gacaccactg tttgaagcgt cttgggatgt tttcagtagg ggacttggag aagtgatgga 33480
aagaggctgg tgggtggggc ttaggcccac cacccctttc tagccagaga accctccatt 33540
catcgaaatg attctgttgt tggaaatgaa acaaaaaata aaatggtctt aggaccttcc 33600
tgtgcaggaa ccatggccga cctttccctt tttgtacaag tgatctattt ttttcactag 33660
caaaaccatt taagatttct atataggatg tgctgtttga ttagtttgac agataatccc 33720
ccaagaaaca tcagatgaac tcactctttg gattaaagtt tgagataata aaacaagaat 33780
acttgtgttg ctgatgtggc cagcccaagt atgtttttcc caccagccca gaggttctgg 33840
gggcaggagg catcctttgc aggcctagaa ggacccaccc tgtgggagcc tggccctgtg 33900
catctgcaga gagcacttgc tggaaatctc agtgggaaat ggtggggatg cctcccatac 33960
ccccacttta aaacaaacaa ctgacaatta cacttttttt tttgagacag agtctcgctc 34020
tgctgcccag gctgaagtgc ggtggcacca tctcagctca ctgcaacctc cgcctcccga 34080
gttcaagcga ttctcctgcc tcagcctctc aagtagctgg gattacagat gcacaccacc 34140
atgcccagct aattttttgt atttttagta gagatggggt ttcaccatga tggccaagct 34200
ggtttcgaac tcctgacaat tatacttttt gagacagggt ctccctctgt cacccaggct 34260
ggagcacagt ggcttgatta tgactcactg cagccttgac ctcctgggct caaacgatcc 34320
tcccgcctta gccccccaag tagctggaac cacaggcacc caccaccgtg tccagctaat 34380
ttttgtattt tttgtagaga tggggttttg ccatgttggc aggctggtct caaactcctg 34440
ggctcaagcg atctgcctgc ctagacctcc caaagtgcta gaattacagg catcatccac 34500
tgttcccggc ccacatacac tttaaaggtg aacaaatcca agccaggcat ggtggcttat 34560
gcctgtaatt ccgcactttg gcaggccaag gcgggcagat cgcttgaggc caggagttca 34620
agactagtct ggccaacaag gcgaaacccc ctctctacta aaaacataaa aattagctgg 34680
gcatagtggc tcatgcctgt aatcccagct tctcaggagg ctgggtcttg aaccctggag 34740
gtgaaggtta accagtgagc cgagatcaca ccagcgcacc ccagcctggg cgaccgagca 34800
agactgtctc aaaaaaaaaa aaaaaagatg ggcaaatcca aatggatctc tggtgttatt 34860
taaatactta tttgggaaaa aaattttccc actatttctg ttaagcagac agctggttac 34920
atggtagttt ttttaatttg tttttaataa catctgaaat gcctggcaga aagtctggag 34980
ttgggggcag agagaaactt aaaatgttag gagctttgcc ctttgaggag ctgtcagaat 35040
tggagaaaaa acatcccaga aattgtaatc agtgaggaaa acaggattcc agctatccac 35100
agcaccccag ctgtacacca gcgtcacggg atgagggatg cctgtgtgca cgtaggcctt 35160
ggtttctgag ctgagacagc agcagccagg ccgacttggt gataggcttg gcctgcccct 35220

gcagtggact gcctgggcct gggcctgggc cttggccttg gtctcagccc tgtggtggat 35280
gatctgagcc ttggctttgg ccctccagtg gatggcctga gccttggcct tggccttggt 35340
cctgccgtgg atgacctgag ccttggtctt ggccttggtc ctgcggtgga cgacctgagc 35400
cctggccttg gccttggccc tccagtggat gacctgaacc ttggccttgg ccttggcctt 35460
ggccctgccg tggatgacct gagccttggt cttggccttg gtcctgcagt agacaacctg 35520
agccttggca attggccttg gccctccagt ggatgacctg agccgtggcc ttggccttgg 35580
ttctgctgtg gacgacctga gccttggcct tgaccttggc cctccagtgg atgacctgag 35640
ccttggcgtt gaccttggcc cgccagtgga tgacctgagc cttggcgttg accttggccc 35700
gccagtggat gacctgagcc ttggccttga cctctgaagc tcttctgtgc aagggtgggg 35760
ctcctgttgg tgacacccac ttgctgagtc tacacctggg aagctcttgg tcagctcggc 35820
ccattgactt gagagaggaa tagttgccgt ccagcgagtg ctgggggtgt aaggtggacc 35880
gggccacacg gctgccaagg ggccggctgg gtgggactga aacctgaccc atgtgggtcc 35940
tcattgcctc ctgacatcag tcatcctgtg atctcttaga aagataaaaa cacattttct 36000
gcgttctggt gcctctcctt gccagacatt ttctcagtaa agtttctgtc tcagcctcgt 36060
tggaagtgcc aggttctgag cccatcagtt tgctgctagc ctgccagctt cccatgacct 36120
gggacctggg accaggggcc cgcaggtgtt tctagctgga gaggagttct agatagttct 36180
gagcggatac cctcctctgt cttcctcggg agagttctgg cttggtttgg tggtgttttc 36240
tgtgaccagc agtggccacc gccaccacca acccctcttc cccctcccct cttcctcccc 36300
ctcccccctc cctcctcctc ctctatctcc atacgccctg agactggcca gtgggcagca 36360
tattgtgcaa gaaacagctg ttgttttgac aaaggacata actcgcctca cttcttgcat 36420
caccagaccc acgaggctct ggattccagg cctctgcccc tgcaggtgga cacacagacc 36480
gtaacgcaga ggctcacgtg tcccacacta tgcattacca tttgtgtacg cactcgttca 36540
catttagcag gattgtaaaa attaatagtt agtgcagttc ttccagctag ataatgcatc 36600
caaatggcaa gacagtccaa agatacacac tatggagtaa gtccctctcc ccaccggccc 36660
ctctggagag agccaggcac ggtcacaagt ttcttctgta tccctctgga gataatcaca 36720
gcctatgcga gcatatggat ggatattctt ttaaacattg tgttgcggtt tgctttttgg 36780
cttggatgta tctgatgatt atcatatccc ttacaataca ccgtgttttt ttacattaca 36840
ggaaaatctg tgaggggcta aaccgacagg gtattgctgg gcatggagga ggttgggtcc 36900
aggttttttg ctctttgtga ctttttaatg cctttcagat gtgacgactc tccatacgcc 36960
ttctcacacg tgacccgtga ctcatgggcc gaggcctctg catttgggac ccccacctcc 37020
acctccccaa gatgcatttc attcccttgt taatgtgtca ttgtagttga cgctttgagg 37080
ataggcaggc ttggtagata ataaaacagt atttggctta tgtttaatcg ctgtctcctc 37140
ccacagcccg agaacgcctt agcgtccttc agaaaggatt tggtggtact ggtttttatg 37200

tgtgtgtgtg ttgtttttta aaaaaatttt ctccaaggga cctggtcaaa gatttcaagc 37260
cttaaatctg atcaacccaa tctggggcgg cggcagctca gtctctcctc ctcgctgtgc 37320
caatctgttt ctgtggtggc gggtggcggc tcggtgttaa atttggagac ccccgtgggt 37380
ttcccatgcc gacctgcata tgtctgcggg cacggggaag ggagggccgc agacccccgt 37440
tgagtccccg actgccagca gcaagttgcc gcaggcgcac tggctctgca gccccgggat 37500
caaggtgggt tcaccttgca ccgggccacc tgtttcagtt tcctcctcca ccaggcaggc 37560
tctctgacag atcagcccag ggaggggcag gtggggaccg agggggacgg aggggcccca 37620
cagacccccg gctcatctgg catcttcccc agcgtttccc acacttgacc cactagatga 37680
gaagttgggg gtcggggctg ggaccccacc ttcatacctc cagcgcctgc caactcaaag 37740
acatcggtag atgcccaccg attgggtaac cgcccccagc acccccaccc tgctaggctt 37800
ccatgcagtg ggggaagtag ggacccccgg ccaaaagcag tcatgttctt tttgtttttc 37860
tcctaagttt tctgcgtctg tgtgcgattg gaacgaaaca tcaactcttt tttttctttt 37920
tttgtcatat ttcaatattg gctttcttcc ccgccttctt tcgaaggctc cttttataac 37980
ttcggctgct gctgcttaaa tatagatgtc aaagccctcc tccctccaga ttgtttatat 38040
ttttgaagag ccttctgctt cgctgtgact gctcctctct tatctgtatc ccccgttcca 38100
ccacattggt ttcggttgct cttttttttt tttccctgcc ttcccttaac tgggatgtgt 38160
ttcaaatgtg gtctatcaaa atgttcagtg tttccctttt ttctgcaata gctttgacat 38220
ctgttcttga attctagccc acgttattcc ttaggacttg tttgcccaag atgtgcctga 38280
atgccagaaa tactaagggg agaagagtgt ggtgtggtgg tatcttcccc tcattgcctg 38340
gtttgttgtt ttattgtcgt tgttgttttt ttttctttcc tcccctgctt tttaaagtgt 38400
ctacgattcc ttccttcttc cttccttcct tccttccctc cctccctccc tccctccctc 38460
tttccctccc tccctccctc tttccctccc tccctccgtc cttccctccc tccgtccttc 38520
cctccttcct tccttccttc ccttcttcct tccttccttc ctttcttctt tctttctttt 38580
ttcccttggg acacctcact catgcccagg ctggagtgca gtggcgtgat ctcggctcac 38640
tgcaacctct gcctccccgg ttcaagcgat tctcttgcct cagcctcctg agtagctggg 38700
attacaggag cccaccacca tgcctggcta atttttgtat ttttagtaca gacagggttt 38760
ctccatgttg gccaggctgg tctcaaactc ctgacctcgg gtgatccacc cacctcggcc 38820
tcccaaagtg ctgggattac aggcgtgagc cacctcgccc ggcctgtcca tgatttgtaa 38880
gttgacagca tgatttcatc ccatccggca tggcccaccc cgtagtagta ccaaggaagc 38940
atttcccagc aagaacttgt aacgtgtagg tttgctggtt aacttccgcc aaggaggaaa 39000
gcctgtggga cttggtgagc tgggtggtgg ggggtggaga aggagaggct ctagtgtgct 39060
tcttggaggc gtggggataa atcttcagct ttgatttttc tcgccgttca gctctgcttg 39120

gactcagggt gatggtccca accgtgagaa gccagctaga gcagccacct tggatcccag 39180

cagaacgggc cgctgtgtgt ctttagttgc caaagcgact cggcaggaaa acaggccttt 39240

tggtggcgac agcagtgtgt ccttgggcgc ctgctgtcag cctggaacac cattctgggg 39300

ccatctgctg caggcttgtt ggaaacagat cctcttggca gctcttccca gaatttctga 39360

ggccagggca caggttctct ggcgcgttga ttatcggttt gttgcgggga gcagagccca 39420

gagtcctcag ccccctagcg ggtccctcat ggggaaccca ctcctggtgg tcctcagcgt 39480

tcccgcgcaa gtaatgtgat ttgagaataa ctgccaatgg tgcctcattt ctgaaaagtg 39540

gttggaaata attattcttg gaatttacct tttaaaggtc cctcttgaaa actaatcata 39600

ggttaatggt ctcgaacatg atgcttggcc tagagtgagc tcaccataaa catttgttga 39660

atgaatacaa accaagtatt actctcagag atcccaggcc cacagcattg aaggacggcc 39720

acacggaatg ccccgaggag gggatagatc agcagaagga ctagagctca ggagagtggc 39780

tggctggaag gaacccttgg gaagaagagg cgtggagcaa cttggggagg cgggtgcttt 39840

gggcattagc ctgatagttg tggctggagc aagaaagatg tttctgtgta ttctgttatg 39900

ggaacagagt catttatggc gtgcacattg aggcacgacc catgatgttg aacgagcttg 39960

gttttctcaa ccacttcaca tgggacggac gtgggatggg gggataatgt tcatttctct 40020

gccttcccgc caccctccag cccctcctct gccaatgagt tgatgttcct tagagccact 40080

ggggtctctc cccagtgttt ctggtcaaat gcaaatagga ccctcctcta gtacacagtg 40140

caattacaat ctcacttttc ctttcctgtt tccccaacag agagaactcg atgccaccgc 40200

aacggtattg gcgaaccggc aggatgaaag tgagcagtcc agaaagcggc ttatcgaaca 40260

gagccgggag ttcaagaaga acactccaga ggtgaggcgc gtgaccatcg tgttcgcttt 40320

gaagggatct tagaatgctg gtgcatgttc aggcgacgct ccgtgagcgt ttcattttca 40380

tcagatgaac gcacggccgg caaacaaccc gtttctttcc ccagatgtct tcagccccat 40440

ttccagcaga acgcatgcca tcctgcaggc tgtggggatg tggaaattga taggttgtct 40500

ggaaatatga aagtcagagc caattccagg tgcagatact ggacaagctt ggtctgtaag 40560

aacacgtggg caggtgtgtg ggtgtctcag accctcgagc tcatcccaga ccctgtccca 40620

tgtcagttag caagccacca aagtccataa gggatcctgt ggggtggaag gtccgcgggg 40680

cctgcttccc tgttgctggt gcaggcggag tgtctgaagg ctgcacgcat ctgggcatag 40740

cagtgcgcct aacgcttctt gtaaaacaga catttcgcct gctaggcctt ttaaatgcct 40800

ctctgtttct tgaaatatgc cgtaaagggc aatggaaatg tgctttttat atactcctgt 40860

tttttctctc gtgagtgtgc aatcggggga cagtgttgag ttgctggggt ggcgtttttc 40920

tgctcgtttc ctggcccctt cttccttccc ttcaacccta tcaggggctt actaagaaaa 40980

aaaaaaaaac atccaagcgt gttgcaggca gatgagcagt cgcgggaatg gctttccggg 41040

tgacatctgc cagtttggtc cccatggcgc tcatcccgcg ggctcggacc ccagcctctc 41100

ttacatcttc cccttgtatg ggaaggggtc gcagcagccc acagcttcgg cccggcttcc 41160

gggcttgggg aatcttctcc gtatcgtagc tcttggctcc tccatataag acataggaac 41220

atgcctggag gcaaagctcc tttctaggag agatgcccct ctcttactta cataatattg 41280

ctgggaaatt atatgtgaat tgcattttta aaagcggact catttaaaat gtttcaaaag 41340

aggcttgcta gtcaagggac tgctggcatg aatcattatg gaaaacaaat taataacctt 41400

ctgtcttcag aataaatatt ttgggagaaa gcttggtagc agagtagaaa gaaggcagcc 41460

tttggccaca gagccagcta agggttcaaa tctcacaccc gctgcttgcc tcggctgcct 41520

ctaaatgtgg gtactccatg tttcacgaga ccaaaaatgc aggtgggagt cactggtgct 41580

tgggggtttc tgccttctct gccagtgttg gggagtgggg ggccctattc tccatgtcag 41640

ccttgccatg agtaaaaaca ggaggaaaaa aagagctggg gacagaacgt ccttcttctg 41700

ttgcctccag cggcttcaga gcagactttc ctggaactcc ggtttcctga gcgcttgtcc 41760

ttgactcagt ttccccagcc caagccccgc cacatccatc gtgctagctc ctcttagtgc 41820

cgttcctgta gctagtggtc acccgccgtt ctgtattgtc actgcccttt cctcggtgac 41880

catatgtctg agggtttcca tagaaaatct tagaggtttg gctgggcgca gtggctcacg 41940

cctataattt caacactttg gaaggctgag gcaagcaatc acttgaggtc aggagttcaa 42000

gaccagcctg ggcaacataa caagactcat ctctgttata aaaggtaaaa agaaaaaaga 42060

aaacgttttg tggttaaaaa caaacaaaga aacaaaaaaa cccagaaaat cttagagggg 42120

caaggccatc cttcagaggc cacggacaat aaatacacga gtgtattcag ccagagaatg 42180

aggttcctaa gaggcctggc ttcctgggtg ttcctcagaa gtccccaact cctagtgaca 42240

gtggaggagg agcgtgtccc ccttcggtca ccctgtccgt tccttatgct ctgctgataa 42300

atgccgcttg tactgcactg agccctatcc ctccatcgtg ccttgtgtcg aattccacat 42360

tagttactca aagacacctg gccttaaact tggcgttttt atctccttct gatcccaata 42420

aatggaagag gcttggggag gcggcagcct ggtaggtacc tgccacttgg ggcatttctt 42480

ctcaggacac atttttagac tctgtcttca tggagaaagg ggctagaacc caccaggaag 42540

ggga 42544

<210> 4
<211> 158298
<212> DNA
<213> Homo sapiens

<400> 4
gaattcttca tttcagccct ctgaagtcaa aaggaattta cttttgtatc tttagtacgt 60

ttaaagaaga tatctaaagt tctttaacat tttgatagga aatagggagc tgagatttct 120

aaatagtttt gcttggtttt agttttttga tctaacgtta tgaacactga agcttcatat 180

tttcatggtt tcttttgata atttggaata ctttctgcca ggttgagtag ttgaattctt 240

gagatcaata gattatttct gtaagaaggt tttgaacatg aatcgcttaa agttggccta 300
ttataaaatt aggatgagac agttataatc aaatgacaaa gtgcagaaaa ataaaattgt 360
ttttgtaatg cccttcaact tgagtctaaa tgggagtact agcgataact tgcaactcat 420
ttgttaaaga aagggtgttg ccagtggatg aactcctagc tttaggggag tcttatttta 480
aaaatgattt tgtgtgcttt aaaggaatga aaaaatatct tatcagttta agcagtttgg 540
atttagaaat cttccctttt ttcacctccc taatcttggc tttaaaagga atccatattc 600
ttcagtttaa taaatttatc tttttgttgt tttgtatgag aaaaatatta aattttcttt 660
tctttctttt tcttttcttt tctttttttt tttgagacag tctcgctctg tcactcaggc 720
tggagtgcag tggtgcgatc ttggctcact gcaacctctg cctcccaggt tcaagcgatt 780
ctcctgcctc agtctcttga gtagctggga ttacaggcgt gtgccaccat gcccggctaa 840
gttttgtatt tttagtggag acggggtttc accatgttgg tcaggctggt cttgaactcc 900
tgacctcgtg atccgcccac cttggcctcc caaagggttg ggattacaga cgtgagccac 960
catgccaggc ctaaaattaa attttctaat gtgactgcaa atagtactca attaaatatg 1020
tttaaactta gtgttattat ttttatagat catttaagta aaatggtttt aatttgtgaa 1080
aacttcagcc tctggtgaaa attcagttag ttagctctct tacagtaact ataatactgt 1140
cttgactctt tgtgaaggaa gttacattca gctcatttgg ttctgaagaa aacagtaggg 1200
aaataggtta tagagatttt tgctgttcta gaaacagtat aactagaatg tactacataa 1260
ttttctcaaa ggatttctta atctaaatgg aacttgtaaa tggtttttat cccactggaa 1320
tagcatgtat tggataaaac ttgtttaaca cattctgaga ttttataata ttttatgtta 1380
cctcaaagat attaggaaaa atctgcaaat atccattcct gaataaaaga tgacaatatc 1440
ctcaatattt taaagattgt agtatataca tattttcctc aaaagttgag agtactcaaa 1500
tttaaaagct agcataattt acccagttat ttttgtgatg caaggaaaga agatatgtgg 1560
gactttttaa tcatgcattt attggttttc taaaagaact tcacaattgt ttttaatctt 1620
gttatgtgga cttctttatt agaaaagatt atgtggtaaa tgtcaacttt ttctactata 1680
atagaatcta actggttctg tgacacattt cttttttatg atacgattct ttattattta 1740
attacaaagt ttataaaaaa tttcaccact attttgtctc cttagatttt agtacttaga 1800
ttttagcagc atatttttct ttttatcaga aaagagaaat tataagctac taaaaagaag 1860
atgattactt ttacttggta ggaaaaccta actttaagga tggtataaca cagtttgagt 1920
atgagtttat ttttcatctt aacaggttta tttgtgagta tggctttttt tctccccgct 1980
actccccaag acggagtctc gctctgtcac ccaggctgga gtgtagctgc acgatctcgg 2040
ctcactgaag cctctgcttc ccaggttcaa gcaatcctcc tgcctcagcc tcccgagtag 2100
ctgggattac agatgtccgc caccacactc agctaatttt tgtattttta gtagagatgg 2160

ggtttcacca tgttgaccag ctgttctcga actgctgacc tcgtgatctg cccgccttag 2220

cctcccaaag tgctgggatt acaggtgtga gccactgtgc ccggcctcaa gtacggcttc 2280

tttattatta aactctgttt atacaggaga gttgtcctgt tttaagcttt aattgctttg 2340

ggtgatgggc agatgatgta ttgctattag gataagtctt gctatttgta taggtgtgta 2400

tgtcagataa ctgttcaaaa acaacttttt taaagtatgg ctactaaaat ctcacctctt 2460

gagttttaaa gcaattatgg agtagaattt gaaatcaaaa taatcaaaat tggcttgtgg 2520

atatgcctaa aaccagtttg gaaaaaaggt caaggcaaaa aagcagtgta attagaagaa 2580

taaaatgctt tcccttaaaa atagtagatg tgaccgggca cggtggctca cgcctgtaat 2640

cccagcactt tgggaggccg aggtgggcag atcacgaggt caggagttcg agaccagcct 2700

ggccaacatg gtgaaacccc atctctacta aaaatacaaa aattagccgg atgggccggg 2760

cgcggtggct cacgcctgta atcccagcac tttgggaggc cgaggcgggc ggatcacgag 2820

atcaggagat cgagaccatc ccggctaaaa cggtgaaacc ccgtctctac taaaaataca 2880

aaaaattagc cgggcgtagt ggcgggcgcc tgtagtccca gctacttggg aggctgaggc 2940

aggagaatgg cgtgaacccg ggaggcggag cttgcagtga gccgagatcc cgccactgca 3000

ctccagcctg ggcgacagag cgagactccg tctcaaaaaa aataaaaata aaaaaaaaaa 3060

aaaaaaaaaa aaaaaattag ccggatgtgg tggcacgtgc ctgtaattcc agctactcgg 3120

gaggctgagg caggagaatc atttgaactt gggagtcaga ggttgcagtg atctgagatt 3180

gcgccattgc actctagcct gggtgacaga gtgagacttt gtctccaaaa aaaaaaaaaa 3240

aaattagtag atcttatatt actgcttttg gaagcaatta gtgaatgatg taggcaaaat 3300

agtgattgaa ttttgaaaag caattagact gaaatttcaa aggggtggag catttcttaa 3360

tagcaacaaa cagatacatg gtcattaatc caaatatctg aaattttctg agcatgtttt 3420

tcagagtttg gaatagagtg gaagtgctaa ttttctgatt atttaagaac cttttaatcc 3480

attcccttag agcatcaggt gttttcagat tgtgaatttt ctaaaataac attggctgat 3540

ttgtgttatt gaacattggt tgtagcaact tttttccttt tttccccttt attttctgcc 3600

tcccatattt tgcttaagat ttttttctga agttttaaag tttcatcagt tatttgggtg 3660

gaccgtcttg tgaatgctct aattgaagtt gtataatata aacagtgttg cttttagtag 3720

ttaggattaa tttttctttt catggaaatc ctttctattt gattatattt tttcctctaa 3780

cttttattaa ttcttagatg agaattagga ttctgcatta aatctttctg cacacatttt 3840

tcttactgtc agtagggacc tatgtcttcc ttgggtaaag aaaatgttat attttggttt 3900

tgccatttaa aatgatataa agacgttatt agtacttttt taaaaaggaa cttttggtat 3960

ttttaaacaa gtacaaaagt aggtatgaac caccatgtac ctgttactca gcttcaaata 4020

gtatcaaatc ttagtcatta cattgtttca ttcgtactag aacttttttt cttttttttt 4080

ttgagatggt ctctgttgcc caggctggag tgcagtggca tgatcctagc tcattgtagc 4140

cttgaactcc tgggttcaag caattctccc acctcagcct ctcaggttgc tctgactaca 4200

ggcacaggac catgcccagc taattttctt attttttgtg gaaatggggg ctcaccctgt 4260

tgcccagggt agtctcaaac ttctgggctt aagtgatcct cttatctcag cctcccaaag 4320

tgctgggatt gcaggtgtga gccactatgc tgtgatataa taaaatgtct ttatttattt 4380

attttgagat ggtgtccgac tctgtcaccc aggctaaagt ggcagtggtg ccatcccggc 4440

tcactgcaac ttctgctttc tgggttcaag cggttctcct gcctcagtct cccaagtagc 4500

tagaactaca ggcgtgtgcc accatgccca gctaattttt atatttgtgg tagagacagg 4560

gtttcaccat gttggccagg ctggtctcaa actcctgacc tcaggtgatc cacctgcctc 4620

agcctcccaa agtgctggga tcacagacgt gagccactgt gccctgccaa aatcaggatt 4680

atttaatcaa cttttctttt tttctttttt tttttttttt ttgagatgga gttttgctct 4740

tgttgctcag gctggagtgc agtggtatga tcttggctca ctgcaacctc cacctcccgg 4800

gttcaagcaa ctctcctgcc tcagcctcct cagtagctgg gattacaggc gcccgccacc 4860

acacctggct aagtttttgt atttttagta gagacagggt ttcaccatgt tggccaggct 4920

ggtcttgaac tcctgacctc aggtgatctg cccgcttcgg cctcccaaag tgttgggatt 4980

acaggcctga gccactgcac ccagccttaa tcaatttttc aaacagactt tgcttatgga 5040

accacttctt ggaaaatttg gcagaacttc tttgtagagt ttatcttgtc aagaatactt 5100

tcataatgat ataattagca ctgtccaaag cagtgtacaa ataatgtgtg ctacatgtag 5160

tatgagtcca gtctgtttgt acagggatat cttcagacta aagtcctagg agtgaggctt 5220

cctgaagaag atgagtcttg aataaagatt gtgtactgtt tgtataggct agcatatcca 5280

gaaggagaac attatgtacg ttctatatag ggaatggaag catgtcttag ttcattttat 5340

gctgctataa cagaatacca cagactggat aattataatc gacagaaatg tatttggctc 5400

atggttttgg agactgggaa gaaccagaac atggcactgg catctggcag ggccgtcatg 5460

ctatgtcatc ccatggtgta aggtagaaga gcaagagagg gtgagagaac aagaacagag 5520

agggcagaac ttgcttttta tcaggaacct acttccatta ctatggcatt atcattgatg 5580

agggtagagc cctcatagcc taatcacctc ttttaatggc ctcaactctt aatactacca 5640

cactggcaat taaatttcaa tatgcgttgt ggaggagaca tttagaccac agtaaagaat 5700

gagcaaacac ttgatagggg ctagcatgat gtgagaggga tgtaaggagt ctgatctcaa 5760

tggaataaag tgttaattac tgggaatggt ggcagataaa tttgtattag tagggtagag 5820

ctagattatg aaaggtgtat tggtgtcagg ttgaatttaa tctgctaagc aataagaagc 5880

cagtagaggt tgatagggag caggagaaaa catgagagtg tttttcagaa agattactcg 5940

ttcaatagtg tgcagcatat tttggggatg acacttgagg tttgtttgaa aaattagaga 6000

ccaagccact gaggtaagga gctattctga gagacgttag gatggaaatt ctacaggatt 6060

tgtcagataa ttgattccat cttctcttta gtccctcatg ccctaaagtg acaccaaggt 6120

tttgaagagt agagaactga ggaaatgtgg tctatttgtc tttgatagta aactgcaatt 6180

tgttatccaa gcccagcttt tgcgaagaaa gtgatagatg attactccaa taacagcctc 6240

aaaaagtttt agtaggcctg gtgtggtggc tcacacctgt aatcccagca ctttgggagg 6300

ctgaggtggg aggattgctt gaggtcagga gtttgagacc agcctggcca acatagtgaa 6360

accccgtctc tactaaaaat acaaaaatta gctaggtatg gtggtgtgcc cctgtaatcc 6420

cagctacttg ggaggctgag gctaggataa ttgcttgaac ccgggaggcg gaggttgcag 6480

tgagccaaga tggcaccact gcactccagc ctgggctaca gaatgaaact ctgtctccaa 6540

aaaaaaaaaa aaaaaggttt agtagaaaat agctaccagt gtttctgaat gattttaaag 6600

atattcactg gagaatagga gcttggaaaa taaagaaata ttatctttat tacagtaaga 6660

gaaatgaaca tacttcattt gtaaaagaga aatttaggcc ttacctgctt cctatgtgta 6720

tattagttca gaacggttat taggagacta ctcacatttg aatagctata accagtaaat 6780

attactggat gactttaata atggcctgat cagagctaaa gaaaattctg atgaagtagg 6840

gcattaaata tttctagcta ccaactccct ttaaggatga ataaaggtag gtccgcaggc 6900

tgaggaagta tacagggaga ttgtgaaatg aaaacttatc ctttcattta cttcgatatc 6960

tttgttttgc acaggtaata ttaaaaaaca ggtctggtta aacatcagcc gtatatgttt 7020

tggttggaat ttttttttta agttctgtga actttgaagt ttttttaaaa ttctttgttt 7080

tggtttgtag accctgcatt aagccaactt tctggatctg ttgtgtattt ctggatagca 7140

tactttgttt tgtgaaggaa aattttacat tttattttaa agctggggga aaaaagtagg 7200

tttcttgttt ctcgctttgt aaatgaggta gatgaataat actgtattgc cacttattat 7260

cttcatgctt ccttcaatgt ctcaggtatt cctctactgt atgtagcatg tggatctcaa 7320

agtttcccac agttgagtct gaaaacatct caactgtgct tataatttta aaattaaact 7380

taattttacc attttcttta ttgttattat aaaatatagg acctatcata atgccatgaa 7440

ccttttagtg acttgggaaa tagtgattaa gatgatgcaa catagtacat tattgggaaa 7500

taatctgttt ttcgttggca tcgcctctat ccttagacct ctttcattct agtaatatga 7560

cagaaatatt tttgacattt taaaacttcc tcatttagct gatcgtgtgt gctgttgaaa 7620

atgaacacta gaatagaaag ccattgcctt ctccttgggg ggtttgtttt aacaggggtt 7680

ttcatgtttt aattttggat ttgagatttg agttgagata tccattgtgt agagaatctt 7740

gagaaggatt atccatcttt cttgctgtca ccagtgtatg tgggagggaa gaaaatatca 7800

aatatagggt atacaggaga accttagtat ttgggaatat cttcagtgat agctgccaaa 7860

tccagtgttt tccccaggaa atttgtattt aatcccttgg agtatgggat gactttatga 7920

gtgtcatagt gggttgctgt gggtgaggag aggagaaaac gactccctgt ttgaaaaatc 7980

ttttccaaac tgttcgaaaa tttgagatta aattgaatta atctcaaatt attaatagtt 8040

tatgtttttt ttttaattca tacagtgctt gggaagaaat tggttttctg tgagtggcct 8100

tgggggtgct ttgggtgctt tccttaccca ggaaaaggga ggagaggcag aaaataaagt 8160

ggggaatagt agctgctgtg gatttttgcc tatggcattg atggcattgt ttttttggct 8220

gcctttatct acataccttt tcttttctag ttaggctgtc attactagtt ggagaggcta 8280

catacagtct agccttttcc aaatcaaact agcatccctg tcctggtttg tttgttttaa 8340

tctctccgtc tcaaaacctg acaaatttat gtcttttctc tgcctgaaga gcaggggaaa 8400

ctggaaagag aagaaaatga taatgttgta gttaggcact ccctatcttt taatatgtca 8460

ggattgtttt atgaaaaaaa cctctgcctt tggattttaa ttttatgtaa tcagttataa 8520

aataattgaa ttctctatga gtccttagaa accctttaaa agatccttta gccgtaaagt 8580

tggtgatagg ttgaagagta cctatatgag ttaaatgctt tggggatttt ttttgcttct 8640

cctaaatttt tttctgtcat acaggaccta gtcctacatg tgaattttgc ttccaaaaag 8700

taaagctggc agtcttttaa tcccagcact ttgggaggcc gaggcaggtg gatcacgaag 8760

tcaggagttc gagaccagcc tggccaacat ggtgaaaccc tgtctctact aaaaatagaa 8820

aaattagccg ggcatggtgg catgtgcctg taatcccagc tactcaggag gctgaggcag 8880

gagaatcact tgaacctagg aggcggaggt tgcagtgagc caagaccatg ccattgcact 8940

ccagcctggg tgacagagca agagtccgtc tcaaaaacaa acaaacaaac ataaaaacct 9000

taaagattgt attcaaatct attttgattc tgatgaaagc aaggatgaca gcaaaatact 9060

ttaatatatg catgctaaca aaagaacaca agttagacct gtgtcaccat atattctttg 9120

tcatgcctag caagtatttt cagctttggg gaactctgaa tgatagatgg tgtattcatg 9180

tttgtagatt gtggttttta ttatatacta ttaaattcta taatttaaac ttgacatatt 9240

agaggaaata ttgttcttca ttatttggag taaaggatta attatacata actttttata 9300

gtcgaatgta gttttcagtt tcagttttga actctatttg tttttattac ttctgctggg 9360

agtatgctgg gtagtatgga accaggcttc ctacatatga atgttggctc tgccacttac 9420

ttactatctg tgagacctag gttgttactt taccgtcttt gggactcagc ttcctcattt 9480

gtaaaatgga gataccagga ttgttgtgtg gaataaatgg gttaatgtat ataaagtgct 9540

tggaatagtg tttggcacat cgtcgctttg tctaagtgtt tgctgttttt atgcatttga 9600

aggaataaaa aacattcatc tttgaatttc tctattaatt cagtacactt gtaaaacaat 9660

gtctaagtac taaacatata ttttcagtaa tcacttgacc acatagaaat ataaggaatg 9720

tgtttgaggg gtaatcagaa gattggcctg cctggaattc ccttgaatca gagagtaatg 9780

gaaaatgaac ctgtatggat acaatagaaa atgattatca agtgttttga actaaggcat 9840

agaaaattag atgctgataa gaggggaatc attgcaaggt tgttattgtt gtttttaagt 9900

catgatagtg atgtgtttaa aatagtattt aggggaagat agttttgagt gtgcttggta 9960

gcatagaatt atttcactat aaggacagag gatttttttt tggtctgttc tgttcactaa 10020

tctattttaa gcaacccaaa cagaacctgg ttgatggtag atgctcaata aatacttaat 10080

tggatgaatg aaccttaaaa gagactatca gagtctttta tttttatttt gagacagtgt 10140

ctcactctct cacccaggct atagtgcagt ggcgcaatct tggctcacca caacctccgc 10200

ctcctgggtt caagcaattc tcctacctta gcctcttgag tagctgggac tacaggcacg 10260

tgccaccatg cctggctaat tattgtattt tttggtaaag atggggtttc actgtgttgg 10320

ccgggctggt cttgagctcc tggcctcaag caatccgcct gcctcggctt cccaaagtgc 10380

tgggattata ggcgtgagcc accatgcctg accctatgag aatataaaaa agagctgggt 10440

gcagtggctc acgcctgtaa tccaacactt tgggagtccg aggctggagg atagcttgag 10500

cccaggagtt caagaccatc ttgggcaatt atagtgagac tccatttctt aaaaaaaaaa 10560

aataattatc caggcttggt ggtgcactca tgtagtccca gctacttggg agtttaagtg 10620

gggaggatcg cttgagcttg atagatcgag gctgcagtga gctgtgattg tgccacttca 10680

ctccagtctg ggtgacagag actctgtctc aaaaaataaa aagaaaatga gagaacagtg 10740

gactcaactt aaacttttgg ggttcctacg tttcaggggt agaagtgaaa aagatacttt 10800

aataagaaca gtaggagagg taaaaggaag atggaaaagt ttggctcaga atccaaagga 10860

agagattttc taagggaaag gtcagagaga gagagagaga gagagaggaa taggaagatt 10920

caggatagtg ttgcttctag gtagccaagg aggtttcagg aagaagggca cagaggtgga 10980

catagtccaa tacgaagttc gaggagaatg agaatagaga aagaatcctt tggcttggtg 11040

gtgtgagggt cactgaccac tgagtacact aagaatggta atataaatca gattgcaggg 11100

aggttaagga agatgttaag cagagactga taaagactat tcagttggcc tggcacagtg 11160

gctcacgcct gtaatcccag cactgtggga ggccaaggcg agcagatgac gtgaggtcag 11220

gagtttgaga ccagcctggc caacatggcg aaaccccgtc tctaataaaa atactgaaat 11280

tagttgggca tggtggtgca tgcctgtaat tccagctact tgggaggctg aggcataaga 11340

atcacttgaa cccgggaggt ggaggttgca gtgagtggag attgcgccac tgcactccag 11400

cctgggtgac ggagtgagac ctcttgtctc aaaaaacaaa aaaacaaaaa ctatttagtc 11460

aagaaaataa ctgtaaaaat ggccagaaaa caatataata ggagcattca cagtttttgt 11520

atgtatactg taagagagtc tgtttattag aggtgagaaa acttgagaca gtttgtgctg 11580

aatattattc tttttcttta ttttagagac aggatcttcc tttgttttcc aggctggagt 11640

gcagtggcac aatcatagct tattgcagcc ttgaactcct gggctcaagc aatcctccca 11700

ccttggcctc ccaaaagcac tgggatttac agatatgagc tattgctccc acccagccct 11760

gaatgttctt aaatttcttt ctttctttct ttctttcttt ctttctttct ttctttcttt 11820

ctttctttct ttctttcttt tttttttttt tttttttgac acagggtctc actctgttac 11880

ctaggctgga gtgcagtggc gagatcttgg ctccttatag cctcccagcc tcaggtaatc 11940

ctcccacctc agcctcccaa gtagctggca ctgtaggtgc atgccatcac attcagctat 12000
tttttttttt tttttttttt ttgaggcaga gtctcgctct gtctcccagg ctggagtgca 12060
atggcgcagt ctcggctcac tgcagcctcc gcctcccggg ttcaagcgat tctcctgcct 12120
cagcctcctg agtagctggg actacaggta tgtgccacca tacccggcta atttttgtat 12180
ttttagtaga gacagggttt caccatgttg gctaggctgg tctcaaactc ctgacctcag 12240
gtaatctacc cgccttggcc tcccaaagtg ctgggattac aggcgtgagc caacttttcg 12300
tattttggtg gagatggagt tttgccatgt ttgttgccca ggctgctctt aaatttcata 12360
atagggaata tgtattgtat atgtttctcc agggctctgc tacttgctaa ccatgtgatc 12420
ttgggaaact ttgcttaata tttttgtgcc tcagttttct gccttgtaaa attgaggcaa 12480
aaggaacacc tatttcatag atctattgtg aggatgtaat tatttaatct tttaaaaaca 12540
cttagaacaa tgctatgtgg taaatgtttc ataaatgtta gctattatct actgctgaag 12600
taaaatagag taaaattggt ggcttgaaga aaaaatagaa gaagaaaaga atttaataat 12660
gcaaactagt ggcaaaaact aaatggaaag gtcttagggc cttttccgtg gatgttattg 12720
gagaaaactt tggatgtcta cttactctat ttatttacac ttggatttca ggcccctttc 12780
ctctaatatt tgcactgttt tacttttttc tataacactt atgttctgac atatcatata 12840
attaatatat atactgtttg ttgttgatct ctttccaggg tagggatatt tgttttgatc 12900
cctgatgtat tccaggttct gggaatagta cttggcacat aataggtgct caatatttgc 12960
ttgttggatt aataagttaa tctaccatag aagtaggatg gatggagaaa agtgttcaaa 13020
gtgctggtgg tccttttttt tttttttttt ttttttgaga cggaatttca ttcttgttgc 13080
ccaggttgta gtgcaatggc gcgatctcgg ctccctgaaa cctccacctc ctgggttcaa 13140
gctattcacc tgtctcagcc tcccgagtag ctgggattac aggcgcgcac taccatgccc 13200
ggctaatttt tgtattttta gtagagacgg ggtttcacca tgttggccag gctggtcttg 13260
aactcctgac ctcaggtgat ctgcccgcct tggcctccca aagtgctggg attacaggtg 13320
tgagccactg cgccccaccg ctggtggtac ttttttagag aagccagtta tcagattgat 13380
ggacactata taggacttta ccttctcaaa gacttcctcc tttttctcaa atgagttttc 13440
tgagtttttt tttttttttt ggtacaggtt ttcttgaaat cacttgggaa acccatttgt 13500
ttaatagctg aggaaaggaa tctataaatg aatctctttt ttttgataca atacttatat 13560
tttacatttg attagctaat gaaagcctat ccgtgagcgc ttattagatg ctaagcacta 13620
ttctaacctc cttattagtt tcaaaaaatt ctataacacc ttacaagtgc attacagttt 13680
ctttgcaagc cattattaag gtttaagggg aggccctttg tactgagacc aaaagcatga 13740
ttccccaaac tgttgtcttc cattgatagg gagcttcaaa taccacattc cttcctccaa 13800
ttttcatagc tttgacactg ctacttgtat tagtccacca ggactaatct cattatgttc 13860

tttatccctt ttccctttct atgtcagtta ctatcagtct cctaggtgat actcatgtcc 13920

ccacaaggtt tggtgtattt actatttttt tcaatcctct tcaactcttg aaatcttgcc 13980

attgtgcaac ccagtggaat tcacagtcta tcatcagcaa aatttcctgt atccttgttt 14040

gtctttctct gaacattttg tctacttcgt actttgctga ggatgctgct tcccctggct 14100

gtcttataca ttggcaatta aatatttcat ttttttctga taatgtcatc aaggatcagt 14160

ggggagtcct atgcactgag acttgaaact acatttcctg gccaggcatg gtagtataca 14220

cctgcagtcc cagctacttg agaggtagag gcaggaggat ccctgagctc agttcaaggc 14280

tagcctagcc tacgtagtga gaccctgtct ctaaaaacaa acaagcaaac aaaccaacca 14340

accaaccaac cattttcctg ctctgaccta ctccatttga acaggagtca agagggagcc 14400

tggttccact gactggaacc aagagcctgg agatggggca gttgtccacc attctcttgt 14460

tctccttaaa acttgtctat taacaggtta tattgtctgt aaatcctcat gtttgctggc 14520

atctactgcc acctgggtta caccttctct tttcttgact ggtttagctc ctggctcatt 14580

gtcattcttc taacactaag tctcttttaa ttcttgataa tttcaatata tatcactatg 14640

gtccctctaa tactgtgacc tcttaatttc taaatgtctt ctttaatgat cttactctct 14700

agcccattta aggcacttca tggtcctatc ttttttatgc attaaaaaag atattttgta 14760

aagatggggt cttgctgtgt tgccaagtct ggtctgaaac tcctggcccc aagtagtcct 14820

cctttcttgg cctcccaaag agctgggatt acaagtgtga gccaccacac ctggcctcat 14880

gatcatgtca tataccttgt tattaccaat aacttgaaac ctcttcattt tttctaagct 14940

ctttctgact gctgtctttt cagcccattc tacctggtac ctcaactcaa cagttcctca 15000

ccgtcattaa aactgtcagt ccactgattc tattgccttt tcactgtacc ttgccccgtt 15060

gatttccttt cttccttcct taccttgtat aattcaatcg ccagtcatat tgcatatgcc 15120

ttcaactcac ttgctccttt ctgccctttt cataattagt tggcaaaaac ctcaatctta 15180

ggtaaatcaa ctctttgagt acttcatatc tacatctgtg cggctgaaag tgactgaaga 15240

aaaatacaaa tatgataact gccctcactt taaattcatg gtaagaaacc tcaggtttct 15300

tgccctgaat attgtctggc aattgtactc tattttctta gtccattcag tctcccctct 15360

catagatgac ttagtttgta cccttgcttt tctcttcaaa cttccaacac tcttcctcat 15420

actcactctc agttgatgga cctgatttct acttcattgt gagatgcaag cagtcagaag 15480

aacacttaca gactcctgcc accacatgta tgtattgact acatctgtac tcaatagacc 15540

ttgctgctgt ctttgtgtta ctatagattt actctctatg tttctatata gtcccaggtc 15600

ctctgattaa tgcattaatg aactgtgaag ttctttttat gccaaccact ctccaaggtt 15660

ctttatatac agtgtagtat gtaactccta tagatttact ctggacattg ccctagccat 15720

tctcccttct ctctgtatat catcaatttg tctcttctgg ataattccta tcaatataca 15780

agcatactgt tacttttttc atcataaaaa tatcttccct caattgctct ttcttgctct 15840

ctttctgtag aaccccttaa agcattgtct aaatgcactt gtcctaattt ctttctcatg 15900

ttctctctta agcctgttct aatcaggctt tgcccctacc actgtgctaa gattgctttt 15960

gttaatgtct tccaacaaat gacattatta aatctagtag tcagttctgt cattatcttg 16020

ggtgatgatc tacgtttgac aaagttattt tctccttgtc tttgatatac ttgtttaact 16080

tgccttttag gctactacat ttacttggtt ttcctcaaaa ttcattggct gctcctcctt 16140

agtcttcctt agtagatttt cctttttgcc tacctctaaa cattggaata tcccaaggct 16200

cactccttgg ttatcttctc tatctatgct cattccctta gcaatctctg ttagtttggt 16260

ggctttgaat atttttctct caaatgaatt ttttcagccc agatctctct ccgtaactcc 16320

ttatttttat atccagttgc ctatgttgaa tggctcttaa acatctcaca ctcaacatgt 16380

ccaaaatgga attcttgctt ttccctccca aacctgctct ctcataccta ctctgtccac 16440

atagccttcc tcatctcagt tcttggcagc tttccagttg ctcaggccag aaaccttgga 16500

gtcattcttg actcttgctg tcttatcttc ttatccaaca ttcattcctt caggaagttg 16560

ttggctgtaa ctttaaaaca tatccagaat tcaaccagtt ttcactgtct ccacagccac 16620

catcatctct tgctgggtaa tggcagcagc cttttaacag gttttcctgc ttctaccctt 16680

gccctctgta gtctgttatt aacacagcag tcaaaaagat cctttgaaaa tttaagtcat 16740

atggcaagag attttgtact acatttaaga agtttcatga gttgataaga ttagagattg 16800

ttctagccaa tgatatttta ctaaatcatg tcagccttga aggcacattt aaggcaggct 16860

ttgagtatgc ttgttagtgt gggtggggaa ggagtggtca gagatggttc atagcctccc 16920

tcttttctcc tcactaacac ttgtcttttc caccatatgc acgaaaagac atatttgttc 16980

ctgaggaaat tagaaaggga gggcctgagt tggttgctat tcctgaaagt ttctttgaat 17040

ggaatactgg atcatcgttt ttgaagaggt ctgttgttac ttcattatta cctcctttag 17100

cagcaccctt gttttcctcc aacctctgcc aagattagtg tcctgtgtct acatttgccc 17160

cttctgtttc tcatactccc agatggaaat attatatttc ttaggacttt tcgctctatt 17220

tgaattagta ttttctactc gaaacccttc agaaatcctg aaatccattc ttatgctttc 17280

tgtgtgagtt ggttttatgt ttgttaataa tgtttcaaca gaatgatagt ggacatgata 17340

gcaaaaggga aattgatact aatatgtaac gaagaaatcg tatgtgaaat tgtgaagaaa 17400

atgaacgata tttttattga gtactttgaa ggtacttgtc ataaactaac aatgctaatg 17460

atttctagag gagttatata ctacactgta tataaagaac cttggagatt ggttggcata 17520

aaaagaactt cacagttcat ttcttttatc tgtttacatt cttttctcta gaggcaaatg 17580

ttctttaatc agttttcctt tgattctctt tcagagaggc tcatgtagtg ggatcaacaa 17640

tcatatcact gttgtggcat tgtatatatg ttaactattg attttgcatt cttttagatt 17700

ataatattta atctcttggt ctgaaggtag atatattcct gttttctcaa gctgtttctt 17760

agacctttat tgtccagtat ggtacccact agccatatgg gactactgag cacaggaaat 17820

gtggctagtc ggccaggcgc agtggctcat gcctgtaatc ccagcacttt tggaggttaa 17880

ggcgggcaga tcacacgagg tcaggagttc aagactagcc tggccaacat ggagaaaccc 17940

tatctctact gaaaatacaa aaattagctg ggtgtggttg tggacgcctg tagtcccagc 18000

tactcgggag gctgaggcag gagaattgct tgagctgggg aagcggaggt tgcagtgagc 18060

tgagatcacg cccttgcact ccagcctggg ggacagagcg agactgtctc aaaaaaaaaa 18120

aaaaaaaaaa aaaagaaatg tgactagtct gaatcaagat tgtgctaagt gcaaaataca 18180

cactggagtt agaatcctta gtaacaaaat tttataaaat gtgtaaaatt tcttattagt 18240

aattttaaaa tatagattat atgttgagct gatttgttgg gttaaataaa attaatttta 18300

cttgtttctt cctatttttt ctaaatatgg ctagtggaaa atgtaaaatt atatatgtga 18360

tttacgttat atttctcttg gatagtgctg tcttaaactt atatttctct ttggccctaa 18420

aacttctgaa gttgttttta actctgttaa tgatctgtta tatattcact aaaatatgat 18480

tttggtatta tgtattataa aataatttgt ttttttctga aggaacaact tttatgtttt 18540

tatccttctt agtcctaact ttgtcaagtc ttgagttttc tttttccttt ttttaaaaac 18600

tctttgactt ttgccatacc ttacccatgg tagtactaca caagcataac ttattctttc 18660

tctacattgc ttgggagaat tttgaaaagg gaatttgaaa ttatgagtat cagagttgat 18720

cttttatttg ctttccttct taatattagg actattaata ataatacatt ctataattcc 18780

tccatgataa cataatttta aaatttaaaa taaaggcaaa ttttatcagg taaaaaagtg 18840

cattgaaaaa gttacatacg aggaaagaga tttttgtctg ctttaacata aaacccagca 18900

aataaacaaa acacctgcac tgactcattc tactggcaca cattttgtac atttttataa 18960

acttagactc taacagccac tattagcttt ttttcttcaa agatttcagt ttaggctggg 19020

cacagtggct cacgcctgta atcctagcac tttgggaggc cgaggcgggc ggatcacttg 19080

aggtcaggag ttcaagacca gcctggccaa cacggtgaaa ccccttctct actaaaaata 19140

tagaatttag ctgggtgtga tggcatgtgc ctgtagtggt acgattgtct tgcctcagga 19200

ggctgagaat cgtaccattc agtaccagtc gtaccactca ggaggctgag gcaagagaat 19260

cgcttgaacc tgggaggtga aggttgcagt gagttgacat catgccactc actgcactcc 19320

agcctgggta acagagctca aaaaaagact taagtttatg gttttattta tattaatttt 19380

ttttttgaaa aaaatccttg aaaacaagat ttatgtagag tttaaagaat gctttttttt 19440

tctttttttt cttttttgag acggagtctc ggagtctcac tctgtcgccc aggctggagt 19500

gcagtggcgc tatctcagct cagtgcaagc tccgcctccc agtggttctc acgccattct 19560

cctgcctcag cctcccgagt agctgggact acaggtgccc gccaccatgc ccggctaatt 19620

ttgtttttgt atttttagta gagacgaggt ttcaccgtgt tagccagggt ggtctcgatc 19680

tcctgacctc gtgatccgcc tgccttggcc tcccaaagtg ctgggaccac aggcgtaagc 19740

caccgtgcct ggcctctgtg ctcgaaaagt atattttgaa agtattccac aatctaatat 19800

caattctgat ttttttttaa aaagcaatgc atgagataat agcaattcat attgctacta 19860

tcaatgtact aacaaaagtg gctttgttag gcatttggaa ataatttatt tactgaagaa 19920

ctattacttt tattgttctt accgactcga aagagtccat aatacctgct taggtacgga 19980

aaattagacc tggtgtggtg actcacatct gtagtcccag cacattttgg ggccaaggtg 20040

ggaggatcac ttgagcccag gagttccaga caagcctggg aaacatacgg atactgcatc 20100

tctacaaaaa atttacaaaa aaaaattatc tgggtgtggt ggtgtatacc accaccacta 20160

cttgggaggc tgaggtggga ggattgcttg agtctgggag atcaaggctg cagtaagctg 20220

tgatggcatc actgcactct agcctgggtg agaggagacc cctgtctcca aaaataagaa 20280

aagaaaagaa aatcatataa aagcacattt acaggaagag tattaaaaaa aaaaaaagat 20340

tgggccaaca agctaaaact tttttttaaa tggttgtaat tgagaaaaca taggttttat 20400

aaacaaggac aatgagagca aaagatatct agggtagaaa aagtagagat aatacagata 20460

ataggaattt ggggatgtaa gaaaatagca aagtcataaa agttcacatt tttggatgct 20520

taatggaagg ataatcttga cagactgaaa caaaaatttt actgcttgtt aattttctgg 20580

atcttagtac tctttacctt ttttattttt tttatgcttt gaggatagga ctgatttctg 20640

ttggctctct agacctagat ttggattgat tcataaacat ttctttaaaa aaaagtgttt 20700

tgttttgttt ttgtcctgtg acacagcctc aggagactat tcttatgtgc cccaaaagaa 20760

caatttctga gttcatctgt gttgtgtata ttaactcttt gttccttttt attgctgagt 20820

agtattcagt ggtgtgggta taccacagtt tagctattta cccactgaag gacattttgg 20880

ttcttttcag tttctggctc aactaaaagc tgttatgaac atttgtgtac atgttttcat 20940

ataacataag tttttatttc tctcagataa acgcccaaga gcaaaattgc tgggtcatct 21000

ggtaagtatg catttagttt tcaaagaaac tggtaaagta ttttccagta tgtgagtaca 21060

attttgcatt ctcataagca ttaaatgagt ggtccagttt ctttgtgttc tagccagata 21120

gggtgttacc agttttttca ttgtagtcat tccaagagat ccgtagtgat attttattgt 21180

ggtgttttct tgcattccct aatggctaaa gatgttcaac attttttcat atgcttattg 21240

gcctttcgta attccttttc agtgaattgt ctgtttgtgt cttttgtcca ttttcaaatt 21300

ggttatcttt actgttagat tttttttaat ctcttgagac ttttctagat atctcatttt 21360

cttctctttc attttatttt tatttttcta ttaagttatt ggggtacagg tggtatttag 21420

ttaagttctt tagcggtgat ttgtgagatt ttggtgagtg ctttatacag tctggatact 21480

agttctttgt cagatatgtg gtttgctagt attttctccc tggttttaac ttgtcttttc 21540

atcctctttg tagggttatt cacagagtga aagtttttaa ttttgatgag gttcagtata 21600

gcagttcttc cttttgtgga tcgtgctttt ggtgtcaagt gtaacaactc tttacctagt 21660

gccaatgcct gaagattttc tctaagtttt ttcctaaaag ttttatactt ttacgtttta 21720
catttaagtg tgtgataaca tttggatcta gtttttgtat aaagtaagtg gtttaggttg 21780
ataactcttc ccccttcttg tcccgcttcc cttccccctt cccttcccca tccatggaag 21840
tcgcaattgc tctcgcacca tttattggaa aggctgtcct tcttttattg aattgcttgt 21900
gcatcattgc gaaaaatcat ttgggcatat ttgtgtggac acatttctgg gttttctgtt 21960
ctgttctatt agtctatatg tctctttctt catcagtacc acagttttaa ttgctgtagc 22020
ggtatagtta tccttaatat cgggaggcat aattctactt gttttttgtt ttcacaaaac 22080
tgctttaggt ctcctaggac ttttcctttc catataaatt ttagaatatg tttatctata 22140
tctacagaaa caaaaaaccg ccagctgtgg tggctcaagc ctgtaatccc agcactttgg 22200
gaggccgagg caggtggatc atgaggtcaa gagatcaaga ctatcctggc caacatggtg 22260
aaagcccgtc tctataaaaa tacaaaaatt agctgggtgt ggcggcacgc acctgtaatc 22320
ccagctactc ggaagactga ggcaggagaa tcgcttgaat ccgggaggca gaggttgcag 22380
tgagccaaga tcgtgccact gcactccagc ctggtgacag agcgagactc catctcaaaa 22440
aacaaacaaa caaacaaaat ccttcctaag attttgctag gaattgcatt atactataca 22500
tcaatttggg gagtattgat atctttacta tgttatattt tcccgtcaat gaacatagta 22560
agtctcttca gttatttaga tctttgattt tacgttagca ttttgtaatt ttcagcatat 22620
agatcctgca tgtattttgt tagatttata cctaactatt tcattttctt tggagcaatt 22680
gtgaatagca ttgtatttta aatttcagct ttcatgttta ttaatatatg gaaatatgat 22740
tgatttttga tttttgattt tttttttttt tttgagacag agtctcactc tcttgcctag 22800
gctagagtgc agtggcacaa tctcgactca ctgcaacctc cgcttcctgg gttcaagtga 22860
tacttgtgtc tcagcctccc aagtagttgg aattacaggt aacaccacca cacccagcta 22920
atttttgtat ttttagtata aatggggttt caccatgttg gccaggctgg tcgcaaactc 22980
ctggtctcaa gtgatctgcc cgactcagct ctcaaagtgc tgggattaca ggcacaaaat 23040
aagactgatt tttgtatgtt gaccttgcat cttataacct tgctaaactt aattattttg 23100
gggggatttt tgggtagatt tcttgggatt ttctagatag tcatgtcatc tacaaatggg 23160
gacagatttt ttcttctgtt ctagtctgtt tgccttttat tttcttgtgc tggctggaat 23220
ttccataatt atgttggata agattggtga gaacagccaa tcttgtttta tacttgaagt 23280
taggggaaaa tatactattt ttctttttta aattaaatta atttatttat tttgagatga 23340
ggtcccactc tgtcacccag gctagagtgc agtggcaaga tctcggccca ctgctacctt 23400
cgcctcccag ggtcaagtga ttcttctgcc tcagcctctt gagtaactgg tactataggc 23460
gcctgccacc acgtctggct aattttttgt atttttagta gagacggggt tctaccatgt 23520
tggccaggct ggtttcggac tcctgatctc aagtgatccg cctaccttgg cctcccaaat 23580
tggtgctagg cttacaggcg tgggccacca cgcctggcct agctataggt ttttttgtag 23640

atgttctttg ccaagttgag gaagttcttc tctattccca gtttgcttag agtttggttt 23700
tgttttgttt ttgagacggg gtctcactct gtcatccagg ctggagtgca atggtgcagt 23760
ttcggcatac tgcagcctca accttaagtg atcctcctac ctcagccccc cgagtggttg 23820
ggactacagg catgtgacac cttgtccggc taatttttgt attttttgta gagacggggt 23880
ttcctcatgt tttgcaggct ggtctcaact tctcggctga agcaatctgc cctccctgct 23940
ctcccaaagt gctgggatta caggcatgaa ccaccgcacc tggcctgctt ggagtttttt 24000
ttaatatcat gaattttgtc aaatgcttct gtcaatgata tgatcatgtg atttttcttc 24060
tttagccttt tgatatggca gattacatta tttttcaaat gttgaaccag ccttgcatac 24120
ctggaataaa tcccatttgg ttgtggtttg taatttttta atatattgct ttatttttat 24180
tttttttttt gagacaaagt cttaaactgt cactcaggct ggaaggcagt ggcatgatta 24240
tggctcactg tagccttgac ctcctgggat caagcaatct tcccacctca gcctccagag 24300
tagctgggag tacaggcatg tggcagctaa tttttttttt ttttttaaat ttttttgtag 24360
agatggggtt ccactatgtt acccaggttg gtctcaaatg ccagggctca agcagtcctc 24420
ctgccttggc ctcccaaagt actgagaaaa caggcatgag ccactgttca cagactagat 24480
tttatttgct aatattttgt tcaggatttt tcatctactt tttttttttt tttttttttg 24540
atatggagtc tcgctctagt tgcccaggct gaagtgcatt ggcatggtct cagctcactg 24600
caacctccac ttcccaggct caggagattc tcctgcttca gcctcctgag tagctgggat 24660
tacaggcacc cactaccatg cctgtctaat ttttgtattt ttacaaaatt agagacaggg 24720
tttcaccacg ttggccaggc tggtcttgaa ctcctgacct caggtcatct gcctgccttg 24780
gcctcccaaa gtgctgggat tacaggcgtt agccactgtg ctcggccttt catctacttt 24840
tatgagaggt attggtctga agttttcttt tctttctttc ttctctctct cttttttttc 24900
ctttttttgt gatatctttg tctggttttg atacctggct ctctggacca ccggatttct 24960
ttctttcttt ttttgataca gggtctcgct ctgtcagcca ggttagagtg cagtggtatg 25020
atcacagctc attgctccac ctcccaggtc caagcgatcc tttcacttta gcctcctgag 25080
tggctgggat tacagttgtg taccaccaca cctgaccgat ttttaatttt tttttgttta 25140
cagggtctct gcatgttgcc caggctgttt ttggactact gggctcaagc aaccctcctg 25200
ccttggcctc ccaaagtgct gggattacag gcatgagcca ccacactcct aggatttctt 25260
tattgggagc ttttgaacta tgaattcagt ttctttaatt ttttagattg atgcataata 25320
tcatatattt atggagtaca tgtgagtatt atatgcatag aatgtgtaat gatcaagtca 25380
gggcatttga ggtatctatc acctttaata tttattattt gtgttgggaa catttaaaat 25440
tctttcttct agctactttg aaatacacag tactttgttg ctgactatag tcaccagtac 25500
tctgctattg aacattagaa gttatttctt ctatttgact gtatattttg tactcattaa 25560

ccaacttctc tttatcccct ctcacccacc caccctctgc agcctctagt atctataatt 25620

ctatttgcta cttctataag attaactctt ttagtttcta catatgagtg agaacatgca 25680

gtatttgtct tgctgtgcct ggcttatttc acttaatata ataacctcca gctcatctat 25740

gttgctgcaa ataacatgat ttcattcttt gaatgccaaa tccattgtgt atatatacta 25800

cattttcttt atttcatctg ctgatggaca cttaagttgg ttctttgtct tggctgttgt 25860

gaatagtgct gcaataaaca catgagtgca ggtatccctt tgatacactg attcttttct 25920

tttgtataaa tacagtggtg ggattgccag atcatatgat aatgatactt ttagtttttt 25980

gagaattctc catactgttt ttcatagtag ttgtatggat ttacattccc accaacagtg 26040

tttaagagtt ctctttttct ctgcatgctt gccagcatct gttattgtgt gtgtgtccgt 26100

gtgttttaag aaatatccgt tctaactggg gtaagataat atctcattgt ggtttagaat 26160

tgtatttccc tgatgattag tgatgttgaa catttttttc atatacttgt tggccatttt 26220

tatgtctttt aagaaatgtc tattcatgtc ctttgcccac tttttaatgg gattatctgt 26280

gtttttttta atggtttgag tttcttgtat attctggata ttagtctctt gtcacattaa 26340

tagtttgcaa atattttctc tcattcaaca ggttatctct tcattatgta gatattccct 26400

ttgctgtaca aaagcttttc agtttaatat agtcccattt gtctattttt atttttgttg 26460

tctgagcttt tgaggtctta gccataaaaa tctttgctta gaccaatgtc ttgatgtgtt 26520

tttttttttt ccagttttat atttttggtc ttacgtttaa gtctttaatc catcttgact 26580

atttttttaa tatggtgaga gagagagaga tccagtttca tttttttttt tttttttgca 26640

tatagatatc cagttttccc agcacaaatt agcctgtcct ttcttcagtg tatgctcttg 26700

gcacttatat tgaaaattag ttgtctctaa atatgtggaa ttacatgaat taagtggata 26760

tatatgattt aggaattgta tattaagctt atagccagga acaaaatgga aggaacattt 26820

gtattcctaa aagacatttc atgggacaca gatgaatgcc tctgcaatgg ttctaatagg 26880

aatttgaaac tgcaatgagc tatgtcatgc cactgcactc caggctgggc aacagagcaa 26940

gacctcatct cttaaagaaa aaaatacaac atttccagaa tctgcatttt tgacaagact 27000

gtgtgatcaa gtttggtagg cactggtttg aagggaagtt aatcaaatag gactgtatgt 27060

aagttgaatt gtagatgaga gaaattatgg taaggagtca gttcagattc ctttatttcc 27120

tatattagta atccacttga agtggagacc aaagggtata taaaaagaga attcttaaat 27180

aaataagtga gatttaatga ctaggattta gagaatggag gagagtgcta tatcaatgat 27240

tatgatcaag ttttggcact gggcaactga aaacttagtg gaccagtgac aagaaatcaa 27300

aaccctaggt ggcataaaaa gccgtggtgg ttattcttta ctcccagact actagaaatt 27360

attgttaaca gaaaatcagc ataataataa cactggagaa aatcttgaaa aaggaattta 27420

gaaaaactca taatttcaaa atcctaaaat aagcagcatt ttttgcaagt tatcttatta 27480

tgtgaacttt ttttagattg ttagtagttt aaagccagtt tgtactttta atgtttctta 27540

tacttgtttc ttcagaattt aattagtttt aaaggaattt ttctcattat aaaatacata 27600
aactctcaaa ttctttttgt tttgttttgt ttttttgaat gtaagttctc actctgtcac 27660
ccagcctgga gtgcagtggt gcgatcttgg cttactgcag cctccacttc ccaggctcaa 27720
gcaatcctcc tgcctcagcc tctcgagtag ctgggaccac aggtaccacg cccgactaat 27780
ttttgtgttt tttatagaga ccaggtttct ccatgttgcc aggctgatct caaactcctg 27840
aactcaagcc atcctcctgc ctccgcctcc caaagcactg ggattacagg tgtgagccac 27900
cttgcctggg cattttcttt tctttttttt gagtcaaatt ctttaaaaaa atacaattta 27960
taaataataa ctccagtatt tatgttttag attggtgagg attaaaatac ttggttctgg 28020
atgggcgcgg tggctcatgc ctgtaatccc agcactttgg gaggccgagg cgggcggatc 28080
acaaggtcaa gagatcgaga ccatcctagc caacataacg aaaccctgtc tctactaaag 28140
aaatacaaaa attagctggg catggtggtg tgctcctgta gtcccagcta ctcgggaggc 28200
tgatgctgaa gaactgcttg aacctgggaa gtggaggttg cagtgagccg agatcacact 28260
actgcactcc agcctggtga cagagcgaga ctccatctcc aaaaaaaaaa aaaaaaaaaa 28320
aaaaaaattt ggttctaata ggctttggtg agactgtaag attggtgcaa cttttaaaat 28380
gacaatttgg caatgtttgt caaagctaaa aaaatgcaca taccatgctt catctagcaa 28440
tttcatttct aagaatttgt tctacaaata tactggctct atgtcaatta atctgtagac 28500
tgactcatca aatttaccaa ttgtgttggt gtattcagga aaattttttt aaatagatat 28560
ggtcaaaatt acatcacttc atggaaaaat ttttcagttt attctgattt cttttcaatc 28620
cttttaaaag ttgttttatg tatttccccc agcatcttag tatttttagg attattttgt 28680
caacttcaaa gtgcctctac caaccttatt ttttggtgtt tttgactaaa gtttacagct 28740
atttatattg gatgggatga tcataataga ttttgaagat ttttgatgag atttggttga 28800
ctttattgat taattgattg attcagacag agtttcattc tgtcacccag gctgaagtgc 28860
agtggtgcga tctctgctca ctgcaacctc cacctcctgg ggctcaagag atcctcccac 28920
cttagcctcc agagtagcag ggaccacagg cacgtgccac catgcctggc taatatttgt 28980
attttttgta aagacagggt ctccccagtt gcccaggccg gtcttgaact cctgaacaca 29040
agtgatctga gccaccgtgc ccagccctat tcttataata tcatttaaag gactgttcaa 29100
cttgtaagct aagggtcatg gcctggttga tttttatgaa tatattcaat atccagaaaa 29160
acatttcaca accagtaatt attatttcac tataactgta acatacatct atcataaatg 29220
tgtgttgaaa aataggcaac tgcatcagta agaaatgcca atattttggt tcttaattgc 29280
tataaaaatt aagagtgata ccttagttga gacattgggg aataataata aaattaccta 29340
acaactagaa caataaaata tttcattcaa ataagaatgt gaaatatcat catgaaagac 29400
aaattaagaa ttataatgac tagaataatt agcaaatatg ttaattagta tagtaataca 29460

tataaatatg ttttcacaaa ttaatattta taaacagata acaccacatt tgattttaat 29520

aaatacatat ttgtgcatgt taataagcac atatctatta tattaaatat aaacatattt 29580

acacacagaa gtacatatag tgtattcata tatcaacata aaatatatat tggcataaaa 29640

tataaggatt atgtgtctga gtggttcagc tctttgaaat aaatatgtaa atttggtaat 29700

ttccatgaat ttattgttca gtgaatcaag ttttatagaa tgagtttgtt tttgttatac 29760

taatatacag gattatatgt tcaagtgcgt gtgttaccag attggaaaca ggagggtcag 29820

ttgtgtgttt attaaattat ttgtccatct gtatgatgaa atgtattgca gccatgaaga 29880

agaatgaagt agagatgcat ctattaagat ggaagaatat gcagtgtcac gtaaagtgaa 29940

aaagggttca gaacaatgta ctcattcttt tccttttcaa ttaaaagagg gtactacaag 30000

atatattcgt gagtgtatgt ttatgtatat attgggtgtg tctataagaa tacacaggaa 30060

ttgtttacag tggtttttat taggacttta aaagatggaa acaaaagtta atgggtgtcc 30120

ttttattgat tttaattctg atcatccagt gagtaaaagt aaaattaatt tttttttttt 30180

ttgagacaag agtctcactc ggtcacccag gctggagtgc agtggcgcaa tcccagctca 30240

ctgcaacctc tgcctcccag gttgaggtga ttctcctgcc tcagcctcct gagtagctgg 30300

cactatgggc atgtgccacc atgcctggct aatttttgta tttttaacgg agacggggtt 30360

ttgccattgt tggccaggct ggcctcgaac tcctgacctc gagtgatctg cccacctcag 30420

cgtcccaaag tgccaggaaa gcaggcgtga gccacaacat ctggcctaaa attaaatatt 30480

tatagcatat atattcatta tagaatatgt gaaaaatgac aaaatagaag aaaagtcaac 30540

caagataact attggtaaaa gtttgcttta atgtgtttcc tttcagtctt tttgtttatt 30600

tgtttgtttt tgcaacttct gtctccctgg ctcaagctgt cctcccacct cagcctcctg 30660

agtagctggg actacagaca tgtaccatta tgctcagcta attattttgt attttttgta 30720

gagatgggat ttcgctgtat tgcccaggct ggtctggaac tccagagctc aagtgatctg 30780

cccgccttgg cctcccaaag tgctggtatt acaggcatga gccactgtac ccagcctttt 30840

ctttttttaa gagacagaat cttgctctgt tgctcaggct ggagtgcagt ggcacaatca 30900

tagcttactg cagcctcaat ctcctgggcc aagtgatcct ctggctttag cctaccccgt 30960

agctaggact acaagtgcat accactatgc ccagctcctt ttaatctttt tattttgtat 31020

tatgctttta ctttttaaat attagtttat ttaaataact taccataaaa ttagtgtttt 31080

atagttttcg tatactccat aacatacttc ctaaaataag acagggacgt aaaatgaact 31140

tccaagcctc atttttagca gtcagttaat aatttcatta taatgatagg aaggcagtgt 31200

gattgtgtgt attaccatat cagttgtctg ggctgacggc tgatcttgtg ggttgggtag 31260

gtgtcctctt cctttctcat ggttatatat ctgtctctct cccagtttcg ttgaagataa 31320

cagcccctac tggttaggaa aatcttctcc cattggacac ttagattttt ctcaggtttt 31380

tttgatgtca tagatattgt agtgatgtat ctttttacta cattttttga cccagtagac 31440

tgatcctcca aggatgttgt cagtccagtt tagtcagttt taccaaaatt aatgaatttt 31500

catcagagta ttttatatga aaaattacac tattttactg gtcaattttc tacatagcaa 31560

ctttaaactt ttttagactt ttttccctca tttttatttt tccattgttg ttattgagac 31620

agggtcttac tctgtcaccc aggctggagt gcagtggcgt gaacatagct cactgcagcc 31680

ctgacttcct gggctcaagc tgtcctccca cttcagactc ctgagaagct gggactacgg 31740

gtgtgcacca ccatgcgtgg ctgatttttt tgtgtgtgtg taatggcagg gtcttgccat 31800

gttggcctgg ctggtctcaa attactgggc tcaagcagtc ctcctgcctt ggcctcccaa 31860

agtgctggga ttacaggtga gagccacctt gcccgaccca tttttatttt actcaaatta 31920

tttttactgg gattattttc cttttacatt ttagaatgaa catttacttt attgaactga 31980

tttttgttct taattttgta aattgtagct ttgtgtttgt tagttttctt ttgtacttct 32040

accaaattca gtttttttaa ttaaaaattt ttttaaccat ttcactgcac actgagagct 32100

ttcctacgtc aaatattgca cactaaaatt ctgtaatcat caatttttag acagcactgt 32160

agtttaccag gtataatttt agtcatgata tgcttaattt tgttgattga ctctgtcctt 32220

gagtctcaga atatcttcgt ttcttttgtg ttttatatgt ttcttcaccc tgaaagtctg 32280

gtcataatgt tggcctcttg ttctggagcc tttgtgttcc agtgatgaga ttctaaaatg 32340

tagaatttgg cttgtttgtg acttatcctt agctttgata aagttttatt ttgtagattt 32400

catttattgt tctgtcatac agataacacc tttggagtac aggatggcaa tttggatgtc 32460

tcaaatatag atctttacac tgccttaaac caggctctag ctcatttaaa ttgactgtaa 32520

tactgactct gaagtgctag gcaaggttga aacagcgcac atttctttaa attttttgct 32580

taagtaaaat ttctagacat gaacttacta gatcagagga tattaccatc attatggttt 32640

ttgcattaat tttcaaaaag attgaggcaa tttgtaatgt catcagcagt gtataagcat 32700

ttcaatttta atatacttcc tttaaaatta ggtatcagta ttttaaaatg ttttacctaa 32760

tgaactaaga gtaaaatggt cttgtgaatt ttctttcatt tacattttca gttattttgt 32820

atagaaattt aataggaaca agaagtcaga agcagattag agacatgcaa aacttaaact 32880

cctaaccaga gcatttggct gtgtagacta ctatgcactg cactagtgtt ttgaatctaa 32940

acctgtttag tccaggcgcc gtggctcatg tctgtaatcc tagcactttg ggaggccact 33000

gtgggcggat cacttgaggt cagaggttca agacctgctt ggccaacgtg atgaaacccc 33060

atctctacta aaaataagct ggatgtagtg gcacccacct gtagttctag ctacttggga 33120

ggctgaggca ggagaattgc tcgaacccag gagatgaagg ttgcagtgag ctgagatcac 33180

gcctctgcac tccagcctgg gcgacagagc gagattctgt ctcaaaaaaa aaaaaaaaaa 33240

aaaaaaaaat ttgggcacag tggctcatgc ctgtaatccc agcactttgg gaggctgagg 33300

caggtgcatc atgaggtcaa gagttcaaga ccagcctggc caagatggtg aaaccctgtc 33360

tctactaaaa ctacaaaaat tagccgggcc cagtggcagg tacctgtaat ctcagctact 33420
cgggaggctg aggcaggaga atcacttgaa ccagggtggc agaggttgca gtgagctgag 33480
attgcaccat tgcactccag cctgggcaac acagtgagac tctgtctcaa aaaaaaaaaa 33540
aaaccacaaa aagacaaaaa ccagttaaaa aattaagata acttattagc atgatgaaat 33600
agagcatccc tggaaagaaa gccataaaca gtactattct actcttggat gggggagttt 33660
gttcctttgt cttatgtcct tctagaatct acttttatcc tgcaatttga cagtcaccat 33720
gtgtaaatgt ctctttgtca catgtgcact ctcactctcc ctcgctttct ttctccctct 33780
taagactctc ttgctcaatt acccaggctg gagtgcagtg gcatgatctc ggctcactgc 33840
ggcctccttc tcctggcctc aagccatctt ctcaccttag cctcccaagt agctgggact 33900
acaggcgtgc accgtcatgc ctggctgatt tttatatttt ttgtagacac aggattatgc 33960
catgttgccc aggcaggtct caaactcgtg ggctcaaggg atccacctgc ctcgccttcc 34020
aaaatgttgg ggttacaggt gtgagccacc acgccaggcc tatttttaaa tcttttgcca 34080
aattagtaat gctttaataa atttttcaga gaaataaatg gaaaactgtc atgtttatta 34140
gtttttctta ttttttttgt tttatttagc caaaaattat tagggtagct ccactttttc 34200
cttattgctt ttgaatatgg tttttaaatt gtaaataact taagagatgc ttggaaatga 34260
tttaaaataa ggcatgttat tagttcattt attagaaaat aaggaatact tatataggag 34320
tttgaattaa tatttttata tactttccca tttatgacac tttcaggact cttattcaaa 34380
cctccaagcc aacagtttta ttaaaacatt aaaactacat tctaaggtac gtgaatcaat 34440
cttttcttaa tgtcagtaag actataaatt gtattttttt tttttgagac agagtcttgc 34500
tctgtcaccc aggctggagt gtagtggcat gatcataacg cactgcaccc tcggcctccc 34560
ggattcaggc aatcctccca cctcaccctc ccatgtagct gggactgtag gcatatgcca 34620
ccatgcctga ctaatctttt ggttttttta tagagacagg gtctccctgt gtttcccagg 34680
ctggccttta actacagggc tcaagtgatc cacccacttc aacctcccaa agtgctagga 34740
ttgtaggtat gagctactgc aaccagcctt tatttgcatc ttttaaaaga tttatttttt 34800
acttttaacc tttgtttaga attttgtgta attcgtgtgt tgcatattta aacttctttg 34860
ttctgtctct ttaatttcct ttggagccaa tcattatctc ttagggacaa ttcgaaaggg 34920
ttaaattctg tgagatgcta tgacgtactt atggtgagga tcactttata tatatatata 34980
tatatatttt tttttttttt tttttttttt tttgagtcgg tgtctcgctc tgtcgcccgg 35040
gctggagtgc agtggcatga tctctgctca ctgcaagctc cgcgtcccag gttcacgcca 35100
ttctcctgcc tcagccttct gagtagctgg gactacaggt gcccgccacc acgcctggct 35160
aattttttgt atttttagta gagacggggt gtcactgtgt tatccaggat ggtctcgatc 35220
tcctgacctc atgatctgcc cacctcggcc tcccaaagtg ctgggattac aggtgtgagc 35280
caccacgccc agcctgtata tttatatatg tatttttgag atagggtctt actctgtggc 35340

ccacgctgga gtacagtggt gtgatctcag ctcactgcaa cctccacctc ccaggctcaa 35400

gcgctactcc tgcctcagcc ccccaagtag ctggggctac aggcacttgc caccacacct 35460

ggctaatttt tgtattttta gtagagacag ggtttcacca tgttgcccag gcaggtctgg 35520

aactcctgag ctcaagtgat ccgcctgcct tggcctccca aaatgctggg attacaggtg 35580

tgagccaccg cgcctggcca atatatttta tatagtaagg tctccttcag ctagtcagca 35640

tgccttccaa ctctccgcta ggcataggaa cactgctcaa ggtgccaata tattggctag 35700

cggagtgcca agatttacgt gtataacgta agacacttat ggaataatac aagtcattgt 35760

atatacagga gctgtagagt tcagagaacg gaagagtgta tgggtcaata gtttactttt 35820

tattatctcc gaccagctga taaaagacaa taattaaata catacatata tacataaaat 35880

agataattta ctttcaaggc tgctattctg cctataattt tatgtcttga taatcttgct 35940

tcccaaaaca taattttatc agatttcttt aaacttagtc tttaacttga ttcttttatg 36000

catacttatt accattttag ttaggaagat gctactttta ggcatgtgtc acaactcata 36060

ataattgcca aatattctac agtttaatgg tttttttgag gaccctcata ttttataagt 36120

ttataataaa gtgtcttaag tttcttaaaa gtatcaactg ttttcgtttg tttgagtggt 36180

ctctgaatga attggcatgc ttagagactt ttttttttta atcagaatct cttatctctg 36240

tttccatact gtctcccagt cttctcactg aaggtttact atgtaattta gcataactgt 36300

tctgagtata ccagagttta gagttgcaag gtatgttcta aaagactacc ttttctccct 36360

caacccactg ttgggtgctt ccctccaagt cttcaccacc agccgaatag tggaatgaaa 36420

ttacatatag taggcttaac tgtaagggtt ctgcagacat ggaattaagt cgagattttg 36480

ttcatggctt aaaaatctgt tttaagtttg aggtcaatta tatacaatga aatacataat 36540

cttaaataag tgttagatga gttttgacaa atgcatgcac ctgtgtaacc cacatcttta 36600

tcaagatata gaacatttcc atcactccag aagcttctct catactcctt cctactcaat 36660

acctgtctcc cagattcaac ctttgttctg attctgtcac cagagcttag ttgtggctgt 36720

tatggagttt tatataaatg gcataatttt gtgactggct tcttttactg agcataatgc 36780

tttccaggtt tgttcatgtt tcacctatta gcacttcttt tttattgacg agtagtattt 36840

agtattctgt tgaatgagta taccacagtt catttattca tttacatgtt gatggacatt 36900

gaggtttttt tttttttaag acagagtcta gctctgttgc ccactctgga gtgcagtggc 36960

acaatctcag ctcactgcaa ccagctcact gtgcctggcc aggtggacat tgaggttctt 37020

tctgttttgg ctattatgaa taaaactgct tatgaacagg ccaagcatgg tagcttacat 37080

ctgtaaatcc agcactttgg gaggctgagg aggagcatag cttgggccca ggagtttgag 37140

accagcctgt gcaatacagt gacgcttcat ctctacaaaa aaattaagaa actagtgggg 37200

catggggtta tgtgcccgtg ggtcctagct actcaggagg ctgaggtggg aggattgctt 37260

gagcctggga ggtcaaggtt gcagtgagcc ataattgcac cactgcagtt gctgtgcctg 37320

ggtgacaaag tgagaccctg gcctcccccc ccaaaaaaac acaccaggct cggcacggtg 37380

tctcactcct gtaatcccag cactttggga ggctgaggag ggtggatcac ttgaggccag 37440

gagttcgaga ccagcctaac caatgtgacg ataccccagc tctactaaaa atacaaaaaa 37500

ttagccaggc atagtggtgc atgcctctag tcccagctac tcgggaggct caggcaggag 37560

aattgcttaa acctaggagg tggaggttgc aatgggctga gattgctcca ctgcactcca 37620

gcctgggcaa cagatcaaga ctccatctca aaaacaaaca aacaaacaaa aaaacccaca 37680

catcaccacc aacaacaaaa aactgctcat gaacatttat gtataagccc ttttgtggat 37740

gtatgttttc atttttcttg ggttaatata tagaattgga attgctaggc catataggaa 37800

atatatgttt aaaactattt tcccctggac catagtgata gaggggaatg gaggtgagag 37860

gtaagtcaag gctataccat aaatgacctt atagttcata ttaaggaggg ttttatccca 37920

agtataaaaa aagccatgga agtgatgtag gagaataaaa ccatcaaatt acatttttac 37980

acctgactgc tttttgtaga ttgagttaaa ggtgaagctg aaggaggaag atccagtgaa 38040

gaggatatgt cagttagaac tctgttgcaa agaacataaa acttgaccaa actagcttaa 38100

acaaaaagag aatttttgtt tcacataaat gagaaggtcc aggaagcatt gttcattctc 38160

aaatatttaa atactaaata tgtctgattc caaaatttgt atttcatcag ctcttacctc 38220

tcttctgaac tctagatatg ttcactaaag agatccttat gcttagaaaa gtacctgatg 38280

tatatactag atactcaata aataatttgt tgaataaaca aatgttcttg ggcatccctc 38340

agagttatac tgggaatcat acttgaaacc tcctgctctt ccgtttatca tcatcaataa 38400

ttatctatcc tcctgacaga ttatttgtca aacttgttca gttttctcaa tcttcactgc 38460

cattacaccc ctgatttctt tcctaccctt cttgagaacc ttctgtggtc ttcacatgct 38520

gcttctttct ccattacagt ctctaaaggt tggagttact caggccttgg tcccatatcc 38580

tcttctattt ctttgtagcc ttccttggat ggaaggtaaa tattaaaaaa ggaagataat 38640

aagtataata ttaagagtaa ggcataaggg tttgaattga ttctgacata tagtgaacta 38700

catgctaatt tgctgtaaag ggaaagtgga aaacatacca ataggtactc taagcaaaag 38760

tccaggaagg aatagatgtg taaatttttt tttttttttt tttttgagac agagtcttgc 38820

tcgtcaccca ggctagagtg gagtgcagtg gcatgatctt ggttcactgc aacctctgcc 38880

tcctgagttc aagcgattct catgtctcag gctcctgagt agctgggact acaggtgccc 38940

accaccacac ctggctaact tttgtatttt tagtagagac agggttttgc catgttggcc 39000

aggctggtct tgaactcctg acctcaggtc acccacctgc cttggcctcc caaagtgttc 39060

ggactacagg tgtgagccac cacgcccagc tgagatgtgt gaaatttttg gtagagttct 39120

tcatggctta tctgagagtg gggaaatgta ttgttccttt gcttctggaa tctacaaatt 39180

aactattcaa aatatgacct taggcacaca tcaagtgatc tgtaataata ttttatttat 39240

ttatttattt atttttattt cattttattt ttttgagaca gagtctcgct ctgtcgccca 39300

ggctggagtg cagtggtgcc atctttgctc actgcaagct ctgcctcccg ggttcacgcc 39360

attctcctgc ctcagccccc caagtagctg ggactacagg cgcctgccac caagcctggc 39420

taattttttt gtatttttag tagagatggg gtttcaccgt gttagccagg atggtctcaa 39480

tcttctgacc tcgtgagccg cccgtctcga gctcccaaag tgctgggatt acaggcgtga 39540

gccaccacgc ctggccgatc tgtaatgatg ttttattttt gtaaaccaat aaatgacttt 39600

aaaatttgtg aaagaaatga agaagtgtgc ttaaattaaa aagtgtgctt aattaaagca 39660

ccaacttcct tgttttctct ctgttctttc ctttattttt accattcgca cactgtgttt 39720

cctcagatat tcatattttc cagaagtttt ttatcttttt ttaattaaaa aaatttttgg 39780

agacagggtc ttgctctgtc atccaggctg gagtgcagta gcatgatcat agctcattgc 39840

atcctcaaac ccctgggctc gagagccttt tgctccaatc tcttaactag ctggaactac 39900

acatgcacgt caccatgctc agctaattta ttttttattt ttgtggatat ggggtcttgc 39960

tatattgtcc aggctggttt tgaactcctg acctcaagct attttttcat ctcagcctcc 40020

caaagtgccg ggattacagg catgatccac tgtgccaggc cccagaaatt ttatgtcttt 40080

acatcataaa gtgttgacta aagaaggctg actcacagcg atggtgggga aagtagtggc 40140

agcatggaat agacagattt gcacagtagt ttgcatagct atgtttagac cgtagttgat 40200

aaattttgaa atcttgaaga acctgaaatt tgcctgtgta aacagagtga aggtgacaga 40260

gtgcttagta aatttattaa tcattaatat ttcttccttg agcaacatca tcatcatcat 40320

gatcatcata aatgcctagt attatgtctg atacatagta tatgcctaat aggatctttc 40380

gttttaaaaa gagttaaact tgagtttcct ggaaaataca cagatggtga aagataaaag 40440

taaacattac taaaaactga aatgctctgt cctagaaatg gaaatgtaca gttatgcatc 40500

gcttaacaat atggctagtt ttgaaaaatg catcattaga ttattttgtt gttgtgtgaa 40560

tgtcatggag tgtacttaca caaacctaga tggtgcagcc tactgtacac ctaggctatt 40620

tggtatagtc tatgctccta ggctacaaac ctgtatggta tgttgctgta gtgaatactg 40680

aaagcatttg taacacaatg gtatttgtgt attatacata tctaaacatg aaaaaggtat 40740

agtaaaaata tggtataaaa gattaagaaa tggtacacct gtttagggca ttcaccatga 40800

atggagcttg taggactgga agttgctctg gatgagttgg tgagtaagtg ttgagggaat 40860

acgaaggcct aggacattac tgtacactac tgtagacatt gtaaacactg tatagttagg 40920

ctacactaaa tttatattaa aaatttcttt caataattaa ccttagcttg ctgctttaac 40980

atttttatat tataaacttt tttttttttt ttttgagaca gtcttgctgt attgcccagg 41040

ctggatggag tgcagtggtg tgatcttggc tcactgcaac ctctgccttt tgggttcagg 41100

cgattctcat acctcagcct cctgagtagc tgggattaca ggtgcatgcc accacgcctg 41160

gctaattttt gtatttttag tagagacatg ttgttggaca ggttgttctc aaactcctga 41220

tctcacatga tccgcccacc tcagactgcc aaagtgctgg gattacaggc atgagccacc 41280

atacatggtc tatattataa acttcttaat ttaaaaaaaa ttttaacttt tataataaca 41340

cagcttaaaa cacacattgt acatatttac acaaattttt cttcatagtg ttactctgta 41400

agctatgtct attttaattt ttaattttta tttattatta tttgagatgg agtctcgctt 41460

tgttgcccag gctggagtgc agtggtacag tctcaactca ctgcaacctc tgcctcccag 41520

gttcaagcga ttctcctgcc tcagcctcca gagtagctgg gattacaggc acttggccac 41580

cacgcccggc taatttttgt agttttagta gagctggggt ttcaccgtgt tggtcaggct 41640

ggtctcgaac tcctgacctc aagtgatcca cctgcctcgg cctcccaaag tgctgggatt 41700

acaggcatga gccaccacac ctggccttat tttaattaaa aaaaaaaatt tttttttttt 41760

gttaaacaca cacacatgct tacattcgcc cacacctaaa cagggtcagg atcatcacca 41820

ttgtcttcca cctccatatc ttgtcccact gggacatgga actggtcatc tctgataaca 41880

gtggccttct tctggaatat tttcttaagg acccgcctga ggctgctttt ccggttaact 41940

ttttttcata agtagaaagg agtaaactct aaaataatga taaaaagtgt agtgtagtaa 42000

atacataaac cagtagcata gtttatcatc aagtattaca tacttcaggc atccattgga 42060

gatcttggaa tgtattcccc ttggataaga ggggacttgt attgaataat tataaattac 42120

tgatctctgc cataaataca aaaatgtggg cgtgtgttgt gggagtagca gtcagcgtgt 42180

atatacacat gtctggtgtg ggtgaagatt ccaaggggag atccaatcta acagtaatag 42240

aaaaaaatat tccattaaaa tgggtgtgtg cttggatttt tgtacattta tataaatata 42300

aacatgtaaa tgaattaaac agtgaactct gtccttttat ttaaaattaa tttattaaaa 42360

ccagagagca gcagcttagg aaatacacca gatttattaa aaatctactt taatagccca 42420

gctgtcttcc aaattatctg tgataattta gtgtatcaac agcaatctga tgtataaaat 42480

aatagtatgt atacaaaaaa gtactctaat tcagattctc ttccaattca aattagtagc 42540

tttaatctgt gtttcttgta ccttaggaaa aactcatttg tgcaatatct ttagattttt 42600

ctggtcatta ctaaacacta aaagccattt attaacagta aatatcaaaa cattgggctt 42660

tattaaataa cagttttctt gagaatggtg acagacttaa attttagaat tataaaatta 42720

ttttattttt tatttttaaa ttttatttta aaatttaata gtattataga atgttgatta 42780

gcattgtatt ttattcctat tgtaggttat cacagttttt ttaaaaactt ttaggtttgg 42840

gggcacatga taaggtttgt tacataggta aacacatgtc acatcaccca gatattaagc 42900

ccagtaccca acagttatct tttctgctcc tctccctcct tccaccctcc ccacttcccc 42960

ctcaagtaga gccatgtctg ttgtttcctt ttttgtattc ataagttttt tatcatttag 43020

ctcacattta taagtgagaa catgcagtat ttggctgtct gttcctgcat tagtttgcta 43080

agaataatag cctccagctc catccatatt cctgcaaaag acatgatctt gttctttttt 43140

tatggctaca tagtattcca tggtgtatat gtaccacatt ttctttaatc tgtcattgat 43200

gggcatttag gttgattcca cgtgtttgct gttgtgaata gcactgcagt gaacattcgt 43260

gtgcatgtga ctttatggta gaatgattta tattcctcta ggtatatact cagtaatggg 43320

attgctggtt tgaatggtag ttctgctttt ggctctgagg aattgccata ctgctttcca 43380

cagtggttga actaatttac attaccacca gcaatataag tgttcccttt tcttcgtaac 43440

cttgccacca tctgtttttt tttttttttt tttttgactt ttttaataat agccatcctg 43500

attggtgtga gatgatacct cattgtggtt ttgatttgca tttctctaat gatcaatgat 43560

attgagggtt ttttttcata tgcttcttgg tggcatatat gtcttctttg agaagtgtct 43620

gttcttgtcc tttgccctct ttttaatgag attgtttgtt tttctcttgt aaatttgttt 43680

gttccttata aatgctggat attagacctt tgtcagatgc atagtttcca aatattttct 43740

cccattctgt aggttgtctg tttattctgt tgatagtttc ttttgctgtg cagaagctct 43800

taagtttaat tagatcccac ttgtcaattt ttgcttttgt tgcagttgct tttggtgtct 43860

ttgtcatgaa atctttgccg gttcctacat ccaggatggt atttgtctag gttgtcttcc 43920

acggttggtt ggttggttgg tttttttttt ttttttgggt ggggggatga agtctcactg 43980

tcgtccccca ggctggagtg caatggcgcg atctcggctc actgcaacct ccacagcctg 44040

ggttcaagcg attctcctgc ctcagccccc cgaatagccg agattacagg cgcctgccac 44100

aacacccagc tagtttttgt atttttaata gagacagggt ttcagcatgt tggccagact 44160

ggtctcgaac tcctgacctc aggtgatcta ctcgcctcgg cctcccaaag tgctgggatt 44220

acaggcatga gccaccgtgc ccggtcttcc agggttttta tagttttggg ttttacattt 44280

aagtctttaa tccatcttca gttagttttt gtgcatggta taaggtagcg gtccagcatt 44340

gttttacctg acttcaaact atactacaag gctgcagtaa ccaaaacagc atgacactgg 44400

tacaaaaaca gggacataga ccaatggaac agaatagagg gcccagaaat aaggctgcac 44460

atctacggct gtctgttctt tgacaaagct gacaaaaaca agcagtgggg aaaagattgc 44520

ctaaccaata aatggtgctg ggataactgg ctagccatat gcagaagatt gaaggttatc 44580

acattattta atccctttaa aatccgttgc ttaactgcaa gaggtttact gtaatcattt 44640

tacattttta gagttttaga atggggaggg atcttagagg tttatctaat acaacctctt 44700

cattttactg accgaagctc aggtctatag aaacctatga cttgaccaac ttctcaaaac 44760

tcgttatttt aagaatccaa gactgtaacc tagatctgct atacacacct tattttctaa 44820

tgctttctaa tttgaatcta attgaaatac agaatgtgct taatatttgc atgttaagag 44880

gtcttcatta gtaagaaaat cttcagaagc tgtaatggac aaaagtataa ggaagtatta 44940

ggaaggaatc gagtttcctg tgagagtaag ggtctttgtg acatgcggtt gataactctc 45000

ctccagtctt ttattatttc attcctttca tccctttttt tctcctggat ccctcctatc 45060

cccttttctc ttagagtgca gatgggatga ctgtaaagtg gaccttttag agtagaccag 45120

aataaggaga ttggatcgag ggagatgggg accagaactg gtaggttaat tatattttgt 45180

aacttcccaa gctggttcta atgttccagc acagttactg gaaaccaaaa gaactaaaaa 45240

catagatttt ttttttttta gaagcactcc acactctgct gaagttggta atattataaa 45300

agttgtagaa ttctctgtta gtcttccaac ctacacctgt caaatctacc ttctttgttt 45360

tctcttttaa aaattttcct tttttttcac cttttttcct tctgttcaaa cattgtttgc 45420

ttcaagtgat ttttttttat aagggaatag gaaatataaa gtgaatatac tcttaagacg 45480

gatattcttt cttttaataa atctctggac tgtcgcagtt cttatccttt tttaaagaga 45540

agagtttgct gaagtggtta aagatatttt actgaaattt catcttggta aaaaaaagac 45600

agttaatttt taatattatt ttattatatt tgctctgctt atttcctttt tctattttat 45660

tttatttttt tgggacggga tctcgctctg tcgcccaggc tggggtgtag tggtgcgatc 45720

tcggcttact gcagcctcca cctcccaggt tcaagcaatt ctcccacctt agcctcctga 45780

atagccataa ttataggggt gcaccaccac gcctggctaa tttttgcatt tttagtagag 45840

acggggtttt gccatgttgg ccaggctggt cttgaactcc tgacctcaga taatccacct 45900

gcctcggcct cccaaagtgt ttggattaca ggcgtgagcc actgcgcgcg gcctctgctt 45960

gttatttcca aagatttaat aggtaaagca gggacaggct gtgaactgag ataaggacaa 46020

ttcgatgatg atatttattc ctgtagctgt gaggcattat gtagccacag ctgggtgtac 46080

agggtttgtt aggagcagcc tttcagggag tatcttctga atatttgata aaagtagttg 46140

ataagttatg aacattagca aaaccattta ttctaataag aatctttaaa attgcttatt 46200

tcttttttgc acactagact tcataaaaat ttaaattata aattttgatt agtctgtgtg 46260

ggggtgagaa ttaaaagagg atatatgaaa tttatttctt aaatgtagct aagaatcatt 46320

acactagact ttcaaaaaaa aatgtgactg gcacacacca cctaaaattg tggggtaatt 46380

ttctgctttc acagatcttt gtagaatgtg atctaaacca ggtgttggca aactatgccc 46440

tgcaggcccc atcctattca tggactgttt tttgtaaagt ccatgagcta aaaatgcttt 46500

ttacattttt acaggtttgt aaaaacaaaa caaagaatat agaacagaga ttgtatgtgc 46560

ctatgtagcc tgaaatacct acaatctggc ccttgcagaa aaaaatttgt tgaatcctga 46620

agaaagctct acatggtcac atagaaccgt ttcaggcaat tcagaattac aggcgtaagt 46680

tgccttattg tgtactgctt taataagtgc ttcatagata ctgctttttt ttcaaattga 46740

aggtttgtgg caaccctgag tcaagcaagt ctatcagtgg catttttcaa cagcatatac 46800

ttgcttcatg tttcggtgtc acatttgggt aattctcaca atatttcaca ctttttaatg 46860

attattatgt ctgttacggt gatctctggt cagtgatctt tgatgttact attatagctg 46920

ttttgagatg ccgggaactg cacctacgta aaatgatgaa cttaattaat acatgttgta 46980

tgtgttctga ctgctctggt gactggctat tgctctgtct gtctccctct gggtcctcgt 47040

tactccctga aacacaatat tgaaattagg tcattttcta agtgttcaaa tgaaagaaag 47100

aatcacatgt ctctcacttt aaaccaaaag ctagaaatga ttaagttcag tgaagaaggc 47160

atgtcaaaag cctcatgaga taggccaaaa agtagggctt ttgcaccaaa aaattagcca 47220

agttctgaat gcaaaggaaa acttgaggga aattgaaagt gctactccag tgaacacatg 47280

aatgataaga aagcgagaca gccttattgt ggacatggag aaagtttgag tcgtctgcct 47340

agaagatgaa accagtcaca taatttcctt aaaccacaac ctaatccaga gcaaagctct 47400

aactcacatc cagagcaagg ctctctcttc cattgtatga aagctgagag aagtgagaaa 47460

gctacagaag aaaactttgc aactagctga gattggctca tgatgtttaa ggaaggaagc 47520

catttccata acataaaagt gctaggtgaa acaccaagtg ctgatgtaga aactgtagtc 47580

agttgtccgg aagatctagc taagatcatg gatgaagatg gctacactaa acagattttc 47640

aatgtaggtg aaacagcctt ctaggacttt gattactaga aaggagaagt cagtgcctgg 47700

cttcaaggct tcaaagcttc aaaggatagg ctgactttta ttaggggtta atgccgctgg 47760

agacttcaag ttgcagtcag tgctcatgga ctattctgaa aaccacaggg cccttaggaa 47820

ttatgctaaa tctaccctgc ctatgctctg taaatggaac aaccatttta tatgagggac 47880

tttatgatct tctgattttt gtatccatag gcgaggaggt cctggagcaa gtcctccaag 47940

gatactgaca gatgattata tttcatgtta tataccacag cttgtaaaag tacatcctta 48000

gaatagagaa accatttgcc taatagtcaa aagatttctt cagggagaag tataagagaa 48060

aaagatttga aagaggtcta ggatttagac ttaacgatca attgtgaggt ttccttgttg 48120

gtttagatca gagcagaggt tggtaaactg cgtcccactg cagaaatcct tcctgctgct 48180

tgtttttgtg aggcatgaga gaatggtagg aagcctggct gacagcctat ctgtttacag 48240

catggtttag ggaacgttaa gcccgctgtt gagacctact gctcagaaaa aaagattcct 48300

ttcaaactgt ccctgtgcat tgccctggtg acctgagagc tttgatggag atgtacaagg 48360

agattaatgt tgttttcatg cctgctaaca caacatctat tctgcagtcc gtggatccag 48420

gagtaatttt gagtttcacg taatcttatt taagaaatac aatgttcata aggctatagt 48480

tgctgtagat agtgattcct ccaatggatc tgggcaaaat aaattgaaaa ccttctggaa 48540

aggattcatc attctagaag ccattaataa catccctgat tgatgggagg aggtcaaaat 48600

accaacatta gcaggggttt ggaagaagtt gatttcatct ctcatggatg actttgagag 48660

gtttaagact tcatcgtggg aagtaactgc agatatggta gaaatagcaa gatgactaga 48720

attagaagta cagcctgaag atgtgactga attgctgcaa tctcatgata caatttgaac 48780

agataaggag atgcctcata agaatgagca aagaaagtgg tttttagaga tgggaccttc 48840

tcctgaagat gctgtgaata ttgttgaaat gacaacaaag gatttagaat atttaataaa 48900

cttatttgac aaagcagtga cagggttcga gaggattgcc tccaattttg aaagaggttc 48960

tacgtgggta aaatgctatc aaacagtatc acatgctaca gagaaatctt tcgtgaaaga 49020

gagagtcaat ggctgtggca aactttattg ttgtcttatt ttaagaaatt gctacagcta 49080

ctccatcttt tggcaacgac caccctgatc aatcagcagc catcaacatc aaggcaagac 49140

cctccaccag caaaaagact gtgactgtct gaaggctcag atgatcttat cattttttag 49200

cagtaaagta tttttaaatt aaggtgtata cattgttttc tagatataat gctattgcat 49260

atgtgatata ctacagtata gtgtaaacat acctttaata tgcaccacaa aaccaaaaaa 49320

tttgtgtgac ttgctttata gtggtaattg caggtgcctg gaactgaatc tacaatatct 49380

tctgggtatg cctgtgtagg tgaaataaca taaatgagga aaatcaacac acatggagtg 49440

tttgttacaa tgttgtttac agtagcaaaa actttgaaac aaatgttcag taacaggaga 49500

ctggttaaat aaattatagg acatctgtgt agtagaatat aatattcatt gagagtcgta 49560

acctatgatt tacacaaagg agaaatgttg attgcattaa gagtggggga gaccaaattc 49620

cagaactgta tgcagtaaga tcctttacaa aacacacaca tgcacataca tatgctcttg 49680

atatctgtat aggtacagaa agttttatat gctatagagt ccttggtgac ttagatattt 49740

tttctaattg ttgcttttat gtcattgcta attgtattac aatgaagtta aataatttcc 49800

ctttgggtat ggaaaaatta agtctgaata gagttttaag tcacaaagag aacagatatt 49860

ttatgtaaaa tagtattaga agcgtggaaa cgccattgaa acttacaaat ttttggaaaa 49920

attaaaatag aacttatctc aaaatcctat taatttatta ttcttaaagt ttaaataggc 49980

tgagcatgta ataatgatag ttagttgtcc aaagaaaata gtactatttt aaggtatatc 50040

ccttacaatg cgagatactg tgtatcatag agaatgcttc tttccacaaa agcatgcatt 50100

ggtgttactt tcagatacag attttgactt ggccaaatca taaaattaaa cctcaaataa 50160

catttcttcc ttccatttct ctttcccttt gtctccttcc cttcccttcc tttcctttca 50220

ctgtgtatta ttgaagtaca gttagataat actgaccttt tacaactgta tgctccctgt 50280

tgttgccaac atccagtctt ctttcttttc tctctctagt attttgtggt ttaatgtaaa 50340

tgtgttgcac atctcattaa atttattcct tgatatttga tgttttattt gatgctgttt 50400

aagtgttatt acaattttac tttgtttcca ttgcatgttg ctagtatttt gaaatacagt 50460

ggatttttat atattggttt tatagccagt gatcttgctg aattagtagt tatagtagtt 50520

ttctgttatt ttggggtttc tatgtacaaa atcttatcat ctgcaaataa agactagttt 50580

tacttctttt cccaccttac acagtagtcc tcccttatcc atgggtgatg cgttccaaga 50640

cccccagtga ttgcctgaaa ccgcagatag tactgaaccc tttatataca gttggccttc 50700

tgtatctgtg gattcaacta cccatggatg gaaaattttc ggggatgggg tggagacaac 50760

aaaaaatata aataaaaaaa ttatgtcaca gtaactgttt atatagcatt taccgagtta 50820

taagtaatct agacatgatt taaaataaat gggaggattg catatgtaat atgcaaatag 50880

taaaccattt tatataaggg acttgagctt cctctgattt ttgtatccat agaaggtggg 50940

gaggtcctgg aacaagtcct ccaatgatac cgacggatga ttatatttca tattatatac 51000

cacagcttgt aaaagtacat ccttagaata gagagaaacc atttgcctaa tatgtagtca 51060

aaagatttct tcaggaagaa gtataagata aaaagatttg aaagaggtct aggatttaga 51120

cttatgatca gttgtgaggt ttcctcgttg atttagatca gagcagaggt tggtaaactg 51180

catcccactg cagaaatcct tcctgctgct tgtttttgtg aggcatgaga gaatggtaga 51240

aaaaataatt caaaaaaatc agtttgttgt aatatgagaa aattaactga aattcagttt 51300

tcaatgtccg tagatagttt tattggaata cagccacact tactcatttg tgtatagcct 51360

gtgactgcct ttgcatttaa gcagtagagt aattgtaatg tttgaatggc cagtaaagca 51420

taagatattt actgtttagc ccttcaacaa aaatgtttcc taatgtctag attctcttct 51480

gttttcgtca atgttacatt tttaaatggg ggttttactt tctaagatgg cagtggtgta 51540

agtgagtttc catcccacaa ttaagtaaac aaaatattct gttctcagat attaacacgc 51600

ttatatgttt tagtcttttt tgtttgtttg tttttttgag acagtcttgc tctgttgccc 51660

caggctggag tgcagtggtg caatcttggc tcactgcaac ttccacctca caggttcacc 51720

tcgtgcctca gcctctcaaa taattgggac tacaggcatg caccaccatg ccccagctaa 51780

ttttttattt ttagtagaga tagggtttca gcatgttggc caggctggtc ttgagctcct 51840

ggcctcaagt aatctgcccg cctcagcctc caaaagtgct gggattacag gcatgagcca 51900

cagtgcctgg ccttagtctt tttttttttt aaacctgtgg gcttaaactt cctgaagaca 51960

cctgaagtat gtatttattt aaatagacca cttttgttgc aaaataaatt cctttgtcca 52020

tggttggtag acttttattt tgaaattgtt ttttaaatgt gtatagaata tatttatgtg 52080

caatttcaag attagtaaaa ctagcaccca tatatgtatt gaccattatt ttctatattc 52140

aaagcacagc ctttgagttg gttgaattgg ttggcattgc tatatgaatt taaagctttt 52200

tttttcgcca catagttttt ttttttttag gagaacagga ttttgctctc ttcccgaggc 52260

tggagtgcac tggtatgatc ctaggtcact gcagcctgga aatccttggc tcaagtgatc 52320

cttccacctc agcctcccaa gtagctggga ctacagacac acaccactgt gcctggctat 52380

tctttctttc tcttcctgtc ctgtcctgtc cttttttcac acagggcctc gctttgtagt 52440

ccaggctggt ttcctagctt caagtgatcc tcttgcctca gcctcccaaa gtgctgggat 52500

tactagtgtg agccactgtg gctgaccaat ttgttttcca caattgtaat tgacctagtg 52560

ttgtgtatat ttggtccttt acgtttacag aatgttttgt tttgttctat tttattttat 52620

tttgagacgc tgtcactctg tcacacaggc tggagtgaag tggcatgatc tcggctcact 52680

acaacctcct cttcccaggc tcaagtgatt cttgtgcctc agccccctgg gactacaggc 52740

acacgccacc atacccagct cattattgta ctttttgtag agatggagtt tcaccacgtt 52800

gcccaggctg gtctcaagct cctttttgtt tttacttatt tacttttttt attttatttt 52860

tttgagacag gcttttactc ttggctcact gcaacttcca cttcccaggc tcaagtgatt 52920

cttctgcctc accctcctgt gtagctggga ccacaggtgc gtaccaccgc acctggctat 52980

ttttttttta attttttaat taatttttta tttttataga gatagggttt cgccatattg 53040

cccaggctgg tctttaactc ttgagctcaa gcgatccacc tgccttggcc tcccagagtg 53100

ctgagattac aggaatgagt tgctgtgccc ggccttgttt tattcttgaa aacattctga 53160

catatacatt ctctgtgaga ctttctgtag taatactaat ttggttaaca ttttctattg 53220

gaaacaggaa tgaactagtt gtatgtggac cagaggactc ttgactttac tattttagtt 53280

ttatggtgta aaacggttac cattctaggc tttactgagt cataatagaa gtgaggcaca 53340

catggagatt agagaggaac aaatgttaaa acactaaatt ttttcttcct tttaaagaag 53400

aattaatagc tatatttctg tattttagac cccttaaaag tatttattat ttagttttat 53460

tccagatggc tgcatggtag catatctcag aaatcaaata tatatatata tatatatata 53520

tatgtatata tattttttat agagataggg tttcgccata ttgcccaggc tggtcttgaa 53580

ctcctgagct caagcgatcc acctgccttg gcctcccaaa gtgctgggat tataggcatg 53640

agctgctgta ccagctatat atattatata tatatatata tatatataaa atatatatat 53700

ttattatata tatttatata taaaaatatt tttgtatcca tatatatgtt atattttata 53760

tatatataat ataacatata tatataaaac aattccagga aaatactcat acataagctt 53820

gattgtttta tttttgttta aattttcaaa aatcattttc tttcatttaa aaatcattat 53880

ttacccagat acctgttatc tgccctcctt tatgaattct gtcttactgt atgcttcctc 53940

catgtcccct tatgccctac ctagcccaaa acactgaaag gttggacttc catatgaatc 54000

aatgctttcc atgaagtaaa actgcagaag tggtggcagg atattcctga atgaagtttg 54060

agacagatac actggcagag atctcttggt gggcaaggag cataagatta gtagcatgcc 54120

atctggggtt agaatttagg aggtggtaga atgtaagtga ctagctgaca gtttggctaa 54180

agaagtggtt gcatttaacc aggctaataa acatgagctt aaaaaaaaat tcactggcta 54240

agactgggtc ttgcacttca ttttcagttt agtgtttctc cttggattaa ttttttctgt 54300

tttatccatt actagtggta tactgtaaat tgatgtacag aagtatctct aaaaagacta 54360

atttatagga gtccagacgg actagaaggg ttttaagctt ataatacctg gatccttttg 54420

ttttaattag gtttctagat aagtccttac aaacttcaaa ggtgttatac cctatcagat 54480

actttttccc ccaaataaaa ctgtattttt cagttgaaaa taaattgata taggcaattc 54540

cttttcttga caaccttcac aagtgtggta gagaattatt tagcacacta attagtctca 54600

gcttcgattt tgtgcagatt tgattttacc tattaataac actttcatcg ggcttagcct 54660

ttaattttat aaactcctgg cctcctcctg agcattaaat tgataggaaa catactgatg 54720

ctaatctcta attatacatt cagaagtttt ttcatctcat cactctattg tcccttttta 54780

tttgtccgtc atacttaaat tttcaactaa tttaatatag tttttctcat tatacaagta 54840

aacgttcatt tctaaagatt gaaaaataaa aggaagaaaa cgaactacct ctagccctct 54900

actcagtgac agacactgct attttctttt ctttttcttt tcttgagatg gagtttcact 54960

cttgttgccc aggctggaat gcagtgatac gacctcagct cactgcaacc tccgcctccc 55020

aggttcatgt ggttctcctg cctcagcctc tcaagtagct gggattacaa gtgtgtgcca 55080

ccacgcttgg ctaatttttg tatttttagt agaaacaggg tttcgccatg ttggccaggc 55140

tggtctcgaa ctcctgacct caggtgatcc acctgccttg gcctcccaaa gttctgggac 55200

aggcgtgagc caccatgcct gtccctattt tcttacatgt cctttgatac actcttttat 55260

tactattcct cttttagaga caggttctta ctctgtcacc caggctatgg tgtagtgacg 55320

tgatcatagc tcactgcagc cttgaactcc tgggctcaag cgatccttct gcctcagcct 55380

ccgcagtagc tggcactaca ggtgtgctcc tccatgccca gctatttttt gtactgatag 55440

ggtctcgcta tgttgcccag gctggtcttg aactcctagc atcaggtgag cctccctcct 55500

cagcctcaca aagtgctggg attacagctg cgagccacta cacacaacct gaatcactgt 55560

tttatttgtc tctctgccat cctccatata cagttttggg agcatataat acttgcacat 55620

gtctatggta cagtgttgaa taacataggc acagtctttg cactcaaagg attcaaagtt 55680

taatactctt taaaaagttg ccttttaaaa attaattgct attatagaag aaatacataa 55740

atagtctctt gcaaaaacac atagataagg ttcagctttc tgattgctcc cattcccttc 55800

tttattttcc taaaggtagt cactattagc agtttatatg tcatttttcc ccctaagggt 55860

ttgcttatac tatacatatg tagagaacat gtatgatttc ttttttagtg taaaaacagt 55920

tggctgggtg tggtagctca tacctgtaat tccagcactt tgggaggtcg aggcgggcag 55980

atcacttgag tccaggagtt cgagaccacc ctgtccaatg tatgaaaccc catttctact 56040

aaaaatacaa aaattagcca ggtgtggtgg tgcatgcctg taatcccagc tactaggaaa 56100

tctgagggat gagaattgct tgaactcggg aggtggatgc tgcaatgagc caagatcatg 56160

ccatgcattc cagcctgggg tacagaatga gaatctctcg aacaaaaaca agacaaaaca 56220

aaacacaact gttatacagt acctgcaatt agtttctttt tcattcagta tgttagtatg 56280

tacagattta gttctttaaa actgctttat ttaattttac tgatacacag taacttagcc 56340

tttcctattg atagacattg ggagtcccaa ttttttgcta ttgcaaaaca ttcctgcaat 56400

tgcttctact catgcagatg tgcaactgtt tctctaaggc tgttatgcag aggtggggtt 56460

agtagatcat agaggattat gtgattaccc ttgaaatttt accattcatg ttactctttt 56520

tttaccctct ttttctggag ggtcttattt ttcttttttg agtcagtctc actttgtcac 56580

ctaggctgga gtgcagtggc gtgatctcgg ctcactgcaa cctctgcctc ctgggttcaa 56640

gtgattctta atgccttgac ctcccgacca ttcatgttac tctttaagtt ggaccttaga 56700

gcagtttatc ttatatcact cccctcccaa tagacatttt atccttttta aatctaacaa 56760

attagacaga cattgttgtt attttataga gaaatgtgtg attcagttta cgcacatttt 56820

taccacatct ttccttacca tgccttcaat ttctgactgt agctgggcac agcggctcac 56880

gcctgtaatc acagcacttt aggaggctga ggcaggtgga tcacttgagg ccaggagttt 56940

gagaacagcc tattcaacat ggtgaaaccc cgtctgtacc aaaaatacaa aaattagcca 57000

ggcgtggtgg tgtgcacctg cctggtaatc tgggtctaag gattactttt aaccagaata 57060

tcagctactc aggaggctga ggaaggagaa tcgcttgaac ccaggaggcg aaggttgcag 57120

tgagccgaga tcacgccact gcactccagc ctgggtgaca gaggagtccc tgacacaaac 57180

aaacaaacag aaaaaaaaat ttctgactgt cctcctggat tattttcctc cttcttaaaa 57240

aacgtcttca gaagtttcat tagtggaggt cttttagtgg taaattgcca gtttttgttt 57300

atttaatttt taatttagcc tcatttctct ttttgagtca gggtcttact atgttgccca 57360

ggctggcctt gaactcctgg gcttaagcga ttctcctgcc tcagtttccc aagtagctgg 57420

gattacacgt gttggccact gtgcctgact atttttagct gtttgtcatg aaaatttttt 57480

aagcatacat gaaagtagaa caaatgctat gatgaaatcc agtatttcca ttcctcagtt 57540

tcctgttatt gatactttat aatttcatgt ttaaagaggg gtctctttac ctctctgtgc 57600

cttccttcct gctggaatat ccaatcatga agtctgtcaa tactttagaa tgtatctcct 57660

agagaagagg ttaaaaaaaa aaaaacccac aatattctca ttcctaacta aattagcagt 57720

aattccttaa catcatctaa cacctagtcc atgtcaaagt cttttcagtt gtctttgaaa 57780

tgtcttttta taattttact ctcatttttt cagggttgtt atttcctagg tgctcagttg 57840

taggttgaca gtaattttct cttcatatct tgaagataca ttactgcctt ctggcttcct 57900

tgctgttact tgctgttact ctaattattc cattgggtga aggattcttt ctttatggct 57960

tcctttatgg ttcttgttgt ctttagtgtt gtgcaatttt actactttgt atctggatgt 58020

gggcttattg atcacatttg ggtatgttgt actttctggg tctagggatt acttttaacc 58080

aattcttgga aatttgcagc tagtgtctct ttcttttaaa actttgaaaa aggaaatata 58140

cattcagaaa catataaaac aggtacagtt tcgttataaa gtagatgtct ttgtagactc 58200

ttccgaggtc aaaagcagaa tatttcacgt acctcagaat ccccctgccc catacacccc 58260

ttgccatttg aagtcgtctc cctttcctta gaggtagtca ctatcatgac ttttatcacc 58320

tttatgtgca tccctaaaat atagtttcct ttgttgaaat ttatatgacc agcatcgtat 58380

actttgtctt gctgtttttg ctcaaattat gtttgggaga ttcatctgtg ttacctgtag 58440

ttcttcattt ttatacagtc atcattactt aagatagttt ttctttccct ttccctctct 58500

ctcctgtctg tttactactc ccattatgtg tatgttcatg tgcttattga tgtttcatat 58560

ttctctgagg ctttgtattt ttttcactct tttttttcct ctctattctt tggattgcat 58620

catctctatc aatctgtctt caagtttgcc agttcttttt tctgccagtt caaatgtact 58680

tttgagctcc tctagtgatt ttttctagtc agtgttcaac tccagaattt ccatttggtt 58740

ctttttaata atgtctcttt atcgatattg tccatttgct gagatactgt cattatacct 58800

tccttctgca attatggtat cctttagttc tttggacata tttataatgg cattttgaag 58860

ttggttaaat ccatcatctg gttgctcttg caggtagtgt cctttgcctt tatttttctc 58920

tggtatatgg gtccttcctt ccttccttcc ttccctccct ccctccctcc cttttttttt 58980

ctttttttga cagagttgct ctgtcaccca ggctgattct ctgctcaccg caacctctgt 59040

ctcctgggtt caaggagttc tcctgcctca gcctccctag tagctgggat tacaggtgcc 59100

cgccaccacg cctggctaat tttttgtatt tttagtggag atggagtttc accatgttgg 59160

ccaggctggt ctcgaactcc tgaccttgtg atccactcat ctcggcctcc caaagtgctg 59220

ggattacagg cgtgagccac cacactcagc actttcctat ttctttacat ggctcgtaat 59280

tttatgttgg aaactgaaca tcttgtataa cattgcaaca actctgggta ctgatctttc 59340

aaccccctgc tcgtttccag ggcttgttac aattatttgc ttatttgttt agtgacttct 59400

gttgccctgt ttcagtgatt tctgtttccc cccttgcagt gtttaagcct ctggtgttgc 59460

tccttagggg cacagacttg tgtatgacca catggcacct ggcagtgttg ggctgacagt 59520

gtttggcaga gctttcttct gtttcttttt ctgaccacac ccagcttttt atctccactg 59580

atttctggct gactactcta ttgtctttaa caatactcta ggacataaat tgcttcttag 59640

ccagatccat acgactctcg aggtcactgc atgtacaatt tgttcagacc cagaggggcg 59700

catcccagct gtcttattcc ctggttctca cctgcaaact agctggccta cagtttagcc 59760

tgtattttga atctcttcca aaatgccttt cactcaacct tgggtttgat attgctttta 59820

ggcttgaacg tcacactctt gcaaatgaag tcagttccat tgggaagaga ttcccagcta 59880

tctctgggga catgttaaat tatatgagat gctttgtatg ctgcttttga tttacatctt 59940

agaaaagtaa aatacaaata ttttactaca taaatagcaa agtgatagag aggtagaaag 60000

cagccctaca tagttgattt ttttgtataa cagcagttta acctttaatg agagaggctt 60060

ttagaagttg tcaggtagat agatttctaa aacataagta agcttttcag cttcctaaag 60120

gtaatacagt cctgagttct atagtatatg aatattcatg gtagatatac attggtactt 60180

tggcacctaa tttaaaaggg gaaattaagt attagtgctg atatttaaac tttttcactt 60240

agcacacagt tcatagctac tctttagtct gttttttgtc ttcgtgtagt ttatgtaaat 60300

aacatgcttt gtgaaatgtg tctaatacag aaacaatgga caacttaaag tgctgtttct 60360

tatttttgaa ggttgtcatt tataacagac atataacaat ttattctttc ttttacgtat 60420

tcatcaaatg tgtgaatgct tagtatttac tggaagccat gttgtagctg catggtatac 60480

cgtcataaat gtgatgcacc gggtcatttc tgccctcctg aagtttccat tctagtggga 60540

acagactaat tgtgaagtcc tgaagtttcc attctagtgg gaacagacta attataaaaa 60600

tatatagttt ataattttat gtatatattg catgtatggt ttttatggaa actttttttt 60660

tctttttctt tttttaatct ttttcacttt ttgatttcta gctagaacca cctccagaat 60720

ttatggaaac ttttaagatg ctatacaata ccaaagtgtg catgtgtgtg tgtgtctgtg 60780

tgtgtgtgtg tctgtgtgtg tatttcatat ctataaatag ctttcaatta tgtataatta 60840

cagtgtatat ataactttgg ttgtatagta tatacttagg agtattattt ctgtgtaact 60900

gtattcagtg gagttgcact agctacatat ttaagttatt aaaaattcat ctattagatt 60960

ggcaaaaaat taaagggtta attaaatata gtaatcattt caaaatttta taaaagtact 61020

gttatgtact atagttaagt gtatacagca tatttttctt tttttgactt ttttattgtg 61080

gcaaaatata tgtaacatat ttaccatttt aaccattttc aaatgtacag ttcagtggca 61140

ttaagtacat tcacattgtt atttaactat caccactgtc tgtttccaga aattttttca 61200

gcatctcaaa cagaaactct gttaaattaa gtaataactc cccattcttc tcccccagcc 61260

ccctagtaac ctttattcca ctttctgtat ctttgaattt gtctattcta gatacctcat 61320

aaaagtgaga tcaggctggt catggtggct catgcctgta atcccagcac tttgggagcc 61380

tgaggtggga ggatcacttg agcccggagt tcaagaccag cctttgcaac acagggagac 61440

cccatctata tagatattta aaaaattagc tgggtgtggt ggtgcacacc tgtggttcca 61500

gctactcagg tggctgaggt gggaggattt gctttataaa gcccagcatt ttgaggctgc 61560

agtgagctat gatcccacca ctcactgcac tgtagcctgg gtgagagagt gagactgtgt 61620

ctcaaaaaca agaaaccaaa agtgaaaaca taaaatattt gtcctcttgt tactggctta 61680

tttcacttaa agcatagtgt tttcagggtt cattcatgtt gtagcatgta tcagaatttg 61740

attccttttg tgactgaata tagtcttttt ttattattat acaacatttt atttaaaaaa 61800

attattttca tagaatacat tttcacatta gagattccca ttgtgcgaaa ataacaattt 61860

attacttata gttttatatt tgtggacaga ttgttttaga acaagtagaa cacatttgag 61920

aaataaatct aagtttacaa ttggtaatat tttgatacgt ctacaagggg aaacttgccc 61980

ttaaatggaa cttctctata ttcagaagca ctccaagcat ttctccctag gatttagaaa 62040

tttataatgt gagatatcag cacttcctaa ttttaaaatt tccctggtat atgtaaccat 62100

cagtaggtgg tatctactga ctagagaggg aagttttcga aaattaatca ctgtctaatt 62160

ttctacaaag tttttattca tgaattaaga gtatttccct ttgtccatta ttcccagggc 62220

aaatatggaa gtttgatcat atactaatag taataaagct ggattctctt taagagattg 62280

ataaattaaa aggcaaaagc tcatatatca tgtttagcta tactgtgagt cttataagaa 62340

gctgggaggc aaccccatta actcaccaga atacagaact cagtctcaca atttcttaga 62400

tatagttcct ctcaaacctt ttcctcaaag attaaattct gaaaataacc ttgtgattaa 62460

gagaagaagg ctgtccacca acagacttat ctgttatttc ttccttattg tgagcttaat 62520

ggcatgacaa agcagaggca aagaggctta catcagttct tcaaagtatg aagtcaaaaa 62580

ggtcagagct tccacagcat ggcaacagct ttgcagatgc ccacatcgtg atagttgaaa 62640

tagcaaagcc cagcaaaggt taaagctgaa agtgccaaaa gccctgcctt ggcagctttc 62700
tgcgaggcat ccccatgaac atagtcagta acaacttgtc caaggcccca gtgaccatga 62760
agagtgaggg tcgcagccag ggaatagccc atcgcagagc aaggattcaa acaagcagcc 62820
ggaagcagac ccaggagcaa aacactgaaa accttcttgc tagtccagtg gagagatgca 62880
gccttggaac cagaatggtg gctcggtgac aagtgtatgt gctgtactcc acatcattct 62940
gggataggtc ggtcctgaag aaatgctgag atataagcag gtctgaccac tggcgttcac 63000
agcaacagag ctcgacctcc ttgggcactg caaagggcac tcagcctcca gagaaccgcc 63060
atctcgttcc tgaggatgac tgaatatatt ctaatattcc atggtatgga tataccacat 63120
tttgtgtatg cctccattga tggacacttg gattactttc cattttttgg ctattgtcaa 63180
aaatactgtt aggaacactg gtgcacaagt atctgttcaa gtcctttcag ttctttgaat 63240
atacacctag gagtgaaatt gctgggtcat acactaattc taagtttaat aaccaccaaa 63300
ctattttctg tagtgtctgt actaccttac attctcgtgt attattatta ttattattat 63360
tattattatt ttttgaaaca gggtttcact gtctccccca ggctggagtg cagcagtgcc 63420
acatcacggc tcactgcagc ctctccctcc tggctcaggt gatcctccta cctcggcctc 63480
ctgagtagct aagactacag gtgtgcacta ccacacccgg ctcatttttg tagagatggg 63540
gtttcatcat gttgcccagg ctggtctcga acccctgggc tcaaacgatt tgcctgcctc 63600
agcctcccaa agtgctagga ctgtgagtgt gagccattgt gtccagcctc tcatgtattc 63660
ttacacatat gttttgaaca ggaccctaaa cacaaaacca tgtatacagc aacactttat 63720
aagtgataag acactttcaa acataatttt gactgtatga tttttatttt gtgtacttac 63780
tacatggtat gtgagatttt tggtgatgca ggagcattat tacacatgtc acagttacta 63840
gttacagttg atgtagagaa caagaactca agtaataagg taaaaatttg ccatagagtt 63900
taacttttta aaaagtaatg tcgttttatt ctttgggcat ttgcactcca cttccaagta 63960
tacatagctc caacatcagt aatcgtgact tctgtcttac catgttggaa agggcgtgag 64020
ttttgaagtt gggtcctgtt tttgaattgt atctttaaca ctctagcttc agtttcatat 64080
tttataaagg atatagtgtg ttcttaccaa gtcactgtga ggattggatc atgtatatga 64140
aaaggctttt tttttttttt gtgacaggat cttgccctgt tgcccaggct ggagtgcagt 64200
ggtgtgatct aggctcactg aaacctctgc ttcctggact ccagcaatct tcccacccca 64260
gcctcccaag tagctgggac tacaggcatg tgccaccaac ccagctaatt tttttttttt 64320
tttttggtag agatagggtt tcgccatggt gcccaggcag gtcttgaact tttggcctca 64380
agagatccat ctgccttggc ctccaaaagt gctggggtta caggtgtgag ccagcgtgcc 64440
tgcagaattg ttgttgagac agggtttcac tctatcaccc aggctagagt acagtggcgt 64500
gaacatggct cactacagcc gcgacctcct ttggtcaagc aatcctcctg cctcagtctc 64560

ccaagtagct ggaaccacag gcacacaaca ccatacttgg ctaattttaa aaatttttca 64620
tagagacaag gtctccttgt gttgcccagg ctggtcttga actcctggcc tcaagtagtc 64680
ctgctgcctt ggccacccaa ggtgctggga ttacaggtgt aagccactgt ttatggctaa 64740
aaatgaatat tttttttccc tgccaaatta attctaagga ctcctgcacc ccactcagga 64800
aatcattggc ttttcttgcc cttttcattt aaaaagatat ttctatgtct tgtaacattt 64860
taaatcttat tatttgaatg cattgtttta ttttatttta cttatttatt tttgaaacga 64920
agtttttgct cttgttgccc aggctgcagt gcagtggcac aatctcggct cactgcaacc 64980
tccgcctcct gtgttcaagc aattctcctt tttcagcctc ccaaatagct gggattacag 65040
gcatgcacca acacgctcgg ctaatttttt tgtattttta gtagagacgg ggtttcacca 65100
tgttggccag gctggtctcg aactcctgac ctcaggtgat ccgcccgcct tggcctccca 65160
aagtgctggg atttacaggc atgagccacc gcgccgccct gtttagtttt aattttaata 65220
ttagttgtct ggcttattaa gtaaaattat tctctaaatg aaaatttggg caagggtatt 65280
ttggagagtc gatttcaaag cttattttaa aagcttagtt cagaggggta ctataataca 65340
tttaggaaat tgctctgtac ttgaaattcc ctaaaagttt gtgtttcttc attgttcaga 65400
tgagtacatc aaattctttt ggatttctta tccaagatca tatagttact ttgtgaaaga 65460
gatgagtcag tcaactcttt gaccttgtat tgcagtgttc taccacatca gactaccctc 65520
ctcctacctt ttaccattta tgagattact tttggcagct ggcttctgtg gcttctctcc 65580
atggtgctag aggtgcacaa atgtacttac ttttgtatta actatctaaa ggatttcttt 65640
ctcctttgtc ccttttttgt tcctctaaac ctccccctaa actgaaagat acatactctt 65700
gtaaactttt ttaaaattaa aaaacagaaa caatcaaaaa gcaaaacatc agtatatcat 65760
tttttattta tggtaattta ccttctagaa agcttttcta ttcataagcc ccaatgtatt 65820
gcttccttcc caacattcca taaattattt cactaaaact ctaataacct gtagttaaag 65880
aaatgatatc ttagaatcaa aggtattaga agtgtagata gcaccatttg attatttcag 65940
tttacttttc tgttattgga accgatattt gagtctgtgt tttctacagg tacagttttg 66000
ggatgtttaa agttggggga cctattagaa gtcagcttta aactcctaac tgtatatatg 66060
ctgaaaactg atagtggcaa atctgggcat ttcttttttt tttttttaat tttattttga 66120
aataattata gattcacaag aagacactat ttcgttgtat ttttcttcta attattagca 66180
aaagtgagaa gtaatttaat ttttctaaag gaaatgaaaa caattttcct ctttttttaa 66240
agggccattt gaattgtgaa aaaatacatt tttttttgca gcaatttaga taatatggat 66300
ttttctttaa aaaattagta gtctcctacc tccgttttgt cttttgtgaa ataacaacat 66360
tttggtgtct aaacgtccat gtgtttattt tcttagcgaa acaaatatat atgcatacag 66420
ggtttatatg ttttaatgaa agtaattgta tgttcatact tgctgttctt taaaaagtat 66480
aactagaata gacattctga tcaacatacc ttatgttata tcatatttac ttaatgattg 66540

tcctattaat ggacagatgg acagtatact gtttgcagtt ttctactctt tttttttttt 66600
ttgagacgga gtctctctct gtcaccaggc tggagtgcaa tgatctcggc tcgctgccac 66660
ctccgcctcc tgggttcaag cagttctctt gcttcagcct cccaagtagc tgggaataca 66720
ggcatgtgcc accacgccca gctaattttt gtatttttag tagagacggg atttcaccat 66780
gttggccagg atgtcttgct ctcttgacct cgtgattcac ctgcctcggc ctctccaagt 66840
gctgggatta taggcgtgag ccactgcacc cggcctgcag ttttctactc ttatattgct 66900
gtagaacata gccaaacact tggctatgtt ggtgtcacta tttatgtaga ataggttctt 66960
agttaggagg tggtataagg gagttgttaa gagcacagct tatctagggc taaaacttcc 67020
tagaaggaat ccaggctata ctaataagtg tgtaacttaa ggcaaaataa tgtgttccca 67080
tttcctcttt tgaaagaggc gctagatcat tgatcctctc tacttctcta ctttgggtga 67140
aaactaactt ctttgacagg ctgatggggt aaaaaaaaaa aaaaagttac ctggctgtta 67200
actaaagatt gatcattttt tcatgtattt taatcacttg taattcttct gtgaactgct 67260
tatcattttc ttgtctcttt tttttttttt tttaacttgt ctgtgtctta ctgctttgta 67320
ggatttcttt atatattctg gacattagct tttcattata tgtagcaaat gtatctttta 67380
ctctgatgct tacttttaat tttatttatg ttttcttctt tccatgttgc taagttgatt 67440
gtctcctttt atggcttctg aatttttgtt ttgcttatga agaccttcta tatcccaaga 67500
tttttaaaaa aatctgtttt ttttttcttt aacacttcaa taatttattg atcactagat 67560
catttgggat tttttaaaat tgcagtatta gattatatac tattttttat tcaaatggat 67620
agcccagtgt cccatcactg tttataaaat ggtccagcct tcccatcttt agttatacat 67680
tttagcaaat attaaaacct cataaattca tgagtctgtt tctagatttt tctgattttg 67740
agacagggtc tctgtcaccc atgctggagt gtggtggcac agttatagct cactgcagcc 67800
ttgaactcct gggctcaagc gatgctcctg cttcaagcct cccaagtagc tgggactaca 67860
gtgtgcgcca ccatgcccag ctaattttaa aatttgttat agacatgagg gctttgctat 67920
gttgtgctgg tcttgaactc ctggctggag ctgaactcct gccctcaagc agtcctcctg 67980
ccttggcctc tcaaagtgct gggattacag tcatgagctg ctgtacccag ctatatctag 68040
attctttagt ttctttgctc ttttgtctaa ttttatacta ataggaaatt atttaaatta 68100
ctgtagcttt atagtataat ttagtatgtg acagggcaaa ttcccacttt tttccccaga 68160
agcttcaaaa tgattctcat gaatttattc ttgtatgtaa cctttaggat cagtttgtcc 68220
aattatgtaa aaaattacca ccgtgatttt ggtggggatt actatagaat tatagattag 68280
tttgcaggct atcagatttt aacttaaagt agtgatttca agctaacctt ttaatctaac 68340
agtggcttta gcctaagata ttacctaaaa atttaaaact ccctataatc taaaaagtta 68400
tattatttaa cttttcaaaa agtgtttgtg atatgaaggt ctatttcttc ccttctttct 68460

tcctgtttat tataggcttt ttgtagtatt accataggaa tacttttgtt taatacgtgt 68520

ttctcagaag ttttcattag catgttatta cataattagt tgtattgtta aggtctatgg 68580

ttctatggca tccgagtttt tctgagtttt agcctatgtt tctctgtgtc aagagtttga 68640

tgttaacatg taactataga tgattgaagt atcttgtttt cattcgattt gttttataaa 68700

ttgtgacaaa ctttagaata gtgtaatagt cttctagtta tgtggcaata ttactaggtt 68760

ttaggtcaaa gatgagacag ttaagagtgc tgttttacct tatttattcc cctaaaatgt 68820

gttatgatga gtgctatagg ctagtactaa atgttttatc gagaaaaaag ggaagttctt 68880

ttttatttat gttgaatcca aaatattaat gatgccaggc cttttgtttt ggtattcaaa 68940

attttcagaa atccattctg aaagccattt caggttgata acagtagttg ctcagtcagt 69000

atttttcaaa ttttttgaca gtgtgactct taattgttga ctgtgccatt tttccagtag 69060

taaacaatgt attttgtttc ttctcatgca ggtcgtcaaa gtcctacatt ttagctttca 69120

gtgctgaact tccttctccc ttaaattatt ataaactttt gctgctgttt aataaacgtg 69180

aagatgtctc gcagtcctga tgcgaaggaa gaccctgtgg agtgccctct ttgcatggag 69240

cccttggaga tagatgatat caactttttc ccttgcacct gtggctacca gatttgccga 69300

ttttgttggc atcgaattcg cactgatgaa aatgggcttt gtcctgcatg tagaaaggta 69360

aatacttcaa aataacttca cagtgatttg ttttaattgt atagtttttt gctgccatgt 69420

atatgagcaa gtgaatttaa aagactgtgc aaacctcacc tgattatatt attttattat 69480

gtgtattatt ttaagatata aggtggaaag aacactgcac tgggagtcag ggccacgtct 69540

tggctttgtt tactagctgt atgatctttg taaagtcatg taacctctct gattggcctt 69600

tagtttgttg ttgttttatc cgaggagaaa gacatggtca gggatgctta ctgcttaagg 69660

aaattaagaa tagagaggta atggatttgg atagggttgc atgaaattag aaaaacattt 69720

tatattacat gtgggaggaa attcacctta attttctagt acaaaatagg agagaagatg 69780

tttactaaaa agagttaaaa tgacagagaa atcctcgttc agaagataat aaatgctcat 69840

ttcttgagcc cttacagcaa ttttaggctg gtattgatat tcccatttta cactcaagga 69900

aacaggagct tagataactt gtccagagtc atcaacttgt gagtagtagg taggaggttt 69960

ggaattcaaa tgtcttttga gtctatagtc tgttctgaac tattgtatta ttgaaaagcc 70020

cctcctggcc gggcgcggtg gctcacgcct gtaatcccag cactttggga ggctgaggca 70080

ggcggatcac aaggtcagga gatcgagacc atcctggcta acacagtgaa accctgtctc 70140

tactaaaaat acaaaaaatt aggcaggcgt ggtggcgagc acctatagtc ccagctactt 70200

gggaggctga ggcaggagaa tggcgtgaac ccggaaggcg gagcttgcag tgagccgaga 70260

tcgtgccaca gcactccagc ctgggcaaca gagcgagact ccgtctcaaa aaaaagaaaa 70320

gaaaagaaaa gaaaagcccc tcctaactct aaaaatctat tatttttaat attctatggg 70380

ttttattgtt ttgataagca ccagcgacac aattacagta tagtgaatta actactagga 70440

taggtaggac tgccacattt gtattttgaa ggactttatt tgtgacctta gggattaatg 70500

caaatatctg ccactttatt atggaatttg ccttttcttt aatgtttgta taatttaaac 70560

tttattttct aattacccat tttaaagacc tcttgtgttc attactatca aattttgaga 70620

aagctgaaat gtgttttaga tatctaaaat ttaaaaaata atctgtctct tagtttcttt 70680

acctgagaat ttataataaa tctcagaagg caatgcattg tttatttatc attagatttt 70740

taaaattaat ccagtggata tgtatatata ttatatgaat aaaaagttag gtctatttca 70800

atgttaatat agtttataca tttatatagt tacacatata ttgtatatgt aacatatata 70860

atgtgtatat ataatttgta tatatataat atttcatgta gttaatatag tttaagaaat 70920

gtgaatggaa aggtaaatga aaaccctaac attagctttg ggaaaaatct atataaattt 70980

ttgtttatgg attttgaaca tttagttata ttttagaaat tggttcccag tcattccata 71040

agaagacaat catattgaac tatttcatca gaagtaatga ctttcaggat tgcccaagac 71100

tctaaggatt gtaaatttga ttggcttcat gatgtttatc tttttctttt cttttttttt 71160

tccctttggg atgttatgaa gtcaccttaa aaggaccagt tattaaattg tcattacaga 71220

aaaataagtt atttggggaa attacaaaga aagtcatttc aatcttaaga gattacctaa 71280

aattacttca agtccttaga aagtaaaaaa agaaaaaaaa atagaagatt ttaaaggtaa 71340

aaacagtata actttagcaa agcttgatat gaataaaagg gtgtcctggg aataccagaa 71400

agtgtgagga atctctgaaa gacaagaagg acattggaat caatttcaag gaggaagcat 71460

tagatcagct caagagaaaa ctgcttaaaa aaaaaaaagg aatgggcgtg cttcaggatc 71520

ctaggaaaaa tatcagtgta gttggtcaag tatggtgtca gagcagcctt ataggcctac 71580

cccctttttt gtgccatgta gcagagacag atgccctcta attaaaacag tgattataag 71640

tttgaatcag agccttaagg ttaatggaaa cattctggag gaactaggag cttctatacc 71700

tagcagatta cctgtccagc aatatgtctt tgctacagtc acaaaggaga atcttgcagt 71760

aaacagaaat agcatttata ggcagattac caggattctc acatgtaaag gtgtgcaaaa 71820

acaagaatta acacagattt caggaaaacc aacatcatga aagagaagca caaaattcag 71880

taaatagaag atcctaacac aggaaacaaa acagaaagac atttaaaact atcattggta 71940

cctttcggaa aggaaagagg gaatgtcata ttcatgtaga aagattatat ttccttataa 72000

aaagaacaaa ttagagaggt cttgaaaaat cttatagatg gcctgaaaag caaaatggac 72060

acattacaag agcaaattga taaattggaa gcttgtgctg agtgggtttt tattgaagaa 72120

tataatgcaa caagagaaaa aatagaaagc atgaaggaaa agcagcatag atccacaggt 72180

tctaatctct gctgagagca gtctcagagg agtgaactaa aaatgaatag gaagaaataa 72240

tcaaagaact aatagaagaa aattcctctt agattagaag ggccctgtga gtgtgaagca 72300

aggtgactga aaaagatcaa tgtttgttta tataattgtg aaattttaga acatctagaa 72360

tagagaaaac tctaaaagct tgtgagagat aaatatttag aatacaacag aacaagacta 72420

aaattaatac cagtattttc attttacaat tacagaaaat aaactgagaa atttattttc 72480

ccaaattttg agggaacgta actttgaaag gagagctctc aattttatta atatgtaaag 72540

gcataataaa acattttcac acatgcaaag attcatgaac tttattattc atgaaccacc 72600

atacaaacag agagttgagg atgactgcag ggttttgact gaacacctga aagtaaagaa 72660

tggccattaa ctgaaatggg gaagactgca ccaggaatag acatttgaga agatcagaaa 72720

ctcagtttta gacatgttaa gtatgagatt cctattagaa atctaagtgg agatattgag 72780

tagacaggtg aatgaatgtt catgtctgga tttcaggtga ggttctggta tggagataca 72840

gatttcggtg tcagttagca tatagtgtgt tatttataac atcacgagac tggataagat 72900

catgaattga aaagatgaaa gattgtctaa ggataagcac caaaactttc caacagttac 72960

agaaaaaaga aatccagatt acagaggtct aagacaacct ccctttaggg tctccaagtt 73020

acatctttag ctgaggtggt tattgttatt aggccaggaa taattttttg gtttcttgtt 73080

gacttaatca catctcctag aacctcttct tggtctcctg gtcaccagac tttgtagtgc 73140

aggcagcaga gagtcttgga gtctgaatcc tggcctagtc ttgccagaga atctgctcag 73200

cgcagcgcaa gataaatatt tgtgaagttg cctgtgtcgg tcggagtcaa gcaagtcttt 73260

ggtctctgga ggccatctac agtggttgtg ttaacacaga ggaaaaccac aatgttgcta 73320

gaggcctaga gagggtcatg aagctgccat ttacatgtaa cccaaccctg caggcacagg 73380

gtctacagag gccatgctgt gaggtggcat attgagatga tctgaagaaa cttgggagat 73440

gcaagtaggg agctagttga atcaagctcc tgttctcagt ctacaatatg aaacgtgcct 73500

gttttgcaga aagtttctct tagctttaat atctttgaaa agcagagaca gccagatttt 73560

aaaaaccaag agagtttagt gttcccgttt ggtcctagct gctaaacaca ggcacaggct 73620

acttggtttt ttcttagact gctttcatgt gtccttttag tgcagcattt ctgagaaagc 73680

tactagtgga aacagcagat atgggaacat aggtggtgca ctgatgtggc attctgtatt 73740

gattcccatg gaaaccagag actatacaag gaaatgagaa cttcaacaga agtattatta 73800

tttttatttc agaaataata gaagataatg tatctatgaa acaaaaacag gatggtggtt 73860

tggacatggt ggctcatgct tgtaatccca acattttggg aggctgaggt gggaggattg 73920

tttgaggtta ggagtttgag accagcctgg gtaacatggc gaggcctcat ctctaataac 73980

aataagaaaa attagctgag tgtggtggca catgcttgta gtcccagcta ctcgggaggc 74040

taaggtggga ggatcgcttg aacccaggag gttgaagcta cagtgagcca tgttcatgcc 74100

actgcactcc agcctgggtg atagagcaag atactacctc aagaaaaaaa aaaaaaaaaa 74160

aaaaagaaca ggatgatata aagaagaata ttcagtgaac agtaaccatt gtcattgaaa 74220

attaaaatat aatagcaaaa ataactgaag gaaagcattg gaagatgaag ttgagaagat 74280

cttttagtga aacaaaaagc aaaagagatg aaatatttgg gagaagagat taagttcatc 74340

tgaggatcaa tcaaggacgt caaataccaa acaattaagt gctctaggaa gagatcagta 74400
ctctaggacg agatcacagt gatacagagg ggaagtaatc atccaagaat atgatcgaag 74460
aatatttccc agaaccaaag aacacatttt cagattgaaa gggcctatca agtgtccagc 74520
acaaactttt ttatttggaa ataccagaat accaaagaca aagaagttct acaagcttcc 74580
acagaggaaa atagcagatc gcatgtaaaa gacaaggaat ccgaatcgtt tcagatttct 74640
caatagcaag acttcaagct agaagatact tctgaaggaa attaattttc atcttcggat 74700
tccgtatctg ctcaactgtt aagtgtgacg gtacactaaa agacattttc agacctgaga 74760
ggcctcaaaa tttttttacc ttccatgcat tctctcccag gaatctattg gaggatgtgc 74820
cccaccaaca tgaaggaata aagaacagag aatcatggga taaaggaaac aggagatcca 74880
acacaggaga gaagcccctg gggaaaggat gacagctgtg cccaaggtaa agagggcaac 74940
cagtccaaaa tgcaacacat cagagaactg caagagggat ttgtagaaaa agataaaatt 75000
aaaagaatac ttggtgttct aaaagagaaa tgtcttgtga gaccattttg ccagtttgaa 75060
gagatttgat ggttgaatta gtaatgaaca cctaaaaata taatcagaca aaacagatta 75120
actctagggg aaacaaaaag ttgtagagaa atgaaaaagc aatcatatta tgctgcagtg 75180
gctcacatgt gaggtagggg tcatgatata tttttttttc ctatgctgat attcttgttg 75240
cagcatcgtt tgcagaaaag actgtccttt cactcttgat tggttttgaa atctttcctg 75300
aaagtcaatt gcccataaat gtataaatcc aatactagca tctgtattct gtccattgat 75360
gattatatct atccatatgc tcataccata ctgtctttat tattgtaagt tttgaaacct 75420
ggcgtgacaa ggttttcaac tttgttattc tttttagaat ttattttggg tattctaagt 75480
atttagcatt tgcacataaa tttttgaatc agtttgtgag tttcaacaaa aagcctactg 75540
ggattttgat ggagattacc ttaactctac agttagatca ttttgaggaa aaatgacatc 75600
ttaaaaatgt tgactccttt agttcatgaa taaggtatat ctttacatgt tttaaaattt 75660
ctttagcaat gttttatagt tttcagtata cataatagta cagcatgttt tgttatatta 75720
tccatatttt atgtttttga gctatggccc atgatattgt ttcttttttt ttttgagatg 75780
gagtttcact catgtcaccc aggctggagt gcagtggtgt gatctcggct cactgcatcc 75840
tccacctgcc gggttcaagt gattctcctg cctcagcctc tggagtagct gggactacag 75900
gcgtgtgcca ccacacctgg ctaatttttt gtatttttag tagagacagg gtttcaccat 75960
gttagccagg atggtctcaa tctcctgacc tcgtgatcca cccgccttgg cctcccaaag 76020
tgtcaggatt acaagcgtga gccaccgcac ccagcccgat attgtttctg aaatttcact 76080
ttccagttgt ttattgctaa tgtaccactt ttgaatgttg accatctata tcaagaagcc 76140
ttgttaatta aataggttcc ttagaatttt ctacataggt gattatgtgt tctatgcaaa 76200
aagacagttt tactctttcc tttccaatct ctgcttttta aaattttttt agagacaagg 76260

tcttgctctt tcgcccaggc tatgaagtgc agtggcacaa atatggctca ctgcagtcta 76320
gaactcctgg gctcaagcag tacttctgtc tcagccttcc caataggtgg gactataggc 76380
atgtgccacc atgcccaact aattgtttaa aaaatttttt tgtaaaggtc tcgctgtgtt 76440
gcccaggctg gtcttgaact cctggcctca agcatccttc cacctcagtc ccctccaaaa 76500
tgctgggatt ataggcatga gtcactgcac ctggctctgt gtttttaaat tctttttctt 76560
gccttaattc tactgggtaa ctcctctagt gtttaatatt aaaaagaagt gataagaaag 76620
atatctttgt tcccaatctt agcaggaagg cattaagtat tacatcatta agtatgttgc 76680
tgtgtgtgca ttttttggtc tggtctttat caggtcaagg aagttttcct ttattcctaa 76740
tttgctgaga gattttatca tgaatggtgt tgaattttgc caaatacctt ttttgatact 76800
attgagatgg ttatactttt ttctcctttt attctgttga aacagtaatt tatgtttttt 76860
ggtttttttt aaagcgatag ggtcttggtc tctcgcccag ggtggagtac agtggcctca 76920
ttgtagctca ctgcagcttc aacctcctgg gctcaagcaa ttctcctgct tcagcctacc 76980
aagtagctgg gactacaggt gtgcaccacc acacctgaat aattaaaaaa aattttttgt 77040
ctttgcagag actgggctcg ctatgttgcc caggttggtc ttgaactact gccctcaggt 77100
gttcctcctg cctcagcctc ctaaagtgtt gggattatag gcacgaacca ctacgtctgg 77160
cctatgttaa tttttaaatc accttttctt ctcaaagata aaccccactt gattataatg 77220
tattaccctt tttatattca gtttgctaat attttcagaa gatttttttg tctgtgtcat 77280
aaagggtatt tctttgtagt tttatattgt tttcagtttt ggtatcaaag gaatactgtc 77340
cacataaaat gagttgggaa atgtttcctc atcttctgcc ttctgaaaga gtttgcgtgt 77400
cattggtatt ctttttttat ttcttttcta aattcatgat tggcatggac tctactaggt 77460
atcttcgttg gaaagacacc tatgaaaact agctatcaca atattcctgt atgtagacct 77520
ctgggattct ttttggacta actccatctt tgtggtattt tgcccccata aatttcagtt 77580
gtctcagcct cttctaattt ttatcactgt ctgtcccctc cattcattat gactcttgaa 77640
cccagtacca ggatctggta agtactggaa gagcagaaag ctggggtagt tatggtattc 77700
atcggtctta tttctttttt ctcagagatc acagtcctgc tgttcattgc ctgtttcttc 77760
agtgtctgaa aatagttatt tcctgtattt tgtctagttc tgcagttgtt tatgatgata 77820
gggcttgtct gttactttca tggctggaag tggaagagtg taaataacat ttgcatttta 77880
taataatgta atcctgaata ttgccctaac caaagttatg gtagaaatta gaaagtggac 77940
tcgggatgtt tgatgagggg aactgacagg gctaaatctt caagttgcat aatgtgatga 78000
tatctaaaac tgagaaatca agaagtagca atatggatgt attatttaga gatatggtag 78060
taaatgacaa agaatcatct aaaagagtca acatgattat ttctaggaag aggcaactgg 78120
ttgtagagga ggagggaaaa tgggggactg ctctttttcc taacaaaact tgtagaacca 78180
attgactaga agcatattca agaaaaatat caatgaaagg agggaaaaat gatacagtgg 78240

gcaaatggaa atcataggaa agaagatata atagtactga tattagtcat aatgaaattc 78300

aagatcaaac acattttaaa agggcaaaag ttccacaaag tcatgaatct ttacttaaca 78360

gtacaacttt gaagtatatt gagccattat aaacaccagg agaaactgac atccgtattt 78420

ataatggaga gacttcccct tctgtatttt agaaatagat gaagtagaca atgaaaagaa 78480

aatggggaat ttgaataaca gaattagcaa gatgtgtgtg tgtatgtgtg tgtgtataac 78540

tttgtacaga taaatttgtc acaatgttaa atacaagagc agtggtcaaa aaacaggcca 78600

caatggaaaa attagccaat tccaaaaagt aaatctccct tgtgtatttt aaaaatttat 78660

cctccttggg agacttttta aatctgtggg ctggtgaatt tccttagttc aagataaaaa 78720

ttaagctatt atctcttcag atacaacctt ttttctcttt cttctcctct gtgactctga 78780

ttaaatattt ttcagatttt ttttcactgt cttctatgtc tcttaatctc ttttctgtat 78840

gttctttctc cttcatacct tagtaaatga tttattctga cctgtcctca tgtgcgaata 78900

gtttgttttg tatccaatct gccactaaat atgtctgttg agtttttaac tattggtttt 78960

gtgttttcca tacctagaag taatgtttgc tctttttttt ctcttgctgt tattttaaaa 79020

tagattatta taccctgtag gtattttcaa ggttatatat tttttaaaca gataagcatt 79080

attttttaaa aacatctttg tccagtaatt ccagtggctg aagtctgtgg gctatatttc 79140

tgcttattgt tgctcatgtt gttgtatttc tttgtgtttg gttaatgtgg ctgcttcctt 79200

gttattgtca ttgagaaaat tatttgtaag gttccccaag gactagtgtg gatttgctcc 79260

tctctagaga ggcttcaatt tggcttctgc tgggtcctgt aagcattggc attagggctg 79320

ccatgtgctt aaaggttttt ggtctagcca agcaatatat ctccatgctg caactcttcc 79380

ctagggttga tttcaacaat ttctccagga tggttttccc ctacctactt tttctttttt 79440

cttaccttag tctgaccttt ctgtagtttg ttcttaccct gagactatcc tctggtttgt 79500

aatttgttct tatcctgagc ccaccgtttg gaggtgggct gtatctcagc ttatcatgga 79560

acctatcttg ccaaagacta ccatgagctg gtgttgggct ttgaatgttc tttctcctta 79620

gctctttctc tccctacccc atttattgtt aatcagattt tctgtttagg ctctgaattt 79680

ttactttcaa ccaaaaactg gcttgggatt tttccagtat agatttttag gatgttagta 79740

agaagaaaaa aaaaattttt atgttgtatt tttcatggtt tttagtaaaa gggttaatta 79800

gctctccatt actggaaact agaaccctaa aaaaatagta taatcaaatt agatgttaac 79860

agcaaaagga ttttttaaaa gttcatttac ctgtaataca taaattatgg tcttccataa 79920

ctcttggttt aaagagatca aaaagaaaat tacaaattat tctgaatcaa tacttggaac 79980

atgatttctg aatatctggc atgaggccca agtggtatgc agcagaaaag ctaaagctgt 80040

gaatacattt attagaaaac aacaaaaatt agaaaaagaa tgagtactat gacagtggaa 80100

gggatcagaa aaagaaatca gaaaaagaat aagccctggg acagtggaag ggagtgagta 80160

agcagaattt tgtgaaatag gaaaaaaaaa agtttttcaa taaagattag agctggttct 80220

ctgaaaagac caataaaata attaaagtct tagcaagttt aataaaaagt aaagagaaga 80280

cacatgtaaa tagcatttgg aataaaagtg gaaacataac tacacatatg gaagaaatta 80340

agtaattaca ctagaaactg caaaccatgt taactagtaa tttggcagtt tataactatt 80400

aaaaatagtg ttatatctaa atacttctcc ataggcaacc tattttgcat tagtttgata 80460

tgtaatgcac aatagtatgt ttaaagagaa atttctgctt tgtaactttt catttgatgc 80520

tatattccta gaaatttaac atgtgtttta gtcaaaaagt tgatcagata tatttttcct 80580

tttttttttt tttttgagat ggagtttcat tcttgttccc caggctggag tacagtggcg 80640

tgatctccac tcactgcaac ctccatctcc cgggttcaag cgattctcct gcctcagcct 80700

cctgagtagc tgggattaca ggcacctgcc atcacgcctg gctcattttt gtatttttag 80760

tagaaacagg gtttcaccat gttggccagg ctggtctcaa actcctgacc tcaggtgatc 80820

cacacgtctc ggcctcccaa agtactggga ttacaggcat gagccactgc acccagcctg 80880

tatttttctt tctttctttc tttttttttt tttttgagac ggagtcttgc tctgtcaccc 80940

aggctggagt gcagtggcac gatctcggct cactgcaagt tctgcctcct gggttcacgc 81000

cattctcctg ctccagcctt ctgagtagct gggactacag gtgcccacca ccacacccgg 81060

ctaatttttt tttattttta gtagagatgg ggtttcaccg tgttagccag gatggtctcg 81120

atttcctgac cttgtggtcc gcccgccttg gcctcccaaa gtgctgggat tacaggcgtg 81180

agccaccgtg cccagccttt gtatttttct ttatatccat ttttagtgct agtaatatta 81240

gctttgtgag tctcactgtc tgcagttatt cgtgtttggt ttcttcattt cccccgcttt 81300

ttttgaaata gggtcttgcc ttgtcaccca agctggagca cagtgatgcc attgtagttc 81360

attgcagcct cgaactcctg tgctcaagca tccctcctgc ctcagcctcc caagtagctg 81420

tgactgtagg tgcacaccac catgcctgga taattttttt ttatttttgg tagagatggg 81480

ggtctcactg tgttgctcag gctgtcttgc aatcctccta cctcaccctc cctaagtgct 81540

ggaattacag gtgtgagcca ccgcacccag ccctcccatc tttatagtct acctctcatc 81600

cttattttat tgtattagtt tattgatatt ctgaccttgg gtttgtgcag taggtatata 81660

tggtggtgtg tgtagtgaag gaattgtaat ggaggaggaa cttttttgga atacatgctt 81720

ttccaatgtt ttaataaaac ttttggacaa gttgaagtta ataaaccttt tgattatgca 81780

tttgctattt gtggttagca tattttatat ttttcaggtg ttaagattaa acaactagta 81840

tttataactt cttttaaggt actggaataa gatatatttg gagggtaaat tgtagtagaa 81900

tactctgtct catgttcttc tgtgtttgta tcttttgtat tatgtagcag ttatgttcca 81960

acaacgttga ctttgaagcc tgatggaaat atatttccat aggaacaaaa cttggaataa 82020

catagcaaaa agactaagag aacaatataa ctcagtgtta tatgcttaac atgatggtgc 82080

atcagtaata tacttgataa gagttgatat tgcaacctga tttttcttac ttgtcactct 82140

ccctcaattt ttttgtgttc atgtattttc aaatattaca tgactttgcc tcatgtgtaa 82200

tcagtatttt atacaacctc atagaatact aacaaaggaa acagcttgag ggtgtttgtc 82260

acattctgcc agttctcaga aaaatctaag tcccagaaaa gttacatcat tgcttcttac 82320

gccttctgtg atgaagaacc agattttttc ttttctttct tttcccctgt ctgttgagga 82380

ctgatataga gtgctattat gcatggctaa tattttgctt gtgtcatatg ttcgactaca 82440

ttgaaaaata tcttattctg cagagtccac tggtgatgga cctgaatgtt ggggtagtgt 82500

cagttgctct gagagtttct gaacgcttat ctcagttttt gtactttcct tgttatggac 82560

tggtaaaagg cagtgcgtca actatacttt gaaacaaaac taggagcaat ccatgtgttt 82620

gatccattca ggcagtggag catcggtgtt ttagatgtca gtgaagagca acagctttca 82680

aatgaacttg tttgtttttt tttgtttgtt tgtttgtttg tttgtttttt gagaaggagt 82740

ctggctctgt cgcccaggct ggagtgcagt ggcacaatct cagctcactg caacctctgc 82800

ctcccaggtt caagcagttc tcctgcctca gcctcccaag tagctgggat tacaggctca 82860

tgctaccaca cccggctagt ttttaaaaat atttttggta gagacatggt tttaccatgt 82920

tggccaggct ggtcttgaac tcctgacctc gagtgatcta cccacctcag cctcccaaag 82980

tgctggaatt acaggcgtga gccaccgcgc ctggcctcaa atgaacttaa ttgtaaacac 83040

ttcagaagga actaccccag gaattctggg gtagaaaaca gcttttctag gtgcaaatga 83100

aaaatttaag ttatcttata gaaaagtgtt ttaattagtt aatatatagt tcacttttat 83160

atctttttac atttgtgcct ttagagctcc aaatagcttc aagggattaa aatcatccag 83220

gcagatagat taagatttga aggagataat taatgtaggt acagaaaagt aagtggagaa 83280

ggaagttttg agaggaaggc attaagaaaa aacttaaatg tggatgacta cgtaattgat 83340

tgaggaactg aacttacctt aatagatctt taactgtttg gttccttaaa ttaaaatgca 83400

agaaataatt tatgttttaa taaaaaatgt gtgtgtgtgt gtgtgtgtgt gtgtgtgtgt 83460

gtgtgtgtgt gtgtgtgttt tgtttgtttg tttgttttgg tggcttttaa aacacagaaa 83520

tgctgagttt tcaattctgg agagtagctt ttgatgtttg cttggcaaat atttattgag 83580

cttctgaaat catactaaat ttatatttga tcctggagaa agtcctaaaa tcataccaaa 83640

atcaggtaaa ttactaagca agctgctttg tatgccttcc actcacaaat aagttggagt 83700

atgccaaata ggatccacta actcaaagat ccagattctt aatgtgggaa tcaagtggta 83760

tgggtgttta catcctacca aactgttaag gattaagcct gaatcttttt tgggtcacca 83820

gctctaattc attgataaca aggtaaacta tgaagagtat taagtttcct taaaaagcag 83880

taagttaaaa ttgaaagtgg gaataaaagg gaaagaatag ggttcttgga gacctcaggg 83940

tatatactag taaaagttgt gaaatataag gtgaaatctc actttcttaa ttgccgtcag 84000

cgttactctg ggtatgttca caaatttgag aggttttttt tttttttaac gcttatatat 84060

gaagagatgg aggttaactt tactttaaag tcatagcaaa atttcaggcc tgtagctatt 84120

tcttccaccc ttccttgttt ccaccttttt ttttttttgg tttggcatta tttctgttgt 84180

tccccactct tgtattctga taggtgccaa tttccctacc agttgagttt ccatccctgg 84240

aatctcttcc tatccagttt aaaatattat ggcatttaat gtgcccctat gttggtgttg 84300

tgtgtgcacg ggtcttgggg gagggaagaa aagaaaaaat aatgtgccgc atcttccatt 84360

tatcattaac tatttgtttt tagtagtaat aattgacttt tgaatactta tatgtaggac 84420

acagtgttaa tcactttata ggtgaataaa taaagtactg tagtttccat tttacagatg 84480

agacgactga ggtagaaagg tcatatgact ttaccaaagt cacttagata gtaatggctg 84540

agctaggatg cagtgccagc attctgtctc cagagccctc atttggtagc cctggcatta 84600

gtagtaggaa tgaaaatggt atcaaaatgc taagaaagaa aagatcacac aacagtgtga 84660

ttgctagtat tatactgaga gattactatg ttccaggcac agtaataatt gctttaagaa 84720

caaatgtgta acttcattaa tctcctagca catagtaggt tttcattaaa tatttgttaa 84780

ttataatgtg taaaacccac atttagatgc aagaacttac actccttgcc tcttacatag 84840

caccttagag gcttaatctt cccataaact ctgttggaca ctgcagggaa tatcaagatt 84900

ggtttgttgt tgttttgcct ttaaaccgta gatgttttag cactaaaagt ggtctgtgct 84960

aagctccaaa aaatttttta ttagaactat tttatttaac atttgtcttt tattaaataa 85020

aaaaattttt taagcagtta aaacattcta ataaagggag aatgtaacag ataaccacat 85080

acccaccacc aagaatcaga taattactta cgcagttttt ttaattaatt taatgctaac 85140

gtctatgttt gtttcatgct taaagccata tccagaagac ccagcagttt ataaaccact 85200

ctcccaggaa gagctgcaaa ggataaagaa tgagaaaaaa cagaaacaaa atgagagaaa 85260

acagaaaata tcagaaaatc gcaaacattt ggctagtgta cgtgtcgtac aaaaaaacct 85320

cgtctttgtt gtaggtttat ctcagcgcct agcagaccca gaggtaagtc ctcttgtttt 85380

ctttgtcatc ttgatatttt ttgtttcctt gtcttacttg gaaataatat ttgaccctgc 85440

tcaattgtgt gattcctctg aacacattat atcttgaagg agaaaataag aaattacttt 85500

tgaataattg ctaagaaacc aatgaccagt cgtgatctta ggcaagtgtc ttaatctttc 85560

ttggcttcaa tttcttcagt tctaaaacta aggagttgaa ctagatttct aacgtgctga 85620

caatctgtga aaattggata atgattctat tttgctgtta aggctaacat gtgatatgaa 85680

tactctgttt cttttctctt ttctgtgtct gtataagaac caacagtgga ggtgcagttg 85740

tttgaactca ctagagattt tagtaaccta gattttaaaa attctgccca agtagtaatt 85800

cttgattttt cagaactatt actatactgt ctcatcattc ttattcattt taagccagtt 85860

tggaggaaga gggagaattt ggtaatgtga aataaaggac ccaatttctt tgtcattcat 85920

tcgggggagg agagagctta accttgtttc ttagtacctc ttttttctct tcttccacca 85980

aaagccttgt tagagtaatg tcgaactcat actttccttg aggcccttgg atatgcatct 86040

ggtaattttt agtttctgat ataccatgag atactgacta gattgttgag aaggcaaata 86100

taagctgatc gacaggcaga gtgtttactt ggtaggagag agctggtttt gggggatctt 86160

ccaaaagatc cttgagaaga atttaaagta atacttggca tggtaccaat cagaggaaag 86220

attgtagtga acatgcttaa ggagatagga accttgggaa ttggctgagc gtgttaaagg 86280

ccccagaagg aggatcttta aggagtctaa tgtctgtttt tgtaaaaatt aatttaaaaa 86340

atagttaatc tttattgatg atgtgtttat atgtgttgca actatacagg gcttcaaaat 86400

caatacgaaa tgattttgac cccgcgaaag tcagggacat tagtaagaag gatctgtcat 86460

ggatgataaa catggaggca actcaagagt tactatgacc taggtccagg gatgctggca 86520

gtcgttagtt acatgatact caggacatca agttctttcc aaatccaaag gagtggtcta 86580

gagttgatat agaaaaagac ttctttagta agaaatgttc agcaaagagc tcattttagg 86640

tgtatgactc accttctgta gccagttctc cctctttttt ttttaagcta gtggttattc 86700

cccagacaga gagaatcctg cctcaccttt gatcgtttcc ttgaacaaca tctcaacttt 86760

gcactaatcc ttttatctcc atcttttaac tctattaaat agagttaaaa aggtgtgagc 86820

tacctttttt ctaaccacag ggcacttcta tatactcctc taaccaacgg gcacttatgc 86880

cagtatttac acctactaat cttactgatt agaatctact tttttttttt tttttttttg 86940

ggagacatgg tctcactttg tcacccagac tggagtgtag tggtatgatc atggttcact 87000

gcagcctcag cctcctgggc tcaagtgatt cttgtgcctc agccccccag gtaactgggt 87060

ctacaggtgc atatcaccac acctggctag tttttgtatt ttttgcagag atggggtttt 87120

gccacattgc ccaggctggt cttgaactcc tgagctcaag caatctgcct gccttggcct 87180

cccaaagtgc tgggattgca ggtatgagcc actgcgccca acctagaatc tactttcacg 87240

tttctgacgt gcagttccag taaatgaatg gaacttttat cttgatttga gagctcttgg 87300

atatggatgt acctgttttt attaatacat cttggatctg atcaactgta gtactggagg 87360

catatgaaat agcatccaaa atttggatga aattaaggca tagactgaat atgatatttg 87420

gattctaagt ttcctacctc ttttgggtat gtgatttata agagggtttt ttgtttgttt 87480

gtttgttttt tgcccaggct ggagtgcagt gcacaatctc agctcactgc agcctccacc 87540

ccctgggttc aagtgattct cctgtctcca ccacctgagg aactgggatt acaggtgtgc 87600

accaccatgc ccagctaatt tttttttttt ttttgatttt tagtggagat ggggtttcac 87660

catgttgact aggctggtct caaactcctg acctcaagtg atccacctgc ctcggccccc 87720

caaagtgctg ggagtacagg tgtgagccat cgtgtttttt atttacttac agagatttct 87780

tatttccgtt tcaagtaact ctagtaagat taatcttaac tattatattg aaggaaatga 87840

atctaagtag tgactactga aagagagtac tttatcatga gtctaagtat acttaaaaat 87900

atactctact agttttatat gtttgaaata attctttctt ggtgagagga cattggattt 87960

tcttaaataa aataaatggg tgctttcctt tatttctctt gaaagtccag aaagaccaga 88020

gacaagtgtt ttgtttttta ttaaatagta ccacttctaa ataatatctt ttaaaattct 88080

cttaatttgt ctttataacc tttttagtat ttggaaaact tctagtgaag ttggcttcac 88140

tcgaaaactc aaaagaaggt tcaaattgtt agcgtcactt tcaagtcaaa gaaggatcct 88200

tgtaatttac ttatctttgg atgatgaaca atttgatatt tgacctaatt ctgattaaca 88260

gaataggaga gtttaggaac aattaaagct tattttgaaa ataatttttg cataccaaaa 88320

agcttcatac caagattgat tgtcaaatgt attcatttgt tggttgatag aaaaaaaaag 88380

atcttagaag acaaggtaat tagagccaac gatagaggta atgatgatca ccttctaagt 88440

cctaaaattt tagatttgtg aggacctaat tcatttattc tgcaaattaa ttcttgaata 88500

cctactattt gctagatatt ttgatgccag ggggcgggta ggggggaagt agttagcaag 88560

ccagacacaa gctcacagac tggtagggaa gacagaattt taagtgctta catgtgtgct 88620

gagtatttat aaaaggaaat tacacagctt tttgggaaca tatattagaa cacttagcta 88680

gtgtggagat tgacaaaaag cttctctgag gaaaggataa ttagggtcag cttgaagaat 88740

taataggtgt tatctaggta gcacataaag aagagtgccc tgggtatatg tgataaccca 88800

ggaaagtagc ttgtttgatg aactatgact ttaaagacac aacatttcat tttacaaata 88860

agaaactgag gcaaaaggaa gttgagtgac ttgtccgtgg tcttacagtt cctattccag 88920

tgtattctgt gttgattatt ggcatgttgt tcatccatcc attcatttat tcactcattc 88980

atccatctga ttaccaagtt tttgtttttt ctctgtagta tctatgaata cttgactgtt 89040

ttcccaagga atatatagca agatattttg gtactagaca tgcccttgaa catttataat 89100

tctgtcacat ggcctcttat tttctatgtt agtaggaaaa ttggcccact gtgaaggaca 89160

tcgtgatatt gagtatatag ataatgtgct gatgtacagg gtgctctgtc tactctccat 89220

aacctttgtt agttcactag aatactattt tgctagttgt ttaggcagaa tctagagtgt 89280

tcaagttaat atggaaagtt taaattcatt gtttttccct tgcctgggca gcctatttct 89340

tatcaaccgc ttgccatttg ctaatacttt gaggcccact attgttacaa atccgtgtaa 89400

ttctttttgt ttaatgctaa gatagttcag tttttcagta agcatttatt gagtacctgc 89460

aatatgccag gcattattta agacactgag catatactga taaacaagta gtagtagata 89520

aataagtagt accttccttt ttacgaaagt aacaatatga gaagtgttgt gtgctgtaaa 89580

ggcacaaggg atagcattcc accttgggag ggaggtacgt acccctctgc tgtctttggg 89640

gtaaagtgct tctggcacag caactataca aatgcttctg gatgagaaag acctcaatct 89700

gcaagtgatt tgtttggctg gaagattaga ttgcagtggt gagaaagaag gtgccaaggt 89760

aagtggtaat ggatcatgaa gggctttcta accacgctaa ggagtttgca ctttatccta 89820

aaggcagtgg aaaacttttg aatgttctta agaacagagt gacacggtta gatttgtgct 89880

ttgacagtac cattctggct gcagtgtgaa catgtcatca taattacata ccaatctcat 89940

gttgttataa aggtgttaca cccaaacgtt ggccagaaaa catttccata aaccagtggc 90000
cttcaacctt atccgactca gtgtccactt tttaaaaacg aatgtaacac cccttgactt 90060
tcatgaaatg acttcttgat aatataattt gtctacacac ataatttgaa aaagtatata 90120
taaacaagga ataaaaggaa agtaattata aaaagactat ttgtacatat aaatgcttgt 90180
acattcctac tctagaacct tgctattcaa ggtgtggcct ctgggcccat aacattaata 90240
tcactggaga gcttattagg caaactcttc agccctgcct cagatctgct ggatctgaat 90300
ctgcatttta actagatcat tgaagaattt ctttacatat tgaggctcta aagtactatt 90360
ttatcctatg taataaagaa gtaagatact tgcacctatt tgtaaaatca tggactgtga 90420
caactacaaa tgcagattgt tactggtgta tgaattggca actcaggtac catgaaacat 90480
caataatgtg atttttctca gataactctt agtaaaattt taaacaaagc tgggaaaata 90540
ctttgtaatt acgtataata tgaaatttgt ttctggctca ataaacagct tcaggcatgt 90600
cttgcagggc attcagaaat tgtgcgggac aattctttgt tgtacggggc tagcctgagc 90660
cttgaaagac atctctggtc cccatccact gaacaccagt agactctccc agtcacttta 90720
acagtctctc ccaataaaag tttccacaaa tttccagact gccccttgag tgcttccctc 90780
attgagaact actgccttaa acctatgatg ctgatgcagg acaacctgtg accggcctga 90840
ctgaaccctt tggagcaggt atcttagcta tgacgctggt tgctgagttg gtatgcatcc 90900
agctggaata ttgaatgcag ttccaaactc cactagccag aaagcttttt caaactctgc 90960
tcttctctct ttatcaaccc tgagactttg ctacggatga cttaagggtc attgtgttag 91020
ccactgttac tatcatatcc ctcaggggtt ttgggtggaa aagagattga aactcagtgt 91080
aattacctca ccttctaatc cattagaatt ggctacagca aacataacag ttaccttata 91140
cttacaagca cctaaaataa aatataagat taattacttt ctattaatta ggtagaagac 91200
taagaattaa tgatacaccc gccgtagatt tattcatgaa gctttgattc tatcaaatgc 91260
agaacataat agctaaatta taagtctgta agaatattat caaatatgaa tgggaagaaa 91320
tactttacac acacttgaat agttacatgc agaaagcttt catttgcaga gagcttgcta 91380
tttggagaga ttgtaggaac agtaggtcta aagatacatt ctaggaaaac agaattttga 91440
ggtagttgca aattatgtga gatgtctttt tatcaagtta cataccggtc aaattagcca 91500
aggttaagtt tgtaagagaa gctgaaagaa tgcatatagt gttcacaaag taaaggagat 91560
gaggtacgta ggtaccacca agaagtaacc aggtaataaa gaaagaaacg tgtaaatttg 91620
tttccgaaat gttacgtacc aggaaaagta ggggaaaaag cctcatgaga cagatgagaa 91680
atggtaaaac tgattgacat aggaaactgt atgcacaatg ataaatcata aagcatagag 91740
ggttgagtgg gcactctgtt acttgaaatt tgtgatttat aaacagtgtt atatacaata 91800
gatttatgag taaagactgt ctttatgtct gttctttcaa ttctgtcttt acctttaata 91860

cataattttg gtcattgttt ttgaagaatt gctttcgtgt aaggatcatc tcttaatggc 91920

aatgtagtta agatctttga tctgttttgt gtttaagaat actgtacagg taagatccca 91980

tagactattg gagcagttat tagaacatag tagataatac caaatttagt gagtctcagt 92040

ttctattcag tgggtcaaga aaaaaaatag cttttaaact cttttgattt cctataggtt 92100

agctacacaa actgcaatta ggaggtagtg acatttatta gaaaacttta aaaattatct 92160

ttttcctttt gctgaagagg gatgatgaat gtctccatta taaggataaa gttgtttaaa 92220

atggggacag tttatacctt atttcttctt cttttatagg ttttaaaacg accagaatat 92280

tttgggaagt ttggtaaaat acataaagtt gtcatcaata atagcacatc atatgcaggc 92340

tcacaggtaa ctaattttag ggatttttgc ttttcctccc tatggtctgg gcttgacctg 92400

ggctgttcca aaggaaactt tgctctctct gattagaaag cttgagggac aaggacttaa 92460

gcaatgtagt gacctctgtt ggtggggtgg gggatttttt gctgcttatt tgacccttct 92520

aggacttaac aattaagagg gaaatagtaa aacaaacatt tagtgatgga gtgaaaggga 92580

gggggaggtg agtctgttat caggcaatgt gataaataga taaggattcg atagtgattt 92640

ctaatctgta atattttcag atattgaaaa cttaatagtg tctttgttat gctgctgtta 92700

tcagatagaa tagttttcca gattgcattt atttttgaga atgtaatttg acatagtctt 92760

taagtcttat tttttaatct aaattttaat ttttttataa ccttgacgag cctgtcaaat 92820

gactgtctaa aaaaataaat cccaagccaa agtaatattt tattcacata tattacagta 92880

ttcttaaaat cagataaaat tcattgagta ttattacttt caaatgagct agtttagtgt 92940

ttaatcaaaa ctgccactac tactactaat tattgtcatt agtaatagta gtagtcaggg 93000

tctttagaat tccctaggtt gcagtagaat ttttttagta ttgtttaatt tttaactaat 93060

aacagtggaa tgggatgttt tcactttagg gtccaagtgc cagtgcttat gtaacctata 93120

tccggtcaga agacgctctc agagccatac agtgtgtcaa caatgtggta gtagatggca 93180

gaacacttaa ggtaatatag tcctcttgat tctttttttt ttttttaagg aagacgagat 93240

aaaatatagt aagtagagga gaactgagca tttatatatt aaatttgaca gttaatttga 93300

agagtctgtc ttagggtcca taccatctat tatagctaat ttgtttactg ctggtttctg 93360

tagggtcctc ttttaccaga taggaataaa cacattcatt atcggggata atcttttaaa 93420

atatcgcatt tttacatata tcttattttg tgaaaatagt accattattt tcttcagaaa 93480

taggtagtcc ttaaatatat ggacattgat tccagtttca gtgaagagtc ccactaagca 93540

aagctttact gttagtggaa ggaggatggc ataattagaa aaaggaattt gccttcttat 93600

gtgtctaacc actcataaca tggaacagat ttttttgtct atctcctctc cttatatcta 93660

gggaagattt gggtgtccaa gttttggaaa ccatagacag atgtttcctt tagttcttcc 93720

aaatgagcct catctataat aaatcctgct aataagagtt tagttctcta gtatttgcag 93780

ccatactttt tgttttgtca tgtttgtgcc tcattttagg tgacttgaaa acactaaaca 93840

tggagattct cattgtgttg tcagttcaca ttgagtcttc ttttccttta cccctcttcc 93900

tcctgcaggc atctctaggt acaacaaaat actgcagtta cttcttaaag aatatgcagt 93960

gtccaaaacc tgactgcatg tatcttcatg aattggggga tgaggcggcc agcttcacaa 94020

aagaggaaat gcaggtagta aagtcatgtg aattatcaac tttgcagaaa agggagtact 94080

ttttttttta ttagttaaca ttgcaaaagc aaaaactaag gatttgattt tgtaaaaact 94140

gatattactt taaagtagca tttgccaaat gaagtgttta atggaaggtc agttctgcag 94200

gatattgtta caataaaaat gtgtctgtgg tcaactaagt ttgaaatgct agattaatga 94260

agatgaacag gtttctttgc tttaagactt ttcagagtcc ttaatatgct aatacagatc 94320

cacgaaggga atttacaacc agaacttagt tgaccaggaa aacactttgg gaaatgctat 94380

tttaagggga atgtcaacaa aatgaaagct aagtgggata ttgtaattat ttgttaaacc 94440

aatctatact ctttacagag cttgctagaa aataatactc tgtcaaatga tgctgataaa 94500

ctaatattat ttttacaaaa ttattctccc catgcaagtg tccactgagt cagtatcctg 94560

taaagggtga attgaaggct taatatccta tgttcttttt tacatttcct cttctcctgt 94620

ctcctttcaa tttgtagtat aaacatgcct taacaagtaa aaatgttgac catacgacat 94680

attaaatgag gattatacca aagcacaatg ctgcacttcc ctattgtaga tgaggacttc 94740

agattgttgc tgtggacttt tcaggaatac ttctcattta gaaggtgggg ggcattcatt 94800

gctacctttc attctccagg tcaaactaga gaaagtctgg taggggctcc tttaagaaaa 94860

gattgttaaa tggagtatta ccttttttga cttggagcta gtacagatta aatcccatta 94920

taataaatac atcagagttt tcttgatagt atggtcttta tgattcaaaa acaatgttaa 94980

aataaggtct atctgatatt taataaggac agatgactta actgatttct taatgctttt 95040

tttgtttgtt ttttggatga aaccttcttg aatgaaatct aaggtagaac catttcaaaa 95100

tttcaaacta aattttaatt agtagagggt atactttatt catatttcct atcaatttct 95160

gtctatcaga atttacaagt cccacccagt tgatctcatt taaagtaata ttaaacaata 95220

aaataaaatg tatgggctgt cctttggaac ttgtttgtaa tgggatttaa cctgtcttta 95280

tgaactgccc ctgtagagtc attttaatat caagttgagc tctttgtggc tttccacaga 95340

tattagtgaa agccgtatct cctgaagtat tgattccaga aataattttc aagacttttc 95400

atgttttaaa aatgtaaatt tcacatctaa tgtattttta aaaataattt aaaactaatt 95460

tcataatgtt gtcctgaatt tatcttgctc ttctttagct gagtttaaaa ttactttaaa 95520

aggggatttt ttgttgttgt tgttaaaaca ctttcttgat gtaggaaaga aatttgaata 95580

aattttatgg caaaaatcag aaaatctttc cttgttactg cgtaactcaa aatttagtgc 95640

cataggaaat atttataggt ccaatagaaa caagcaagtg gcctctcaga acagggaacg 95700

atgcattgtg cagtatatat gacacaatta tgctcagtta agtgcaattg ccctcacttg 95760

ttattgtttt gatttgttcc gttttggttt tgttgtataa catctattta gaccataatt 95820

taaaaaaatt aacatccctg aaggtaagta aaactattct tccagtctag atatacatgc 95880

aaatagtaaa atgttactca aagtaagttc attatcattt atttttgtgt ctaattagct 95940

tccacaggca atctttgcct aatttttccc ataatatttg tgacattgcc ttttgatgta 96000

cacctaccca gctagagtaa gcatagcatt cttttgtgta tagagcagag gttggcaaac 96060

tttttctgta aaggccagtt agtaaatatt ttagggtttc tgagctatgt atgtagtctt 96120

ctgtcacaac tactttactc tgcctctgtt gcactactac atcctgcctt tgtagcagaa 96180

tggcagccaa atacaatatg taaatgaatg ggcatgacca tgttccagta aaactttatt 96240

tataaaaaca ggcaactggc tgtaaattgc tgtccctggg tctagagttt aaaatacatt 96300

aatttttatt ttacttttga taaccataga agaaaagaga aagcaaaaac taattcatgg 96360

aagttttact tagtgtcaag ataatgaaga atatagtaaa actgttcatc tatgtattac 96420

ttcaagtgct aatctccaaa ttaaaaagga gactgatgaa ttttggctga ttttcagaaa 96480

actcagtgtt acaagactta attcttacat gaaagacttg gaagtaggtt taaagtggac 96540

cttacacttt ttgagctagc aaaaatgttc ttcccttaat atgcttttct gtacaataaa 96600

tgggttgatg aaatgcctta atttaattta tcagatatat atcatataat tttgttggaa 96660

tgactttcat tgatctagaa gttttgtgat atcctcacag gtttctctta cttgttttat 96720

ttaaaaattt atttaaaatt tatttaatat tttatttttt aaaaatatta tttattttat 96780

ttaaaatata tttaataatt cagaagatta ttcagtattt tataccagcc attttgtgaa 96840

tcccactgca gttttaattt cttctttcgt taggcgggta aacaccaaga atatgaacag 96900

aagctacttc aagaattata taaattaaat cccaattttc ttcagctatc tacgggttca 96960

gttgataaaa ataagaacaa agtgacacca ctgcagaggt acgatacgta agtattgata 97020

ttgatagtca gacatcttac cttcttatca gttgatcttc tcttttcatt ttagatcttt 97080

ttcaggcaaa atccttgttg acaaggtctg tcatttatgc agaattgttt agctgtatgg 97140

gaaatgttga gtcacaattt ctgggtttga atgcttgctg cctacttttc cctggtaaca 97200

cttcagacta gctttttgat gggttcacct tttgtggtga tttgcagact actctgagcc 97260

cagggaatac tttccttatt aaagagaaaa ttaaggattt ttaggtttta aaaggctctg 97320

attacctaat tgaggtcccc cttgatataa ttagggggaa aatactataa aagggaaagt 97380

gggtgttatc tgaaggaagc cttgtgtgtt ggggacacag atattcaagg atatctagtt 97440

ccaggaatca ctaaagtcat ttaaatcctg taaatgaaca gggagtatcc catattcaga 97500

atcttactta tcttaggaga ccagtcatct cctatcttcc atctcattat ggttttgggt 97560

aaactatatt gtgtcaatgc ttaaataaat ttatggtctt cagtagagag tctcattctg 97620

atatgttgat cttcttatct tagattatat tttgttatgg cctgtcagaa aatcatatta 97680

ataaatataa tttgggaatt ttattctatc ctgaactttc tttaaatttg atgtgttagg 97740

ttttttgttc ttttttattt gataagttta acttttatca aatgaatttt aaaagtaaat 97800
gttgtaatta atttatgttg atgatctttt tttaaaattc caaagttaaa tttgtttgaa 97860
tgttgtccta atgtctaggt gttaaatatt atagcccagt gtctgtttgg tgctaaaatg 97920
atgtaagaac tattttcaaa catagctaat aataactaca tggattattg tttgagtcca 97980
tttcttcaaa aacacattct ttaaattttg aagtctgtgt ttattcattt taagagtaaa 98040
gattatttaa attattaaaa actaaattag acacatatat atgtgtatgt aaatataaat 98100
aaatataaaa taggccacaa agcgaaacat aaagttactg tatagtaaac tatgtaaaat 98160
ttgtttatat caaagctaag aataaaagct aagtcaaaac agatctttct ggtttgacaa 98220
gctttgatct tttcctttaa tcttttgtac ccaccctttt ctagccctgt atggttattt 98280
tatggcaaac catatgctat tacttatctt attcgttgcc acctctgtag tgcttggtat 98340
aagaatgaag ttattgtagt aaacagtggt ttattttaga tgcttttaac atttatgcac 98400
atcattgttt gcattaaaaa tgttgtcatt ctaatttaag tcaaattctg ggtttatagc 98460
ccttggtgat ttcagagcaa aaatttaaaa tttagatgat ctctttgaga taacatttgt 98520
tgtcttctga atataaaact atatgttttt attgaaggaa atgtgaaaaa tttatgaaag 98580
catacagaat aaaataaaca ttgcatatga tcttaatacc cagagctttt cattgaaagt 98640
gatttgtgta cagcatgccc tccaataaca tcatttcatt cagtattgtt ttgttacaat 98700
gttgatgaga aaaaaatcga ttcccagttg gggccactgc ctctgtggac tttgcctgtt 98760
ctccccatgt ctgcgtggat ttcctctggg cactctggtt tcctcccaca tcccaaagct 98820
gtgcacatta agtgaattga catgtccaaa ttgtcccatt gtgagtgagt gtgagtgttt 98880
atgtgtgtga ggggaccctg ccaaggttgg ttcctgcctt gccctctgat ttgctaggat 98940
gagctctggc cacctgcaac cctgaacccc agtaagtagg ttggaagatg aatgcatgaa 99000
taaatacaaa ttattgtaaa atgtaaattt gtaaagtgtt agtgtatttt cttatacact 99060
aattccttgc attgtgtatt ttcttagtct tttttcttct tgcgaatgcc tataaaccat 99120
tatacacaga gtaccttggt ttgttacatt tgaaaaccta atacatatta ttatgaataa 99180
tttccccatg tcactaaaac ataatttttt atggctatgt agtatttcac tatatgttca 99240
tattttaaat ttattaagcc aaccttctgc tattgtatgt tatgttgact ttgttgatat 99300
tattgataac actgtgatat gaacatcttt acatataaat gttatatggt tgtaacatat 99360
aaatggttat agctttgatt gttcctacaa gtagaactgc tgagtcagaa tatgagagta 99420
cctatttcct tgaattgggt attttttttt gccttctgca tacttgccaa tttgataaac 99480
aaaaaaagct gtctcatttt aacttgtatt tgattaacag agtgaacttt ttatgcactg 99540
tgagtttatg tttcccatgt tttatttgtg tgtttaaaag ttttatatga gttacagaga 99600
tctgaacttc cccaatcagt tgatcaatta tttgtgtttc aaaatacatt gaatttgtaa 99660

atcagtattg gaagaattta tattctgtga cactgaggcc gggcctggtg gcacatgcct 99720
gtaatcccag aacttcatga ggctgagaca ggcggatcac ttcaggccag gagttcgaga 99780
gcagcctggg caacaaagca agacccctgc ctctacaaaa aaacaaaaac aaaacattga 99840
ctcctcccaa ccattagcat agcatatctc tcttgtgcct ttcgggaaag attttctttt 99900
tcctgtttta ttgtattgga tctcctacca cctctttcta tttgaaaaat gcttaggcta 99960
aacagtgcaa aaacagtgtt agactggtag cactagcagg tgtttttacc tcatacttac 100020
tttcatgggg atgtctctag tgttttattt actctttaag gatgattttg atcatttact 100080
tgagataagt atttttaatc acaatatgaa ataccttgtt attcctcatt tattgagact 100140
tttttccttt agattggaaa tgaatattgg aatttaccaa aagtatcttt ggcatttata 100200
acaagtagtt agcttttact gtgtttagtc tgttcttgca ttgctataaa gaaatacttg 100260
agactggata atttataaag aagagagatt catttggctc acagttctgc aggctgtaca 100320
agcatggtgc tgacatcact tggcttctgg gcaaagcggg aggcaggcac atcatataca 100380
gcgatagcaa gagggagggg gttggagggg gaaggtacca cacactttta aatgaccaga 100440
tctcctgtga actcagagca agagcttact catcaccaag gggatggccc aagccattca 100500
tgagacgtcc acccccatga ttcaaatgcc tcccaccagg ccccaccttc aacactgggg 100560
attataattc aacaggagat ttggtgggga catacattca aactatatta tttacttatt 100620
tttgtaatca gttattaata gatttgctaa cattttattg actttgcctt tattataaag 100680
tatagtcagg tagtccatag agcacattca taactgtttt ggtagcagga gaatgtctag 100740
gagccacaag ttgtaaaggg ggaaacagga ggaaaagagg aaatataaaa caagtaaact 100800
catttactaa ttttgcccaa agccagtgct acagaacctc agctgggacc agaataaaaa 100860
aaaatttttg agagtcttgc tgtcacctgg gctggagcac agtggtgtaa tcgtagctca 100920
ctgcagcctt gacctcccag gctcaaacga tcatcccacc tcagccttct gaatagctga 100980
aactacaggt gcacatcaac acacccagtt aatttttctt ttttttggta gagatggcgt 101040
ttcaccatgt tgcccaggct ggtctcaaac tcctgggctc aagcgattcg cctgcctgtg 101100
gcctcccaaa gagctgggat tataggtatg agccactgtg cctggccaga gcaaattttg 101160
acataggtgt acatgctttg cataagtaca tgatatagaa actttttttg gggggagggg 101220
tggggggtgg ggatgttttc tgttttaatg cggttaccta aatttattaa gtgacaatta 101280
atggcccaag ttacagagag taagcctttg gctgactgtc agtcatagcg aaaggtctgc 101340
tttggtttcc acttccacat tgccatcacc cagggtaggc atacagaggc agtaggtagt 101400
agggatgaat ttgtagataa gatttctcac aggaaagact tgccaaatta gagtgatgag 101460
cagtgtaact ctttccaagg tcatacccaa gagataggtt tcttcatctt caaggtaagt 101520
taagacttaa actttctagg aaaaaaagaa actcaaagaa gggtttcatt ttcacatttc 101580
ttggcattcc tagatagtgg gtagcagttt tacagaaaga attgcaagaa agcaaagcct 101640

ggaaccttat agattgaggc tctaagctta aaatgtgatt ttctcttatt ttcaattaag 101700

ccagagtaca aatcttaaga ttaccatagc tattaatcct cactgaaccc tgacatggtc 101760

cccgatgcac atatatgtta gtaagtgaat ttttctataa tatattttac tcttatttca 101820

cctagtgagc agaaaattct aaaaaaggaa tttgtcatct gctccccttc attattatga 101880

attcgagagt atatcagtct taatgagttt tgaactatat atgtgctatg aataagttgc 101940

tttgaaatga tgaatacaag taatgagagt gggaagtgat gaagccttgc agtaatgatg 102000

cttaaggact ggctatttta ttatttttaa gtttggggct ttgaaatatc ttcatttaga 102060

ttgagatgct atttaagtac caactgtgaa aggaatagta actaggtagg ctgctgtgcc 102120

caagacagtg agcaggccta gttacaagaa ttgtggggta tgcagatcac tcggtgtaaa 102180

tggttatgac ttgatattgt ggctgaaaca agccatcatt gaggaaatgg agatgttcgg 102240

ctttagattt aggtggagcg agggtggaat aaaagttttg agtcttctgt gttcatttaa 102300

tatcctcagt aggcctgtgt ggtatatatt gttttcttac tacgggtaaa aaaacttaag 102360

tctcagagag tttaaacagt ttaccagtat cccacacaaa taagcagtag ataagattct 102420

gaccaaagtc tctgtgactt taaagcccaa gctcatccct cattctccca ctcttcagtc 102480

tagaagaggt aaaattctta agagcagctc ttaggaaaaa ctttttaaaa cataaatcaa 102540

atttgcttaa atgaaactgt gccataacta tatgtagttt gatcttgaaa atatctggtg 102600

cttgaggctt gttatctgaa gacagatgta gtggatggat tgtaaattcc aaggaattga 102660

tgttattttt agcaaacttt ccctgagcca cagattggtg gcagtaatgg attagctaga 102720

gaaatggtga aagggaaact tttataggaa tttatcgatt tttattacat ttgagaagga 102780

gtatacatct tttaaaataa aaatacagct atatccttac tgaaaatcaa gtataatctc 102840

tgatagactg aaggtatagt cagagtacct tattagttca gcagaatggg aatggtcact 102900

gcatgaattc cattctcata attatttggt aaaactcttg agatttagaa aagacatagt 102960

tcatagattt gtagaaaaag gtctcaagca gagattgtta gattatgaat aggcctcagg 103020

atatttgtga acactgaaat tttatgtaac attttgcatc tgtgcacatt cttttgagag 103080

agagaactga cctaaaaata gttatcaatt aaatattggt gtagagggca ctggtctgct 103140

gatacttcta gatagattag aggtgaattc aggagtggaa taaagcagtg attctcaaac 103200

tatggtcagg tgccatgaga aatggactag taagttaagt gagtgagtta aagtttgcta 103260

agattttata tttacattaa aatcagcaga ttactaggtt tttctgagtt tatatctcag 103320

aatcttcctt atttctcttg aaaagttttc ttcctatgtc gtatttggct acatacagag 103380

ttagtgcaca tgtgcatatg cagtgaaaaa ctagtaagtg aactgggata tcttggccca 103440

cctaagtaga atatggtcaa aaaagaagtt agaaccactt gtctaaagta tttgttccag 103500

agggtggggt gggatgagtg tggggttaag gggcagttaa agatagggtt gtgtgtgccc 103560

ttgtttacta gaatgaaagc aaataagaag tgtgtttagg tttaacttaa atataaatct 103620
tttaaaaaca ttaagtgtat tactgaaata cagaatttat gaataagtgg agacttgctg 103680
tttttgaagg ttaccacatt ttgtgatatg tttaggctag tagatgtcca tattttgact 103740
ttattattaa aaactaattc tttgtactta ttaatagtta tcaaaacatt tattttcatt 103800
catgttaagt aggtcatggg gttatgagaa aattctaatt aaaatcatat ttacacaact 103860
acaatcgttc taggtttaat aaggcacatt tttcttacct ttttatttta tttttttatt 103920
ttttaattta ttctaaagag agaggatctt gctgtgttgc ccaggctgtc ctctaattcc 103980
cgagctcaag tgatcttcct gcctcagcct cctgagcaac taggactata ggcacgtagt 104040
gctacaccca gcttcttgtt ctgttaatag ctaattatgc cacttagatg tggccatgtt 104100
ttagtagcaa agtaatactt tacatgttaa tgtatatttg aagcctgtgt caaagtatat 104160
tctgtaaagg caagttttac ctgttgataa cttaaatata tcctttgtgt tagcaagtgt 104220
ttaaaaatat ctcagaatta cttagggact ctggtaaatg gcaaaattaa gatagattct 104280
ttgaaatgaa tatatgtggg agtatttaat ttagaagcaa aatgaatatg tatttgtata 104340
tatggtaaca tgacagtgaa atttggaaat gtctgttatg ttgactttta tccttatgta 104400
aattttatgt ttaaaatgta agtaaatttt atattttgtt taaaatgtaa gtaaaaatca 104460
gtaagtcact ttgactattt ttcttttgaa tttggtaagc agaaaaacta aaccttctaa 104520
ctttttttta gtccaccccc atccttgaaa atgttttttc tttgattctt ctactatgta 104580
attggtttat gttgcaaatt tttttaaagt aatcatgaga ctatatttag tgatttggtt 104640
ggcatcgcca ttaaggaagt agtaaaagaa agtaaaattc aagcattgcc tttggagcaa 104700
gttagaagaa atcaaaactt ggctgaacat ctacaaatcc aaactgagac ccagctgtgt 104760
cagtgactac ctgtttgcag acagttttga atttgatttc tttatctgta aaacaaagta 104820
attaaatttg attttagtga tttcttctac ttgtgaaatc taataatttg agagactttg 104880
aggagtcttg atgccttttc attgtgttcc tcttctcttt gtttcagtga tcatcaaata 104940
ccagatgcct caatttagac taattgaata gaattttcat gatggagtct ggatatatac 105000
tttttacttt aaataaataa atatgtatat attttgtgca tatatgtatt ttaaaaagct 105060
ctgttgccct tttgggttaa tttacaaata tttcctccta agaaatatgt aatctcagcc 105120
aggtgccgtg gctcacgcct gaatcccagc attttgggag gccaagcctg gcagatcact 105180
tgaggccagg aggttaagac catcctggcc aacatggcga aaccccacct ctactaaaaa 105240
tacaaaaatt agccaggcat ggtggcatac atccgtaatc tcagctattc gggaggctga 105300
ggcacaagaa tcacttgcat ctgggatgtg gaggttgcag tgagccaaga tagtgctact 105360
gcactccagc ctgggcaaca gagcaagact ctgtctcaaa aaaaaaaaaa aagatatagt 105420
cttaccatgg atttttaaaa caatttagga ctgaaacttt aaaaaaaagg aaaaaattag 105480
atcgtttgta tgaaaataca tgtttttgaa tttttatgta cagggattta tacatacagc 105540

attgccttct gggaatatct tgattgtgtt aggagaccag attacctatt agtatttctt 105600

agtcccactt gaatccagta cttttcaatg aactgcatgg tatttatgga cttcagcatc 105660

ttgatttctc tttctctcag atcatgacag tttcattgta tgagtgaagt acaggtgtag 105720

tcagtcttct caactcctag ccttttcaga gatggttgtc aacctctgtt tttgcctctc 105780

tcattctttg ttttagttca gtttatatta tctgcttttc tttgtttcta tccttgagga 105840

tagaattggt atctccaatt tttcaaacac ctttctagct ttttccctca ctaggcaact 105900

tctgccccac tgctttttgc tgtacaaatg gtttttttaa aaaaaatctc tcccattgaa 105960

aatggaatga acaacaaaac tttagcttca aaatgcaagt aattttgttt agtgcagtag 106020

aaagaggttg atttaagaaa acctgtaggt tttttgtttg tttttaaata aaaaaagccc 106080

tgtgcaattt atcttagtgc atccaaattc ttatatctga taacattggg acctggcaat 106140

ttttgaccag ggaatagtcc atgtcatgga atactctctt catgagtaaa tctggtattt 106200

attaaagttt ttattttctt agcagttacc ctcttggcat ataatgggtg ggtggatgct 106260

gaaagtaaaa gaaatttatt aatattacca gaccttaagc agactcagtg cttatccttc 106320

atgaggaagg aagttctaca tctgatgcca gtggagttgt gaggactctg acctgttggt 106380

cgtagtctca agataactct tttctctcca gccattagag aaacaaggag ttcacattta 106440

ttggaatttg taaatatagt ttttctttct attggggtta cccatgtatt ttcagggatt 106500

ctcctatctt ttattctttt ccactgatag ttccaattat taatataata tcaggtgtaa 106560

ctataattga cttgttatag tccacatagg aagtaaaata atttctagtt atatctttat 106620

tctttaagct tttcatttat tattccttta atttgttatt gttaaaataa attgagaaga 106680

atatagtaaa actgagtaga acatacattt actggtctct tgccataatc ctgatttttc 106740

agcagctatt atcggataga taacacaatc tacaaatttt aaaacatctt tgataaaacc 106800

ttcccaggta aagcataaag tattatgaat cagtgcttag gaaattgcag ttggatggtg 106860

gacaaagtga tataagttat tctaattagt tgtaatccag aaacatcctt aagatttgtt 106920

ttttatgtaa tattttcata tgttgccctg gccattagca agacctcatt tatattgaaa 106980

attattattt agtgctctta tggaagcact aaattgggtg gtgactggga agatgaacca 107040

agatccagac actagaaatc gtcctttgta atatctttct tttagccttt tgtttctgtt 107100

accacctctt tctctcagct tgaagccctt attaccatag tattaagtat agtgcaagtt 107160

tatctcggat tatcgctatt atgttccgcc accaaattaa tctttctaaa acactgctta 107220

tttaggtcat ttctgtggcc tatggagcct aacctgtgtt tacctttctg tccttacctt 107280

ccattattat tccagctatc ccatttgtta gctaaactaa atacttagct ctgccaacac 107340

tgtctccgac attttgaccc ttgacacagg aggtattcct acctttaatg ctgttctttc 107400

ccctttcccc ctttcttctc ctgctgagta aatcttttta aggacctact taaatttgaa 107460

ctcccctagg aaactttccc taattgcccc acttgtagta gctttgcttt cttcttaaga 107520

tttatggcat tgtttttgtt tgtttgttta aacaattcat ttgatagcaa gcactggctg 107580

atgtttgttt gtacttacct tatttgtgca cttatcttct ttcccccaaa tacttctaaa 107640

cttagtgggc tcaggtctat atctttaaac tgtttgcatt acccatatta cttaatattc 107700

attgatttat tgctatttta tgaataataa gtaaaaatgc tcttcaagta aatttcaaat 107760

ctgtttgctt ataatcaaga acatctggca atgtctgcag aagatgcacc tgggattcag 107820

attgggtact aacccttgtt agatattttt tttagagcaa ttaaatatgg tatcagtaag 107880

ttatctgttt gaaaggggac tttgtgtata ttttcaggga ataaaataat tttaagaaat 107940

ttggaatctt aatttttttc ctataacaca gtatcagaac aatacatttg agtaacgata 108000

gtcatgtacc acatatcaat gttttgtttt ggtcaacaat ggctggacca catatacaat 108060

ggtggttcta tataattaga aataactttt ttactgtacc ttttctattt ttagatatat 108120

ttagatacac aaatgcttac ccttatgtta cattgcctac agtattcaaa acagtaacat 108180

gctgtacagg tttgcagcgt aggagcacta ggcactatat agcctcagtg tgtagtaaac 108240

catgccatct aggtttatgt gagtgcacac tttaatgttt ccacaatgac aaaatcacct 108300

atcaatgcat gtctcaaaat gtatcccatc attaagcaac ccatgactat ttttgttcat 108360

gtgctgagtt aaataaagtt agttatagga atgatatcag tacactgttg cagtgcttat 108420

agagatttta ttttgtcttt aagtcagtca agataaaatg gatttgaagt cagtgattta 108480

ctttaccatg gtactacatt ttctctatgt gaaaataaaa tgttaatgtt tttgtttgaa 108540

tcagtagttt gcccaaaaat gtttcttttg tctcatttct catgccttct ctagggttat 108600

gattagtata tagaaatatt aaaagaaata tattgctctt aaaaacctat ggagaatatt 108660

tcaataactt ttatgccctc tgtcaaacac ccagaaaaat gtcagtatta tttcaaaaac 108720

tgaaatgggg aatatgttta tcacatgcac aaaaataaat ggtaggttag acttttgatc 108780

tattaattgt aagcagtgcc ttttttcccc ctataagcta ttattttacc cattattttt 108840

ggagaatcta attacaatgt aagttcctta agggtagaga ttttatacag gttgagtatc 108900

cctagttgaa aactcttggg aacagaagtg ttttggattt cagatttttt tttcttttgg 108960

attttgggat acttgcatta tacttattgg gcgaacaccc caaatctgag aatctgaact 109020

ctgaaatgct ctagtgacca attttctttg agtgtcatgt cagcgtgcaa taagtttcaa 109080

attttgaagc agttcagata ttggattttc tgatttggga tgctcaacct gtacttcttt 109140

gtgtcctcaa catttaatca atgttttact tcccagtacc tcaataaaca tttgttaaga 109200

tggtaaagtg tctgtttttt ttttcttttg aggagtttgt ttttattgta ggagaatgac 109260

tatattctga ttttattcaa tttgttttct ttttatagcc ccattgacaa accttcagat 109320

tctctcagta tagggaacgg tgataattcc cagcaggtaa gatttgaata cagtatcact 109380

tgatttattt gccatactcc gaaagcaagg tattccatgc aggtgaattt tctaaaaata 109440

ctttactgac acaatttaaa aacacttttg atgctcatct ttattaatca tattataatt 109500

agccttttcc ccccagaata tatagagcat agtttaaccc ttttttaatg actcagaatt 109560

atcattatga ccagatacta ttatctttag agtctgctaa tgttattggt ctttttgacc 109620

ctgtgccaaa tggtcccagg ctagcatgcc ttctgagttc cttgctttta agaattgtat 109680

tcctaccaca tactcccctt gccccttgct ctctacttta attttctatt tcagtcaagt 109740

gtatttgtcc ctaacagttt cccttcttat tttccccaaa tgactatcag ctttgaaatg 109800

ataagccaga taaactgttc aaagttgaat ttactatgaa actgagtgga gtgctgaccg 109860

ttagagcatg ctttatgtca tcttgtctta ttgctcaggg aacttttttt tttttttggc 109920

tagtttttca ctccttgagt ttctatacat cttcttacat tggtaaaata aatgtaattt 109980

agtatctccc gtaaagaata gccagtcctc cacagtattt atgaaaaact tagagatcta 110040

aaatataatg cttttctaaa cgttttaatg acacattttg taaaagaatg tggaaatctg 110100

tatatcccct tgtgtatttt agttgcccat taacattttt tacaggttaa aagtagttgc 110160

aaaggatgta atttccagca tattagaaat attggtatct gaaaaattta atgttttgat 110220

atcccatcat aatgttttga tcataattct gttttattat cctgtcatcc atttaaaaaa 110280

atacacatgc tctcctttaa caatcagaag ttttacatta tttttttctc tgtgaactaa 110340

tttttctctt tctgttatta tagagtttta tcttaatgca atatagtttt tgcttgaaaa 110400

gcatatactt ctcttaaaaa taggcatata aatttttgga ttgaaataat gtttttgcgg 110460

tcacagactt caaatgccaa tttttgtctt ctgaatttta ttagtagtat tgaggtgaca 110520

ctatacataa atgtagattt taattttaaa aaccatgaaa caaatagtaa taaatggtaa 110580

aattatagta aaataaatag taaaattata ttgaaaattc atctcttaat gaagaatagg 110640

gccattttaa aattggtttg tatgtatctc tagattaatg ttacctattg gtaaaagagc 110700

caagtttcag cagcaatcta gtcgtgtttt ttatttttct aagatgtaag tgttgagata 110760

agatactttg ttcatataaa taggtagatg gaaggaggag tattatatca gtaccactca 110820

agtctttcaa tttcttctga attaaatacc taagaatcat acaaagattt attatacaaa 110880

attattttta atgatgtgtt tggaaacaac ttaattagat ctttctttaa taatctagcc 110940

attacagtca gtttctctct tatcaccaat ccatgtattt ctaataattt gtgtttggcc 111000

tctggaaagt tttcactctt gagcaaaata ctgtagtaaa atcccagaat gagaaatatt 111060

attttcttgg taaagtgaga gaagtaaagt tgtaaaaaga gaggagatgg ccgggcacgg 111120

tggctcatgc ctgtaattcc agcactttgg gaggccgagg cgggtggatc acgaggtcag 111180

gagatcgaga ccatcctgga taatgcggtg aaaccccgtc tctactaaaa atacaaaaaa 111240

ttagccgggc gtggtggcac gcgcctgtag tcccagctac ttgggaggct gaggcaggag 111300

aattgcttga accccggagg ccgaggttgc agtgagccga gatcaagcca ctgcactcca 111360

gcctgggcaa cagagtgaga ctccatctca aaaaaaaaaa aaaaaaaaaa aaaaaaaggg 111420

agactagttt aatcttttca agcagattaa tattttttta aagagtatat atgtaagtta 111480

atgatttaat tgattttctg aaacttcatg ctttcattaa aacatcttca agtaccagtt 111540

tgtgaaacac tattctaata catttagttg caaacttttt aattgtggct tattaaaatg 111600

tataaacatg tagttattat ttgttttcct tttttattca gatatctaac agtgatacgc 111660

cttcaccacc acctggtttg tcaaaatcca atccagtcat ccccatcagt tcatccaatc 111720

acagtgcacg gtcccctttt gaaggggcag taacagagtc acagtcgtta ttctcagaca 111780

attttcgcca tcccaaccct atcccaagtg ggcttcctcc tttccccagc tccccacaga 111840

catccagtga ctggcctaca gcaccagaac cacagagcct cttcacatca ggtatataac 111900

aatagtacca agtattagta attatgctct taacgtattg ttgacagact aacatgaaac 111960

tcattagtgg atatattttt atcttttgcc tctctgttct cttttaataa aaatgcaaat 112020

atttaaacta catggaatta ggtggttaaa tacattgttg tgtgacctag aaattactgt 112080

gttaaaagat ttctccagaa gtaaagggtt atattgtttt cccaatagta aaaagcttat 112140

tccattggga attttttttt tcatttttac tttttggagg tgggggtctt ggtctctcgc 112200

ccaggctgga gtgcaagtag tgtgatctta gctcactgca gcctcacacc ctgggttcaa 112260

gtgatcctcc cacctcagcc tcctggttaa tttttaaatt ttttgtagag acagggtctt 112320

gctatactgc ccaggtcttg aactcctggc ctcaagtgat cctcccaccc aagccttcca 112380

aagtgctggg attacaggca tgaagcactg cacctggcaa ggacttagat ttttaattag 112440

atttgtattt ttatttaatt agatttgtgt ttaagatttt ccacccccag ctgacaaaga 112500

tgcaagttat ttagtagtaa cagttatgtt actactaaat ggtatttttg agccatttcc 112560

atttttatcc attctaaatt ctctcattac tatgaaaggt ggcataaaag taacattgac 112620

aacagcacct gtggagcaga aaattccaaa aaactatgag gattaatata aatacttttt 112680

aattaaatta tatttttctt agtatagaaa gcatgtattc aaaattattt gtactttgat 112740

aaatacattt aaaaatcaac tttactaatt aaaatctgtt atatttattt gatggaagtg 112800

acttaggttg gttttgatta tgaatggttg agagtataat tggagtgtca agtacatttt 112860

tttcctttga ttagcaaaat cagtctatat cattcagttt ttgaagctag tagcttaaga 112920

gttaagtaac tgctagaagt acataaggcc ttttgtaatt atatgatctt aatgtgctaa 112980

gaagtcacat agattttgca cttacttaat ttgtaaaatg acatgtttaa ctttcagtag 113040

catggatact tattaagtat gaaaatgagc tatgtatctg atattcatca ctatttttct 113100

tcccaacaga aacaatccca gtatcatcct ctacagactg gcaagcagct tttggctttg 113160

gttcttctaa acaaccagag gatgacttgg gttttgatcc cttcgatgtc actcgaaaag 113220

ccttagcaga cctgattgag aaggaactgt ccgttcaaga ccaaccttcc ctttcgccca 113280

catctcttca gaactcctct tcacacacta caaccgccaa aggtccaggc tctggattcc 113340

tgcatcctgc tgcagctaca aatgccaatt ctctcaatag taccttttca gtcttgcccc 113400

agaggttccc tcaatttcag cagcaccgag cggtttataa ttcattcagt tttccaggcc 113460

aggcagcccg ctatccttgg atggcctttc cacgcaatag catcatgcac ttgaaccaca 113520

cagcaaaccc cacctcaaat agtaatttct tggacttgaa tctcccgcca cagcacaaca 113580

caggtctggg agggatccct gtagcaggta ggttcattta aaacctttga gaatttttga 113640

ataactcata ttttcaggtt gttgggtgga tagggaagta actgttgaat aacagagtac 113700

taatatcttg gaaatgtcta tgaaagatta gtctttatca taatctaaaa tatatttttc 113760

attagatgga tgccttgtaa tattgagctt tataaggatg gtggagtatt atacttagtt 113820

attgtttgga agcattttac ttaataggat aattaccaaa ctttcatatg gtatacaaat 113880

gcaaaatgaa aaagaagaaa atggtctctg aagttatggt actataaagt ccaacaccgt 113940

ggtgaaaatt atgattttat attctaaagt aaaatggtta aaaaaaattt ttttaatgat 114000

aattaatgag agcatgaggg ataaagccac tctctaccta cttttggcag ttacttgttt 114060

tgactgatac acatatacac acacatcaaa actgagctat aaatatactt gagaactctg 114120

attatgaatg ttttacaagc atatcatatg tctcagacat acaccttttg tgaaagagaa 114180

attgttcaag gaaagatact ttcccatttg atctgccatg tttttatttc tggggaaaaa 114240

tatgaagtat atataaggta atgtaagttt gatgccaaaa tacttctggt tttgtactgc 114300

tacccttgaa ggtttcagaa cttttgggtt ttaagattct atataacatt ttttagatga 114360

agtatcttct atatccaagg gaattattct tgttaatagt gttctcgttt aactttggct 114420

ttagagatga tgttaaaaaa agtttgttcc tctcctgatt ctctcaagtg aaatagaatc 114480

atgattctct caagtagaat acaatagttt ctcactctgt tagaatagtt cttcacataa 114540

ttcatacaat ttattgttat cccttgttag atatggcatg gcacagtatt attgtaatag 114600

gatactttta tcagcagcaa ggcagaagtt cacgctcttg tttttagcaa ggctgataga 114660

tgaatttaca gaggtctatc aaagttcttt atccctgttc cctttgggac cgacaatatt 114720

tcatgtttta gacgagtagc taggttaaga gaatatagtt tgtggctgtt gacatagtga 114780

aatttaccgt tgatacagtg aagttaattt acataagcct ggtagcacgt aatgtagtta 114840

tttgcacagt gttgggactc ttatttgttc atttaattat ggtagttagt aggtaaccag 114900

agtgacattt ttcagtcctc ttcttctaca tgatacttac ttgtgaaaca atgtgttcag 114960

ttccaaatac tccttttatt tccttccatt caagatagca tattagaatg taattttctt 115020

tgattttact ttgctttaat cattgagaaa tgtggcattt taattactag tagtgtatct 115080

cataattgat tataacattc attaaaagtt aaagttaagt tctcccaaat catcaaaaca 115140

tttaaagatt agaccaatta cagtgaatat gagggtttct atttgcccat caatgaatta 115200

ggacatgtgg gacatattaa agaagtttaa gatatggata aaatttccac atacttaaag 115260

tatagttgaa gagactaaaa taatgtgtga aagtggtaac tatatgtctg atactggaat 115320

ctgagagaca tcaatgtacc ctgtaattct tggggaggct tcattgaata agtaggaatt 115380

gatccctact atcatatctt agatttgggt tagcagcagg ggaagagaaa aggcctttta 115440

tgttgatgga aacctcctga gtaaaggcat agaggcagga attagtatgt cattacactg 115500

agaagtaata cagctacctc ctataacagg aggtgcttct taaattgtat agatataatg 115560

gatgagaaga tgagaaactc tgacaatgag gcaaaatggc atggacttgg tatgatagga 115620

agtaggatgc ctttatggga ttctagttat tagctgtctt ctttttgtgc ttttatttga 115680

cttattacat gacgatcttt gattttcttc caatttcata gtcatcatat tatcccttaa 115740

cactgaagag tagttttaac ccatatattt caaaccaata gttattctca gaatgctgca 115800

ctggtaatga gaaatcgctg attagtgagt ttagttgtcc ttgtgttagt catttaaaat 115860

cttcgtctaa agtagatgct ttatggaaaa aatgtgacat taaattatgg tcctatcttt 115920

ggggattttg ttttaatttt taaaactttt accatatact caaccataga gcattaaaac 115980

cttttttttt cagagatgct aaaaatgtag tcaccaactt taggttctga atcgttgtac 116040

tatttggtag ggggtgggag agtttatttt ggattttcaa tttcaggcta tgcagacttg 116100

cttggtattt aaatggtgac ttatggttaa gcttattatt aggttatttt taaggctctg 116160

atgactagag aaattttagt tttaatgtac caaaatttaa aaatttttat caagagtctc 116220

acacaccaga atcctctcat ggattataaa tatttggtag atgccccaga aaactgaatg 116280

tgctaagcct cagagcagaa tagctgtgta attgtagcca gaagatttag gattttcagt 116340

aactattaga aatgacagaa agtgaaaatc tggaaaacta tatatacatc ttaactatgt 116400

ctaatttgaa gcaatttaat acctaggtaa ctcacttgca cctgttgtaa ctcacttgca 116460

cctgttctaa ctcacttgca cctgagttga actattggca aggattcaac tttgtatctt 116520

ttagcagtta tcatgatacc ttacacaggg cagctgtcac taaatgcttc agtaatggag 116580

agagtacata cctgtttcag tgggacctgc tactttaaga tatacttaga caatatttta 116640

aactaattct acactctgga aaggcatata tatttattct ctaaatatgt agagcaaaat 116700

atggtctctg acctgtaaga gcaaatcaga gatggcagtc acctacatgt catacagata 116760

gaatgtctac ttactataca tttgtactta aatttttatt tttggtttgt tcagtgaatt 116820

attaaagatt gatatatctg aaacctatca tgggaaaaac agttgccaga ggtttgacag 116880

aaatgcagct tccttttttt ttgatctaat tgaaaactgt ttttgtgttt aaaattagta 116940

gttatttcct taattaggtg aaaccttttt agcagcattt ggtttactaa gtttagaaat 117000

atttttgaca taaatattct tgggggaaat tttgattcgt gggcagttac agtaaattca 117060

ttcagatctt tttttttatt attattggaa ccatatgcct tctttctaca aagtgattta 117120

ccttgtccta aaacgtgact ttgttttgtc caattccttc attttaggaa tagcaaagct 117180

gtgttccaga aagcttaaat aatttgttaa aggtcacata ttcaattagt agtggaatta 117240

gggtaagaac ttagatctca aattcaacaa tggaaatgtt tttgcactac aacgatgccc 117300

tccttcataa atgaagatca ctggcatgtg ctttaacttg gctttcttcc cgtggcaatt 117360

gtttttgctc tcacacactt ggaagtccct ttttatcaac agtatcaact catgagacaa 117420

attggcccag ctcaggggta tatggcctat tgttacaaaa tagaataaag tgtatcgact 117480

tgatagaaat cagtctgttt cttagtcctt attaatatca ttttcttgct aagaactcta 117540

gtctaaaatt tgctttacag agcctcattg ctatgttctt ggggtttata agaatctttg 117600

ccaatttact gaaaaactac aaaatcacct gttatatgga ttgacagact ctttttctgc 117660

tttgtcagtt atccttccac atgtaatttt gcagatttgt tttaaaaaaa tattctgcca 117720

agttgttttt attccatgat gttaaaaatt atttggaaaa cataagtcag taagcattgt 117780

tgtttcaaga tatacctcaa aaaatatttg aaactgttta ggattggatt tttttttcat 117840

gtttttgtta atttaaactt tacttttgag agggcatttt tctttaaatt ttctttgttt 117900

ctatatgaac tgttttaaca gtgacttgat tgaattttga gtcatgtgtg aaacaagtca 117960

cttgtagtca aataattttc agttataatc aggagaaccg atctcattca tctataattc 118020

tctgacctag aagtagtcat ctcattttca gtcaattgga tcattgtact gttaacattt 118080

tactattttt caattacttc aaaggtaaac ttgaattttc agatgctgta tgttaattta 118140

ctagttaata tttatagctt tcattttttt ctagtacata atttatgctt tcaaaacatg 118200

gatttgtaat ttttctcttt taaaaatcag ttttcacttc tgtaaatcaa ggtttaattt 118260

tttcacaaaa tactattaat atttaccatt tactaggagt atctataagg gtccaattgg 118320

cttggaaaag aatggctttc ctggtctttt tttttttttt tttttttggt agtatgttta 118380

taatctaaat ctattgtatt tgttcttatc tctgaaacaa tattactcag gatatattta 118440

gaatataact actttccaag tacttgataa gtttatattt taaaattcat gtcttgtttc 118500

acacctggat tccaaacatt aagcatgtat tttatatatg tatcagtatt ccaaatattg 118560

aaatcatttt taaagggact gttaacaaat ctagtcaccc ataactagac catgactgac 118620

ttttccacag gggaagaaga ggtgaaggtt tcgaccatgc cactgtcaac ctcttcccat 118680

tcattacaac aaggacagca gcctacaagt ctccacacta ctgtggcctg acaacagaac 118740

tgagaggaga ggattagact ctggggtgct tgcatgggca actggatttt tgcatgattc 118800

ctttatgatt ttgcttttaa tgtatacacc cagaagagcc aatataaacg ttcctcatgc 118860

ctacagctag cgtgttacct catagttgtt gaagtatttc ttcattaggc ctttaacata 118920

atttattagt gtaataattt tggcattgtt tctcatgagt gatactattt ctaactcata 118980

aaaataaact tgtagtacta agatcccagg tgagattagc tagaattgtt gttattttgg 119040

ctctattatt gaaaaattat acaaaggtaa tggtagtatc ttgttttctg gtttgtgaaa 119100

aaataatatc ctaatctcct gaaaaatttg caaattaaca agataatgtc tattgcattc 119160

tttgaatact tgagaagaaa gaatacatat aaaacaaaat tggtggtgtc atttaatgct 119220
cagaaggata ttgttggcga ttgtttttag ttgtctagat ttatatccta taatatgcat 119280
tacatgtgta gaaactgttt tgttctctct cttttgcact tttgcacttt ctttttgaat 119340
ggttgttaat tatgcataga tggttaaaag ataattattg tagcctaaat gcaatataat 119400
ctcttttcta atgcactgcc tagtatacca ggaaatgcct caaaaacaga acttcagttt 119460
atagttgact tatctgaaac ttgaatacgg agtaaacatt gtatgtgaaa agcaaccata 119520
gtagtgtaac attgttactg aacaaacagt aattctgtgg gatattgttt tggaaatttc 119580
attgtttaat aattaaaatg tgataataag ataatattat aagtacctga ccagaatgag 119640
actaaggcag aagagaagag attggatgtc ctctaaaatt aggccaccta aattgtaaca 119700
gataatttta atcaagagaa tagtggagta ctttattagg acataatgtg aagtgatatt 119760
tctgaaaaca catttcttga aatagtttta aaactgataa taaaggaaaa gaaactgtac 119820
ttagtttgga gcagtggaca aaagctgatt cagaaaacat gaccatattc tgcctacatg 119880
aagcatctgg gaagaaaggg aaagtcacac ctcctcttga gattttaacc attatgataa 119940
aaggtatgct ttaatttgtt aaaaaaaaaa gtatgaaagg ggataatgtg aatacaaaaa 120000
agaagcctag agattgaaaa taataataat agaacatgtt attaggagat ggtacttaat 120060
gtcatgttgg agttggagtt ttcaaggtta cctctatata aaattggatt attttctgtt 120120
agatataatg tgacaaatta aatgacttta tcccaaggta cagtgaggta aacaaaaatc 120180
tattcttaga tatccttaga tttggttaac tgaggctgta gaagcagcga attgtctagc 120240
catttaagtt atttttgtaa aattcagcat aaacatcaat ctctaatttc tatctagaag 120300
tgactttaag acattacaag aacattgcca atttggaaaa tgccaataaa atactgtaga 120360
cttattaata ttgtagactt attatcagtc ttttactata gcttaattta attatggttt 120420
tcatttttac tgtaagaggc caagacttca cttatgtctc ctgtggagct gaggctcctg 120480
ggccttgtta ttataacaaa ctctggctcc ttgtaatgat gcttactagc tcaaagcctt 120540
ctccagcatt cagtgttagc ctttcattgc ctggatcttc ttacaaagcc atagcaagtg 120600
gttagtgcca tttgctcctt ggtgttgaga gatatacctg ttaaatgagg cttctttcct 120660
ttctcattca tagccagcgt ggaaaaatca tcaggtagtt tgcccagatt tgccagagcc 120720
ctaaataaaa tggcaaaacg atctccctgc ctgcttctcc tccccacccc cacttacatg 120780
cacaccgtaa atcaagaaat gctttttggg caattagact ttatcttcta aatttttgca 120840
tagaatggct aaacatgatc ttttgcctca ctttgtaatt cctacacctt ccttgtaagc 120900
ttcacctttt gcttacctaa tattttattt aaacatggtt ttttaaaaaa cctgaagctt 120960
taaaatcata gtcataatta attttacact aaaggacaga agaaattata caacacataa 121020
aagaagccac tacttcacat agcaagcaaa ttgaaatcaa aatgacagaa gaatgagaaa 121080
ttctttaaaa gcttcaggtt ttttttgttg ttgttgttaa attcctgcat gatgcatcat 121140

gtgcaaggga aacttaatat acttttacac ccttaacctt tataaagtga ctagcttttc 121200
ccttaacata ggaattttta acctggagcc cagatttttg agagtacaca caaaattgtg 121260
ggtttgtgtg catgtacata tgtccatttt tttctaggga gaagatccat agttattaga 121320
tttgtgaagg atttggtgat ccccttctcc catcaaaaaa aaaaaaaaaa agaacagctg 121380
ccttgaaatg tttttagtgc cactttaggg aatagagtcc tgtactgaat gattttagtt 121440
gtatgatgaa ttataagtgc ctctttattt tctctaagat tgcattagaa aatatttttt 121500
tctgactaat aatgctttcc ttttcattct attattgaat tttggttttc catgtgttac 121560
ttttgtgtac tctcccctta tttcaacaaa gcattaatta aatatttgag tacctattct 121620
gtatagtgac ttgaactaga tctttagaga tacaaggttg aataaaatac agaccctatc 121680
atcaaggata attctagtga caattatatt tcacattatt tctgtcaggt cttgataata 121740
ttttaatcac aggaaccacc atagcagtcc agactcattt tattatttat catctctcag 121800
taactgctcc gacagtggca acaaagggta ttgaatactt atatttcaaa ttttaaaatt 121860
tatgataatt tggagggagg tgaaaaaacc ttactaggaa agacaaacat tcattattct 121920
acgtgtgtgt gagctcatgt ctcttactct tagcatcctg ggaattaagt acagccttct 121980
gtgtaggtgt tcctttaaag aaaacaactt taatgcattt tacctctgca catttccact 122040
gacatgactc ctatttgtac agtgttccga gcactgtcaa gactgccaga gctctggcac 122100
aggtccatag gtactcccac ggaggcattg cctcagaaag acaagagaaa ggaacaaatt 122160
gcagttgctt tgaaatctta cagtggccta taggggttgg gatagggcag taaggagatc 122220
cagctgaggt attcagggac tggtaagctt ggataagtgt gtttaaagcc ttgaaaacca 122280
gaagaagagt ttagtcttga tgtgaatgtg gtcagtggta gagaactata ggttcttagc 122340
aaaaatgtag tttgaacata gcattttaag aacagtaatg gggtaataga tttgattgca 122400
attaggaccg gttagaaagt aattccagta gtatgccatt gctggccagt gcaaataagt 122460
tggcctagac tagcacagta gtggttggaa tggagaagaa ctatgaatta gaaattggaa 122520
agcgtaagag ctgagaaaag gccttcagat ttggttagat ctgtggtgat ctttaaaaat 122580
gtaactttca tagaaaagta cagaatggaa gcccatcttt ttaggattaa tgaggaaatg 122640
tcaagaatat gtatgcagca atcattgact acttagtgtt aagacatttg acaacaaagt 122700
tgaagagaca aacaagatgc taactagatg ggtggtagat cttagaaggc attgctttaa 122760
tataagggag aagaagaacc taaatattta aaggcagacc ttacagagcc aattgagagg 122820
aagagactgg cagtgctgaa gagaagatag gctcattcag taggataata gatgtcgttg 122880
ggtggtggga agagataagc tcctgagacg agcatgtggt atagcttaag agaggtgggt 122940
ggccaggcgc ggtggctcac gcctgtaatc ccaacacttt gggaggccaa ggcaggcaga 123000
tcacaaggtc aggagatcga gaccatcctg gctaacacgg tgaaaccccg tctctactaa 123060

aaatacaaaa aaattagctg ggcgtggtgg cgggcacctg tagccccagc tactcgggag 123120

gctgaggcag gagaatggcg cgaacccagg aggtggagct tgcagtgagc cgagatggtg 123180

ccactgtgct ccagcctggg caacagagcg agactccgtc taaaaaaaaa aaaaagagag 123240

agagagaggt gggctgtatc ttcctctgtg atttgtaaga gcaagaagaa cctggggaaa 123300

gagaaagatg agaattgatg aaatggctca tatcagtaat tattaaaatg cattattgtt 123360

tgaaaatggg ggtgaaagag tggaattggg agccttttaa aggagattaa aaaaaaaagt 123420

cttttgtgag aataagccca gaaatgcatt gtaaatagtt gcttagttgc tgtgtaaaga 123480

taagagtcag cttaaagatc tctaaagttg aaatatatgt ggttttgtat gttcctctgg 123540

tttagcacaa agcataggag tggaaaaagc aaatattgag tgtttggtta aaaggggaaa 123600

aagacaaaga catagagaca ttcaagaaga ccacattgaa atgtataatc ttgtgccaag 123660

aagagtctat taaggcagtt tcctgtgaag aaatggtgtg agtgatgtgg aagggagttg 123720

aatgatttta gattttgatg atgacaaaga acatgttagt gttgctaagg gaatgataac 123780

taggagactg tggtcactgt gataaccact caatgctaaa tgatgattga gaaccagaga 123840

aaggagaggg ttggagtttg gaaagaagct tgtcttttgt aaccgtctat atactctctc 123900

ctccagtctt ttcatgagtg taggaagtca aactatgtga gagagaattg tagaaagcat 123960

ggaagcaatt ccatgcatcc gcagatgggc acctggggaa tcctgggtca ggtttccggt 124020

gagttgaaaa tgatcaaagg aaaacttcct tcctcaaagc atcgtaggag gacatggaag 124080

cagagtgctg gggaggacca ggaaaagcca gggatgagat gagacagcta taagtaagca 124140

gccccagtga gtgtgaagga ttggtagaaa tgttactgat gatgtgaaag atttagacag 124200

agggattagg tatggtgtat gatgagtttg gtagttgagg agcccttatt acaggtaata 124260

tagcttctcc caagctcact tgcaaactgg aaggcttttt taaaaatggg cttccaccta 124320

tgaagggaaa gcatctgctc ttacatagtt ggattaggtc attctgcttt atttatatat 124380

caaataagaa ttaaggtact ggtgaacagt agagataaag taagaatttt attgtaacag 124440

gttataaaaa tattttgata atttccccca aagagaatgt actatgaata aataaaatct 124500

acaaattatt gggctaattt acccttgttt cttatatttt gtatacttag gaggtgacct 124560

ctaggaaaaa attgctgaag aggtagtaga gcagggtgaa gataccgtat actgtcatcc 124620

ctaggtatct gtaggggatt ggttccacag attgcaaaat tcttggattc tcaagtcctc 124680

gatataagac agtgtagtat ttgcatatga cctgcacata tatgggaggc cgtatatttt 124740

aaataatctc tagattactt ctaataccta ttacaatgta agtgctatgt aaatagttgt 124800

tatattgttt agagactgac gaaaactgca tgcttaataa attacacaat tttttaaaaa 124860

aattttctat tcgatgttgg ttgaatccac aaatgtgaaa accccaggta cctggagtca 124920

actgtgtggt tttttttttt tctcctagtc agttaataat aaatattcct agttgggaag 124980

caaccttagt tttctaataa agtattttta aactcactcc gtatatgtca gctgaaatat 125040

caactgtgac atttaaaaag gataatgctt ctaagtacaa atcattgctc aagaatacta 125100

tgtttgtgaa accttgttga tggattgaga agccactgct ccttggacct ccttatcacc 125160

ctgtgatttg cttctgtgag ttgtaatcga gccacctaac attgctggtc atactagcct 125220

tccacttctt agtcaggggc tatttccaaa gaatcactct tgataatttg agatgcatta 125280

agaagattct actctgtttt ttggtctttt agagagtctt ctctgggttt gagttataga 125340

gaaatcgagt ttaggtacct agaactgaga gcaagcctaa gtaatctaag ctacttgaga 125400

aatggactga agtacccaga tgttccataa aacagcttga agttgttgac cttgagaacc 125460

atttcctttt ctccaggttc ctcttgacca cacttcctaa ggcaactatt gacttcactg 125520

tcttagccca ctttaccttt attctaagag ttcctggaag ctccaagctg accctggaac 125580

atactgtgct tataacaatt ttgttattcc agctcctgtt gggtgctcag acccactatt 125640

gtgctgttac aaagcaacat cccaaaatgc tttttcccct catataattt ataaagcctt 125700

taatgcatcc attacctttc tttctttttt atagcgggtg aaattcacat aacgttcaat 125760

taattatttt aaagtgtatg atgcagtgac ccagtaccat tctttaatta tctcaatggt 125820

aaaatagcag aataagtaac ctactctgct gtaagaattt tagtctgttt cttgttgggg 125880

ctgtgtggta atagctgctt ctagactctg tgttttaccc agtaggctga aaggcaaatg 125940

ttaaaccttt aaatattccg tatttctgca tagcagtact actgcaactt aagcttgtga 126000

atcaagcaac ttgtatacac ccttttaaaa aggcagtggg gggcagtgca aggagctcta 126060

ggcaagagac acttaggaga tttgcttttc tgagtggtaa aaatgtttga agcaactcta 126120

aatagctttg aaatgttagt aatagtctta ctgtcttcat gacatttaat tagccttctg 126180

taagagtttg tttagcaaag gtaaaccata agcctagccc tctatatgca tgtgtggttg 126240

aatgtgtgtg tgtttttata taaagcaatt ttcagttaaa tcttccactt aatgaatgta 126300

gtagaggaag atgtctcatg tcattttggt gaccttgacc tatgtctatt catgtattta 126360

ttcctgctgt gttccaattc atatcttcag gataaatgct gtatctttat tgtctttctc 126420

caattttttt ttgaaggaac caaagaccct atttaaatgc aataaggttg taatactggt 126480

gcccacttca ccctggtcat gttttttctg aacaaatatt tcatagcttt tcctcatcaa 126540

attccagtcc cactgctaag atacccatct atgacacagt ctttgggaaa acccaaggaa 126600

aggataactt aaaaatgatt tgtattggaa tcatgacact tcagagctat gaataaataa 126660

atataacata gtctttaaca atccttttaa agaccgaata ataacaatga tgataataaa 126720

caaggctccg aaggattgtg atttgtctat agtcacattc taacaacaat ttgaagtttc 126780

tttagccctc accatgtaaa tgtgctttca tgcttgaaac tcagtggagg aaaaaacttt 126840

taagattcag aatctgtctc ctctaaacat tattgtgtct tacagtttaa ttacagtagg 126900

tctctcaagt caagggctta caactgtgaa ttcattgcaa acaatgaatt tgaaggcttt 126960

caaaatgacc accagctggc agctgttgtc acagatcctg taggttttca aagctgccct 127020

gtgttaaatg tggcccagtg atcttggagt aactttagca ggggaaaaaa ttgtaacctt 127080

gcaggtactc ttaaaataaa cttatgctaa ctacaaaatt accagtgtca gaactaaaag 127140

agtaaaacag aggtggagtt taatttaccc aggatttttc tgcagtacca aagagaattt 127200

aggggacaac acacataacc actagactgg tgacatctct aatattacaa aatggaaaaa 127260

aacttatagt aatggcacca agatgtatta catacacact aaagatgtga tctccgattc 127320

atctttgaag aagagctact ctgtgttaca ccatatagaa tgatagcata tctgctgggt 127380

ttttttgttt tttttttttt ccccaatcaa ggaagttgtt gttttcagca taaggaaaca 127440

gggagagctc cacaaggatt taaagatgat aatgctatca ctatctccaa gaagaaatgc 127500

aagaaatctg caatcaactt ttgtagccta gcactgttgc ttaatatagt gagatcttgg 127560

cccaaggact gcacttagca tttacactct gttatcagac ttgcatactg aaataatgta 127620

ttgatggaac agaaagtagt gatctagaac tttagtgggg tgttctaaaa cattgtcgtg 127680

ttatatgttg gcatttgcaa gaagtctgac aactgtatat ttttattttt ttcctttatt 127740

acatctatat gtttagtatc cagctaaagg taaaagattg cctgggttat gacttcatca 127800

gccaagtagg aagttttttc attggcctta aactgaatgt ttggttacag ccatagttcc 127860

acttaggttt tctctgttaa aaatgttagt ttctattaaa attcatgatt atggatggct 127920

gatatatata tacacacaca catatatata tacacatata ggcacacaca taccctactc 127980

cccccaatcc ccatttagag taagatttgc taccagatat gtttcctttt ccttctaaac 128040

tgttaacatt tactaatttt ttccttctga actgttaaca tttactaatt aatctaccca 128100

tagtcttccc ctggttccag acaagtgtaa catatcttga tttctagtct gcccagtgtt 128160

gatagatgta aactcctaag acctcacctg cattttaaat ggtagatggc tatttaattg 128220

aaacgaagca tattattaaa ataaggattt tatataatct ccaaatacac aagccctgca 128280

acctttgttt tataacacgc tttcaggttt taactattga ctattataag tctctttccg 128340

gtagcttgtg ttcacccctc taaccagttt ctttttcttt ggacaaagga gaagtttctt 128400

tctccatgta atttaccaca attaacaggg aagtcatgac taaatcttaa tgtacactac 128460

ctatatgatt ttcctactgg attcctgaca ggcttgaatt ttcccttttc ttcttcttct 128520

tccttttttt tttttttttt gagacagggt ttcactgtgt tgcccagatt ggagtgcagt 128580

ggcgcaaaca cggcttactg caacctcgac ctccctggct caagtgatcc tcccacctca 128640

accctccaag tagctggggc tacatgcaca caccaccatg cgtggctaat ttttgtattc 128700

ttctagagac agggttttgc tttgttgccc aggctggtct gaaactcctg atctcaagtg 128760

atccatgtgc cttggccacc aaaagtgctg ggattacagg cgtgagccac cgtgcccggc 128820

taattttccc tcttcaaagg agagaagtct ctctccatca gtctactgag tgttttctca 128880

gtctttttac tcaattacca gagacagcag accctctatc tgcccaataa ctactcttac 128940

tcacctgtca gcttattcca aactgccttt gatcctaaac gagtatattc ttgttttttt 129000
ttttttcctt ctctctattt ttattttgtt tgagacactc tcgcccaggc tggagggcag 129060
tggtgcgacg tcggctcact gcagcctctg cctcccaggt tcaagtgagt ctcctgcctc 129120
agcctcccaa atagctggga ttacaggcat atgccaccat gcctggctaa tttttgtatt 129180
tttagtacag acagggtttc acattgttgg ccaggctggt ctcgagctcc tgacctcaga 129240
tgatccacct gccttggcct cccaaagtgc tgggattaca ggcgtgaccc actgcgccca 129300
gccttctctc tcttttaaat caaaacaaca catttaaaca gaaatcattg tctggatttt 129360
ggagcagatt ggggaacgac taatgcactg taaaattttg aatcaataaa gatttttgac 129420
attgtaaaac ttgggcatta ctcagttccc ccaacttcta aggcaattct agctcctata 129480
aaaagtcact taacacttct caacaatatg actaaaattc agagttttac aaatgtgttt 129540
ttaaataaat ttttttcagt aaaacagttc tagttcagta tacagcacta gcatatctga 129600
agtaagaata ttgctgtttc tgatttgtat ttgaaaatat tttcatctag tcttcttttt 129660
tgccttcctt cttttgaaat aagattcaat attgactgag aattggttct gtcaagattg 129720
tctctggcaa tctgtcccta attcaactat aaattattgc taaggatgaa cacttaaata 129780
actatatatt ttaaatgatt tttaaactac ggccccttag taatgtgatg agcatataaa 129840
tttttgaatt cagggtcttt tgaaactctg gagtatcata tgtatgtata ggtctggaaa 129900
agatgtgttc ctattaagac ttaattcttt ttagattgag acaccatttg caaattgaaa 129960
acaaaaaaag tttgtatttc ccctagtatg tgaatatact gtaaggagga ataaaaagca 130020
aaatagtctt tttaaaacat agactttaat ttttagagct attttaggtt cacagcaaaa 130080
ttgagtggaa ggtacaaaga cttctcatta tattccctgc cccatcacat gcacagtgtt 130140
ctccattatc agcaccccca accagggtgg aacatatgtt acagtggatg aagccaattg 130200
atgatctggg tggtgaatag gtggaacaca gaggagtttc agggtcattt ttagaaagta 130260
cagattagtt ttaaaactaa tgtagctttt tcagattgtc ttcttccact tagtaatgtg 130320
tgtttaagtt tccccagcct taatttttaa tcacacattt tacatagcta aaatcattga 130380
gtttcagagt atcaatgctt attttaaaaa acagacattt attttaaaaa caatctgaaa 130440
accttaataa atgtctctta atgagaaaga actcttaaag atgtatgctt ggtctgttgt 130500
attggaaatg tttatcacca taactatatc catctccatt tcatttagtt ttataaaata 130560
atgaagttat atcattttct gaaataattt ttcttctgaa atgaaagtaa atactcttaa 130620
tgcctaaaaa atactttcta aaaatgaagc tcataaccag attatgaaga atctgtcaat 130680
taaaatgatg tttcagaaaa gtgggaattt tattcttatt tgggattttt gttatgttga 130740
tttaaatgct taagacaatt cgatgctgat aggagccatc ttttagaaaa agataagact 130800
gactgatctt tactattgaa ataaagtgtg ctgtgtaatc aaatgcagta aagaactggg 130860

tttgatgttt taaataactt ttagataact tggaagtctt ttaatttgga aaattatgaa 130920

catcagcaat cttaggaatt tcaataggta tataaccata aagggatttg ctgtatagga 130980

agcacaatac tttcatctga atgtaaacct gaagagagac ttctgcccca ataaaaatat 131040

tttattgcta ctaaacaatc aaaagctatt actgacttca tttggaatgg ccagtcccga 131100

gtgatctgct aagaaataag cagaacttca cttgactagc cttaaaaaaa taaaaattaa 131160

aattaaaaaa aagcagaaca gcatggtttt taggaggctg ttagagaatg tcatagcatc 131220

aagctccaga gaaaatggta cagagtagac atgcaataaa tgtttgtgga aggaaaattg 131280

atggttgaga gtggtgtcaa ttcggaggcc tgcaaagcat ggatgtgttt tgtgttaatc 131340

tggacttttg gaagcattta gtcagattca tttgcatatg agttataatt cattttttgg 131400

ttcttttctg gcattgtggt gttcattgtt aagattttcc cccctacacg tcacccactg 131460

actatgtgat taatttttct tctttctttt ttttttttcc tctgaacttg ggaaacctca 131520

gctcttggct tttccttatt tttccaacac cccctgtaca cacatacaca cacacacaca 131580

ccccttttgt atattttttt gagacggagt ctcgcactgt cacctgggtt ggagtgcaat 131640

ggcacgatct cggctcactg caacctctgc ctcccaggtt caagcaattc tcctgcctca 131700

gcctcctgac tagctggaat tacaggcgcc caccaccatg cctggctaat tttttgtatt 131760

ttttagcaga gacggggttt ctctatgttg gccaggctgg tcttgaaccc ctgaccccgt 131820

gatcctcccg cctcggcctc ccaaagtgct gggattacag gcgtgagcca ccgcgcccag 131880

ccctaacaca cgccttttaa aaaaactaac acatttttct taccaggcga gtcctcattt 131940

ttgataccca taaatctata tataaatatt ggtaaatctt ccgtgaaaca catacacaca 132000

gtccagaaag tctctctaat acctactttt tcagaatgct ttgtgtgtac tcctgaggtg 132060

ttttaggagc ttcgtacttt gctaggtagg gagttcttta gggtaaaatt gttagaaatt 132120

ttttatcttg gttaagccaa aatcttttac caattgaact tcttaggtgc attttgttaa 132180

acacatgaag accacttgtc tgttaacttc tggatgtaga tcaaacatcc tcacttcttt 132240

gaaccattcc acacttaaca tgattttgag ttctctctca gtttaattct ctttacacat 132300

attttgactt gacgttgcct ttcttaaaat atgaagctca cacttgaata taataatcca 132360

catatgatca gataatagat gtcttttatt aaaacttgtt actaatattt gtattttccc 132420

ttatgtttat agagttcttt tataaaaata tcttgtaggg tagttatctc cattttataa 132480

atgagaagca gagtggttat cattcgtgat ttgcctaagg tcacacaatt gctaatttag 132540

acattgcaca tctatttata tggtcttaat gttacttttt taggcgttcc attacccagt 132600

tgccagacca agtatcataa gcctatttaa gtaaaaccac tagacctttt cccatacacg 132660

ctaatggtta gatcttttcc attctaccct ttccaaatgc atttttaaaa atctaatgta 132720

tatctgttaa attgtatgtg tttggatttg gtattgttag aatgtatcct gtttgtatat 132780

ccccatctta gaagtttgtc ttatgaaaga actatgtgat tataaagtga tagatgcatg 132840

ttattaataa cactattaat aacatttgtc aaagaatgtt aagagtttta atttaagaat 132900

agttgtagtt tccactattc tgtcatctgt agaattggct ttccgtttgc atatttaaat 132960

gaactttgtg gcttttgtta agtataataa aaagcatgga gtcaaatata agccaagagt 133020

attacagaga cttttaggct gactcagtat ctcaagttct gtgtagattc atctaaacac 133080

tgctgttatc catgctatac tttaccatgt tatcccaaaa gggaatcatc agcaaatttt 133140

accagaaact gctgaattca agatatattc aatatatatt atacttctga catcctagga 133200

agcctatcca aagaatacat tactttgata gaatttgttc tttatgaaaa ttcattttga 133260

ctctcattga taactttatt ccattttggg ggaggactga ggagtcagtg ggatgggaac 133320

agagctaact acaaagtctt tgagtttaga tgggcagcag aaggggaaag gaagtaggcc 133380

gtgggatata taaggacttt tccaatggaa aacaattgtc agtggaacct ctatgactac 133440

ttgttcaatt tcagaattaa acttcctgta tattttaggt ggaatcaagc tgagttctag 133500

tcaaaatgct acgcattatt tcccatgaaa aataccccca aacacaagca gacagaacag 133560

tggttgataa acccatcata ttcatttctg aagaaatcat caagccccaa atctgtttta 133620

gaaatttctc aagaactaat tctagactgg gcatggtggc tcaggcctgt aatcccagtg 133680

ctttgggagg caaggcaggc agatcatccg aggtcaggag ttcgagacca gcctggtcaa 133740

caaggtgaaa ccccgtctct actaaaaata caaaaaaaaa attagctggg cttggtggca 133800

cttgcctgta atcccagcta ctcaggaggc tgaggcggga gaattgcttg aacctgggag 133860

gtggaggttg cagtgagccg agatcgagca ttgcactcca gcctaggtga cagagcaaga 133920

cttggtttca aaaaataaaa ataaaaaaat aataattcta agcatgttgc ttccagagtc 133980

tgccatttct tccattttgg gtatttttct aacagtggtt ttgtgctctc acctgtgagg 134040

attaagagcc tgggataaaa ctcattttca gtctaaagat ttaaactcat ttgaattgag 134100

tagatgcttt tgtgatgtct gcatctctcc tgacttaata gttcccttct tcacattact 134160

taatctgtcc ttttggttag aagatcatgc tcttacctaa agatagaagg aagcaaaatg 134220

aacatggaac tacattgccc tgccttttct cctgtcaaat atggcatcag gctgagcagt 134280

ggcttttcct gatattcccc ttattcaaaa catagctttg tcttgctttc agcatttttc 134340

actttcctca gatcattatg gattctgact ttaatccgta agaattatac ttttatatac 134400

aagtaggtta tatacctcta cccatctttt ctaagctttc tttgcaaatc tagtcccatc 134460

agattctccc tgtgtaacaa ccaacttctt tagatttcct ctaattttct tttctttata 134520

gatttattta ttattgcata gtcaaaatgt catgttttag catcttctgt ggcctgtgaa 134580

tcactgctct ctataaccat gggaccactc ccacccatcc ttttcccgat ctttttttgg 134640

atagttgttt tcttaaaaaa tctggaagca ggctgggtgc ggtggctcac gcctgtaatc 134700

ccagcacttt gggaggctga ggctggtgga tcacgaggtc aggcattcaa gaccagcctg 134760

accaacatgg taaaaccctg tctctactaa aaatacaaaa attagccggg tgtgatggtg 134820

tgtgcctgta atcccagcta tttgggaaac tgagatagga gaatcgcttg aacttgggag 134880

gcagaggttg cagtgagccg aggtcgcacc attgccctcc aggctgagcg acgcagcgag 134940

actccgtctc caaaaaaaaa aaaaaaaaac atggtagcaa acgttcaggg ttatatgttt 135000

gggaaacata cagtacctac tgaagttcag gttcaggcat tgtctactac atagaaatga 135060

ttaaatcgag gtccttttcc tagaggagct caaagtctag ttgtaaaaag tgaacatgta 135120

aacaaatgat tgcaatacac tgtggtattt gcaaaatagc gaggatgcct gccatctaag 135180

aactcaagta taattattcc cattccctgc ttttgtaaat cctaaaatta tgtcacgtcc 135240

tcctagggtt tctgttattt ttgcagtagc caaccagttc ttcgttggtc agaattaagt 135300

ccagaataaa agttttcttc cttattttca ccttggaaaa gagagatcgt cagcaaggaa 135360

tgttttatca ggtttatttt cttctagcag atttagactt cagagatgtt agggtggttg 135420

acaccttcca tgtctctgca attttgtcat ttgttttggg gaagtcatat tgggtgaata 135480

ttctggtgat ctgtgtattg tctccttatt ttacattccc ttgaccatct ttggatctcc 135540

tcacagggct ttgttcatgg tcactgctca gttttgtgct cacttaaatg cttccctcca 135600

ggctgctgct ttgacattgt aaagatcttt tatatgaatc ttttttgtcc cttgccctca 135660

tttagcgttg cttacatatt gtggttcggg tctcatctga tccagtctgt gatgcttcta 135720

agggtgtatg tatcttttat attcaaattt gtttactttt tcctcttttt ttatataact 135780

ttgcacatga gtatttttta tttttttcta gattctgatt ccttagatcc ttcataaagc 135840

agccatctag attcttctgg ttctcattaa tctgattctc cttcaaaaca gatttataga 135900

tccttatagg aacattctag aagagaataa tatacattat gccaaacaca tctacttata 135960

ttttgaaatg ttcccactgt atttttttat ttgctgcctg tggcctgtgt gtccctgtgt 136020

tctctatggg atgccctttc cctcttccac atcagctgct tttcctctga tgttttctta 136080

tttgccgttt atcctatatg tagaatgcaa gaattattca cttccttctt tatgccccac 136140

aggtctttgc tcatactaga atagcattta ttatcccatg ttgtagttat ttaaacacat 136200

ttacccttcc cttctgttag cccatgagtc ctgcccagga cacagactgg ttggtagtat 136260

gcttgatggc aagaacaaat gctttggagt cagactgatt ttaattgctg actgcaacac 136320

tttaactcca taacctggga caaattcttt aaactacagt gcctactcag cctcctcatt 136380

tgtaaaatga acctactact taacctcatt ggattgttga tactaaagta attgagtaca 136440

taaagtgcat agtgagtgaa ggaatagtgg ctgaagtaga aaaatgggta gaatatatct 136500

tagagacttt tgtttaatat cctgcacaca taatagtgga gtatttgttt ttaataaaac 136560

attccttctt taaacagttt tgatgcgttg gactttttaa aagtatattt gcttgtgttt 136620

tggcatctga aatgctatta aaaacatatc atcttagtgg ctcatttcct tcagtttaca 136680

tgcagagaag cctcttggac cccaaaactt aacttactcc tcctgcattc agaaagcttt 136740

gcatcttttt ggagagaacc atctgttcac agcattatac agataggaat taaacagatt 136800

ttgttgggga aagtcacttt ccagctcttc tcaagtcaga ccctcaagtc tgcagatatc 136860

ttgatgcatg taacatttct aagacactga gctacatgct gaagaatgta caggatgtcc 136920

ccatcaggct tttcagcgga gttgtgagag gtgaagtatg agtacatgga aaggtggaca 136980

gtaactgcag tcgatggcac ggcatggtgt gctgccagat gaatggttca gaccctaagt 137040

gctggccggt ggaagagaag agacttcgac ttttggaaga ttgaataaaa actacttgaa 137100

gacaacaaga aagcattcta gacaaggaga atggagggaa tagagtttag aaagcacaag 137160

tcatatgtag cgtgtctgga tttttctgtt tatgtcattt ctactagtat aaacaaatat 137220

tgcctttttt gttgttgttg ttttgactgt tactttgttc aggttgtttc tgctcagaac 137280

tcttttttta tttgggttct taagattttc cacttaatcc aaattctact ttcagatcta 137340

ttaaaccctt aagattttgt ctgtgaccta gtggagtatt ggtgtgtctt gcattataat 137400

aaacatagtt gtagaaattt gagtgtaaac tatttaacta gaataacatt tttcataaac 137460

tgattttata atgtctgtaa caattcattt actaagaagt cattttctct gcatatttta 137520

gttaataaat tggaatgatt tttgtgactg ccctgatgta cagtacactt atattatgaa 137580

ggctttcttt taaatttcaa tatgacaaat tttgtctgct ctttgaagtt tagttcagca 137640

taaatcattt gtatttgagt cacttagaga ttcattgttg aggtttgagg aagtttagac 137700

tttgttcaaa ccaagacttt gttcaaatga agatgcactc catcccaaaa ctggtaggag 137760

atcacttata agagggaggg gtgtgggggc aggagctaag acaggttatt ccctatgtcc 137820

ttctaaaaat gcacaaaggt ttgaaagtct aaatggcaaa gagttaatca tagtaggaac 137880

caggtttctg atgtgtagcc acttgcacca agaaagattt gtttaagtaa cctaaccact 137940

ggagcctgtg gttttgaact ataaaggaca agagtgaaga tggaagacaa aagcttttta 138000

gaaaaacaaa atcaggttgt attgagcctt ttggtaatga ccattaaata tggctcaaaa 138060

actgaagtgc agaatgaaca cttttaggtg tattttgtga ctgggtctct cagtgactgc 138120

ttcccggtaa gggtgtattt attatttaat gagaagacct tgaggagggg gtaccaaaaa 138180

taaagtagag gcaggaattc caagacggca caatatcctg atttggtggc acaaaaatat 138240

attcgtggaa acaaatctac tggaattgtc ttgtttgggg gcttgtttta catgtgtttt 138300

atctcactat gttgtcatta tgcagaggtc ttctatgtct ttaccctttt agcagcagat 138360

cttatttttc aaattaaatc tcacatggta ccctaataca taaactacat gaaatcaagg 138420

tgggaaggaa ggatgccttg tcctggtacc ctgcgccctc aacttcctcc attctttcag 138480

gtgaagcttt tatgccagct ggaaaaaccc attggcttgt tgccagtgac agcttactgt 138540

taggagcagg ttttcccaca ttttgctatt tatagtttta catttcataa taattaacat 138600

attgcagtta acctggagaa cagaaagata cccactaggt gctattaatg ctaactgtgt 138660

gtgcttgccc aagcagtgtt ctgtgaaagg tctgatgtca gaattcattc tgaaatatag 138720

tctataatat agtcattgtt taaatgcctt tctgtgtact tcctagtaaa cagttggact 138780

agtacagtgt tatgcagaaa tactaaagat agtttcaacc tgttgtgaga ttttcaggtt 138840

tggtgtggga ggagggggag aactatgtag taactaaaat ggcaactcca cgaatgctac 138900

tgttcttcac ccaccaacac gttcccgtac gtgtcccaac tggcctgtct ttttactgaa 138960

cttatgtagc atattttaat catataccac cttggcattt cttctgttgc tttttcagat 139020

gtagttgttg aacttttcat ttattttcat tgtcccactg tctagacttt tagccccttg 139080

aaaacagagg tcatggttta agtgcttttg tgtttcatgt acactgtagg cattcagtaa 139140

agagttgctt tgatttttag cttggtggtc ttaatatatg cattttatac tgatttttta 139200

aactgttgtt gttgctgaaa actagatttg tcagcttgtt tagtgcatca tgatccgctt 139260

actatggatt ctgctacctt gaccagcaag gtagcagaat tttaactata atttcttctt 139320

taataagaga ttttctgagg caggtaaatg aagtatactg atacttctta cacagtggaa 139380

tatgaaagtg tgacacgtcg catcttgaaa gtactttaaa ttgtgtgcct ttgagttcta 139440

cactgaaaaa taaaactact accaacaact tgaccccctt tcctgccctc aaattgttta 139500

gaactgaaaa tagtgttttg ttgtacatat ttatattcca taaagtaaaa ttttttaaca 139560

tcagaaaaca tctaaatata ttatcgattc cttaagaata aacactcatt ccctgatgta 139620

ttatactatt actaattact ttaaaaataa aattagtatt gtttttaata taaaatgttt 139680

ggtaacttga tttttattat actttgcttg ctcttttcac atcttatggt aagtttaaag 139740

tactttctga tgtcttgtct ttgtaatgtt ttagtgataa gaagactcat tattagattg 139800

actacacagt acctctgaga atgatgagtg ttattcctaa gttcctaaaa ggagactagt 139860

acacagggct agttcctggc agtaagaaat accctgcctt agctttttgg aaaatttact 139920

atccgttcgg tattgaccac cagtggagca tttgttatta ggcatttagc aagtaatgtg 139980

ctccaaattg catttccttc tcttattttt atccttatga gagagtaagg caggtgttat 140040

ttttagttat gaattaggtg tcctgggatg tacatgccta aattatattg acagccagtt 140100

gtggagatgc cttatacaac ctatctacaa tgttttctct atgagacctg ctgacttata 140160

cacgttgaat tgtactccag tgtgaagtcc tgtataaaga aaagaaaatg aacttgcatc 140220

aggttgagtc agtgggcaaa gtcctggtac tatttgaaag gaaaatgtgc attcaagcga 140280

gatcatataa gagccctgga agcaaaggaa tcgacttcct ttgtgtcagt ggttctcaac 140340

tggatacaat tttgcttccc tagaagacat tttcaacgtc taaagacaat tttggctgtc 140400

aaaactgggg agttggtacc cctagcatct agtgggtaga ggccggcaat gctgctaaac 140460

ctcccacagt gcttaggaca gcactccaca acagagaatt atgtagttca aactgtcagt 140520

ggtgatgagg ttgataaacc ctgctgtatg tgtttcaaag aatggaattg ccagaagccc 140580

tcgcagccaa gacaaatttg ggtcattggg agggaatagg gaatcaagga ggtgacttca 140640

ggtatggccg gactgagatg agctattttg atgggattag gattaagctg atttcctttc 140700

tctcaccttt tctttgtgcc agcacttggc atagcaatag tggaaaaaac agtgaaataa 140760

acttcttttc ttctaagcta gatggaatat gatcctattc caagagctaa aagcatcatt 140820

ctacttccct gtaacacttg cagctttact gtaagccaca tccatgctgt caggctccca 140880

aaatgtcaag gcacatgatt ttttgggggg aggaggggtg tcaagattgt tgcattataa 140940

actagctaat ttatttccca aggtatcctg taatcatagt gcacctggga ggatctaaat 141000

ttctcactgt ttctgcatca tgcgccatat gtgaatttct ttggctaaca tacccttgct 141060

tgatttgatt agtctacaat tacaggaaca aatgtccctt aaaacaaact tttttttaga 141120

gtagttttag gttcacagca aaactgagtg ggaagtacgg agaattgtca tataacccct 141180

ctgcctttat tgtcttttga ctttattact aattttcaaa taagatttat gtaaatgcaa 141240

gtgttattct gttaatgcaa aattaaatgt tcctcaattt cattgctcac taaatggtaa 141300

agcaaaagtt aggagcagat tgttctgagt tatgggatat tctctctctc cctattcagt 141360

agaagaggga ggatcatttg catggacttc ttagggacat tagccactcg gtgtaagatc 141420

atttatcttc ctgaaaatgt tcccatgtag ttatacatca tctttttatt tctgggggat 141480

tttatttttc ttataggaag ttttccctgt ttggcttttg cctaattgga agctatttta 141540

agttattcag catgacagtt ggactgcttt aaaagttaga gtgtaccata gccgtaaacc 141600

ttactgtgat tgagagtgtt ctaggactta tatttatgat cttgtttgtg tctcatgtag 141660

ttagggagcc agggggaaac tagtgattgt attcttgagc agaatttttt tctcctggcc 141720

gcaaagtcca gtttcttcct ttagtcacaa tatctctctg ctaatcatgg ccagtggaca 141780

cactataatg cttctctgtt tctcttttgc ctcctttcaa tcatgggtct tcccctgggc 141840

tttcccttta atatttgacc ctttaaaaaa ttaatagtct gtacagagta gatcgagaga 141900

gtaagcttgc ttgctttttt ttggacaggg ttcaggttca agttctcaat aacaagtttt 141960

gtgaacttgg ggaaattatt cctgagtctg tttctttatc tagtaaatgc agattatcct 142020

tgtctgcctg cctctcaggg ctgttgtgaa gatcacatga aataattgac tatgagagtg 142080

ccttgtaaac catacaacac atacaaatgt aaaataatat gcaaatataa gaaatgtgag 142140

gtttagtaaa ttacatcaat tatgaaatga aagtagggtt ggagaattta ttttcccttc 142200

tgttgtcttg ttttgtgtag cctagagtgc tttttcttca tcttaataag ttcagagatg 142260

accttcgttc agtttagttg tagtatttta tctttttata aataaggtca atggagttaa 142320

atgcttcact tgttgaaaca tgatataatt tggcattgat cagtggttgg tgcaaagcaa 142380

aatttcaaaa agaaaattct gattttgcta agcagcatag atcgtgcctg ttagaacatc 142440

ctcctcctcc ttctcagcgt tgattgccaa tcagttctgt agaatcagaa atatttttct 142500

atgctttttt caaaaactcc agtttgtaat ttgacttaca ttgctatttt caaaaataaa 142560

acaactatct aagtatttgt taaacaatta ggtagttgtt atacttcacc cttttccccc 142620

tgaccagtta gttatactat gtggattctg gatggtagta gatcaagtta gtttagtcac 142680

aatttataaa aagccttttc tatgttgcta gtgtcttctc atgtttgata ctattcataa 142740

atagacccag aaaactgtag gactccggga ctcaggctgt gaatgattgg tagaactgtt 142800

ggagtagagc aggcagactg tgttggaaat tattccatct agaaggtatc agctcctgcc 142860

tcctcggcct tagtgactaa atggaaatac attcttttca aattcaaaag cctgttcatt 142920

actgcttttc tcttctgaag cacattgcca gttttataat gaagaaggac ttaattattt 142980

ttctttttct attgcttatg tttattcttc ataaaatgac ttttcttcca tttgtgggct 143040

gagaaataag ctcctatatt ggactttgca gacttggctt ttttatagcg taaatcagct 143100

cagctggcta aatgcctcac taagtgaaac ttcataatgt agctggaact aaattgtgct 143160

ctggagcatt gagtctttaa actggattat tgatttacaa acccataatg gtgaagaggc 143220

tatagagaga caagaacctc cctctcctga tccttgagca agtttttcct cacttcaggg 143280

gctctgagct cacgagcaaa atcctaacca agtttatctt gctaggacaa cccattttaa 143340

atgctcaata aagaaagaat aattcttctc ccgcttaacc actaaagaac aacatatgtt 143400

tctaacgtag ctttttattt tttttatctt ttcatttttt tgttggtaaa tctcccagac 143460

aacagcagtt ctgtagagag tttaaatatg aaggaatggc aggacgggct aagggcactt 143520

ctacccaaca ttaacatcaa ctttggtgga ctgcccaatt cttcttcccc ctccaacgcc 143580

aaccacagtg caccaacgtc caacactgcc accaccgaca gcctgagttg ggacagccct 143640

ggcagctgga cagacccagc catcatcaca ggtaactttc tcactccctt cagctacact 143700

gactgccaac agtttggttt ttagcacaga ttaaataaaa accgagatct cttcaacata 143760

agctgctttt cacaaagccg caagtgtgga gtggcttgtt tcgttctcag gggtcatgca 143820

agtctcattt aacctgtcgg cctagttgtg tattaataag aaggcatttg gtgcctaggt 143880

tgcttcttct ggtttaagta ggtgttaaca tctggcaaca tgtgtatttt gtaggcctca 143940

aagtacaaaa ggatctgact tactcttctt ataaggaaca tactccactt tttgttataa 144000

agctctctat tataaggctg tcttgttacg catccctgtc agaggaggaa aagcacaatt 144060

gggaaatgcc atcaggcagt tctggggaat ggtgttttgc agctctgcct ctgtgacagt 144120

gggggactaa gtctagttca agaggaggca tgggtttgtg tgccaataag ttctttgttg 144180

ttgggggagg attggtttaa aaggtcttat ttggaaggag tttaaaattg taatctcttc 144240

tgtctttcct tttggttttg ctttctaaat ttgtttgacg accttattct ttccacacaa 144300

actcaccatt ttccctcttt tttcttttct ctccttaggt attccagcgt cttcaggaaa 144360

cagtttagac tctcttcaag atgacaatcc tccacactgg ctaaaatccc ttcaggccct 144420

cacagagatg gacggcccca gcgctgctcc atcacagacc caccacagcg cccccttcag 144480

cacacagatc ccgctgcaca gagccagttg gaatccctac cctcctcctt caaacccttc 144540

cagcttccac tccccacccc caggctttca gacagccttc agacccccca gcaaaacccc 144600
cacagattta ctacagagtt caacactgga ccgccattag gcaaagagga gcaaccatta 144660
gaaaatgcag gatttgtccc actctgtttt ctccctctca gcccaccacc cacagctccc 144720
ttctcatctc ttatgttctg aagaatccag tacctgatca attttttttc tccctaaccc 144780
cagatgcaat gcgatcacag ggtcattacc atctctttat taattgtaaa aatttttgtt 144840
gatccagcat ttgagtgaca ctgttgaatg tttctaaaaa tgccttttta aggagagaaa 144900
aaaaaatcaa agggcagtct caatacttag aaaattattg tttgtctgtt gcgcataata 144960
atgacataat ctgcttagca gaaaatgacg attaatctat aggaaagctc aagtaaatgc 145020
attatcaact gcagaagttt gaaaaccagg ttcatttacg tgagattgct aaatgcatgg 145080
gggaaagcag tggtcctagc atccatcttg tattcagctt atcattattg cagggaaaat 145140
gcttttaatt taaattaatt tttaattctt ttggcaagtt gatggcaagg acttgattgt 145200
gtcattaagc aaaagaatgt attggaagtt gatggaaaga caaattcatc tgtagtagta 145260
actggccgat tgctaaagag ttcataagga ggtgagaagt aattttttta aaggagaaaa 145320
attttttggc tttagattta aagtaaattg aaatgtttta aagaaaaaag tattcacaga 145380
tttaatacct attaataata taagagctga aatgtaagtc atttcttcag tccttctcct 145440
ctgtcggaat cttttttgtt ttaccataaa ttcacctgac gagggcactc tgagatagca 145500
ctgctctggg gccatctgat caccatcggg agcaaatctc tgacctcctt gcctgcagct 145560
tttacttaac cctgtagttt ctggacgttt gtgcagtatt gaaaagacag gagaaaagaa 145620
aacagaaacc tggttataac ctgacgctaa aactaaaaac aaggaaatgt acctctttct 145680
tcagaattaa aactaaaatc ttaaataaaa cagaaaactt gatggtgaca cttgggttgt 145740
ccttgttttt gtttttctgt tttgttggat gtgagtttga aaggttttgt gacaagtagc 145800
catcagatgt ttccatttga ttttacatct tcaacagtgg ggagggagga tggtttagaa 145860
gaagaaagtg gaggagaaac aaccatttta ttgacagtca atggcatcct tgacgttcag 145920
cccatcttgt cctcaagaat ccctcttcca gtgcctttca gtagaagatt cctctttctg 145980
ctattgtatt atgcatgcca agccttccca actgagaagc cttatgtgcc agtaatggag 146040
aggttattga catgttgaga tgttggttct ctttagggag acctggcagg agcagcagtc 146100
actatgtcac acaagtggca tctctttgtg agtgccatga tgggaaagag atcgggaaac 146160
actgatgtag atgatccaca gacacatctt ttatgactga ccattttagg aagtacctgt 146220
tgatggggca acgatcgcac cactgaccaa aagagggtag aggatgaaag ttacctgttc 146280
cccaacagag caccaggatc tgtgtggttt gtatgtcttg ccttgggctg cattcagaag 146340
cccaaagctg gaactggcat attttcagcc atgtccatta agggatgtga tgtaggatca 146400
actaaataga tctagatcgt acgttctgtg ctttcaggtg ggtttttttc gtccttacct 146460

ttatgctgta ctttaatttg ttaaaatttc aacacaattt ttagaaactt aaacatgata 146520

ttctcaaata aatgtcacca gaaatagatg gtgatcaagt ggatagtaaa ttgttttgta 146580

aaactaacaa aatttccctg gataagagga gaggactaga aatgacaggc tctctttgcc 146640

cttgaacttc acttcagtct cctgaacctt cacattgtac tgcaaagtga tggaccaatg 146700

cacaaataat attcagatgg cagtgaattg taatcaaggc tttttgcggg gatgcgggga 146760

agtcctgaga tgggcatatc aataaaaatg ttgctttttt tgtaaaagga gggaactcct 146820

accttataag gctgtgctgt aattgtgtgt gtgtttaatc agtcatacag aagagtttat 146880

aaaaagcatg actttataaa aagtatgaag aataaaaata ataatgacct gtgtgtctac 146940

caccaaggtt aaaaatagag gccaggcatg gtggctcatg cctgtaagct cagcactttg 147000

ggaggccgag gcgggcagat cactgtaggt caggagttcg agaccagcct ggctaacatg 147060

gtgaaacccc atttctacta aaactacaaa acaaaaatta gctgggcatg gtggctcgtg 147120

cctataatcc cagctattcg ggaggctgag gcaagagaat ctcttgaact caagaggcag 147180

aggttgcagt gagtccaggt cacaccattg cactccagct tgggcaacaa gagtgagact 147240

ccgtctcaaa aaaaaaaaaa aaaaaataga acctctggta tcttaaaacc tctatttgcc 147300

cctctccagt tagacttcat tttctttcct tatcgtttca aatagccatt attttaattt 147360

ctgtgttaat cttcaagagt tttgccagct atgtatctat ccctgaatag attgtttagt 147420

tttaaaaagc tgagtaataa acccaagcct agctgtagaa ggaaagggtg atgttgtcac 147480

tattagatta agacttttag gagaagataa actaaatagc ttctggggca tagtttggct 147540

ttagcgtctc cgatcattta tgaaagcctc ttagaagtac agtggccaag gttatgagtg 147600

agtcctggaa aacagggctt gccagttggg gtaggtcttg tcaggtaagc acagtcccgg 147660

gtgtctggca gaaatatggt ctacaggatc agtttgctgt ctgtttcctg tgtcaaagct 147720

caccatggct tccctccagt cctctggagt agatcacaga cacttagtga attaagtgtc 147780

ttaacccttt ggggtgagag gtggcgttta aaacaagtgt cagctgatta attcaccagg 147840

gcttggcggg gagaacagaa acttcattag gcttggtcaa aatcagaaag tacagaaaag 147900

agcctgaacc aaactgactt aggtggcctg ttggtataat agcagtggga atcttggtac 147960

tgacagctgt catggatcac agtactcaga tctggtacac agcagtcatt tctagtattt 148020

cactttgttc tctctccttt cttagatgat cttcctatat tcctagatcc cacaccctag 148080

tcttttattt atttattttt ttaaatctca ttttgctgga gtgacatcct ccagtatttc 148140

ttaaggaaag gtacatggga gataaatatt ttagtaattt gcttgtctga aataatattt 148200

aattctactt ctcagttgat agtttggtaa atacaggatt ctaggttgta gatatttttt 148260

tctgcagaaa tttaaaggct tattaacttc ttgcttccag ttttgttgtt gaacaatcca 148320

aagatgctgt tatcctctat atgtaatctt tctccctctc tggaagcttg tagcaagttt 148380

gtcttctgtg ttctggaatt ccatagtgat ctcccttggt gtgggtctat tttattttat 148440

ttatttattt atttattatt ttttttatta ttatttattt atttattttt tgagacggat 148500

tctcactctg ttgcccaggc tggagggcag tggtgcgatc ttggctcact gcaacctccg 148560

tctctcaggt tcaagtaatc cttctgcctc agcctctcag gtagctggga ttacaggtgc 148620

ccaccaccac gcctggctaa gttttgtatt tttagtagaa acagggtttc accatgttga 148680

ccaggctggt ctcaaactcc tgacctcagg tgatctgcct gcctcggcct cccaaggtgc 148740

tgggattaca ggcgtgagcc actgctccca gccaggtggg ggtctatttt aattcatttt 148800

ggtggacaca gtggacatac agtatgcttt taaaatctta tgactcattt tcttcagttc 148860

tcggaaattt tcttgaatta ttagtgaact tcttgcactt actttctctg ttctcccttt 148920

ctaaaaattc tattatttaa atactagact ttctggactt ctgcttttta aaatattttc 148980

tccaattttc tatctgtgtc tttggctctt ctttctagga agtttttttc tcaactttat 149040

agttttcttc tccttgtgtt gtctgtttct ccaagttgct tttatctgtt tgctttaatt 149100

tctgtcttcc ttgttagaga cttctacact tgatcttagc caaaaggcca agaactgatt 149160

atattagaga ctccttagat gcttggtaat ccttggttat ctgctcatga atgagactga 149220

aaaattgatg gggagttttg aaaacatgag tagggcttgc caactttgaa ttcccctatt 149280

ggaagatctg ggtggtctat tctactgggt aacctctgtt gttattgtct ttagcacttg 149340

tctgttggcc ttggctggtc agattccata cagaagagtc ttataaggac tggcctagct 149400

ggtaagggtc tgactgccag ggcttcagag ctgtgttggt ggtctctgta tgtatttaat 149460

attatactac atataatctc tctgacgtag tctttacctc gccagagatt aactctccaa 149520

atatctgccc atttatgaga agaatagcat ggagttgggg aatgaatcta gagagttaac 149580

ttcttaaaac aactttctta gtaatcttta ttttagcccc actgcacaca ctttatctct 149640

ggttttagtg gtatctggtc ctgtcagttt ctgcgccctt gaggattttg tgatgtaaat 149700

tggattggat ctcagttttc cccactgtta gcttaaggtt ggactttctc aaatgagcta 149760

ggtgagtcca tccaaccatc tactttctag gtccttggat tttattgctg tcatttcctc 149820

ttgttattcc tgtttttctt tttttcttac ttgaatttta atggaccttt aggaagaagt 149880

aaaattaggt atgtaagttc catctaccat cttagcccag atctgtgctc tgattttata 149940

aaccagtagg catagtttgt ctgccagtga agtagagtaa agtagaggaa gcactatctt 150000

ttgctattgg ccaatgagtg ctagccacca tgttgtatat cccttggttt gaatagtacc 150060

tgtttgtttc atttaccttg aagtggttta tctttttaac atcccctttt ccaatgaagc 150120

tcttttggtt tacagtcagt agtttgtttt tcaaggggta atcacacggg attttaatgg 150180

ttaaacttta ggctggatgt taaaggcaaa ttttactgca gttttagggt catttgcaag 150240

tcagatggat gtgggtcatt ttctaggctt agctgaatta gaacaagtct agaaattgca 150300

gagagggaaa aaataaccta ctcttactta tgtatgctgt gtatgaattg tgttttaagg 150360

atgcataata gtaactgtca acaatgtata tctacagaga ttatgctata atataataat 150420

acagagctga attcagttag cactctccaa caaggtttgc ttaaacttga agacgcaagg 150480

tatactgcac atagcttttg agttttatgt taaatattga ccaacatcat ttaattttag 150540

atccttatta ttatagtatg taaactctta aaagttagaa aacttacata tcttacacat 150600

aattttgaga tatgcccttt gtcagatgta tttacttctg cccaagtgat ctatccaagt 150660

gaaagatgac tgcaggtcat tagcatgaac tatttgaaga aatcaaatat ttatcttgtc 150720

ccaaaaatca gtactctatt ttttgtgctt gttcagtaat tgttaattta gttcagacag 150780

tcacaggcag ttttccagca ggaagagctg cctttgaaaa tacttctttt tttttttttt 150840

tttttttgtt tgagacagag tctcactctg ttgcccaggc tggactgcag tggcacagtt 150900

ttggctcact gcaacctccg cctcctgggt tcaagcaatt ctcctgcctc agcctcccaa 150960

gtagctggga ttacaggcct gcaccaccac tcccagctga tttttgtatt tttcgtagat 151020

acagacaggg tttcaccatg ttgaccaggt tggtctcgaa ctcctgacct caggtgatcc 151080

gccttcttag cctcccaaag tgctaggcat gagccatcat gcctggcctg aaaatacatt 151140

ttaaaagttg tttctgaaca aattgattta aggaaaatga gtaaattatt taataaattc 151200

ctttaaggat aaaataatta tttttattgg tgataaccca gatgatttgg gaaagaacat 151260

gcatcttggc ccaaatgttc ttggtacacc ttggccattc tcaagaatat attctgcgtt 151320

tagtccatct ctgtgggaaa ctgagggtat taggcaggtg ttagtaggtg tagtcattgg 151380

acctccttcc atggcttcct ttcatgggtg aaggaagtaa gtgtacaaga tcaatagaaa 151440

tgaataggcc aaatagatat ttttggtggt ctgaatgatt taaaaactct aataaagaac 151500

tcaagagcat ttaaagggaa acaataatct gtggctttct tattttcagc tttagtattt 151560

gcaaagctaa agtactctta ataaaatgcc tggcatatga catcaagtcc agaggacttt 151620

tttaatgttg ttggtttcgt acaatgaatt ccagtactct tatcagatcc catcatgtgg 151680

gaaaagtagg caaacaagct cagagatgtt gagggtttgt cctgggtcac aggttttata 151740

gccgagctag aactacttgt tacccatcct cacctacatt tgcatgactt tggttgtcct 151800

tcctttctgt ttcaaagttg caagtgcttt tctagctttc ttgtttatgg aattaccaag 151860

atgagggtgg tattatttgt ccctccctta tattaggtaa gtagtggtga gtgaaacggt 151920

gtgaagagtt tctgtatttg ttcatcccag atcataaggt atgagtcaaa gtttagttgg 151980

gaagtccctt agggacgtct tgattatttt gtgctcatat tgagttgtat ttacattcag 152040

ttgaactacc aggttagcca gtatcttctg gattttatgt agctaaggaa cagggatgag 152100

atacttgtct tccaatgttt gaaagacagt tatgtagaag aagaaatagg atgaagcatg 152160

cagcttagac taagacgcgg aagagagatt tgggttcaat ttagttttaa caatttttaa 152220

aaacaccttt aggctcagca ttatgccagg tgcttcaggg ggtacaaaag taaaatgatc 152280

cctgctctca aactgaaaag gtttggatag tcagctctaa aactgaaact gagtgtgaga 152340

agtgcaaagt gccaaggtag ctcaaagaaa gagctttgga caaacttgag acttggattg 152400

gagggaaccc tgaacaaaca aatacatcaa catgagcctg cctggggcat gtttagggct 152460

tgcaacttgg ggttcctgta ggggacttgt gagaaataag gcgggaaagg tgagttgggc 152520

actgcttttg tttcaaatga caagttctaa gtaggagaaa ggtaggatag gattttgctt 152580

taaattctaa gtgggaatga tgaagatgag agagccactg aggtagggtt ctttgggtct 152640

atgaacccaa atgaatgaaa ggacccaaag aaatctaagg tttggccaca aagggactat 152700

cagtgaccct agaaattgta tgcaaaaatg gtatgtgcat tagtgtttta tcaaattatt 152760

tttaaaatgt gcatgatgca ataaagtcta gtaagctaaa agaatagttc tggtgaaggt 152820

tttgggctga taaagggatt gaaaatggaa agaagaagaa agaattcaag agagatttca 152880

gaagggctag agatttttgc aagttactag ccatagttaa aggatgaaag aaaaacaatt 152940

tcacagccat cttagatttc ttagatcctc cactagactg taagcttttt gagagcagac 153000

agcatagcta gcttgttaaa cattacattc tcagaaccta gctagcacat agtaggctct 153060

caataaatat ttcagtaatt cttgcctaga attactgaaa tatttgcata tttcaatact 153120

gcatattgaa atattacatg ttttgagcca gatgattgga ataattgtat catgttgaaa 153180

ataaatgtgg actcatgaaa aggaacaatt gcaaaaagat gaatttgttg acttgaggtt 153240

ccagcaggtc atccagccgg tggtgtccaa gaaatactgg gaaatttgtg tgggatacag 153300

agaagaaata cagtgttgga gtcatcttta tagggagaga ggtgagatgg gagccttggg 153360

aaaaacagat gctagtggtc taaaatgaag gagatctgag tgtatggtgt cggtgtagaa 153420

cagtttctag gcaagaatta ctggaaattt tgtgctatgt cagtcagggt ttcatcagga 153480

agccagggat ttcacaacca ctggaagggc taggagaaga aaggtcagga agacctctgc 153540

tggctttcag gagctccaga agtacaggag tcatagaaaa agcctccacc aaaaagccct 153600

cagctgccta cagcaccaag tcggtaattc ccaggaagac tctgggaagt ggctacccaa 153660

ttccccgttt gccaggggcc acctgcctgc ccagagatga cacccgtaga atgatggctt 153720

tttctcttct atctttcaaa ttgccctaag tacctcccat tagtagggtc taagctggga 153780

tcctgttgga aggaatcgga actgtgatgc aggagagagt agcaggggtg gggctgatgt 153840

caacagacaa gctagcccct gtggtttaga gcatctcaca gggtcatgtc acctctttgt 153900

caaaactttc caaaaacatc tctcccccaa taccttcata atgaaattga agttttgctg 153960

gtgtgggacc gggtgccctc tcccctaaca ggaggatatt ctttatttca tgcgactgtg 154020

tctctgttcc atgcatatct gttttctcag ccttgagtgt atatactatt cctatcctca 154080

ccctggcaat ctgcttattg cagctttttc tcacacctct aggcagattt aggtggccct 154140

ccctgccttt gtatcttata tacgccttca ttatactgca acctccttga agaagggata 154200

cttcttttca tctgttatac acagggctta gcacagttcc cgacattcgg ttcgaatgaa 154260

tgacggaagg aaagcagctt tgggtagtgg ggtgctatta agttttattg gcaggagatc 154320
agtcatgaac tttaaggcac tggttttaat ccagcggagg tactggaagc caattgcagt 154380
atgttgaaga atggagaatg actaggagtt aaagaactgg agatagtata ctctgaagaa 154440
gtttgataaa gggacgacaa taacacagtt tcaagtaaag gaaggttttt ttcctcaaag 154500
gaagtcataa gccttttaga ggagacaaaa ttatttgaaa aagtaaattt gaacaagaaa 154560
gctgagttga atggaatcag gaacatgcat agttgcttcc ctccaagata acagcaaaga 154620
aaataagaga gagggtttta agataaagaa aggaatatta agggaacccc ttggcttagc 154680
atagtgaggt agcaggagat ggaataggaa tttggagctt gagaatatag aaaatgtgca 154740
acactttggg gaatgtaaca ggaaggtaaa agaatgggtt aaaagagtag taagcaagca 154800
gcaagggcct agttgaggtt agcatgtata gatccaatca gaataattcg atgtggcctg 154860
gactgttcat caatcttaga gagaagcaaa ggaggcatgg agattaccag gagcttctga 154920
ccgatagaaa tggtggaaaa tcatggagtt gaggctttgg gagggggcga agttatcaga 154980
gtgctcacca cggcccatag tatgaaggta agtgctcacc atggcccata gaaggccagc 155040
agtagggagc gctcttcaat cattctctca atctggaaat ggattggtgc ccacagctgg 155100
gcacacagaa agtacacaac ctattgtcag agcacacagc catacagttg gacccctcta 155160
tgatccctaa agtttctttc aacatttaag attttatgat aattttggga ttactgcata 155220
aattaccaat agccagaatt caaaagtaag tgtaaaattt aacagaatgt atacagaatg 155280
aattatttta taaaactcac aatagcctaa ttcatatatt gaatttcttc cagggatacc 155340
tgtattttct tgttaataat acccacatca ttcttttttt ccccatctgc tcagtaacca 155400
tttattgagc atggaggtac tagactgtga tatgcgttag gtatgcattt gggaatagaa 155460
cagtcataac tctgtctttc ttagagctca gacttaggtg gtgaagatga tcattaaaca 155520
atccagtaaa tatactagtc atatgaaaat gcagaagaat atatttgaag tatgtgtaag 155580
caatatccaa aatacaagac cccaaagaaa aaatattgac aaacttatcc tgaatttaag 155640
acttctgtaa gacaaaacac cataagcaaa attacatcaa caatggactg ggagaaaata 155700
tttgtaaccc atgtaaaaga ctgaggatta atatcccaaa tatataaaga atgcctacaa 155760
atcagtcttt cttaaaaata caaaaagcca aagacaacta atagacaatg attgctgaga 155820
atgttttgtt cttatgatta agaaaaacaa aaaatattgg taagggtata agaaaatgga 155880
tatcttatgt acttttgctg tgattataaa ttggtacatc cttttatggg gaatgaactg 155940
acaatatgaa attgcctgtt cacttcaacc caataatttc cctgcttgta ctcatacaca 156000
gagaggaata cataaaagca ttcattatag cattgtttat aagaagaaaa gtacaaaata 156060
gcctgaatgt tcaacagttc attggtaggc aatactacag aacaccatga gaagaataat 156120
tacatgtatt tctgtgtgca gtcacacgat gaaaaaacta tatgtaaatc tacgggtttt 156180
ttgtttgttt gtttgttttt gagacagagt ctccctctgt cacccaagct ggagtgcagt 156240

tgcgcaatct cggctcactg caacctccgc ctcctgggtt caagtgattc tgcctcagcc 156300

tcctgagtag ctgggattac aggcatgcgc caccacaccc ggctaatttt tttgtatttt 156360

tagtacagac gaggtttcgc catgttgccc agactggtgg tctgagctca ggcaatccaa 156420

cccccttggc ctcccaaagt gctaggatta taggcgtgag ctactgcacc cggcctaaat 156480

atacttctaa agtatatctg cgatattttc tacattaaac acacaagaac taaaactaca 156540

tatttgtatg tgcctatgta aatggcaatg catagacaag acttggaaga gttcatgcca 156600

aaatgatgat agcaattacc cctatgaaga ggagtgatgt ggggttgggg ctggccagga 156660

gacattaagg agaatttctt gcatgactga tactgtttga tctttttaaa ccataataat 156720

gtattaatat attataaaat gaaaacattc atataattaa aaattgtgct ttgaaaggaa 156780

agtataacat gcaataagta tgcaatggag aaactgcttt tcatcagggc atcagaatgt 156840

aaacgcactt ggagaacaaa cagaatattt gactttgttt ttgtttttgt ttttgaggta 156900

gggtctctgt cacccaggct ggagtgcagt ggcatgacta tggcttacta tagcttcaac 156960

ctccccaggc tcaggtgatt ctcctacctc agcctcctgg tgcaccacca cccccagcta 157020

atttttgtat tttttgtata gatggggtct caccatgttg ctcaggctgg tctcgaactc 157080

ctggctcaag ggatccacct gccttgccct cccaaaatgc tgagattata ggcatgagca 157140

attgcgcctg gctgcgtttg acttgtttac tcctgtctac ccagcaaaga catcagatgg 157200

atgggtgggc agatggatgg atgagaaagc ctcactgaga aagtgacatg ccaatcaaaa 157260

gaagaagaac aaggaaccag ccctgccagt tccaccagca ggagagcagt gtgtgtgagc 157320

atgtggagag accccaccac gggaacctgg gcatatccag ctggaggcag cccatgttgc 157380

ctggtgcaca atgaataagg aggagaatgg tatgagatga agtatgtgag ttgatcaggg 157440

gcctcaggcc tcatggcttt agtagctagt catgaggagt tctatttagt ctaaaaagtg 157500

ataagtatac cctgacaatg actgaggtgt ctctatggaa tgagtatact cactaagaaa 157560

aataagtagc ataatgtgat ttgacttgct gccagtcaca gtgagtgggt gatgaggatt 157620

aagacttggt tcttagcagc cagtgcagtg gctcacgcct ataatcccag cttgaggcca 157680

ggattttgag agtagcctgg gcaacatagt gagacctcac ctctacaaaa cattagaaaa 157740

aaaaagactt ggttcttaga aataccagcc cacattctgt ccatttagca catgaagctt 157800

tttttttttt cttttttctt tttttccttg aactggaggt agggggatag tgtagagaaa 157860

gaatagaaaa gggtttatag tataatggtt tagagttacg aactcagact gcctgcattt 157920

aattgccaga gttcccattt gccatctggg tgacaatgga taagttacac aaatctctac 157980

acctcaacct cctttccctg tatgtcaaat caggataata aagtgtgtac tattatctca 158040

gaggtatggt gaggtttaaa taaaataacc cacattcaat acttagcatt gttcctgtcc 158100

agtggctcga gggatctgga acatttgggc cagaaaagag aggagaccac tcacaaatat 158160

ttgaaagttt tgctgagaag aggaattata cattatttgc atgtcctatt gaggacaatg   158220

aagagttatg ggagagaatt caagggcaag ggcaacccaa ggtaaggaag acccaacaca   158280

gccaaggatc aataaagg                                                 158298

<210>  5
<211>  24
<212>  DNA
<213>  Artificial Sequence

<220>
<223>  Forward primer of CUX1 gene (exon 24)

<400>  5
catctaatgg cttcagagcg ataa                                          24

<210>  6
<211>  24
<212>  DNA
<213>  Artificial Sequence

<220>
<223>  Reverse primer of CUX1 gene (exon 24)

<400>  6
gcaatctctt agtggcaact gaac                                          24

<210>  7
<211>  22
<212>  DNA
<213>  Artificial Sequence

<220>
<223>  Forward primer of ABL gene

<400>  7
gccctagctt tacgctcatc ac                                            22

<210>  8
<211>  24
<212>  DNA
<213>  Artificial Sequence

<220>
<223>  Reverse primer of ABL gene

<400>  8
gctgtaagaa ccgcataaaa cgat                                          24

<210>  9
<211>  20
<212>  DNA
<213>  Artificial Sequence

<220>
<223>  Forward primer of D7S527 microsatellite marker

<400>  9
cattgcaaac tcaggagata                                               20

<210>  10
<211>  20
<212>  DNA
<213>  Artificial Sequence

<220>
<223>  Reverse primer of D7S527 microsatellite marker

<400>  10
taacagaggc atgaaaacca                                                    20


<210>  11
<211>  20
<212>  DNA
<213>  Artificial Sequence

<220>
<223>  Forward primer of D7S2480 microsatellite marker

<400>  11
accttgactg tggtagttgg                                                    20


<210>  12
<211>  23
<212>  DNA
<213>  Artificial Sequence

<220>
<223>  Reverse primer of D7S2480 microsatellite marker

<400>  12
gttcacctca taggaaaatc ttg                                                23


<210>  13
<211>  20
<212>  DNA
<213>  Artificial Sequence

<220>
<223>  Forward primer of D7S662 microsatellite marker

<400>  13
gttgacagac aagcacagac                                                    20


<210>  14
<211>  18
<212>  DNA
<213>  Artificial Sequence

<220>
<223>  Reverse primer of D7S662 microsatellite marker

<400>  14
agctgtttcc catttcca                                                      18


<210>  15
<211>  16
<212>  DNA

<213>  Artificial Sequence

<220>
<223>  Forward primer of D7S2536 microsatellite marker


<400>  15
acactccgcc accttg                                                    16


<210>  16
<211>  21
<212>  DNA
<213>  Artificial Sequence

<220>
<223>  Reverse primer of D7S2536 microsatellite marker

<400>  16
caacaactgt tcctaaagcc t                                              21


<210>  17
<211>  24
<212>  DNA
<213>  Artificial Sequence

<220>
<223>  Forward primer of D7S515 microsatellite marker

<400>  17
gggagttact accctcactt aatg                                           24


<210>  18
<211>  16
<212>  DNA
<213>  Artificial Sequence

<220>
<223>  Reverse primer of D7S515 microsatellite marker

<400>  18
ggactgggca gcaaag                                                    16


<210>  19
<211>  20
<212>  DNA
<213>  Artificial Sequence

<220>
<223>  Forward primer of D7S500 microsatellite marker

<400>  19
ccagaattga aaactcagca                                                20


<210>  20
<211>  25
<212>  DNA
<213>  Artificial Sequence

<220>
<223>  Reverse primer of D7S500 microsatellite marker

<400>  20
attgattgag gaactgaact tacct                                          25


<210>  21
<211>  22
<212>  DNA
<213>  Artificial Sequence

<220>
<223>  Forward primer of D7S500 microsatellite marker

<400>  21
tctcatctgt gaatgcacta tc                                             22


<210>  22
<211>  21
<212>  DNA
<213>  Artificial Sequence

<220>
<223>  Reverse primer of D7S500 microsatellite marker

<400>  22
gcagttgggt tatttcaagt c                                              21


<210>  23
<211>  18
<212>  DNA
<213>  Artificial Sequence

<220>
<223>  Forward primer of CUX1 gene(exons 2-4)

<400>  23
cgaaccggca ggatgaaa                                                  18


<210>  24
<211>  23
<212>  DNA
<213>  Artificial Sequence

<220>
<223>  Reverse primer of CUX1 gene (exons 2-4)

<400>  24
tctgggacgt caatcaatct ttt                                            23


<210>  25
<211>  18
<212>  DNA
<213>  Artificial Sequence

<220>
<223>  Forward primer of SH2B2 gene (exón 6)

<400>  25
ctgcgcctgt ccctgaac                                                  18

<210>  26
<211>  18
<212>  DNA
<213>  Artificial Sequence

<220>
<223>  Reverse primer of SH2B2 gene (exon 6)

<400>  26
ggaagtggcg gagcatgt                                                    18


<210>  27
<211>  24
<212>  DNA
<213>  Artificial Sequence

<220>
<223>  Forward primer of SH2B2 gene (exons 3-4)

<400>  27
cacattcgtc ctcaaggtag agaa                                             24


<210>  28
<211>  18
<212>  DNA
<213>  Artificial Sequence

<220>
<223>  Reverse primer of SH2B2 gene (exons 3-4)

<400>  28
cacgcagccc tggatgtc                                                    18


<210>  29
<211>  20
<212>  DNA
<213>  Artificial Sequence

<220>
<223>  Forward primer of MUC3A/MUC3B gene (exón 1)

<400>  29
cctcattgca aacttcactc                                                  20


<210>  30
<211>  20
<212>  DNA
<213>  Artificial Sequence

<220>
<223>  Reverse primer of MUC3A/MUC3B gene (exon 1)

<400>  30
agcccacatt ttctgtactg                                                  20


<210>  31
<211>  21
<212>  DNA
<213>  Artificial Sequence

<220>
<223>  Forward primer of MUC3A/MUC3B gene

<400>  31
ctcacccaat gcttccagtt c                                                21


<210>  32
<211>  20
<212>  DNA
<213>  Artificial Sequence

<220>
<223>  Reverse primer of MUC3A/MUC3B gene

<400>  32
atggtgagcg ggatggtaga                                                  20


<210>  33
<211>  21
<212>  DNA
<213>  Artificial Sequence

<220>
<223>  Forward primer of MUC3A/B gene

<400>  33
ggaaaatggg tgagtgagta g                                                21


<210>  34
<211>  20
<212>  DNA
<213>  Artificial Sequence

<220>
<223>  Reverse primer of MUC3A/B gene

<400>  34
gtaggcgatt ttggtagtgg                                                  20


<210>  35
<211>  20
<212>  DNA
<213>  Artificial Sequence

<220>
<223>  Forward primer of MUC3A/B gene

<400>  35
ttgaaaccac tccacccacc                                                  20


<210>  36
<211>  17
<212>  DNA
<213>  Artificial Sequence

<220>
<223>  Reverse primer of MUC3A/B gene

<400>  36
gaagtgattg aagaagt                                                     17

<210>  37
<211>  20
<212>  DNA
<213>  Artificial Sequence

<220>
<223>  Forward primer of CUX1 gene (exon 2)

<400>  37
gacccatgat gttgaacgag                                                    20


<210>  38
<211>  20
<212>  DNA
<213>  Artificial Sequence

<220>
<223>  Reverse primer of CUX1 gene (exon 2)

<400>  38
aagatccctt caaagcgaac                                                    20


<210>  39
<211>  20
<212>  DNA
<213>  Artificial Sequence

<220>
<223>  Forward primer of CUX1 gene (exon 3)

<400>  39
ggccttaacc aatgtttccc                                                    20


<210>  40
<211>  20
<212>  DNA
<213>  Artificial Sequence

<220>
<223>  Reverse primer of CUX1 gene (exon 3)

<400>  40
caccacagct ctgtgtattc                                                    20


<210>  41
<211>  20
<212>  DNA
<213>  Artificial Sequence

<220>
<223>  Forward primer of CUX1 gene (exon 4)

<400>  41
tctgctcctc ccttgtttac                                                    20


<210>  42
<211>  20
<212>  DNA

<213>  Artificial Sequence

<220>
<223>  Reverse primer of CUX1 gene (exon 4)

<400>  42
agaatgagcc aaaaacccac                                                    20